# EUROPEAN PATENT SPECIFICATION

(11) **EP 1 045 904 B1**
(45) Date of publication and mention of the grant of the patent: **25.04.2007**
(21) Application number: 99902983.8
(22) Date of filing: 14.01.1999
(51) Int. Cl.: C12N 15/12, C12N 9/00, C12N 15/62, G01N 33/68, C12Q 1/68, C07K 16/40

(54) **FATTY ACID TRANSPORT PROTEINS**
FETTSÄURE TRANSPORTPROTEINE
PROTEINES TRANSPORTEUSES D'ACIDES GRAS

(30) Priority: 15.01.1998 US 71374 P; 20.07.1998 US 93491 P; 04.12.1998 US 110941 P; 14.01.1999 US 232197; 14.01.1999 US 232200; 14.01.1999 US 232201; 14.01.1999 US 232195
(43) Date of publication of application: 25.10.2000
(73) Proprietor: WHITEHEAD INSTITUTE FOR BIOMEDICAL RESEARCH, Cambridge, MA 02142 (US); MILLENNIUM PHARMACEUTICALS, INC., Cambridge, MA 02142-1017 (US)
(72) Inventor: STAHL, Andreas, Allston, MA 02134 (US); HIRSCH, David, J., Brookline, MA 02146 (US); LODISH, Harvey, F., Brookline, MA 02146 (US); GIMENO, Ruth, E., Wellesley, MA 02481 (US); TARTAGLIA, Louis, A., Newton, MA 02159 (US)
(74) Representative: Arends, William Gerrit
(86) International application number: PCT/US1999/000182
(87) International publication number: WO 1999/036537

(56) References cited:
- US-A- 4 935 450
- DATABASE EMBL - EMHUM1 [Online] Entry/Acc.no. D88308, 1 December 1997 (1997-12-01) KAMIJO, K.: "Homo sapiens mRNA for very-long-chain acyl-CoA synthase, complete cds." XP002107854
- DATABASE EMBL - EMEST1 [Online] Entry/Acc.no. Aa581592, 11 September 1997 (1997-09-11) STRAUSBERG, R.: "nc84e10.s1 NCI_CGAP_GC1 Homo sapiens cDNA clone IMAGE:797514." XP002116951
- DATABASE EMBL - EMEST25 [Online] Entry HSAA69992, Acc.no. AA469992, 21 June 1997 (1997-06-21) HILLIER, L. ET AL.: "zu10c02.r1 Soares testis NHT Homo sapiens cDNA clone 731426 5'." XP002116952
- DATABASE EMBL - EMEST1 [Online] Entry/Acc.no. Aa614135, 13 October 1997 (1997-10-13) STRAUSBERG, R.: "no82f09.s1 NCI_CGAP_AA1 Homo sapiens cDNA clone IMAGE:1113353 similar to TR:G563829 G563829 FATTY ACID TRANSPORT PROTEIN." XP002116953
- DATABASE EMBL - EMEST14 [Online] Entry HS808343, Acc.no.W48808, 30 May 1996 (1996-05-30) HILLIER, L. ET AL.: "zc44h06.r1 Soares senescent fibroblasts NbHSF Homo sapiens cDNA clone 325211 5' similar to PIR:A55093 A55093 fatty acid transport protein precursor - mouse." XP002116954
- DATABASE EMBL - EMEST1 [Online] Entry/Acc.no. AA614445, 13 October 1997 (1997-10-13) STRAUSBERG, R.: "nn89d05.s1 NCI_CGAP_Br2 Homo sapiens cDNA clone IMAGE:1098345 similar to TR:G563829 G563829 FATTY ACID TRANSPORT PROTEIN." XP002116955
- UCHIYAMA, A. ET AL.: "Molecular cloning of cDNA encoding rat very long-chain acyl-CoA synthetase." JOURNAL OF BIOLOGICAL CHEMISTRY, vol. 271, no. 48, 29 November 1996 (1996-11-29), pages 30360-5, XP002107850
- HARMON, C.M. ET AL.: "Labelling of an 88 kDa adipocyte membrane protein by sulpho-N-succinimidyl long-chain fatty acid: inhibition of fatty acid transport." BIOCHEMICAL SOCIETY TRANSACTIONS, vol. 20, no. 4, November 1992 (1992-11), pages 811-3, XP002107851
- DATABASE EMBL - EMEST25 [Online] Entry HSAA70762, Acc.no.AA470762, 21 June 1997 (1997-06-21) STRAUSBERG, R.: "ne19b11.s1 NCI_CGAP_Co3 Homo sapiens cDNA clone IMAGE:881661." XP002116956
- SCHAFFER ET AL: "Expression cloning and characterization of a novel adipocyte long chain fatty acid transport protein" CELL, vol. 79, 4 November 1994 (1994-11-04), pages 427-436, XP002081438 cited in the application
- SCHAFFER ET AL: "Cloning and structure-function analysis of human heart fatty acid transport protein" CIRCULATION, vol. 96, no. 8-suppl., 9 November 1997 (1997-11-09), page 2031 XP002082456
- GHOSH, B. ET AL.: "Molecular cloning and sequencing of human palmitoyl-CoA ligase and its tissue specific expression." MOLECULAR AND CELLULAR BIOCHEMISTRY, vol. 151, 1995, pages 77-81, XP002107852
- FITSCHER, B.A. ET AL.: "Tissue distribution and cDNA cloning of a human fatty acid transport protein (hsFATP4)." BIOCHIMICA ET BIOPHYSICA ACTA, vol. 1443, 22 December 1998 (1998-12-22), pages 381-5, XP002116950
- HIRSCH, D. ET AL.: "A family of fatty acid transporters conserved from mycobacterium to man" PROC.NAT'L.ACAD.SCI.USA, vol. 95, July 1998 (1998-07), pages 8625-9, XP002107853

## Description

### GOVERNMENT SUPPORT

The invention was supported, in whole or in part, by National Institutes of Health Grant DK 47618 and National Institutes of Health Grant 5 T32 CA 09541. The United States Government has certain rights in the invention.

### BACKGROUND OF THE INVENTION

Long chain fatty acids (LCFAs) are an important source of energy for most organisms. They also function as blood hormones, regulating key metabolic functions such as hepatic glucose production. Although LCFAs can diffuse through the hydrophobic core of the plasma membrane into cells, this nonspecific transport cannot account for the high affinity and specific transport of LCFAs exhibited by cells such as cardiac muscle, hepatocytes, enterocytes, and adipocytes. The molecular mechanisms of LCFA transport remains largely unknown. Identifying these mechanisms can lead to pharmaceuticals that modulate fatty acid uptake by the intestine and by other organs, thereby alleviating certain medical conditions (e.g. obesity).

### SUMMARY OF THE PRESENT INVENTION

According to one aspect of the present invention, there is provided an isolated polypeptide that:
a) comprises the amino acid sequence of FATP4 in SEQ ID NO: 53;
b) consists of the amino acid sequence of SEQ ID NO:53, or
c) comprises at least 360 contiguous amino acids residues of SEQ ID NO: 53, wherein the polypeptides has FATP4 activity.

According to another aspect of the present invention, there is provided a fusion protein comprising a polypeptide of the invention fused with a heterologous moiety.

According to another aspect of the present invention, there is provided an isolated nucleic acid that:
a) encodes a polypeptide according to Claim 1, or the complement of a nucleic acid that encodes the polypeptide;
b) comprises the nucleotide sequence of SEQ ID NO: 52, or the complement thereof;
c) consists of the nucleotide sequence of SEQ ID NO: 52, or the complement thereof; and/or
d) comprises the nucleotide sequence of the coding region of SEQ ID NO: 52.

According to other aspects of the present invention, there are provided a vector comprising a nucleic acid molecule of the invention or encoding a polypeptide or fusion protein of the invention. Also provided are isolated and/or non-human host cells comprising a vector of the invention.

According to another aspect of the present invention, there is provided a method for producing a polypeptide or a fusion protein of the invention comprising cutting a host cell of the invention under conditions in which the nucleic acid is expressed, thereby producing the polypeptide or fusion protein.

According to a further aspect of the present invention, there is provided a method for identifying an agent which binds to a polypeptide of the invention comprising the steps of contacting the agent with the isolated polypeptide under conditions appropriate for binding of the agent to the isolated polypeptide, and detecting a resulting agent-polypeptide complex.

According to another aspect of the present invention, there is provided a method for identifying an agent which inhibits interaction between a polypeptide of the invention and a ligand of said polypeptide, comprising
(a) combining:
   (1) said isolated polypeptide;
   (2) the ligand of said polypeptide; and
   (3) a candidate agent to be assessed for its ability to inhibit binding between said polypeptide of (1) and the ligand of (2), under conditions appropriate for binding between the said polypeptide of (1) and the ligand of (2);
(b) determining the extent to which said polypeptide of (1) and the ligand of (2) bind; and
(c) comparing the extent determined in (b) with the extent to which binding of said polypeptide of (1) and the ligand of (2) occurs in the absence of the candidate agent to be assessed and under the same conditions appropriate for binding of said polypeptide of (1) with the ligand of (2);
wherein if the extent to which binding of said polypeptide of (1) and the ligand of (2) occurs is less in the presence of the candidate agent than in the absence of the candidate agent, the candidate agent is an agent which inhibits binding between said polypeptide and the ligand of said polypeptide.

According to another aspect of the present invention, there is provided a method for identifying an agent which is an inhibitor or fatty acid uptake by a polypeptide of the invention comprising the steps of:
a) maintaining test cells expressing said polypeptide in the presence of a fatty acid and an agent to be tested as an inhibitor of fatty acid uptake;
b) measuring uptake of the fatty acid in the test cells; and
c) comparing uptake of the fatty acid in the test cells with uptake of the fatty acid in suitable control cells;
wherein lower uptake of the fatty acid in the test cells compared to uptake of the fatty acid in the control cells is indicative that the agent is an inhibitor of fatty acid uptake by said polypeptide.

According to a further aspect of the invention, there is provided a method for identifying an agent which is an inhibitor of a polypeptide of the invention, comprising the steps of
(a) introducing into host cells one or more vectors comprising a polynucleotide expressing said polypeptide;
(b) culturing a first aliquot of the host cells with fatty acid substrate of said polypeptide and with an agent being tested as an inhibitor of said polypeptide;
(c) culturing a second aliquot of the host cells with fatty acid substrate of said polypeptide;
(d) measuring, in the first and second aliquots, uptake of the fatty acid substrate of the host cells;
wherein less uptake of the fatty acid substrate in the first aliquot compared to the second aliquot is indicative that the agent is an inhibitor of said polypeptide.

According to another aspect of the present invention, there is provided a method for identifying an agent which is an inhibitor of a polypeptide of the invention, comprising the steps of :
(a) introducing into cells one or more vectors comprising a polynucleotide encoding said polypeptide;
(b) contacting the host cells with anti-cell surface protein antibody and labeled fatty acid substrate of FATP4;
(c) contacting a first aliquot of the host cells with an agent being tested as an inhibitor of FATP4, while leaving a second aliquot of the host cells uncontacted with the agent;
(d) identifying, in the first and second aliquots, the host cells expressing the cell surface protein by detecting the anti-cell surface protein antibody bound to the host cells; and
(e) measuring, in the first and second aliquots, uptake of the fatty acid substrate of the host cells identified as expressing the cell surface protein;
wherein less uptake of the fatty acid substrate in the first aliquot compared to the second aliquot is indicative that the agent is an inhibitor of FATP4.

According to another aspect of the present invention, there is provided a method for detecting FATP4 in a sample of cells comprising the steps of adding an agent that specifically binds to FATP4 to the sample, and detecting agent specifically bound to FATP4, wherein the agent is an antibody that binds to FATP4.

According to another aspect of the present invention there is provided a method for modulating fatty acid uptake of cells in culture, comprising adding one or more agents that modulate fatty acid uptake by FATP4 to cells comprising FATP4, wherein the agent is a nucleic acid of the invention; an antisense oligonucleotides FATP4; or an antibody that binds the polypeptide of the invention.

According to a further aspect of the invention there is provided a polypeptide, fusion protein, nucleic acid molecule, vector or antibody according to the invention for the use in medicine.

According to a further aspect of the present invention there is provided a pharmaceutical composition comprising a polypeptide, a fusion protein, a nucleic acid, a vector or an antibody of the invention.

According to another aspect of the present invention, there is provided the use of an agent which is an inhibitor of fatty acid uptake by FATP4 protein in the small intestine and the preparation of a medicament for treating a disorder characterized by excessive fatty acid uptake in the small intestine, wherein the agent comprises an antisense oligonucleotide or an antibody of the present invention.

Also described herein is a diverse family of fatty acid transport proteins (FATPs) which are evolutionarily conserved; these FATPs are plasma membrane proteins which mediate transport of LCFAs across the membranes and into cells. Members of the FATP family described herein are present in a wide variety of organisms, from mycobacteria to humans, and exhibit very different expression patterns in tissues among the organisms. FATP family members are expressed in prokaryotic and eukaryotic organisms and comprise characteristic amino acid domains or sequences which are highly conserved across family members. In addition, the function of the FATP gene family is conserved throughout evolution, as shown by the fact that the *Caenorhabditis (C). elegans* and mycobacterial FATPs described herein facilitate LCFA uptake when they are overexpressed in COS cells or *Escherichia (E.) coli,* respectively. FATPs are expressed in a wide variety of tissues, including all tissues which are important to fatty acid metabolism (uptake and processing).

FATPs described herein are from such diverse organisms as humans *(Homo (H.) sapiens),* mice, (*Mus* (M.) musculus), *F. rubripes, C. elegans, Drosophila (D.) melanogaster, Saccharomyces (S.) cerevisiae, Aspergillus nidulans, Cochliobolu heterostrophus, Magnaporthe grisea* and *Mycobacterium (M.),* such as *M. tuberculosis.* As described herein, four novel mouse FATPs, referred to as mmFATP2, mmFATP3, mmFATP4 and mmFATP5, and six human FATPs, referred to as hsFATP1, hsFATP2, hsFATP3, hsFATP4, hsFATP5 and hsFATP6, have been identified. All four novel murine FATPs (mmFATP2-5) and a previously identified murine FATP (renamed herein FATP1) have orthologs in humans (hsFATP1-5); the sixth human FATP (hsFATP6) does not as yet have a mouse ortholog. The expression patterns of these FATPs vary, as described in detail below.

There are disclosed FATP family members from prokaryotes and eukaryotes, nucleic acids (DNA, RNA) encoding FATPs, and nucleic acids which are useful as probes or primers (e.g., for use in hybridization methods, amplification methods) for example, in methods of detecting FATP-encoding genes, producing FATPs, and purifying or isolating FATP-encoding DNA or RNA. Also the subject of this invention are antibodies (polyclonal or monoclonal) which bind an FATP or FATPs; methods of identifying additional FATP family members (for example, orthologs of those FATPs described herein by amino acid sequence) and variant alleles of known FATP genes; methods of identifying compounds which bind to an FATP, or modulate or alter (enhance or inhibit) FATP function; compounds which modulate or alter FATP function; methods of modulating or altering (enhancing or inhibiting) FATP function and, thus, LCFA uptake into tissues of a mammal (e.g. human) by administering a compound or molecule (a drug or agent) which increases or reduces FATP activity; and methods of targeting compounds to tissues by administering a complex of the compound to be targeted to tissues and a component which is bound by an FATP present on cells of the tissues to which the compound is to be targeted. For example, a complex of a drug to be delivered to the liver and a component which is bound by an FATP present on liver cells (e.g., FATP5) can be administered.

Also disclosed herein modulating or altering (enhancing or inhibiting/reducing) LCFA uptake in the small intestine and, thus, increasing or reducing the number of calories in the form of fats available to an individual. Also disclosed is inhibiting or reducing LCFA uptake in the small intestine in order to reduce circulating fatty acid levels; that is, LCFA uptake in the small intestine is reduced and, therefore, circulating (blood) levels are not as high as they otherwise would be. FATP4 has been shown to be expressed in epithelial cells of the small intestine and particularly in the brush border layer of the small intestine. FATP2 has also been shown to be expressed at low levels in epithelial cells of the small intestine, particularly in the duodenum. In contrast, FATP1, FATP3, FATP5 and FATP6 were not detected in any of the intestinal tissues. Thus, also described herein are FATPs which are present in the epithelial cell layer of the small intestine where they mediate LCFA uptake. These FATPs, particularly FATP4 and also FATP2, are targets for methods and drugs which block their function or activity and are useful in treating obesity, diabetes and heart disease. The ability of these FATPs to mediate fat uptake can be modulated or altered (enhanced or inhibited), thus modulating fat uptake in the small intestine. This can be done, for example, by administering to an individual, such as a human or other animal, a drug which blocks interaction of LCFAs with FATP4 and/or FATP2 in the small intestine, thus inhibiting LCFA passage into the cells of the small intestine. As a result, fat absorption is reduced and, although the individual has consumed a certain quantity of fat, the LCFAs are not absorbed to the same extent they would have been in the absence of the compound administered.

Thus, there is disclosed a method of reducing LCFA uptake (absorption) in the small intestine and, as a result, reducing caloric uptake in the form of fat. A further disclosure is a compound (drug) useful in inhibiting or reducing fat absorption in the small intestine. Another disclosure is of a method of reducing circulating fatty acid levels by administering to an individual a compound which blocks interactions of LCFAs with FATP4 and/or FATP2 in the small intestine, thus inhibiting LCFA passage into cells of the small intestine. As a result, fatty acids pass into the circulatory system at a diminished level and/or rate, and circulating fatty acid levels are lower than they would be in the absence of the compound administered. This method is particularly useful for therapy in individuals who are at risk for or have hyperlipidemia. That is, it can be used to prevent the occurrence of elevated levels of lipids in the blood or to treat an individual in whom blood lipid levels are elevated. Also disclosed is a method of identifying compounds which alter FATP function (and thus, in the case of FATP2 and/or FATP4, alter LCFA uptake in the small intestine).

There is also disclosed herein a method of modulating or altering (enhancing or inhibiting) the function of FATP6, which is expressed at high levels in the heart. A method of inhibiting FATP6 function is useful, for example, in individuals with heart disease, such as ischemia, since reducing LCFA uptake into heart muscle in an individual who has ischemic heart disease, which may be manifested by, for example, angina or heart attack, can reduce symptoms or reduce the extent of damage caused by the ischemia. In this method, a drug which inhibits FATP6 function is administered to an individual who has had or is having a heart attack, to reduce LCFA uptake by the individual's heart and, as a result, reduce the damage caused by ischemia. There is also disclosed a method of targeting a compound, such as a therapeutic drug or an imaging reagent, to heart tissue by administering to an individual (e.g., a human) a complex of the compound and a component (e.g., a LCFA or LCFA-like compound) which is bound by an FATP (e.g., FATP6) present in cells of heart tissue.

In a further method disclosed herein, LCFA uptake by the liver is modulated or altered (enhanced or reduced), in an individual. For example, a drug which inhibits the function of an FATP present in liver (e.g., FATP5) is administered to an individual who is diabetic, in order to reduce LCFA uptake by liver cells and, thus reduce insulin resistance.

Thus there are disclosed methods which are useful to alter, particularly reduce, LCFA uptake in individuals and, as a result, to alter (particularly reduce), availability of the LCFAs for further metabolism. A specific disclosure is of methods useful to reduce LCFA uptake and, thus, fatty acid metabolism in individuals, with the result that caloric availability from fats is reduced, and circulating fatty acid levels are lower than they otherwise would be. These methods are useful, for example, as a means of weight control in individuals, (e.g., humans) and as a means of preventing elevated serum lipid levels or reducing serum lipid levels in humans. FATPs expressed in the small intestine, such as FATP4, are useful targets to be blocked in treating obesity (e.g., chronic obesity) or to be enhanced in treating conditions in which enhanced LCFA uptake is desired (e.g., malabsorption syndrome or other wasting conditions).

The identification of this evolutionarily conserved fatty acid transporter family will allow a better understanding of the mechanisms whereby LCFAs traverse the lipid bilayer as well as yield insight into the control of energy homeostasis and its dysregulation in diseases such as diabetes and obesity.

### BRIEF DESCRIPTION OF THE DRAWINGS

Figure 1 shows the amino acid sequence alignment of FATPs: mmFATP1 (SEQ ID NO:92), mmFATP2 (SEQ ID NO:93), mmFATP3 (SEQ ID NO:94), mmFATP4 (SEQ ID NO:95), mmFATP5 (SEQ ID NO:96), ceFATPa (SEQ ID NO:97), scFATP (SEQ ID NO:98) and mtFATP (SEQ ID NO:99). The underlining (amino acid residues 204-212 of mtFATP) indicates an AMP binding motif which is found in many classes of proteins; the underlining at amino acid residues 204-507 of the mtFATP sequence indicates the FATP 360 amino acid signature sequence.

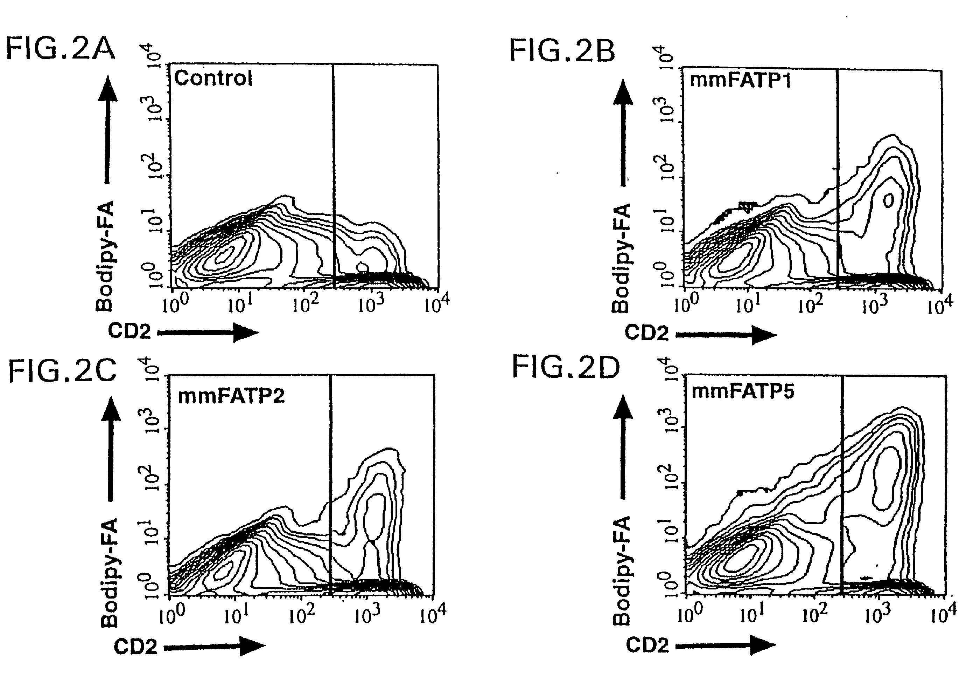

Figures 2A-2D show results of LCFA uptake assays. Figures 2A-2D: COS cells were cotransfected using the DEAE-dextran method with the mammalian expression vectors pCDNA-CD2 either alone (control; Figure 2A) or in combination with one of the FATP-containing expression vectors (pCDNA-mmFATP1, Figure 2B; pCDNA-mmFATP2, Figure 2C; or pCMV-SPORT2-mmFATP5, Figure 2D) as described in Materials and Methods for Example 2. COS cells were gated on forward scatter (FSC) and side scatter (SS), and the results shown represent >10,000 cells. Cells exhibiting >300 CD2 fluorescence units (vertical line) representing 15% of all cells were deemed CD2 positive.
Figure 3 is a graph of fluorescence of cells expressing a FATP gene. As in Figures 2A-2D, COS cells were cotransfected with pCDNA-CD2 either alone (control) or in combination with one of the FATP-containing expression vectors (pCDNA-mmFATP1, pCDNA-mmFATP2, pCMV-SPORT2-mmFATP5, or pCDNA-ceFATPb). The mean BODIPY-FA fluorescence of the CD2-positive cells is plotted; results shown represent the average of three experiments, each consisting of greater than 50,000 COS cells. Note that a logarithmic scale is used on the ordinate.
Figure 4 is a graph of the uptake of palmitate with time. The full-length coding region of mtFATP (squares) or a control protein (TFE3; circles) was subcloned into the inducible, prokaryotic expression vector pET (Novagen). Expression from the resulting plasmid was induced (solid symbols) in transformed *E. coli* cells with 1 mM isopropyl-β-D-thiogalactoside (LPTG) for 1 hour, or cells were left uninduced (open symbols). Data points were done in triplicate and counts were normalized to the number of bacteria as determined by OD₆₀₀.
Figure 5 is a phylogenetic tree produced by aligning complete and partial sequences for *FATP* genes from human, rat, mouse, puffer fish, *D. melanogaster, C. elegans, S. cerevisiae,* and *M*. *tuberculosis* using CluslalX and using these data to produce a phylogenetic tree using TreeViewPPC. The bar indicates the number of substitutions per residue, i.e., 0.1 corresponds to a distance of 10 substitutions per 100 residues.

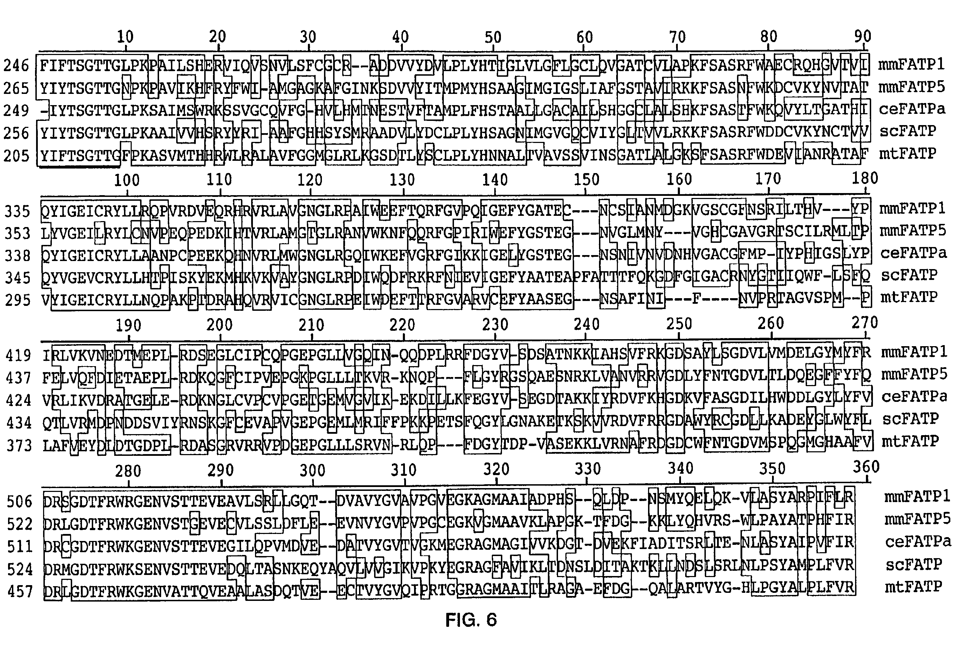

Figure 6 shows a comparison of the FATP signature sequences of mmFATP1 (SEQ ID NO:1), mmFATP5, (SEQ ID NO:2), ceFATPa (SEQ ID NO:3), scFATP (SEQ ID NO:4) and mtFATP (SEQ ID NO:5).

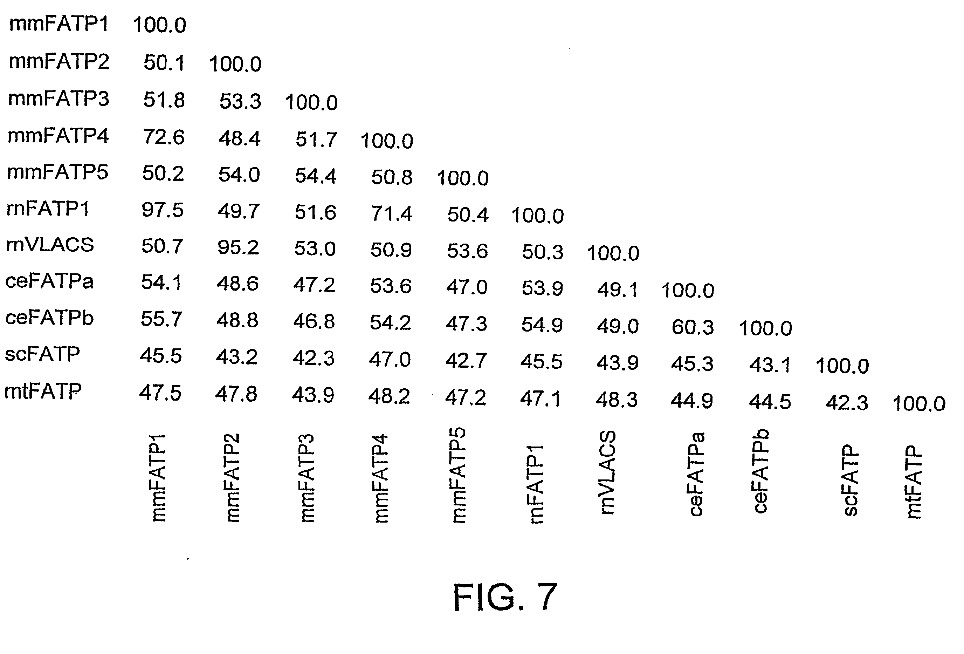

Figure 7 shows the sequence identity among the FATP family members and VLACs, based on the 360 amino acid signature sequence of FATP from Figure 1.

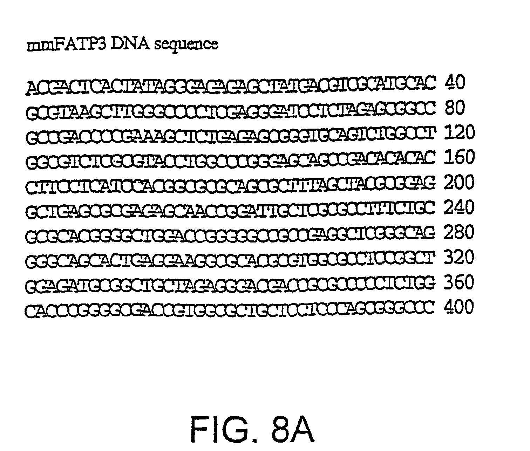

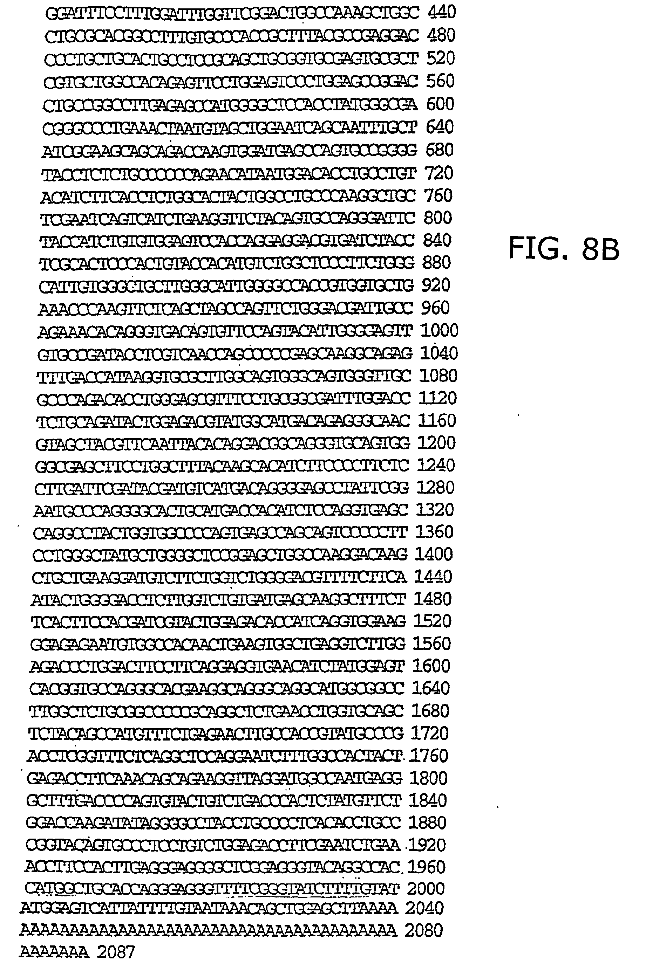

Figures 8A and 8B are the mmFATP3 DNA sequence (SEQ IID NO:6).

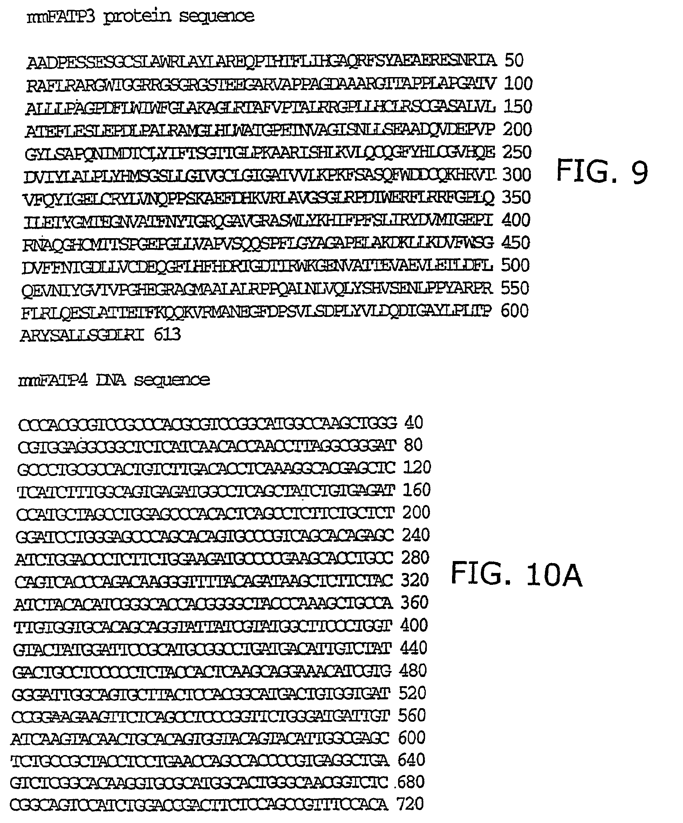

Figure 9 is the mmFATP3 protein sequence (SEQ ID NO:7).

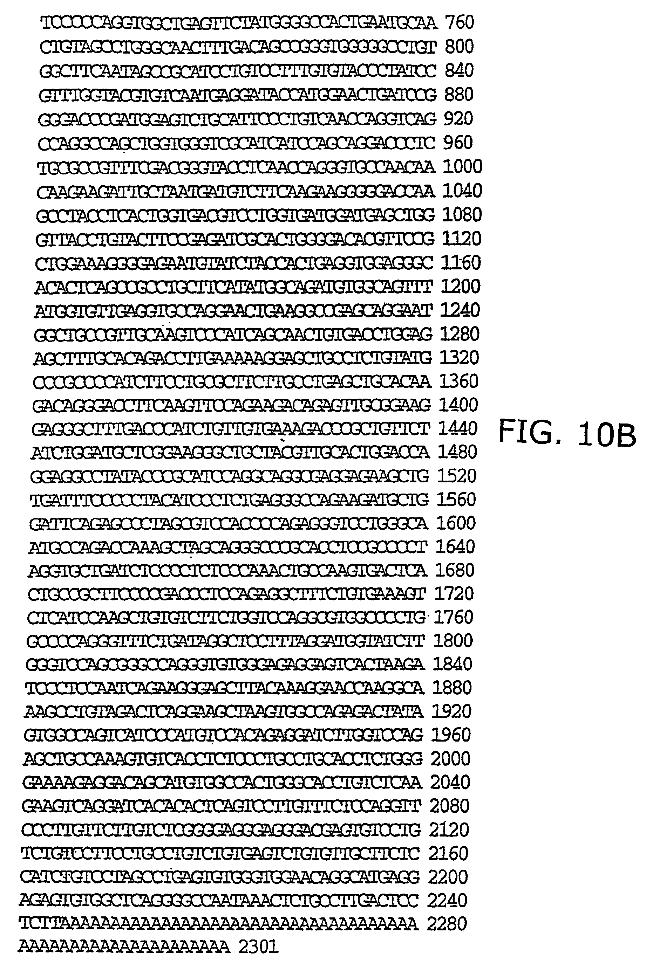

Figures 10A and 10B are the mmFATP4 DNA sequence (SEQ ID NO:8).

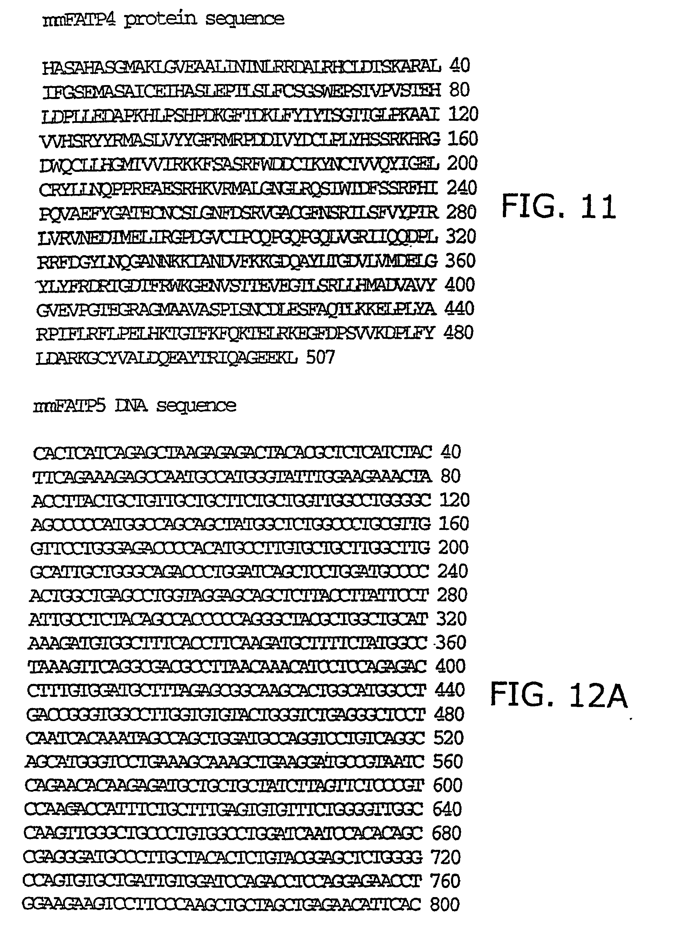

Figure 11 is the mmFATP4 protein sequence (SEQ ID NO:9).

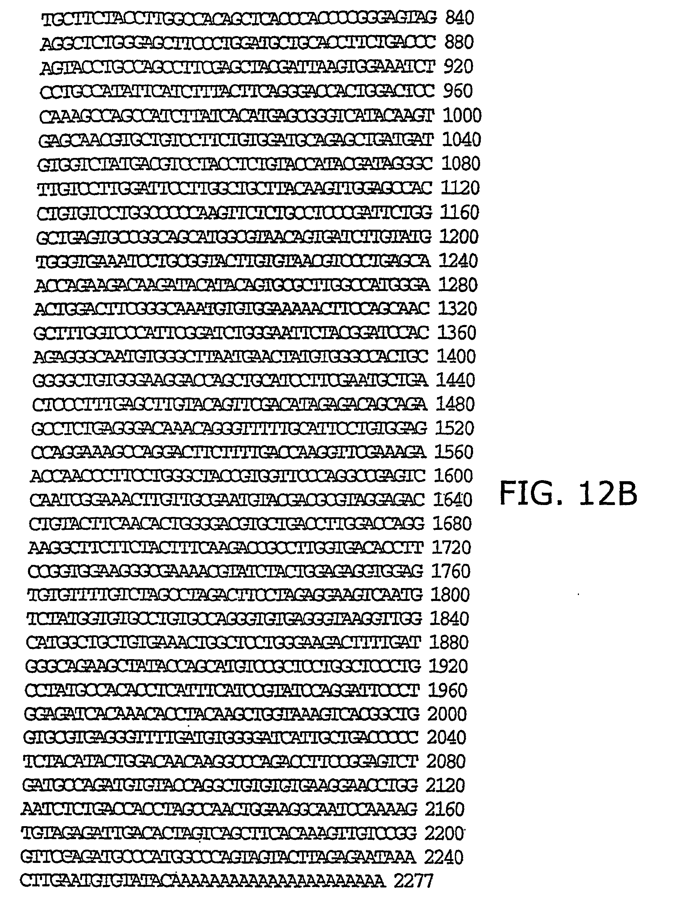

Figures 12A and 12B are the mmFATP5 DNA sequence (SEQ ID NO:10).

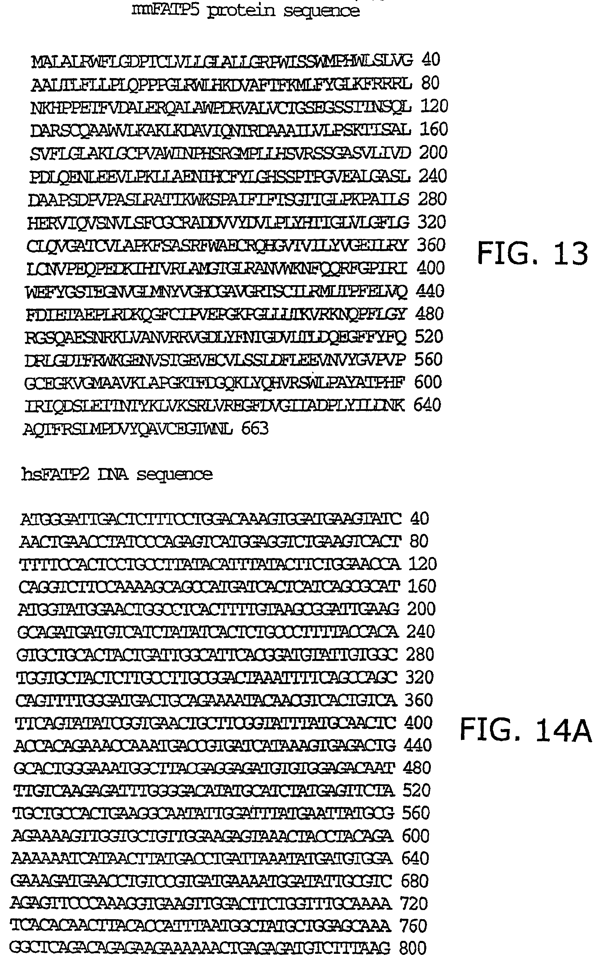

Figure 13 is the mmFATP5 protein sequence (SEQ ID NO:11).

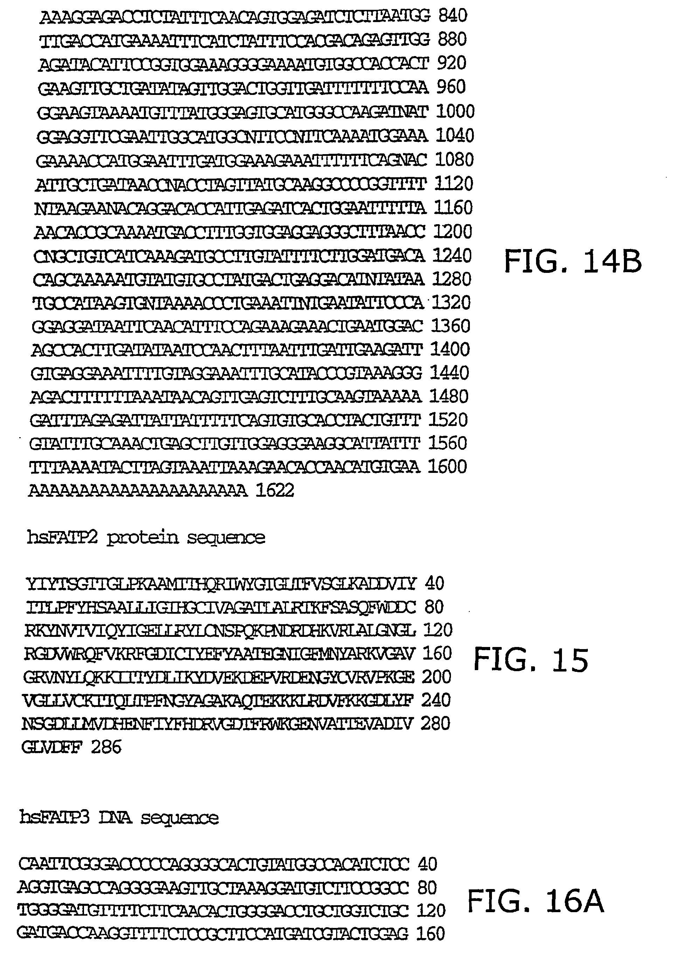

Figures 14A and 14B are the hsFATP2 DNA sequence (SEQ ID NO:12).
Figure 15 is the hsFATP2 protein sequence (SEQ ID NO:13).

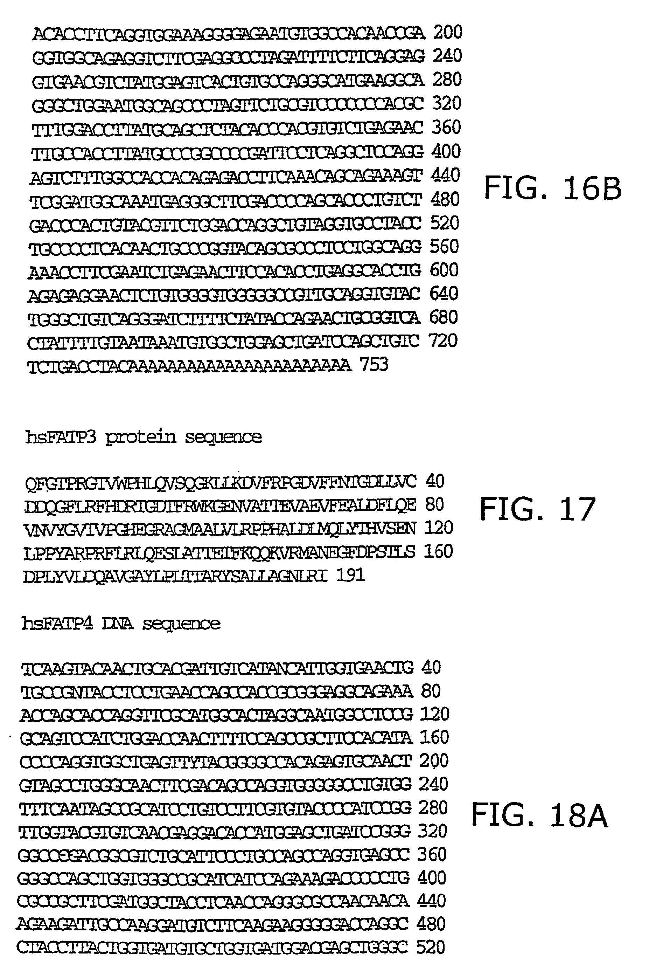

Figures 16A and 16B are the hsFATP3 DNA sequence (SEQ ID NO:14).
Figure 17 is the hsFATP3 protein sequence (SEQ ID NO:15).

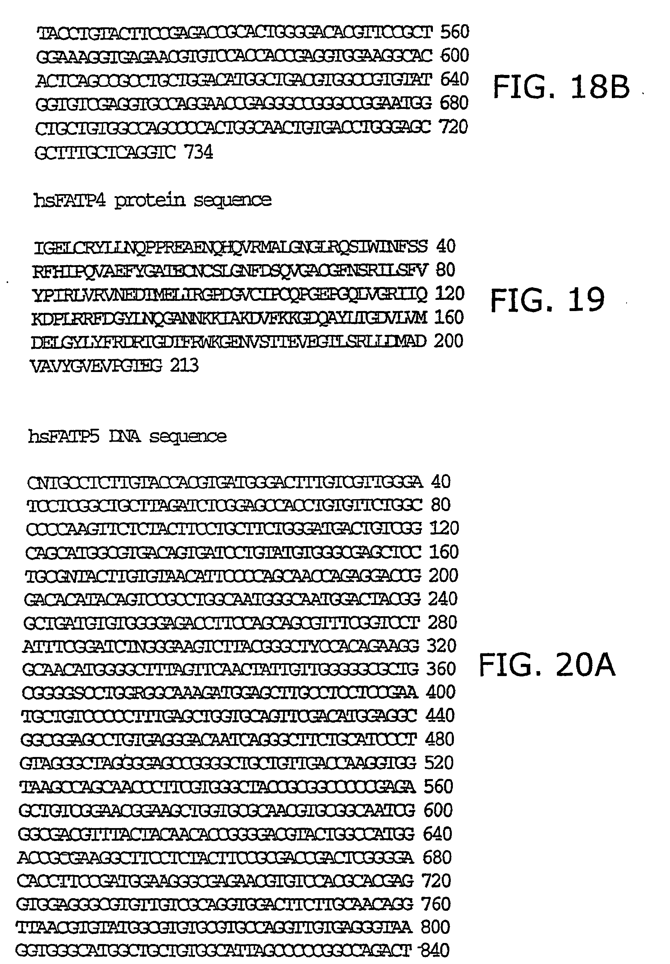

Figures 18A and 18B are the hsFATP4 DNA sequence (SEQ ID NO:16).
Figure 19 is the hsFATP4 protein sequence (SEQ ID NO:17).

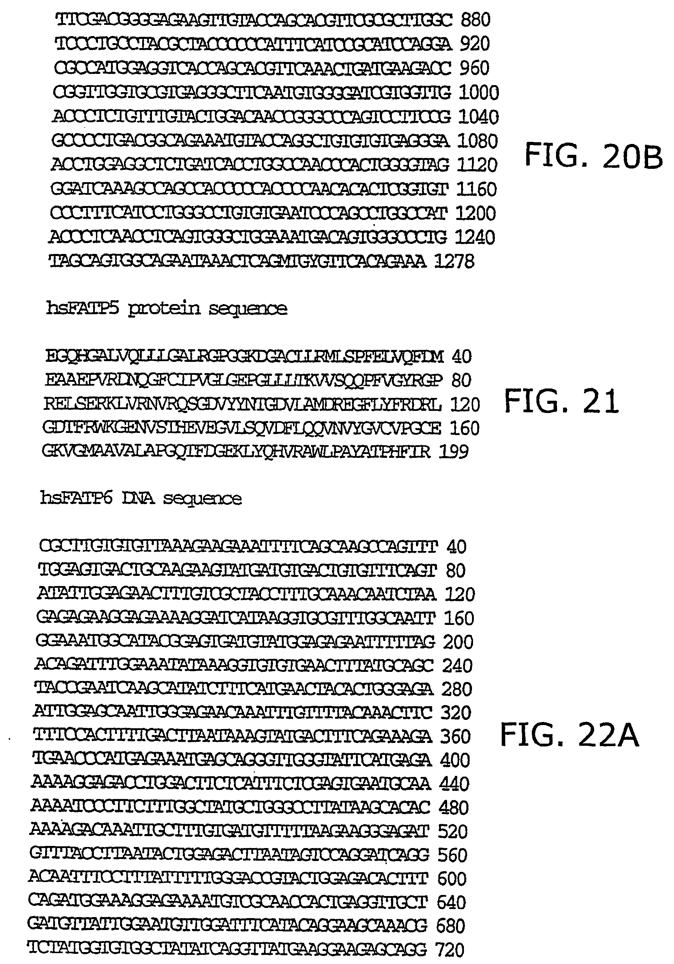

Figures 20A and 20B are the hsFATP5 DNA sequence (SEQ ID NO:18).
Figure 21 is the hsFATP5 protein sequence (SEQ ID NO:19).

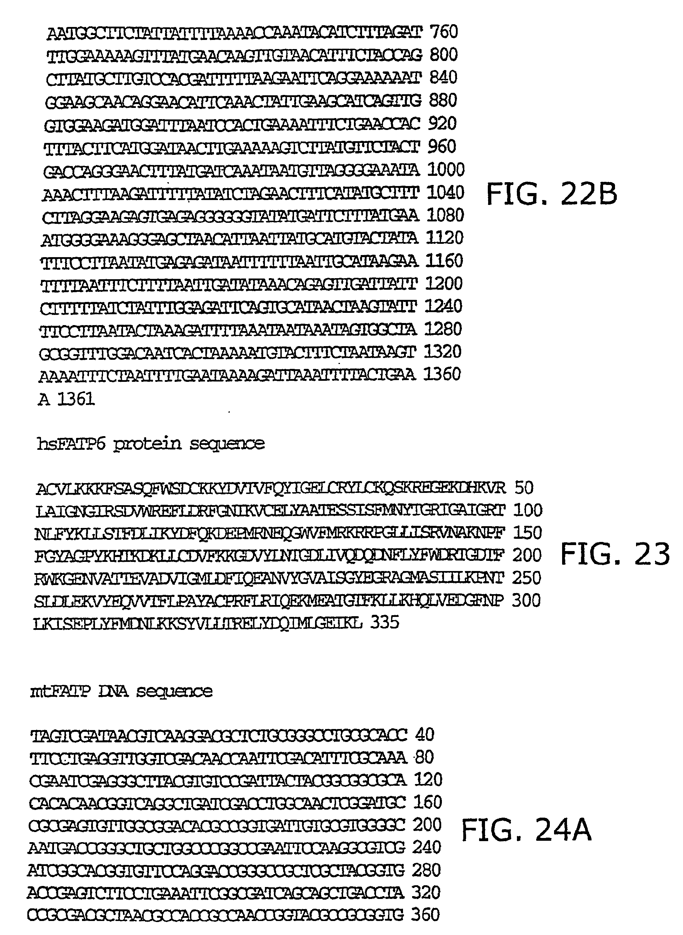

Figures 22A and 22B are the hsFATP6 DNA sequence (SEQ ID NO:20).
Figure 23 is the hsFATP6 protein sequence (SEQ ID NO:21).

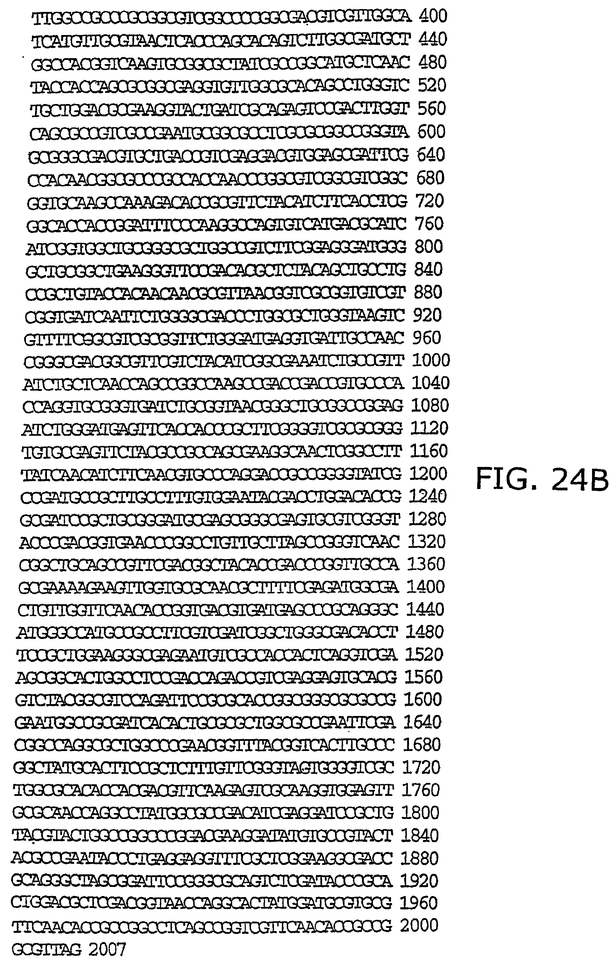

Figures 24A and 24B are the mtFATP DNA sequence (SEQ ID NO:22).

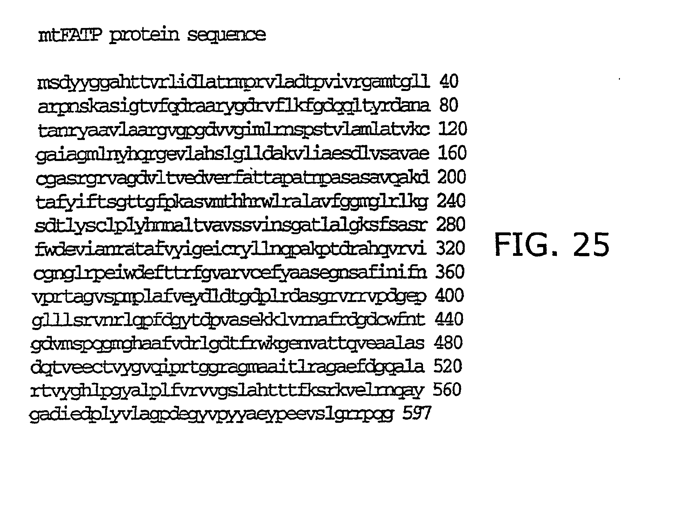

Figure 25 is the mtFATP protein sequence (SEQ ID NO:23).

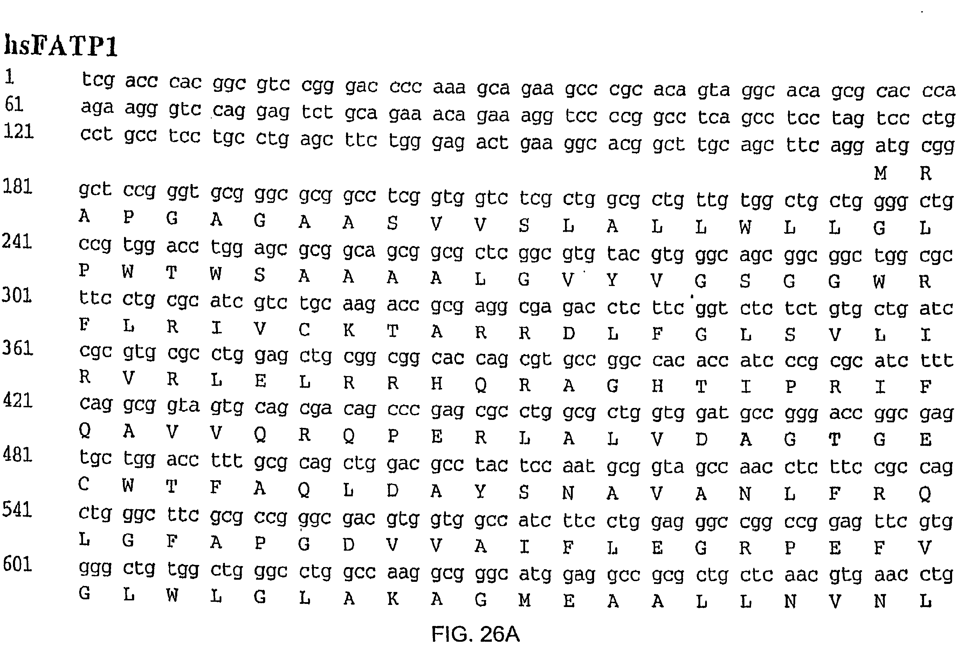

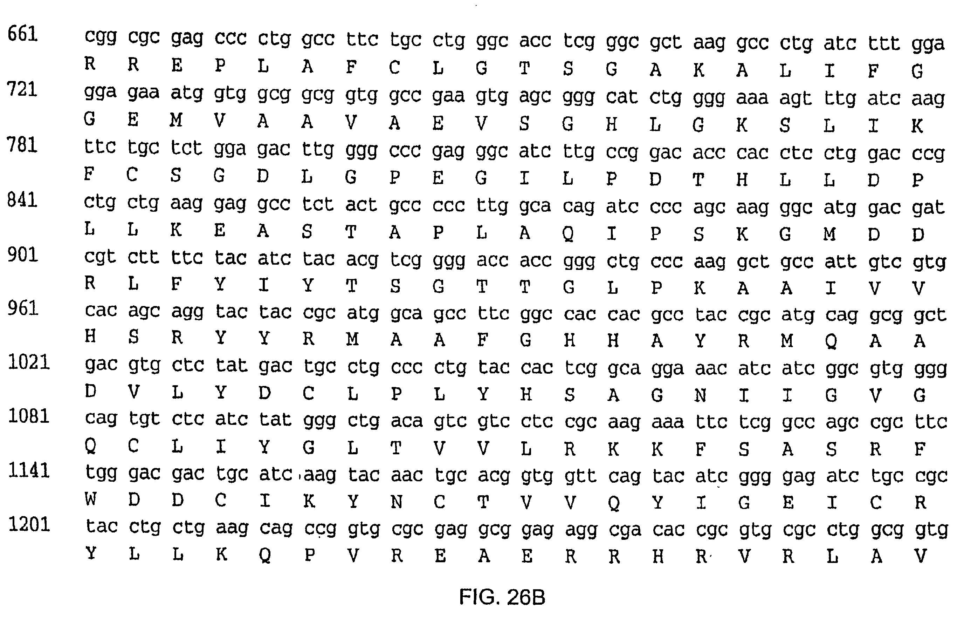

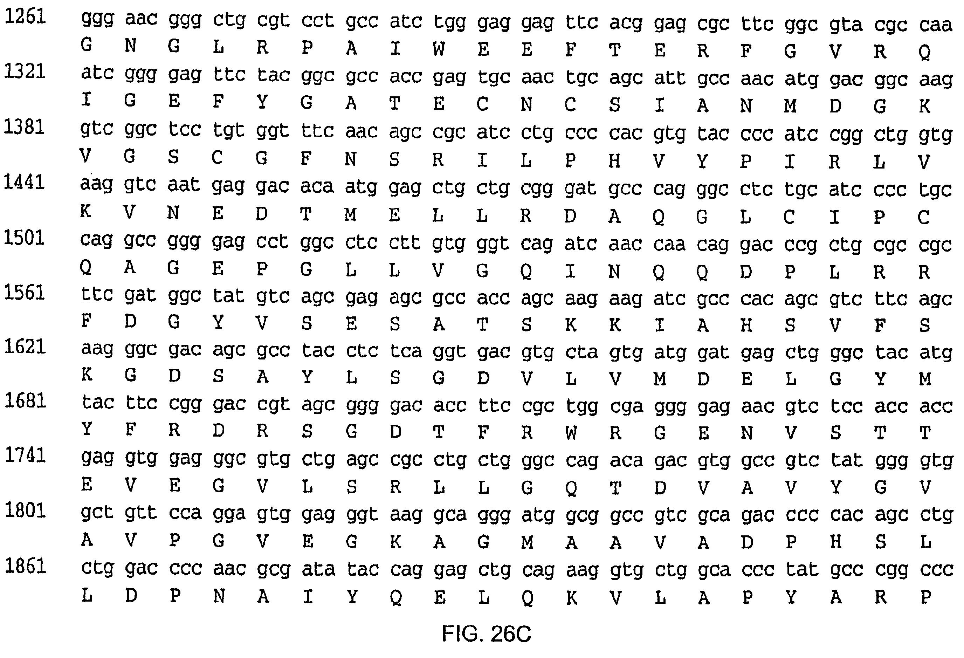

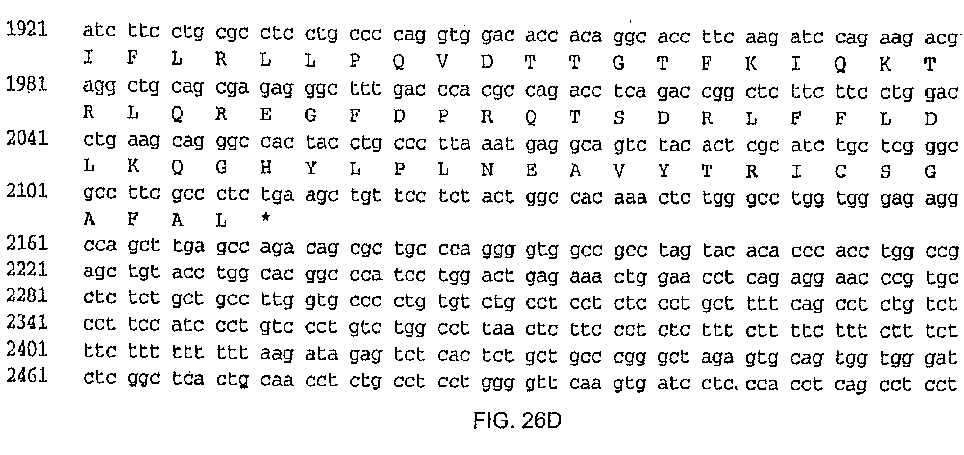

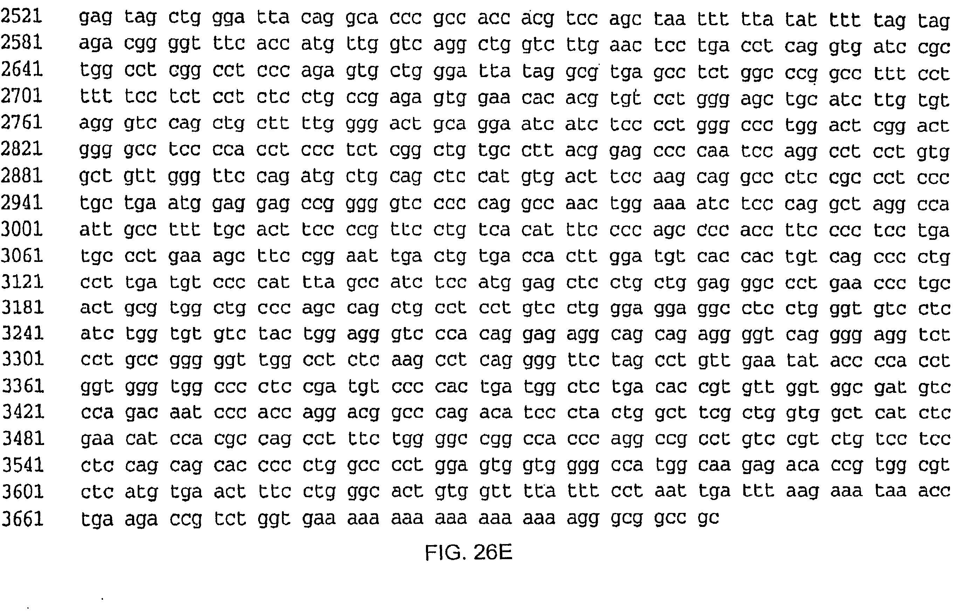

Figures 26A-26E show the DNA sequence (SEQ ID NO:24) and predicted amino acid sequence (SEQ ID NO:25) of human FATP1.

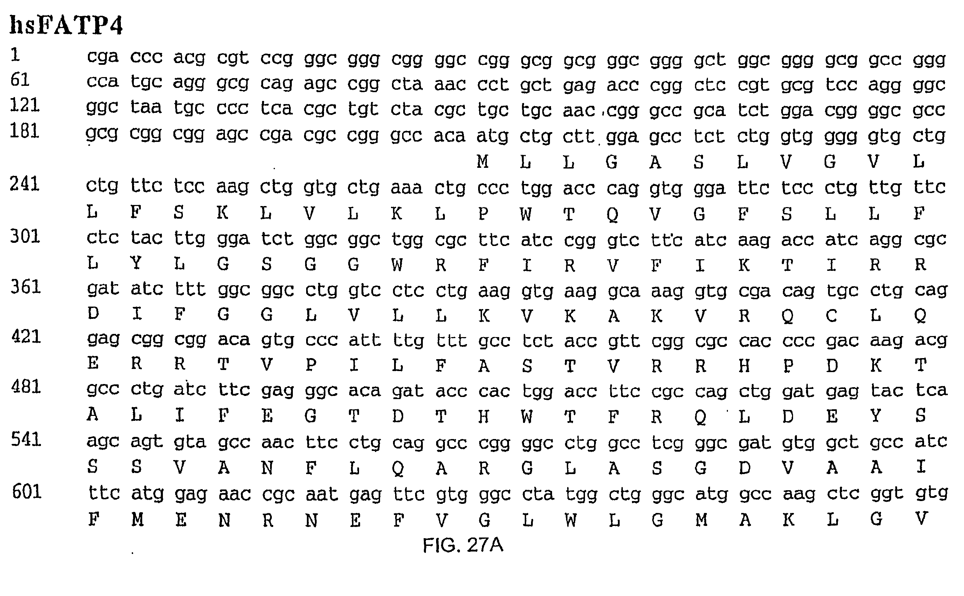

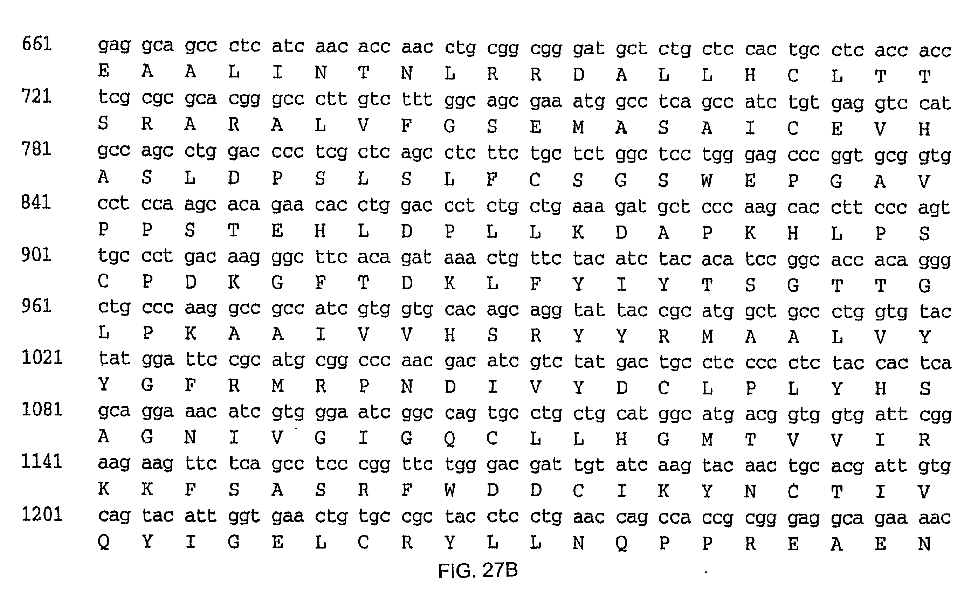

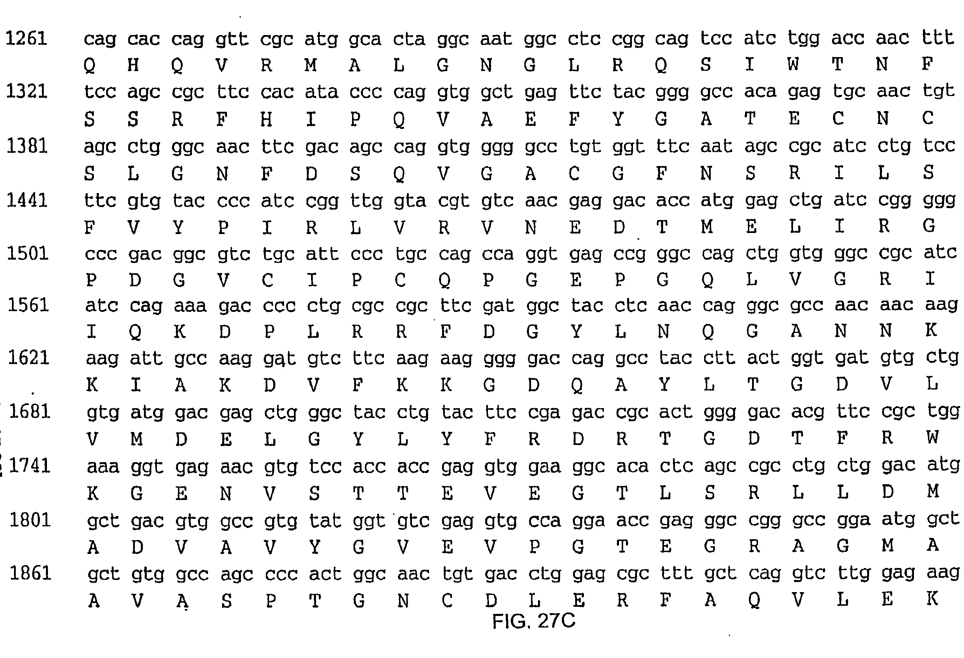

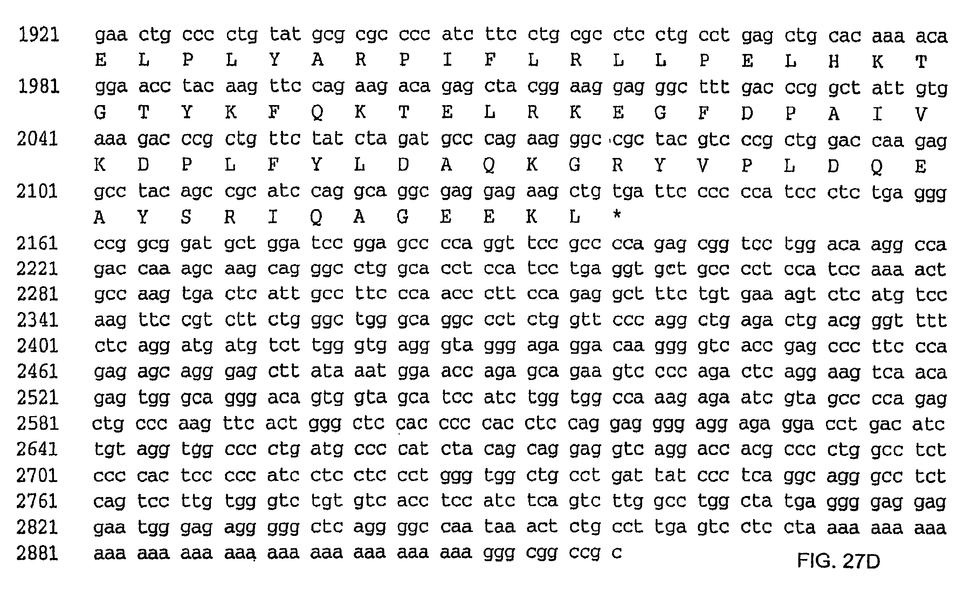

Figures 27A-27D show the DNA sequence (SEQ ID NO:26) and predicted amino acid sequence (SEQ ID NO:27) of human FATP4.
Figure 28A is a hydrophobicity plot for hsFATP1, showing that it has multiple membrane-spanning domains.
Figure 28B is the amino acid composition of hsFATP1.
Figure 28C is a hydrophilicity plot for hsFATP1, made using the Kyte-Doolittle method, averaging hydrophilicity values for 18 amino acid residues at a time.
Figure 29A is a hydrophobicity plot for hsFATP4, showing that it has multiple membrane-spanning domains.
Figure 29B is a listing of the amino acid composition of hsFATP4.
Figure 29C is a hydrophilicity plot for hsFATP4, made using the Kyte-Doolittle method, averaging hydrophilicity values for 18 amino acid residues at a time.
Figures 30A-30I show a comparison of the nucleotide sequence of human FATP 1 (SEQ ID NO:28) and the nucleotide sequence of mouse FATP 1 (SEQ ID NO:29).
Figures 31A-31I show a comparison of the nucleotide sequence of human FATP4 (SEQ ID NO:30) and the nucleotide sequence of mouse FATP4 (SEQ ID NO:31).
Figures 32A-32C show a comparison of the amino acid sequence of human FATP1 (SEQ ID NO:32) and the amino acid sequence of mouse FATP1 (SEQ ID NO:33).
Figures 33A-33C show a comparison at the amino acid level of human FATP4 (SEQ ID NO:34) and mouse FATP4 (SEQ ID NO:35).

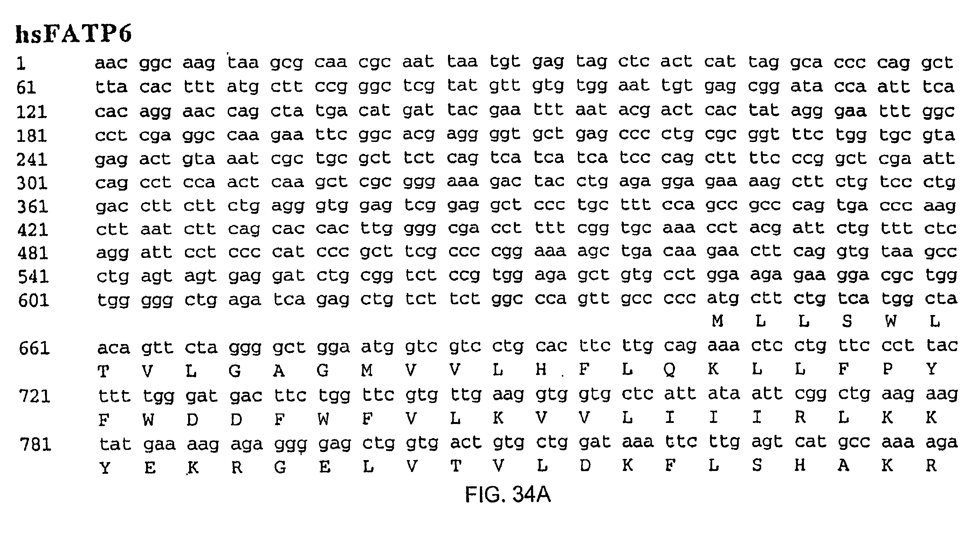

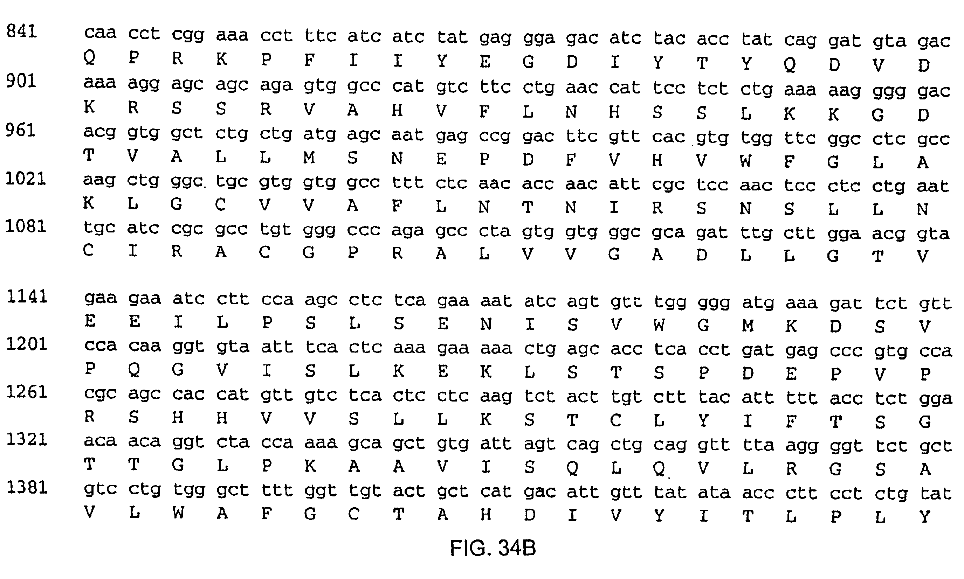

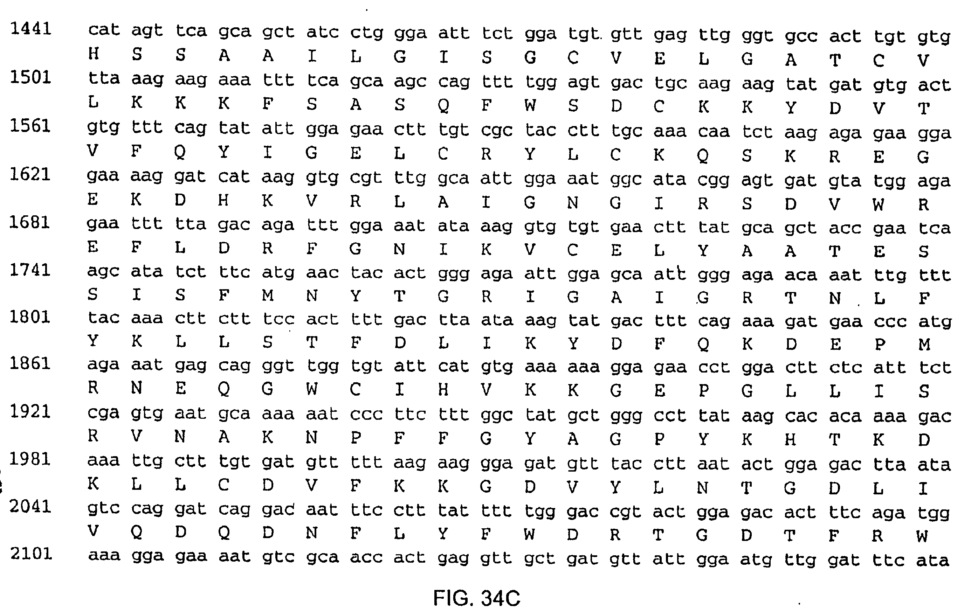

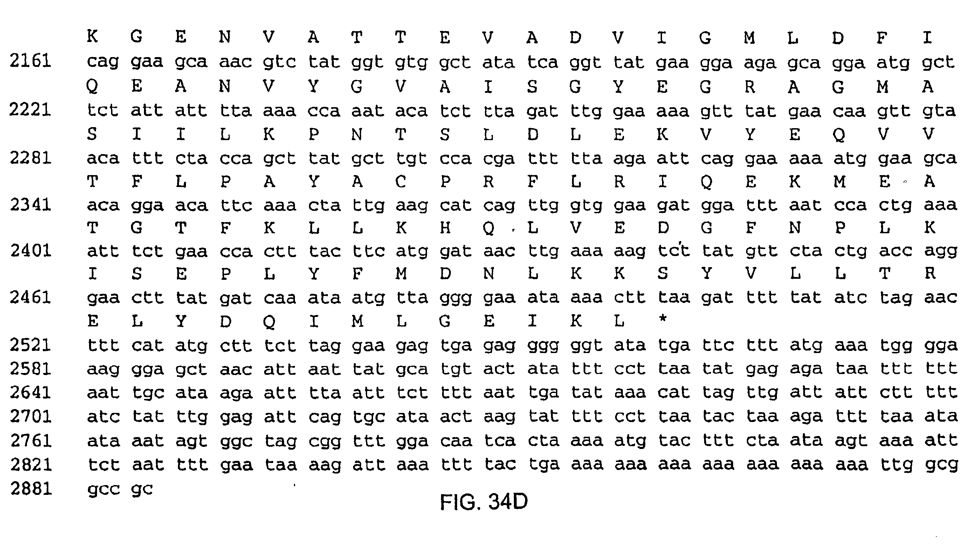

Figures 34A-34D show the nucleotide sequence (SEQ ID NO:36) and predicted amino acid sequence (SEQ ID NO:37) of hsFATP6.
Figure 35A is a hydrophobicity plot for hsFATP6, showing that it has multiple membrane-spanning domains.
Figure 35B is a listing of the amino acid composition of hsFATP6.
Figure 35C is a hydrophilicity plot for hsFATP6, made using the Kyte-Doolittle method, averaging hydrophilicity values for 18 amino acid residues at a time.
Figures 36A-36G show an alignment of the amino acid sequences of hsFATP1 (SEQ ID NO:38), hsFATP4 (SEQ ID NO:39) and hsFATP6 (SEQ ID NO:40).
Figure 37 shows results of assessment of fatty acid uptake by human FATP1 and human FATP4. The percent of CD2-positive cells exhibiting a BODIPY-fluorescence of more than 300 arbitrary units is plotted for the three different conditions tested.
Figure 38 is a graph showing uptake of tritiated oleate, with time, by 293 cells transfected with either (diamonds) a plasmid for expression of human FATP4 or (squares) a control plasmid.

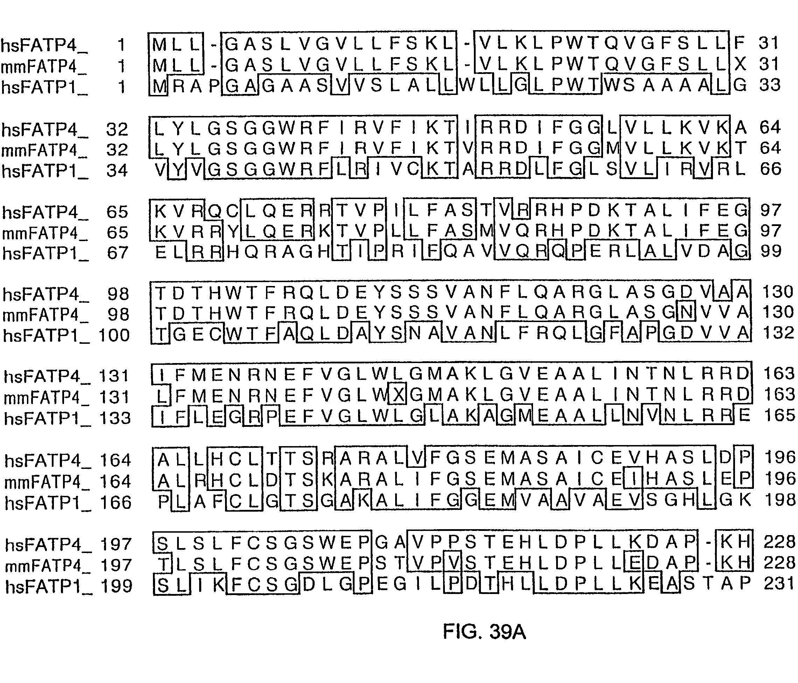

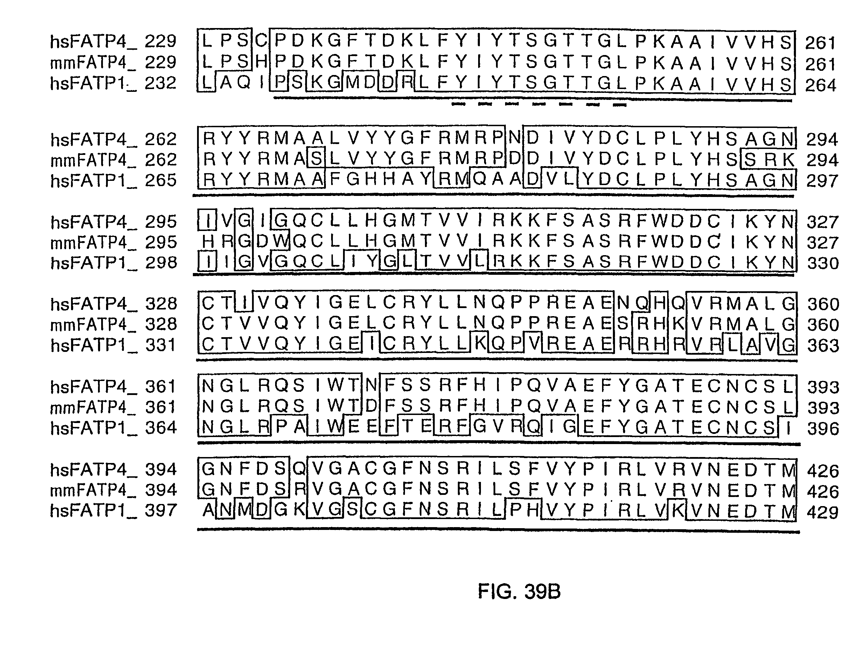

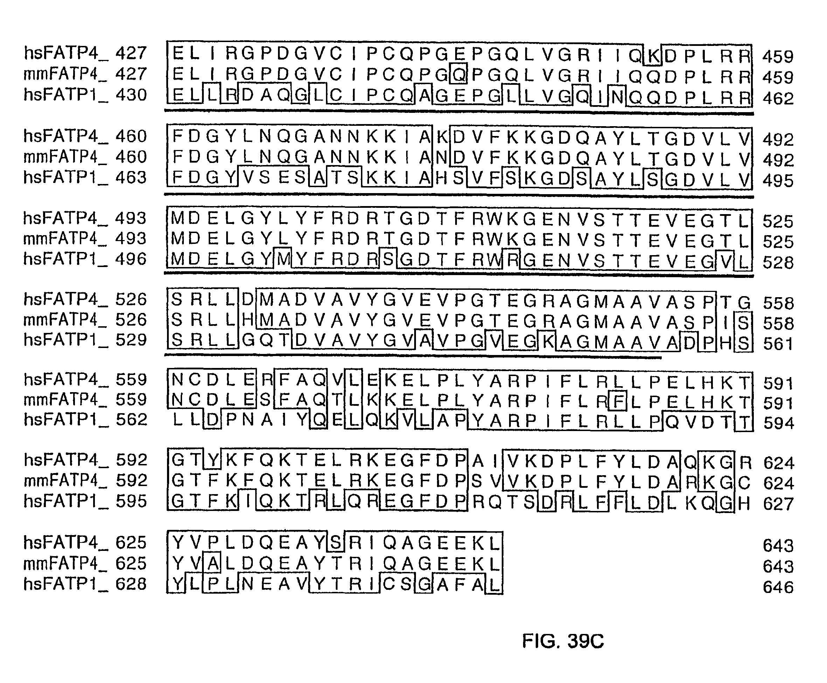

Figures 39A-39C show the amino acid sequences of human FATP4 (SEQ ID NO:41) and mouse FATP4 (SEQ ID NO:42) compared to human FATP1 (SEQ ID NO:43). Shown by underlining are the FATP consensus sequence (236-556 of hsFATP1) and the AMP-binding motif (246-254 of hsFATP1). The human FATPs were cloned by screening libraries with sequences from ESTs (expressed sequence tags). Mouse FATP4 was cloned by PCR using degenerate primers.
Figure 40 is a graph showing the uptake, with time, of tritiated oleate by mouse enterocytes in the presence of no oligonucleotide (squares), sense oligonucleotide (circles) or antisense oligonucleotide (diamonds).
Figure 41 is a bar graph showing uptake of tritiated oleate, by mouse enterocytes in the presence of various concentrations of antisense (solid bars), mismatch (stippled bars) or sense (lined bars) oligonucleotides.
Figure 42 is a bar graph showing uptake of tritiated oleate and uptake of ³⁵S-labeled methionine by mouse enterocytes to which were added no oligonucleotide, the antisense oligonucleotide, or the mismatch oligonucleotide.
Figures 43A-43B show the nucleotide sequence of the gene encoding mouse FATP4 (SEQ ID N0:44).
Figure 43C is the amino acid sequence of mouse FATP4 protein (SEQ ID NO:45).
Figures 44A, 44B, 44C, and 44D are the hsFATP1 DNA sequence (SEQ ID NO:46). Coding region: 175-2115 (1941 nt).
Figure 45 is the hsFATP1 protein sequence (SEQ ID NO:47).
Figures 46A and 46B are the hsFATP2 DNA sequence (SEQ ID NO:48). Coding region: 223-2085 (1863 nt).
Figure 47 is the hsFATP2 protein sequence (SEQ ID NO:49).
Figure 48 is the partial DNA sequence of hsFATP3 (SEQ ID NO:50). Coding region: 1-993.
Figure 49 is the partial protein sequence of hsFATP3 (SEQ ID NO:51).
Figures 50A, 50B, 50C, and 50D are the hsFATP4 DNA sequence (SEQ ID NO:52). Coding region: 208-2139 (1932 nt).
Figure 51 is the hsFATP4 protein sequence (SEQ ID NO:53).
Figure 52 is the hsFATP5 partial DNA sequence (SEQ ID NO:54). Coding region: 1-1062.
Figure 53 is the hsFATP5 partial protein sequence (SEQ ID NO:55).
Figures 54A, 54B, 54C, and 54D are the hsFATP6 DNA sequence (SEQ ID NO:56). Coding region: 643-2502 (1860 nt).
Figure 55 is the hsFATP6 protein sequence (SEQ ID NO:57).
Figures 56A, 56B, 56C, and 56D are the mFATP1 DNA sequence (rn*=Rattus norvegicus;* (SEQ ID NO:58). Coding region: 75-2015 (1941 nt).
Figure 57 is the rnFATP1 protein sequence (SEQ ID NO:59).
Figure 58A, 58B, 58C, and 58D are the rnFATP2 DNA sequence (SEQ ID NO:60). Coding region: 795-2657 (1863 nt).
Figure 59 is the rnFATP2 protein sequence (SEQ ID NO:61).
Figure 60A and 60B are the rnFATP4 partial DNA sequence (SEQ ID NO:62). Coding region: 1-1218.
Figure 61 is the rnFATP4 partial DNA sequence (SEQ ID NO:63).
Figures 62A, 62B, 62C, and 62D are the mmFATP1 DNA sequence (SEQ ID NO:64). Coding region: 1-1944.
Figure 63 is the mmFATP1 protein sequence (SEQ ID NO:65).
Figures 64A and 64B are the mmFATP2 DNA sequence (SEQ ID NO:66). Coding region: 121-1992 (1872 nt).
Figure 65 is the mmFATP2 protein sequence (SEQ ID NO:67).
Figures 66A and 66B are the mmFATP3 partial DNA sequence (SEQ ID NO:68). Coding region: 1-1830.
Figure 67 is the mmFATP3 partial protein sequence (SEQ ID NO:69).
Figures 68A, 68B, 68C, and 68D are the mmFATP4 DNA sequence (SEQ ID NO:70). Coding region: 1-1932.
Figures 69 is the mmFATP4 protein sequence (SEQ ID NO:71).
Figures 70A and 70B are the mmFATP5 DNA sequence (SEQ ID NO:72). Coding region: 60-2129.
Figure 71 is the mmFATP5 protein sequence (SEQ ID NO:73).
Figures 72A and 72B are the dmFATP partial DNA sequence (dm=*Drosophila melanogaster;* SEQ ID NO:74). Coding region: 1-1773.
Figures 73 is the dmFATP partial protein sequence (SEQ ID NO:75).
Figure 74 is the drFATP partial DNA sequence (dr=*Danio rerio,* zebrafish; SEQ ID NO:76) Coding region: 1-173.
Figure 75 is the drFATP partial protein sequence (SEQ ID NO:77).
Figure 76A and 76B are the ceFATPa DNA sequence (SEQ ID NO:78). Coding region: 1-1953.
Figure 77 is the ceFATPa protein sequence (SEQ ID NO:79).
Figures 78A and 78B are the ceFATPb DNA sequence (SEQ ID NO:80). Coding region: 1-1968.
Figure 79 is the ceFATPb protein sequence (SEQ ID NO:81).
Figures 80A and 80B are the chFATP DNA sequence (SEQ ID NO:82; *ch*=*Cochliobolu heterostrophus).* Coding region: 1-1932.
Figure 81 is the chFATP protein sequence (SEQ ID NO:83).
Figure 82 is the anFATP partial protein sequence (*an*=*Aspergillus nidulans;* SEQ ID NO:84). Coding region: 1-597.
Figure 83 is the anFATP partial protein sequence (SEQ ID NO:85).
Figure 84 is the mgFATP partial DNA sequence (mg= *Magnaporthe grisea,* rice blast; SEQ ID NO:86). Coding region: 1-522.
Figure 85 is the mgFATP partial protein sequence (SEQ ID NO:87).
Figures 86A and 86B are the scFATP DNA sequence (SEQ ID NO:88). Coding region: 1-1872.
Figure 87 is the scFATP protein sequence (SEQ ID NO:89).
Figures 88A and 88B are the mtFATP DNA sequence (SEQ ID NO:90).
Figure 89 is the mtFATP protein sequence (SEQ ID NO:91). Coding region: 1-1794.
Figure 90 is a concensus sequence of the FATP signature sequence (SEQ ID NO: 100), based on 23 independent sequences aligned in ClustaIX. The height of the bar at each amino acid residue position indicates the degree of conservation at that position. Gaps have been inserted to maintain the strength of the alignment.
Figure 91 is a hydrophilicity plot for hsFATP2, made using the Kyte-Doolittle method, averaging hydrophilicity values for 18 amino acid residues at a time.
Figure 92 is a hydrophilicity plot for the hsFATP3 partial protein, made using the Kyte-Doolittle method, averaging hydrophilicity values for 18 amino acid residues at a time.
Figure 93 is a hydrophilicity plot for the hsFATP5 partial protein, made using the Kyte-Doolittle method, averaging hydrophilicity values for 18 amino acid residues at a time.
Figures 94A-94J are a representation of the DNA sequence (SEQ ID NO:101) of the hsFATP5 gene, and the amino acid sequence (SEQ ID NO: 102) of the hsFATP5 protein.

### DETAILED DESCRIPTION OF THE INVENTION

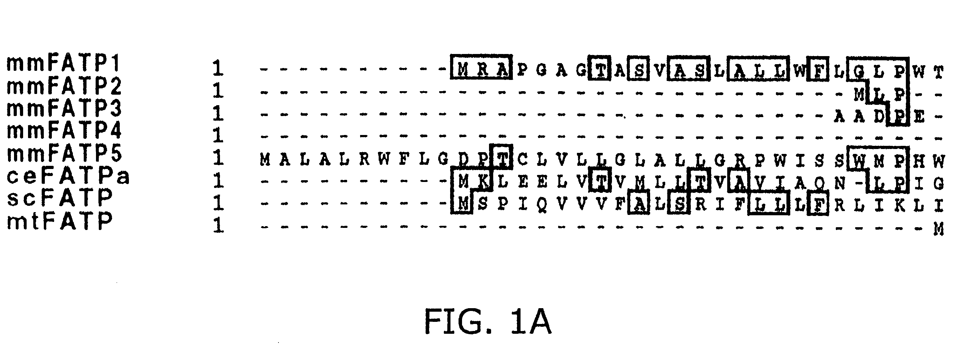

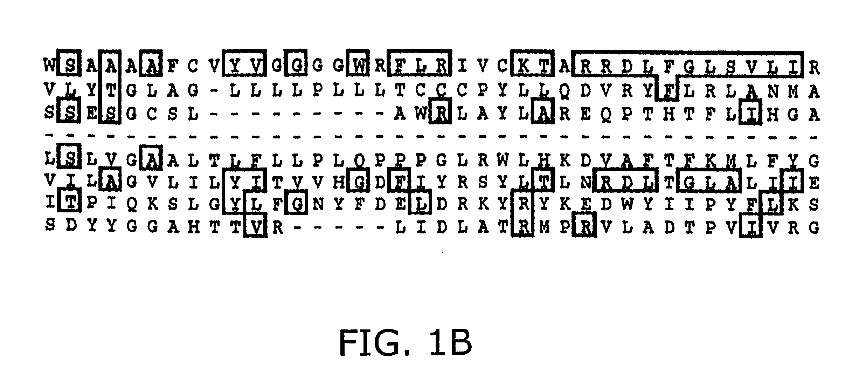

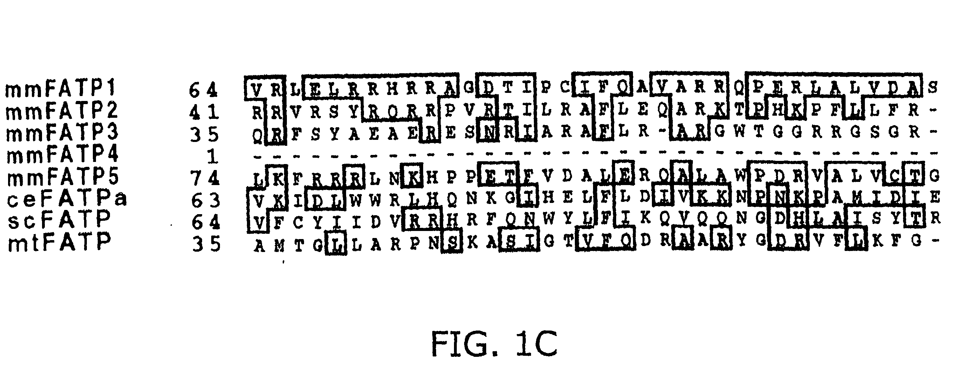

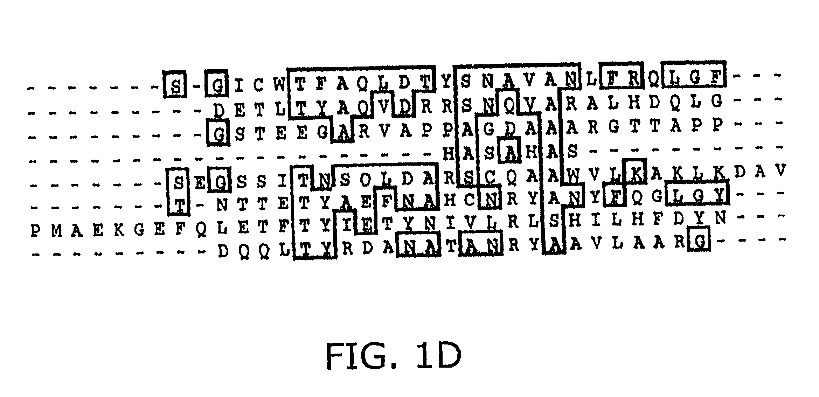

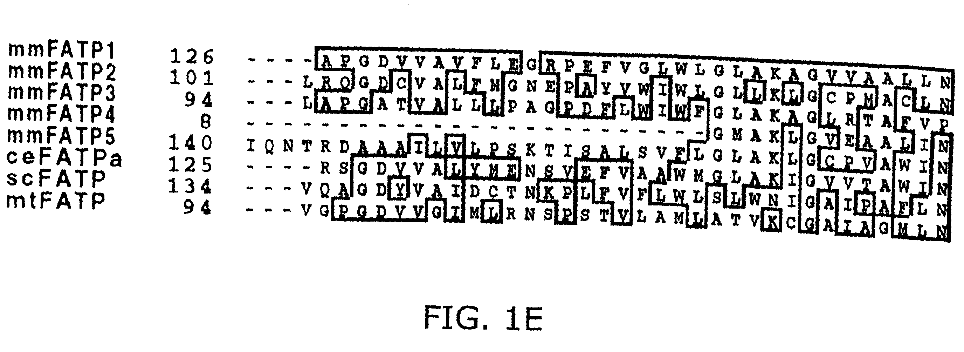

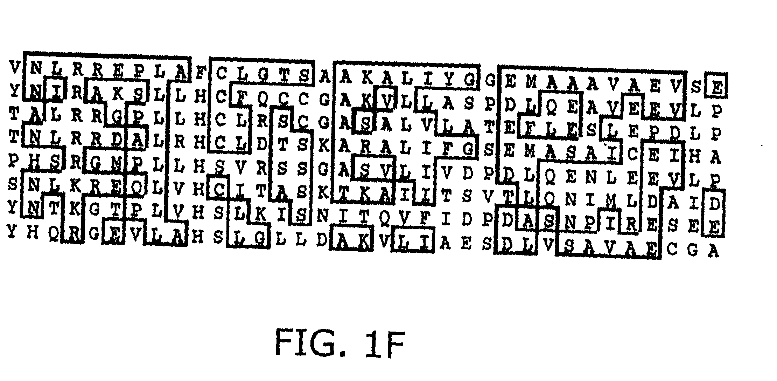

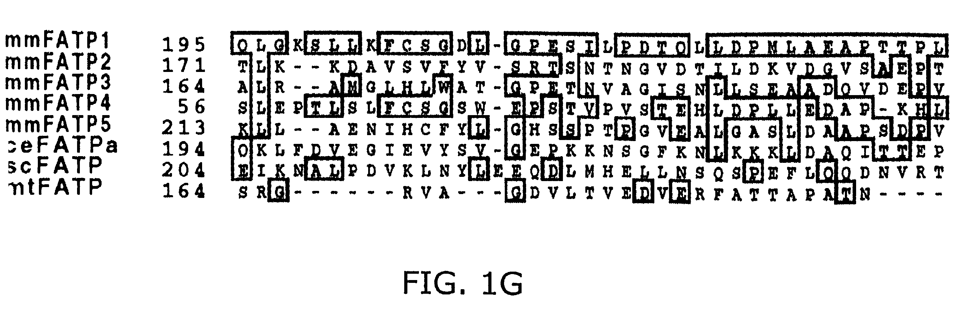

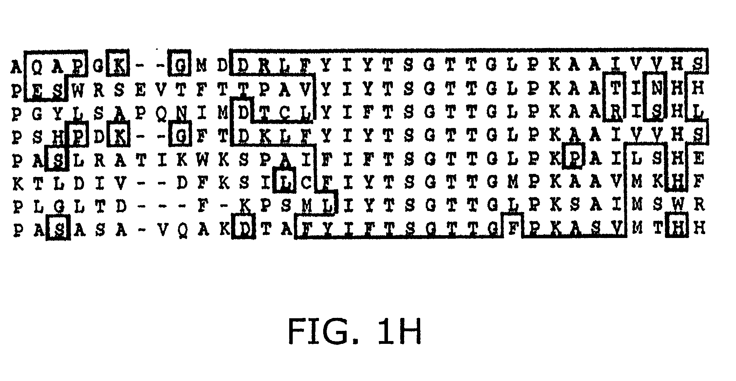

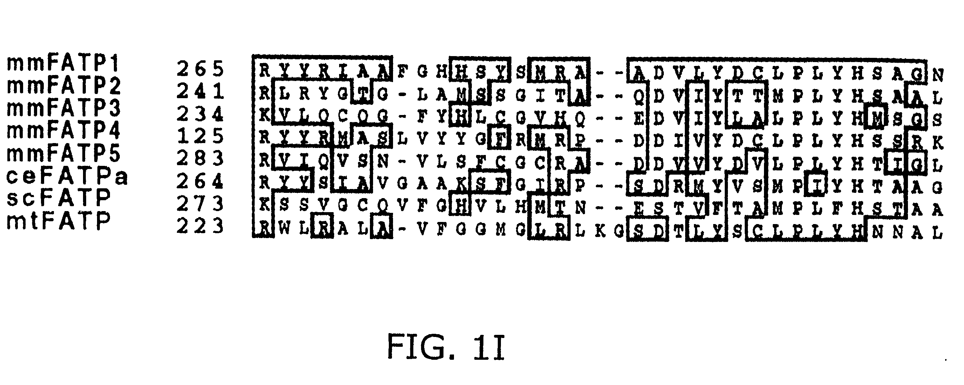

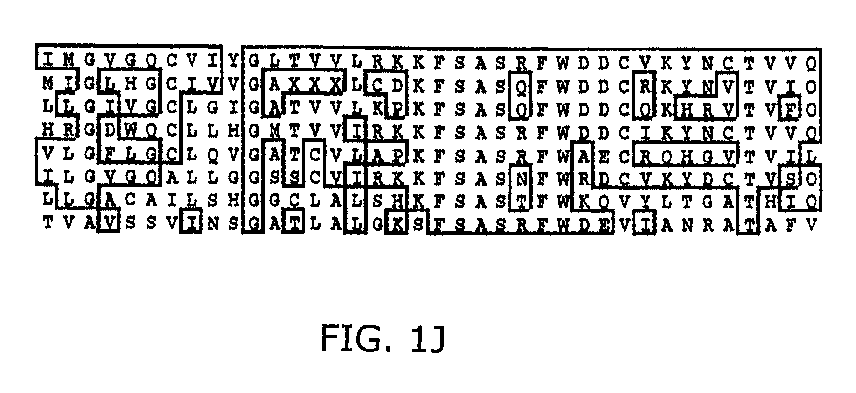

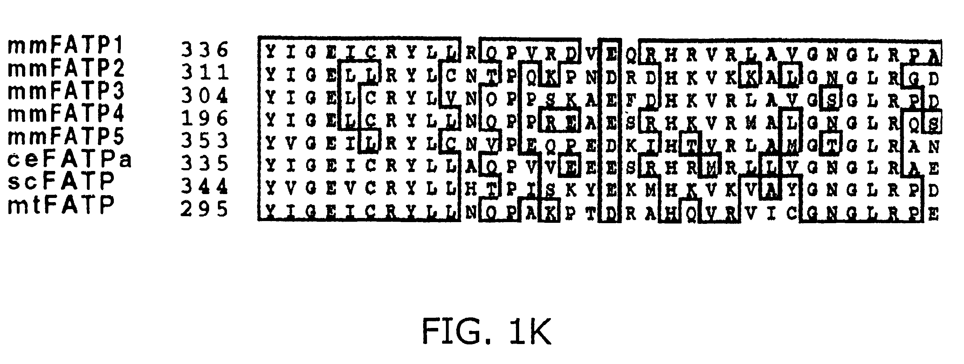

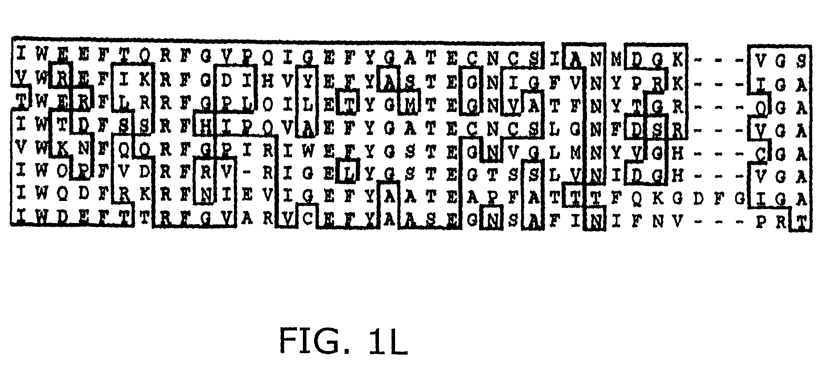

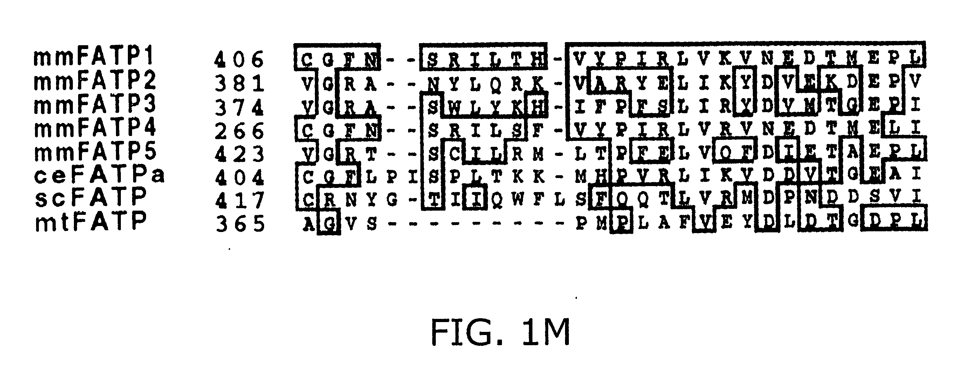

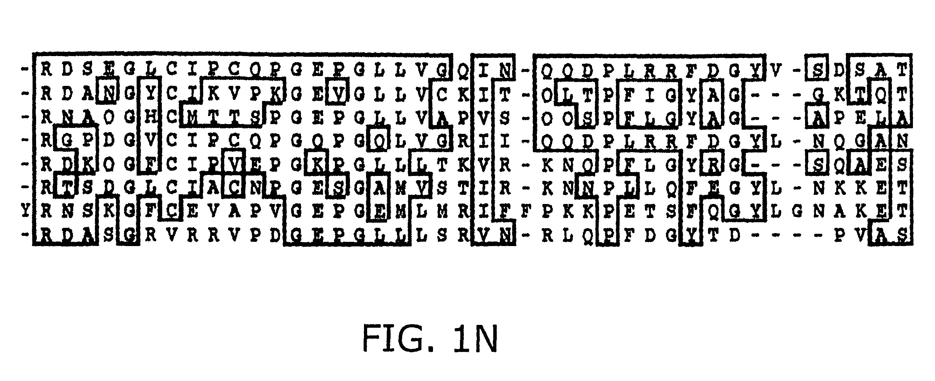

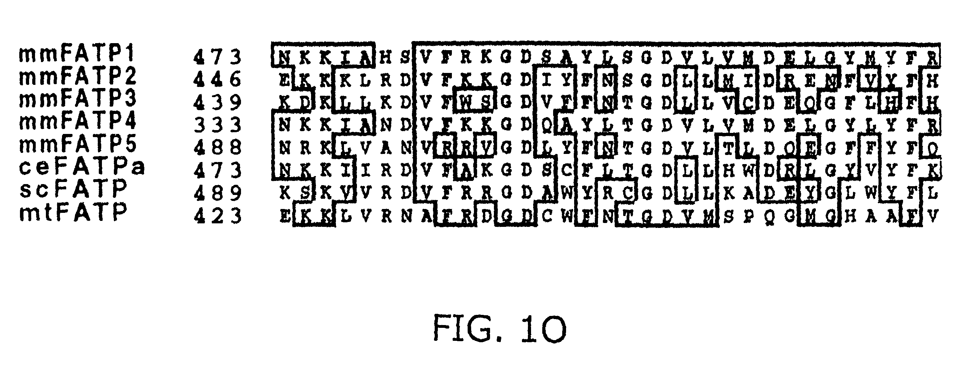

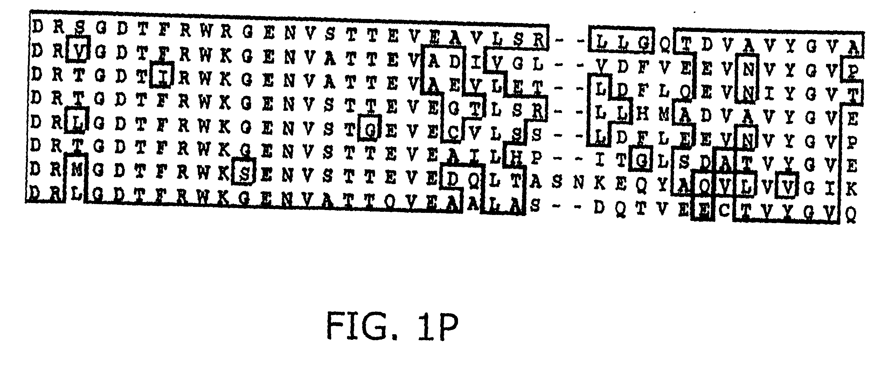

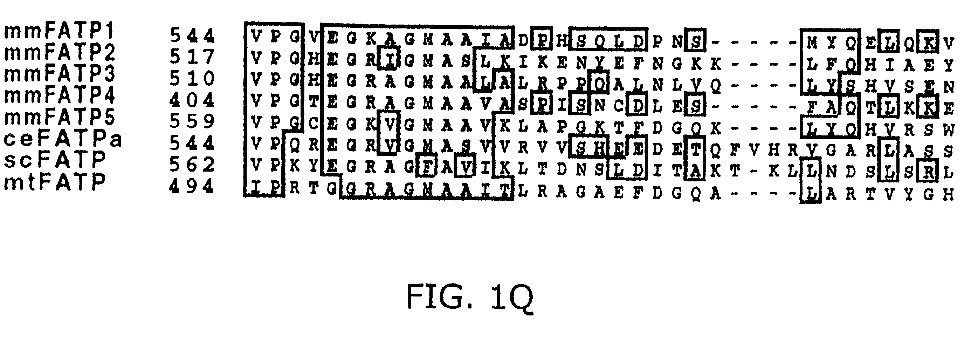

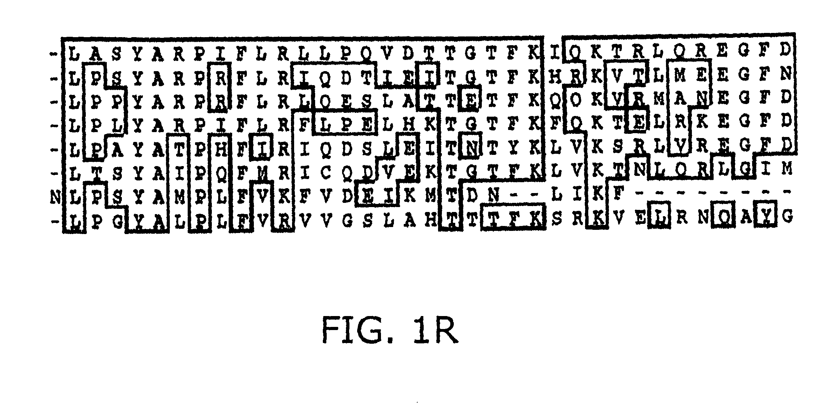

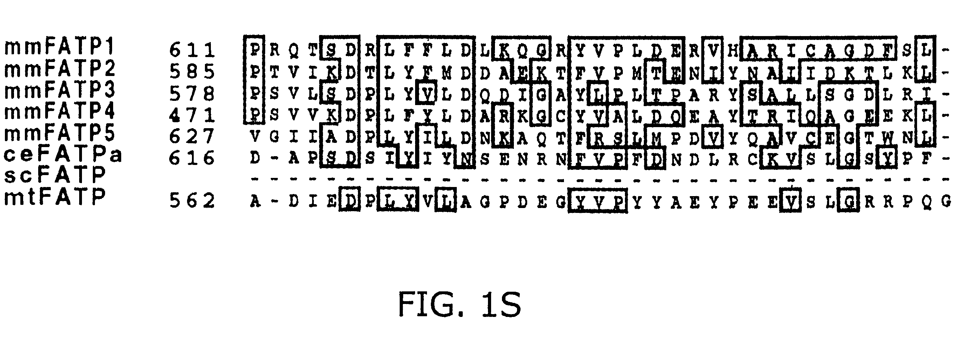

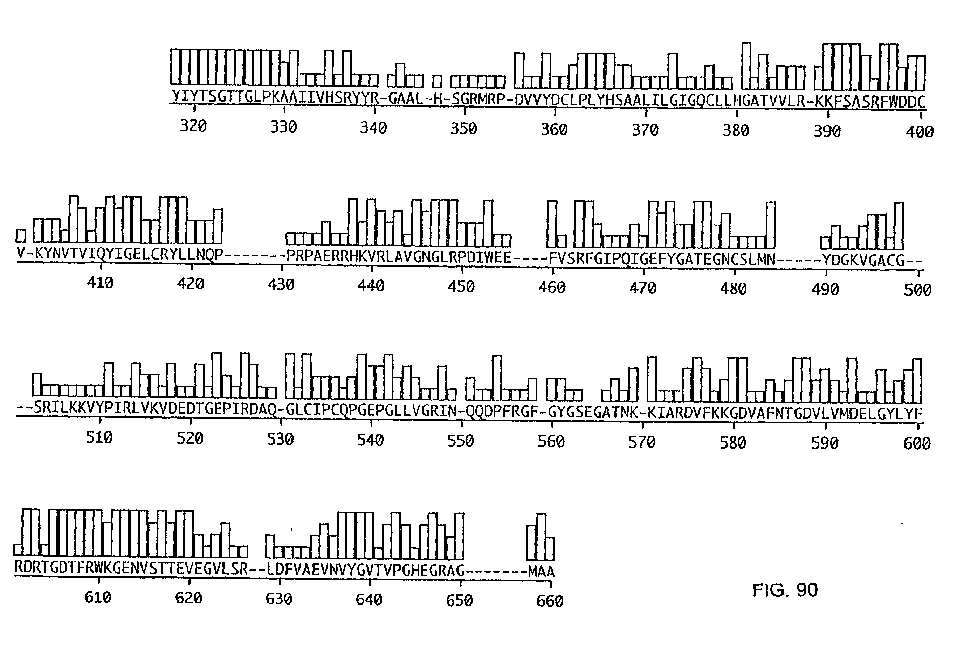

As described herein, FATPs are a large evolutionarily conserved family of proteins that mediate the transport of LCFAs into cells. The family includes proteins which are conserved from mycobacteria to humans and exhibit very different expression patterns in tissues. Specific embodiments described include FATPs from mice, humans, nematodes, fungi and mycobacteria which have been shown to be functional LCFA transporters. The term "fatty acid transport proteins" ("FATPs") as used herein, refers to the proteins described herein as FATP1, FATP2, FATP3, FATP4, FATP5 and FATP6, which have been described in one or more species of mammals, as well as mtFATP, ceFATP, scFATP, anFATP, mgFATP, and chFATP, and other proteins sharing at least about 50% amino acid sequence similarity, preferably at least about 60% sequence similarity, more preferably at least about 70% sequence similarity, and still more preferably, at least about 80% sequence similarity, and most preferably, at least about 90% sequence similarity in the approximately 360 amino acid signature sequence. The approximaely 360 amino acid FATP signature sequence is shown in Figure 1. The concensus sequence of the signature sequence is shown in Figure 90. The nomenclature used herein to refer to FATPs includes a species-specific prefix (e.g., mm, *Mus musculus;* hs or h, *Homo sapiens* or human; mt *M. tuberculosis;* dm. *D. melanogaster;* ce, *C. elegans;* sc, *Saccharomyces cerevisiae*) and a number such that mammalian homologues in different species share the same number. For example, six human and five mouse *FATP* genes which are expressed in a variety of tissues are described herein and are referred to, respectively, as hsFATP1-hsFATP6 and mmFATP1-mmFATP5; for example, hsFATP4 and mmFATP4 are the human and mouse orthologs.

Expression patterns of human and mouse FATPs have been assessed and are described below. Briefly, results of these assessments show that FATP5 is a liver-specific gene. FATP2 is highly expressed in liver and kidney. Both of these proteins, as well as FATP4 and FATPs from nematodes and mycobacteria, have been shown to be functional LCFA transporters. Results have also shown that FATP4 mRNA is present at high levels in epithelial cells of two regions of the small intestine (the jejunum and ileum) and at lower, but significant, levels in a third region (the duodenum). They further showed that FATP2 mRNA is present in epithelial cells of the duodenum at a level similar to that of FATP4 mRNA levels, but is present at lower levels in the jejunum and ileum. FATP4 mRNA was absent from other cell types of the small intestine and no FATP4 mRNA could be detected in any cells of the colon. No signals above background could be detected for FATP1, FATP3 and FATP5 in any of the intestinal tissues. Thus, FATP4 is the major FATP in the mouse small intestine, which supports a major role for FATP4 (along with FATP2 to a lesser extent) in absorption of free fatty acids. hsFATP4 was clearly expressed in the jejunum and ileum; expression was absent in the stomach. This, too, is consistent with a major role for FATP4 in absorption of fatty acids in the human gut. Analysis of FATP expression in human tissues, also described in detail below, showed that hsFATP6, which has no mouse ortholog as yet, is expressed at high levels in the heart and at low levels in the placenta, but is undetectable in the other tissues assessed (Example 9). This is consistent with a major role for FATP6 in absorption of fatty acids in the heart.

Long chain fatty acids (LCFAs) are an important energy source for pro- and eukaryotes and are involved in diverse cellular processes, such as membrane synthesis, intracellular signaling, protein modification, and transcriptional regulation. In developed Western countries, human dietary lipids are mainly di- and triglycerides and account for approximately 40% of caloric intake (Weisburger, J. H. (1997) *J*. *Am. Diet. Assoc. 97*:S16-S23). These lipids are broken down into fatty acids and glycerol by pancreatic lipases in the small intestine (Chapus, C., Rovery, M., Sarda, L & Verger, R. *(1988) Biochimie 70*:1223-34); LCFAs are then transported into brush border cells, where the majority is re-esterified and secreted into the lymphatic system as chylomicrons (Green, P.H. & Riley, J.W. (1981) *Aust. N.Z.J. Med. 11*:84-90). Fatty acids are liberated from lipoproteins by the enzyme lipoprotein lipase, which is bound to the luminal side of endothelial cells (Scow, R.O. & Blachette-Mackie, E.J. (1992) *Mol. Cell. Biochem 116*:181-191). "Free" fatty acids in the circulation are bound to serum albumin (Spector, A.A. (1984) *Clin. Physiol. Biochem 2*:123-134) and are rapidly incorporated by adipocytes, hepatocytes, and cardiac muscle cells. The latter derive 60-90% of their energy through the oxidation of LCFAs (Neely, J.F. Rovetto, M.J. & Oram, J.F. (1972) *Prog. Cardiovasc. Dis: 15*:289-329). Although saturable and specific uptake of LCFAs has been demonstrated for intestinal cells, hepatocytes, cardiac myocytes, and adipocytes, the molecular mechanisms of LCFA transport across the plasma membrane have remained controversial (Hui, T.Y. & Bernlohr, D.A. (1997) *Front. Biosci. 15*:d222-31-d231; Schaffer, J.E. & Lodish, H.F, (1995) *Trends Cardiovasc. Med. 5*:218-224)*.* Described herein is a large family of highly homologous mammalian LCFA transporters which show wide expression, including in all tissues relevant to fatty acid metabolism. Further described are novel members of this family in other species, including mycobacterial and nematode FATPs which, like their mammalian counterparts, are functional fatty acid transporters.

The discovery of a diverse but highly homologous family of FATPs is reminiscent of the glucose transporter family. In a manner similar to the FATPs, the glucose transporters have very divergent patterns of tissue expression (McGowan, K.M., Long, S.D. & Pekala, P.H. (1995) *Pharmacol. Ther. 66*:465-505). The FATPs, like glucose transporters, may also differ in their substrate specificities, uptake kinetics, and hormonal regulation (Thorens, B. (1996) *Am. J. Physiol. 270*:G541-G553). Indeed, the levels of fatty acids in the blood, like those of glucose, can be regulated by insulin and are dysregulated in diseases such as noninsulin-dependent diabetes and obesity (Boden, G. (1997) *Diabetes 46:*3-10). The underlying mechanisms for the regulation of free fatty acid concentrations in the blood are not understood, but could be explained by hormonal modulation of FATPs.

Insulin-resistance is thought to be the major defect in non insulin-dependent diabetes mellitus (NIDDM) and is one of the earliest manifestations of NIDDM (McGarry (1992) *Science* 258:766-770). Free fatty acids (FFAs) may provide an explanation for why obesity is a risk factor for NIDDM. Plasma levels of FFAs are elevated in diabetic patients (Reaven *et al.* (1988) *Diabetes* 37:1020). Elevated plasma free fatty acids (FFAs) have been demonstrated to induce insulin-resistance in whole animals and humans (Boden (1998) *Front. Biosci.* 3:D169-D175). This insulin-resistance is likely mediated by effects of FFAs on a variety of issues. FFAs added to adipocytes *in vitro* induce insulin resistance in this cell type as evidenced by inhibition of insulin-induced glucose transport (Van Epps-Fung *et al.* (1997) *Endocrinology* 138:4338-4345). Rats fed a high fat diet developed skeletal muscle insulin resistance as evidenced by a decrease in insulin-induced glucose uptake by skeletal muscle (Han *et al.,* (1997) *Diabetes* 46:1761-1767). In addition, elevated plasma FFAs increase insulin-suppressed endogenous glucose production in the liver (Boden (1998) *Front. Biosci.* 3:D169-D175), thus increasing hepatic glucose output. It has been postulated that the adverse effects of plasma free fatty acids are due to the FFAs being taken up into the cell, leading to an increase in intracellular long chain fatty acyl CoA; intracellular long chain acyl CoAs are thought to mediate the effects of FFAs inside the cell. Thus, fatty acid induced insulin-resistance may be prevented by blocking uptake of FFAs into select tissues, in particular liver (by blocking FATP2 and/or FATP5), adipocyte (by blocking FATP1), and skeletal muscle (by blocking FATP1). Blocking intestinal fat absorption (by blocking FATP4) is also expected to reduce plasma FFA levels and thus improve insulin resistance.

During the pathogenesis of NIDDM insulin-resistance can initially be counteracted by increasing insulin output by the pancreatic beta cell. Ultimately, this compensation fails, beta cell function decreases and overt diabetes results (McGarry (1992) *Science* 258: 766-770). Manipulating beta cell function is a second point where fatty acid transporter blockers may be beneficial for diabetes. While no FATP homolog has been identified so far that is expressed in the beta cell of the pancreas, the data described below suggest the existence of such a transporter and the sequence information included herein provides the means to identify such a transporter by degenerate PCR, using primers to regions conserved in all FATP family members or by low stringency hybridization. It has been demonstrated that exposure of pancreatic beta-cells to FFAs increases the basal rate of insulin secretion; this in turn leads to a decrease in the intracellular stores of insulin, resulting in decreased capacity for insulin secretion after chronic exposure (Bollheimer *et al.,* (1998) *J*. *Clin. Invest.* 101:1094-1101). The effects of FFAs are again likely to be mediated by intracellular long chain fatty acyl CoA molecules (Liu *et al.,* (1998) *J. Clin. Invest.* 101:1870-1875). FFAs have also been demonstrated to increase beta cell apoptosis (Shimabukuro *et al.,* (1998) *Proc. Nat. Acad. Sci. USA* 95:2498-2502), possibly contributing to the decrease in beta cell numbers in late stage NIDDM.

Another finding with potentially broad implications is the identification of a FATP homologue in *M. tuberculosis.* Tuberculosis causes more deaths worldwide than any other infectious agent and drug-resistant tuberculosis is re-emerging as a problem in industrialized nations (Bloom, B.R. & Small, P.M. (1998) *N. Engl. J. Med. 338*:677-*678). Mycobacterium tuberculosis* has about 250 enzymes involved in fatty acid metabolism, compared with only about 50 in *E. coli.* It has been suggested that, living as a pathogen, the mycobacteria are largely lipolytic, rather than lipogenic, relying on the lipds within mammalian cells and the tubercle (Cole, S.T. *et al., Nature 393*:537-544 (1998)). The *de novo* synthesis of fatty acids in *Mycobacterium leprae* is insufficient to maintain growth (Wheeler, P.R., Bulmer, K & Ratledge, C. (1990) *J. Gene. Microbiol. 136*:211-217)*.* Thus, it is reasonable to expect that inhibitors of mtFATP will serve as therapeutics for tuberculosis. FATPs expressed in mycobacteria can be targeted to reduce or prevent replication of mycobacteria (e.g., to reduce or prevent replication of *M. tuberculosis)* and, thus, reduce or prevent their adverse effects. For example, a FATP or FATPs expressed by *M. tuberculosis* can be targeted and inhibited, thus reducing or preventing growth of this pathogen (and tuberculosis in humans and other mammals). An inhibitor of an *M. tuberculosis* FATP can be identified, using methods described herein (e.g., expressing the FATP in an appropriate host cell, such as *E*. *coli* or COS cells; contacting the cells with an agent or drug to be assessed for its ability to inhibit the FATP and, as a result, mycobacterial growth, and assessing its effects on growth). A drug or agent identified in this manner can be further tested for its ability to inhibit a *M. tuberculosis* FATP and *M. tuberculosis* infection in an appropriate animal model or in humans. A method of inhibiting mycobacterial growth, particularly growth of *M. tuberculosis,* and compounds useful as drugs for doing so are also the subject of this invention.

An isolated polynucleotide encoding mtFATP, like other polynucleotides encoding FATPs of the FATP family, can be incorporated into vectors, nucleic acids of viruses, and other nucleic acid constructs that can be used in various types of host cells to produce mtFATP. This mtFATP can be used, as it appears on the surface of cells, or in various artificial membrane systems, to assess fatty acid transport function, to identify ligands and molecules that are modulators of fatty acid transport activity. Molecules found to be inhibitors of mtFATP function can be incorporated into pharmaceutical compositions to administer to a human for the treatment of tuberculosis.

Particularly disclosed herein are polynucleotides encoding a FATP of *Cochliobolus (Helminthosporium) heterostrophus* or portions or variants thereof, the isolated or recombinantly produced FATP, methods for assessing whether an agent binds to the chFATP, and further methods for assessing the effect of an agent being tested for its ability to modulate fatty acid transport activity. *Cochliobolus heterostrophus* is an ascomycete that is the cause of southern corn leaf blight, an economically important threat to the corn crop in the United States. The related species *C*. *sativus* causes crown rot and common root rot in wheat and barley. One or more FATPs of *C. heterostrophus* can be targeted for the identification of an inhibitor of chFATP function, which can be then be used as an agent effective against infection of plants by C. *heterostrophus* and related organisms. Methods described herein that were applied in studying the expression of a FATP gene and the function of the FATP in its natural site of expression or in a host cell, can be used in the study of the chFATP gene and protein.

*Magnaporthe grisea* (rice blast) is an economically important fungal pathogen of rice. Also disclosed herein are nucleic acid molecules encoding a FATP of *Magnaporthe grisea,* portions thereof, or variants thereof, isolated mgFATP, nucleic acid constructs, and engineered cells expressing mgFATP. Also disclosed are assays to identify an agent which binds to mgFATP and assays to identify an agent which modulates the function of mgFATP in cells in which mgFATP is expressed or in artificial membrane systems. Agents identified as inhibiting mgFATP activity can be developed into anti-fungal agents to be used to treat rice infected with rice blast.

*Caenorhabditis elegans* is a nematode related to plant pathogens and human parasites. An isolated polynucleotide which encodes ceFATP, like other polynucleotides encoding FATPs of the FATP family described herein, can be incorporated into nucleic acid vectors and other constructs that can be used in various types of cells to produce ceFATP. ceFATP as it occurs in cells or as it can be isolated or incorporated into various artificial or reconstructed membrane systems, can be used to assess fatty acid transport, and to identify ligands and agents that modulate fatty acid transport activity. Agents found by such assays to be inhibitors of ceFATP activity can be incorporated into compositions for the treatment of diseases caused by genetically related organisms with a FATP of similar sensitivity to the agents.

*Aspergillus nidulans* is one of a family of fungal species that can infect humans. Also disclosed in the family of polynucleotides encoding FATPs are polynucleotides encoding a FATP of *Aspergillus nidulans,* and vectors and host cells that can be constructed to comprise such polynucleotides. Also disclosed are a polypeptide encoded by such polynucleotides, portions thereof having one or more functions characteristic of a FATP, and various methods. The methods include those for identifying agents that bind to anFATP and those for assessing the effect of an agent being tested for its ability to modulate fatty acid transport activity. Those agents found to inhibit fatty acid transport function can be used in compositions as anti-fungal pharmaceuticals, or can be modified for greater effectiveness as a pharmaceutical.

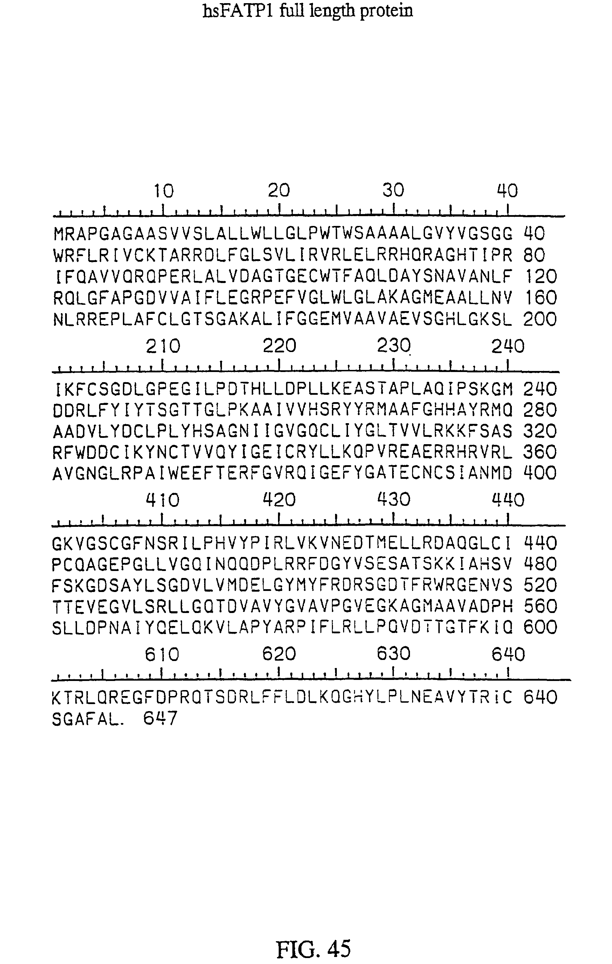

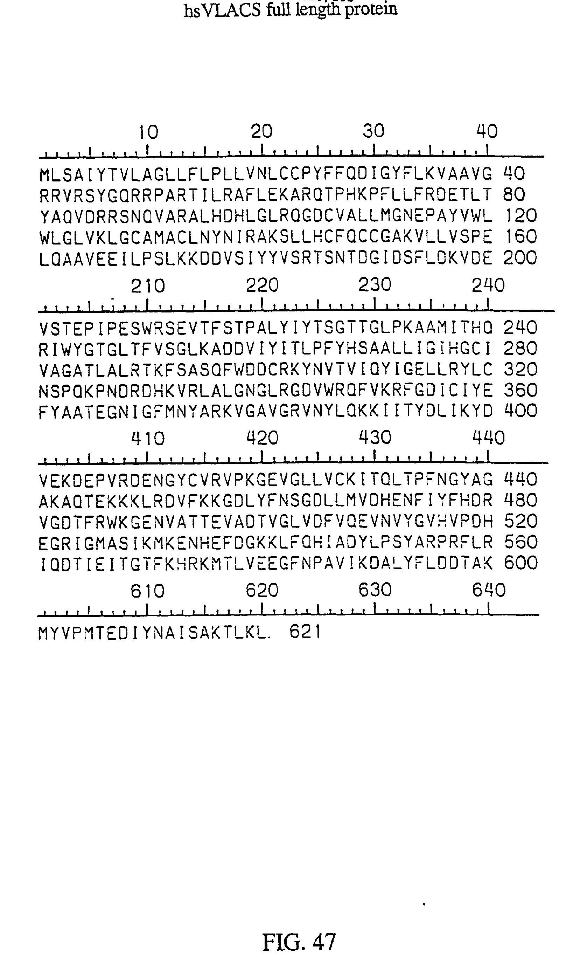

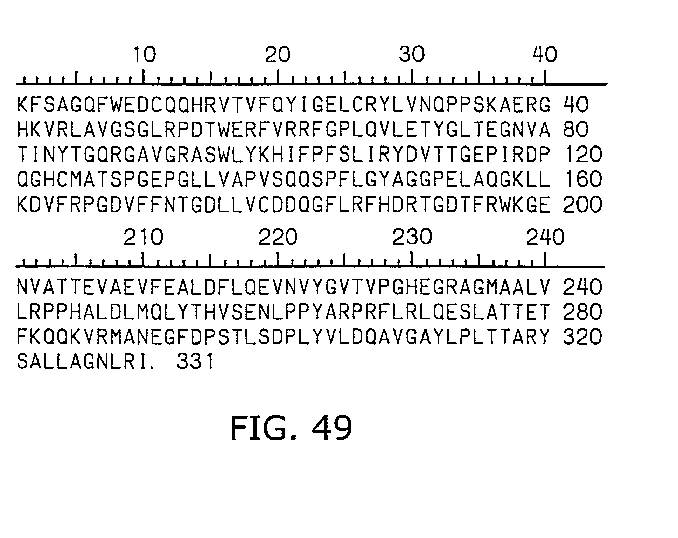

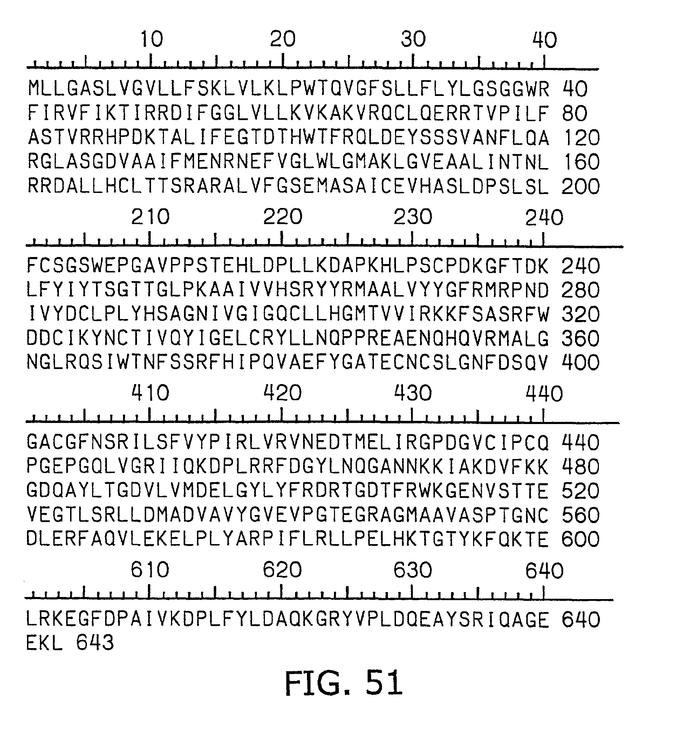

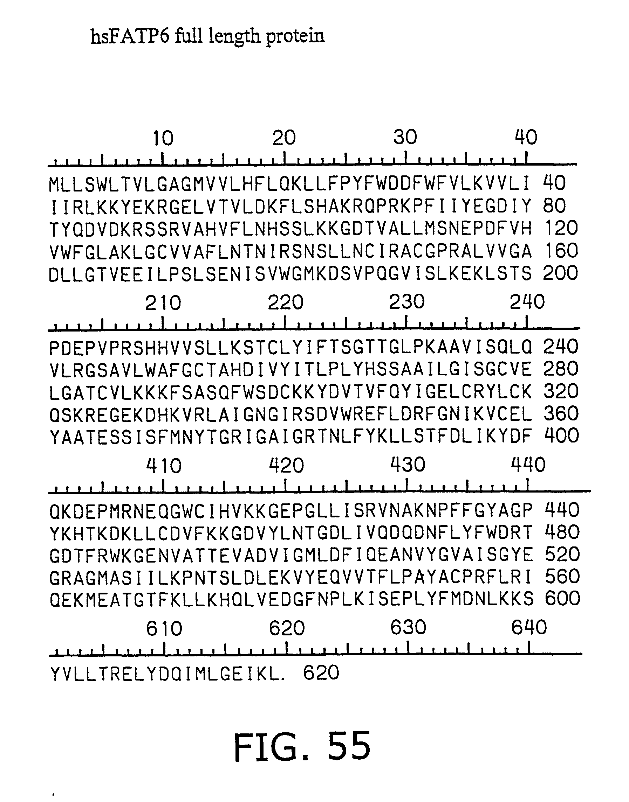

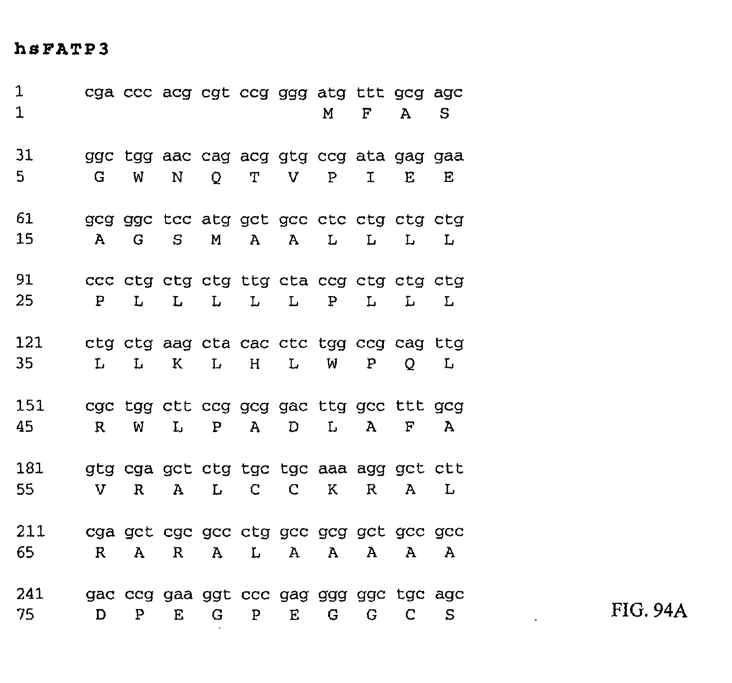

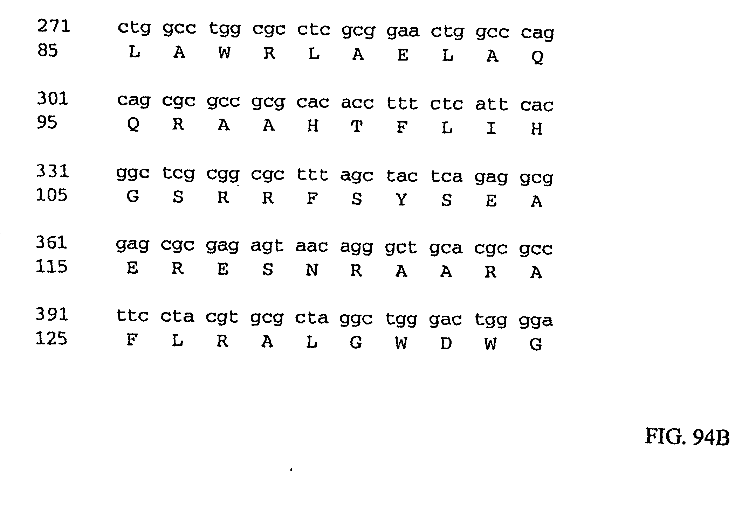

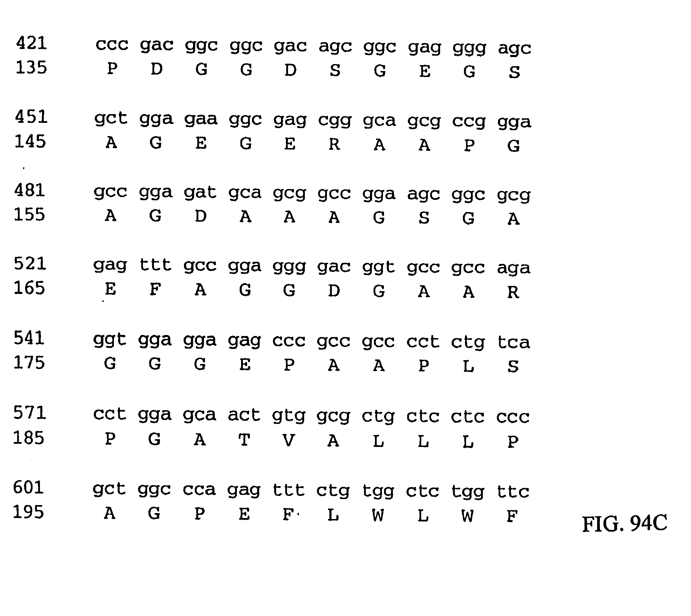

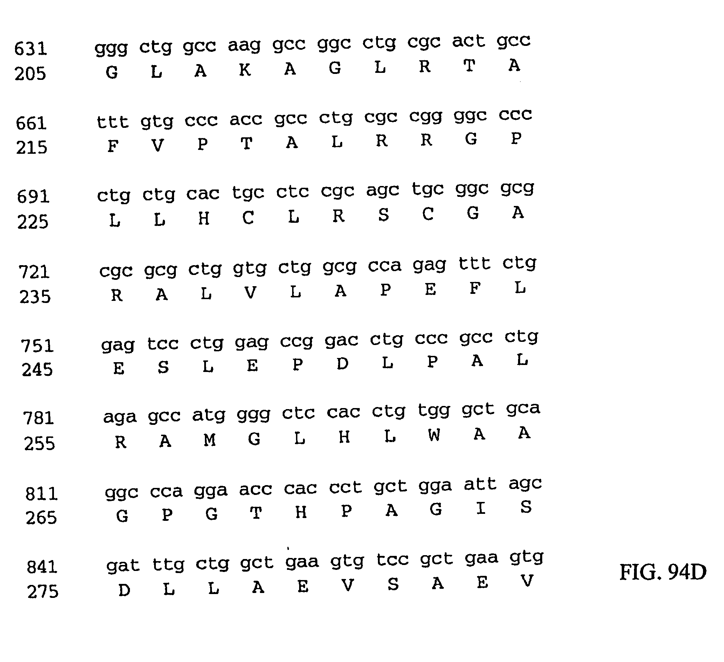

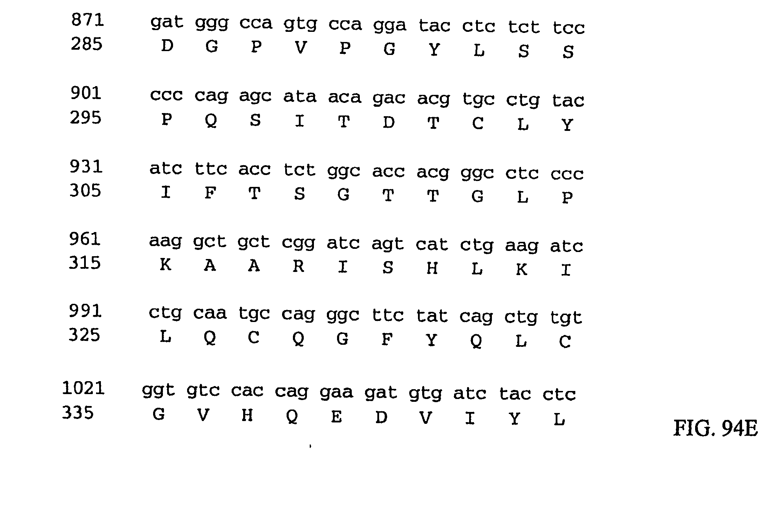

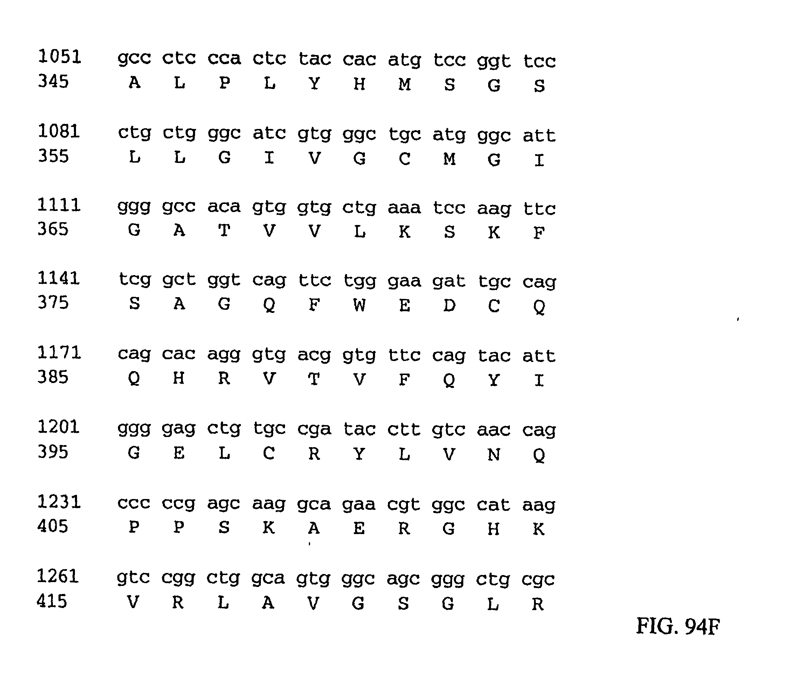

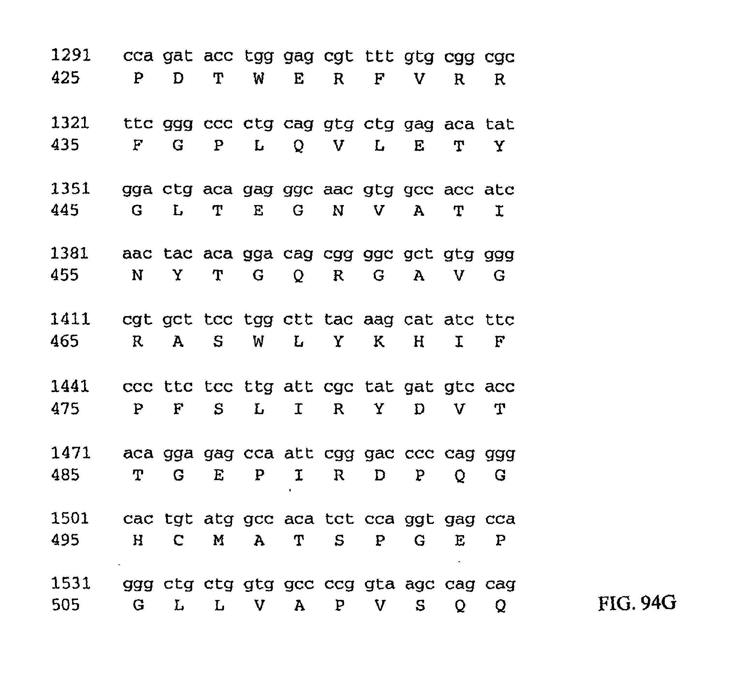

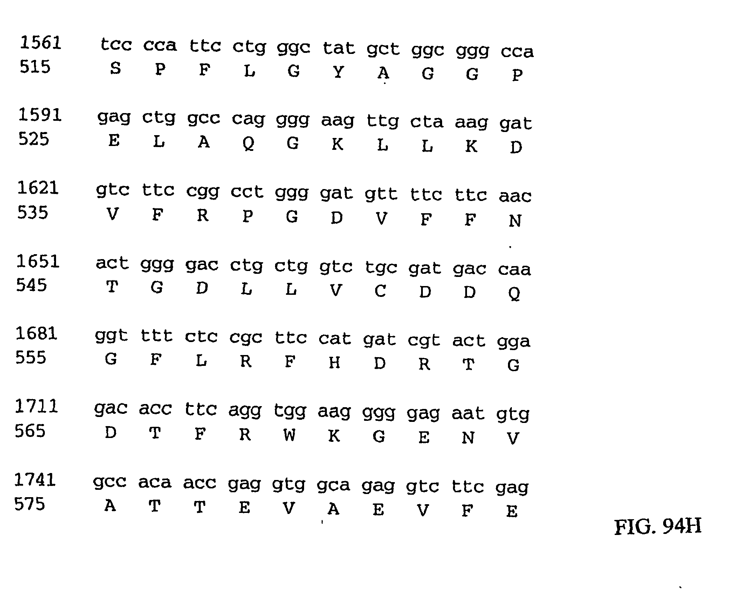

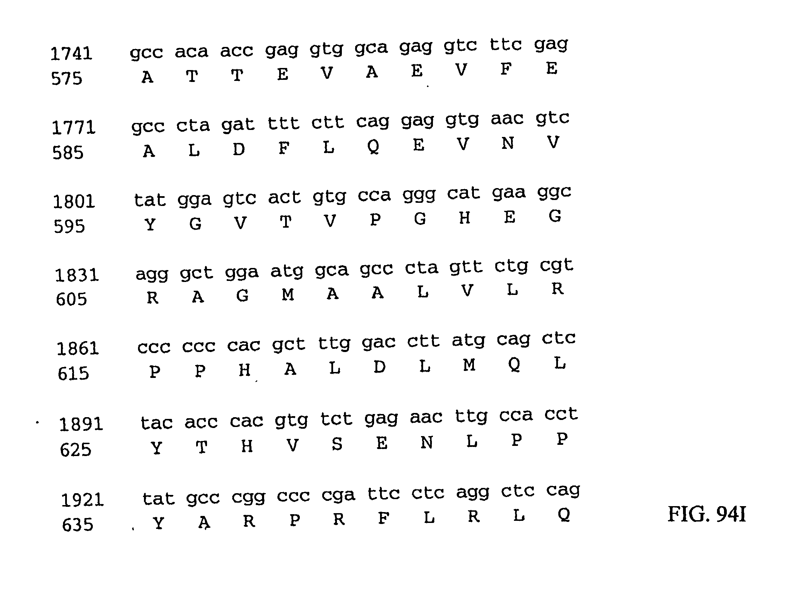

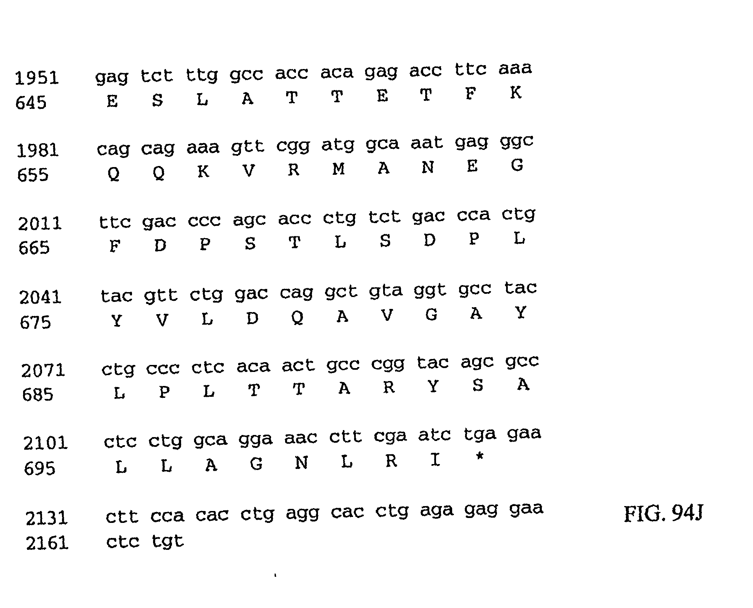

There are now disclosed isolated nucleic acids that encode a FATP as described herein, such as those FATPs having an amino acid sequence in Figure 45 (SEQ ID NO:47), Figure 47 (SEQ ID NO:49), Figure 49 (SEQ ID NO:51), Figure 51 (SEQ ID NO:53), Figures 94A and 94B (SEQ ID NO:102), and Figure 55 (SEQ ID NO:57) and nucleic acids closely related thereto as described herein.

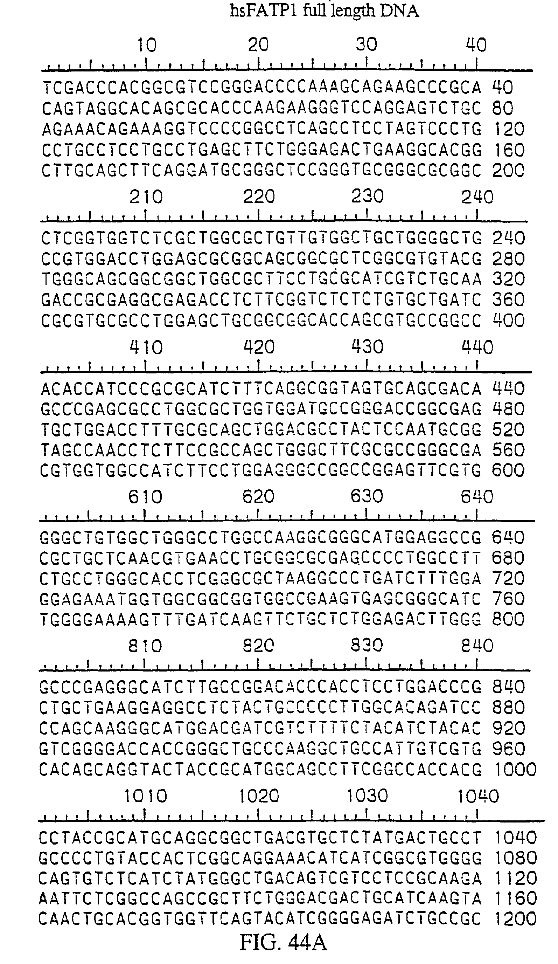

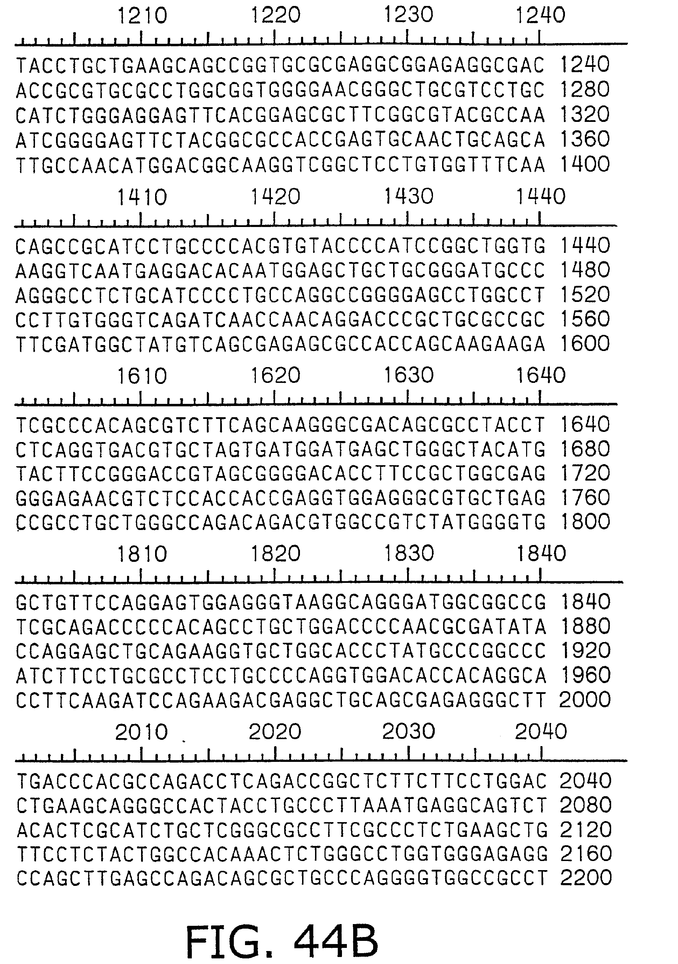

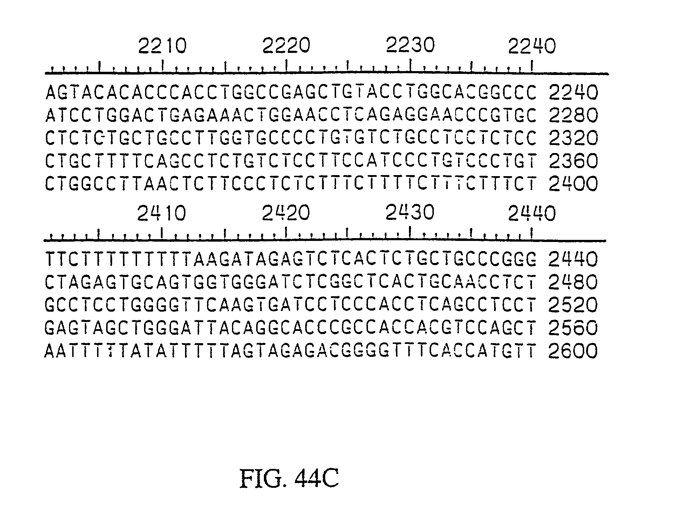

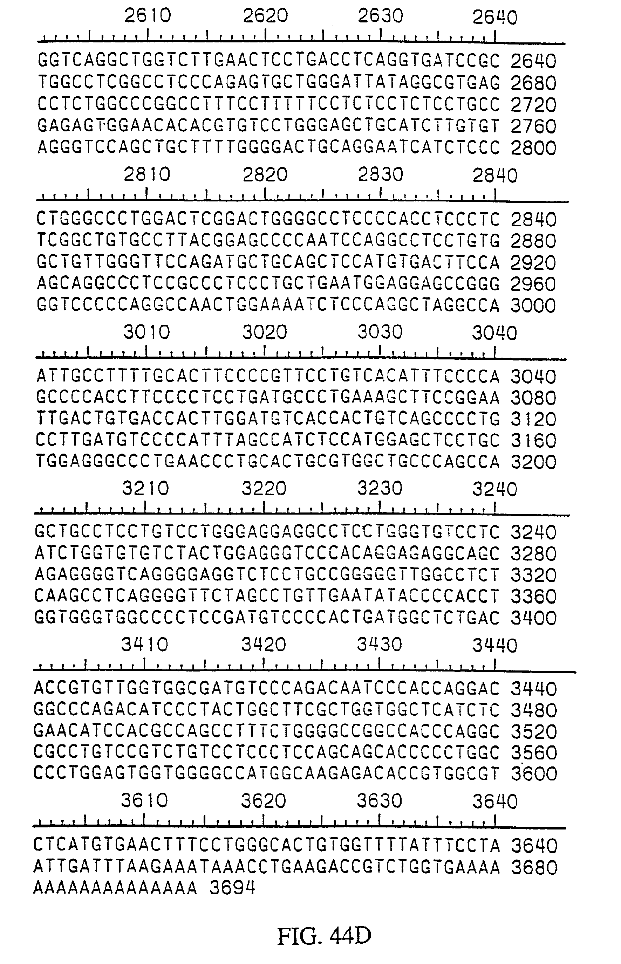

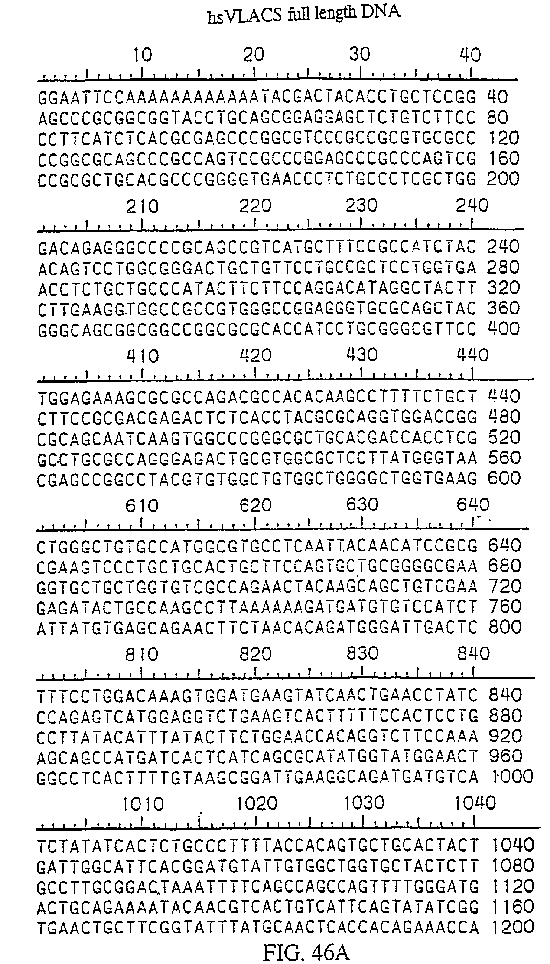

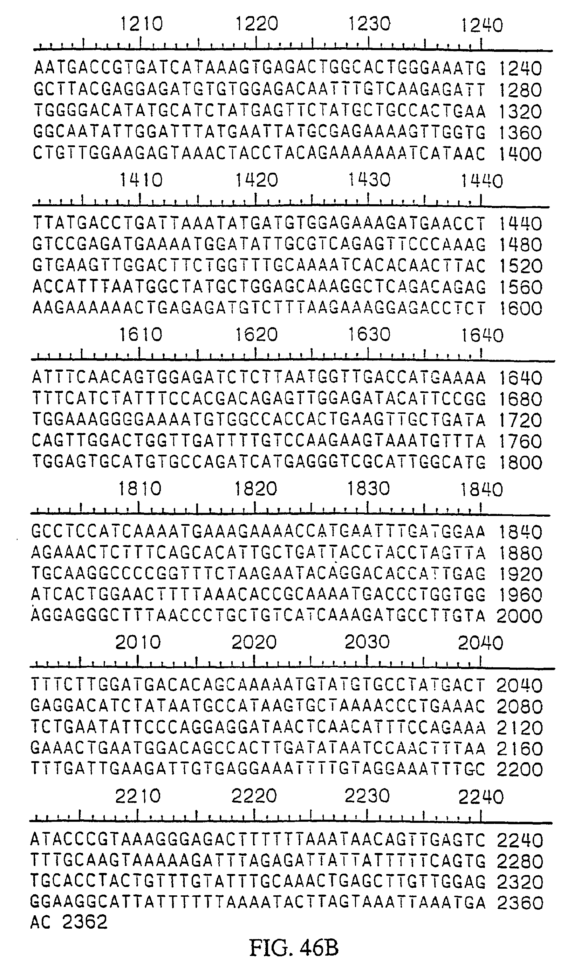

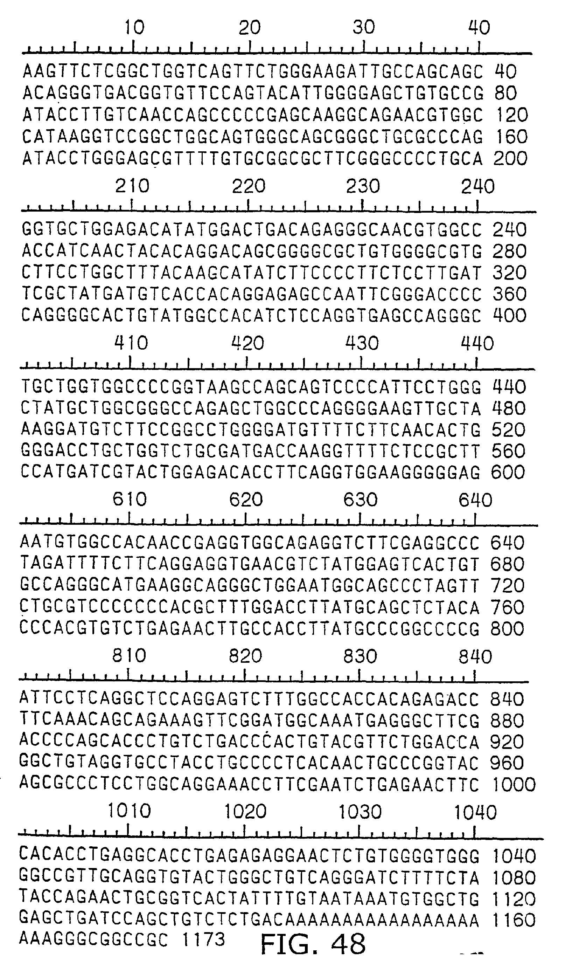

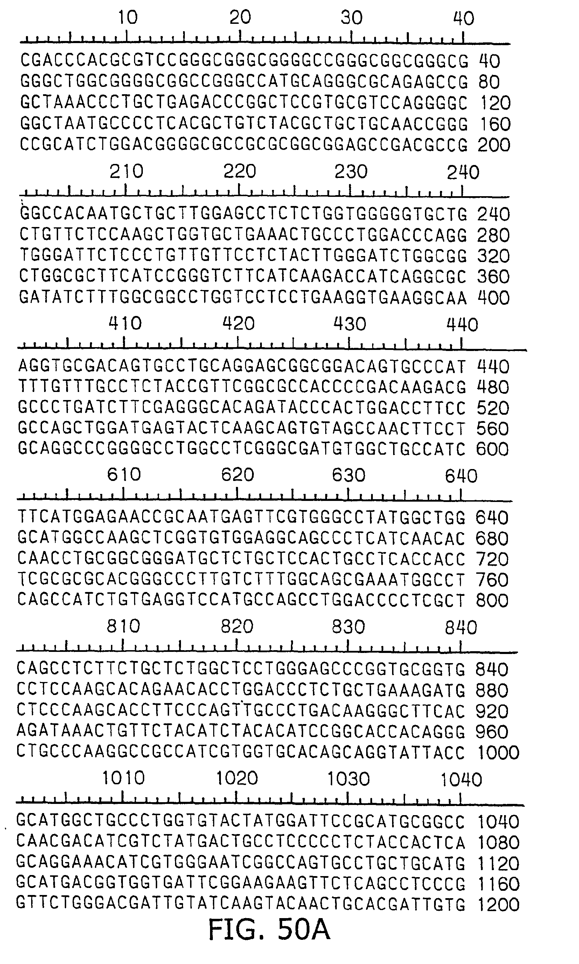

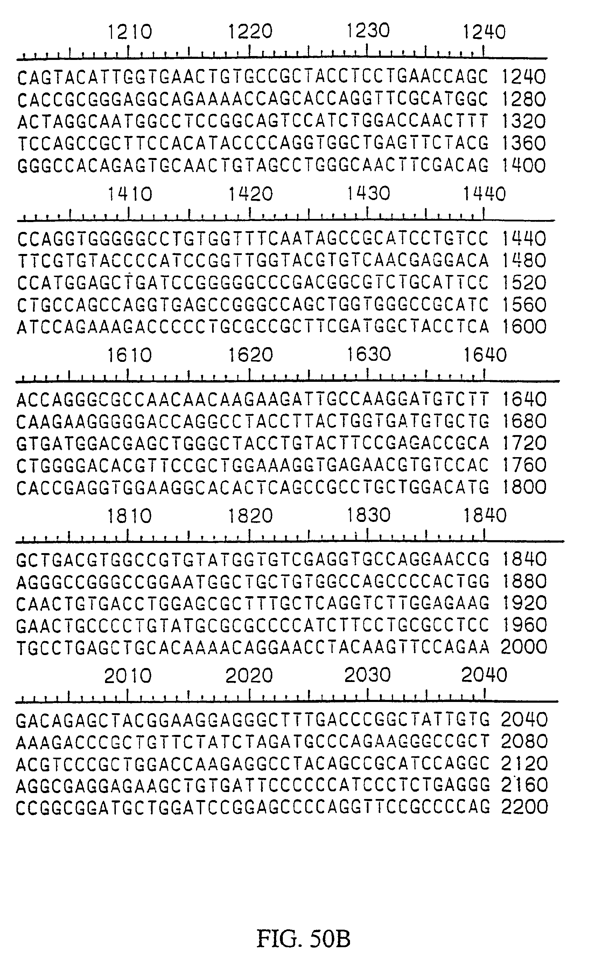

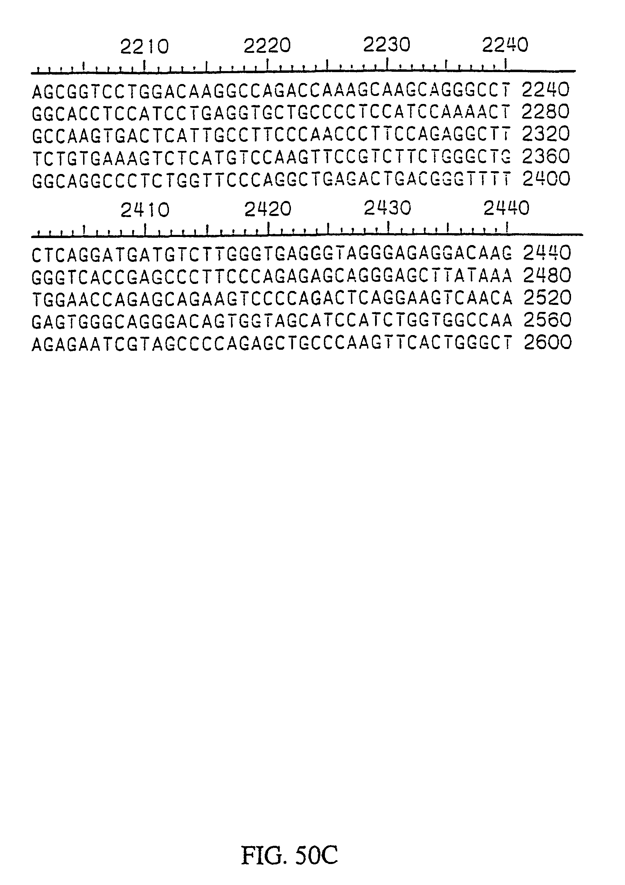

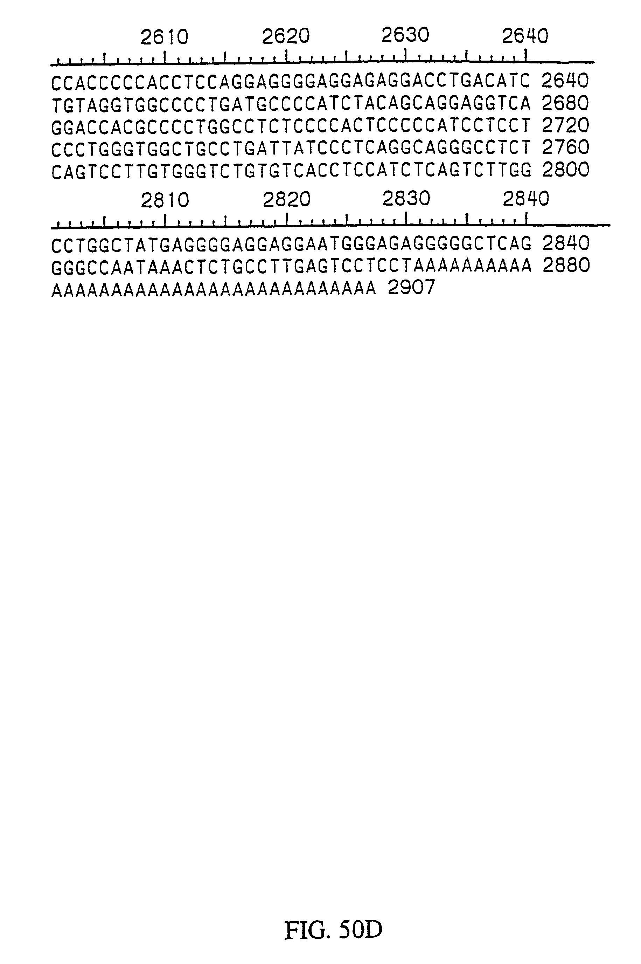

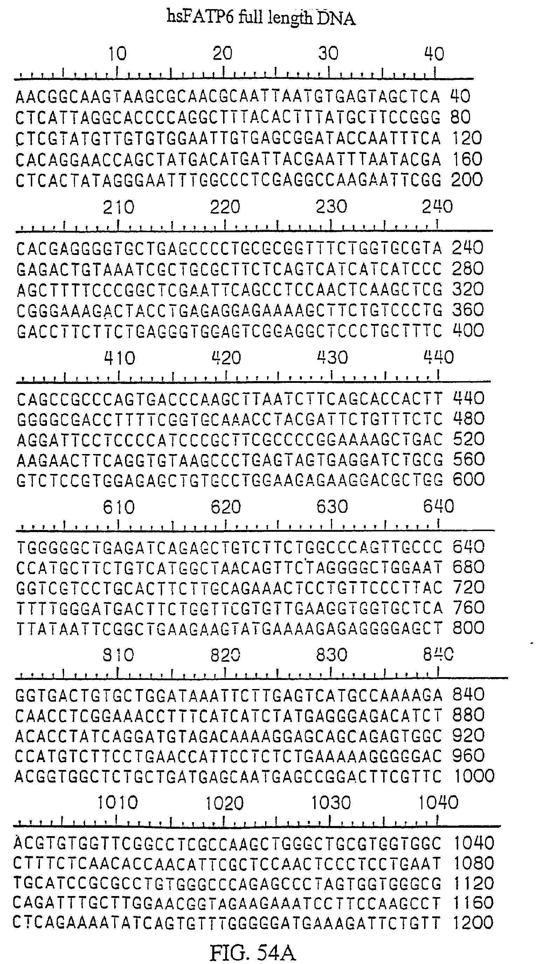

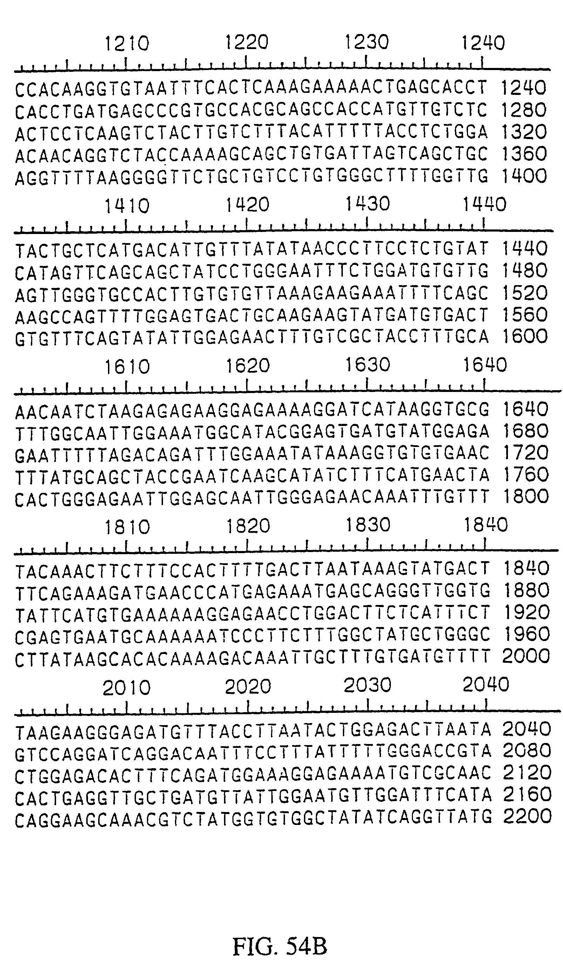

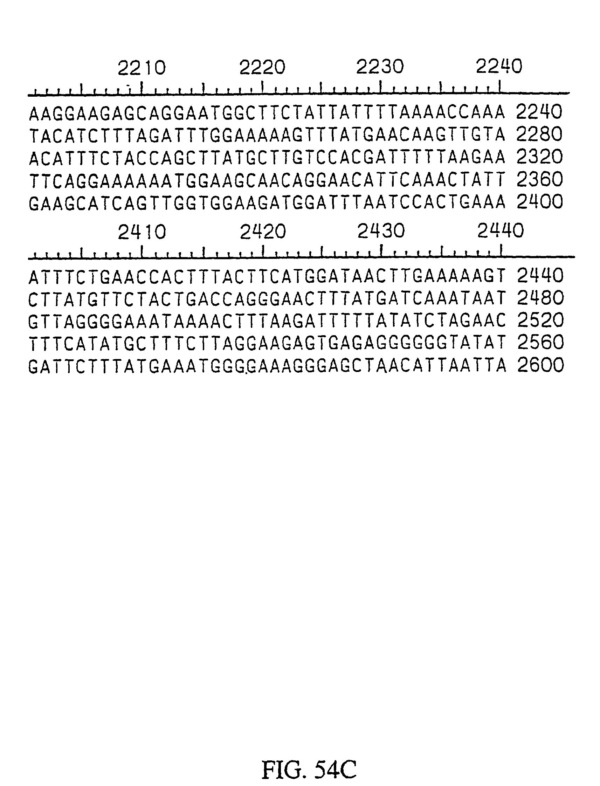

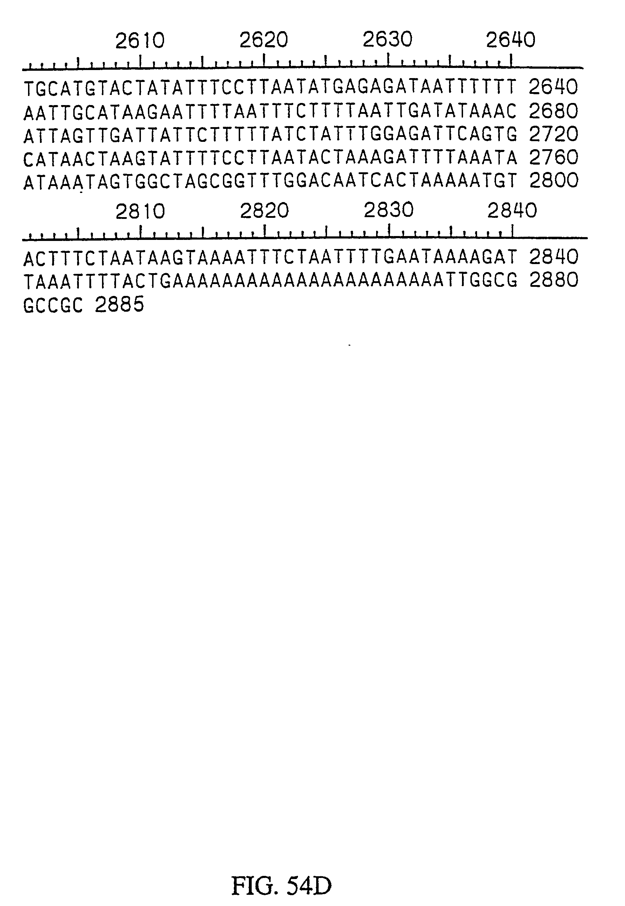

Using the information provided herein, such as a nucleic acid sequence set forth in Figures 44A-44C (SEQ ID NO:46), Figures 46A and 46B (SEQ ID NO:48), Figure 48 (SEQ ID NO:50), Figures 50A-50C (SEQ ID NO:52), Figures 94A and 94B (SEQ ID NO: 101), and Figures 54A-54C (SEQ ID NO:56), a nucleic acid encoding a FATP polypeptide may be obtained using standard cloning and screening methods, such as those for cloning and sequencing cDNA library fragments, followed by obtaining a full length clone. For example, to obtain such a nucleic acid, a library of clones of cDNA of human or other mammalian DNA can be probed with a labeled oligonucleotide, such as a radiolabeled oligonucleotide, preferably about 17 nucleotides or longer, derived from a partial sequence. Clones carrying DNA identical to that of the probe can then be distinguished using stringent (also, "high stringency") hybridization conditions. By sequencing the individual clones thus identified with sequencing primers designed from the original sequence it is then possible to extend the sequence in both directions to determine the full length sequence. Suitable techniques are described, for example, in *Current Protocols in Molecular Biology* (F.M.. Ausubel et al, eds), containing supplements through Supplement 42,1998, John Wiley and Sons, Inc., especially chapters 5, 6 and 7.

There are disclosed herein isolated nucleic acid molecules comprising any of the following nucleotide sequences: *1*.) a nucleotide sequence which encodes a protein comprising the amino acid sequence of hsFATP1 (SEQ ID NO:47), the amino acid sequence ofhsFATP2 (SEQ ID NO:49), the amino acid sequence of hsFATP3 (SEQ ID NO: 51), the amino acid sequence of hsFATP4 (SEQ ID NO: 53), the amino acid sequence of hsFATP5 (SEQ ID NO:102) or the amino acid sequence of hsFATP6 (SEQ ID NO:57); 2.) nucleotide sequences of hsFATP1, hsFATP2, hsFATP3, hsFATP4, hsFATP5, or hsFATP6 (SEQ ID NO:46,48, 50, 52, 101, or 56, respectively); *3*.) a nucleotide sequence which is complementary to the nucleotide sequence ofhsFATP1 (SEQ ID NO:46), hsFATP2 (SEQ ID NO:48), hsFATP3 (SEQ ID NO:50), hsFATP4 (SEQ ID NO:52), hsFATP5 (SEQ ID NO:101) or hsFATP6 (SEQ ID NO:56); *4.*) a nucleotide sequence which consists of the coding region of hsFATP1 (SEQ ID NO:46), the coding region of hsFATP2 (SEQ ID NO:48), the coding region of hsFATP3 (SEQ ID NO:50), the coding region of hsFATP4 (SEQ ID NO:52), the coding region of hsFATP5 (SEQ ID NO:101), or the coding region of hsFATP6 (SEQ ID NO:56).

Also disclosed are nucleic acids (nucleic acid molecules or polynucleotides) having nucleotide sequences identical over their entire length to those shown in the figures, for instance Figures 44A-44C (SEQ ID NO:46), Figures 46A and 46B (SEQ ID NO:43), Figure 48 (SEQ ID NO:50), Figures 50A-50C (SEQ ID NO:52), Figures 94A and 94B (SEQ ID NO:101), and Figures 54A-54C (SEQ ID NO:56). Also disclosed herein is DNA, which due to the degeneracy of the genetic code, encodes a FATP encoded by one of the FATP-encoding DNAs, whose amino acid sequence is provided herein. Also disclosed herein are nucleic acids having the coding sequences for the mature polypeptides or fragments in reading frame with other coding sequences, such as those encoding a leader or secretory sequence, a pre-, or pro- or prepro- protein sequence. These nucleic acids encompass nucleic acids that include a single continuous region or discontinuous regions encoding the polypeptide, together with additional regions, that may also contain coding or non-coding sequences. The nucleic acids may also contain non-coding sequences, including, for example, but not limited to, non-coding 5' and 3' sequences, such as the transcribed, non-translated sequences, termination signals, ribosome binding sites, sequences that stabilize mRNA, introns, polyadenylation signals, and additional coding sequences which encode additional amino acids. For example, a marker sequence that facilitates purification of the fused polypeptide can be encoded. The marker sequence can be a hexa-histidine peptide, as provided in the pQE vector (Qiagen, Inc.) and described in Gentz *et al., Proc. Natl. Acad. Sci. USA 86:* 821-824 (1989), or an HA tag (Wilson *et al., Cell 37:* 767 (1984)), or a sequence encoding glutathione S-transferase of *Schistosoma japonicum* (vectors available from Pharmacia; see Smith, D.B. and Johnson K.S., *Gene 67*:31 (1988) and Kaelin, W.G. *et al., Cell 70*:351 (1992)). Nucleic acids disclosed herein also include, but are not limited to, nucleic acids comprising a structural gene and its naturally associated sequences that control gene expression.

Also disclosed herein are variants, including naturally-occurring allelic variants, of those nucleic acids described specifically herein by DNA sequence, that encode variants of such polypeptides as those having the amino acid sequences shown in Figure 45 (SEQ ID NO:47), Figure 47 (SEQ ID NO:49), Figure 49 (SEQ ID NO:51), Figure 51 (SEQ ID NO:53) Figures 94A and 94B (SEQ ID NO: 102), or Figure 55 (SEQ ID NO:57). Such variants include nucleic acids encoding variants of the above-listed amino acid sequences, wherein those variants have several, such as 5 to 10, 1 to 5, or 3, 2 or 1 amino acids substituted, deleted, or added, in any combination. Variants include polynucleotides encoding polypeptides with at least 95% but less than 100% amino acid sequence identity to the polypeptides described herein by amino acid sequence. Variant polynucleotides hybridize, under low to high stringency conditions, to the alleles described herein by DNA sequence. In one embodiment, variants have silent substitutions, additions and deletions that do not alter the properties and activities of the FATP. Allelic variants of the polynucleotides encoding hsFATP1 (Figure 45; SEQ ID NO:47), hsFATP2 (Figure 47; SEQ ID NO:49), hsFATP3 (Figure 49; SEQ ID NO:51), hsFATP4 (Figure 51; SEQ ID NO:53), Figures 94A and 94B (SEQ ID NO:102) and hsFATP6 (Figure 55; SEQ ID NO:57) will be identified as mapping to chromosomal locations listed for the corresponding wild type genes in Table 2 in Example 1.

Orthologous genes are gene loci in different species that are sufficiently similar to each other in their nucleotide sequences to suggest that they originated from a common ancestral gene. Orthologous genes arise when a lineage splits into two species, rather than when a gene is duplicated within a genome. Proteins that are orthologs are encoded by genes of two different species, wherein the genes are said to be orthologous.

There are also disclosed herein polynucleotides encoding polypeptides which are orthologous to those polypeptides having a specific amino acid sequence described herein, such as the amino acid sequences shown in Figure 45 (SEQ ID NO:47), Figure 47 (SEQ ID NO:49), Figure 49 (SEQ ID NO:51), Figure 51 (SEQ ID NO:53), Figures 94A and 94B (SEQ ID NO:102), or Figure 55 (SEQ ID NO:57). These polynucleotides, which can be called ortholog polynucleotides, encode orthologous polypeptides that can range in amino acid sequence identity to a reference amino acid sequence described herein, from about 65% to less than 100%, but preferably 70% to 80%, more preferably 80% to 90%, and still more preferably 90% to less than 100%. Orthologous polypeptides can also be those polypeptides that range in amino acid sequence similarity to a reference amino acid sequence described herein from about 75% to 100%, within the signature sequence. The amino acid sequence similarity between the signature sequences of orthologous polypeptides is preferably 80%, more preferably 90%, and still more preferably, 95%. The ortholog polynucleotides encode polypeptides that have similar functional characteristics (e.g., fatty acid transport activity) and similar tissue distribution, as appropriate to the organism from which the ortholog polynucleotides can be isolated.

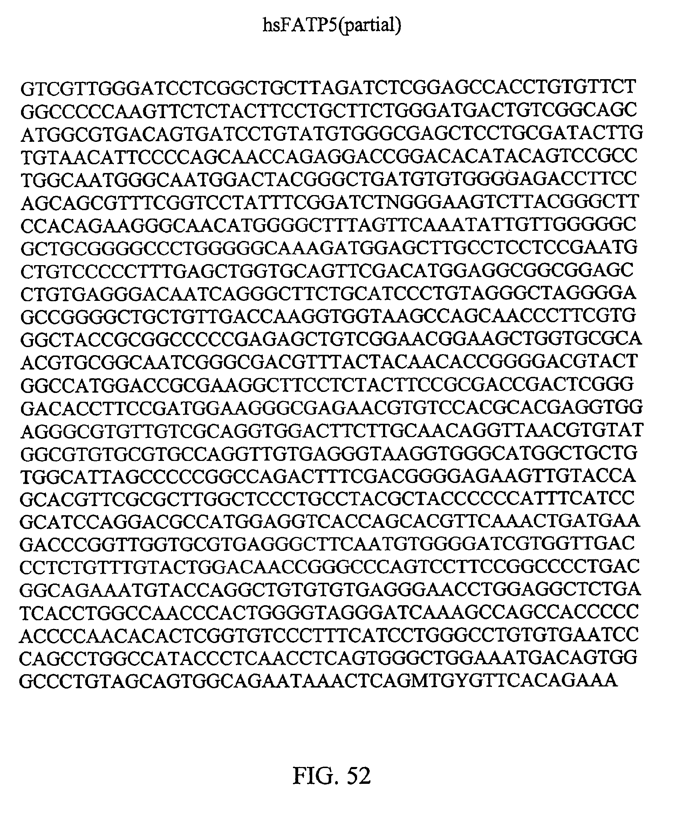

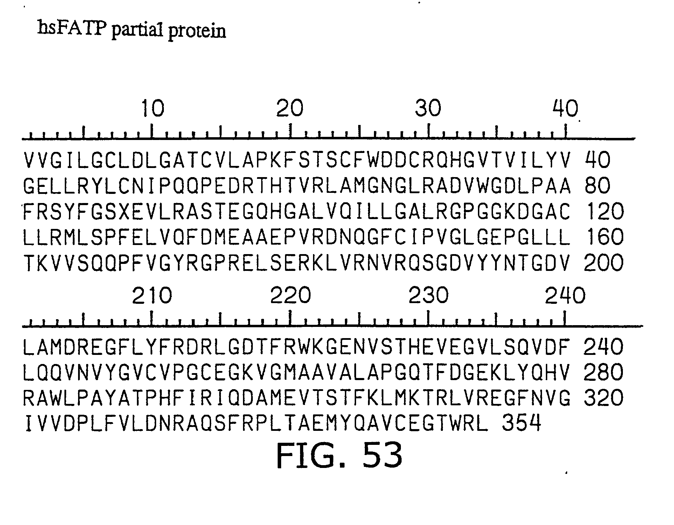

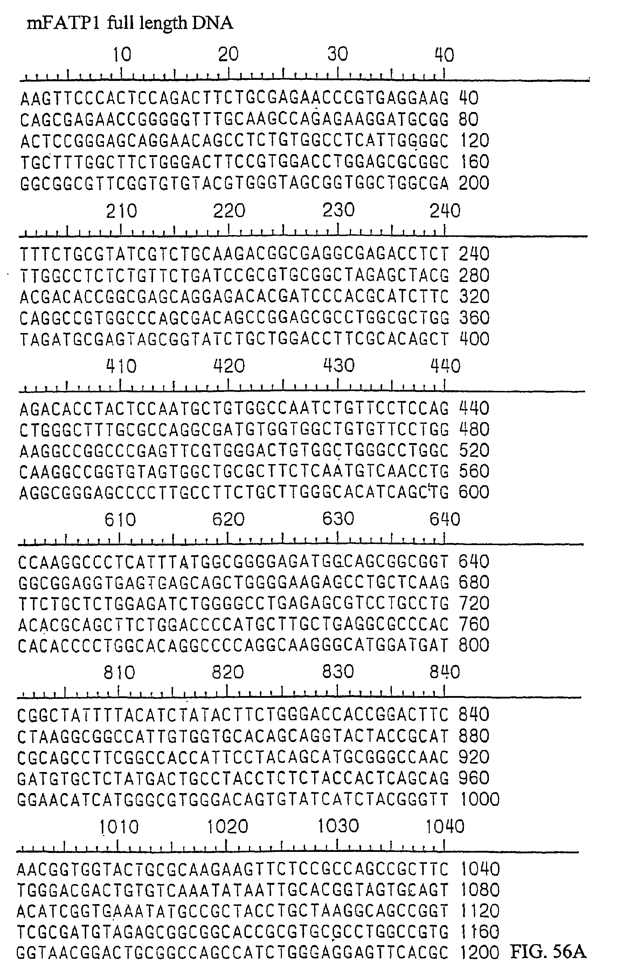

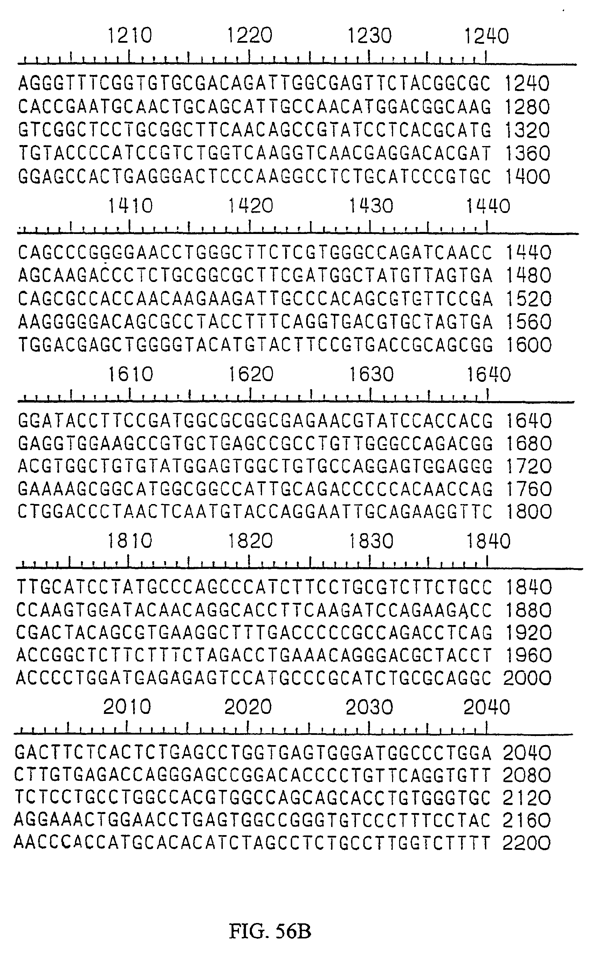

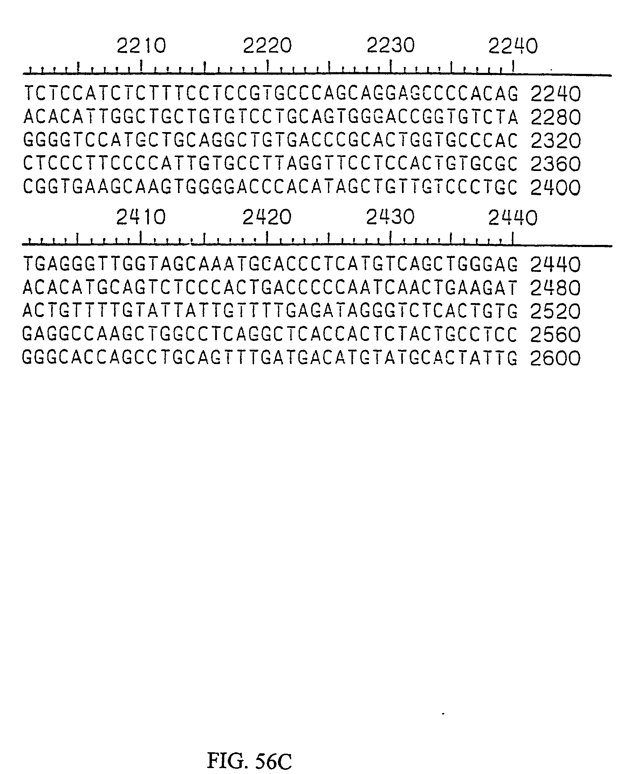

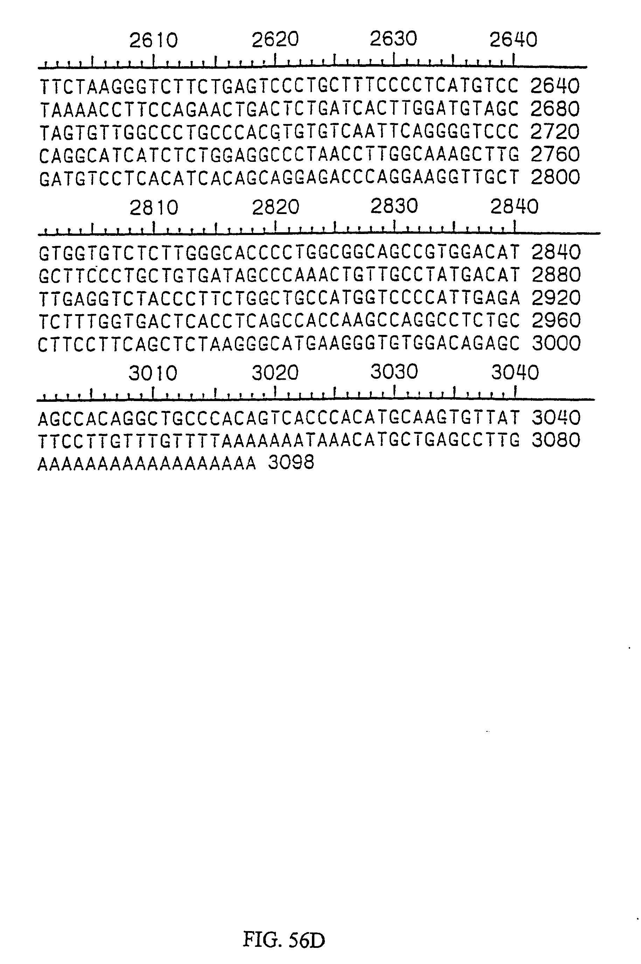

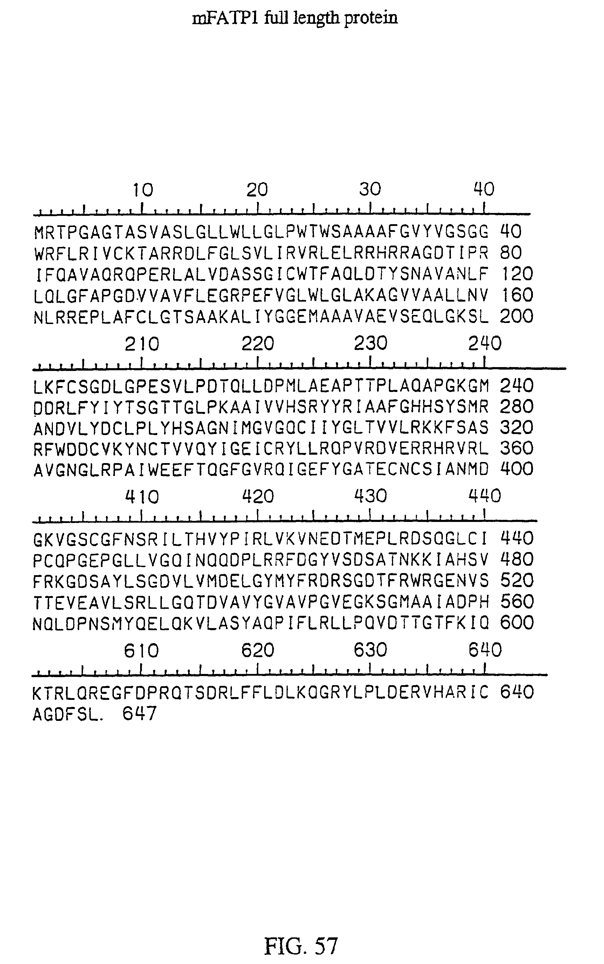

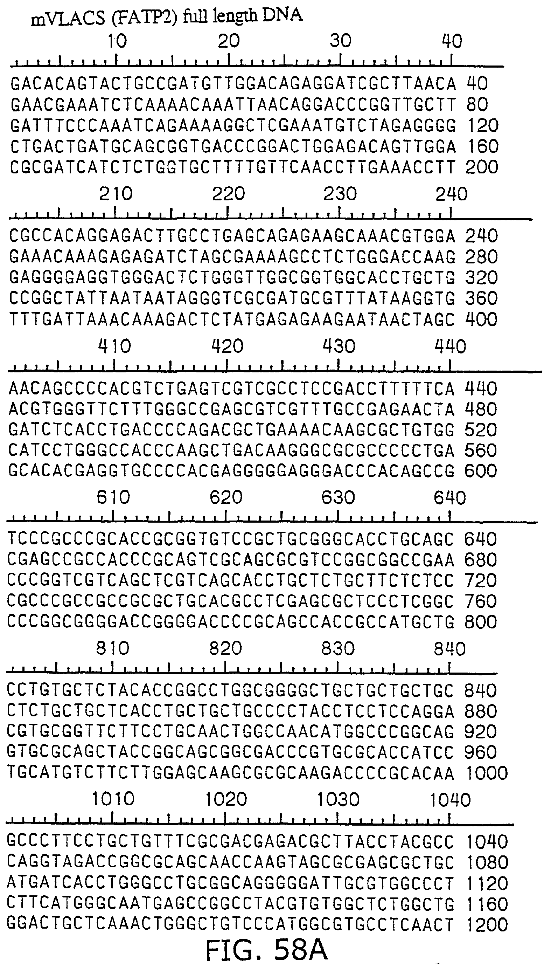

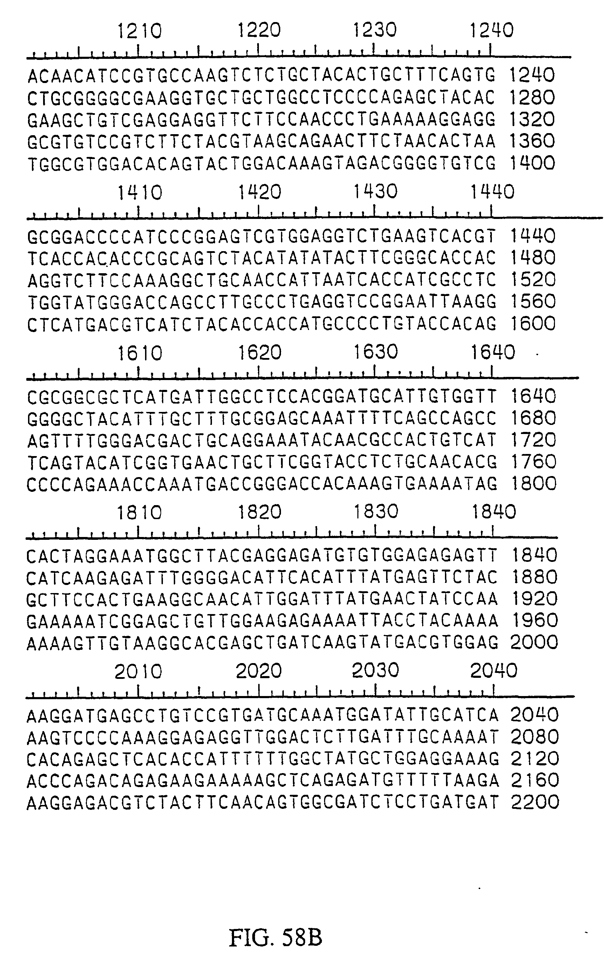

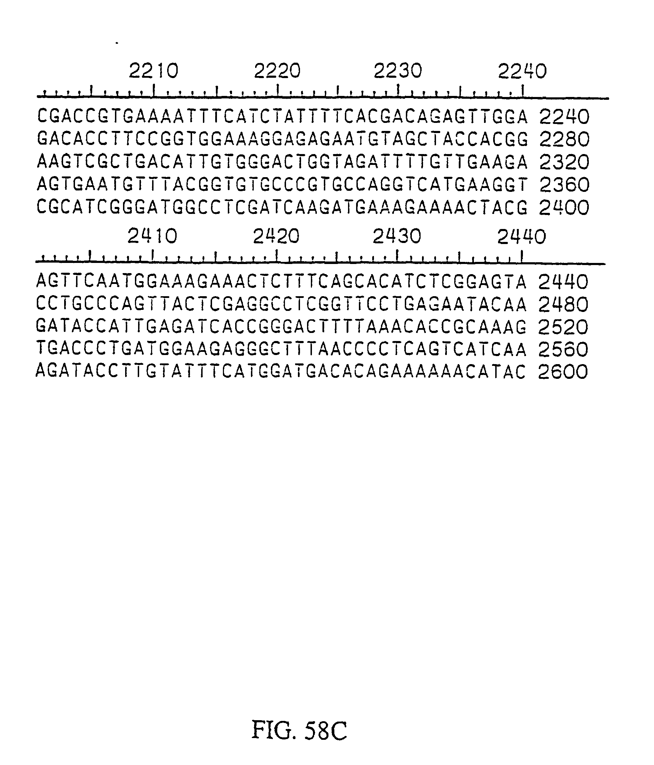

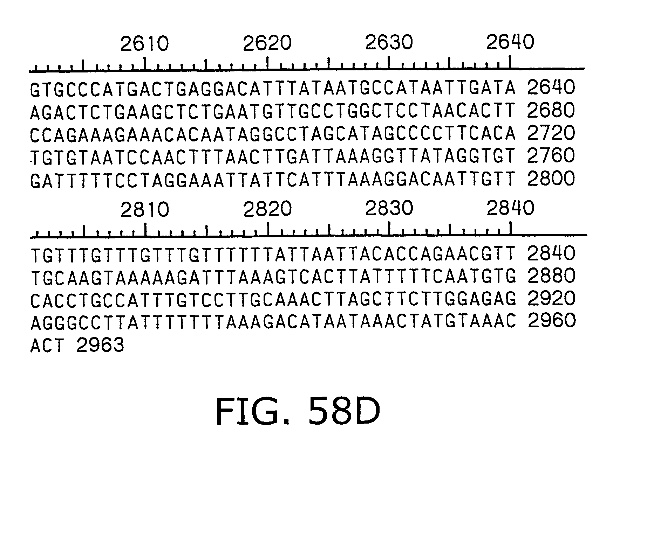

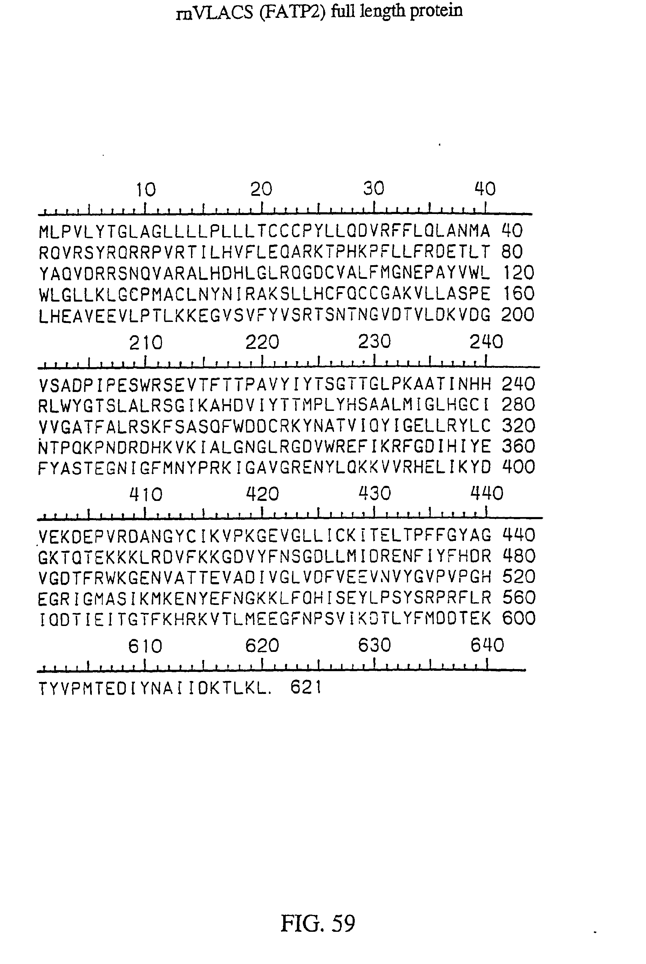

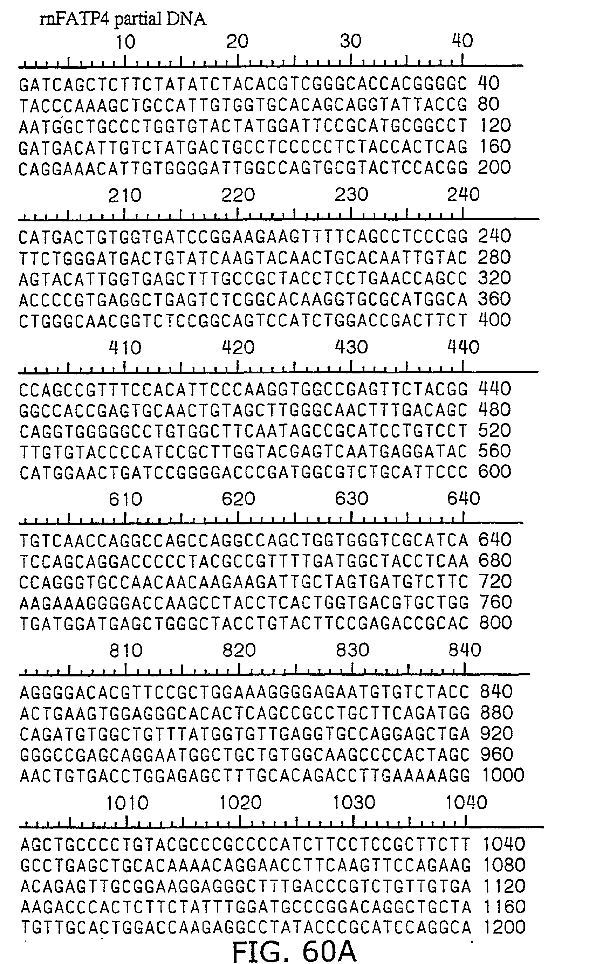

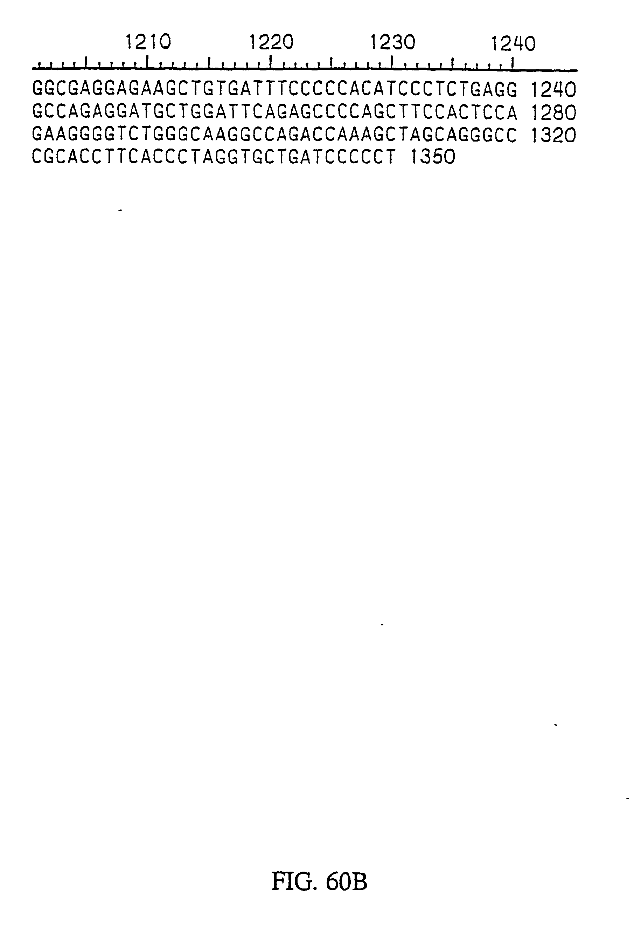

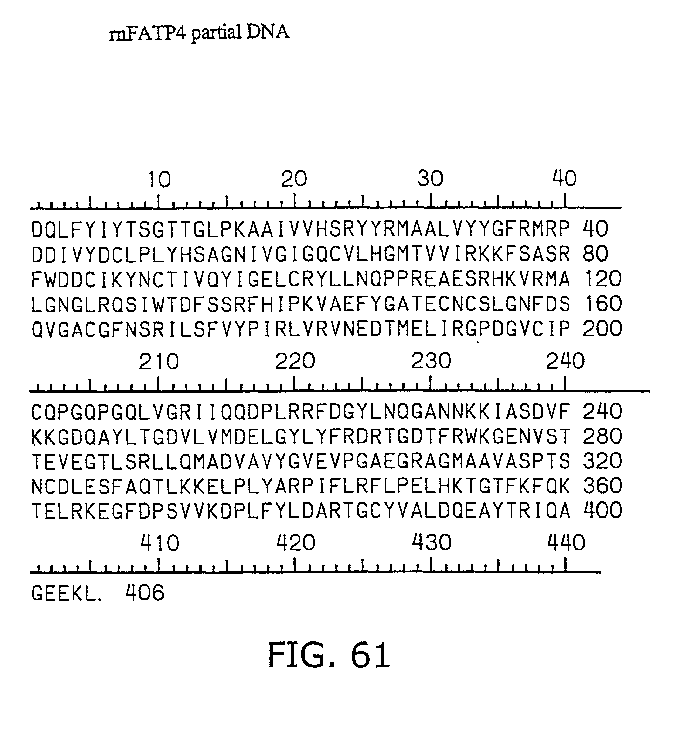

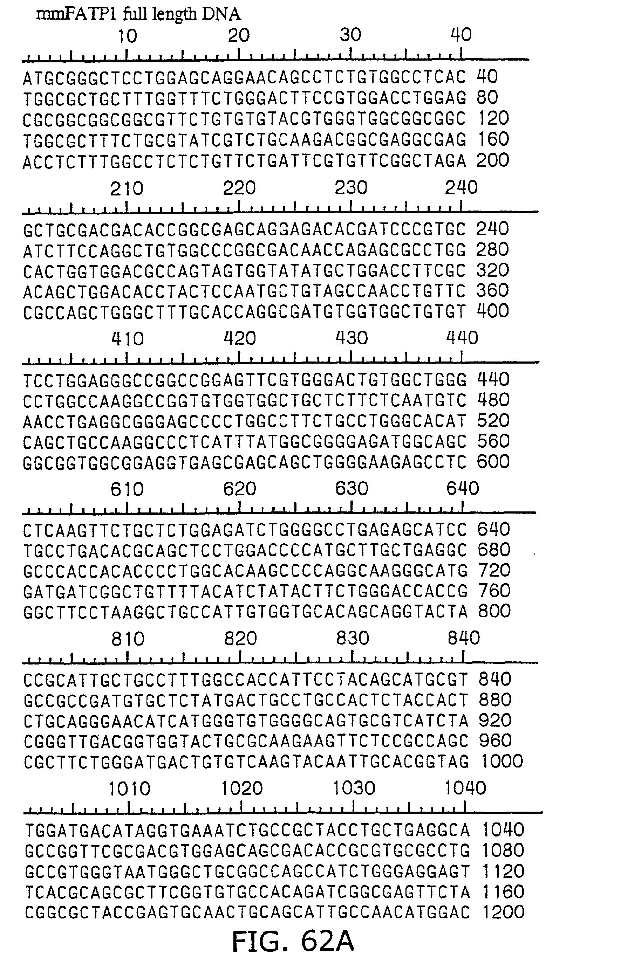

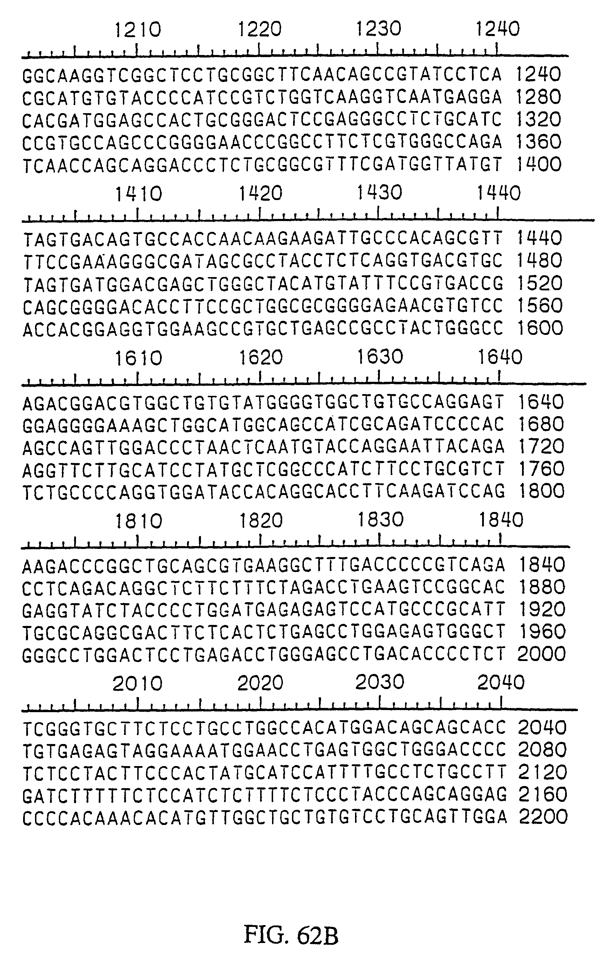

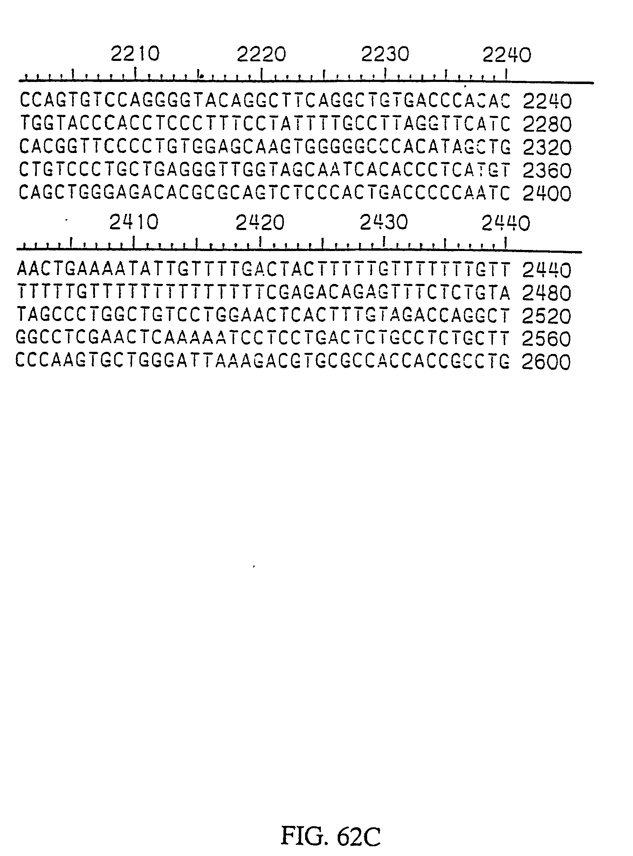

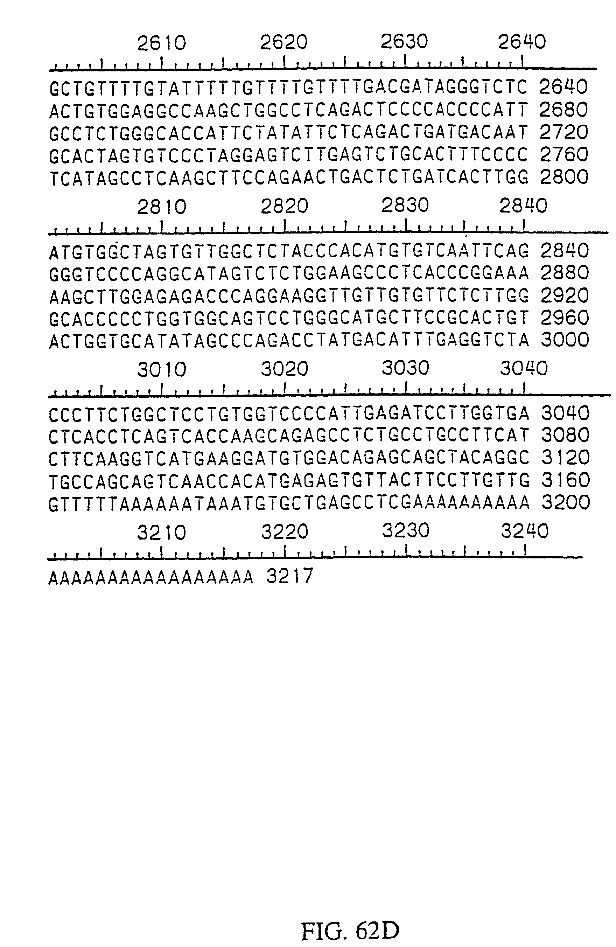

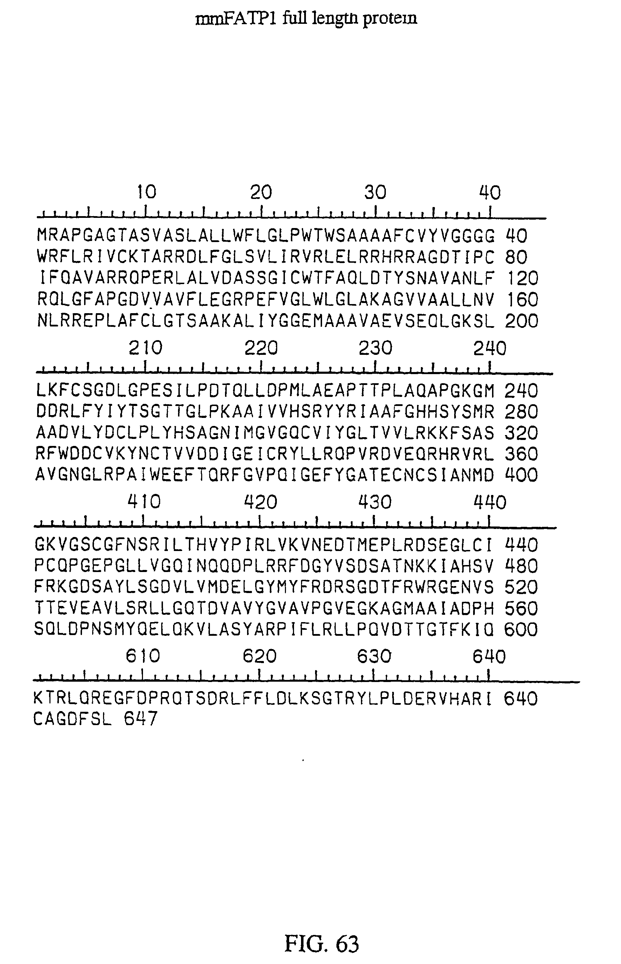

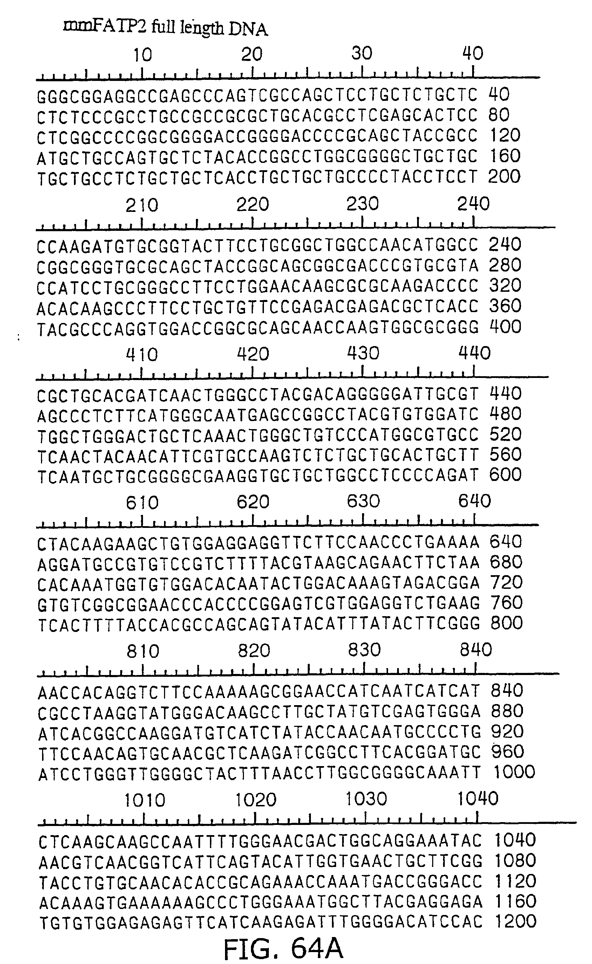

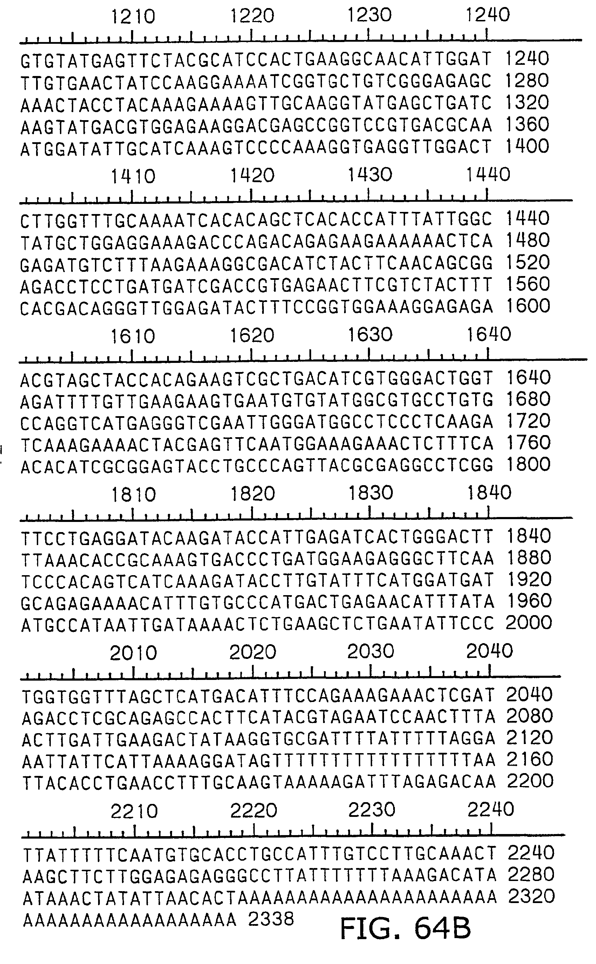

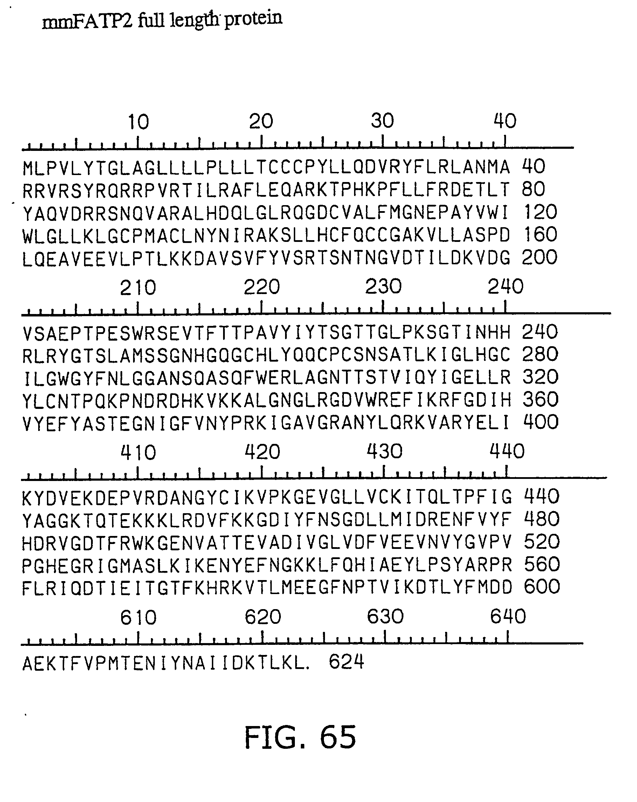

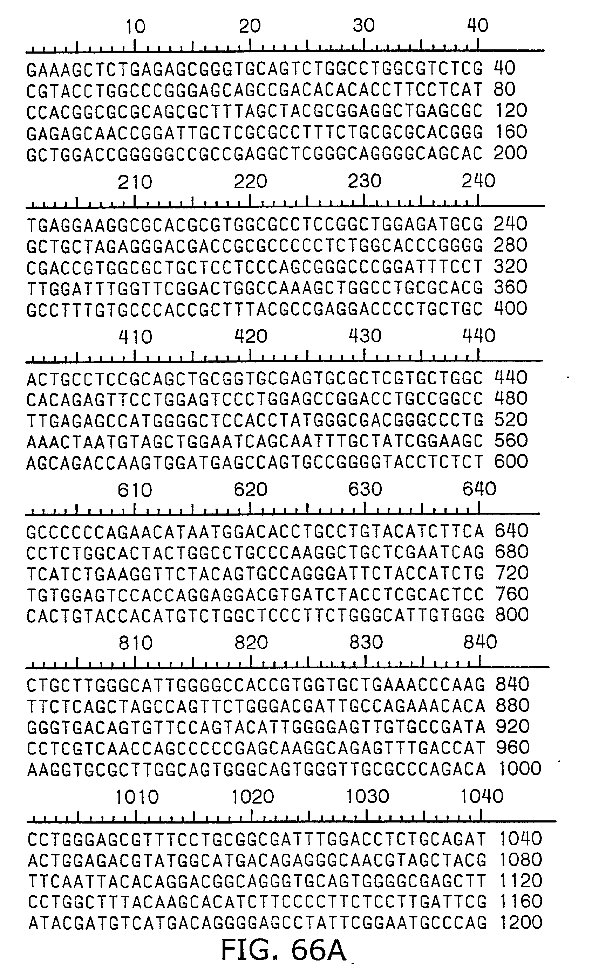

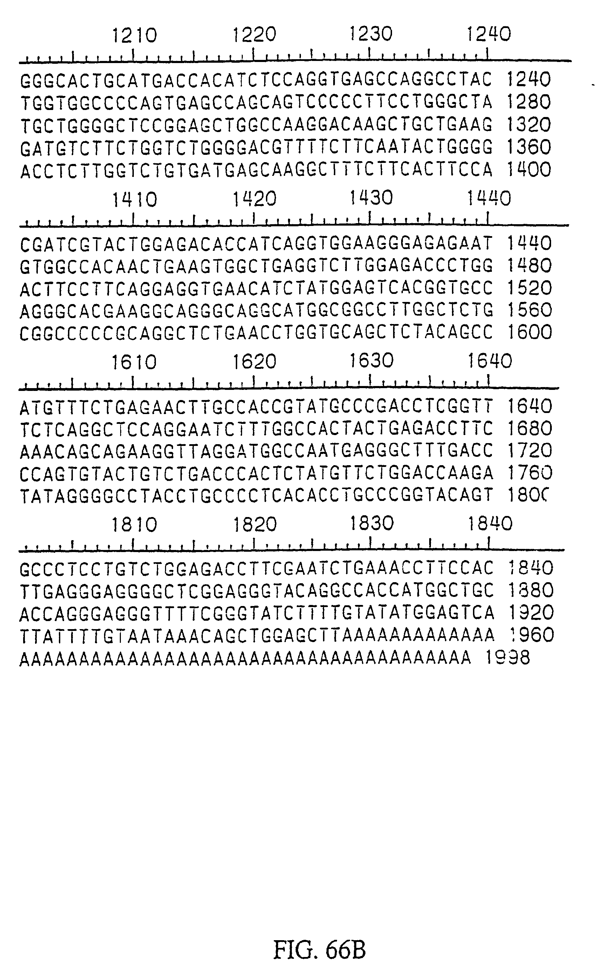

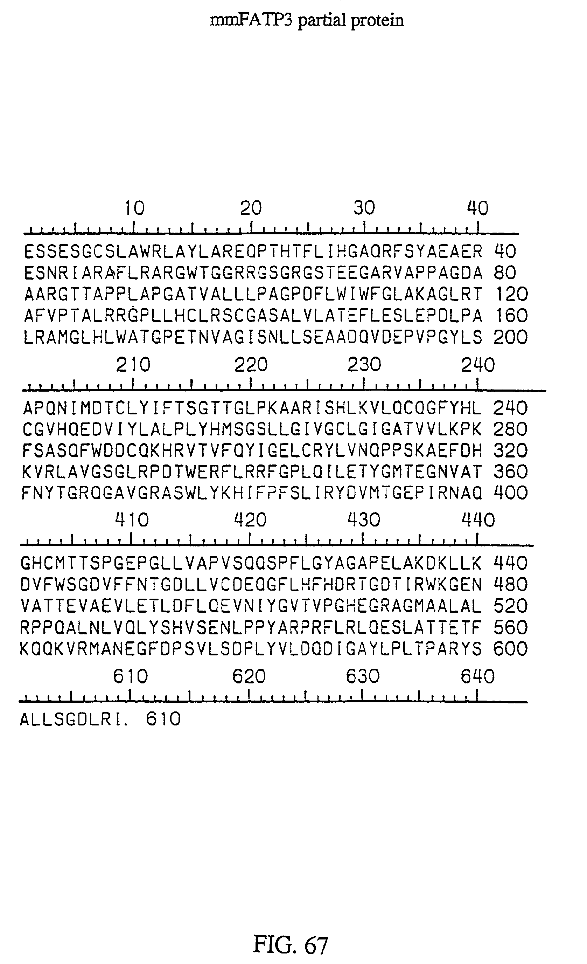

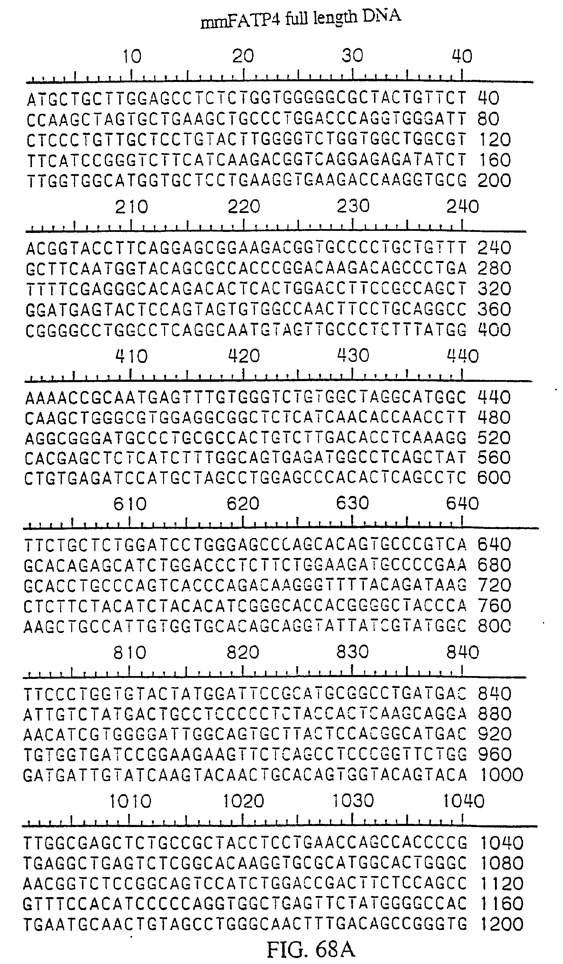

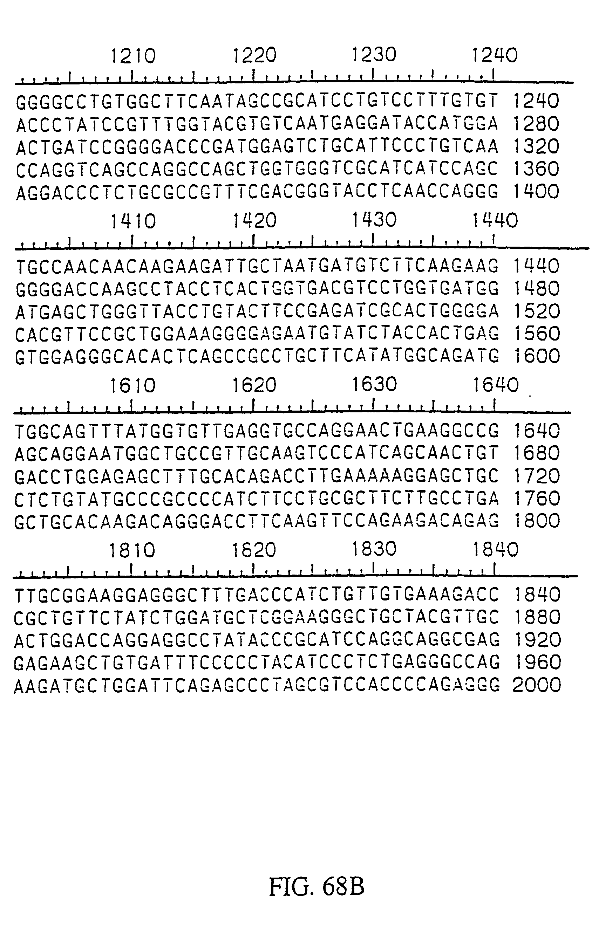

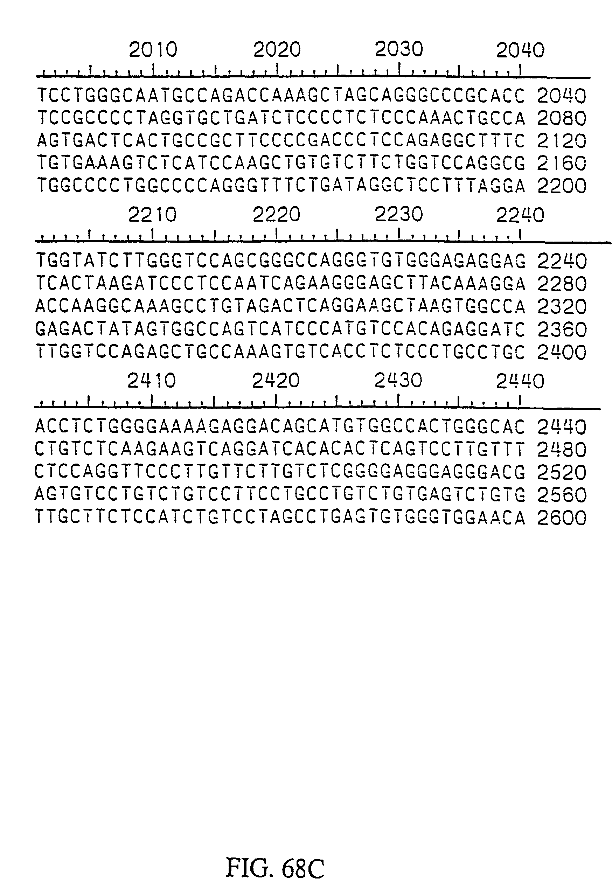

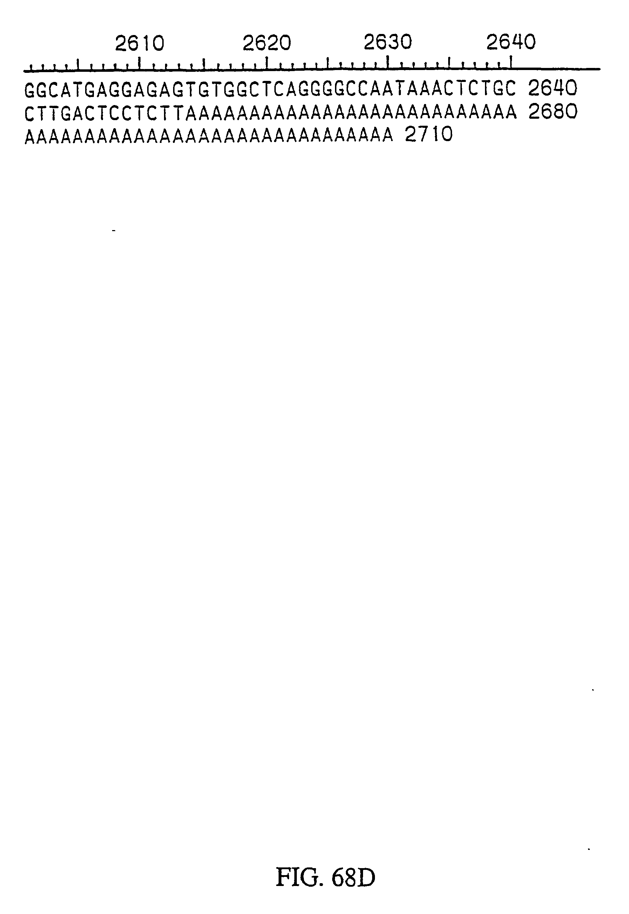

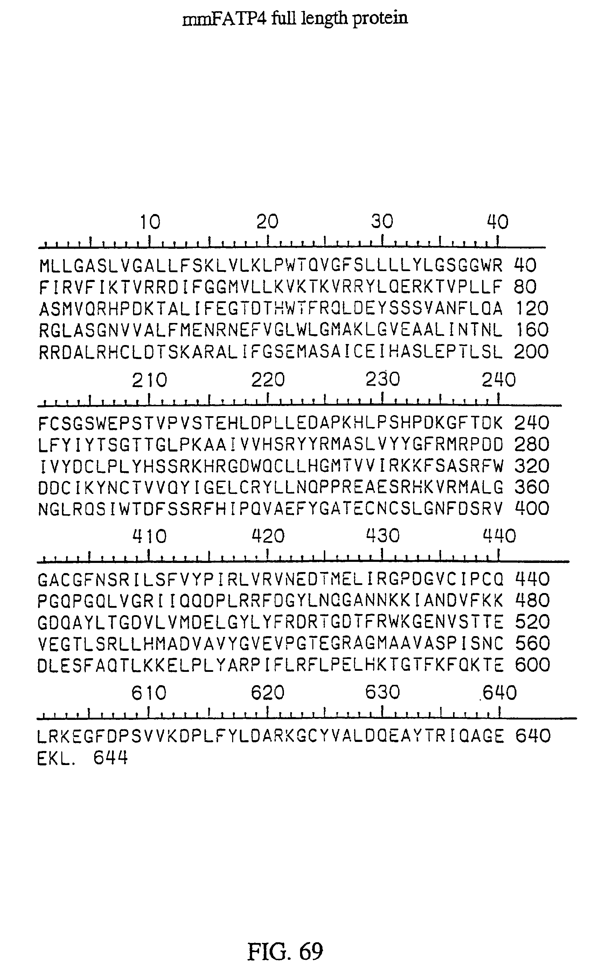

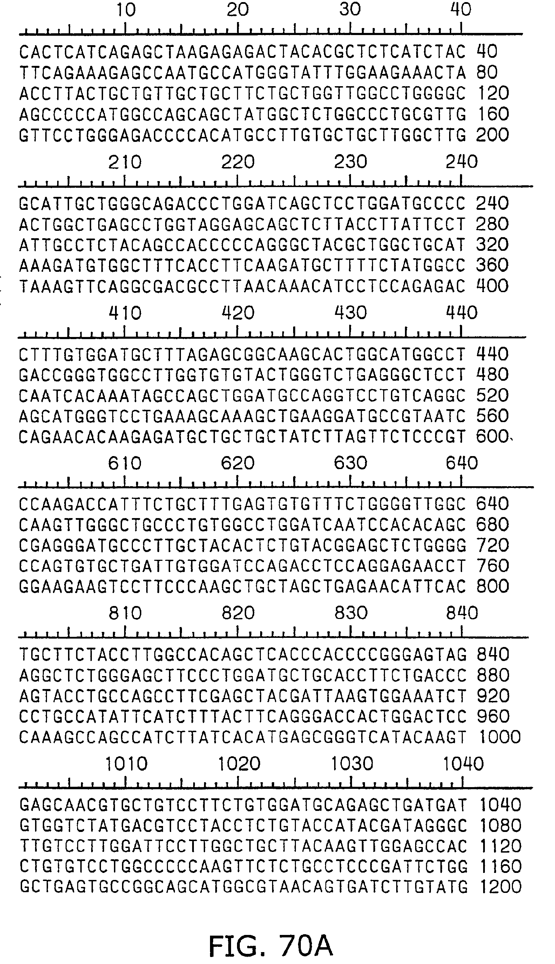

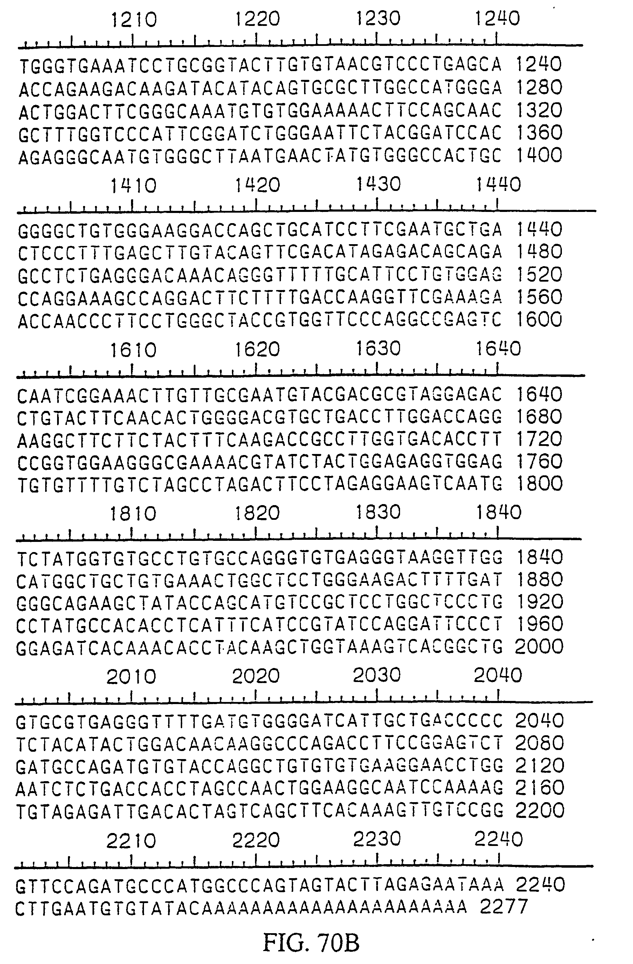

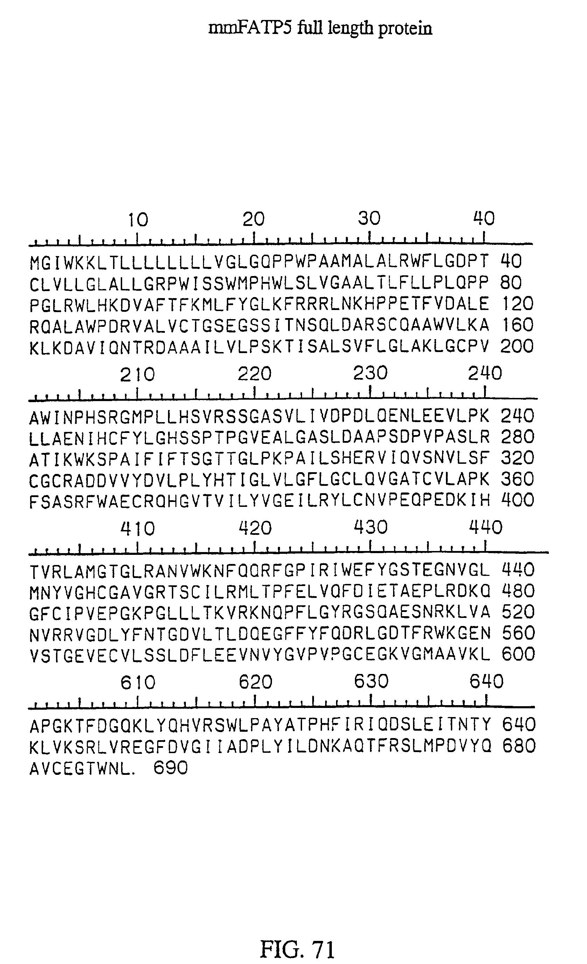

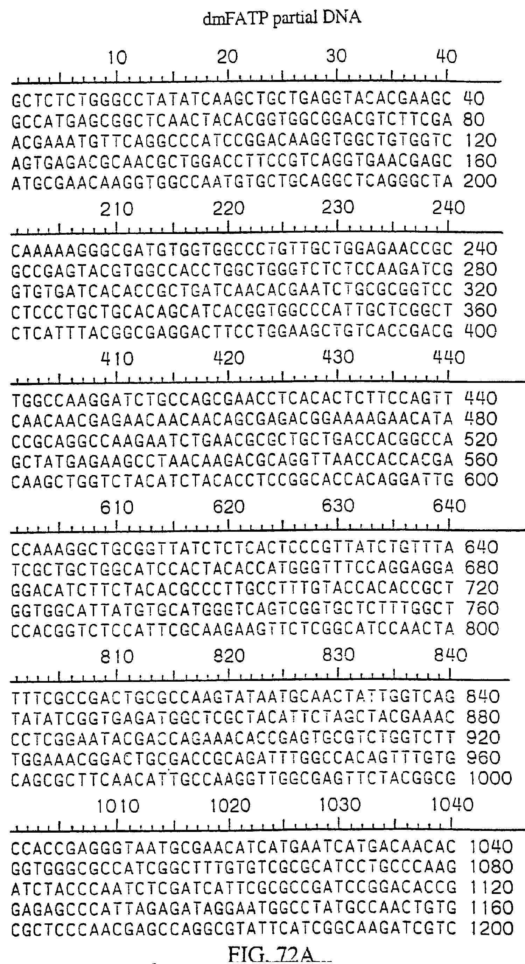

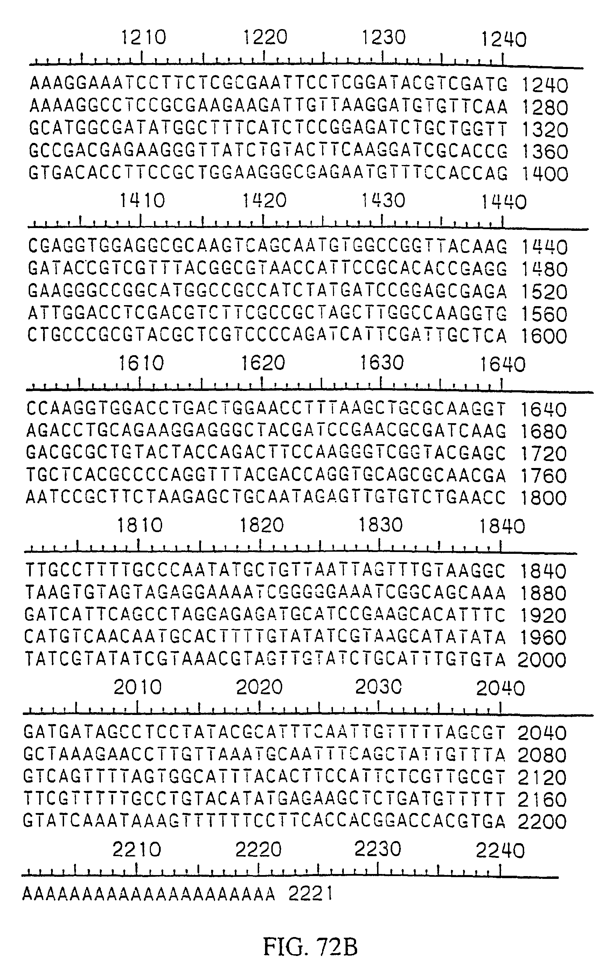

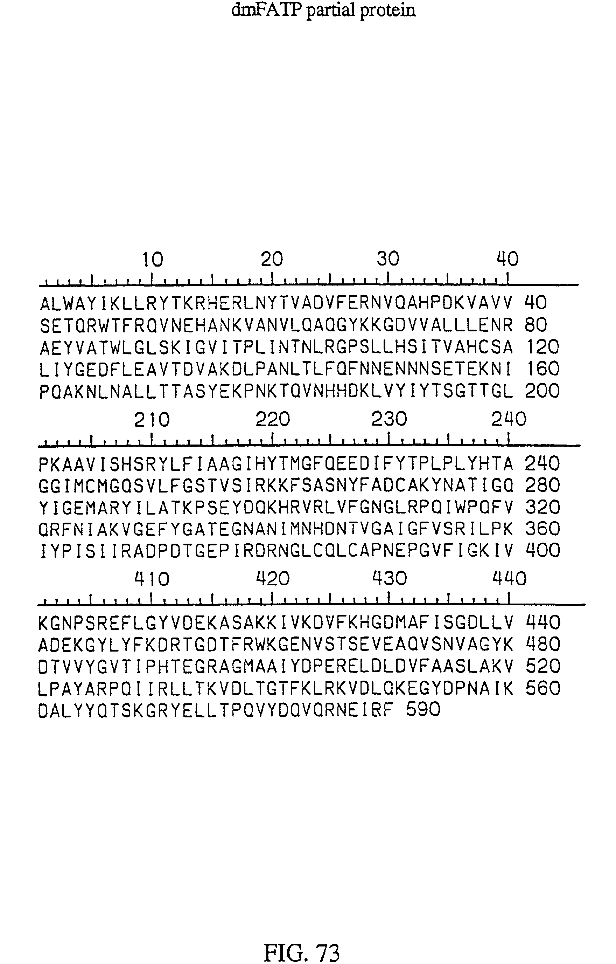

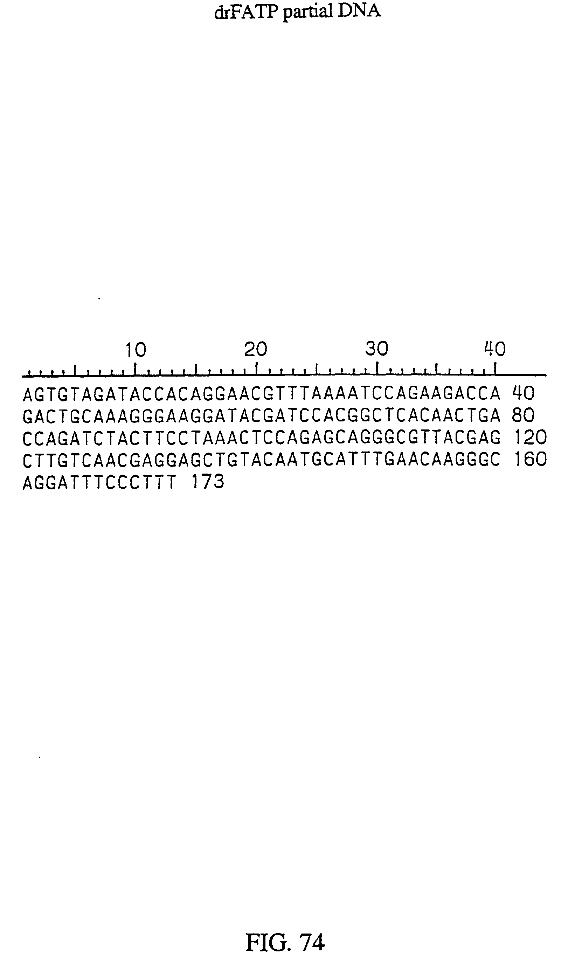

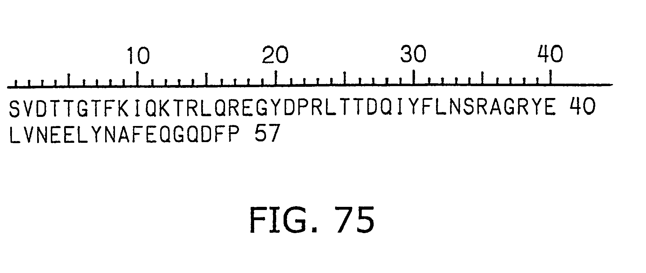

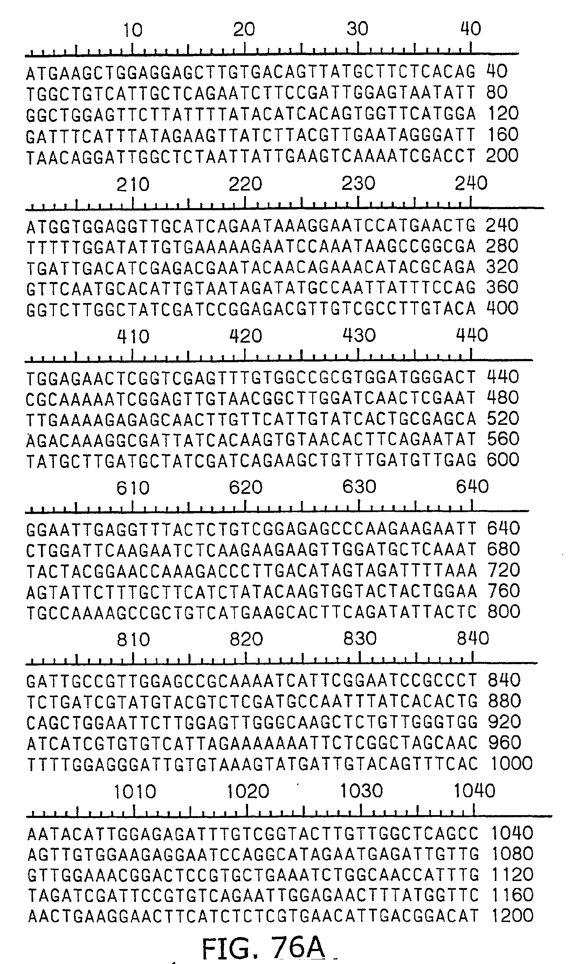

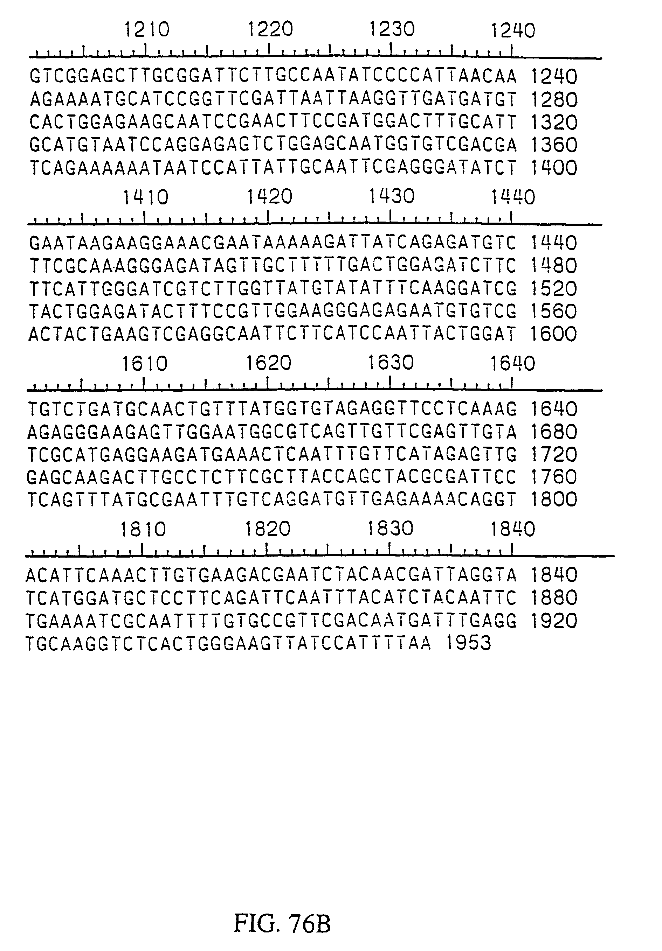

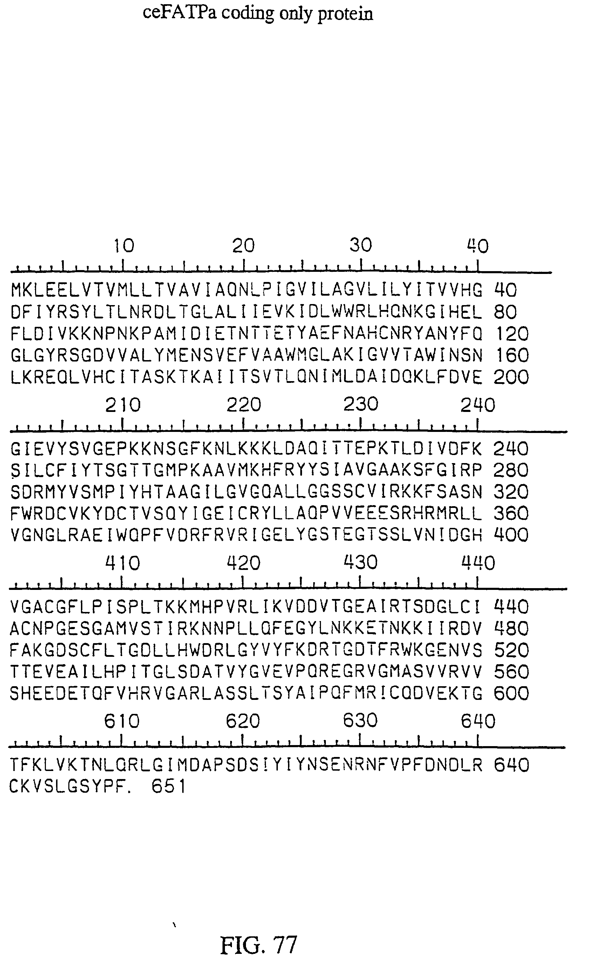

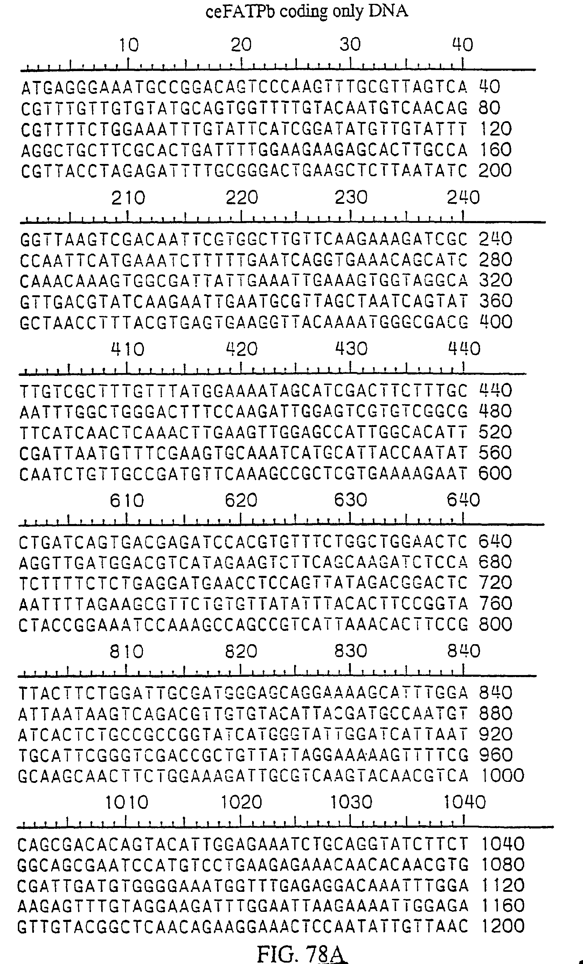

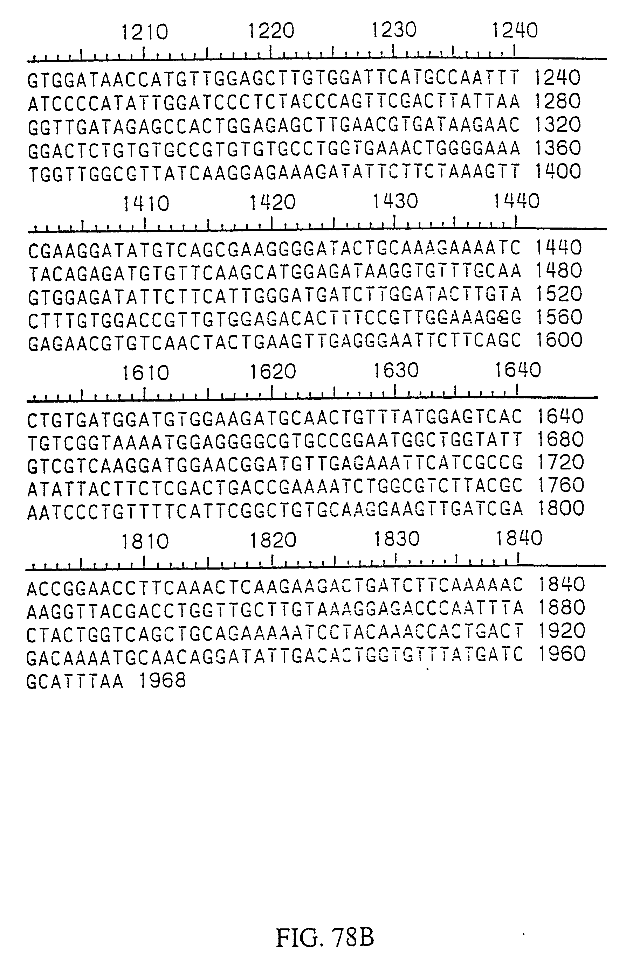

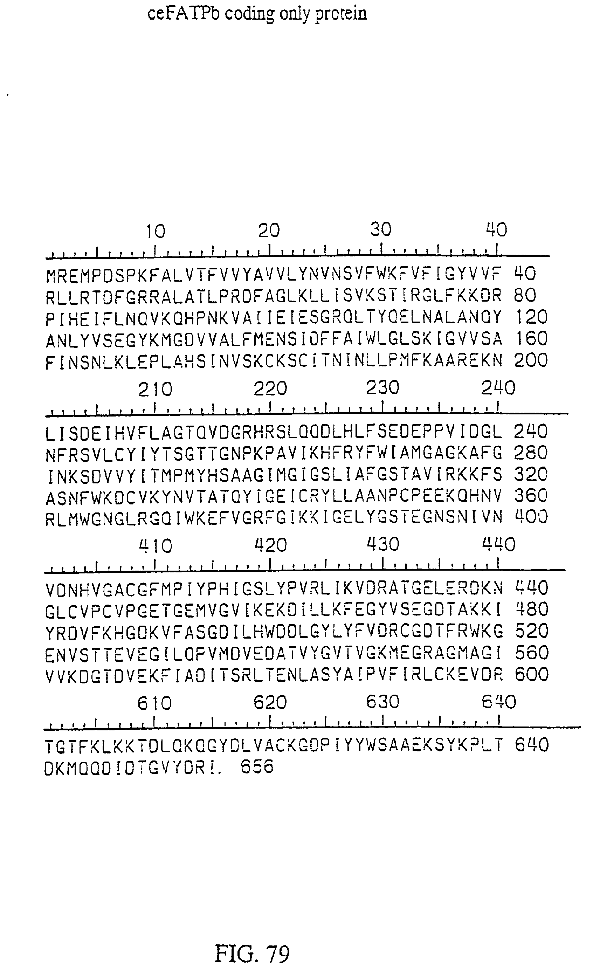

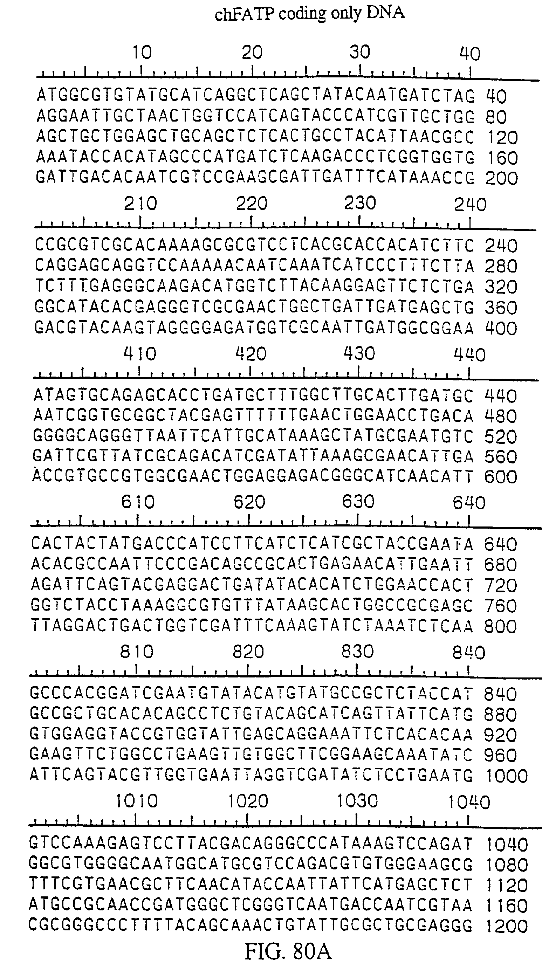

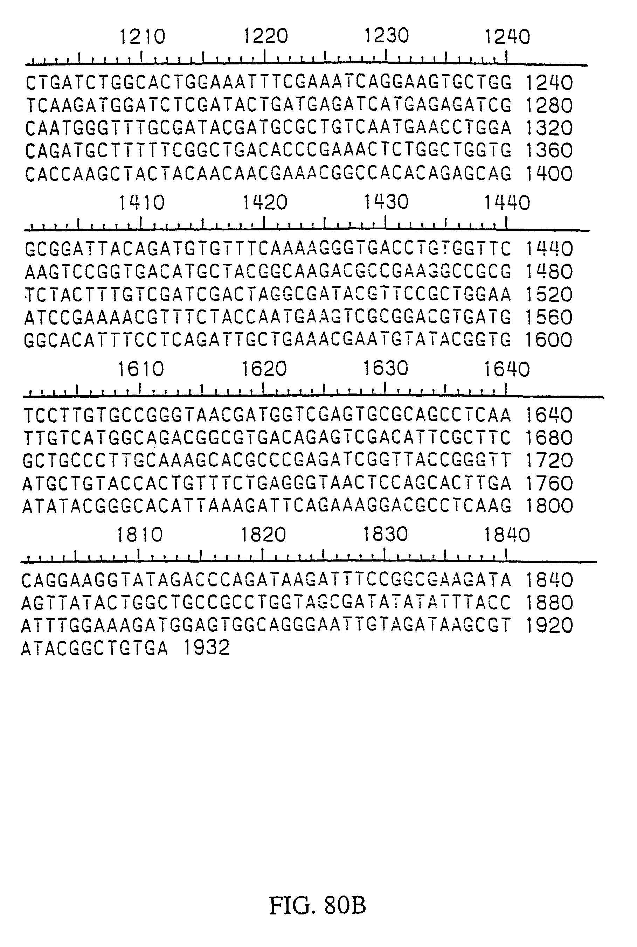

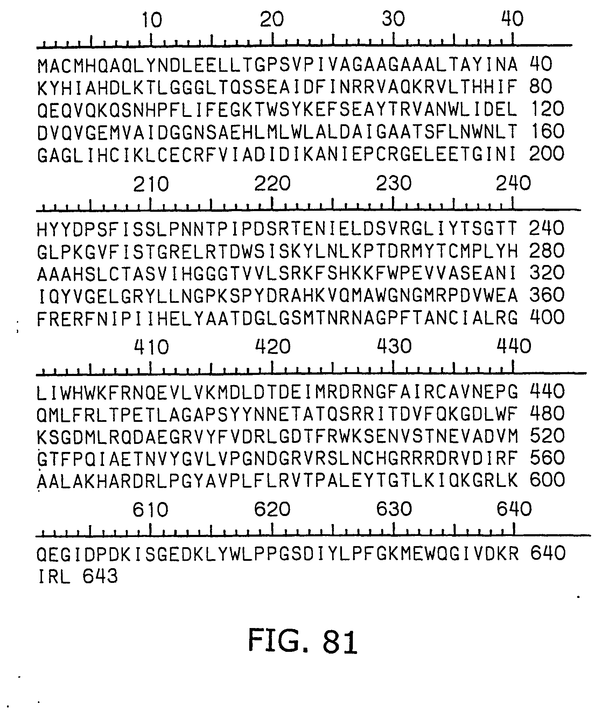

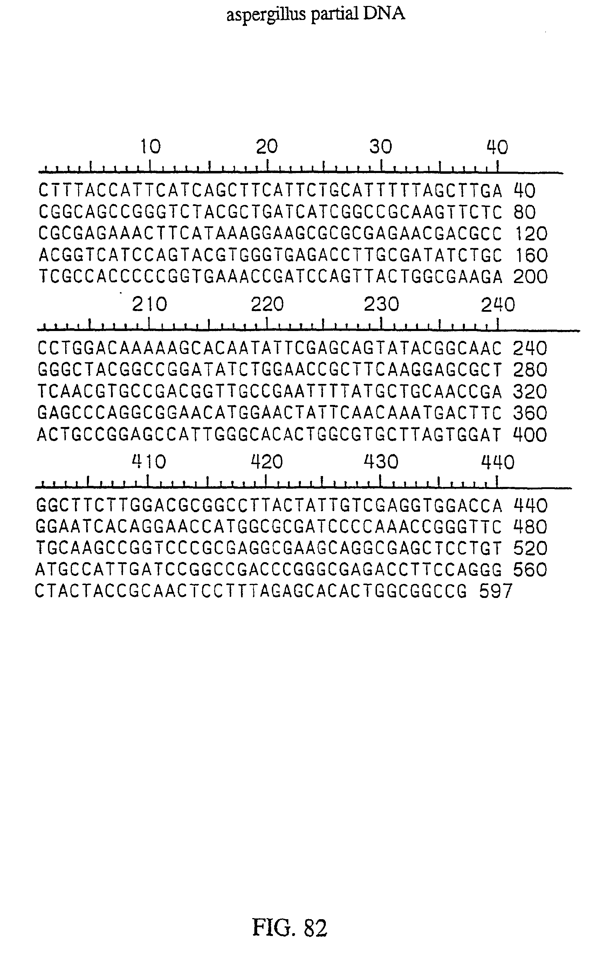

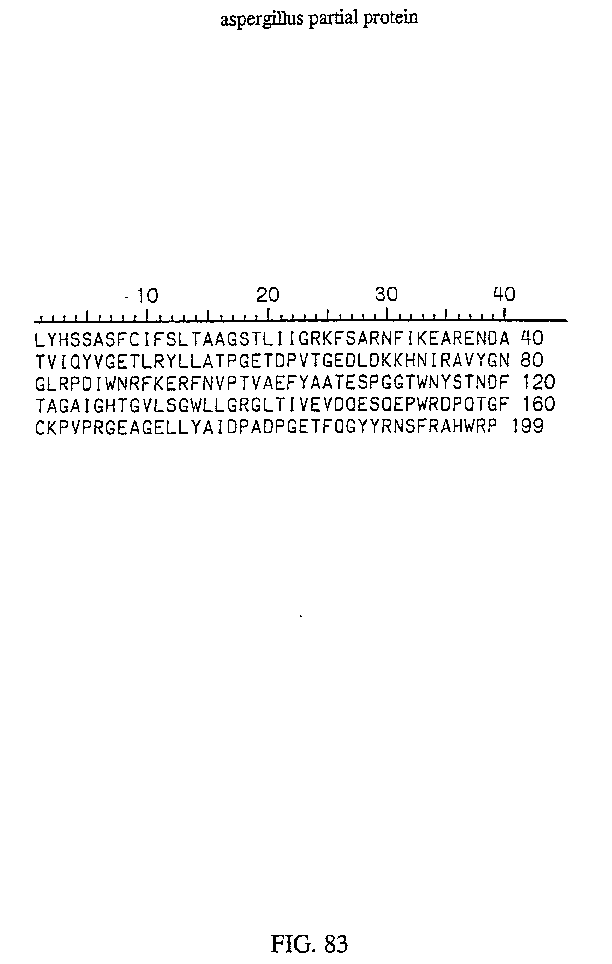

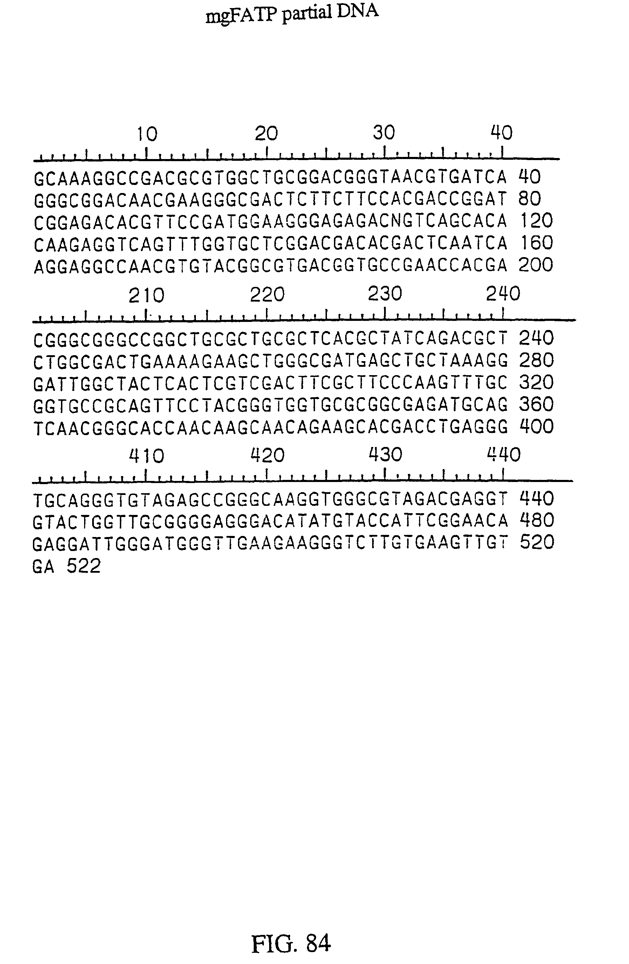

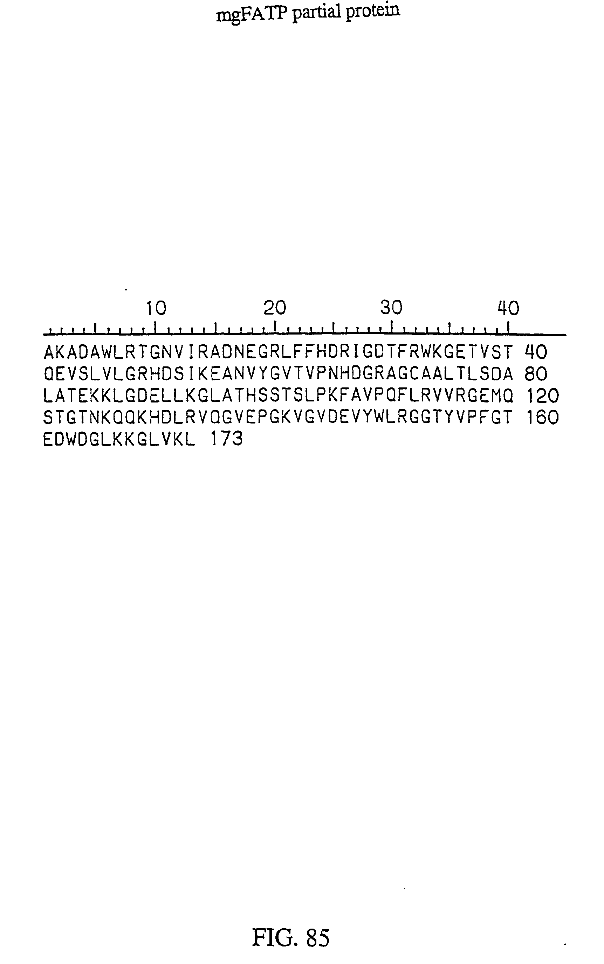

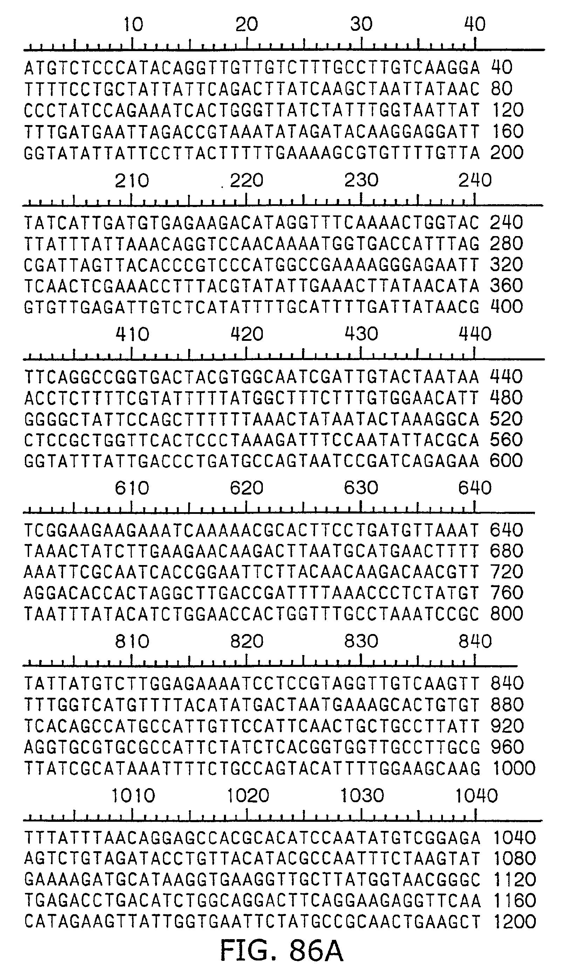

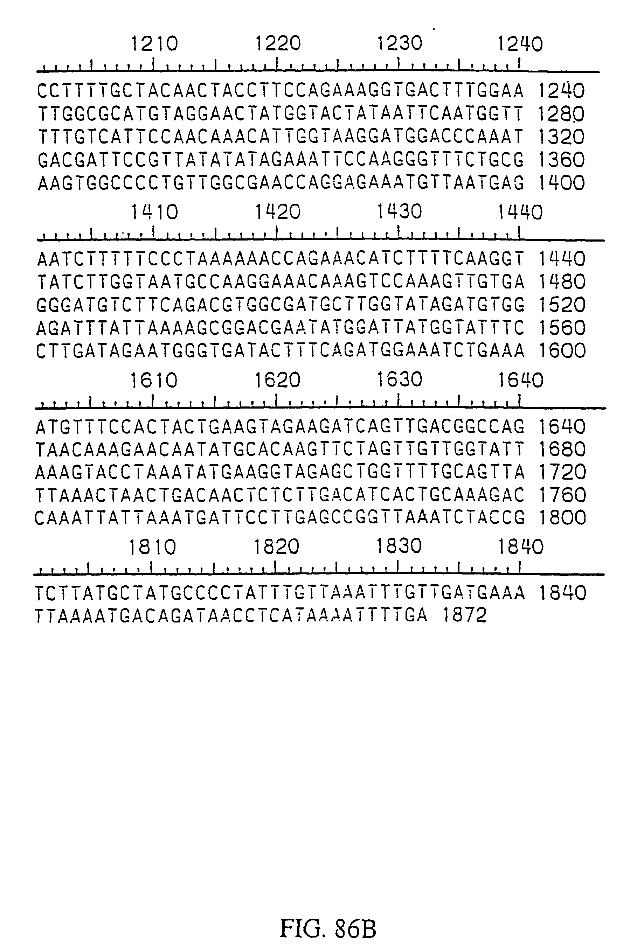

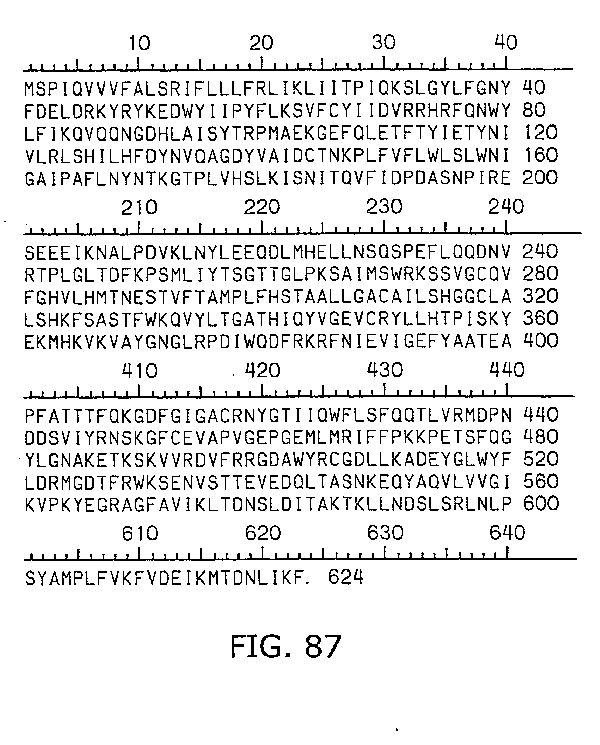

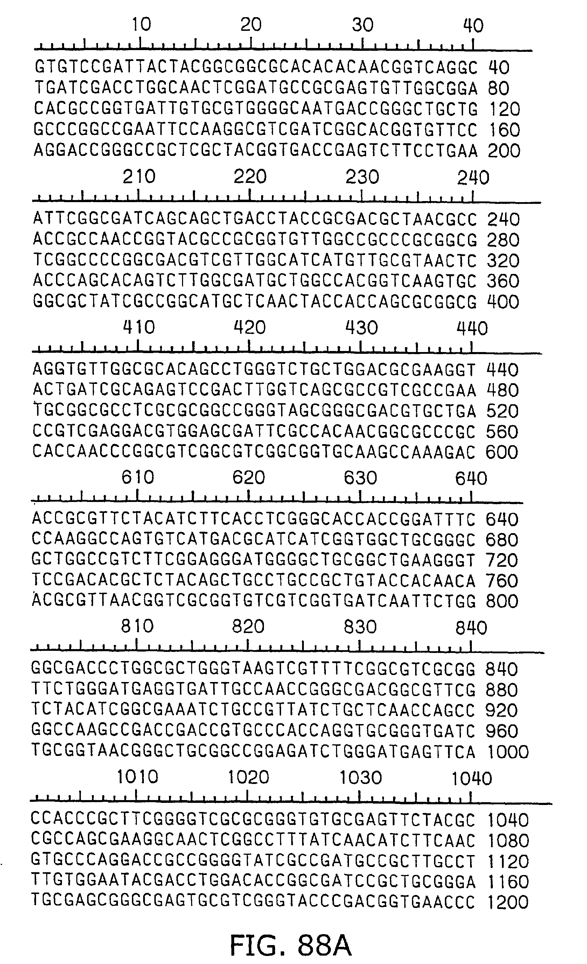

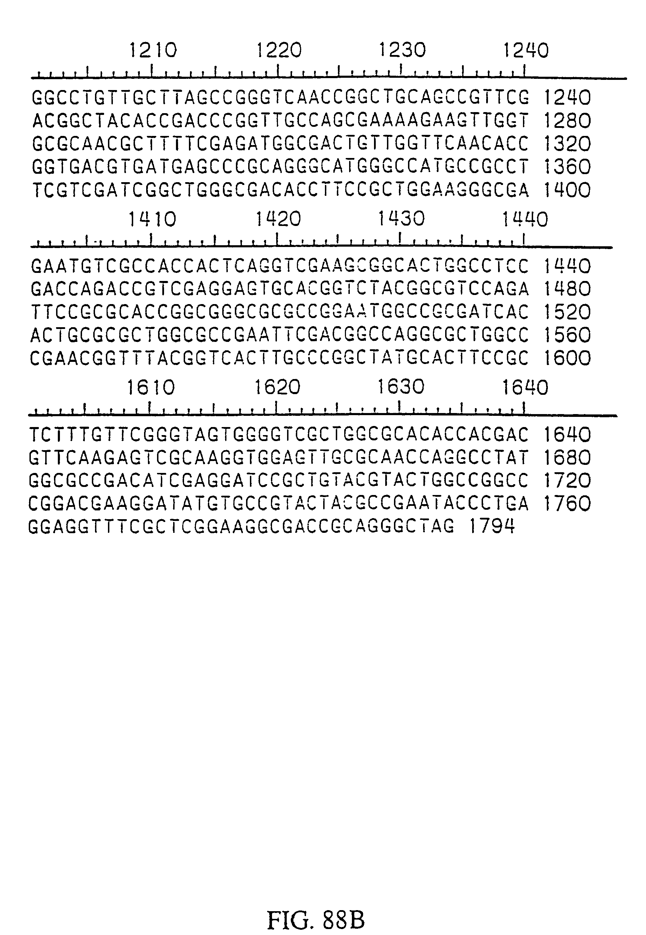

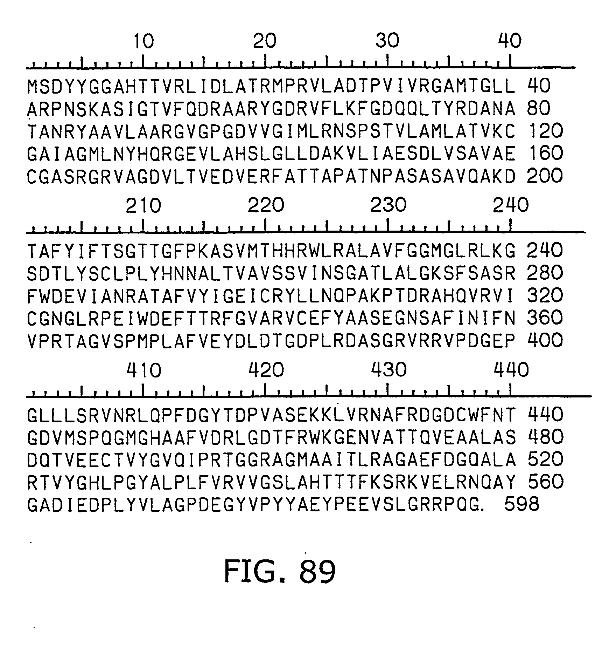

Ortholog polynucleotides can be isolated from (e.g., by cloning or nucleic acid amplification methods) a great number of species, as shown by the sample of FATPs from evolutionarily divergent species described herein (see, e.g., Figures 44A-C through Figure 89). Ortholog polynucleotides corresponding to those in Figure 45 (SEQ ID NO:47), Figure 47 (SEQ ID NO:49), Figure 49 (SEQ ID NO:51), Figure 51 (SEQ ID NO:53), Figures 94A and 94B (SEQ ID N0:102) and Figure 55 (SEQ ID NO:57) are those which can be isolated from mammals such as rat, dog, chimpanzee, monkey, baboon, pig, rabbit and guinea pig, for example.

Further variants that are fragments of the nucleic acids described above may be used to synthesize full-length nucleic acids of the invention, such as by use as primers in a polymerase chain reaction. As used herein, the term primer refers to a single-stranded oligonucleotide which acts as a point of initiation of template-directed DNA synthesis under appropriate conditions (e.g., in the presence of four different nucleoside triphosphates and an agent for polymerization, such as DNA or RNA polymerase or reverse transcriptase) in an appropriate buffer and at a suitable temperature. The appropriate length of a primer depends on the intended use of the primer, but typically ranges from 15 to 30 nucleotides. Short primer molecules generally require cooler temperatures to form sufficiently stable hybrid complexes with the template. A primer need not reflect the exact sequence of the template, but must be sufficiently complementary to hybridize with a template. The term primer site refers to the area of the target DNA to which a primer hybridizes. The term primer pair refers to a set of primers including a 5' (upstream) primer that hybridizes with the 5' end of the DNA sequence to be amplified and a 3' (downstream) primer that hybridizes with the complement of the 3' end of the sequence to be amplified.

Also disclosed herein are nucleic acids that are at least 80% identical over their entire length to a nucleic acid described herein, for example a nucleic acid having the nucleotide sequence in Figures 44A-44C (SEQ ID NO:46), Figures 46A-46B (SEQ ID NO:48), Figure 48 (SEQ ID NO:50), Figures 50A-50C (SEQ ID NO:52), Figures 94A and 94B (SEQ ID NO:101), and Figures 54A-54C (SEQ ID NO:56). Additional embodiments are nucleic acids, and the complements of such nucleic acids, having at least 90% nucleotide sequence identity to the above-described sequences, and nucleic acids having at least 95% nucleotide sequence identity. In preferred embodiments, DNA of the present invention has 97% nucleotide sequence identity, 98% nucleotide sequence identity, or at least 99% nucleotide sequence identity with the DNA whose sequences are presented herein.

Also disclosed herein are nucleic acids that are at least 80% identical in nucleotide sequence to a nucleic acid encoding a polypeptide having an amino acid sequence as set forth in Figure 45 (SEQ ID NO:47), Figure 47 (SEQ ID NO:49), Figure 49 (SEQ ID NO:51), Figure 51 (SEQ ID NO:53), Figures 94A and 94B (SEQ ID NO:102) or Figure 55 (SEQ ID NO:57), or as such amino acid sequences are set forth elsewhere herein, and nucleic acids that are complementary to such nucleic acids. Specifically, nucleic acids having at least 90% nucleotide sequence identity to a nucleic acid encoding a polypeptide having an amino acid sequence as described in the list above, nucleic acids having at least 95% sequence identity, and nucleic acids having at least 97% sequence identity are disclosed herein.

The terms "complementary" or "complementarity" as used herein, refer to the natural binding of polynucleotides under permissive salt and temperature conditions by base-pairing. Complementarity between two single-stranded molecules may be "partial" in which only some of the nucleic acids bind, or it may be complete when total complementarity exists between the single-stranded molecules (that is, when A-T and G-C base pairing is 100% complete). The degree of complementarity between nucleic acid strands has significant effects on the efficiency and strength of hybridization between nucleic acid strands. This is of particular importance in amplification reactions, which depend on binding between nucleic acid strands.

Also disclosed herein are nucleic acids that hybridize to the above-described nucleic acids, especially those nucleic acids that hybridize under stringent hybridization conditions. "Stringent hybridization conditions" or "high stringency conditions" generally occur within a range from about Tₘ minus 5°C (5° C below the strand dissociation temperature or melting temperature (Tₘ ) of the probe nucleic acid molecule) to about 20° C to 25° C below Tₘ. As will be understood by those of skill in the art, the stringency of hybridization may be altered in order to identify or detect molecules having identical or related polynucleotide sequences. An example of high stringency hybridization follows. Hybridization solution is (6x SSC/10 mM EDTA/0.5% SDS/5x Denhardt's solution/100 µg/ml sheared and denatured salmon sperm DNA). Hybridization is at 64-65°C for 16 hours. The hybridized blot is washed two times with 2x SSC/0.5% SDS solution at room temperature for 15 minutes each, and two times with 0.2x SSC/0.5% SDS at 65 °C, for one hour each. Further examples of high stringency conditions can be found on pages 2.10.1-2.10.16 (see particularly 2.10.8-11) and pages 6.3.1-6 in *Current Protocols in Molecular Biology* (Ausubel, F.M. *et al.,* eds., containing supplements up through Supplement 42, 1998). Examples of high, medium, and low stringency conditions can be found on pages 36 and 37 of WO 98/40404.

Also disclosed herein are nucleic acids obtainable by screening an appropriate library with a probe having a nucleotide sequence such as that set forth in Figures 44A-44C (SEQ ID NO:46), Figures 46A-46B (SEQ ID NO:48), Figure 48 (SEQ ID NO:50), Figures 50A-50C (SEQ ID NO:52), Figures 94A and 94B (SEQ ID NO:101) or Figures 54A-54C (SEQ ID NO:56), or a probe which is a sufficiently long fragment of any of the above; and isolating the nucleic acid. Such probes generally can comprise at least 15 nucleotides. Nucleic acids obtainable by such screenings may include RNAs, cDNAs and genomic DNA, for example, encoding FATPs of the FATP family described herein.

Further uses for the nucleic acid molecules disclosed herein whether encoding a full-length FATP or whether comprising a contiguous portion of a nucleic acid molecule such as one given in SEQ ID NO:46,48, 50, 52, 101, or 56, include use as markers for tissues in which the corresponding protein is preferentially expressed (to identify constitutively expressed proteins or proteins produced at a particular stage of tissue differentiation or stage of development of a disease state); as molecular weight markers on southern gels; as chromosome markers or tags (when labeled, for example with biotin, a radioactive label or a fluorescent label) to identify chromosomes or to map related gene positions; to compare with endogenous DNA sequences in a mammal to identify potential genetic disorders; as probes to hybridize and thus identify, related DNA sequences; as a source of information to derive PCR primers for genetic fingerprinting; as a probe to "subtract-out" known sequences in the process of discovering other novel nucleic acid molecules; for selecting and making oligomers for attachment to a "gene chip" or other support, to be used, for example, for examination of expression patterns; to raise anti-protein antibodies using DNA immunization techniques; and as an antigen to raise anti-DNA antibodies or to elicit another immune response.

Further methods to obtain nucleic acids encoding FATPs of the FATP family include PCR and variations thereof (e.g., "RACE" PCR and semi-specific PCR methods). Portions of the nucleic acids having a nucleotide sequence set forth in Figures 44A-44C (SEQ ID NO:46), Figures 46A-46B (SEQ ID NO:48), Figure 48 (SEQ ID NO:50), Figures 50A-50C (SEQ ID NO:52), Figures 94A and 94B (SEQ ID NO:101) or Figures 54A-54C (SEQ ID NO:56), (especially "flanking sequences" on either side of a coding region) can be used as primers in methods using the polymerase chain reaction, to produce DNA from an appropriate template nucleic acid.

Once a fragment of the FATP gene is generated by PCR, it can be sequenced, and the sequence of the product can be compared to other DNA sequences, for example, by using the BLAST Network Service at the National Center for Biotechnology Information. The boundaries of the open reading frame can then be identified using semi-specific PCR or other suitable methods such as library screening. Once the 5' initiator methionine codon and the 3' stop codon have been identified, a PCR product encoding the full-length gene can be generated using genomic DNA as a template, with primers complementary to the extreme 5' and 3' ends of the gene or to their flanking sequences. The full-length genes can then be cloned into expression vectors for the production of functional proteins.

There are also disclosed herein isolated proteins or polypeptides such as those encoded by nucleic acids disclosed herein. Isolated proteins can be purified from a natural source or can be made recombinantly. Proteins or polypeptides referred to herein as "isolated" are proteins or polypeptides that exist in a state different from the state in which they exist in cells in which they are normally expressed in an organism, and include proteins or polypeptides obtained by methods described herein, similar methods or other suitable methods, and also include essentially pure proteins or polypeptides, proteins or polypeptides produced by chemical synthesis or by combinations of biological and chemical methods, and recombinant proteins or polypeptides which are isolated. Thus, the term "isolated" as used herein, indicates that the polypeptide in question exists in a physical milieu distinct from that in which it occurs in nature. Thus, "isolated" includes existing in membrane fragments and vesicles membrane fractions, liposomes, lipid bilayers and other artificial membrane systems. An isolated FATP may be substantially isolated with respect to the complex cellular milieu in which it naturally occurs, and may even be purified essentially to homogeneity, for example as determined by PAGE or column chromatography (for example, HPLC), but may also have further cofactors or molecular stabilizers, such as detergents, added to the purified protein to enhance activity. In one embodiment, proteins or polypeptides are isolated to a state at least about 75% pure; more preferably at least about 85% pure, and still more preferably at least about 95% pure, as determined by Coomassie blue staining of proteins on SDS-polyacrylamide gels. Proteins or polypeptides referred to herein as "recombinant" are proteins or polypeptides produced by the expression of recombinant nucleic acids.

There is disclosed herein an isolated polypeptide comprising a FATP, a functional portion thereof, or a functional equivalent of the FATP, has at least one function characteristic of a FATP, for example, transport activity, binding function (e.g., a domain which binds to AMP), or antigenic function (e.g., binding of antibodies that also bind to a naturally-occurring FATP, as that function is found in an antigenic determinant). Functional equivalents can have activities that are quantitatively similar to, greater than, or less than, the reference protein. These proteins include, for example, naturally occurring FATPs that can be purified from tissues in which they are produced (including polymorphic or allelic variants), variants (e.g., mutants) of those proteins and/or portions thereof. Such variants include mutants differing by the addition, deletion or substitution of one or more amino acid residues, or modified polypeptides in which one or more residues are modified, and mutants comprising one or more modified residues. Portions or fragments of a FATP can range in size from four amino acid residues to the entire amino acid sequence minus one amino acid.

The isolated proteins disclosed herein preferably include mammalian fatty acid transport proteins of the FATP family of homologous proteins. The extent of amino acid sequence similarity between a polypeptide having one of the amino acid sequences shown in Figure 45 (SEQ ID NO:47), Figure 47 (SEQ ID NO:49), Figure 49 (SEQ ID NO:51), Figure 51 (SEQ ID NO:53), Figures 94A and 94B (SEQ ID NO: 102), or Figure 55 (SEQ ID NO:57), and the respective functional equivalents of these polypeptides may be at least about 88%. Alternatively, the degree of amino acid sequence similarity between a FATP and its respective functional equivalent may be at least about 91 %, at least about 94%, or at least about 97%.

The polypeptides disclosed herein also include those FATPs encoded by polynucleotides which are orthologous to those polynucleotides, the sequences of which are described herein in whole or in part. FATPs which are orthologs to those described herein by amino acid sequence, in whole or in part, are, for example fatty acid transport proteins 1-6 of dog, rat chimpanzee, monkey, rabbit, guinea pig, baboon and pig, and are also embodiments of the invention.

To determine the percent identity or similarity of two amino acid sequences or of two nucleic acid sequences, the sequences are aligned for optimal comparison purposes (e.g., gaps can be introduced in one or both of a first and a second amino acid or nucleic acid sequence for optimal alignment, and non-homologous (dissimilar) sequences can be disregarded for comparison purposes). The length of a reference sequence aligned for comparison purposes may be at least 30%, preferably at least 40%, more preferably at least 50%, even more preferably at least 60%, and even more preferably at least 70%, 80%, or 90% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein, amino acid or nucleic acid "identity" is equivalent to amino acid or nucleic acid "similarity"). The percent identity between the two sequences is a function of the number of identical positions shared by the sequences, taking into account the number of gaps, and the length of each gap, which need to be introduced for optimal alignment of the two sequences.

Also disclosed are polypeptides having a lower degree of identity but having sufficient similarity so as to perform one or more of the same functions performed by the polypeptides described herein by amino acid sequence. Similarity for a polypeptide is determined by conserved amino acid substitution. Such substitutions are those that substitute a given amino acid in a polypeptide by another amino acid of like characteristics. Conservative substitutions are likely to be phenotypically silent. Typically seen as conservative substitutions are the replacements, one for another, among the aliphatic amino acids Ala, Val, Leu, and Ile; interchange of the hydroxyl residues Ser and Thr, exchange of the acidic residues Asp and Glu, substitution between the amide residues Asn and Gin, exchange of the basic residues Lys and Arg and replacements among the aromatic residues Phe, Tyr, Guidance concerning which amino acid changes are likely to be phenotypically silent is found in Bowie *et al., Science* 247:1306-1310 (1990).

**TABLE 1. Conservative Amino Acid Substitutions**

| | | | |
|---|---|---|---|
| Aromatic | | Phenylalanine | |
| | | Tryptophan | |
| | | Tyrosine | |
| Hydrophobic | | Leucine | |
| | | Isoleucine | |
| | | Valine | |
| Polar | | Glutamine | |
| | | Asparagine | |
| Basic | | Arginine | |
| | | Lysine | |
| | | Histidine | |
| Acidic | | Aspartic Acid | |
| | | Glutamic Acid | |
| Small | | Alanine | |
| | | Serine | |
| | | Threonine | |
| | | Methionine | |
| | | Glycine | |

The comparison of sequences and determination of percent identity and similarity between two sequences can be accomplished using a mathematical algorithm. (*Computational Molecular Biology,* Lesk, A.M.,ed., Oxford University Press, New York, 1988; *Biocomputing: Informatics and Genome Projects,* Smith, D.W., ed., Academic Press, New York, 1993; *Computer Analysis of Sequence Data, Part I,* Griffin, A.M., and Griffin, H.G., eds., Humana Press, New Jersey, 1994; *Sequenc*e *Analysis in Molecular Biology,* von Heinje, G., Academic Press, 1987; and *Sequence. Analysis Primer,* Gribskov, M. and Devereaux, J., eds., M. Stockton Press, New York, 1991). The percent identity between two amino acid sequences may be determined using the Needleman and Wunsch (*J. Mol. Biol.* (48):444-453 (1970)) algorithm which has been incorporated into the GAP program in the GCG software package (available at http://www.gcg.com), using either a Blossom 62 matrix or a PAM250 matrix, and a gap weight of 16, 14, 12, 10, 8, 6, or 4 and a length weight of 1, 2, 3, 4, 5, or 6. The percent identity between two nucleotide sequences may alternatively be determined using the GAP program in the GCG software package (Devereux, J., *et al., Nucleic Acids Res. 12(1)*:387 (1984)) (available at http://www.gcg.com), using a NWSgapdna.CMP matrix and a gap weight of 40, 50, 60, 70, or 80 and a length weight of 1, 2, 3, 4, 5, or 6. Alternatively, the percent identity between two amino acid or nucleotide sequences is determined using the algorithm of E. Meyers and W. Miller (*CABIOS,* 4:11-17 (1989)) which has been incorporated into the ALIGN program (version 2.0), using a PAM120 weight residue table, a gap length penalty of 12 and a gap penalty of 4.

The nucleic acid and protein sequences of the present invention can further be used as a "query sequence" to perform a search against databases to, for example, identify other family members or related sequences. Such searches can be performed using the NBLAST and XBLAST programs (version 2.0) of Altschul, *et al.* (*J. Mol. Biol.* 215:403-10 (1990)). BLAST nucleotide searches can be performed with the NBLAST program, score = 100, word length = 12 to obtain nucleotide sequences homologous to (with calculatably significant similarity to) the nucleic acid molecules of the invention. BLAST protein searches can be performed with the XBLAST program, score = 50, word length = 3 to obtain amino acid sequences homologous to the proteins of the invention. To obtain gapped alignments for comparison purposes, Gapped BLAST can be utilized as described in Altschul *et al., (Nucleic Acids Res. 25*(17):3389-3402 (1997)). When utilizing BLAST and gapped BLAST programs, the default parameters of the respective programs (e.g., XBLAST and NBLAST) can be used. See http://www.ncbi.nlm.nih.gov.

Similarity for nucleotide and amino acid sequences can be defined in terms of the parameters set by the Advanced Blast search available from NCBI (the National Center for Biotechnology Information; see, for Advanced BLAST page, www.ncbi.nlm.nih.gov/cgi-bin/BLAST/nph-newblast?Jform=1). These default parameters, recommended for a query molecule of length greater than 85 amino acid residues or nucleotides have been set as follows: gap existence cost, 11, per residue gap cost, 1; lambda ratio, 0.85. Further explanation of version 2.0 of BLAST can be found on related websitc pages and in Altschul, S.F. *et al., Nucleic Acids Res. 25:*3389-3402 (1997).

There are also disclosed herein fusion proteins, comprising a FATP or functional portion thereof (as described above) as a first moiety, linked to second moiety not occurring in the FATP as found in nature. Thus, the second moiety can be an amino acid, peptide or polypeptide. The first moiety can be in an N-terminal location, C-terminal location or internal to the fusion protein. The fusion protein may comprise a FATP as the first moiety, and a second moiety comprising a linker sequence and an affinity ligand. Fusion proteins can be produced by a variety of methods. For example, a fusion protein can be produced by the insertion of a FATP gene or portion thereof into a suitable expression vector, such as Bluescript SK +/(Stratagene), pGEX-4T-2 (Pharmacia), pET-24(+) (Novagen), or vectors of similar construction. The resulting construct can be introduced into a suitable host cell for expression. Upon expression, fusion protein can be purified from cells by means of a suitable affinity matrix (See e.g., *Current Protocols in Molecular Biology,* Ausubel, F.M. *et al.,* eds., Vol. 2, pp. 16.4.1-16.7.8, containing supplements up through Supplement 42, 1998).

Also disclosed are enzymatically produced, synthetically produced, or recombinantly produced portions of a fatty acid transport protein. Portions of a FATP can be made which have full or partial function on their own, or which when mixed together (though fully, partially, or nonfunctional alone), spontaneously assemble with one or more other polypeptides to reconstitute a functional protein having at least one function characteristic of a FATP.

Fragments of a FATP can be produced by direct peptide synthesis, for example those using solid-phase techniques (Roberge, J.Y. *et al., Science 269*:202-204 (1995); Merrifield, J., *J*. *Am. Chem. Soc. 85*:2149-2154 (1963)). Protein synthesis can be performed using manual techniques or by automation. Automated synthesis can be carried out using, for instance, an Applied Biosystems 431 A Peptide Synthesizer (Perkin Ehner). Various fragments of a FATP can be synthesized separately and combined using chemical methods.

Also disclosed herein is a peptide or polypeptide having the amino acid sequence of a portion of a fatty acid transport protein which is hydrophilic rather than hydrophobic, and ordinarily can be detected as facing the outside of the cell membrane. Such a peptide or polypeptide can be thought of as being an extracellular domain of the FATP, or a mimetic of said extracellular domain. It is known, for example, that a portion of human FATP4 that includes a highly conserved motif is involved in AMP-CoA binding function (Stuhlsatz-Krouper, S.M. *et al., J.Biol. Chem. 44*:28642-28650 (1998)).

The term "mimetic" as used herein, refers to a molecule, the structure of which is developed from knowledge of the structure of the FATP of interest, or one or more portions thereof, and, as such, is able to effect some or all of the functions of a FATP.

Portions of an FATP can be prepared by enzymatic cleavage of the isolated protein, or can be made by chemical synthesis methods. Portions of a FATP can also be made by recombinant DNA methods in which restriction fragments, or fragments that may have undergone further enzymatic processing, or synthetically made DNAs are joined together to construct an altered FATP gene. The gene can be made such that it encodes one or more desired portions of a FATP. These portions of FATP can be entirely homologous to a known FATP, or can be altered in amino acid sequence relative to naturally occurring FATPs to enhance or introduce desired properties such as solubility, stability, or affinity to a ligand. A further feature of the gene can be a sequence encoding an N-terminal signal peptide directed to the plasma membrane.

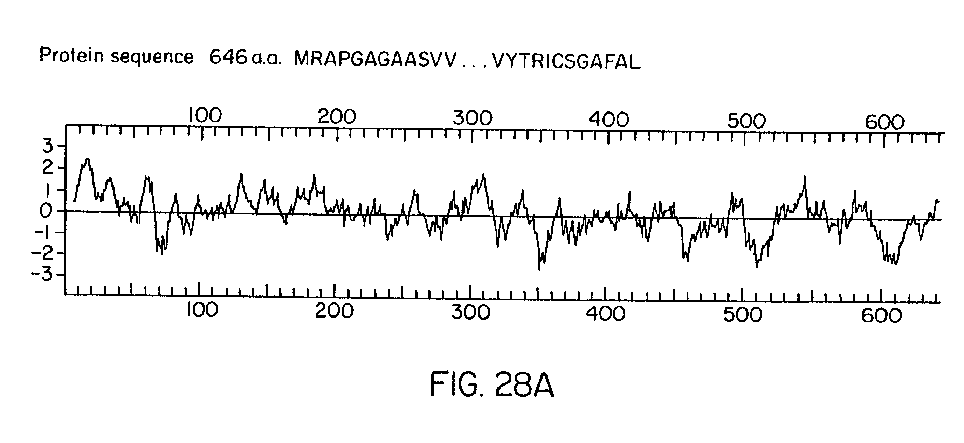

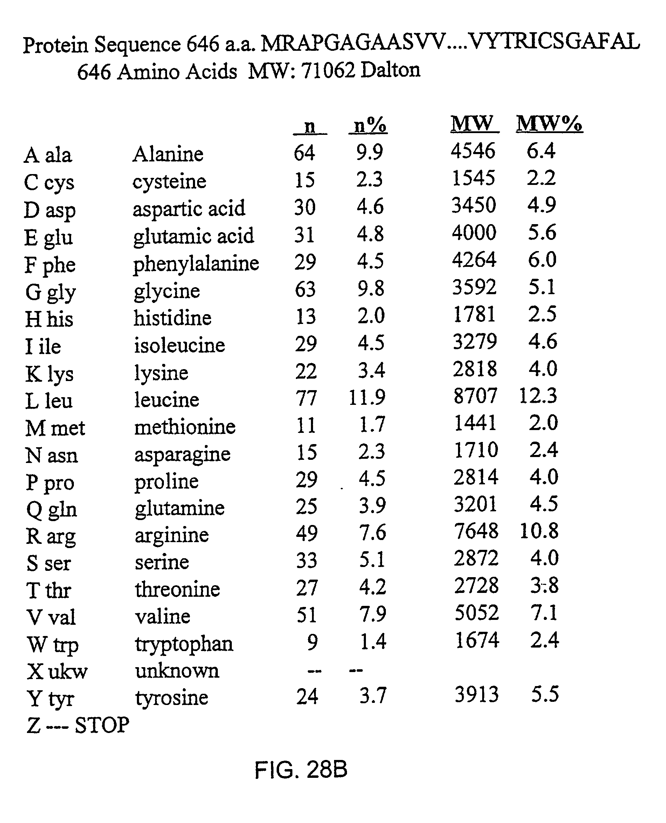

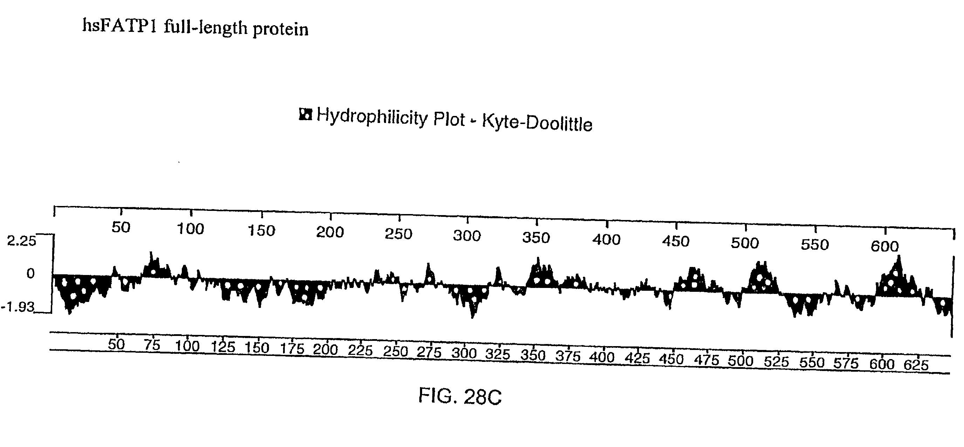

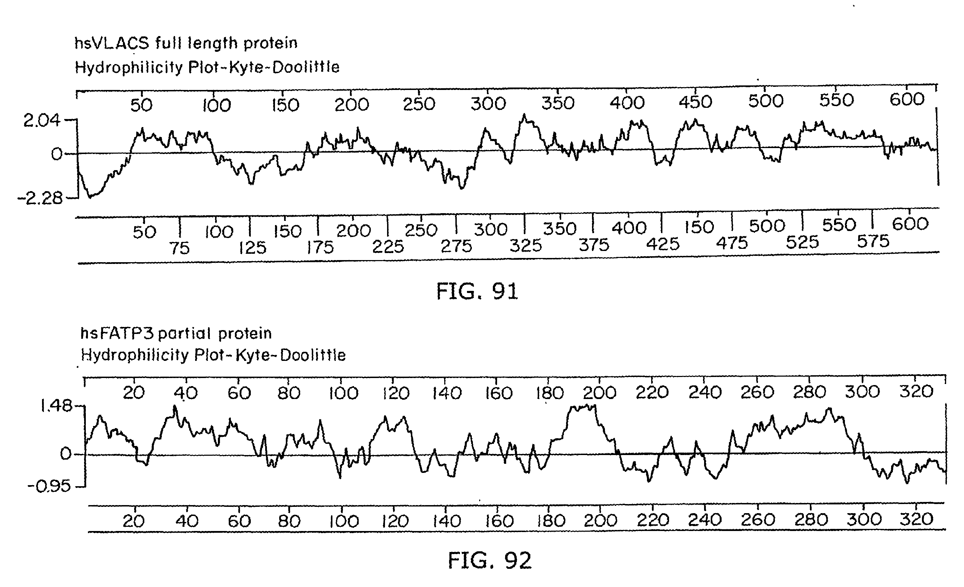

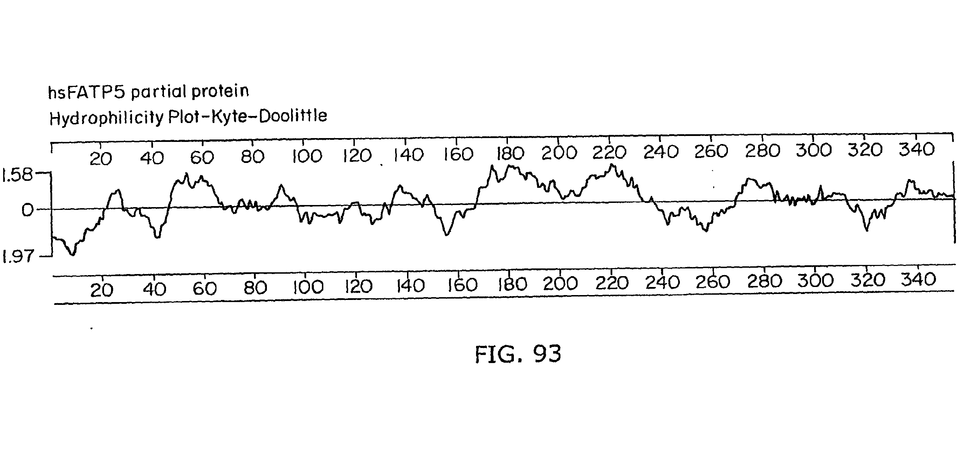

An extracellular domain can be determined by a hydrophobicity plot, such as those shown in Figures 28A, 29A, and 35A, or by a hydrophilicity plot such as those shown in Figures 28C, 29C, 35C, 91, 92 and 93. A polypeptide or peptide comprising all or a portion of a FATP extracellular domain can be used in a pharmaceutical composition. When administered to a mammal by an appropriate route, the polypeptide or peptide can bind to fatty acids and compete with the native FATPs in the membrane of cells, thereby making fewer fatty acid molecules available as substrates for transport into cells, and reducing the amount of fatty acids taken up by, for example, the heart, in the case of FATP6.

Also disclosed herein is a method of producing a fatty acid transport protein, variants or portions thereof, and to expression systems and host cells containing a vector appropriate for expression of a fatty acid transport protein.

Cells that express a FATP, a variant or a portion thereof, or an ortholog of a FATP described herein by amino acid sequence, can be made and maintained in culture, under conditions suitable for expression, to produce protein in the cells for cell-based assays, or to produce protein for isolation. These cells can be procaryotic or eucaryotic. Examples of procaryotic cells that can be used for expression include *Escherichia coli, Bacillus subtilis* and other bacteria. Examples of eucaryotic cells that can be used for expression include yeasts such as *Saccharomyces cerevisiae, Schizosaccharomyces pombe, Pichia pastoris* and other lower eucaryotic cells, and cells of higher eucaryotes such as those from insects and mammals, such as primary cells and cell lines such as CHO, HeLa, 3T3 and BHK cells, preferably COS cells and human kidney 293 cells, and more preferably Jurkat cells. (See, e.g., Ausubel, F.M. *et al.,* eds. *Current Protocols in Molecular Biology,* Greene Publishing Associates and John Wiley & Sons, Inc., containing Supplements up through Supplement 42,1998)).

Host cells that produce a recombinant FATP, or a portion thereof, a variant, or an ortholog of a FATP described herein by amino acid sequence, can be made as follows. A gene encoding a FATP, variant or a portion thereof can be inserted into a nucleic acid vector, e.g., a DNA vector, such as a plasmid, phage, cosmid, phagemid, virus, virus-derived vector (e.g., SV40, vaccinia, adenovirus, fowl pox virus, pseudorabies viruses, retroviruses) or other suitable replicon, which can be present in a single copy or multiple copies, or the gene can be integrated in a host cell chromosome. A suitable replicon or integrated gene can contain all or part of the coding sequence for a FATP or variant, operably linked to one or more expression control regions whereby the coding sequence is under the control of transcription signals and linked to appropriate translation signals to permit translation. The vector can be introduced into cells by a method appropriate to the type of host cells (e.g., transfection, electroporation, infection). For expression from the FATP gene, the host cells can be maintained under appropriate conditions (e.g., in the presence of inducer, normal growth conditions, etc.). Proteins or polypeptides thus produced can be recovered (e.g., from the cells, as in a membrane fraction, from the periplasmic space of bacteria, from culture medium) using suitable techniques. Appropriate membrane targeting signals may be incorporated into the expressed polypeptide. These signals may be endogenous to the polypeptide or they may be heterologous signals.

Polypeptides disclosed herein can be recovered and purified from cell cultures (or from their primary cell source) by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and high performance liquid chromatography. Known methods for refolding protein can be used to regenerate active conformation if the polypeptide is denatured during isolation or purification.

Also disclosed herein are methods for assessing the transport function of any of the fatty acid transport proteins or polypeptides described herein, including orthologs, and in variations of these, methods for identifying an inhibitor (or an enhancer) of such function and methods for assessing the transport function in the presence of a candidate inhibitor or a known inhibitor.

A variety of systems comprising living cells can be used for these methods. Cells to be used in fatty acid transport assays, and further in methods for identifying an inhibitor or enhancer of this function, express one or more FATPs. See Examples 3, 6, 9,12 and 14 for data on tissue distribution of expression of FATPs, and Examples 10 and 11 describing recombinant cells expressing FATP. Cells for use in cell-based assays described herein can be drawn from a variety of sources, such as isolated primary cells of various organs and tissues wherein one or more FATPs are naturally expressed. In some cases, the cells can be from adult organs, and in some cases, from embryonic or fetal organs, such as heart, lung, liver, intestine, skeletal muscle, kidney and the like. Cells for this purpose can also include cells cultured as fragments of organs or in conditions simulating the cell type and/or tissue organization of organs, in which artificial materials may be used as substrates for cell growth. Other types of cells suitable for this purpose include cells of a cell strain or cell line (ordinarily comprising cells considered to be "transformed") transfected to express one or more FATPs.

Also disclosed is a method for detecting, in a sample of cells, a fatty acid transport protein, a portion or fragment thereof, a fusion protein comprising a FATP or a portion thereof, or an ortholog as described herein, wherein the cells can be, for instance, cells of a tissue, primary culture cells, or cells of a cell line, including cells into which nucleic acid has been introduced. The method comprises adding to the sample an agent that specifically binds to the protein, and detecting the agent specifically bound to the protein. Appropriate washing steps can be added to reduce nonspecific binding to the agent. The agent can be, for example, an antibody, a ligand or a substrate mimic. The agent can have incorporated into it, or have bound to it, covalently or by high affinity non-covalent interactions, for instance, a label that facilitates detection of the agent to which it is bound, wherein the label can be, but is not limited to, a phosphorescent label, a fluorescent label, a biotin or avidin label, or a radioactive label. The means of detection of a fatty acid transport protein can vary, as appropriate to the agent and label used. For example, for an antibody that binds to the fatty acid transport protein, the means of detection may call for binding a second antibody, which has been conjugated to an enzyme, to the antibody which binds the fatty acid transport protein, and detecting the presence of the second antibody by means of the enzymatic activity of the conjugated enzyme.

Similar principles can also be applied to a cell lysate or a more purified preparation of proteins from cells that may comprise a fatty acid transport protein of interest, for example in the methods of immunoprecipitation, immunoblotting, immunoaffinity methods, that in addition to detection of the particular FATP, can also be used in purification steps, and qualitative and quantitative immunoassays. See, for instance, chapters 11 through 14 in *Antibodies: A Laboratory Manual,* E. Harlow and D. Lane, eds., Cold Spring Harbor Laboratory, 1988.

Isolated fatty acid transport protein or, an antigenically similar portion thereof, especially a portion that is soluble, can be used in a method to select and identify molecules which bind specifically to the FATP. Fusion proteins comprising all of, or a portion of, the fatty acid transport protein linked to a second moiety not occurring in the FATP as found in nature, can be prepared for use in another embodiment of the method. Suitable fusion proteins for this purpose include those in which the second moiety comprises an affinity ligand (e.g., an enzyme, antigen, epitope). FATP fusion proteins can be produced by the insertion of a gene encoding the FATP or a variant thereof, or a suitable portion of such gene into a suitable expression vector, which encodes an affinity ligand (e.g., pGEX-4T-2 and pET-15b, encoding glutathione S-transferase and His-Tag affinity ligands, respectively). The expression vector can be introduced into a suitable host cell for expression. Host cells are lysed and the lysate, containing fusion protein, can be bound to a suitable affinity matrix by contacting the lysate with an affinity matrix.

The fusion protein can be immobilized on a suitable affinity matrix under conditions sufficient to bind the affinity ligand portion of the fusion protein to the matrix, and is contacted with one or more candidate binding agents (e.g., a mixture of peptides) to be tested, under conditions suitable for binding of the binding agents to the FATP portion of the bound fusion protein. Next, the affinity matrix with bound fusion protein can be washed with a suitable wash buffer to remove unbound candidate binding agents and non-specifically bound candidate binding agents. Those agents which remain bound can be released by contacting the affinity matrix with fusion protein bound thereto with a suitable elution buffer. Wash buffer can be formulated to permit binding of the fusion protein to the affinity matrix, without significantly disrupting binding of specifically bound binding agents. In this aspect, elution buffer can be formulated to permit retention of the fusion protein by the affinity matrix, but can be formulated to interfere with binding of the candidate binding agents to the target portion of the fusion protein. For example, a change in the ionic strength or pH of the elution buffer can lead to release of specifically bound agent, or the elution buffer can comprise a release component or components designed to disrupt binding of specifically bound agent to the target portion of the fusion protein.

Immobilization can be performed prior to, simultaneous with, or after, contacting the fusion protein with candidate binding agent, as appropriate. Various permutations of the method are possible, depending upon factors such as the candidate molecules tested, the affinity matrix-ligand pair selected, and elution buffer formulation. For example, after the wash step, fusion protein with binding agent molecules bound thereto can be e.luted from the affinity matrix with a suitable elution buffer (a matrix elution buffer, such as glutathione for a GST fusion). Where the fusion protein comprises a cleavable linker, such as a thrombin cleavage site, cleavage from the affinity ligand can release a portion of the fusion with the candidate agent bound thereto. Bound agent molecules can then be released from the fusion protein or its cleavage product by an appropriate method, such as extraction.

One or more candidate binding agents can be tested simultaneously. Where a mixture of candidate binding agents is tested, those found to bind by the foregoing processes can be separated (as appropriate) and identified by suitable methods (e.g., PCR, sequencing, chromatography). Large libraries of candidate binding agents (e.g., peptides, RNA oligonucleotides) produced by combinatorial chemical synthesis or by other methods can be tested (see e.g., Ohlmeyer, M.H.J. *et al., Proc. Natl. Acad. Sci. USA 90*:10922-10926 (1993) and DeWitt, *S.H. et al., Proc. Natl. Acad. Sci. USA 90*:6909-6913 (1993), relating to tagged compounds; see also Rutter, W.J. *et al.* U.S. Patent No. 5,010,175; Huebner, V.D. *et al.,* U.S. Patent No. 5,182,366; and Geysen, H.M., U.S. Patent No. 4,833,092). Random sequence RNA libraries (see Ellington, A.D. *et al., Nature 346:*818-822 (1990); Bock, *L.C. et al., Nature 355*:584-566 (1992); and Szostak, J.W., *Trends in Biochem. Sci. 17*:89-93 (March, 1992)) can also be screened according to the present method to select RNA molecules which bind to a target FATP or FATP fusion protein. Where binding agents selected from a combinatorial library by the present method carry unique tags, identification of individual biomolecules by chromatographic methods is possible. Where binding agents do not carry tags, chromatographic separation, followed by mass spectrometry to ascertain structure, can be used to identify binding agents selected by the method, for example.

There is also disclosed herein a method for identifying an agent which inhibits interaction between a fatty acid transport protein (e.g., one comprising the amino acid sequence in SEQ ID NO:47, SEQ ID NO:49, SEQ ID NO:51, SEQ ID NO:53, SEQ ID NO:102, or SEQ ID NO:57), and a ligand of said protein. The FATP can be one described by amino acid sequence herein, a portion or fragment thereof, a variant thereof, or an ortholog thereof, or a FATP fusion protein. Here, a ligand can be, for instance, a substrate, or a substrate mimic, an antibody, or a compound, such as a peptide, that binds with specificity to a site on the protein. The method comprises combining, not limited to a particular order, the fatty acid protein, the ligand of the protein, and a candidate agent to be assessed for its ability to inhibit interaction between the protein and the ligand, under conditions appropriate for interaction between the protein and the ligand (e.g., pH, salt, temperature conditions conducive to appropriate conformation and molecular interactions); determining the extent to which the protein and ligand interact; and comparing (1) the extent of protein-ligand interaction in the presence of candidate agent with (2) the extent of protein-ligand interaction in the absence of candidate agent, wherein if (1) is less than (2), then the candidate agent is one which inhibits interaction between the protein and the ligand.

The method can be facilitated, for example, by using an experimental system which employs a solid support (column chromatography matrix, wall of a plate, microtiter wells, column pore glass, pins to be submerged in a solution, beads, etc.) to which the protein can be attached. Accordingly, the protein can be fixed to a solid phase directly or indirectly, by a linker. The candidate agent to be tested is added under conditions conducive for interaction and binding to the protein. The ligand is added to the solid phase system under conditions appropriate for binding. Excess ligand is removed, as by a series of washes done under conditions that do not disrupt protein-ligand interactions. Detection of bound ligand can be facilitated by using a ligand that carries a label (e.g., fluorescent, chemiluminescent, radioactive). In a control experiment, protein and ligand are allowed to interact in the absence of any candidate agent, under conditions otherwise identical to those used for the "test" conditions where candidate inhibiting agent is present, and any washes used in the test conditions are also used in the control. The extent to which ligand binds to the protein in the presence of candidate agent is compared to the extent to which ligand binds to the protein in the absence of the candidate agent. If the extent to which interaction of the protein and the ligand occurs is less in the presence of the candidate agent than in the absence of the candidate agent, the candidate agent is an agent which inhibits interaction between the protein and the ligand of the protein.

There is also a disclosure that an inhibitor (or an enhancer) of a fatty acid transport protein can be identified. The method comprises steps which are, or are variations of the following: contacting the cells with fatty acid, wherein the fatty acid can be labeled for convenience of detection; contacting a first aliquot of the cells with an agent being tested as an inhibitor (or enhancer) of fatty acid uptake while maintaining a second aliquot of cells under the same conditions but without contact with the agent; and measuring (e.g., quantitating) fatty acid in the first and second aliquots of cells; wherein a lesser quantity of fatty acid in the first aliquot compared to that in the second aliquot is indicative that the agent is an inhibitor of fatty acid uptake by a fatty acid transport protein. A greater quantity of fatty acid in the first aliquot compared to that in the second aliquot is indicative that the agent is an enhancer of fatty acid uptake by a fatty acid transport protein.

Identifying an inhibitor or enhancer of fatty acid transport function may employ the above steps, but also employ additional steps preceding those given above: introducing into cells of a cell strain or cell line ("host cells" for the intended introduction of, or after the introduction of, a vector) a vector comprising a fatty acid transport protein gene, wherein expression of the gene can be regulatable or constitutive, and providing conditions to the host cells under which expression of the gene can occur.

The terms "contacting" and "combining" as used herein in the context of bringing molecules into close proximity to each other, can be accomplished by conventional means. For example, when referring to molecules that are soluble, contacting is achieved by adding the molecules together in a solution. "Contacting" can also be adding an agent to a test system, such as a vessel containing cells in tissue culture.

The term "inhibitor" or "antagonist", as used herein, refers to an agent which blocks, diminishes, inhibits, hinders, limits, decreases, reduces, restricts or interferes with fatty acid transport into the cytoplasm of a cell, or alternatively and additionally, prevents or impedes the cellular effects associated with fatty acid transport. The term "enhancer" or "agonist", as used herein, refers to an agent which augments, enhances, or increases fatty acid transport into the cytoplasm of a cell. An antagonist will decrease fatty acid concentration, fatty acid metabolism and byproduct levels in the cell, leading to phenotypic and molecular changes.

In order to produce a "host cell" type suitable for fatty acid uptake assays and for assays derived therefrom for identifying inhibitors or enhancers thereof, a nucleic acid vector can be constructed to comprise a gene encoding a fatty acid transport protein, for example, human FATP1, FATP2, FATP3, FATP4, FATP5, FATP6, a mutant or variant thereof, an ortholog of the human proteins, such as mouse orthologs or orthologs found in other mammals, or a FATP family protein of origin in an organism other than a mammal. The gene of the vector can be regulatable, such as by the placement of the gene under the control of an inducible or repressible promoter in the vector (e.g., inducible or repressible by a change in growth conditions of the host cell harboring the vector, such as addition of inducer, binding or functional removal of repressor from the cell millieu, or change in temperature) such that expression of the FATP gene can be turned on or initiated by causing a change in growth conditions, thereby causing the protein encoded by the gene to be produced, in host cells comprising the vector, as a plasma membrane protein. Alternatively, the FATP gene can be constitutively expressed.

A vector comprising an FATP gene, such as a vector described herein, can be introduced into host cells by a means appropriate to the vector and to the host cell type. For example, commonly used methods such as electroporation, transfection, for instance, transfection using CaCl₂, and transduction (as for a virus or bacteriophage) can be used. Host cells can be, for example, mammalian cells such as primary culture cells or cells of cell lines such as COS cells, 293 cells or Jurkat cells. Host cells can also be, in some cases, cells derived from insects, cells of insect cell lines, bacterial cells, such as *E*. *coli,* or yeast cells, such as *S*. *cerevisiae.* It is preferred that the fatty acid transport protein whose function is to be assessed, with or without a candidate inhibitor or enhancer, be produced in host cells whose ancestor cells originated in a species related to the species of origin of the FATP gene encoding the fatty acid transport protein. For example, it is preferable that tests of function or of inhibition or enhancement of a mammalian FATP be carried out in host mammalian cells producing the FATP, rather than bacterial cells or yeast cells.

Host cells comprising a vector comprising a regulatable FATP gene can be treated so as to allow expression of the FATP gene and production of the encoded protein (e.g., by contacting the cells with an inducer compound that effects transcription from an inducible promoter operably linked to the FATP gene).

The test agent (e.g., an agonist or antagonist) is added to the cells to be used in a fatty acid transport assay, in the presence or absence of test agent, under conditions suitable for production and/or maintenance of the expressed FATP in a conformation appropriate for association of the FATP with test agent and substrate. For example, conditions under which an agent is assessed, such as media and temperature requirements, can, initially, be similar to those necessary for transport of typical fatty acid substrates across the plasma membrane. One of ordinary skill in the art will know how to vary experimental conditions depending upon the biochemical nature of the test agent. The test agent can be added to the cells in the presence of fatty acid, or in the absence of fatty acid substrate, with the fatty acid substrate being added following the addition of the test agent. The concentration at which the test agent can be evaluated can be varied, as appropriate, to test for an increased effect with increasing concentrations.

Test agents to be assessed for their effects on fatty acid transport can be any chemical (element, molecule, compound), made synthetically, made by recombinant techniques or isolated from a natural source: For example, test agents can be peptides, polypeptides, peptoids, sugars, hormones, or nucleic acid molecules, such as antisense nucleic acid molecules. In addition, test agents can be small molecules or molecules of greater complexity made by combinatorial chemistry, for example, and compiled into libraries. These libraries can comprise, for example, alcohols, alkyl halides, amines, amides, esters, aldehydes, ethers and other classes of organic compounds. Test agents can also be natural or genetically engineered products isolated from lysates of cells, bacterial, animal or plant, or can be the cell lysates themselves. Presentation of test compounds to the test system can be in either an isolated form or as mixtures of compounds, especially in initial screening steps.

Thus, there is disclosed a method for identifying agents which alter fatty acid transport, the method comprising providing the test agent to the cell (wherein "cell" includes the plural, and can include cells of a cell strain, cell line or culture of primary cells or organ culture, for example), under conditions suitable for binding to its target, whether to the FATP itself or to another target on or in the cell, wherein the transformed cell comprises a FATP.

In greater detail, to test one or more agents or compounds (e.g., a mixture of compounds can conveniently be screened initially) for inhibition of the transport function of a fatty acid transport protein, the agent(s) can be contacted with the cells. The cells can be contacted with a labeled fatty acid. The fatty acid can be, for example, a known substrate of the fatty acid transport protein such as oleate or palmitate. The fatty acid can itself be labeled with a radioactive isotope, (e.g., ³H or ¹⁴C) or can have a radioactively labeled adduct attached. In other variations, the fatty acid can have chemically attached to it a fluorescent label, or a substrate for an enzyme occurring within the cells, wherein the substrate yields a detectable product, such as a highly colored or fluorescent product. Addition of candidate inhibitors and labeled substrate to the cells comprising fatty acid transport protein can be in either order or can be simultaneous.

A second aliquot of cells, which can be called "control" cells (a"first" aliquot of cells can be called "test" cells), is treated, if necessary (as in the case of transformed "host"cells), so as to allow expression of the FAT.P gene, and is contacted with the labeled substrate of the fatty acid transport protein. The second aliquot of cells is not contacted with one or more agents to be tested for inhibition of the transport function of the protein produced in the cells, but is otherwise kept under the same culture conditions as the first aliquot of cells.

In a further step of a method to identify inhibitors of a fatty acid transport protein, the labeled fatty acid is measured in the first and second aliquots of cells. A preliminary step of this measurement process can be to separate the external medium from the cells so as to be able to distinguish the labeled fatty acid external to the cells from that which has been transported inside the cells. This can be accomplished, for instance, by removing the cells from their growth container, centrifuging the cell suspension, removing the supernatant and performing one or more wash steps to extensively dilute the remaining medium which may contain labeled fatty acid. Detection, of the labeled fatty acid can be by a means appropriate to the label used. For example, for a radioactive label, detection can be by scintillation counting of appropriately prepared samples of cells (e.g., lysates or protein extracts); for a fluorescent label, by measuring fluorescence in the cells by appropriate instrumentation.

If a compound tested as a candidate inhibitor of transport function causes the test cells to have less labeled fatty acid detected in the cells than that detected in the control cells, then the compound is an inhibitor of the fatty acid transport protein. Procedures analogous to those above can be devised for identifying enhancers (agonists of FATPs) of fatty acid transport function wherein if the test cells contain more labeled fatty acid than that detected in the control cells, or if the fatty acid is taken up at a higher rate, then the compound being tested can be concluded to be an enhancer of the fatty acid transport protein.

Example 13 describes use of an assay of this type to identify an inhibitor of a FATP. In Example 13, an antisense oligonucleotide which specifically inhibits biosynthesis of mmFATP4 was demonstrated to inhibit fatty acid uptake into mouse enterocytes. Similarly, antisense oligonucleotides directed towards specifically inhibiting the biosynthesis of FATP6 in heart cells, FATP5 in liver cells, FATP3 in lung cells, and FATP2 in colon cells, can be demonstrated as examples of "test agents" that inhibit fatty acid transport.

Another assay to determine whether an agent is an inhibitor (or enhancer) of fatty acid transport employs animals, one or more of which are administered the agent, and one or more of which are maintained under similar conditions, but are not administered the agent. Both groups of animals are given fatty acids (e.g., orally, intravenously, by tube inserted into stomach or intestine), and the fatty acids taken up into a bodily fluid (e.g., serum) or into an organ or tissue of interest are measured from comparable samples taken from each group of animals. The fatty acids may carry a label (e.g., radioactive) to facilitate detection and quantitation of fatty acids taken up into the fluid or tissue being sampled. This type of assay can be used alone or can be used in addition to *in vitro* assays of a candidate inhibitor or enhancer.

An agent determined to be an inhibitor (or enhancer) of FATP function, such as fatty acid binding and/or fatty acid uptake, can be administered to cells in culture, or *in vivo,* to a mammal (e.g. human) to inhibit (or enhance) FATP function. Such an agent may be one that acts directly on the FATP (for example, by binding) or can act on an intermediate in a biosynthetic pathway to produce FATP, such as transcription of the FATP gene, processing of the mRNA, or translation of the mRNA. An example of such an agent is antisense oligonucleotide.

Antisense methods similar to those illustrated in Example 13 can be used to determine the target FATP of a compound or agent that has an inhibitory or enhancing effect on fatty acid uptake. For example, antisense oligonucleotide directed to the inhibition of FATP4 biosynthesis can be added to lung cells or cell lines derived from lung cells. In addition, antisense oligonucleotides directed to the inhibition of other FATPs, except for FATP3, can also be added to the lung cells. The administration of antisense oligonucleotides in this manner ensures that the predominant FATP activity remaining in the cells comes from FATP3. After a period of incubation of the cells with the antisense oligonucleotides sufficient to deplete the plasma membrane of the FATPs whose biosynthesis has been inhibited, a test agent, preferably one that has been shown by some preliminary test to have an inhibitory or enhancing activity on fatty acid transport, can be added to the lung cells. If the test agent is now demonstrated, after treatment of the cells with antisense oligonucleotides, to have an inhibitory or enhancing activity on fatty acid transport in the lung cells, it can be concluded that the target of the test agent is FATP3, or a molecule involved in the biosynthesis or activity of FATP3.

In another type of cell-based assay for uptake of fatty acids, a change of intracellular pH resulting from the uptake of fatty acids can be followed by an indicator fluorophore. The fluorophore can be taken up by the cells in a preincubation step. Fatty acids can be added to the cell medium, and after some period of incubation to allow FATP-mediated uptake of fatty acids, the change in λₘₐₓ of fluorescence can be measured, as an indicator of a change in intracellular pH, as the λₘₐₓ of fluorescence of the fluorophore changes with the pH of its environment, thereby indicating uptake of fatty acids. One such fluorophore is BCECF (2', 7'-bis(2-carboxyethyl)-5(6)-carboxyfluorescein; Rink, T.J. *et al., J. Cell. Biol. 95*: 189 (1982)).

In assays similar to those described above, a candidate inhibitor or enhancer of fatty acid transport function can be added (or mock-added, for control cultures) to cultures of cells engineered to express a desired FATP to which fatty acid substrate is also added. Inhibition of fatty acid uptake is indicated by a lack of the drop in pH, indicating fatty acid uptake, that is seen in control cells. Enhancement of fatty acid uptake is indicated by a decrease in intracellular pH, as compared to control cells not receiving the candidate enhancer of fatty acid transport Function.

Yeast cells can be used in a similar cell-based assay for the uptake of fatty acids mediated by a FATP, and such an assay can be adapted to a screening assay for the identification of agents that inhibit or enhance fatty acid uptake by an FATP. Yeast cells lacking an endogenous FATP activity (mutated, disrupted or deleted for *FAT1;* Faergeman, N.J. *et al., J. Biol. Chem. 272*(13):8531-8538 (1997); Watkins, P.A. *et al., J. Biol. Chem. 273*(29):18210-18219 (1998)) can be engineered to harbor a related gene of the family of FATP-encoding genes, such as a mammalian FATP (e:g., human FATP4).

Examples of expression vectors include pEG (Mitchell, D.A., *et al., Yeast 9*:715-723 (1993)) and pDAD1 and pDAD2, which contain a *GAL1* promoter (Davis, L. I. and Fink, G. R., *Cell 61*:965-978 (1990)). A variety of promoters are suitable for expression. Available yeast vectors offer a choice of promoters. the inducible *GAL1* promoter may be used. Alternatively, the constitutive *ADH1* promoter (alcohol dehyrodrogenase; Bennetzen, J. L. and Hall, B. D., *J Biol. Chem. 257*:3026-3031 (1982)) can be used to express an inserted gene on glucose-containing media. An example of a vector suitable for expression of a heterologous FATP gene in yeast is pQB 169.

With the introduced FATP gene providing the only fatty acid transport protein function for the yeast cells, it is possible to study effect of the heterologous FATP on fatty acid transport into the yeast cells in isolation. Assays for the uptake of fatty acids into the yeast cells can be devised that are similar to those described above and/or those assays that have been illustrated in the Examples. Tests for candidate inhibitors or enhancers of the heterologous FATP can be done in cultures of yeast cells, wherein the yeast cells are incubated with fatty acid substrate and an agent to be tested as an inhibitor or enhancer of FATP function. FATP uptake after a period of time can be measured by analyzing the contents of the yeast cells for fatty acid substrate, as compared with control yeast cells incubated with the fatty acid, but not with the test agent. Yeast cells have the additional advantage, over mammalian cells in culture, for example, that yeast cells can be forced to rely upon fatty acids as their only source of carbon, if the growth medium supplied to the yeast cells is formulated to contain no other source of carbon. Thus, the effect of the heterologous FATP on fatty acid uptake and metabolism in the engineered yeast cells can be amplified. An agent that efficiently blocks transport function of the heterologous FATP could result in death of the yeast cells. Thus, in this case, inhibition of function of the heterologous FATP can result in loss of viability. A simple measure of viability is turbidity of the yeast suspension culture, which can be adapted to a high throughput screening assay for effects of various agents to be tested, using microtiter plates or similar devices for small-volume cultures of the engineered yeast cells.

Cell-free assays can also be used to measure the transport of fatty acids across a membrane, and therefor also to assess a test treatment or test agent for its effect on the rate or extent of fatty acid transport. An isolated FATP, for example in the presence of a detergent that preserves the native 3-dimensional structure of the FATP, or partially purified FATP, can be used in an artificial membrane system typically used to preserve the native conformation and activity of membrane proteins. Such systems include liposomes, artificial bilayers of phospholipids, isolated plasma membrane such as cell membrane fragments, cell membrane fractions, or cell membrane vesicles, and other systems in which the FATP can be properly oriented within the membrane to have transport activity. Assays for transport activity can be performed using methods analogous to those that can be used in cells engineered to predominantly express one FATP whose function is to be measured. A labeled (e.g., radioactively labeled) fatty acid substrate can be incubated with one side of a bilayer or in a suspension of liposomes constructed to integrate a properly oriented FATP. The accumulation of fatty acids with time can be measured, using appropriate means to detect the label (e.g., scintillation counting of medium on each side of the bilayer, or of the contents of liposomes isolated from the surrounding medium). Assays such as these can be adapted to use for the testing of agents which might interact with the FATP to produce an inhibitory or an enhancing effect on the rate or extent of fatty acid transport. That is, the above-described assay can be done in the presence or absence of the agent to be tested, and the results compared.

For examples of isolation of membrane proteins (ADP/ATP carrier and uncoupling protein), reconstitution into phospholipid vesicles, and assays of transport, see Klingenberg, M. *et al., Methods Enzymol. 260*:369-389 (1995). For an example of a membrane protein (phosphate carrier of *Saccharomyces cerevisiae)* that was purified and solubilized from *E*. *coli* inclusion bodies, see Schroer, A*. et al., J. Biol. Chem. 273:* 14269-14276 (1998). The Glutl glucose transporter of rat has been expressed in yeast. A crude membrane fraction of the yeast was prepared and reconstituted with soybean phospholipids into liposomes. Glucose transport activity could be measured in the liposomes (Kasahara, T. and Kasahara, M., *J. Biol. Chem. 273*: 29113-29117 (1998)). Similar methods can be applied to the proteins and polypeptides of the invention.

Also disclosed herein is a method in a method for inhibiting fatty acid uptake in a mammal (e.g., a human), comprising administering to the mammal a therapeutically effective amount of an inhibitor of the transport function of one or more of the fatty acid transport proteins, thereby decreasing fatty acid uptake by cells comprising the fatty acid protein(s). Where it is desirable to reduce the uptake of fatty acids, for example, in the treatment of chronic obesity or as a part of a program of weight control or hyperlipidemia control in a human, one or more inhibitors of one or more of the fatty acid transport proteins can be administered in an effective dose, and by an effective route, for example, orally, or by an indwelling device that can deliver doses to the small intestine. The inhibitor can be one identified by methods described herein, or can be one that is, for instance, structurally related to an inhibitor identified by methods described herein (e.g., having chemical adducts to better stabilize or solubilize the inhibitor). Also disclosed are compositions comprising inhibitors of fatty acid uptake in a mammal, which may further comprise pharmaceutical carriers suitable for administration to a subject mammal, such as sterile solubilizing or emulsifying agents.

Also disclosed herein is a method of enhancing or increasing fatty acid uptake, such as enhancing or increasing LCFA uptake in the small intestine (e.g., to treat or prevent a malabsorption syndrome or other wasting condition) or in the liver (e.g., by an enhancer of FATP5 transport activity to treat acute liver failure) or in the kidney (e.g., by an enhancer of FATP2 transport activity to treat kidney failure). A therapeutically effective amount of an enhancer of the transport function of one or more of the fatty acid transport proteins can be administered to a mammalian subject, with the result that fatty acid uptake in the small intestine is enhanced. One or more enhancers of one or more of fatty acid transport proteins is administered in an effective dose and by a route (e.g., orally or by a device, such as an indwelling catheter or other device) which can deliver doses to the gut. The enhancer of FATP function (e.g., an enhancer of FATP4 function) can be identified by methods described herein or can be one that is structurally similar to an enhancer identified by methods described herein.

Aerobic reperfusion of ischemic myocardium is a common clinical event which can occur during such treatments as cardiac surgery, angioplasty, and thrombolytic therapy after a myocardial infarction. During reperfusion, a rapid recovery of myocardial energy production is essential for the complete recovery of contractile function. Not only the extent of recovery of myocardial energy metabolism but also the type of energy substrate used by the heart during reperfusion are important determinants of functional recovery. Circulating fatty acid levels increase following acute myocardial infarction or during cardiac surgery, such that during and following ischemia the heart muscle can be exposed to very high concentrations of fatty acids (Lopaschuk, G.D. and W. C. Stanley, *Science and Medicine* (November/December 1997)). High plasma fatty acid concentrations increase the severity of ischemic damage in a number of experimental models of cardiac ischemia and have been linked to depression of mechanical function during aerobic reperfusion of previously ischemic hearts. Further data show that modifying fatty acid utilization can be beneficial for heart function in ischemia and can be a useful approach for the treatment of angina. See, e.g., Desideri and Celegon, *Am. J. Cardiol. 82(5A):*50K-53K; Lopaschuk, *Am. J. Cardiol. 82(5A):*14K-17K. Plasma fatty acid concentrations can be reduced by administering to a human subject or other mammal an effective amount of an inhibitor of a FATP such as FATP2 or FATP4, thereby providing a way of reducing fatty acid utilization by the heart.

There is also disclosed herein that a therapeutically effective amount of an inhibitor of hsFATP6 can be administered to a human patient by a suitable route, to reduce the uptake of fatty acids by cardiac muscle. This treatment is desirable in patients who are diagnosed as having, or who are at risk of, abnormal accumulations of fatty acids in the heart or a detrimentally high rate of uptake of fatty acids into the heart, because of ischemic heart disease, or following ischemia or trauma to the heart.

Also disclosed are antibodies that bind to an isolated or recombinant fatty acid transport protein of the FATP family, including portions of antibodies, which can specifically recognize and bind to one or more FATPs. The antibodies and portions thereof include those which bind to one or more FATPs of mouse or other mammalian species. It is preferred that the antibodies specifically bind to a naturally occurring FATP of humans. The antibodies can be used in methods to detect or to purify a protein of the present invention or a portion thereof by various methods of immunoaffinity chromatography, to inhibit the function of a protein in a method of therapy, or to selectively inactivate an active site, or to study other aspects of the structure of these proteins, for example.

The antibodies disclosed herein can be polyclonal or monoclonal. The term antibody is intended to encompass both polyclonal and monoclonal antibodies. Antibodies of the present invention can be raised against an appropriate immunogen, including proteins or polypeptides disclosed herein such as an isolated or recombinant FATP1, FATP2, FATP3, FATP4, FATP5, FATP6, mtFATP, ceFATPa, ceFATPb, scFATP or portions thereof, or synthetic molecules, such as synthetic peptides (e.g., conjugated to a suitable carrier). Preferred antibodies bind to any of the following: hsFATP1, hsFATP2, hsFATP3, hsFATP4, hsFATP5 or hsFATP6. The immunogen can be a polypeptide comprising a portion of a FATP and having at least one function of a fatty acid transport protein, as described herein.

The term antibody is also intended to encompass single chain antibodies, chimeric, humanized or primatized (CDR-grafted) antibodies and the like, as well as chimeric or CDR-grafted single chain antibodies, comprising portions from more than one species. For example, the chimeric antibodies can comprise portions of proteins derived from two different species, joined together chemically by conventional techniques or prepared as a single contiguous protein using genetic engineering techniques (e.g., DNA encoding the protein portions of the chimeric antibody can be expressed to produce a contiguous protein chain. See, e.g., Cabilly et al., U.S. Patent No. 4,816,567; Cabilly *et al.,* European Patent No. 0,125,023 B1; Boss *et al.,* U.S. Patent No. 4,816,397; Boss *et al.,* European Patent No. 0,120,694 B1; Neuberger, M.S. *et al. ,* WO 86/01533; Neuberger, M.S. *et al.*, European Patent No. 0,194,276 B1; Winter, U.S. Patent No. 5,225,539; Winter, European Patent No. 0,239,400 B 1; Queen *et al.,* U.S. Patent No. 5,585,089; and Queen *et al.,* European Patent No. EP 0 451 216 B1. See also, Newman, R. *et al., BioTechnology, 10:*1455-1460 (1992), regarding primatized antibody, and Ladner *et al.,* U.S. Patent No. 4,946,778 and Bird, R.E. *et al., Science, 242*:423-426 (1988) regarding single chain antibodies.)

Whole antibodies and biologically functional fragments thereof are also encompassed by the term antibody. Biologically functional antibody fragments which can be used include those fragments sufficient for binding of the antibody fragment to a FATP to occur, such as Fv, Fab, Fab' and F(ab')₂ fragments. Such fragments can be produced by enzymatic cleavage or by recombinant techniques. For instance, papain or pepsin cleavage can generate Fab or F(ab')₂ fragments, respectively. Antibodies can also be produced in a variety of truncated forms using antibody genes in which one or more stop codons have been introduced upstream of the natural stop site. For example, a chimeric gene encoding a F(ab')₂ heavy chain portion can be designed to include DNA sequences encoding the CH₁ domain and hinge region of the heavy chain.

Preparation of immunizing antigen (whole cells comprising FATP on the cell surface or purified FATP), and polyclonal and monoclonal antibody production can be performed using any suitable technique. A variety of methods have been described (See e.g., Kohler *et al*., *Nature, 256:* 495-497 (1975) and *Eur.J. Immunol. 6:* 511-519 (1976); Milstein *et al., Nature 266: 550-552 (1977);* Koprowski *et al.,* U.S. Patent No. 4,172,124; Harlow, E. and D. Lane, 1988, *Antibodies: A Laboratory Manual,* (Cold Spring Harbor Laboratory: Cold Spring Harbor, NY); Chapter 1 In *Current Protocols In Molecular Biology,* Vol. 2 (containing supplements up through Supplement 42, 1998), Ausubel, F.M. *et al.,* eds., (John Wiley & Sons: New York, NY)). Generally, a hybridoma can be produced by fusing a suitable immortal cell line (e.g., a myeloma cell line such as SP2/0) with antibody producing cells. The antibody producing cells, preferably those obtained from the spleen or lymph nodes, can be obtained from animals immunized with the antigen of interest. Immunization of animals can be by introduction of whole cells comprising fatty acid transport protein on the cell surface. The fused cells (hybridomas) can be isolated using selective culture conditions, and cloned by limiting dilution. Cells which produce antibodies with the desired specificity can be selected by a suitable assay (e.g., ELISA).

Other suitable methods of producing or isolating antibodies (including human antibodies) of the requisite specificity can used, including, for example, methods which select recombinant antibody from a library (e.g., Hoogenboom *et al.,* WO 93/06213; Hoogenboom *et al.,* U.S. Patent No. 5,565,332; WO 94/13804, published June 23, 1994; and Dower, W.J. *et al.,* U.S. Patent No. 5,427,908), or which rely upon immunization of transgenic animals (e.g., mice) capable of producing a full repertoire of human antibodies (see e.g., Jakobovits *et al., Proc. Natl. Acad. Sci. USA, 90*: 255 1-2555 (1993); Jakobovits *et al., Nature, 362*:255-258 (1993); Lonberg *et al.,* U.S. Patent No. 5,569,825; Lonberg *et al.,* U.S. Patent No. 5,545,806; Surani *et al.,* U.S. Patent No. 5,545,807; and Kucherlapati, R*. et al.,* European Patent No. EP 0 463 151 B 1).

Also disclosed herein is a method for directing an agent to cardiac muscle. The differential expression of FATP6 in cardiac muscle but not in other tissue types allows for the specific targeting of drugs, diagnostic agents, tagging labels, histological stains or other substances specifically to cardiac muscle. A targeting vehicle can be used for the delivery of such a substance. Targeting vehicles which bind specifically to FATP6 can be linked to a substance to be delivered to the cells of cardiac muscle. The linkage can be, for instance, via one or more covalent bonds, or by high affinity non-covalent bonds. A targeting vehicle can be an antibody, for instance, or other compound (e.g., a fatty acid or fatty acid analog) which binds to FATP6 with high specificity.

Targeting vehicles specific to the heart-specific protein FATP6 have *in vivo* (e.g., therapeutic and diagnostic) applications. For example, an antibody which specifically binds to FATP6 can be conjugated to a drug to be targeted to the heart (e.g., a cardiac glycoside to treat congestive heart failure, or β-adrenergic agents, sodium channel blockers or calcium channel blockers to treat arrhythmias). A substance (e.g., a radioactive substance) which can be detected (e.g., a label) *in vivo* can also be linked to a targeting vehicle which specifically binds to a heart-specific protein such as FATP6, and the conjugate can be used as a labeling agent to identify cardiac muscle cells.

Targeting vehicles specific to FATP6 find further applications *in vitro.* For example, an FATP6-specific targeting vehicle, such as an antibody (a polyclonal preparation or monoclonal) which specifically binds to FATP6, can be linked to a substance which can be used as a stain for a tissue sample (e.g., horseradish peroxidase) to provide a method for the identification of cardiac muscle in a sample, as can be used in embryology studies, for example.

In a similar manner, an agent can be directed to the liver of a mammal, as FATP5 is expressed in liver but not in other tissue types. A targeting vehicle which specifically binds to FATP5 can be conjugated to a drug for delivery of the drug to the liver, such as a drug to treat hepatitis, Wilson's disease, lipid storage diseases and liver cancer. As with targeting vehicles specific to FATP6, targeting vehicles specific to FATP5 can be used in studying tissue samples *in vitro.*

Also disclosed herein are compositions comprising a modulator of FATP function. The term "modulate" as used herein refers to the ability of a molecule to alter the function of another molecule. Thus, modulate could mean, for example, inhibit, antagonize, agonize, upregulate, downregulate, induce, or suppress. A modulator has the capability of altering function of its target. Such alteration can be accomplished at any stage of the transcription, translation, expression or function of the protein, so that, for example, modulation of a target gene can be accomplished by modulation of the DNA or RNA encoding the protein, and the protein itself.

Antagonists or agonists (inhibitors or enhancers) of the FATPs disclosed herein, antibodies that bind a FATP, or mimetics of a FATP can be employed in combination with a non-sterile or sterile carrier or carriers for use with cells, tissues or organisms, such as a pharmaceutical carrier suitable for administration to a mammalian subject. Such compositions comprise, for instance, a media additive or a therapeutically effective amount of an inhibitor or enhancer compound to be identified by an assay disclosed herein and a pharmaceutically acceptable carrier or excipient. Such carriers may include, but are not limited to, saline, buffered saline, dextrose, water, ethanol, surfactants, such as glycerol, excipients such as lactose and combinations thereof. The formulation can be chosen by one of ordinary skill in the art to suit the mode of administration. The chosen route of administration will be influenced by the predominant tissue or organ location of the FATP whose function is to be inhibited or enhanced. For example, for affecting the function of FATP4, a preferred administration can be oral or through a tube inserted into the stomach (e.g., direct stomach tube or nasopharyngeal tube), or through other means to accomplish delivery to the small intestine. Also disclosed are diagnostic and pharmaceutical packs and kits comprising one or more containers filled with one or more of the ingredients of the aforementioned compositions.

Compounds disclosed herein which are FATPs, FATP fusion proteins, FATP mimetics, FATP gene-specific antisense poly- or oligonucleotides, inhibitors or enhancers of a FATP may be employed alone or in conjunction with other compounds, such as therapeutic compounds. The pharmaceutical compositions may be administered in any effective, convenient manner, including administration by topical, oral, anal, vaginal, intravenous, intraperitoneal, intramuscular, subcutaneous, intranasal, transdermal or intradermal routes, among others. In therapy or as a prophylactic, the active agent may be administered to an individual as an injectable composition, for example as a sterile aqueous dispersion, preferably isotonic.

Alternatively, the composition may be formulated for topical application, for example, in the form of ointments, creams, lotions, eye ointments, eye drops, car drops, mouthwash, impregnated dressings and sutures and aerosols, and may contain appropriate conventional additives, including, for example, preservatives, solvents to assist drug penetration, and emollients in ointments and creams. Such topical formulations may also contain compatible conventional carriers, for example cream or ointment bases, and ethanol or oleyl alcohol for lotions.

In addition, the amount of the compound will vary depending on the size, age, body weight, general health, sex, and diet of the host, and the time of administration, the biological half-life of the compound, and the particular characteristics and symptoms of the disorder to be treated. Adjustment and manipulation of established dose ranges are well within the ability of those of skill in the art.

Also disclosed herein is a method to identify a polymorphism, or the presence of an alternative or variant allele of a gene in the genome of an organism (of interest here, genes encoding FATPs). As used herein, polymorphism refers to the occurrence of two or more genetically determined alternative sequences or alleles in a population. A polymorphic locus may be as small as a base pair. Polymorphic markers include restriction fragment length polymorphisms, variable number of tandem repeats (VNTR's), hypervariable regions, minisatellites, dinucleotide repeats, trinucleotide repeats, tetranucleotide repeats, simple sequence repeats, and insertion elements such as Alu. The first identified alleleic form, or the most frequently occurring form can be arbitrarily designated as the reference (usually, "wildtype") form, and other allelic forms are designated as alternative (sometimes, "mutant" or "variant"). Dipolid organisms may be homozygous or heterozygous for allelic forms.

An "allele" or "allelic sequence" is an alternative form of a gene which may result from at least one mutation in the nucleotide sequence. Alleles may result in altered mRNAs or polypeptides whose structure or function may or may not be altered. Any given gene may have none, one, or many allelic forms (polymorphism). Common mutational changes which give rise to alleles are generally ascribed to natural deletions, additions, or substitutions of nucleotides. Each of these types of changes may occur alone, or in combination with the others, one or more times in a given sequence.

Several different types of polymorphisms have been reported. A restriction fragment length polymorphism (RFLP) is a variation in DNA sequence that alters the length of a restriction fragment (Botstein *et al., Am. J. Hum. Genet. 32*:314-331 (1980)). The restriction fragment length polymorphism may create or delete a restriction site, thus changing the length of the restriction fragment. RFLPs have been widely used in human and animal genetic analyses (see WO 90/13668; WO 90/11369; Donis-Keller, *Cell 51*:319-337 (1987); Lander et al., *Genetics 121*:85-99 (1989)). When a heritable trait can be linked to a particular RFLP, the presence of the RFLP in an individual can be used to predict the likelihood that the individual will also exhibit the trait.

Other polymorphisms take the form of short tandem repeats (STRs) that include tandem di-, tri- and tetra-nucleotide repeated motifs. These tandem repeats are also referred to as variable number tandem repeat (VNTR) polymorphisms. VNTRs have been used in identity and paternity analysis (US 5,075,217; Armour *et al., FEBS Lett. 307*:113-115 (1992); Hom *et al.,* WO 91/14003; Jeffreys, EP 370,719), and in a large number of genetic mapping studies.

Other polymorphisms take the form of single nucleotide variations between individuals of the same species. Such polymorphisms are far more frequent than RFLPs, STRs (short tandem repeats) and VNTRs (variable number tandem repeats). Some single nucleotide polymorphisms occur in protein-coding sequences, in which case, one of the polymorphic forms may give rise to the expression of a defective or other variant protein and, potentially, a genetic disease. Other single nucleotide polymorphisms occur in noncoding regions. Some of these polymorphisms may also result in defective protein expression (e.g., as a result of defective splicing). Other single nucleotide polymorphisms have no phenotypic effects.

Many of the methods described below require amplification ofDNA from target samples and purification of the amplified products. This can be accomplished by PCR, for instance. See generally, *PCR Technology, Principles and Applications for DNA Amplification* (ed. H.A. Erlich), Freeman Press, New York, NY, 1992; *PCR Protocols: A Guide to Methods and Applications* (eds. Innis, et al.), Academic Press, San Diego, CA, 1990; Mattila *et al, Nucleic Acids Res. 19*:4967 (1991); Eckert *et al., PCR Methods and Applications 1*:17 (1991); *PCR* (eds. McPherson *et al.,* IRS Press, Oxford); and US 4,683,202.

Other suitable amplification methods include the ligase chain reaction (LCR) (see Wu and Wallace, *Genomics 4*:560 (1989); Landegren *et al., Science 241*:1077 (1958)), transcription amplification (Kwoh *et al., Proc. Natl. Acad. Sci. USA 86:*1173 (1989), self-sustained sequence replication (Guatelli *et al., Proc. Natl. Acad. Sci. USA 87*:1874 (1990), and nucleic acid based sequence amplification (NASBA). The latter two amplification methods involve isothermal reactions based on isothermal transcription, which produce both single stranded RNA (ssRNA) and double stranded DNA (dsDNA) as the amplification products in a ratio of about 30 or 100 to 1, respectively.

Also disclosed herein is a method for detecting a variant allele of a human FATP gene, comprising preparing amplified, purified FATP DNA from a reference human and amplified, purified, FATP DNA from a "test" human to be compared to the reference as having a variant allele, using the same or comparable amplification procedures, and determining whether the reference DNA and test DNA differ in DNA sequence in the FATP gene, whether in a coding or a noncoding region, wherein, if the test DNA differs in sequence from the reference DNA, the test DNA comprises a variant allele of a human FATP gene. The following is a discussion of some of the methods by which it can be determined whether the reference FATP DNA and test FATP DNA differ in sequence.

Direct Sequencing. The direct analysis of the sequence of variant alleles disclosed herein can be accomplished using either the dideoxy chain termination method or the Maxam and Gilbert method (see Sambrook *et al., Molecular Cloning: A Laboratory Manual,* 2nd ed., Cold Spring Harbor Press, New York 1989; Zyskind *et al., Recombinant DNA Laboratory Manual,* Acad. Press, 1988)).

Denaturing Gradient Gel Electrophoresis. Amplification products generated using the polymerase chain reaction can be analyzed by the use of denaturing gradient gel eletrophoresis. Different alleles can be identified based on the different sequence-dependent strand dissociation properties and electrophoretic migration of DNA in solution (chapter 7 in Erlich, ed. *PCR Technology, Principles and Applications for DNA Amplification,* W.H. Freeman and Co., New York, 1992).

Single-strand Conformation Polymorphism Analysis. Alleles of target sequences can be differentiated using single-strand conformation polymorphism analysis, which identifies base differences by alteration in electrophoretic migration of single stranded PCR products, as described in Orita *et al., Proc. Natl. Acad. Sci. USA 86*:2766-2770 (1989). Amplified PCR products can be generated as described above, and heated or otherwise denatured, to form single-stranded amplification products. Single-stranded nucleic acids may refold or form secondary structures which are partially dependent on the base sequence. The different electrophoretic mobilities of single-stranded amplification products can be related to base-sequence differences between alleles of target sequences.

Detection of Binding by Protein That Binds to Mismatches. Amplified DNA comprising the FATP gene or portion of the gene of interest from genomic DNA, for example, of a a normal individual is prepared, using primers designed on the basis of the DNA sequences provided herein. Amplified DNA is also prepared, in a similar manner, from genomic DNA of an individual to be tested for bearing a distinguishable allele. The primers used in PCR. carry different labels, for example, primer 1 with biotin, and primer 2 with ³²P. Unused primers are separated form the PCR products, and the products are quantitated. The heteroduplexes are used in a mismatch detection assay using immobilized mismatch binding protein (MutS) bound to nitrocellulose. The presence of biotin-labeled DNA wherein mismatched regions are bound to the nitrocellulose via MutS protein, is detected by visualizing the binding of streptavidin to biotin. See WO 95/12689. MutS protein has also been used in the detection of point mutations in a gel-mobility-shift assay (Lishanski, A. *et al., Proc. Natl. Acad. Sci. USA 91*:2674-2678 (1994)).

Other methods, such as those described below, can be used to distinguish a FATP allele from a reference allele, once a particular allele has been characterized as to DNA sequence.

Allele-specific probes. The design and use of allele-specific probes for analyzing polymorphims is described by e.g., Saiki *et al., Nature 324:*163-166 (1986); Dattagupta, EP 235,726, Saiki, WO 89/11548. Allele-specific probes can be designed so that they hybridize to a segment of a target DNA from one individual but do not hybridize to the corresponding segment from another individual due to the presence of different polymorphic forms in the respective segments from the two individuals. Hybridization conditions should be sufficiently stringent that there is a significant difference in hybridization intensity between alleles, and preferably an essentially binary response, whereby a probe hybridizes to only one of the alleles. Some probes are designed to hybridize to a segment of target DNA such that the polymorphic site aligns with a central position (e.g., in a 15-mer at the 7 position; in a 16-mer, at either the 8 or 9 position) of the probe. This design of probe achieves good discrimination in hybridization between different allelic forms.

Allele-specific probes are often used in pairs, one member of a pair showing a perfect match to a reference form of a target sequence and the other member showing a perfect match to a variant form. Several pairs of probes can then be immobilized on the same support for simultaneous analysis of multiple polymorphisms within the same target sequence.

Allele-specific Primers. An allele-specific primer hybridizes to a site on target DNA overlapping a polymorphism, and only primes amplification of an allelic form to which the primer exhibits perfect complementarity. See Gibbs, *Nucleic Acid Res. 17*:2427-2448 (1989). This primer is used in conjunction with a second primer which hybridizes at a distal site. Amplification proceeds from the two primers, resulting in a detectable product which indicates the particular allelic form is present. A control is usually performed with a second pair of primers, one of which shows a single base mismatch at the polymorphic site and the other of which exhibits perfect complementarity to a distal site. The single-base mismatch prevents amplification and no detectable product is formed. The method works best when the mismatch is included in the 3'-most position of the oligonucleotide aligned with the polymorphism because this position is most destabilizing to elongation from the primer (see, e.g., WO 93/22456).

Gene Chips. Allelic variants can also be identified by hybridization to nucleic acids immobilized on solid supports (gene chips), as described, for example, in WO 95/11995 and U.S. Patent No. 5,143,854. WO 95/11995 describes subarrays that are optimized for detection of a characterized variant allele. Such a subarray contains probes designed to be complementary to a second reference sequence, which is an allelic variant of the first reference sequence.

The present method is illustrated by the following examples, which are not intended to be limiting in any way.

### EXAMPLES

### Materials and Methods

The following Materials and Methods were used in the work described in Examples 1-5.

Sequence Alignment of FATP Clones. The DNA sequence for mouse FATP 1 was obtained from the National Center for Biotechnology Information nonredundant database. cDNAs for mmFATP2, 3, 4, and 5 were obtained by screening mouse expression libraries (purchased from GIBCO/BRL) with probes derived from the cloned expressed sequence tags (ESTs) (Research Genetics, Huntsville, AL). Full-length clones were obtained for mmFATP2 and 5 and partial sequences for mmFATP3 and 4. The sequences described herein have been deposited in the GenBank database (Accession Nos. FATP2, AF072760; FATP3, AF072759; FATP4, AF072758; FATP5, AF072757).

Neither FATP2 nor FATP5 contains an in-frame stop codon upstream of the putative initiator methionine; initiator methionines were assigned by homology with that in mmFATP1 and by the presence of a signal sequence immediately after it. The *Mycobacterium tuberculosis, Caenorhabditis elegans,* and *Saccharomyces cerevisiae* sequences were present in the dbEST database as part of the sequencing projects for these organisms. Sequences were aligned utilizing a ClustalX algorithm and the resulting alignment exported to Seq Vu. Homologous amino acid substitutions are boxed in Figure 1 and were determined using the Dayhoff 250 method with a 50% homology cutoff.

Cell Transfection and LCFA Uptake. COS cells were cotransfected using the DEAE-dextran method with the mammalian expression vector pCDNA 3.1 (Invitrogen) expressing the gene for CD2 (pCDNA-CD2) in combination with either a pCDNA 3.1 or pCMVSPORT2 (GIBCO/BRL) expression vector containing one of the murine or nematode *FATP* genes (*pCDNA-mmFATP1, pCDNA-FATP2, pCMVSPORT-FATP5, pCDNA-ceFATPb).* Two days after transfection, cells were assayed for CD2 expression with a phycoerythrin-coupled anti-CD2(PE-CD2) monoclonal antibody (PharMingen), and fatty acid uptake was assayed with a BODIPY-labeled fatty acid analogue (Molecular Probes). Briefly, cells were washed twice with PBS (phosphate buffered saline) and stained with PE-CD2 at 4°C for 30 min in PBS containing 10% fetal calf serum. They were then washed three times with PBS/fetal calf serum for 5 min followed by an incubation for 2 min at 37°C in fatty acid uptake solution, which contained 0.1 µM BODIPY-FA and 0.1 % fatty acid-free BSA (bovine serum albumin) in PBS (Schaffer, J.E. & Lodish, H.F. (1994) *Cell 79*:427-436). After 2 min, the cells were washed four times with ice-cold PBS/0.1% BSA. The cells were then removed from the plates with PBS containing 5 mM EDTA and resuspended in PBS containing 10% fetal calf serum and 10 mM EDTA. PE-CD2 and BODIPY-FA fluorescence were measured using a FACScan (Becton Dickinson). COS cells were gated on forward scatter (FSC) and side scatter (SS). Cells exhibiting more than 300 CD2 fluorescence units (dsim) representing 15% of all cells were deemed CD2 positive and their BODIPY-FA fluorescence was quantitated.

*E*. *coli-*Based LCFA Uptake Assay. The full-length coding region of mtFATP and a control protein, the mammalian transcription factor TFE3, were subcloned into the inducible, prokaryotic expression vector pET (Novagen). Expression was induced with 1 mM isopropyl β-D-thiogalactoside (IPTG) for 1 hour, or cells were left uninduced. Cells were washed in PBS/0.1 % BSA and resuspended in 1 ml PBS/0.1% BSA containing 0.1 µM [³H]palmitate (NEN) at 37°C. Uptake was stopped after the indicated incubation time by transferring the cells onto filter paper using a cell harvester (Brandel, Bethesda, MD). Filters were washed extensively with ice-cold PBS/0.1% BSA, and [³H]palmitate was quantitated by scintillation counting.

Northern Blots. Northern blot analysis of murine FATP expression was done using poly(A) mRNA blots (Clontech). Probes of each of the FATPs were derived from the 3' untranslated regions of each gene and were <60% identical in sequence. Probes were labeled by random priming (Boehringer Mannheim) and hybridized at 65 °C. Blots were extensively washed in 0.2% SSC/0.1% SDS at 65 °C.

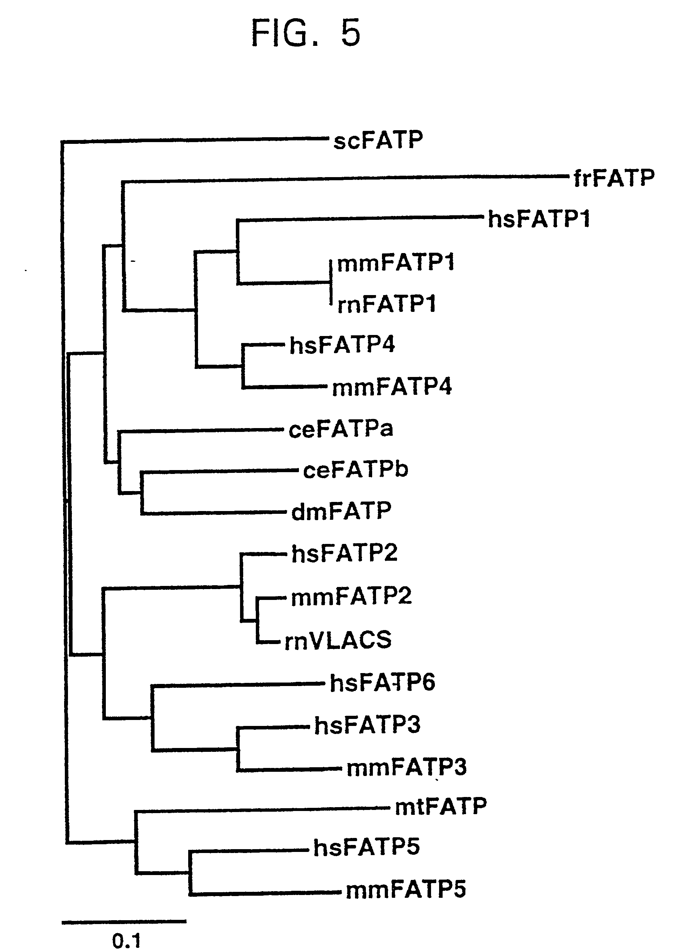

Generation of Phylogenetic Trees. Complete and partial sequences for *FATP* genes from human, rat, mouse, puffer fish, *Drosophila melanogaster, C. elegans, S. cerevisiae,* and *M. tuberculosis* were aligned using ClustaIX. A homologous region of 48 amino acids (residues 472-519 in mmFATP1) from all of the genes was used to determine phylogenetic relationship within ClustaIX. Based on these data a phylogenetic tree was generated using Tree View PPC (Figure 5).

Nomenclature. It is proposed that the *FATP* genes be given a species specific prefix (mm, *Mus musculus;* hs, *Homo sapiens;* mt, *M. tuberculosis;* dm, *D. melanogaster;* ce, *C*. *elegans,* sc, *S. cerevisiae*) and numbered such that mammalian homologues in different species share the same number but differ in their prefix. Since the two *C*. *elegans* genes cannot be paired with a specific human or mouse FATP, they have been designated *ceFATP*a and *ceFATPb.*

### Example 1: Identification of Novel Mammalian FATPs

The National Center for Biotechnology Information EST database was screened, using the mouse FATP protein sequence (mmFATP1), to identify novel FATPs. This strategy led to the identification of more than 50 murine EST sequences which could be assembled into five distinct contiguous DNA sequences (contigs). One contig was identical to the previously cloned FATP, which has been renamed FATP1. Another, which has been renamed FATP2, is the murine homologue of a rat gene previously identified by others as a very long chain acyl-CoA synthase (Uchiyama, A., Aoyama, T., Kamijo, K., Uchida, Y., Kondo, N., Orii, T. & Hashimoto, T. (1996) *J. Biol. Chem. 271*:30360-30365). The other three contigs represented novel genes (*FATP3, 4,* and *5*). Full-length clones for *FATP2* and *FATP5* and nearly complete sequences for *FATP3* and *4* (Figure 1) were obtained by screening cDNA libraries made from mouse day 10.5 embryos and adult liver. Also identified were human homologues for each of the murine genes in the EST database. A sixth human gene was also identified; whether this gene is also present in the mouse will require additional studies. Map positions are given in Tables 2 and 3.

The genetic loci for all of the human genes, with the exception of FATP5 which was already mapped as an unknown EST, were determined using the radiation hybrid panels. The map positions given below show the distance (in centiRays) from the closest framework marker. As a guideline, there are approximately 300kb/cR.

**Table 2. Mapping Data for Human Genes**

| | |
|---|---|
| hsFATP1 | Chromosome Chr19 places 13.35 cR from WI-6344 (lod>3.0) |
| hsFATP2 | Chromosome Chr15 places 4.92 cR from D15S126 (lod>3.0) |
| hsFATP3 | Chromosome Chr1 places 13.24 cR from WI-2862 (lod>3.0) |
| hsFATP4 | Chromosome Chr9 places 7.80 cR from WI-9685 (lod>3.0) |
| hsFATP5 | unknown EST previously mapped to near D19S418 |
| hsFATP6 | Chromosome Chr5 places 1.41 cR from WI-4907 (lod>3.0) |

The mouse map is an internal backcross panel consisting of 188 mouse backcross DNA's plus 4 controls (B6, Spretus, Fl, Water). The backcross was constructed by crossing B6 by Spretus animals and then crossing those Fl's back to B6. Mapping is accomplished by taking advantage of recombinational events during meiosis, and the use of PCR primers to detect the differences (by size or re-annealing events) at any given locus between the B6 and Spretus allele.

For the purposes of mapping, a novel set of primers (gene of interest) is used to amplify from all 188 DNA's and then typed as being a B6 ("B") or a Spretus ("S"). This string of B's and S's is entered into the Map Manager program, which does a best fit calculation by comparing the string of 188 typings from the gene of interest to all loci already extant in the panel, for all 20 chromosomes. The gene of interest is then assigned to a particular area on a particular chromosome according to a number of parameters, including the minimalization of double cross-overs, and the highest LOD scores. Indicated in Table 3 are distances to the closest markers on either side of the FATP locus.

**Table 3. Mapping Data for Mouse Genes**

| | |
|---|---|
| mmFATP 1 | Chromosome 8 places 2.82 cM from D8Mit132 (lod 43.4) and 1.81 cM from D8Mit74 (lod 43.5) |
| mmFATP2 | Chromosome 2 places 1.29 cM from D2Mit258 (lod 47.9) and 1.75 cM from D2NDS3 (lod 44.9) |
| mmFATP3 | Chromosome 3 places 2.54 cM from D3Mit22 (lod 29.5) and 19.62 cM from D3Mit42 (lod 13.6) |
| mmFATP4 | Chromosome 2 places 13.78 cM from D2Mit1 (lod 22.9) and 3.85 cM from D2Mit65 (lod 41.9) |
| mmFATP5 | Chromosome 7 places 7.28 cM proximal of D7Mit21 (lod 28.3) |

### Example 2: Assessment of Function

The ability of the newly identified mouse genes to function as fatty acid transporters was assessed using a fluorescence-activated cell sorting-based assay. COS cells were transiently cotransfected with expression vectors encoding the cell surface protein CD2 and either mmFATP1, mmFATP2, or mmFATP5, respectively. Two days after transfection, COS cells were stained with an antibody to CD2 and then incubated with a BODIPY-labeled fatty acid [BODIPY-FA, (Schaffer, J.E. & Lodish, H.F. (1994) *Cell 79*:427-436)]. The cells were then washed extensively, lifted off the dish, and analyzed by fluorescence-activated cell sorting. As judged by the number of CD2-positive cells, the transfection efficiency was approximately 20-30%. Fatty acid uptake was quantitated in the transiently transfected COS cells by measuring the BODIPY-FA fluorescence of the CD2-positive cells. Expression of CD2 had no effect on fatty acid uptake as shown by the finding that COS cells expressing only the transfected CD2 cDNA (CD2-positive) had the same low level of BODIPY-FA uptake as did untransfected (CD2-negative) control cells (Figure 2A, control). In COS cells cotransfected with CD2 and mmFATP1, mmFATP2, or mmFATP5, uptake of BODIPY-FA by the transfected (CD2-positive) cells was increased between 15- to 90-fold over control (CD2 cDNA only) cells (Figures 2A-2D).

### Example 3: Expression Patterns of Murine FATPs

Expression patterns of members of the murine *FATP* gene family were characterized by Northern blot analysis; to avoid cross-hybridization, the probes used were from the 3' untranslated region of these genes, which are less than 60% identical in sequence. The expression pattern ofFATP1 agrees with that previously found (Schaffer, J.E. & Lodish, H.F. (1994) *Cell 79*:427-436). Here, expression was seen primarily in heart and kidney. FATP2 is expressed almost exclusively in liver and kidney, which corresponds to the reported tissue distribution of the rat homologue [very long chain acyl-CoA (VLACS)] as assessed by Western blotting (Uchiyama, A., Aoyama, T., Kamijo, K., Uchida, Y., Kondo, N., Orii, T. & Hashimoto, T. (1996) *J*. *Bio!. Chem. 271*:30360-30365). FATP3 is present in lung, liver, and testis. FATP5 is expressed only in liver and cannot be defected in other tissues even when the blot is overexposed. The human homologue of FATP5 is also liver specific and is not expressed in a wide array of other tissues tested, including fetal liver.

### Example 4: FATPs Are Evolutionarily Conserved

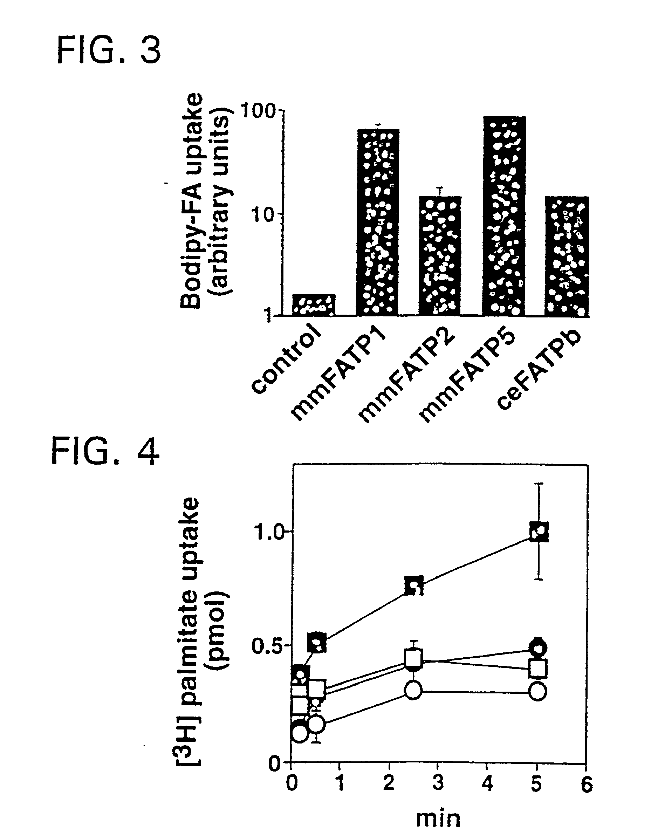

The EST database was searched, using sequences conserved among the five murine FATP genes, for *FATP* genes in other organisms. Two homologues were found in *C*. *elegans* and one in *M. tuberculosis.* One of the *C. elegans* genes was cloned from a cDNA library and expressed in COS cells, as described for the murine FATPs. Overexpression of the nematode FATP resulted in a 15-fold increase of BODIPY-FA uptake compared with control cells (Figure 3). The mycobacterial *FATP* gene was isolated from a phage library and assessed for its ability to facilitate fatty acid uptake. *E. coli* transformed with a prokaryotic, isopropyl β-D-thiogalactoside-inducible expression vector containing the mycobacterial *FATP* gene demonstrated a significant increase in the rate of [³H]palmitate uptake after induction, compared with uninduced bacteria or *E. coli* transformed with a control protein (Figure 4). Novel *FATP* genes were also identified in *F*. *rubripes* (puffer fish) and *D. melanogaster.*

### Example 5: Phylogenetic Tree of FATPs

Faergeman *et al.* (Faergeman, N.J., DiRusso. C.C., Elberger, A., Knudsen, J. & Black, P. N. (1997) *J. Biol. Chem. 272*:8531-8538) identified three regions of very strong conservation between the *scFATP* and *mmFATP1* genes. The sequences of the FATPS were compared over a 311-amino acid FATP "signature sequence" which includes these conserved regions corresponding to amino acids 246-557 in mmFATP1 (underlined in Figure 1). When compared with the National Center for Biotechnology Information nonredundant database, only one region of the "FATP signature sequence" shows significant homology to other proteins. This small stretch of amino acids (underlined in Fig. 1) is an AMP-binding motif found in a multitude of other proteins, such as acyl-CoA synthase, several CoA lipases, and gramicidin S synthetase component II (Schaffer, J.E. & Lodish, H.F. (1994) *Cell 79*:427-436). The relevance of this motif to fatty acid transport is unclear. Other highly conserved regions among the FATPs, including long stretches of amino acids >90% identical from mycobacteria to humans, are not found in any other class of proteins. A 48-amino acid segment of the FATP signature sequence was used to construct a phylogenetic tree (Figure 5). Each of the human and mouse genes form their own branch; hsFATP6, which as yet has no murine homologue, is most closely related to hsFATP3 and mmFATP3. As expected, rnVLACS is closer in sequence to mmFATP2 than to hsFATP2. The *FATP* genes of invertebrates i.e., *C. elegans* and *D. melanogaster,* are most closely related to each other. Surprisingly, the mycobacteral gene is more closely related to the human and mouse *FATP5* genes than to the FATPs of any of the lower organisms. Whether this reflects coevolution of the mycobacterial and human genes awaits further study.

### Materials and Methods

The following materials and methods were used in the work described in Examples 6-10.

### Isolation of full-length human FATP1 and 4

Full-length clones encoding human FATP1 and human FATP4 were identified by searching databases for sequences similar to murine FATP1-5 coding regions using the BlastX algorithm (Altschul *et al., J. Mol. Biol. 215:* 403-410, 1990).

A concatamer of nucleotide sequences comprising the coding sequences of mmFATP1 (Genbank Accession U 15976), mmFATP2, mmFATP3 (SEQ ID NO:6), mmFATP4 (SEQ ID NO:8) and mmFATP5 (SEQ ID NO: 10) was used to search the Millennium database using the BLASTX algorithm. Sequences with a score > 150 were evaluated for whether they represented known FATP coding sequences.

Human clones with similarity to the 5' end of murine FATP sequences were sequenced completely. Clones encoding full-length human FATP1 were obtained from a heart cDNA library constructed in the mammalian expression vector pMET7 (Tartaglia *et al., Cell, 83*:1263-1271, 1995). Clones encoding full-length human FATP4 were obtained from a spleen cDNA library constructed in the mammalian expression vector pMET7. Isolation of full-length human FATP6

Several clones encoding human FATP6 were identified by searching public databases as described above. Five clones were analyzed further by restriction digestion and DNA sequencing. One of these clones (Genbank Accession # AA412064) appeared to be full-length and its entire insert was sequenced.

### DNA Sequence Analysis

Sequences were aligned with the DNAStar program using the Clustal method. Hydrophobicity plots were generated with DNA Strider using the Kyte Doolittle method.

### In situ hybridization

Tissues were collected from 8 week old C57/B16 mice. Tissues were fresh frozen, cut on a cryostat at 10 µm thickness and mounted on Superfrost Plus slides (VWR). Sections were air dried for 20 minutes and then incubated with ice cold 4% paraformaldehyde (PFA)/phosphate buffered saline (PBS) for 10 minutes. Slides were washed 2 times 5 minutes with PBS, incubated with 0.25% acetic anhydride/l M triethanolamine for 10 minutes, washed with PBS for 5 minutes and dehydrated with 70%, 80%, 95% and 100% ethanol for 1 minute each. Sections were incubated with chloroform for 5 minutes. Hybridizations were performed with ³⁵S-radiolabeled (5x10⁷ cpm/ml) cRNA probes generated from the 3' untranslated regions of mouse FATPs by PCR followed by *in vitro* transcription in the presence of 50% formamide, 10% dextran sulfate, 1x Denhardt's solution, 600 mM NaCl, 10 mM DTT, 0.25% SDS and 10 µg/ml tRNA for 18 hours at 55°C. After hybridization, slides were washed with 10 mM Tris-HCl pH 7.6, 500 mM NaCl, 1 mM EDTA (TNE) for 10 minutes, incubated in 40 µg/ml RNase A in TNE at 37°C for 30 minutes, washed in TNE for 10 minutes, incubated once in 2x SSC at 60°C for 1 hour, once in 0.2x SSC at 60°C for 1 hour, once in 0.2x SSC at 65°C for 1 hour and dehydrated with 50%, 70%, 80%, 90% and 100% ethanol. Localization of mRNA transcripts was detected by dipping slides in . Kodak NBT-2 photoemulsion and exposing for 7 days at 4°C, followed by development with Kodak Dektol developer. Slides were counter stained with haematoxylon and eosin and photographed. Controls for the in situ hybridization experiments include the use of a sense probe which showed no signal above background in all cases.

### Northern Blotting

Human mRNA blots were obtained from Invitrogen or Clontech. PCR fragments from the 3' untranslated regions of human FATPs were used as probes. Blots were probed with ³²P-labeled DNA probes using the Rapid-Hyb buffer (Amersham) according to the manufacturer's instructions.

Cell transfection and LCFA uptake. COS cells were cotransfected, using lipofectamine (GIBCO BRL) according to the manufacturer's instructions, with the mammalian expression vector pCDNA3.1 (Invitrogen) expressing the gene for CD2 in combination with a pMET7 expression vector (Tartaglia *et al., Cell,* 83:1263-1271, 1995) containing hsFATP1 (pMET7-hsFATP1) or hsFATP4 (pMET7-hsFATP4) or pMET7 alone. Two days after transfection, cells were assayed for CD2 expression with a phycoerythrin-coupled anti-CD2 (PE-CD2) monoclonal antibody (PharMingen), and fatty acid uptake was assayed with a BODIPY-labeled fatty acid analog (Molecular Probes) as described above.

### Example 6: Determination of Expression of mmFATPs

mmFATP4, and to lesser extent mmFATP2, are expressed at high levels in the brush border layer of the small intestine.

Cell transfection and LCFA uptake. COS cells were cotransfected, using lipofectamine (GIBCO BRL) according to the manufacturer's instructions, with the mammalian expression vector pCDNA3.1 (Invitrogen) expressing the gene for CD2 in combination with a pMET7 expression vector (Tartaglia *et al., Cell,* 83:1263-1271, 1995) containing hsFATP1 (pMET7-hsFATP1) or hsFATP4 (pMET7-hsFATP4) or pMET7 alone. Two days after transfection, cells were assayed for CD2 expression with a phycocrythrin-coupled anti-CD2 (PE-CD2) monoclonal antibody (PharMingen), and fatty acid uptake was assayed with a BODIPY-labeled fatty acid analog (Molecular Probes) as described above.

Absorption of dietary fat requires transport of free fatty acids across the apical membrane of epithelial cells in the small intestine. Previous studies suggested that this transport is protein-mediated; however, the transport protein had not yet been identified. In situ hybridization was performed on each of the three regions of the small intestineduodenum, jejunum and ileum -- as well as the colon, using probes from the 3' untranslated regions of mmFATP1, mmFATP2, mmFATP3, mmFATP4 and mmFATP5, to determine whether any of the mouse FATPs are expressed in the small intestine. It was expected that a protein involved in fatty acid absorption would be expressed in the epithelial cells of the small intestine, but absent from the colon.

Expression of mmFATPs in the jejunum was identical to that in the ileum in all cases. High levels of mmFATP4 mRNA were present in the epithelial cells of the jejunum and ileum, and lower, but significant, amounts were detected in the epithelial cells of the duodenum. Significantly, FATP4 mRNA was absent from other cell types of the small intestine and no FATP4 mRNA could be detected in any of the cells of the colon. FATP2 mRNA was present in the epithelial cells of the duodenum at a level similar to that of FATP4, but was present at lower levels in the jejunum and ileum. No signals above background were detected for mmFATP1, mmFATP3 and mmFATP5 in any of the intestinal tissues. mmFATP3 and FATP5 were clearly detectable by in situ hybridization in adult liver and mmFATP1 could be detected in a variety of tissues on a whole embryo in situ, indicating that the FATP1, 3, and 5 probes were working.

mmFATP4 expression is predominant in the small intestine compared to the other organs of the mouse embryo. In the small intestine, FATP4 expression is limited to differentiated enterocytes, while no signal is detected in the connective tissue or the undifferentiated epithelial cells in the crypts. Differentiated enterocytes are known to be the cells that mediate the uptake of fatty acids. FATP4 is specifically and strongly expressed in the epithelial cells of adult murine duodenum and ileum but not colon. Other FATPs, such as FATP5, are not expressed in the small intestine. Thus, FATP4 is the major FATP in the mouse small intestine. Given its high level of expression, it is likely that FATP4, and to a lesser extent FATP2, play an important role in the absorption of fatty acids.

### mmFATP2, and mmFATP5 are expressed in hepatocytes

Northern analysis of mmFATP2, mmFATP3, mmFATP4 and mmFATP5 showed expression in the liver. To determine whether these proteins are present in hepatocytes or other cells types present in liver homogenates, in situ hybridizations were performed. mmFATP2, and mmFATP5 mRNA was clearly present in hepatocytes, and was not concentrated in other cell types such as endothelial cells or macrophages. No signal above background was detected for mmFATP1 in any of the cell types in the liver, consistent with the results of the Northern blotting.

### Example 7: Isolation and Sequence Analysis of Full-length Human FATP1 and Full-length Human FATP4

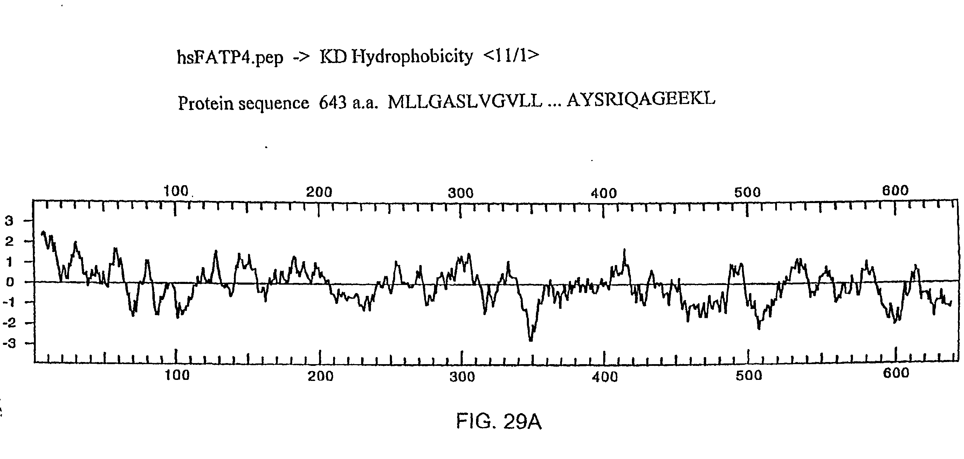

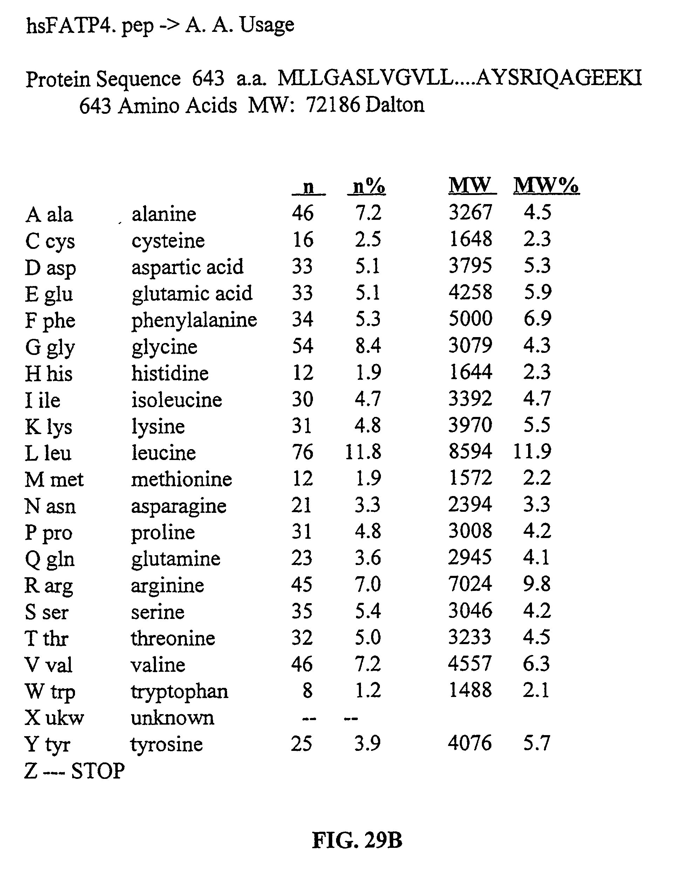

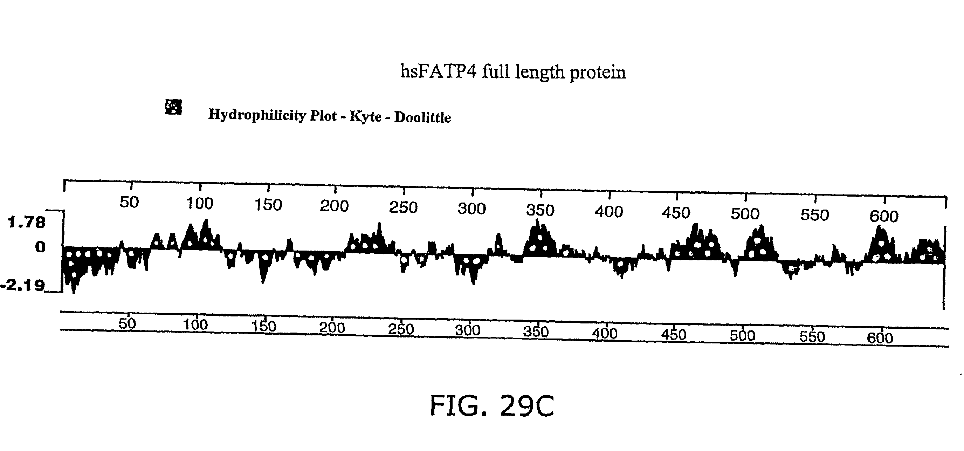

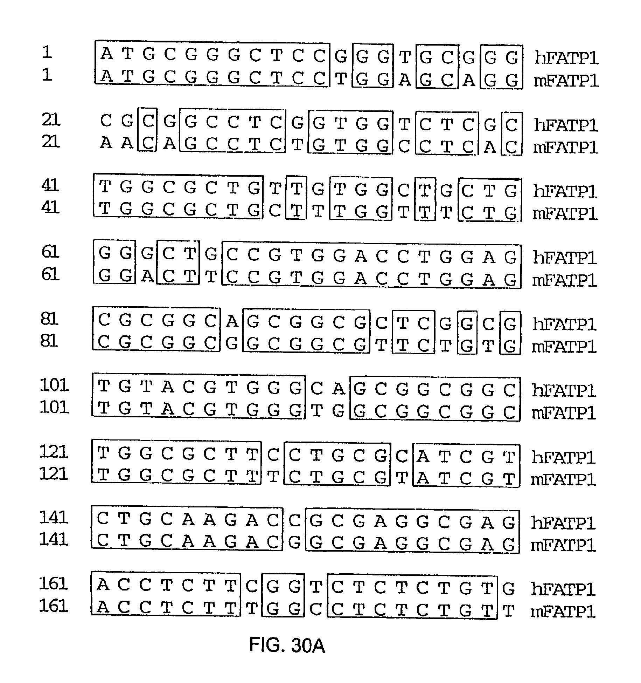

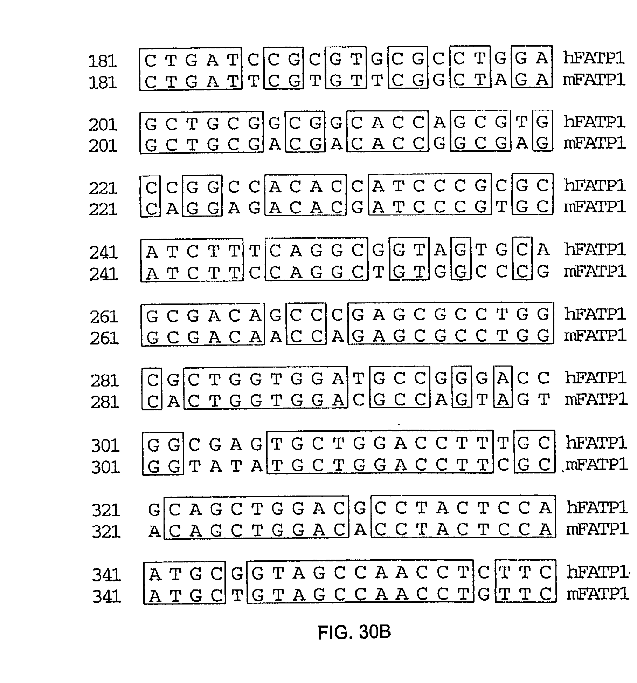

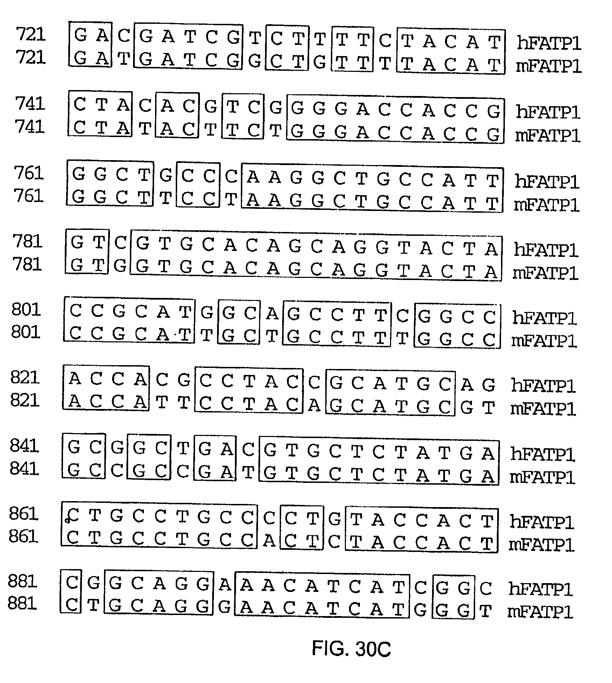

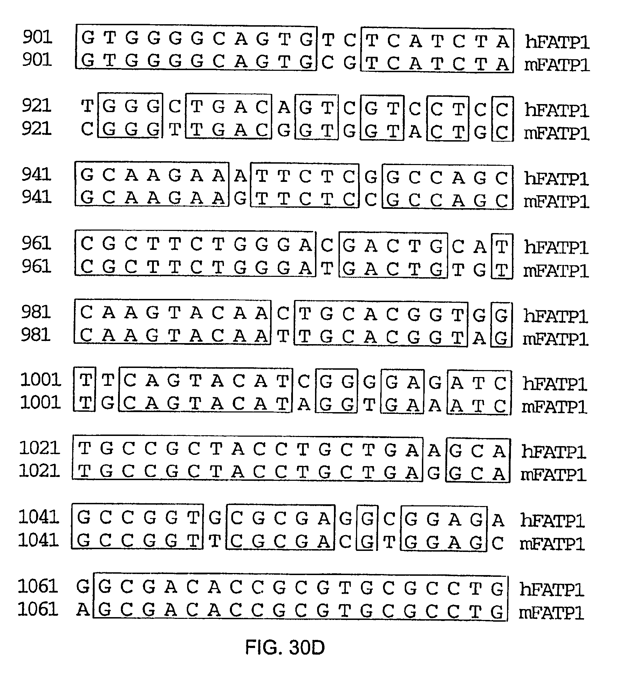

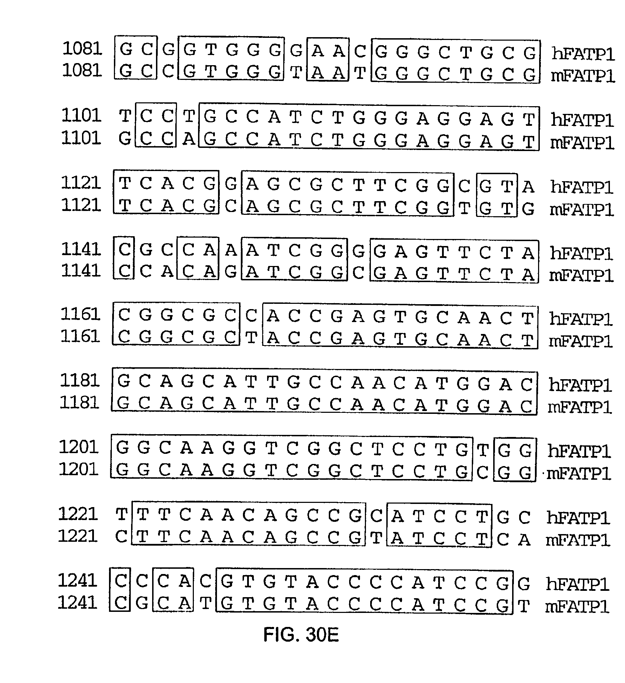

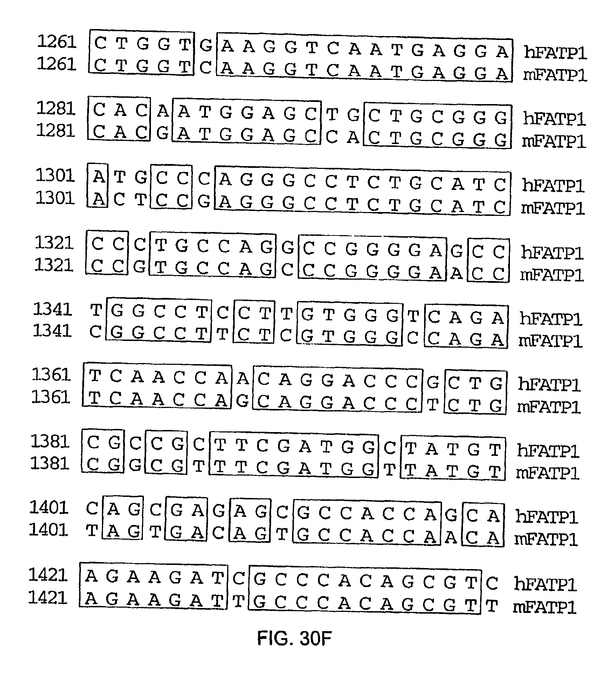

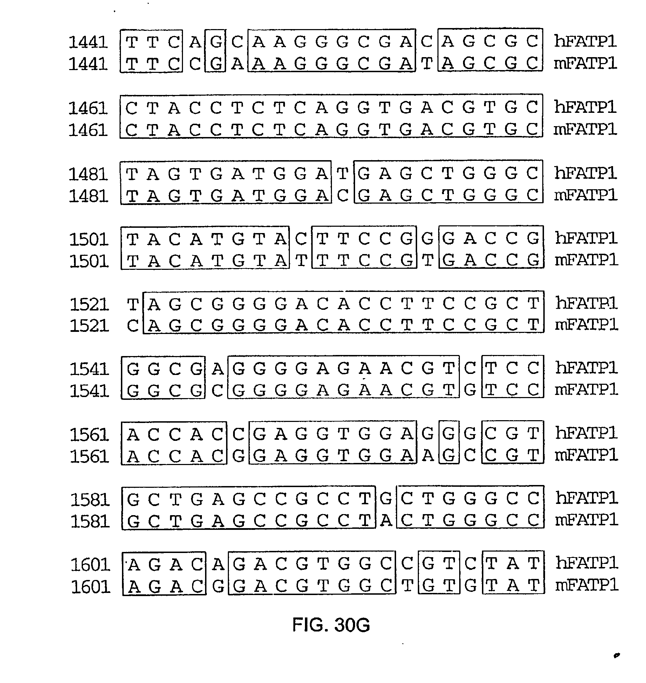

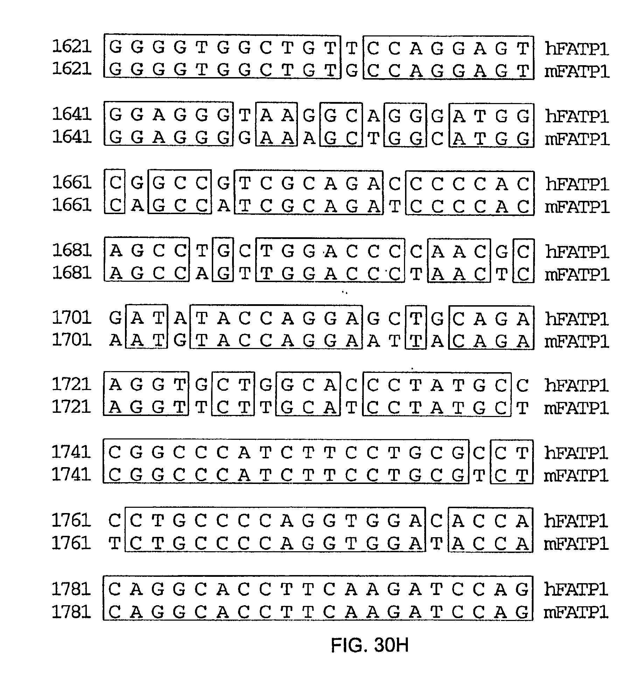

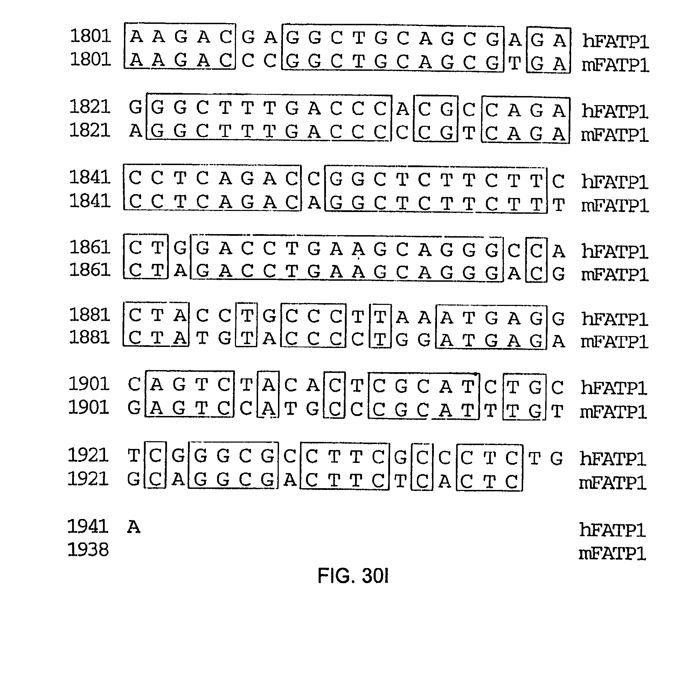

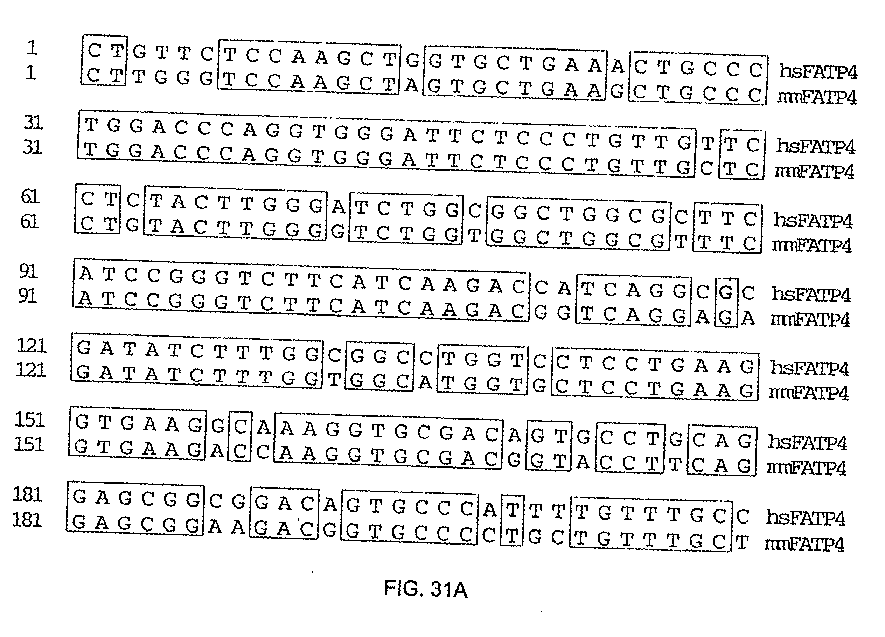

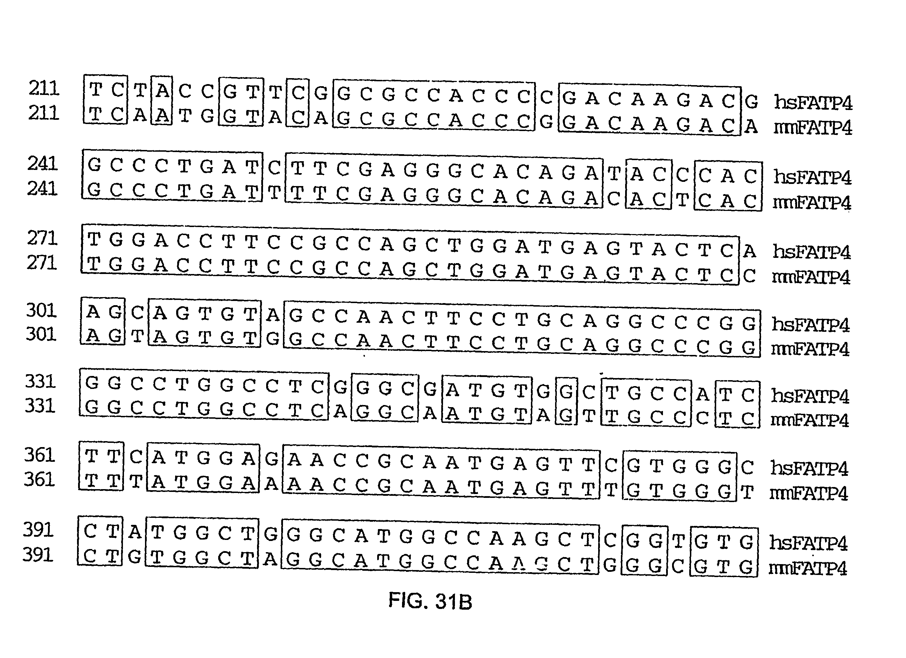

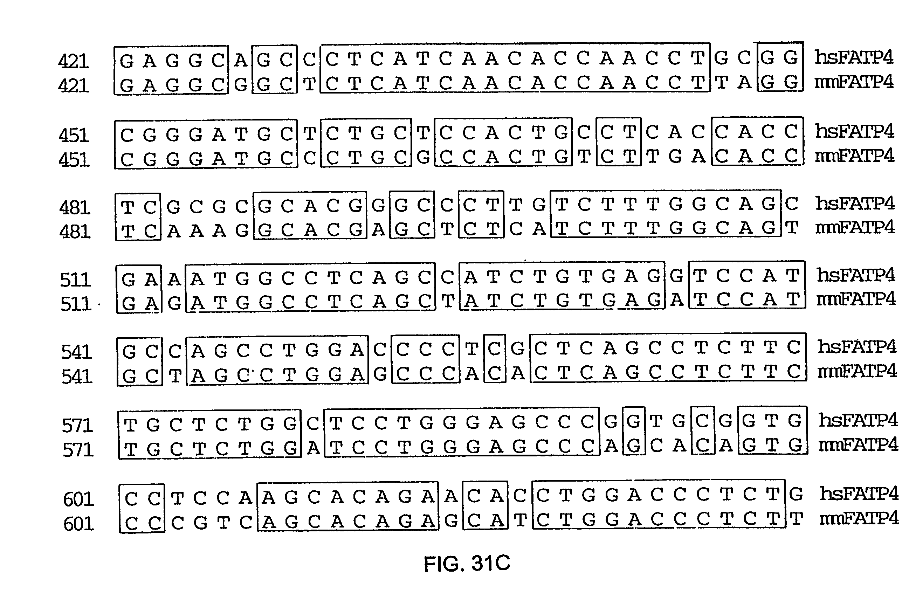

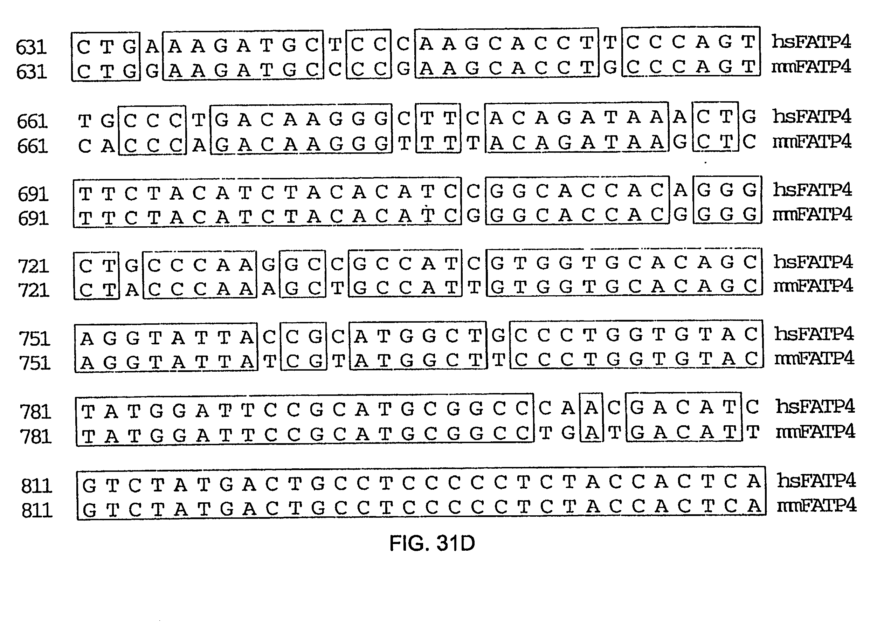

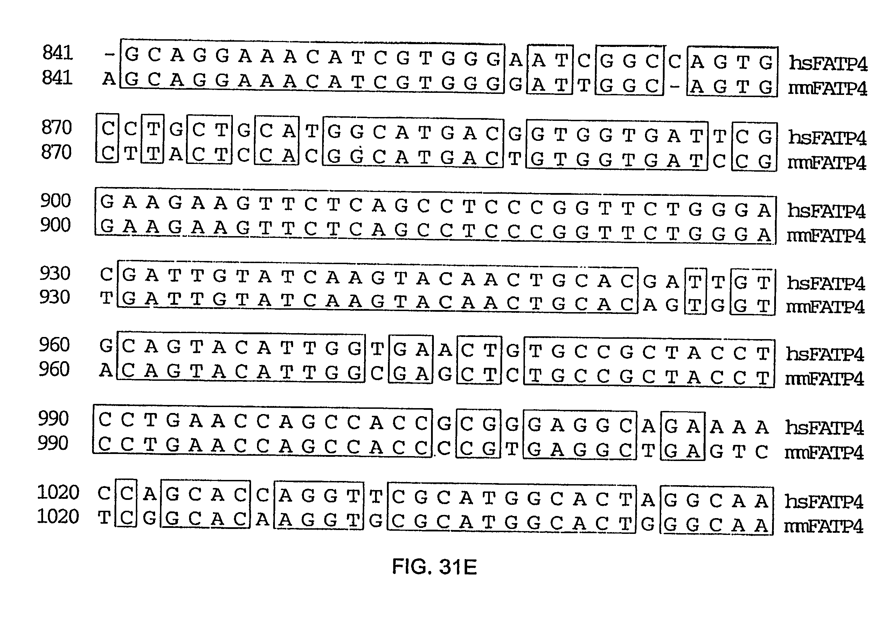

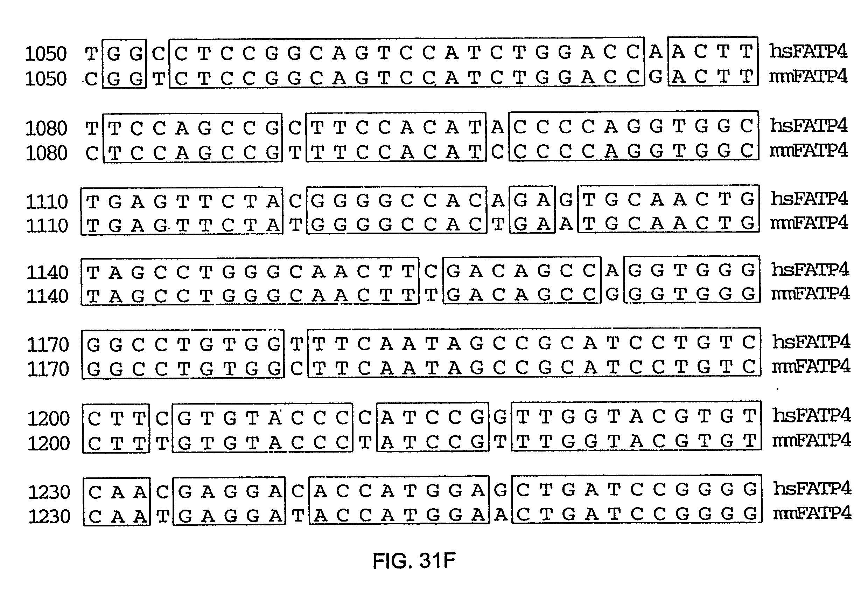

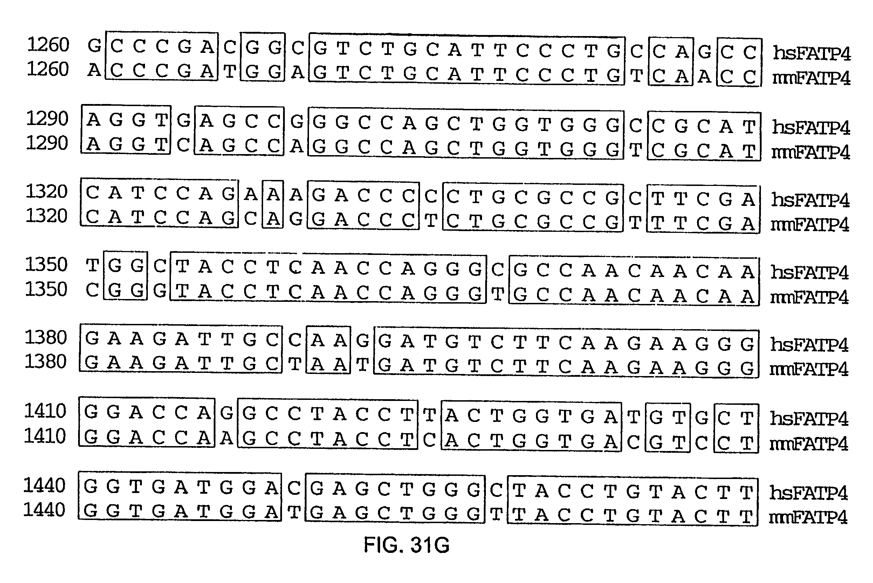

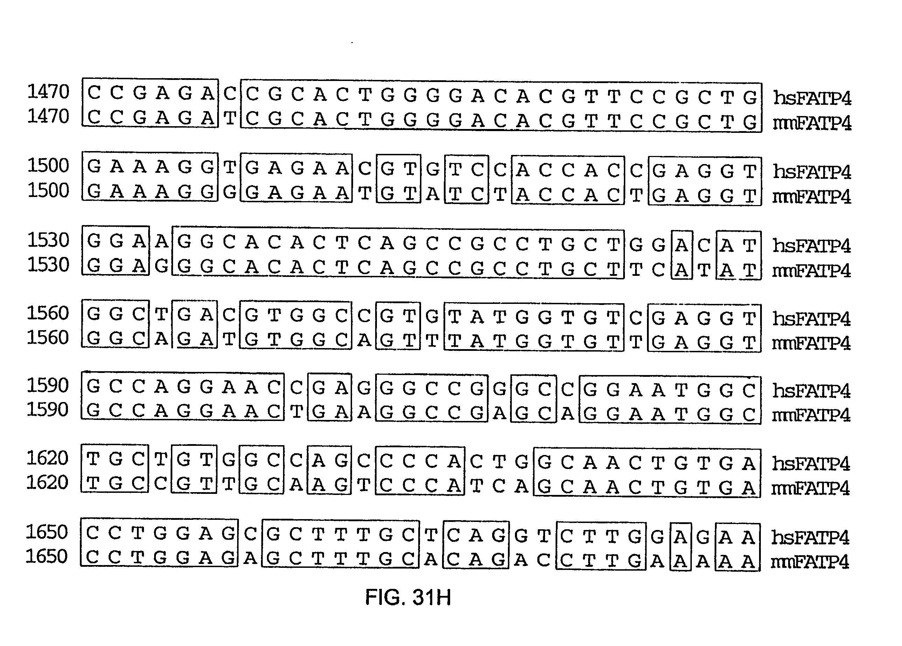

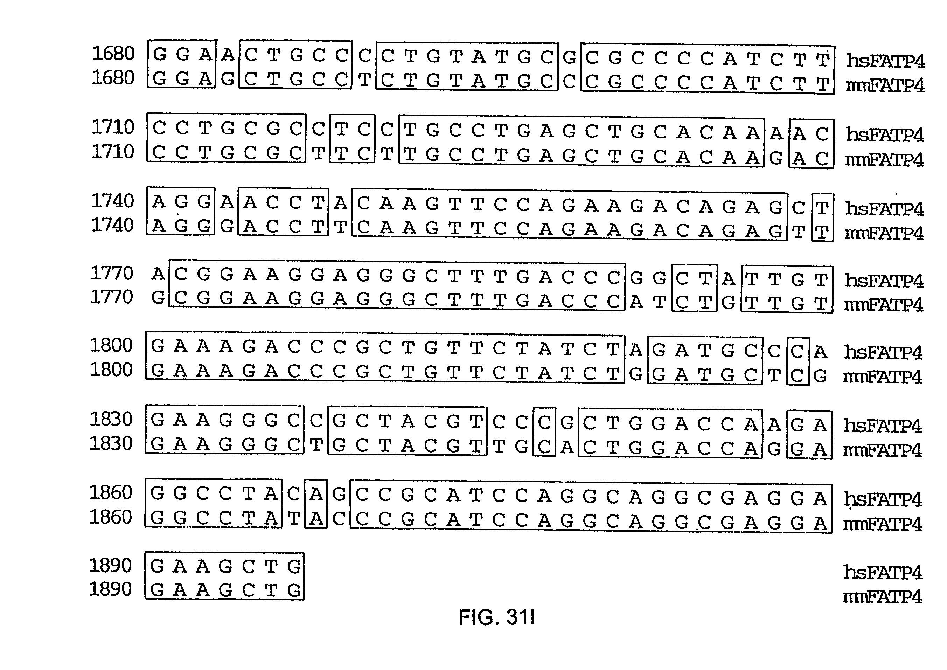

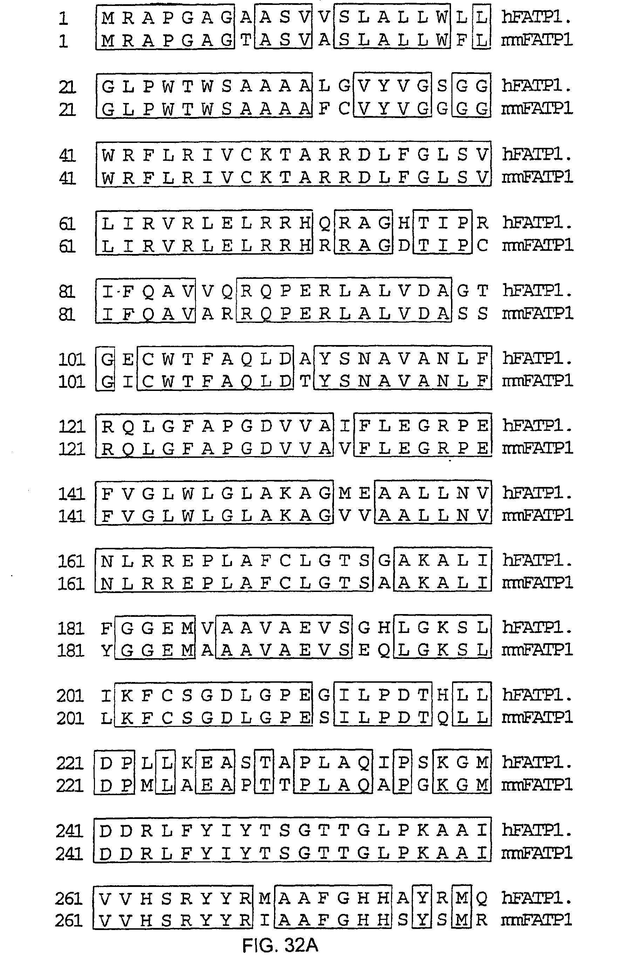

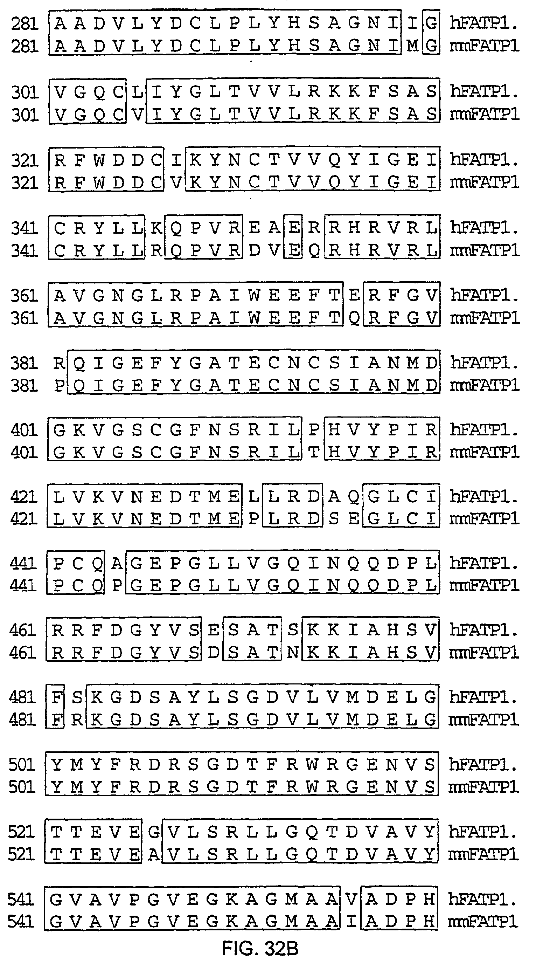

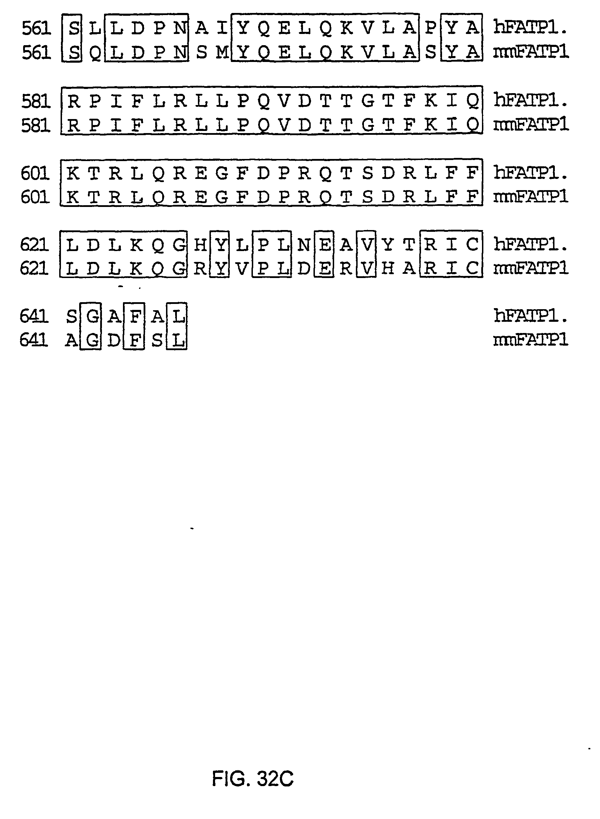

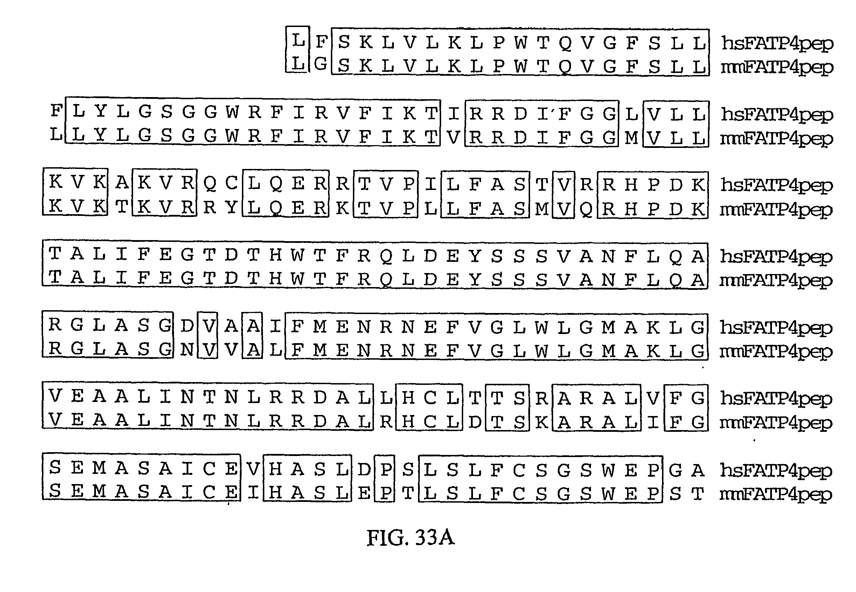

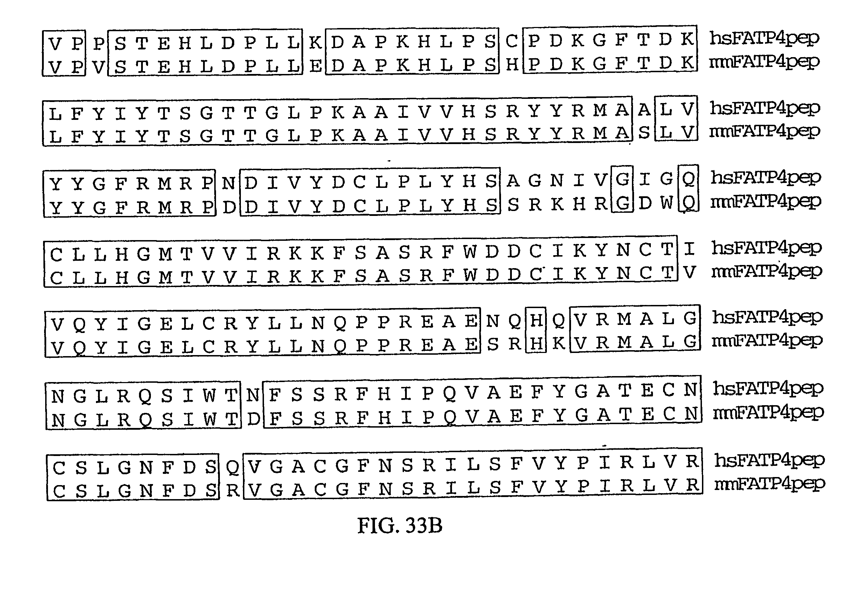

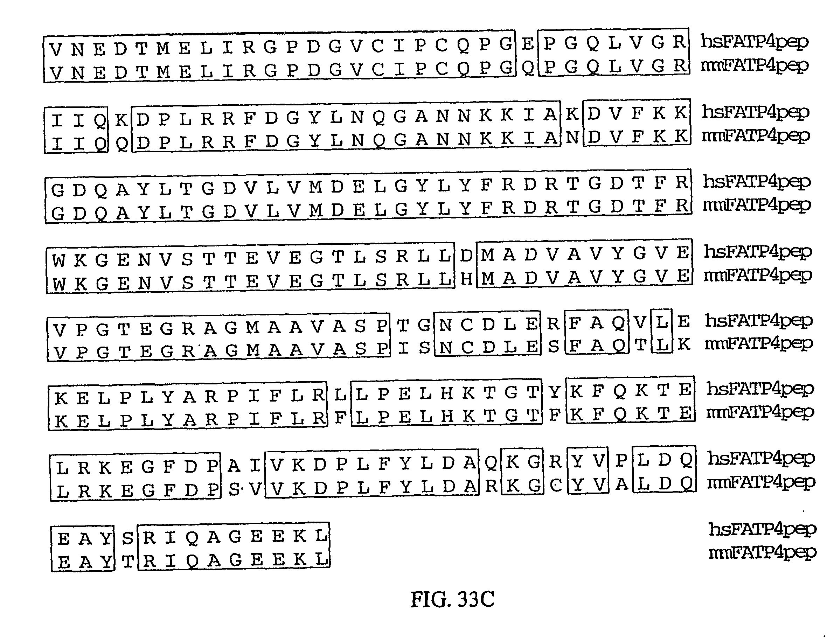

To identify human cDNA clones encoding FATP family members, Millennium databases were searched for sequences similar to murine FATP1-5 coding regions. Two clones were analyzed in detail; inspection of the entire DNA sequence of these two clones showed that they encode the human orthologs of mmFATP1 and mm FATP4, respectively. These two clones were designated hsFATP 1 and hsFATP4, and their DNA and predicted protein sequences are shown in Figures 44A-44C and 45, and 50A-50C and 51. hsFATP1 is predicted to encode a 646 amino acid, 71 kD protein with multiple membrane-spanning domains (Figure 28A). HsFATP4 is predicted to encode a 643 amino acid, 72 kD protein with multiple membrane spanning domains (See Figure 29A). A comparison of the DNA sequences of mouse and human FATP1 and mouse and human FATP4 (Figures 30A-30B and 31A-31B) shows that the mouse and human orthologs are 85% (FATP1) and 87% (FATP4) identical to each other within the coding sequences given in these figures. At the amino acid level, hsFATP 1 and hsFATP4 are ~90% identical to their respective mouse orthologs within the coding region shown in these figures (Figures 32 and 33). The sequence identities between mouse and human FATP1 1 and FATP4 are considerably higher than the ones observed between different FATP family members within one species (~40%-60%) and are present in the N-terminal part of the protein, a region that is poorly conserved between different FATP family members. This high degree of sequence conservation clearly demonstrates that the newly identified human FATPs are orthologs of mouse FATP1 and FATP4 rather than novel FATP family members.

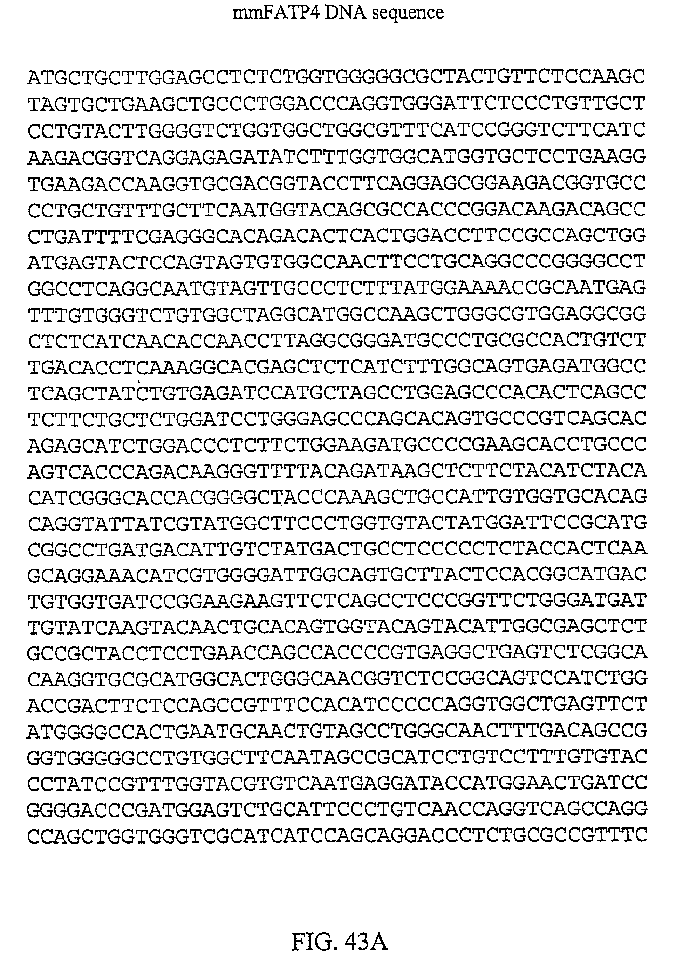

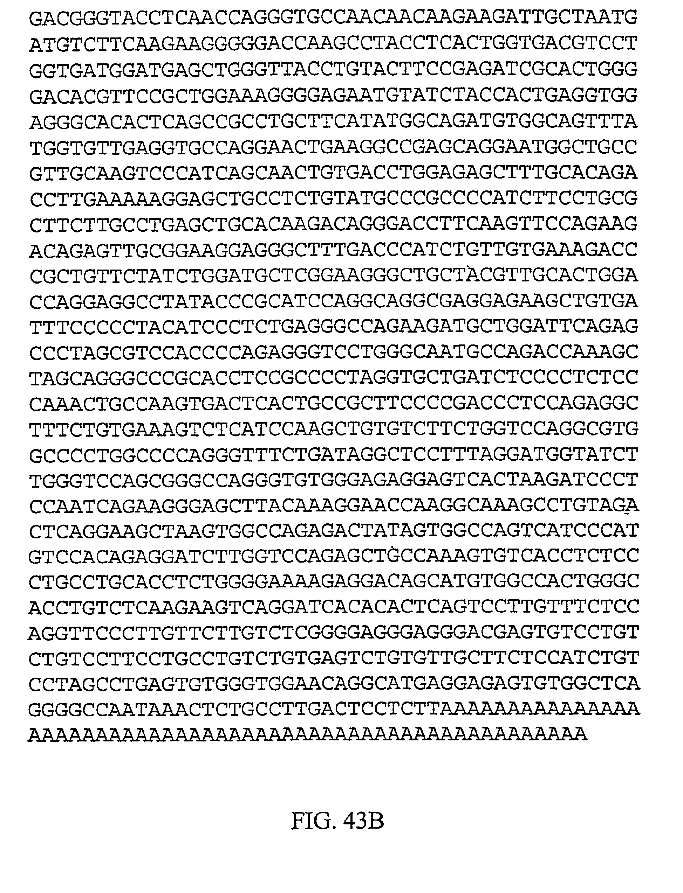

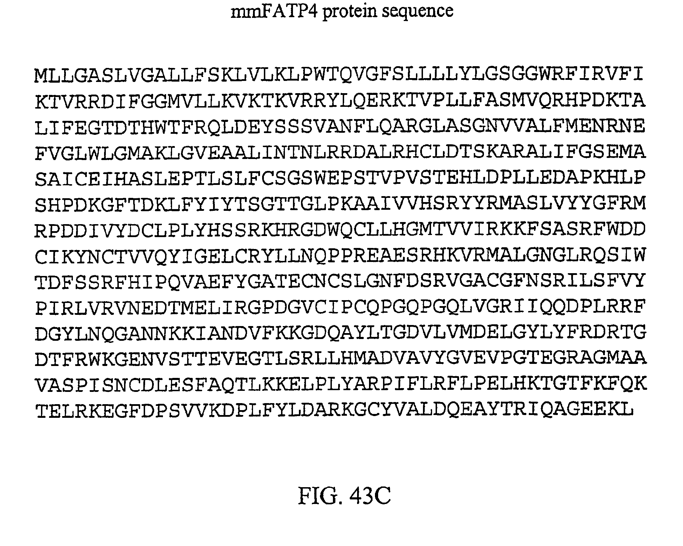

Table 4 is an identity/similarity matrix comparing the amino acid sequences of FATP and 4 from human and mouse. This shows that the gene whose sequence is shown in Figure 43A is indeed human FATP4, since it is 91 % identical with the murine FATP4 but only 62% identical with the closest related human FATP, which is FATP 1.

| Table 4 | | | | |
|---|---|---|---|---|
| Identity/Similarity Matrix | | | | |
| | hsFATP4 | mmFATP4 | hsFATP1 | mmFATP1 |
| hsFATP4 | --- | 93.2 | 72.3 | 72.0 |
| mmFATP4 | 91.0 | --- | 71.2 | 71.1 |
| hsFATP1 | 61.9 | 61.0 | --- | 92.4 |
| mmFATP1 | 60.7 | 59.6 | 89.5 | --- |

### Example 8: Isolation and Sequence Analysis of Full-length Human FATP6

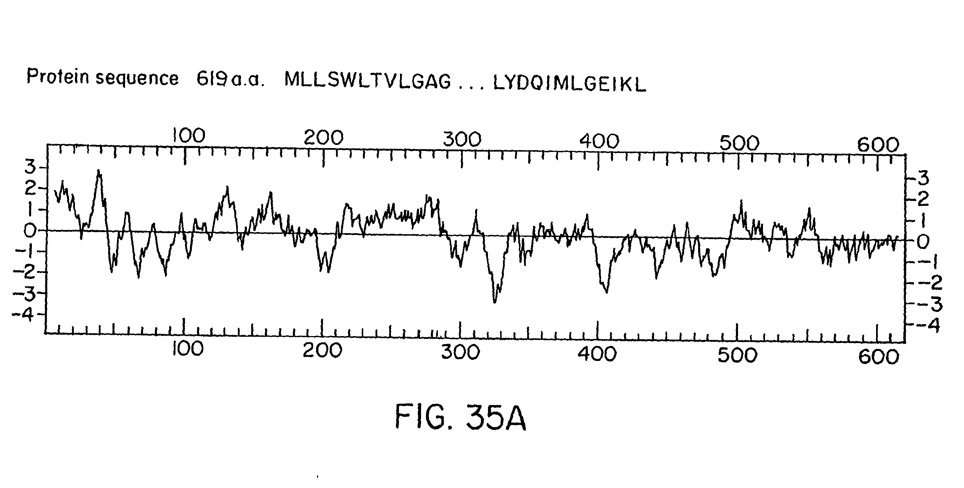

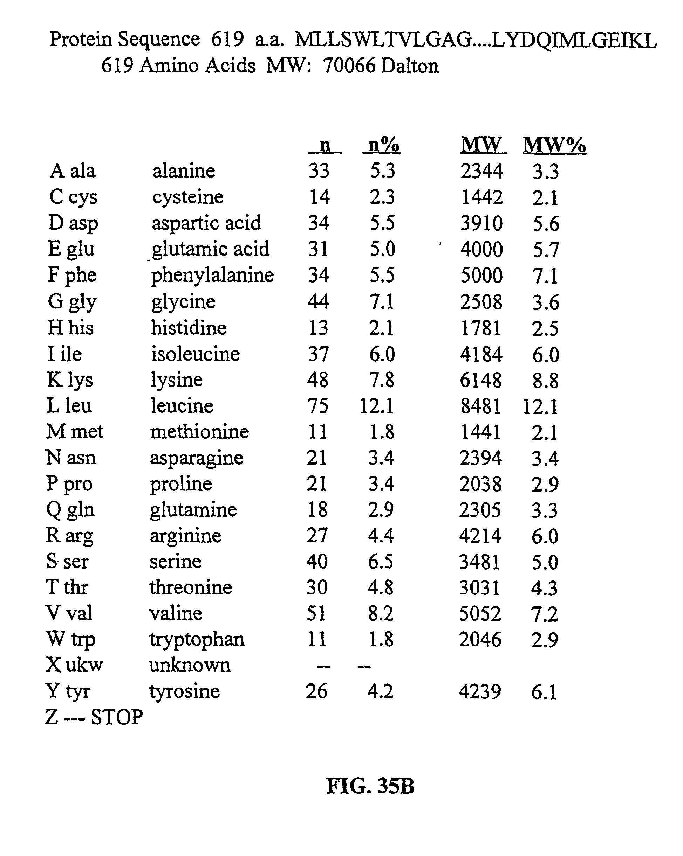

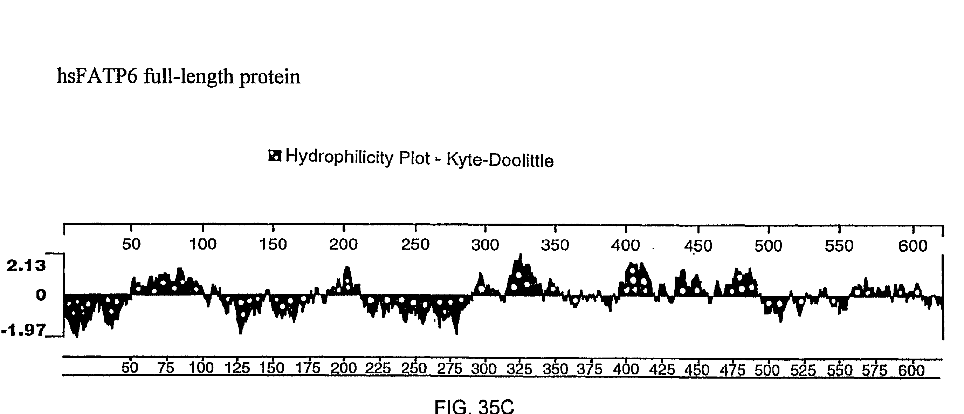

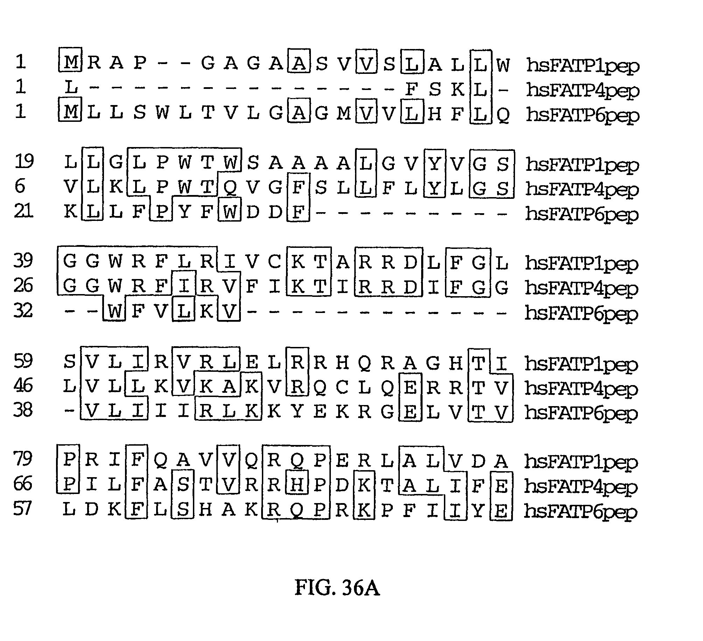

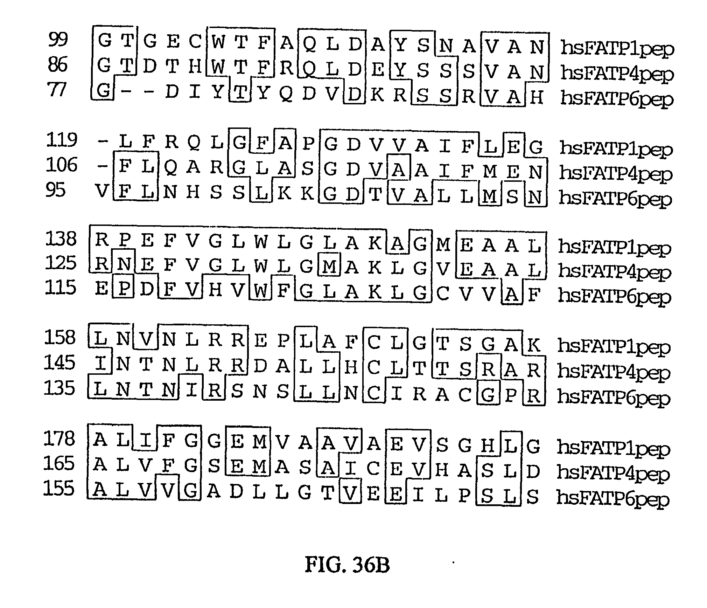

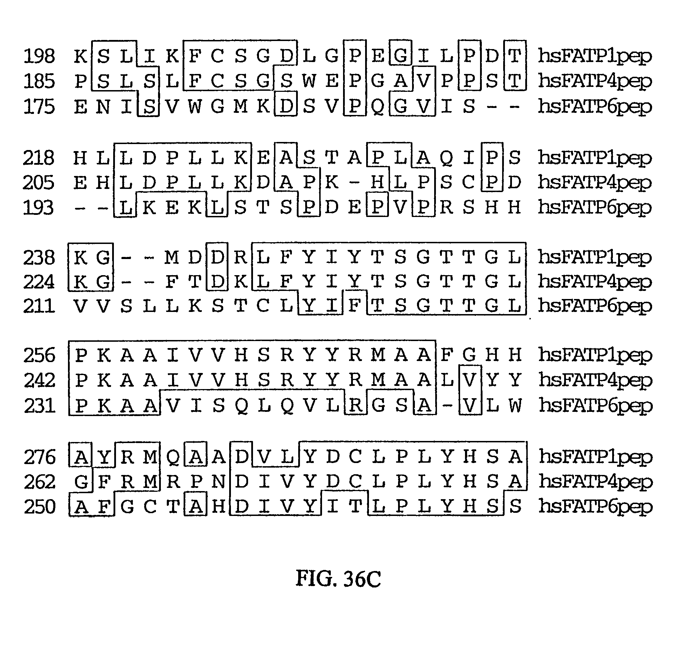

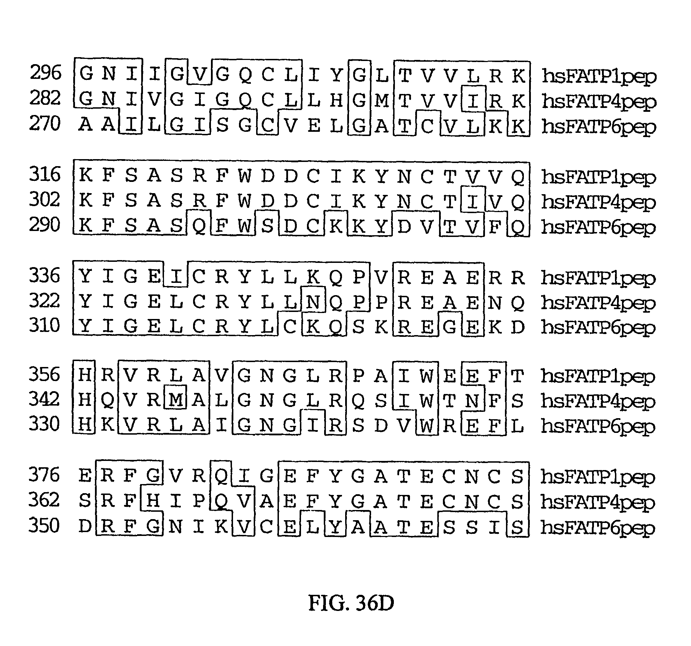

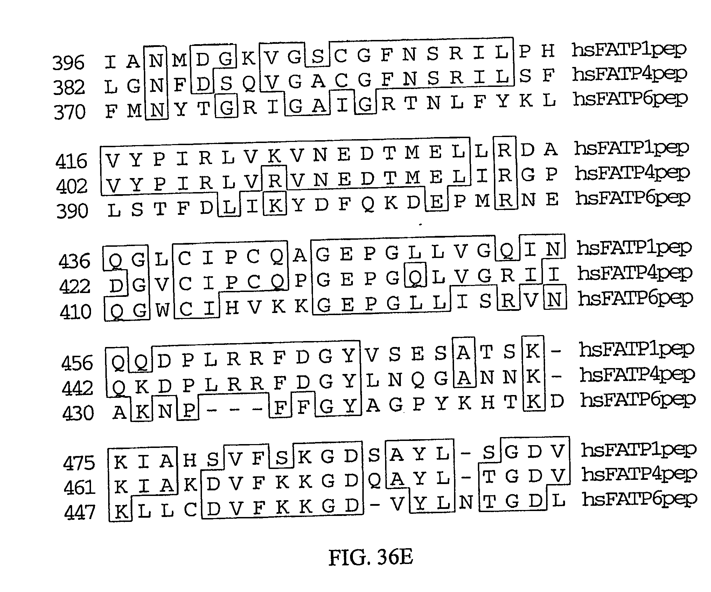

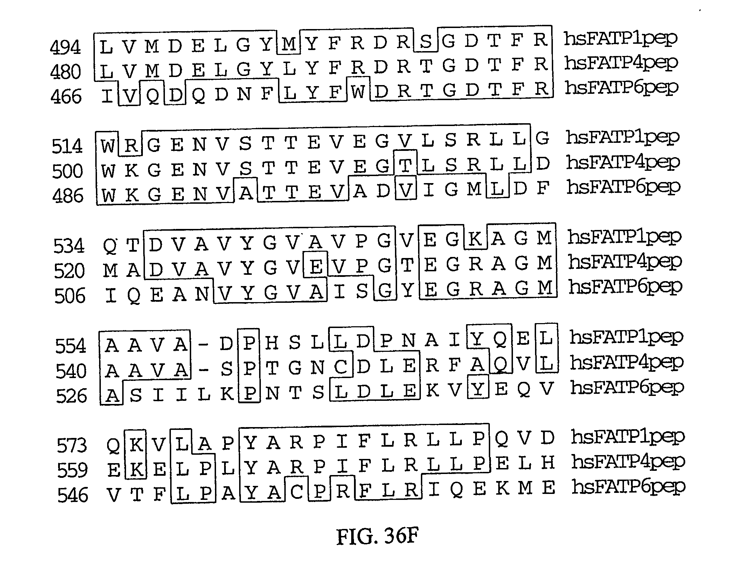

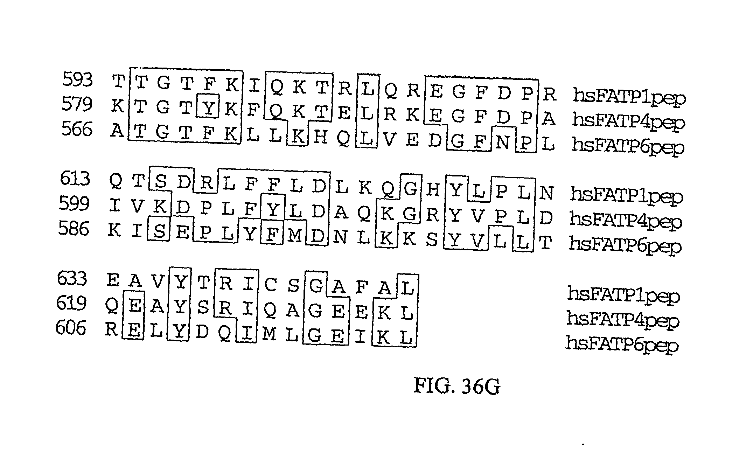

A search of EST databases identified a set of overlapping human sequences that were similar to FATPs, but did not have a clear mouse ortholog. One of these EST clones was found to encode a full-length cDNA. The entire insert of this clone was sequenced and designated hsFATP6. The DNA and predicted protein sequences of hsFATP6 are shown in Figures 54A-54C and 55. HsFATP6 is predicted to encode a 619 amino acid, 70 kD protein with multiple membrane-spanning domains (Figure 35A). A comparison of the amino acid sequences of hsFATP6 with other human FATPs shows about 37% identity to either hsFATP1 or hsFATP4 (Figure 36). This degree of sequence identity is similar to what is observed between different mouse FATPs. The phylogenetic analysis described above clearly demonstrates that hsFATP6 is a member of the FATP family, but not an ortholog of any of the mouse FATPs. Comparisons were done with "ALIGN" (E. Myers and W. Miller, "Optimal Alignments in Linear Space," *CABIOS 4*:11-17 (1988) using standard settings.

### Example 9: Tissue Distribution of Human FATPs

The tissue distribution of human FATPs was assessed by Northern blotting. Human FATP3 was expressed in a large variety of tissues. In contrast, human FATP5 was present at high levels in the liver, but was undetectable in all other tissues examined. Thus, both hsFATP3 and hsFATP5 recapitulate the expression pattern of their mouse orthologs (see above). HsFATP6 is a novel FATP with no mouse ortholog as yet. Northern blotting shows that hsFATP6 is expressed at high levels in the heart, but is undetectable in other tissues, including skeletal and smooth muscle. This tissue distribution suggests that human FATP6 performs an important role in energy metabolism in the heart; blocking FATP6-mediated fatty acid transport may therefore be beneficial for a number of heart diseases, e.g., ischemic heart disease.

To identify the major FATP expressed in the human small intestine, Northern blotting was performed on a blot containing mRNA from human stomach, jejunum, ileum, colon, rectum and lung. hsFATP5 and hsFATP6 were undetectable in any of these tissues. FATP5 is only expressed in liver and FATP6 only in heart. hsFATP2 was weakly expressed in the colon, and an even weaker signal was detectable in jejunum; ileum and lung lanes, hsFATP3 was expressed well in the lung, but was only weakly expressed in the other tissues tested. Importantly, no difference was seen in the expression of hsFATP3 between small intestine and stomach or colon, suggesting that the expression observed is not related to fatty acid absorption in the small intestine. hsFATP4 was clearly expressed in both jejunum and ileum; expression was significantly lower in the colon and was absent in the stomach. This expression pattern is consistent with a major role for FATP4 in absorption of fatty acids in the human gut.

### Example 10: Expression of hsFATP1 and hsFATP4 Promotes Transport of Fatty Acids

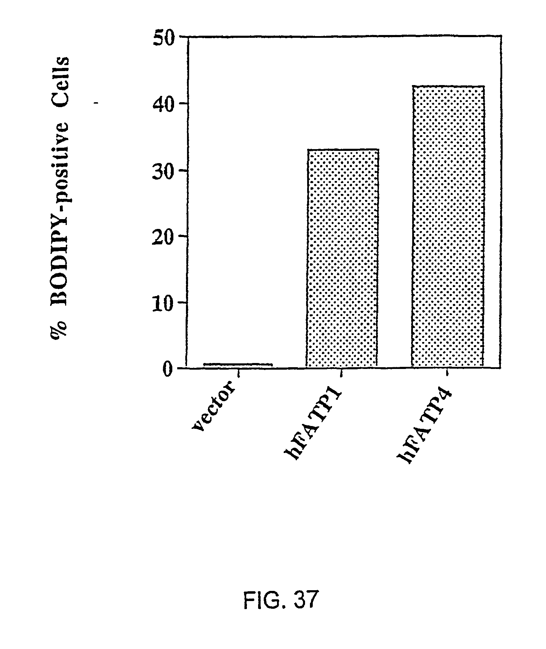

COS cells were cotransfected using lipofectamine with the mammalian expression vector pCDNA-CD2 in combination with one of the FATP-containing expression vectors (pMET7-hsFATP1 or pMET7-hsFATP4) or an insertless expression vector (pMET7, control) as described in Materials and Methods for Examples 6-10. COS cells were gated on forward scatter and side scatter. Cells exhibiting more than 400 CD2 fluorescence units representing ~30% of all cells were deemed CD2-positive. The percent of CD2-positive cells exhibiting a BODIPY-fluorescence of >300 is plotted for the three different vectors tested (Figure 37).

### Example 11: Stable Expression of Human FATP4 in 293 Cells

Stable cell lines were generated as follows. A DNA fragment containing the entire hsFATP4 coding sequence as well as 100 nucleotides of 5' and 50 nucleotides of 3' untranslated region was inserted into the vector pIRES-neo (Clontech) using standard cloning techniques. The resulting construct or a vector control (pIRES-neo) was transfected into 293 cells using the lipofectamine method (Gibco BRL) according to the manufacturer's directions. Cells that had taken up the DNA were selected with 1 mg/ml G418 (Gibco BRL). Single colonies were picked 1 to 2 weeks after transfection and grown in medium containing 0.8 mg/ml G418. Colonies were screened for the ability to take up fatty acids by measuring uptake of a fluorescently labeled fatty acid (BODIPY-FA). About 40 colonies transfected with the pIRES-neo containing FATP4 and ~20 colonies transfected with pIRES-neo control were analyzed. All 20 of the vector control clones showed amounts of BODIFY-FA uptake similar to each other and to untransfected 293 cells. In contrast, among the 40 FATP4 transfected clones, 3 had a 5-to 10-fold increased BODIPY-FA uptake compared to any of the vector controls, and a large number (~20) showed an approximately two-fold increase in BODIPY-FA levels. This distribution is consistent with FATP4 conferring increased fatty acid uptake in these cells. One of the cell lines with the highest amount of BODIPY-FA uptake was selected to be used for measuring uptake of tritiated fatty acid.

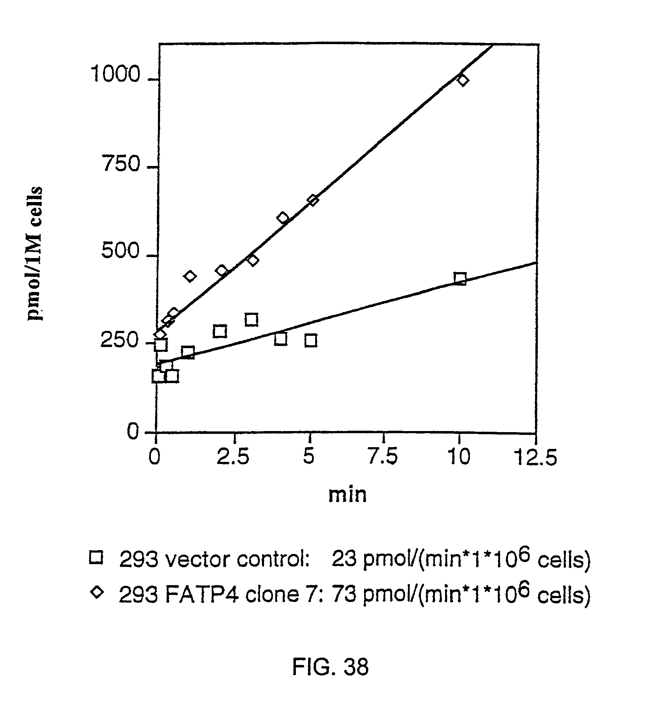

The uptake of tritiated oleate over time by either FATP4 expressing or control cells was assayed over time. Expression of FATP4 increases the rate of fatty acid uptake by over 3-fold, demonstrating that FATP4 is, like the other FATPs, a functional fatty acid transporter (Figure 38).

### Example 12: Immuno-staining with FATP4-Specific Antiserum

A polyclonal antiserum against the C-terminus of mmFATP4 was raised using a GST-fusion protein having mmFATP4-specific amino acid sequence 552-643 (A.VASP...GEEKL). In western blot experiments, the purified antibody reacted strongly with a synthetic peptide matching the C-terminus of mmFATP4, but not with a corresponding region of mmFATP2, mmFATP3, or mmFATP5. The mmFATP4 specific polyclonal antiserum detects, in western blot experiments with enterocyte lysates from 3 different mice, a ~70 kDa protein, which is in accordance with mmFATP4's predicted molecular weight of 72 kDa. The binding is specific for mmFATP4, since it can be completely abolished by preincubation of the antiserum with the GST-fusion peptide used to raise the antibody.

Immunofluorescence experiments were performed using the anti-mmFATP4 antiserum on fresh frozen sections of murine small intestine. The antibody binding demonstrates strong expression of mmFATP4 in enterocytes, confirming the results of the in situ hybridization experiments. At higher magnifications it is apparent that mmFATP4 is expressed at the apical side of the enterocyte, indicating that the transporter is present in the brush border membrane, which is known to mediate the uptake of fatty acids from the intestinal lumen.

Immuno-electron microscopy studies were performed on fresh frozen murine intestinal cells. The gold particles used, appearing as black specks on the electron micrographs, indicate the subcellular localization of mmFATP4 to be on the microvilli of the enterocyte. It can be seen from the electron micrographs that mmFATP4 is localized exclusively in membranes, preferentially the apical plasma membrane, confirming that it is indeed a membrane protein.

### Example 13: Inhibition of Fatty Acid Uptake Specific to FATP4 Demonstrated in Isolated Mouse Enterocytes

Phosphorothioate derivatives of the following oligonucleotides were synthesized:
FATP4-AS2 CCCCCACCAGAGAGGCTCC (SEQ ID NO: 100)
FATP4-AS2MM CCACCCCCGGAAAGCCTGC (SEQ ID NO:101)
FATP4-S2 GGAGCCTCTCTGGTGGGGG (SEQ ID NO: 102)
FATP4 AS2 is the antisense oligo; it is designed to be complementary to the sequence extending from nucleotide 10 to nucleotide 28 of the mouse FATP4 coding sequence. FATP4-AS2MM is a control oligo; in the oligo every third nucleotide was changed creating mismatches; the overall nucleotide composition is identical to FATP4-AS2 (same number of G, A, T, C). FATP4-S2 is the sense control.

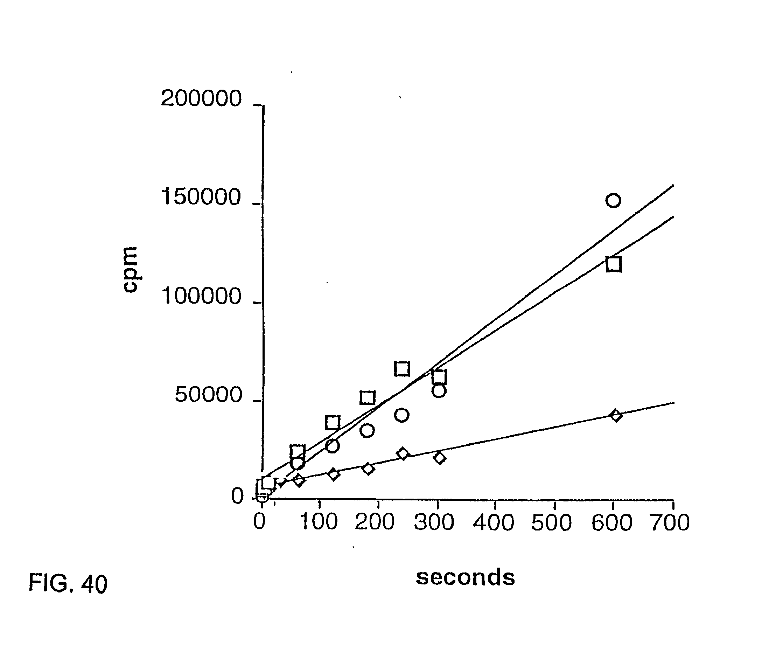

Enterocytes were isolated from the small intestine of mice and incubated for 48h in tissue culture (Figure 40) either without oligonucleotides (squares) or with 100 µM FATP4 specific sense (circles) or antisense (diamonds) oligonucleotides. The uptake over time of 25 µM oleate was then measured. While the FATP4 sense oligonucleotide did not significantly influence the uptake, the antisense oligonucleotide inhibited fatty acid uptake by ~50%.

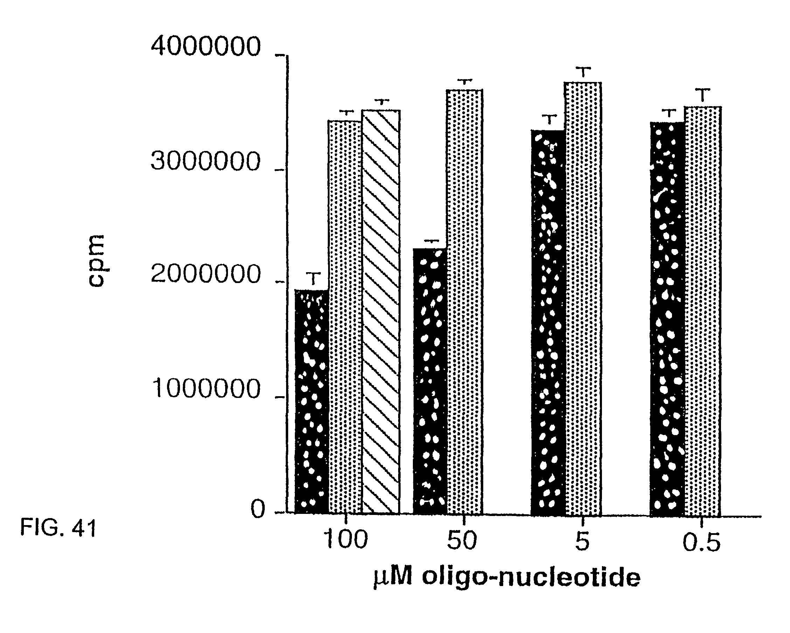

The effect of either FATP4 sense, antisense or mismatch sequence oligonucleotides on the uptake of fatty acids was measured in enterocytes. Isolated enterocytes were incubated with increasing concentrations of FATP4 antisense oligonucleotides (solid bars in Figure 41), or a mismatch control oligonucleotide with identical nucleotide composition (stippled bars), or with 100 µM of the FATP4 sense-oligonucleotide (lined bar). The medium for this incubation was Dulbecco's modified Eagle's medium with 4.5 g/L glucose, 1 mM sodium pyruvate, 0.01 mg/ml human transferrin and 10% fetal bovine serum. After 48 hours of incubation the uptake of oleate by enterocytes was measured over a 5 minute time interval. Measurements were done in quadruplicate. The uptake assay was done in Hank's buffered salt solution with 10 mM taurocholate. Only the enterocytes given FATP4 antisense oligonucleotide showed a concentration dependent decrease of fatty acid uptake, inhibiting it at a 100 µM concentration by ~50%. This effect was FATP4 specific, since only the antisense oligonucleotide which can bind to the FATP4 mRNA and block its translation inhibited uptake, but not a control oligonucleotide differing only in the sequence but not the nucleotide content, ruling out a toxic or otherwise nonspecific inhibitory effect of this oligonucleotide due to its chemical composition.

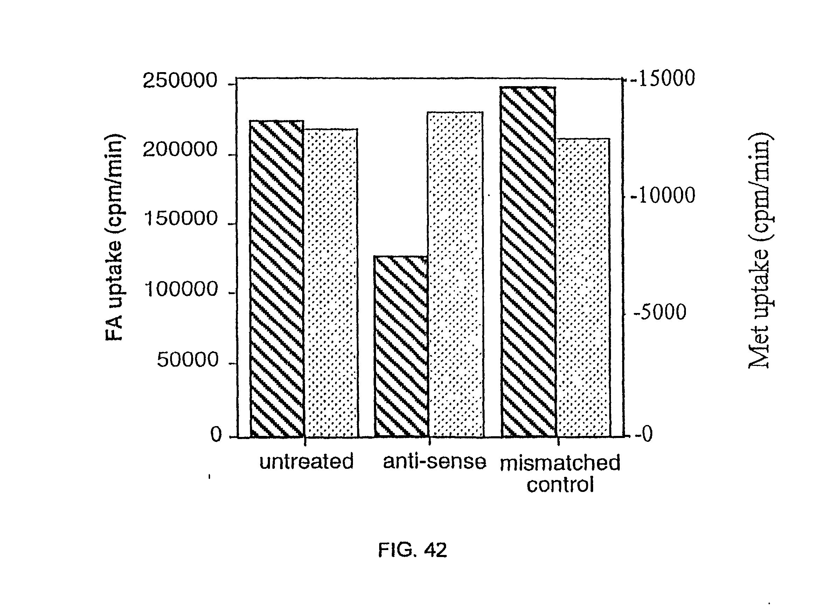

As a further control experiment, the uptake of oleate was measured along with the uptake of methionine in the same cultured enterocytes. Antisense oligonucleotide, mismatch sequence oligonucleotide, or no oligonucleotide was added to a concentration of 100 µM to cultures of enterocytes. After incubation for 48 hours, the uptake of both ³H-labeled oleate and ³⁵S-labeled methionine was assayed. Results are shown in Figure 42. Fatty acid uptake is at the left side of the paired bars; methionine uptake is on the right side of the paired bars. The fact that amino acid uptake was not influenced by the antisense oligonucleotide treatment further supports the conclusion that the antisense oligonucleotide causes a specific reduction in translation of FATP4-specific mRNA.

### Example 14: mmFATP2 Is Expressed in Proximal Renal Tubule Epithelium

Northern analysis showed that mmFATP1, mmFATP2, and mmFATP4 are present in the kidney. In situ hybridization (methods as for Example 6) was performed to determine which cell type(s) of the kidney these mRNAs are expressed in. mmFATP1 mRNA was present in virtually all cells throughout the kidney with no obvious preference for a particular cell type. In contrast, mmFATP2 was expressed only in the renal cortex. Within the cortex, expression of mmFATP2 was restricted to the epithelial cells of the proximal renal tubules. The primary function of proximal renal tubule cells is the reabsorption of filtered salts and nutrients (e.g., glucose), a process that requires mitochondrial oxidation and that can utilize fatty acids as energy substrates. Based on the localization of mmFATP2, it is possible that mmFATP2 is important for reabsorption in the kidney by allowing uptake of an energy source (fatty acids) from the blood into renal epithelial cells. Alternatively, if fatty acids need to be reabsorbed in the kidney, similarly to glucose, FATP2 could be involved in the reabsorption of fatty acids. Determination of the subcellular localization of FATP2 will distinguish between these two possibilities.

Table 5 summarizes data on expression of the mouse FATPs in various organs.

**Table 5. Mouse FATP mRNA Expression**

| Mouse Probes | mFATP1 | mFATP2 | mFATP3 | mFATP4 | mFATP5 |
|---|---|---|---|---|---|
| E18.5 embryo expression | everywhere, brain = thymus> heart> brown fat, others | liver (hepatocytes) | - | Brain, small intestine, superior cervical ganglion (SCG), dorsal root ganglion (DRG), other regions have lower expression | Mouse Probes |
| Duodenum | - | villi (surface epithelium) | - | villi (surface epithelium) | - |
| Jejunum | - | villi (surface epithelium) | - | villi (surface epithelium) | - |
| Ileum | - | villi (surface epithelium) | - | villi (surface epithelium) | - |
| Colon | low expression in the crypt | very low level in the crypt | - | - | - |
| Kidney | cortex and medulla | proximal tubules | - | - | - |
| Liver | - | hepatocytes | hepatocytes | - | hepatocytes |
| Pancreas | exocrine secretory units or acinar cells; endocrine pancreas (islet) are negative | exocrine secretory units or acinar cells; endocrine pancreas (islet) are negative | - | - | - |
| Brain | Neuronal expression throughout the brain including hypothalamus | - | - | Neuronal expression throughout the brain including hypothalamus | - |
| Heart | myocytes | - | - | | |
| Testis | seminiferous tubules | - | seminiferous tubules | | |
| Lung | bronchiole | - | - | | |
| Adipose | adipocyte | adipocyte | - | | |

### Example 15: Isolation of full-length human FATP3

Full-length clones encoding human FATP3 were identified by searching databases for sequences similar to the murine FATP1-5 coding regions using the BlastX algorithm (Altschul *et al., J. Mol. Biol. 215:* 403-410, 1990). Human clones with similarity to the 5' end of murine FATP sequences were sequenced completely. A clone encoding full-length human FATP3 was obtained from a human bone library constructed in the mammalian expression vector pMET7 (Tartaglia, L.A. *et al., Cell 83:* 1263-1271, 1995). To identify human cDNA clones encoding FATP family members, databases were searched for sequences similar to murine FATP1-5 coding regions. One clone was found to encode the human ortholog of mmFATP3 and was designated hsFATP3. The DNA and predicted protein sequences of hsFATP3 are shown in Figures 94A and 94B. hsFATP5 is predicted to encode a 703 amino acid 75.6 kD protein with multiple membrane-spanning domains. A comparison of the DNA sequences of mouse and human FATP3 shows that the mouse and human orthologs are 81 % identical to each other within the coding region. At the amino acid level, hsFATP3 is~ 86% identical to mm FATP3 within the coding region. The sequence identities between mouse and human FATP3 are considerably higher than those observed between different FATP family members within one species (-40%) and are present in the N-terminal part of the protein, a region that is poorly conserved between different FATP family members.

While this invention has been particularly shown and described with references to preferred embodiments thereof, it will be understood by those skilled in the art that various changes in form and details may be made therein without departing from the scope of the invention as defined by the appended claims.

## Claims

1. An isolated polypeptide that:
a) comprises the amino acid sequence of FATP4 in SEQ ID NO: 53;
b) consists of the amino acid sequence of SEQ ID NO: 53; or
c) comprises at least 360 contiguous amino acids residues of SEQ ID NO: 53, wherein the polypeptide has FATP4 activity.

2. A fusion protein comprising a polypeptide as defined in Claim 1, when fused with a heterologous moiety.

3. The fusion protein of Claim 2 comprising an affinity ligand.

4. The fusion protein of Claim 2 or Claim 3, wherein the fusion protein transports fatty acids across a cell membrane or an artificial cell membrane system.

5. An isolated nucleic acid that:
a) encodes a polypeptide according to Claim 1, or the complement of a nucleic acid that encodes the polypeptide;
b) comprises the nucleotide sequence of SEQ ID NO: 52, or the complement thereof;
c) consists of the nucleotide sequence of SEQ ID NO: 52, or the complement thereof; and/or
d) comprises the nucleotide sequence of the coding region of SEQ ID NO: 52.

6. An isolated nucleic acid molecule comprising a nucleotide sequence encoding a fusion protein according to any of Claims 2 to 4.

7. A vector comprising a nucleic acid molecule according to Claim 5 or Claim 6, or encoding a polypeptide according to Claim 1 or a fusion protein according to any of Claims 2 to 4.

8. An isolated host cell comprising the vector of Claim 7.

9. A non-human host cell comprising the vector of claim 7.

10. A method for producing a polypeptide according to Claim 1 or a fusion protein according to any of Claims 2 to 4, comprising culturing the host cell of Claim 8 or 9 under conditions in which the nucleic acid is expressed, thereby producing the polypeptide or fusion protein.

11. A method for identifying an agent which binds to a polypeptide according to Claim 1 comprising the steps of contacting the agent with the isolated polypeptide under conditions appropriate for binding of the agent to the isolated polypeptide, and detecting a resulting agent-polypeptide complex.

12. A method for identifying an agent which inhibits interaction between a polypeptide according to Claim 1 and a ligand of said polypeptide, comprising:
(a) combining:
(1) said isolated polypeptide;
(2) the ligand of said polypeptide; and
(3) a candidate agent to be assessed for its ability to inhibit binding between said polypeptide of (1) and the ligand of (2), under conditions appropriate for binding between the said polypeptide of (1) and the ligand of (2);
(b) determining the extent to which said polypeptide of (1) and the ligand of (2) bind; and
(c) comparing the extent determined in (b) with the extent to which binding of said polypeptide of (1) and the ligand of (2) occurs in the absence of the candidate agent to be assessed and under the same conditions appropriate for binding of said polypeptide of (1) with the ligand of (2);
wherein if the extent to which binding of said polypeptide of (1) and the ligand of (2) occurs is less in the presence of the candidate agent than in the absence of the candidate agent, the candidate agent is an agent which inhibits binding between said polypeptide and the ligand of said polypeptide.

13. The method of Claim 11, wherein the step of contacting the agent with the polypeptide is performed in an artificial membrane system, or the method of Claim 12, wherein step (a) is performed in an artificial membrane system.

14. The method of Claim 11 or Claim 12, wherein the isolated polypeptide is in an isolated plasma membrane.

15. A method for identifying an agent which is an inhibitor of fatty acid uptake by a polypeptide according to Claim 1, comprising the steps of:
a) maintaining test cells expressing said polypeptide in the presence of a fatty acid and an agent to be tested as an inhibitor of fatty acid uptake;
b) measuring uptake of the fatty acid in the test cells; and
c) comparing uptake of the fatty acid in the test cells with uptake of the fatty acid in suitable control cells;
wherein lower uptake of the fatty acid in the test cells compared to uptake of the fatty acid in the control cells is indicative that the agent is an inhibitor of fatty acid uptake by said polypeptide.

16. A method for identifying an agent which is an inhibitor of a polypeptide according to Claim 1, comprising the steps of:
(a) introducing into host cells one or more vectors comprising a polynucleotide expressing said polypeptide;
(b) culturing a first aliquot of the host cells with fatty acid substrate of said polypeptide and with an agent being tested as an inhibitor of said polypeptide;
(c) culturing a second aliquot of the host cells with fatty acid substrate of said polypeptide;
(d) measuring, in the first and second aliquots, uptake of the fatty acid substrate of the host cells;
wherein less uptake of the fatty acid substrate in the first aliquot compared to the second aliquot is indicative that the agent is an inhibitor of said polypeptide.

17. A method for identifying an agent which is an inhibitor of a polypeptide according to Claim 1, comprising the steps of:
(a) introducing into cells one or more vectors comprising a polynucleotide encoding said polypeptide;
(b) contacting the host cells with anti-cell surface protein antibody and labeled fatty acid substrate of FATP4;
(c) contacting a first aliquot of the host cells with an agent being tested as an inhibitor of FATP4, while leaving a second aliquot of the host cells uncontacted with the agent;
(d) identifying, in the first and second aliquots, the host cells expressing the cell surface protein by detecting the anti-cell surface protein antibody bound to the host cells; and
(e) measuring, in the first and second aliquots, uptake of the fatty acid substrate of the host cells identified as expressing the cell surface protein;
wherein less uptake of the fatty acid substrate in the first aliquot compared to the second aliquot is indicative that the agent is an inhibitor of FATP4.

18. The method of Claim 17, wherein the host cells are prokaryotes.

19. The method of Claim 17 or 18, wherein the fatty acid is a radioactively labeled fatty acid.

20. The method of any of Claims 17 to 19, wherein the cell surface protein is CD2.

21. The method of any of Claims 17 to 20 wherein the fatty acid substrate is BODIPY-labeled.

22. The method of any of Claims 17 to 21 wherein the host cells regulably express the polynucleotide encoding said polypeptide.

23. The method of any of Claims 12 to 22 further comprising the steps of:
a) administering the agent to one or more test animals;
b) measuring exogenously supplied fatty acids in one or more samples of tissue or bodily fluid from said test animals;
c) measuring exogenously supplied fatty acids in one or more comparable samples of tissue or bodily fluid from suitable control animals;
d) comparing the fatty acids of b) with the fatty acids of c);
whereby lower fatty acids in step b) than in step c) is indicative that the agent is an inhibitor of said polypeptide.

24. A method for detecting FATP4 in a sample of cells or a sample of cell lysate comprising the steps of adding an agent that specifically binds to FATP4 to the sample, and detecting agent specifically bound to FATP4, wherein the agent is an antibody that binds to FATP4.

25. An isolated antibody or antigen-binding fragment thereof that specifically binds to a polypeptide according to Claim 1.

26. The isolated antibody of Claim 25, wherein the antibody is a polyclonal antibody.

27. The isolated antibody of Claim 25, wherein the antibody is a monoclonal antibody.

28. A method for modulating fatty acid uptake of cells in culture, comprising adding one or more agents that modulate fatty acid uptake by FATP4 to cells comprising FATP4, wherein the agent is a nucleic acid of claim 5; an antisense oligonucleotide to FATP4, or an antibody that binds to the polypeptide of Claim 1.

29. A polypeptide according to Claim 1, a fusion protein according to any one of Claims 2 to 4, a nucleic acid molecule according to Claim 6, a vector according to Claim 7, or an antibody according to any one of Claims 25 to 27; for use in medicine.

30. A pharmaceutical composition comprising a polypeptide according to Claim 1, a fusion protein according to any one of Claims 2 to 4, a nucleic acid according to Claim 6, a vector according to Claim 7, or an antibody according to any one of Claims 25 to 27.

31. The use of an agent which is an inhibitor of fatty acid uptake by FATP4 protein in the small intestine in the preparation of a medicament for treating a disorder **characterized by** excessive fatty acid uptake in the small intestine, wherein the agent comprises an antisense oligonucleotide or an antibody according to any one of Claims 25 to 27.

32. The use of Claim 31, wherein the medicament is for oral administration.

## Patentansprüche

1. Isoliertes Polypeptid, das:
a) die Aminosäuresequenz von FATP4 in SEQ ID NO: 53 umfasst;
b) die Aminosäuresequenz von SEQ ID NO: 53 umfasst oder
c) mindestens 360 aneinandergrenzende Aminosäurereste von SEQ ID NO: 53 umfasst, wobei das Polypeptid FATP4-Aktivität aufweist.

2. Fusionsprotein, das ein wie in Anspruch 1 definiertes Polypeptid umfasst, wenn es mit einem heterologen Teil fusioniert wird.

3. Fusionsprotein nach Anspruch 2, das einen Affinitätsliganden umfasst.

4. Fusionsprotein nach Anspruch 2 oder 3, wobei das Fusionsprotein Fettsäuren über eine Zellmembran oder ein künstliches Zellmembransystem transportiert.

5. Isolierte Nukleinsäure, die:
a) ein Polypeptid nach Anspruch 1 kodiert, oder das Komplement einer Nukleinsäure, die das Polypeptid kodiert;
b) die Nukleotidsequenz von SEQ ID NO: 52 oder das Komplement davon umfasst;
c) aus der Nukleotidsequenz von SEQ ID NO: 52 oder dem Komplement davon besteht und/oder
d) die Nukleotidsequenz der kodierenden Region von SEQ ID NO: 52 umfasst.

6. Isoliertes Nukleinsäuremolekül, das eine Nukleotidsequenz umfasst, die ein Fusionsprotein nach einem der Ansprüche 2 bis 4 kodiert.

7. Vektor, der ein Nukleinsäuremolekül nach Anspruch 5 oder 6 umfasst oder ein Polypeptid nach Anspruch 1 oder ein Fusionsprotein nach einem der Ansprüche 2 bis 4 kodiert.

8. Isolierte Wirtszelle, die den Vektor nach Anspruch 7 umfasst.

9. Nichtmenschliche Wirtszelle, die den Vektor nach Anspruch 7 umfasst.

10. Verfahren zum Herstellen eines Polypeptids nach Anspruch 1 oder eines Fusionsproteins nach einem der Ansprüche 2 bis 4, wobei das Verfahren das Kultivieren der Wirtszelle nach Anspruch 8 oder 9 unter Bedingungen, in denen die Nukleinsäure exprimiert wird, umfasst, wodurch das Polypeptid oder das Fusionsprotein hergestellt wird.

11. Verfahren zum Identifizieren eines Agens, das an ein Polypeptid nach Anspruch 1 bindet, wobei das Verfahren die Schritte des In-Kontakt-Bringens des Agens mit dem isolierten Polypeptid unter Bedingungen, die zur Bindung des Agens an das isolierte Polypeptid geeignet sind, und des Nachweisens eines resultierenden Agens/Polypeptid-Komplexes umfasst.

12. Verfahren zum Identifizieren eines Agens, das die Interaktion zwischen einem Polypeptid nach Anspruch 1 und einem Liganden des Polypeptids inhibiert, wobei das Verfahren Folgendes umfasst:
(a) Vereinen:
(1) des isolierten Polypeptids;
(2) das Liganden des Polypeptids und
(3) eines Kandidatenagens, das auf seine Fähigkeit zum Inhibieren der Bindung zwischen dem Polypeptid von (1) und dem Liganden von (2) beurteilt werden soll, unter Bedingungen, die zur Bindung zwischen dem Polypeptid von (1) und dem Liganden von (2) geeignet sind;
(b) Bestimmen des Ausmaßes, zu dem das Polypeptid von (1) und der Ligand von (2) binden; und
(c) Vergleichen des in (b) bestimmten Ausmaßes mit dem Ausmaß, zu dem die Bindung des Polypeptids von (1) und des Liganden von (2) bei Abwesenheit des zu beurteilenden Kandidatenagens und unter denselben Bedingungen, die zur Bindung des Polypeptids von (1) mit dem Liganden von (2) geeignet sind, erfolgt;
wobei das Ausmaß, zu dem die Bindung des Polypeptids von (1) und des Liganden von (2) erfolgt, bei Gegenwart des Kandidatenagens geringer ist als bei Abwesenheit des Kandidatenagens, wobei es sich bei dem Kandidatenagens um ein Agens handelt, das die Bindung zwischen dem Polypeptid und dem Liganden des Polypeptids inhibiert.

13. Verfahren nach Anspruch 11, wobei der Schritt des In-Kontakt-Bringens des Agens mit dem Polypeptid in einem künstlichen Membransystem durchgeführt wird, oder Verfahren nach Anspruch 12, wobei Schritt (a) in einem künstlichen Membransystem durchgeführt wird.

14. Verfahren nach Anspruch 11 oder 12, wobei das isolierte Polypeptid in einer isolierten Plasmamembran ist.

15. Verfahren zum Identifizieren eines Agens, das ein Inhibitor der Fettsäureaufnahme durch ein Polypeptid nach Anspruch 1 ist, wobei das Verfahren die folgenden Schritte umfasst:
a) Aufbewahren von Testzellen, die das. Polypeptid exprimieren, in Gegenwart einer Fettsäure und eines Agens, das als ein Inhibitor der Fettsäureaufnahme getestet werden soll;
b) Messen der Aufnahme der Fettsäure in den Testzellen und
c) Vergleichen der Aufnahme der Fettsäure in den Testzellen mit der Aufnahme der Fettsäure in geeigneten Kontrollzellen;
wobei eine geringere Aufnahme der Fettsäure in den Testzellen verglichen mit der Aufnahme der Fettsäure in den Kontrollzellen anzeigt, dass das Agens ein Inhibitor der Fettsäureaufnahme durch das Polypeptid ist.

16. Verfahren zum Identifizieren eines Agens, das ein Inhibitor eines Polypeptids nach Anspruch 1 ist, wobei das Verfahren die folgenden Schritte umfasst:
(a) Einführen eines oder mehrerer Vektoren, die ein Polynukleotid umfassen, das das Polypeptid exprimiert, in Wirtszellen;
(b) Kultivieren eines ersten Aliquots der Wirtszellen mit Fettsäuresubstrat des Polypeptids und mit einem Agens, das als ein Inhibitor des Polypeptids getestet wird;
(c) Kultivieren eines zweiten Aliquots der Wirtszellen mit Fettsäuresubstrat des Polypeptids;
(d) Messen der Aufnahme des Fettsäuresubstrats durch die Wirtszellen in dem ersten und dem zweiten Aliquot;
wobei eine geringere Aufnahme des Fettsäuresubstrats in dem ersten Aliquot verglichen mit dem zweiten Aliquot anzeigt, dass das Agens ein Inhibitor des Polypeptids ist.

17. Verfahren zum Identifizieren eines Agens, das ein Inhibitor eines Polypeptids nach Anspruch 1 ist, wobei das Verfahren die folgenden Schritte umfasst:
(a) Einführen eines oder mehrerer Vektoren, die ein Polynukleotid umfassen, das das Polypeptid exprimiert, in Zellen;
(b) In-Kontakt-Bringen der Wirtszellen mit Anti-Oberflächenprotein-Antikörper und markiertem Fettsäuresubstrat von FATP4;
(c) In-Kontakt-Bringen eines ersten Aliquots der Wirtszellen mit einem Agens, das als ein Inhibitor von FATP4 getestet wird, wohingegen ein zweites Aliquot der Wirtszellen nicht mit dem Agens in Kontakt gebracht wird;
(d) Identifizieren der Wirtszellen, die das Oberflächenprotein exprimieren, in dem ersten und dem zweiten Aliquot, indem der an die Wirtszellen gebundene Anti-Oberflächenprotein-Antikörper nachgewiesen wird;
(e) Messen der Aufnahme des Fettsäuresubstrats durch die Wirtszellen, die als das Oberflächenprotein exprimierend identifiziert wurden, in dem ersten und dem zweiten Aliquot;
wobei eine geringere Aufnahme des Fettsäuresubstrats in dem ersten Aliquot verglichen mit dem zweiten Aliquot anzeigt, dass das Agens ein Inhibitor von FATP4 ist.

18. Verfahren nach Anspruch 17, wobei es sich bei den Wirtszellen um Prokaryonten handelt.

19. Verfahren nach Anspruch 17 oder 18, wobei es sich bei der Fettsäure um eine radioaktiv markierte Fettsäure handelt.

20. Verfahren nach einem der Ansprüche 17 bis 19, wobei es sich bei dem Oberflächenprotein um CD2 handelt.

21. Verfahren nach einem der Ansprüche 17 bis 20, wobei das Fettsäuresubstrat BODIPY-markiert ist.

22. Verfahren nach einem der Ansprüche 17 bis 21, wobei die Wirtszellen das Polynukleotid, das das Polypeptid kodiert, regulierbar exprimieren.

23. Verfahren nach einem der Ansprüche 12 bis 22, das weiterhin die folgenden Schritte umfasst:
a) Verabreichen des Agens an ein oder mehrere Versuchstiere;
b) Messen von exogen zugeführten Fettsäuren in einer oder mehreren Proben von Gewebe oder Körperflüssigkeit von den Versuchstieren;
c) Messen von exogen zugeführten Fettsäuren in einer oder mehreren Vergleichsproben von Gewebe oder Körperflüssigkeit von geeigneten Kontrolltieren;
d) Vergleichen der Fettsäuren von b) mit den Fettsäuren von c);
wobei geringere Fettsäuren in Schritt b) als in Schritt c) anzeigen, dass das Agens ein Inhibitor des Polypeptids ist.

24. Verfahren zum Nachweisen von FATP4 in einer Probe von Zellen oder einer Probe von Zelllysat, wobei das Verfahren die Schritte des Zugebens eines Agens, das spezifisch an FATP4 bindet, zu der Probe und des Nachweisens des Agens, das spezifisch an FATP4 gebunden hat, umfasst, wobei es sich bei dem Agens um einen Antikörper handelt, der an FATP4 bindet.

25. Isolierter Antikörper oder antigenbindendes Fragment davon, der bzw. das spezifisch an ein Polypeptid nach Anspruch 1 bindet.

26. Isolierter Antikörper nach Anspruch 25, wobei es sich bei dem Antikörper um einen polyklonalen Antikörper handelt.

27. Isolierter Antikörper nach Anspruch 25, wobei es sich bei dem Antikörper um einen monoklonalen Antikörper handelt.

28. Verfahren zum Modulieren der Fettsäureaufnahme durch Zellen in Kultur, das das Zugeben eines oder mehrerer Agentien, die die Fettsäureaufnahme durch FATP4 modulieren, zu Zellen, die FATP4 umfassen, umfasst, wobei es sich bei dem Agens um eine Nukleinsäure nach Anspruch 5, ein Antisense-Oligonukleotid zu FATP4 oder einen Antikörper, der an das Polypeptid nach Anspruch 1 bindet, handelt.

29. Polypeptid nach Anspruch 1, Fusionsprotein nach einem der Ansprüche 2 bis 4, Nukleinsäuremolekül nach Anspruch 6, Vektor nach Anspruch 7 oder Antikörper nach einem der Ansprüche 25 bis 27 zur Verwendung in der Medizin.

30. Pharmazeutische Zusammensetzung, die ein Polypeptid nach Anspruch 1, ein Fusionsprotein nach einem der Ansprüche 2 bis 4, eine Nukleinsäure nach Anspruch 6, einen Vektor nach Anspruch 7 oder einen Antikörper nach einem der Ansprüche 25 bis 27 umfasst.

31. Verwendung eines Agens, das ein Inhibitor der Fettsäureaufnahme durch FATP4-Protein im Dünndarm ist, bei der Herstellung eines Arzneimittels zum Behandeln einer Erkrankung, die durch übermäßige Fettsäureaufnahme im Dünndarm **gekennzeichnet** ist, wobei das Agens ein Antisense-Oligonukleotid oder einen Antikörper nach einem der Ansprüche 25 bis 27 umfasst.

32. Verwendung nach Anspruch 31, wobei das Arzneimittel zur oralen Verabreichung ist.

## Revendications

1. Polypeptide isolé qui:
a) comprend la séquence d'acides aminés de la FATP4 dans la séquence SÉQ ID N° : 53 ;
b) consiste en la séquence d'acides aminés SÉQ ID N° : 53 ; ou
c) comprend au moins 360 résidus acides aminés contigus de la séquence SÉQ ID N° : 53,
où le polypeptide a une activité FATP4.

2. Protéine de fusion comprenant un polypeptide selon la revendication 1 qui est condensé à un fragment hétérologue.

3. Protéine de fusion selon la revendication 2, qui comprend un ligand d'affinité.

4. Protéine de fusion selon revendication 2 ou la revendication 3, où la protéine de fusion transporte des acides gras au travers d'une membrane cellulaire ou d'un système à membrane cellulaire artificielle.

5. Acide nucléique isolé qui :
a) code pour un polypeptide selon la revendication 1, ou la séquence complémentaire d'un acide nucléique qui code pour le polypeptide ;
b) comprend la séquence nucléotidique SÉQ ID N° : 52, ou la séquence complémentaire de celle-ci ;
c) consiste en la séquence nucléotidique SÉQ ID N° : 52, ou la séquence complémentaire de celle-ci ; et/ou
d) comprend la séquence nucléotidique de la région codante de la séquence SÉQ ID N° : 52.

6. Molécule d'acide nucléique isolée comprenant une séquence nucléotidique qui code pour une protéine de fusion selon l'une quelconque des revendications 2 à 4.

7. Vecteur comprenant une molécule d'acide nucléique selon la revendication 5 ou la revendication 6 ou codant pour un polypeptide selon la revendication 1 ou une protéine de fusion selon l'une quelconque des revendications 2 à 4.

8. Cellule hôte isolée comprenant le vecteur selon la revendication 7.

9. Cellule hôte non humaine comprenant le vecteur selon la revendication 7.

10. Méthode de production d'un polypeptide selon la revendication 1 ou d'une protéine de fusion selon l'une quelconque des revendications 2 à 4, qui comprend la culture de la cellule hôte selon la revendication 8 ou 9 dans des conditions dans lesquelles l'acide nucléique est exprimé, produisant ainsi le polypeptide ou la protéine de fusion.

11. Méthode d'identification d'un agent qui se lie au polypeptide selon la revendication 1, qui comprend les étapes consistant à mettre en contact l'agent avec le polypeptide isolé dans des conditions appropriées à la liaison de l'agent au polypeptide isolé et à détecter le complexe agent-polypeptide ainsi formé.

12. Méthode d'identification d'un agent qui inhibe l'interaction entre un polypeptide selon la revendication 1 et un ligand dudit polypeptide, la méthode comprenant les étapes qui consistent à .
(a) combiner :
(1) ledit polypeptide isolé ;
(2) le ligand dudit polypeptide ;
(3) et un agent candidat testé pour évaluer son aptitude à inhiber la liaison entre polypeptide de (1) et le ligand de (2), dans des conditions appropriées à une liaison entre ledit polypeptide de (1) et le ligand de (2) ;
b) déterminer le degré auquel ledit polypeptide de (1) et le ligand de (2) se lient ; et
c) comparer le degré de liaison déterminé en (b) au degré auquel une liaison a lieu entre ledit polypeptide de (1) et le ligand de (2) en l'absence de l'agent candidat évalué dans les mêmes conditions appropriées à une liaison entre ledit polypeptide de (1) et le ligand de (2);
où, si le degré de liaison auquel une liaison a lieu entre ledit polypeptide de (1) et le ligand de (2) est plus bas en la présence de l'agent candidat qu'en son absence, l'agent candidat est un agent qui inhibe la liaison entre ledit polypeptide et le ligand dudit polypeptide.

13. Méthode selon la revendication 11, où l'étape de mise en contact entre l'agent testé et le polypeptide est effectuée dans un système à membrane artificielle, ou méthode selon la revendication 12, où l'étape (a) est effectuée dans un système à membrane artificielle.

14. Méthode selon la revendication 11 ou la revendication 12, où le polypeptide isolé est dans une membrane plasmatique isolée.

15. Méthode d'identification d'un agent qui est un inhibiteur de la captation d'un acide gras par un polypeptide selon la revendication 1, la méthode comprenant les étapes qui consistent à :
a) maintenir des cellules utilisées pour le test qui expriment ledit polypeptide en la présence d'un acide gras et d'un agent à tester en tant qu'inhibiteur de la captation de l'acide gras;
b) mesurer la captation de l'acide gras dans les cellules utilisées pour le test ; et
c) comparer la captation de l'acide gras dans les cellules utilisées pour le test à la captation de l'acide gras dans des cellules témoins appropriées ;
où une captation de l'acide gras plus basse dans les cellules utilisées pour le test que dans les cellules témoins est indicative du fait que l'agent est un inhibiteur de la captation de l'acide gras par ledit polypeptide.

16. Méthode d'identification d'un agent qui est un inhibiteur d'un polypeptide selon la revendication 1, la méthode comprenant les étapes qui consistent à :
a) introduire dans des cellules hôtes un ou plusieurs vecteurs comprenant un polynucléotide qui exprime ledit polypeptide ;
b) cultiver une première aliquote des cellules hôtes avec un acide gras substrat dudit polypeptide et avec un agent à tester en tant qu'inhibiteur dudit polypeptide ;
c) cultiver une seconde aliquote des cellules hôtes avec un acide gras substrat dudit polypeptide ;
d) mesurer, dans les première et seconde aliquotes, la captation de l'acide gras substrat dans les cellules hôtes ;
où une captation de l'acide gras substrat qui est plus basse dans la première aliquote que dans la seconde aliquote est indicative du fait que l'agent est un inhibiteur dudit polypeptide.

17. Méthode d'identification d'un agent qui est un inhibiteur d'un polypeptide selon la revendication 1, la méthode comprenant les étapes qui consistent à :
a) introduire dans des cellules un ou plusieurs vecteurs comprenant un polynucléotide qui code pour ledit polypeptide ;
b) mettre en contact les cellules hôtes avec un anticorps dirigé contre une protéine de surface des cellules et avec un acide gras radiomarqué substrat de la FATP4 ;
c) mettre en contact une première aliquote des cellules hôtes avec un agent à tester en tant qu'inhibiteur de la FATP4, tout en laissant une seconde aliquote des cellules hôtes sans contact avec l'agent à tester ;
d) identifier, dans les première et seconde aliquotes, les cellules hôtes exprimant la protéine de surface des cellules par détection de l'anticorps dirigé contre la protéine de surface des cellules lié aux cellules hôtes ; et
e) mesurer, dans les première et seconde aliquotes, la captation de l'acide gras substrat dans les cellules hôtes identifiées comme exprimant la protéine de surface des cellules ;
où une captation de l'acide gras substrat qui est plus basse dans la première aliquote que dans la seconde aliquote est indicative du fait que l'agent est un inhibiteur de la FATP4.

18. Méthode selon la revendication 17, où les cellules hôtes sont des cellules procaryotes.

19. Méthode selon la revendication 17 ou 18, où l'acide gras est un acide gras radiomarqué.

20. Méthode selon l'une quelconque des revendications 17 à 19, où la protéine de surface des cellules est CD2.

21. Méthode selon l'une quelconque des revendications 17 à 20, où l'acide gras substrat est marqué au fluorophore BODIPY.

22. Méthode selon l'une quelconque des revendications 17 à 21, où les cellules hôtes expriment d'une manière régulable le polynucléotide codant pour ledit polypeptide.

23. Méthode selon l'une quelconque des revendications 12 à 22, qui comprend également les étapes consistant à :
a) administrer l'agent à un ou plusieurs animaux d'expérience ;
b) mesurer la teneur en acides gras d'origine exogène dans un ou plusieurs échantillons d'un tissu ou d'un liquide organique obtenus chez lesdits animaux d' expérience ;
c) mesurer la teneur en acides gras d'origine exogène dans un plusieurs échantillons d'un tissu ou d'un liquide organique comparables obtenus chez des animaux témoins appropriés ;
d) comparer les teneurs en acides gras mesurées en b) aux teneurs en acides gras mesurées en c) ;
où une teneur en acides gras mesurée à l'étape b) plus basse que celle mesurée à l'étape c) est indicative du fait que l'agent est un inhibiteur dudit polypeptide.

24. Méthode de détection de la protéine FATP4 dans un échantillon de cellules ou un échantillon de lysat cellulaire, la méthode comprenant les étapes qui consistent à ajouter à l'échantillon un agent qui se lie spécifiquement à la FATP4 et à détecter l'agent spécifiquement lié à la FATP4, où l'agent est un anticorps qui se lie à la FATP4.

25. Anticorps isolé ou fragment de liaison à l'antigène de celui-ci qui se lie spécifiquement à un polypeptide selon la revendication 1.

26. Anticorps isolé selon la revendication 25, où l'anticorps est un anticorps polyclonal.

27. Anticorps isolé selon la revendication 25, où l'anticorps est un anticorps monoclonal.

28. Méthode permettant de moduler la captation d'un acide gras dans des cellules en culture, comprenant l'addition de un ou plusieurs agents qui modulent la captation d'un acide gras par la FATP4 dans des cellules comportant la FATP4, où l'agent est un acide nucléique selon la revendication 5, un oligonucléotide antisens dirigé contre la FATP4 ou un anticorps qui se lie au polypeptide selon la revendication 1.

29. Polypeptide selon la revendication 1, protéine de fusion selon l'une quelconque des revendications 2 à 4, molécule d'acide nucléique selon la revendication 6, vecteur selon la revendication 7 ou anticorps selon l'une quelconque des revendications 25 à 27 destiné(e) à une utilisation en médecine.

30. Composition pharmaceutique qui comprend un polypeptide selon la revendication 1, une protéine de fusion selon l'une quelconque des revendications 2 à 4, une molécule d'acide nucléique selon la revendication 6, un vecteur selon la revendication 7 ou un anticorps selon l'une quelconque des revendications 25 à 27.

31. Utilisation d'un agent qui est un inhibiteur de la captation des acides gras par la protéine FATP4 dans l'intestin grêle dans la préparation d'un médicament indiqué dans le traitement d'une affection **caractérisée par** une captation excessive d'acides gras dans l'intestin grêle, où l'agent comprend un oligonucléotide antisens ou un anticorps selon l'une quelconque des revendications 25 à 27.

32. Utilisation selon la revendication 31, où le médicament est conçu pour être administré par voie orale.


### SEQUENCE LISTING

<110> Whitehead Institute for Biomedical Research
   Millennium Pharmaceuticals, Inc.
   Stahl, Andreas
   Hirsch, David J.
   Lodish, Harvey F.
   Gimeno, Ruth E.
   Tartaglia, Louis A.
<120> FATTY ACID TRANSPORT PROTEINS
<130> WHI97-21p3M2 PCT
<140> PCT/US99/00182
   <141> 1999-01-14
<150> 60/071,374
   <151> 1998-01-15
<150> 60/093,491
   <151> 1998-07-20
<150> 60/110,941
   <151> 1998-12-04
<150> 09/232,197
   <151> 1999-01-14
<150> 09/232,200
   <151> 1999-01-14
<150> 09/232,201
   <151> 1999-01-14
<150> 09/232,195
   <151> 1999-01-14
<160> 105
<170> FastSEQ for Windows Version 3.0
<210> 1
   <211> 340
   <212> PRT
   <213> Mus musculus
<400> 1
   Phe Ile Phe Thr Ser Gly Thr Thr Gly Leu Pro Lys Pro Ala Ile Leu
    1               5                  10                  15
   Ser His Glu Arg Val Ile Gln Val Ser Asn Val Leu Ser Phe Cys Gly
               20                  25                  30
   Cys Arg Ala Asp Asp Val Val Tyr Asp Val Leu Pro Leu Tyr His Thr
           35                  40                  45
   Ile Gly Leu Val Leu Gly Phe Leu Gly Cys Leu Gln Val Gly Ala Thr
   50 55 60
   Cys Val Leu Ala Pro Lys Phe Ser Ala Ser Arg Phe Trp Ala Glu Cys
   65 70 75 80
   Arg Gln His Gly Val Thr Val Ile Gln Tyr Ile Gly Glu Ile Cys Arg
   85 90 95
   Tyr Leu Leu Arg Gln Pro Val Arg Asp Val Glu Gln Arg His Arg Val
   100 105 110
   Arg Leu Ala Val Gly Asn Gly Leu Arg Pro Ala Ile Trp Glu Glu Phe
   115 120 125
   Thr Gln Arg Phe Gly Val Pro Gln Ile Gly Glu Phe Tyr Gly Ala Thr
   130 135 140
   Glu Cys Asn Cys Ser Ile Ala Asn Met Asp Gly Lys Val Gly Ser Cys
   145 150 155 160
   Gly Phe Asn Ser Arg Ile Leu Thr His Val Tyr Pro Ile Arg Leu Val
   165 170 175
   Lys Val Asn Glu Asp Thr Met Glu Pro Leu Arg Asp Ser Glu Gly Leu
   180 185 190
   Cys Ile Pro Cys Gln Pro Gly Glu Pro Gly Leu Leu Val Gly Gln Ile
   195 200 205
   Asn Gln Gln Asp Pro Leu Arg Arg Phe Asp Gly Tyr Val Ser Asp Ser
   210 215 220
   Ala Thr Asn Lys Lys Ile Ala His Ser Val Phe Arg Lys Gly Asp Ser
   225 230 235 240
   Ala Tyr Leu Ser Gly Asp Val Leu Val Met Asp Glu Leu Gly Tyr Met
   245 250 255
   Tyr Phe Arg Asp Arg Ser Gly Asp Thr Phe Arg Trp Arg Gly Glu Asn
   260 265 270
   Val Ser Thr Thr Glu Val Glu Ala Val Leu Ser Arg Leu Leu Gly Gln
   275 280 285
   Thr Asp Val Ala Val Tyr Gly Val Ala Val Pro Gly Val Glu Gly Lys
   290 295 300
   Ala Gly Met Ala Ala Ile Ala Asp Pro His Ser Gln Leu Asp Pro Asn
   305 310 315 320
   Ser Met Tyr Gln Glu Leu Gln Lys Val Leu Ala Ser Tyr Ala Arg Pro
   325 330 335
   Ile Phe Leu Arg
   340
<210> 2
   <211> 339
   <212> PRT
   <213> Mus musculus
<400> 2
   Tyr Ile Tyr Thr Ser Gly Thr Thr Gly Asn Pro Lys Pro Ala Val Ile
   1               5                   10                  15
   Lys His Phe Arg Tyr Phe Trp Ile Ala Met Gly Ala Gly Lys Ala Phe
               20                  25                  30
   Gly Ile Asn Lys Ser Asp Val Val Tyr Ile Thr Met Pro Met Tyr His
           35                  40                  45
   Ser Ala Ala Gly Ile Met Gly Ile Gly Ser Leu Ile Ala Phe Gly Ser
       50                  55                  60
   Thr Ala Val Ile Arg Lys Lys Phe Ser Ala Ser Asn Phe Trp Lys Asp
   65                  70                  75                  80
   Cys Val Lys Tyr Asn Val Thr Ala Thr Leu Tyr Val Gly Glu Ile Leu
                   85                  90                  95
   Arg Tyr Leu Cys Asn Val Pro Glu Gln Pro Glu Asp Lys Ile His Thr
               100                 105                 110
   Val Arg Leu Ala Met Gly Thr Gly Leu Arg Ala Asn Val Trp Lys Asn
           115                 120                 125
   Phe Gln Gln Arg Phe Gly Pro Ile Arg Ile Trp Glu Phe Tyr Gly Ser
       130                 135                 140
   Thr Glu Gly Asn Val Gly Leu Met Asn Tyr Val Gly His Cys Gly Ala
   145                 150                 155                 160
   Val Gly Arg Thr Ser Cys Ile Leu Arg Met Leu Thr Pro Phe Glu Leu
                   165                 170                 175
   Val Gln Phe Asp Ile Glu Thr Ala Glu Pro Leu Arg Asp Lys Gln Gly
               180                 185                 190
   Phe Cys Ile Pro Val Glu Pro Gly Lys Pro Gly Leu Leu Leu Thr Lys
           195                 200                 205
   Val Arg Lys Asn Gln Pro Phe Leu Gly Tyr Arg Gly Ser Gln Ala Glu
       210                 215                 220
   Ser Asn Arg Lys Leu Val Ala Asn Val Arg Arg Val Gly Asp Leu Tyr
   225                 230                 235                 240
   Phe Asn Thr Gly Asp Val Leu Thr Leu Asp Gln Glu Gly Phe Phe Tyr
                   245                 250                 255
   Phe Gln Asp Arg Leu Gly Asp Thr Phe Arg Trp Lys Gly Glu Asn Val
               260                 265                 270
   Ser Thr Gly Glu Val Glu Cys Val Leu Ser Ser Leu Asp Phe Leu Glu
           275                 280                 285
   Glu Val Asn Val Tyr Gly Val Pro Val Pro Gly Cys Glu Gly Lys Val
       290                 295                 300
   Gly Met Ala Ala Val Lys Leu Ala Pro Gly Lys Thr Phe Asp Gly Lys
   305                 310                 315                 320
   Lys Tyr Gln His Val Arg Ser Trp Leu Pro Ala Tyr Ala Thr Pro His
                   325                 330                 335
   Phe Ile Arg
<210> 3
   <211> 345
   <212> PRT
   <213> Caenorhabditis elegans
<400> 3
   Ile Tyr Thr Ser Gly Thr Thr Gly Leu Pro Lys Ser Ala Ile Met Ser
    1               5                  10                  15
   Trp Arg Lys Ser Ser Val Gly Cys Gln Val Phe Gly His Val Leu His
               20                  25                  30
   Met Thr Asn Glu Ser Thr Val Phe Thr Ala Met Pro Leu Phe His Ser
           35                  40                  45
   Thr Ala Ala Leu Leu Gly Ala Cys Ala Ile Leu Ser His Gly Gly Cys
       50                  55                  60
   Leu Ala Leu Ser His Lys Phe Ser Ala Ser Thr Phe Trp Lys Gln Val
   65                  70                  75                  80
   Tyr Leu Thr Gly Ala Thr His Ile Gln Tyr Ile Gly Glu Ile Cys Arg
                   85                  90                  95
   Tyr Leu Leu Ala Ala Asn Pro Cys Pro Glu Glu Lys Gln His Asn Val
               100                 105                 110
   Arg Leu Met Trp Gly Asn Gly Leu Arg Gly Gln Ile Trp Lys Glu Phe
           115                 120                 125
   Val Gly Arg Phe Gly Ile Lys Lys Ile Gly Glu Leu Tyr Gly Ser Thr
       130                 135                 140
   Glu Gly Asn Ser Asn Ile Val Asn Val Asp Asn His Val Gly Ala Cys
   145                 150                 155                 160
   Gly Phe Met Pro Ile Tyr Pro His Ile Gly Ser Leu Tyr Pro Val Arg
                   165                 170                 175
   Leu Ile Lys Val Asp Arg Ala Thr Gly Glu Leu Glu Arg Asp Lys Asn
               180                 185                 190
   Gly Leu Cys Val Pro Cys Val Pro Gly Glu Thr Gly Glu Met Val Gly
           195                 200                 205
   Val Ile Lys Glu Lys Asp Ile Leu Leu Lys Phe Glu Gly Tyr Val Ser
       210                 215                 220
   Glu Gly Asp Thr Ala Lys Lys Ile Tyr Arg Asp Val Phe Lys His Gly
   225                 230                 235                 240
   Asp Lys Val Phe Ala Ser Gly Asp Ile Leu His Trp Asp Asp Leu Gly
                   245                 250                 255
   Tyr Leu Tyr Phe Val Asp Arg Cys Gly Asp Thr Phe Arg Trp Lys Gly
               260                 265                 270
   Glu Asn Val Ser Thr Thr Glu Val Glu Gly Ile Leu Gln Pro Val Met
           275                 280                 285
   Asp Val Glu Asp Ala Thr Val Tyr Gly Val Thr Val Gly Lys Met Glu
       290                 295                 300
   Gly Arg Ala Gly Met Ala Gly Ile Val Val Lys Asp Gly Thr Asp Val
   305                 310                 315                 320
   Glu Lys Phe Ile Ala Asp Ile Thr Ser Arg Leu Thr Glu Asn Leu Ala
                   325                 330                 335
   Ser Tyr Ala Ile Pro Val Phe Ile Arg
               340                 345
<210> 4
   <211> 356
   <212> PRT
   <213> Saccharomyces cerevisiae
<400> 4
   Tyr Ile Tyr Thr Ser Gly Thr Thr Gly Leu Pro Lys Ala Ala Ile Val
    1               5                  10                  15
   Val His Ser Arg Tyr Tyr Arg Ile Ala Ala Phe Gly His His Ser Tyr
               20                  25                  30
   Ser Met Arg Ala Ala Asp Val Leu Tyr Asp Cys Leu Pro Leu Tyr His
           35                  40                  45
   Ser Ala Gly Asn Ile Met Gly Val Gly Gln Cys Val Ile Tyr Gly Leu
       50                  55                  60
   Thr Val Val Leu Arg Lys Lys Phe Ser Ala Ser Arg Phe Trp Asp Asp
   65                  70                  75                  80
   Cys Val Lys Tyr Asn Cys Thr Val Val Gln Tyr Val Gly Glu Val Cys
                   85                  90                  95
   Arg Tyr Leu Leu His Thr Pro Ile Ser Lys Tyr Glu Lys Met His Lys
               100                 105                 110
   Val Lys Val Ala Tyr Gly Asn Gly Leu Arg Pro Asp Ile Trp Gln Asp
           115                 120                 125
   Phe Arg Lys Arg Phe Asn Ile Glu Val Ile Gly Glu Phe Tyr Ala Ala
       130                 135                 140
   Thr Glu Ala Pro Phe Ala Thr Thr Thr Phe Gln Lys Gly Asp Phe Gly
   145                 150                 155                 160
   Ile Gly Ala Cys Arg Asn Tyr Gly Thr Ile Ile Gln Trp Phe Leu Ser
                   165                 170                 175
   Phe Gln Gln Thr Leu Val Arg Met Asp Pro Asn Asp Asp Ser Val Ile
               180                 185                 190
   Tyr Arg Asn Ser Lys Gly Phe Cys Glu Val Ala Pro Val Gly Glu Pro
           195                 200                 205
   Gly Glu Met Leu Met Arg Ile Phe Phe Pro Lys Lys Pro Glu Thr Ser
       210                 215                 220
   Phe Gln Gly Tyr Leu Gly Asn Ala Lys Glu Thr Lys Ser Lys Val Val
   225                 230                 235                 240
   Arg Asp Val Phe Arg Arg Gly Asp Ala Trp Tyr Arg Cys Gly Asp Leu
                   245                 250                 255
   Leu Lys Ala Asp Glu Tyr Gly Leu Trp Tyr Phe Leu Asp Arg Met Gly
               260                 265                 270
   Asp Thr Phe Arg Trp Lys Ser Glu Asn Val Ser Thr Thr Glu Val Glu
           275                 280                 285
   Asp Gln Leu Thr Ala Ser Asn Lys Glu Gln Tyr Ala Gln Val Leu Val
       290                 295                 300
   Val Gly Ile Lys Val Pro Lys Tyr Glu Gly Arg Ala Gly Phe Ala Val
   305                 310                 315                 320
   Ile Lys Leu Thr Asp Asn Ser Leu Asp Ile Thr Ala Lys Thr Lys Leu
                   325                 330                 335
   Leu Asn Asp Ser Leu Ser Arg Leu Asn Leu Pro Ser Tyr Ala Met Pro
               340                 345                 350
   Leu Phe Val Lys
           355
<210> 5
   <211> 334
   <212> PRT
   <213> Mycobacterium tuberculosis
<400> 5
   Tyr Ile Phe Thr Ser Gly Thr Thr Gly Phe Pro Lys Ala Ser Val Met
   1 5 10 15
   Thr His His Arg Trp Leu Arg Ala Leu Ala Val Phe Gly Gly Met Gly
   20 25 30
   Leu Arg Leu Lys Gly Ser Asp Thr Leu Tyr Ser Cys Leu Pro Leu Tyr
   35 40 45
   His Asn Asn Ala Leu Thr Val Ala Val Ser Ser Val Ile Asn Ser Gly
   50 55 60
   Ala Thr Leu Ala Leu Gly Lys Ser Phe Ser Ala Ser Arg Phe Trp Asp
   65 70 75 80
   Glu Val Ile Ala Asn Arg Ala Thr Ala Phe Val Tyr Ile Gly Glu Ile
   85 90 95
   Cys Arg Tyr Leu Leu Asn Gln Pro Ala Lys Pro Thr Asp Arg Ala His
   100 105 110
   Gln Val Arg Val Ile Cys Gly Asn Gly Leu Arg Pro Glu Ile Trp Asp
   115 120 125
   Glu Phe Thr Thr Arg Phe Gly Val Ala Arg Val Cys Glu Phe Tyr Ala
   130 135 140
   Ala Ser Glu Gly Asn Ser Ala Phe Ile Asn Ile Phe Asn Val Pro Arg
   145 150 155 160
   Thr Ala Gly Val Ser Pro Met Pro Leu Ala Phe Val Glu Tyr Asp Leu
   165 170 175
   Asp Thr Gly Asp Pro Leu Arg Asp Ala Ser Gly Arg Val Arg Arg Val
   180 185 190
   Pro Asp Gly Glu Pro Gly Leu Leu Leu Ser Arg Val Asn Arg Leu Gln
   195 200 205
   Pro Phe Asp Gly Tyr Thr Asp Pro Val Ala Ser Glu Lys Lys Leu Val
   210 215 220
   Arg Asn Ala Phe Arg Asp Gly Asp Cys Trp Phe Asn Thr Gly Asp Val
   225 230 235 240
   Met Ser Pro Gln Gly Met Gly His Ala Ala Phe Val Asp Arg Leu Gly
   245 250 255
   Asp Thr Phe Arg Trp Lys Gly Glu Asn Val Ala Thr Thr Gln Val Glu
   260 265 270
   Ala Ala Leu Ala Ser Asp Gln Thr Val Glu Glu Cys Thr Val Tyr Gly
   275 280 285
   Val Gln Ile Pro Arg Thr Gly Gly Arg Ala Gly Met Ala Ala Ile Thr
   290 295 300
   Leu Arg Ala Gly Ala Glu Phe Asp Gly Gln Ala Leu Ala Arg Thr Val
   305 310 315 320
   Tyr Gly His Leu Pro Gly Tyr Ala Leu Pro Leu Phe Val Arg
   325 330
<210> 6
   <211> 2087
   <212> DNA
   <213> Mus musculus
<400> 6
   acgactcact atagggagag agctatgacg tcgcatgcac gcgtaagctt gggcccctcg      60
   agggatcctc tagagcggcc gccgaccccg aaagctctga gagcgggtgc agtctggcct     120
   ggcgtctcgc gtacctggcc cgggagcagc cgacacacac cttcctcatc cacggcgcgc     180
   agcgctttag ctacgcggag gctgagcgcg agagcaaccg gattgctcgc gcctttctgc     240
   gcgcacgggg ctggaccggg ggccgccgag gctcgggcag gggcagcact gaggaaggcg     300
   cacgcgtggc gcctccggct ggagatgcgg ctgctagagg gacgaccgcg ccccctctgg     360
   cacccggggc gaccgtggcg ctgctcctcc cagcgggccc ggatttcctt tggatttggt     420
   tcggactggc caaagctggc ctgcgcacgg cctttgtgcc caccgcttta cgccgaggac     480
   ccctgctgca ctgcctccgc agctgcggtg cgagtgcgct cgtgctggcc acagagttcc     540
   tggagtccct ggagccggac ctgccggcct tgagagccat ggggctccac ctatgggcga     600
   cgggccctga aactaatgta gctggaatca gcaatttgct atcggaagca gcagaccaag     660
   tggatgagcc agtgccgggg tacctctctg ccccccagaa cataatggac acctgcctgt     720
   acatcttcac ctctggcact actggcctgc ccaaggctgc tcgaatcagt catctgaagg     780
   ttctacagtg ccagggattc taccatctgt gtggagtcca ccaggaggac gtgatctacc     840
   tcgcactccc actgtaccac atgtctggct cccttctggg cattgtgggc tgcttgggca     900
   ttggggccac cgtggtgctg aaacccaagt tctcagctag ccagttctgg gacgattgcc     960
   agaaacacag ggtgacagtg ttccagtaca ttggggagtt gtgccgatac ctcgtcaacc    1020
   agcccccgag caaggcagag tttgaccata aggtgcgctt ggcagtgggc agtgggttgc   1080
   gcccagacac ctgggagcgt ttcctgcggc gatttggacc tctgcagata ctggagacgt    1140
   atggcatgac agagggcaac gtagctacgt tcaattacac aggacggcag ggtgcagtgg    1200
   ggcgagcttc ctggctttac aagcacatct tccccttctc cttgattcga tacgatgtca    1260
   tgacagggga gcctattcgg aatgcccagg ggcactgcat gaccacatct ccaggtgagc    1320
   caggcctact ggtggcccca gtgagccagc agtccccctt cctgggctat gctggggctc    1380
   cggagctggc caaggacaag ctgctgaagg atgtcttctg gtctggggac gttttcttca    1440
   atactgggga cctcttggtc tgtgatgagc aaggctttct tcacttccac gatcgtactg    1500
   gagacaccat caggtggaag ggagagaatg tggccacaac tgaagtggct gaggtcttgg    1560
   agaccctgga cttccttcag gaggtgaaca tctatggagt cacggtgcca gggcacgaag    1620
   gcagggcagg catggcggcc ttggctctgc ggcccccgca ggctctgaac ctggtgcagc    1680
   tctacagcca tgtttctgag aacttgccac cgtatgcccg acctcggttt ctcaggctcc    1740
   aggaatcttt ggccactact gagaccttca aacagcagaa ggttaggatg gccaatgagg    1800
   gctttgaccc cagtgtactg tctgacccac tctatgttct ggaccaagat ataggggcct    1860
   acctgcccct cacacctgcc cggtacagtg ccctcctgtc tggagacctt cgaatctgaa    1920
   accttccact tgagggaggg gctcggaggg tacaggccac catggctgca ccagggaggg    1980
   ttttcgggta tcttttgtat atggagtcat tattttgtaa taaacagctg gagcttaaaa    2040
   aaaaaaaaaa aaaaaaaaaa aaaaaaaaaa aaaaaaaaaa aaaaaaa                  2087
<210> 7
   <211> 613
   <212> PRT
   <213> Mus musculus
<400> 7
   Ala Ala Asp Pro Glu Ser Ser Glu Ser Gly Cys Ser Leu Ala Trp Arg
   1 5 10 15
   Leu Ala Tyr Leu Ala Arg Glu Gln Pro Thr His Thr Phe Leu Ile His
   20 25 30
   Gly Ala Gln Arg Phe Ser Tyr Ala Glu Ala Glu Arg Glu Ser Asn Arg
   35 40 45
   Ile Ala Arg Ala Phe Leu Arg Ala Arg Gly Trp Thr Gly Gly Arg Arg
   50 55 60
   Gly Ser Gly Arg Gly Ser Thr Glu Glu Gly Ala Arg Val Ala Pro Pro
   65 70 75 80
   Ala Gly Asp Ala Ala Ala Arg Gly Thr Thr Ala Pro Pro Leu Ala Pro
   85 90 95
   Gly Ala Thr Val Ala Leu Leu Leu Pro Ala Gly Pro Asp Phe Leu Trp
   100 105 110
   Ile Trp Phe Gly Leu Ala Lys Ala Gly Leu Arg Thr Ala Phe Val Pro
   115 120 125
   Thr Ala Leu Arg Arg Gly Pro Leu Leu His Cys Leu Arg Ser Cys Gly
   130 135 140
   Ala Ser Ala Leu Val Leu Ala Thr Glu Phe Leu Glu Ser Leu Glu Pro
   145 150 155 160
   Asp Leu Pro Ala Leu Arg Ala Met Gly Leu His Leu Trp Ala Thr Gly
   165 170 175
   Pro Glu Thr Asn Val Ala Gly Ile Ser Asn Leu Leu Ser Glu Ala Ala
   180 185 190
   Asp Gln Val Asp Glu Pro Val Pro Gly Tyr Leu Ser Ala Pro Gln Asn
   195 200 205
   Ile Met Asp Thr Cys Leu Tyr Ile Phe Thr Ser Gly Thr Thr Gly Leu
   210 215 220
   Pro Lys Ala Ala Arg Ile Ser His Leu Lys Val Leu Gln Cys Gln Gly
   225 230 235 240
   Phe Tyr His Leu Cys Gly Val His Gln Glu Asp Val Ile Tyr Leu Ala
   245 250 255
   Leu Pro Leu Tyr His Met Ser Gly Ser Leu Leu Gly Ile Val Gly Cys
   260 265 270
   Leu Gly Ile Gly Ala Thr Val Val Leu Lys Pro Lys Phe Ser Ala Ser
   275 280 285
   Gln Phe Trp Asp Asp Cys Gln Lys His Arg Val Thr Val Phe Gln Tyr
   290 295 300
   Ile Gly Glu Leu Cys Arg Tyr Leu Val Asn Gln Pro Pro Ser Lys Ala
   305 310 315 320
   Glu Phe Asp His Lys Val Arg Leu Ala Val Gly Ser Gly Leu Arg Pro
   325 330 335
   Asp Thr Trp Glu Arg Phe Leu Arg Arg Phe Gly Pro Leu Gln Ile Leu
   340 345 350
   Glu Thr Tyr Gly Met Thr Glu Gly Asn Val Ala Thr Phe Asn Tyr Thr
   355 360 365
   Gly Arg Gln Gly Ala Val Gly Arg Ala Ser Trp Leu Tyr Lys His Ile
   370 375 380
   Phe Pro Phe Ser Leu Ile Arg Tyr Asp Val Met Thr Gly Glu Pro Ile
   385                 390                 395                 400
   Arg Asn Ala Gln Gly His Cys Met Thr Thr Ser Pro Gly Glu Pro Gly
               405                 410                 415
   Leu Leu Val Ala Pro Val Ser Gln Gln Ser Pro Phe Leu Gly Tyr Ala
           420                 425                 430
   Gly Ala Pro Glu Leu Ala Lys Asp Lys Leu Leu Lys Asp Val Phe Trp
       435                 440                 445
   Ser Gly Asp Val Phe Phe Asn Thr Gly Asp Leu Leu Val Cys Asp Glu
       450                 455                 460
   Gln Gly Phe Leu His Phe His Asp Arg Thr Gly Asp Thr Ile Arg Trp
   465                 470                 475                 480
   Lys Gly Glu Asn Val Ala Thr Thr Glu Val Ala Glu Val Leu Glu Thr
                   485                 490                 495
   Leu Asp Phe Leu Gln Glu Val Asn Ile Tyr Gly Val Thr Val Pro Gly
               500                 505                 510
   His Glu Gly Arg Ala Gly Met Ala Ala Leu Ala Leu Arg Pro Pro Gln
           515                 520                 525
   Ala Leu Asn Leu Val Gln Leu Tyr Ser His Val Ser Glu Asn Leu Pro
       530                 535                 540
   Pro Tyr Ala Arg Pro Arg Phe Leu Arg Leu Gln Glu Ser Leu Ala Thr
   545                 550                 555                 560
   Thr Glu Thr Phe Lys Gln Gln Lys Val Arg Met Ala Asn Glu Gly Phe
                   565                 570                 575
   Asp Pro Ser Val Leu Ser Asp Pro Leu Tyr Val Leu Asp Gln Asp Ile
               580                 585                 590
   Gly Ala Tyr Leu Pro Leu Thr Pro Ala Arg Tyr Ser Ala Leu Leu Ser
           595                 600                 605
   Gly Asp Leu Arg Ile
       610
<210> 8
   <211> 2301
   <212> DNA
   <213> Mus musculus
<400> 8
   cccacgcgtc cgcccacgcg tccggcatgg ccaagctggg cgtggaggcg gctctcatca     60
   acaccaacct taggcgggat gccctgcgcc actgtcttga cacctcaaag gcacgagctc    120
   tcatctttgg cagtgagatg gcctcagcta tctgtgagat ccatgctagc ctggagccca    180
   cactcagcct cttctgctct ggatcctggg agcccagcac agtgcccgtc agcacagagc    240
   atctggaccc tcttctggaa gatgccccga agcacctgcc cagtcaccca gacaagggtt    300
   ttacagataa gctcttctac atctacacat cgggcaccac ggggctaccc aaagctgcca    360
   ttgtggtgca cagcaggtat tatcgtatgg cttccctggt gtactatgga ttccgcatgc    420
   ggcctgatga cattgtctat gactgcctcc ccctctacca ctcaagcagg aaacatcgtg    480
   gggattggca gtgcttactc cacggcatga ctgtggtgat ccggaagaag ttctcagcct    540
   cccggttctg ggatgattgt atcaagtaca actgcacagt ggtacagtac attggcgagc    600
   tctgccgcta cctcctgaac cagccacccc gtgaggctga gtctcggcac aaggtgcgca    660
   tggcactggg caacggtctc cggcagtcca tctggaccga cttctccagc cgtttccaca    720
   tcccccaggt ggctgagttc tatggggcca ctgaatgcaa ctgtagcctg ggcaactttg    780
   acagccgggt gggggcctgt ggcttcaata gccgcatcct gtcctttgtg taccctatcc    840
   gtttggtacg tgtcaatgag gataccatgg aactgatccg gggacccgat ggagtctgca    900
   ttccctgtca accaggtcag ccaggccagc tggtgggtcg catcatccag caggaccctc    960
   tgcgccgttt cgacgggtac ctcaaccagg gtgccaacaa caagaagatt gctaatgatg   1020
   tcttcaagaa gggggaccaa gcctacctca ctggtgacgt cctggtgatg gatgagctgg   1080
   gttacctgta cttccgagat cgcactgggg acacgttccg ctggaaaggg gagaatgtat   1140
   ctaccactga ggtggagggc acactcagcc gcctgcttca tatggcagat gtggcagttt   1200
   atggtgttga ggtgccagga actgaaggcc gagcaggaat ggctgccgtt gcaagtccca   1260
   tcagcaactg tgacctggag agctttgcac agaccttgaa aaaggagctg cctctgtatg   1320
   cccgccccat cttcctgcgc ttcttgcctg agctgcacaa gacagggacc ttcaagttcc  1380
   agaagacaga gttgcggaag gagggctttg acccatctgt tgtgaaagac ccgctgttct   1440
   atctggatgc tcggaagggc tgctacgttg cactggacca ggaggcctat acccgcatcc   1500
   aggcaggcga ggagaagctg tgatttcccc ctacatccct ctgagggcca gaagatgctg   1560
   gattcagagc cctagcgtcc accccagagg gtcctgggca atgccagacc aaagctagca   1620
   gggcccgcac ctccgcccct aggtgctgat ctcccctctc ccaaactgcc aagtgactca   1680
   ctgccgcttc cccgaccctc cagaggcttt ctgtgaaagt ctcatccaag ctgtgtcttc   1740
   tggtccaggc gtggcccctg gccccagggt ttctgatagg ctcctttagg atggtatctt   1800
   gggtccagcg ggccagggtg tgggagagga gtcactaaga tccctccaat cagaagggag   1860
   cttacaaagg aaccaaggca aagcctgtag actcaggaag ctaagtggcc agagactata   1920
   gtggccagtc atcccatgtc cacagaggat cttggtccag agctgccaaa gtgtcacctc   1980
   tccctgcctg cacctctggg gaaaagagga cagcatgtgg ccactgggca cctgtctcaa   2040
   gaagtcagga tcacacactc agtccttgtt tctccaggtt cccttgttct tgtctcgggg  2100
   agggagggac gagtgtcctg tctgtccttc ctgcctgtct gtgagtctgt gttgcttctc   2160
   catctgtcct agcctgagtg tgggtggaac aggcatgagg agagtgtggc tcaggggcca   2220
   ataaactctg ccttgactcc tcttaaaaaa aaaaaaaaaa aaaaaaaaaa aaaaaaaaaa   2280
   aaaaaaaaaa aaaaaaaaaa a                                             2301
<210> 9
   <211> 506
   <212> PRT
   <213> Mus musculus
<400> 9
   His Ala Ser Ala His Ala Ser Gly Met Ala Lys Leu Gly Val Glu Ala
   1 5 10 15
   Ala Leu Ile Asn Thr Asn Leu Arg Arg Asp Ala Leu Arg His Cys Leu
   20 25 30
   Asp Thr Ser Lys Ala Arg Ala Leu Ile Phe Gly Ser Glu Met Ala Ser
   35 40 45
   Ala Ile Cys Glu Ile His Ala Ser Leu Glu Pro Thr Leu Ser Leu Phe
   50 55 60
   Cys Ser Gly Ser Trp Glu Pro Ser Thr Val Pro Val Ser Thr Glu His
   65 70 75 80
   Leu Asp Pro Leu Leu Glu Asp Ala Pro Lys His Leu Pro Ser His Pro
   85 90 95
   Asp Lys Gly Phe Thr Asp Lys Leu Phe Tyr Ile Tyr Thr Ser Gly Thr
   100 105 110
   Thr Gly Leu Pro Lys Ala Ala Ile Val Val His Ser Arg Tyr Tyr Arg
   115 120 125
   Met Ala Ser Leu Val Tyr Tyr Gly Phe Arg Met Arg Pro Asp Asp Ile
   130 135 140
   Val Tyr Asp Cys Leu Pro Leu Tyr His Ser Ser Arg Lys His Arg Gly
   145 150 155 160
   Asp Trp Gln Cys Leu Leu His Gly Met Thr Val Val Ile Arg Lys Lys
   165 170 175
   Phe Ser Ala Ser Arg Phe Trp Asp Asp Cys Ile Lys Tyr Asn Cys Thr
   180 185 190
   Val Val Gln Tyr Ile Gly Glu Leu Cys Arg Tyr Leu Leu Asn Gln Pro
   195 200 205
   Pro Arg Glu Ala Glu Ser Arg His Lys Val Arg Met Ala Leu Gly Asn
   210 215 220
   Gly Leu Arg Gln Ser Ile Trp Thr Asp Phe Ser Ser Arg Phe His Ile
   225 230 235 240
   Pro Gln Val Ala Glu Phe Tyr Gly Ala Thr Glu Cys Asn Cys Ser Leu
                   245                 250                 255
   Gly Asn Phe Asp Ser Arg Val Gly Ala Cys Gly Phe Asn Ser Arg Ile
               260                 265                 270
   Leu Ser Phe Val Tyr Pro Ile Arg Leu Val Arg Val Asn Glu Asp Thr
           275                 280                 285
   Met Glu Leu Ile Arg Gly Pro Asp Gly Val Cys Ile Pro Cys Gln Pro
       290                 295                 300
   Gly Gln Pro Gly Gln Leu Val Gly Arg Ile Ile Gln Gln Asp Pro Leu
   305                 310                 315                 320
   Arg Arg Phe Asp Gly Tyr Leu Asn Gln Gly Ala Asn Asn Lys Lys Ile
                   325                 330                 335
   Ala Asn Asp Val Phe Lys Lys Gly Asp Gln Ala Tyr Leu Thr Gly Asp
               340                 345                 350
   Val Leu Val Met Asp Glu Leu Gly Tyr Leu Tyr Phe Arg Asp Arg Thr
           355                 360                 365
   Gly Asp Thr Phe Arg Trp Lys Gly Glu Asn Val Ser Thr Thr Glu Val
       370                 375                 380
   Glu Gly Thr Leu Ser Arg Leu Leu His Met Ala Asp Val Ala Val Tyr
   385                 390                 395                 400
   Gly Val Glu Val Pro Gly Thr Glu Gly Arg Ala Gly Met Ala Ala Val
                   405                 410                 415
   Ala Ser Pro Ile Ser Asn Cys Asp Leu Glu Ser Phe Ala Gln Thr Leu
               420                 425                 430
   Lys Lys Glu Leu Pro Leu Tyr Ala Arg Pro Ile Phe Leu Arg Phe Leu
           435                 440                 445
   Pro Glu Leu His Lys Thr Gly Thr Phe Lys Phe Gln Lys Thr Glu Leu
       450                 455                 460
   Arg Lys Glu Gly Phe Asp Pro Ser Val Val Lys Asp Pro Leu Phe Tyr
   465                 470                 475                 480
   Leu Asp Ala Arg Lys Gly Cys Tyr Val Ala Leu Asp Gln Glu Ala Tyr
                   485                 490                 495
   Thr Arg Ile Gln Ala Gly Glu Glu Lys Leu
               500                 505
<210> 10
   <211> 2277
   <212> DNA
   <213> Mus musculus
<400> 10
   cactcatcag agctaagaga gactacacgc tctcatctac ttcagaaaga gccaatgcca      60
   tgggtatttg gaagaaacta accttactgc tgttgctgct tctgctggtt ggcctggggc     120
   agcccccatg gccagcagct atggctctgg ccctgcgttg gttcctggga gaccccacat     180
   gccttgtgct gcttggcttg gcattgctgg gcagaccctg gatcagctcc tggatgcccc     240
   actggctgag cctggtagga gcagctctta ccttattcct attgcctcta cagccacccc     300
   cagggctacg ctggctgcat aaagatgtgg ctttcacctt caagatgctt ttctatggcc     360
   taaagttcag gcgacgcctt aacaaacatc ctccagagac ctttgtggat gctttagagc     420
   ggcaagcact ggcatggcct gaccgggtgg ccttggtgtg tactgggtct gagggctcct     480
   caatcacaaa tagccagctg gatgccaggt cctgtcaggc agcatgggtc ctgaaagcaa     540
   agctgaagga tgccgtaatc cagaacacaa gagatgctgc tgctatctta gttctcccgt     600
   ccaagaccat ttctgctttg agtgtgtttc tggggttggc caagttgggc tgccctgtgg     660
   cctggatcaa tccacacagc cgagggatgc ccttgctaca ctctgtacgg agctctgggg     720
   ccagtgtgct gattgtggat ccagacctcc aggagaacct ggaagaagtc cttcccaagc     780
   tgctagctga gaacattcac tgcttctacc ttggccacag ctcacccacc ccgggagtag     840
   aggctctggg agcttccctg gatgctgcac cttctgaccc agtacctgcc agccttcgag     900
   ctacgattaa gtggaaatct cctgccatat tcatctttac ttcagggacc actggactcc     960
   caaagccagc catcttatca catgagcggg tcatacaagt gagcaacgtg ctgtccttct     1020
   gtggatgcag agctgatgat gtggtctatg acgtcctacc tctgtaccat acgatagggc     1080
   ttgtccttgg attccttggc tgcttacaag ttggagccac ctgtgtcctg gcccccaagt     1140
   tctctgcctc ccgattctgg gctgagtgcc ggcagcatgg cgtaacagtg atcttgtatg     1200
   tgggtgaaat cctgcggtac ttgtgtaacg tccctgagca accagaagac aagatacata     1260
   cagtgcgctt ggccatggga actggacttc gggcaaatgt gtggaaaaac ttccagcaac     1320
   gctttggtcc cattcggatc tgggaattct acggatccac agagggcaat gtgggcttaa     1380
   tgaactatgt gggccactgc ggggctgtgg gaaggaccag ctgcatcctt cgaatgctga     1440
   ctccctttga gcttgtacag ttcgacatag agacagcaga gcctctgagg gacaaacagg     1500
   gtttttgcat tcctgtggag ccaggaaagc caggacttct tttgaccaag gttcgaaaga     1560
   accaaccctt cctgggctac cgtggttccc aggccgagtc caatcggaaa cttgttgcga     1620
   atgtacgacg cgtaggagac ctgtacttca acactgggga cgtgctgacc ttggaccagg     1680
   aaggcttctt ctactttcaa gaccgccttg gtgacacctt ccggtggaag ggcgaaaacg     1740
   tatctactgg agaggtggag tgtgttttgt ctagcctaga cttcctagag gaagtcaatg     1800
   tctatggtgt gcctgtgcca gggtgtgagg gtaaggttgg catggctgct gtgaaactgg     1860
   ctcctgggaa gacttttgat gggcagaagc tataccagca tgtccgctcc tggctccctg     1920
   cctatgccac acctcatttc atccgtatcc aggattccct ggagatcaca aacacctaca     1980
   agctggtaaa gtcacggctg gtgcgtgagg gttttgatgt ggggatcatt gctgaccccc     2040
   tctacatact ggacaacaag gcccagacct tccggagtct gatgccagat gtgtaccagg     2100
   ctgtgtgtga aggaacctgg aatctctgac cacctagcca actggaaggc aatccaaaag     2160
   tgtagagatt gacactagtc agcttcacaa agttgtccgg gttccagatg cccatggccc     2220
   agtagtactt agagaataaa cttgaatgtg tatacaaaaa aaaaaaaaaa aaaaaaa        2277
<210> 11
   <211> 662
   <212> PRT
   <213> Mus musculus
<400> 11
   Met Ala Leu Ala Leu Arg Trp Phe Leu Gly Asp Pro Thr Cys Leu Val
    1               5                  10                  15
   Leu Leu Gly Leu Ala Leu Leu Gly Arg Pro Trp Ile Ser Ser Trp Met
               20                  25                  30
   Pro His Trp Leu Ser Leu Val Gly Ala Ala Leu Thr Leu Phe Leu Leu
           35                  40                  45
   Pro Leu Gln Pro Pro Pro Gly Leu Arg Trp Leu His Lys Asp Val Ala
       50                  55                  60
   Phe Thr Phe Lys Met Leu Phe Tyr Gly Leu Lys Phe Arg Arg Arg Leu
   65                  70                  75                  80
   Asn Lys His Pro Pro Glu Thr Phe Val Asp Ala Leu Glu Arg Gln Ala
                   85                  90                  95
   Leu Ala Trp Pro Asp Arg Val Ala Leu Val Cys Thr Gly Ser Glu Gly
               100                 105                 110
   Ser Ser Ile Thr Asn Ser Gln Leu Asp Ala Arg Ser Cys Gln Ala Ala
           115                 120                 125
   Trp Val Leu Lys Ala Lys Leu Lys Asp Ala Val Ile Gln Asn Thr Arg
       130                 135                 140
   Asp Ala Ala Ala Ile Leu Val Leu Pro Ser Lys Thr Ile Ser Ala Leu
   145                 150                 155                 160
   Ser Val Phe Leu Gly Leu Ala Lys Leu Gly Cys Pro Val Ala Trp Ile
                   165                 170                 175
   Asn Pro His Ser Arg Gly Met Pro Leu Leu His Ser Val Arg Ser Ser
               180                 185                 190
   Gly Ala Ser Val Leu Ile Val Asp Pro Asp Leu Gln Glu Asn Leu Glu
           195                 200                 205
   Glu Val Leu Pro Lys Leu Leu Ala Glu Asn Ile His Cys Phe Tyr Leu
       210                 215                 220
   Gly His Ser Ser Pro Thr Pro Gly Val Glu Ala Leu Gly Ala Ser Leu
   225                 230                 235                 240
   Asp Ala Ala Pro Ser Asp Pro Val Pro Ala Ser Leu Arg Ala Thr Ile
                   245                 250                 255
   Lys Trp Lys Ser Pro Ala Ile Phe Ile Phe Thr Ser Gly Thr Thr Gly
               260                 265                 270
   Leu Pro Lys Pro Ala Ile Leu Ser His Glu Arg Val Ile Gln Val Ser
           275                 280                 285
   Asn Val Leu Ser Phe Cys Gly Cys Arg Ala Asp Asp Val Val Tyr Asp
       290                 295                 300
   Val Leu Pro Leu Tyr His Thr Ile Gly Leu Val Leu Gly Phe Leu Gly
   305                 310                 315                 320
   Cys Leu Gln Val Gly Ala Thr Cys Val Leu Ala Pro Lys Phe Ser Ala
                   325                 330                 335
   Ser Arg Phe Trp Ala Glu Cys Arg Gln His Gly Val Thr Val Ile Leu
               340                 345                 350
   Tyr Val Gly Glu Ile Leu Arg Tyr Leu Cys Asn Val Pro Glu Gln Pro
           355                 360                 365
   Glu Asp Lys Ile His Thr Val Arg Leu Ala Met Gly Thr Gly Leu Arg
       370                 375                 380
   Ala Asn Val Trp Lys Asn Phe Gln Gln Arg Phe Gly Pro Ile Arg Ile
   385                 390                 395                 400
   Trp Glu Phe Tyr Gly Ser Thr Glu Gly Asn Val Gly Leu Met Asn Tyr
                   405                 410                 415
   Val Gly His Cys Gly Ala Val Gly Arg Thr Ser Cys Ile Leu Arg Met
               420                 425                 430
   Leu Thr Pro Phe Glu Leu Val Gln Phe Asp Ile Glu Thr Ala Glu Pro
           435                 440                 445
   Leu Arg Asp Lys Gln Gly Phe Cys Ile Pro Val Glu Pro Gly Lys Pro
       450                 455                 460
   Gly Leu Leu Leu Thr Lys Val Arg Lys Asn Gln Pro Phe Leu Gly Tyr
   465                 470                 475                 480
   Arg Gly Ser Gln Ala Glu Ser Asn Arg Lys Leu Val Ala Asn Val Arg
                   485                 490                 495
   Arg Val Gly Asp Leu Tyr Phe Asn Thr Gly Asp Val Leu Thr Leu Asp
               500                 505                 510
   Gln Glu Gly Phe Phe Tyr Phe Gln Asp Arg Leu Gly Asp Thr Phe Arg
           515                 520                 525
   Trp Lys Gly Glu Asn Val Ser Thr Gly Glu Val Glu Cys Val Leu Ser
       530                 535                 540
   Ser Leu Asp Phe Leu Glu Glu Val Asn Val Tyr Gly Val Pro Val Pro
   545                 550                 555                 560
   Gly Cys Glu Gly Lys Val Gly Met Ala Ala Val Lys Leu Ala Pro Gly
                   565                 570                 575
   Lys Thr Phe Asp Gly Gln Lys Leu Tyr Gln His Val Arg Ser Trp Leu
               580                 585                 590
   Pro Ala Tyr Ala Thr Pro His Phe Ile Arg Ile Gln Asp Ser Leu Glu
           595                 600                 605
   Ile Thr Asn Thr Tyr Lys Leu Val Lys Ser Arg Leu Val Arg Glu Gly
       610                 615                 620
   Phe Asp Val Gly Ile Ile Ala Asp Pro Leu Tyr Ile Leu Asp Asn Lys
   625                 630                 635                 640
   Ala Gln Thr Phe Arg Ser Leu Met Pro Asp Val Tyr Gln Ala Val Cys
                   645                 650                 655
   Glu Gly Thr Trp Asn Leu
               660
<210> 12
   <211> 1622
   <212> DNA
   <213> Homo sapiens
<220>
   <221> misc_feature
   <222> (1)...(1622)
   <223> n = A,T,C or G
<400> 12
   atgggattga ctctttcctg gacaaagtgg atgaagtatc aactgaacct atcccagagt      60
   catggaggtc tgaagtcact ttttccactc ctgccttata catttatact tctggaacca     120
   caggtcttcc aaaagcagcc atgatcactc atcagcgcat atggtatgga actggcctca     180
   cttttgtaag cggattgaag gcagatgatg tcatctatat cactctgccc ttttaccaca     240
   gtgctgcact actgattggc attcacggat gtattgtggc tggtgctact cttgccttgc     300
   ggactaaatt ttcagccagc cagttttggg atgactgcag aaaatacaac gtcactgtca     360
   ttcagtatat cggtgaactg cttcggtatt tatgcaactc accacagaaa ccaaatgacc     420
   gtgatcataa agtgagactg gcactgggaa atggcttacg aggagatgtg tggagacaat     480
   ttgtcaagag atttggggac atatgcatct atgagttcta tgctgccact gaaggcaata     540
   ttggatttat gaattatgcg agaaaagttg gtgctgttgg aagagtaaac tacctacaga     600
   aaaaaatcat aacttatgac ctgattaaat atgatgtgga gaaagatgaa cctgtccgtg     660
   atgaaaatgg atattgcgtc agagttccca aaggtgaagt tggacttctg gtttgcaaaa     720
   tcacacaact tacaccattt aatggctatg ctggagcaaa ggctcagaca gagaagaaaa     780
   aactgagaga tgtctttaag aaaggagacc tctatttcaa cagtggagat ctcttaatgg     840
   ttgaccatga aaatttcatc tatttccacg acagagttgg agatacattc cggtggaaag     900
   gggaaaatgt ggccaccact gaagttgctg atatagttgg actggttgat ttttttccaa     960
   ggaagtaaaa tgtttatggg agtgcatggg ccaagatnat ggaggttcga attggcatgg    1020
   cnttccnttc aaaatggaaa gaaaaccatg gaatttgatg gaaagaaatt ttttcagnac    1080
   attgctgata accnacctag ttatgcaagg ccccggtttt ntaagaanac aggacaccat    1140
   tgagatcact ggaattttta aacaccgcaa aatgaccttt ggtggaggag ggctttaacc    1200
   cngctgtcat caaagatgcc ttgtattttc ttggatgaca cagcaaaaat gtatgtgcct    1260
   atgactgagg acatntataa tgccataagt gntaaaaccc tgaaattntg aatattccca    1320
   ggaggataat tcaacatttc cagaaagaaa ctgaatggac agccacttga tataatccaa    1380
   ctttaatttg attgaagatt gtgaggaaat tttgtaggaa atttgcatac ccgtaaaggg    1440
   agactttttt aaataacagt tgagtctttg caagtaaaaa gatttagaga ttattatttt    1500
   tcagtgtgca cctactgttt gtatttgcaa actgagcttg ttggagggaa ggcattattt    1560
   tttaaaatac ttagtaaatt aaagaacacc aacatgtgaa aaaaaaaaaa aaaaaaaaaa    1620
   aa                                                                   1622
<210> 13
   <211> 286
   <212> PRT
   <213> Homo sapiens
<400> 13
   Tyr Ile Tyr Thr Ser Gly Thr Thr Gly Leu Pro Lys Ala Ala Met Ile
   1 5 10 15
   Thr His Gln Arg Ile Trp Tyr Gly Thr Gly Leu Thr Phe Val Ser Gly
   20 25 30
   Leu Lys Ala Asp Asp Val Ile Tyr Ile Thr Leu Pro Phe Tyr His Ser
   35 40 45
   Ala Ala Leu Leu Ile Gly Ile His Gly Cys Ile Val Ala Gly Ala Thr
   50 55 60
   Leu Ala Leu Arg Thr Lys Phe Ser Ala Ser Gln Phe Trp Asp Asp Cys
   65 70 75 80
   Arg Lys Tyr Asn Val Thr Val Ile Gln Tyr Ile Gly Glu Leu Leu Arg
   85 90 95
   Tyr Leu Cys Asn Ser Pro Gln Lys Pro Asn Asp Arg Asp His Lys Val
   100 105 110
   Arg Leu Ala Leu Gly Asn Gly Leu Arg Gly Asp Val Trp Arg Gln Phe
   115 120 125
   Val Lys Arg Phe Gly Asp Ile Cys Ile Tyr Glu Phe Tyr Ala Ala Thr
   130 135 140
   Glu Gly Asn Ile Gly Phe Met Asn Tyr Ala Arg Lys Val Gly Ala Val
   145 150 155 160
   Gly Arg Val Asn Tyr Leu Gln Lys Lys Ile Ile Thr Tyr Asp Leu Ile
   165 170 175
   Lys Tyr Asp Val Glu Lys Asp Glu Pro Val Arg Asp Glu Asn Gly Tyr
   180 185 190
   Cys Val Arg Val Pro Lys Gly Glu Val Gly Leu Leu Val Cys Lys Ile
   195 200 205
   Thr Gln Leu Thr Pro Phe Asn Gly Tyr Ala Gly Ala Lys Ala Gln Thr
   210 215 220
   Glu Lys Lys Lys Leu Arg Asp Val Phe Lys Lys Gly Asp Leu Tyr Phe
   225 230 235 240
   Asn Ser Gly Asp Leu Leu Met Val Asp His Glu Asn Phe Ile Tyr Phe
   245 250 255
   His Asp Arg Val Gly Asp Thr Phe Arg Trp Lys Gly Glu Asn Val Ala
   260 265 270
   Thr Thr Glu Val Ala Asp Ile Val Gly Leu Val Asp Phe Phe
   275 280 285
<210> 14
   <211> 753
   <212> DNA
   <213> Homo sapiens
<400> 14
   caattcggga cccccagggg cactgtatgg ccacatctcc aggtgagcca ggggaagttg      60
   ctaaaggatg tcttccggcc tggggatgtt ttcttcaaca ctggggacct gctggtctgc     120
   gatgaccaag gttttctccg cttccatgat cgtactggag acaccttcag gtggaaaggg     180
   gagaatgtgg ccacaaccga ggtggcagag gtcttcgagg ccctagattt tcttcaggag     240
   gtgaacgtct atggagtcac tgtgccaggg catgaaggca gggctggaat ggcagcccta     300
   gttctgcgtc ccccccacgc tttggacctt atgcagctct acacccacgt gtctgagaac     360
   ttgccacctt atgcccggcc ccgattcctc aggctccagg agtctttggc caccacagag     420
   accttcaaac agcagaaagt tcggatggca aatgagggct tcgaccccag caccctgtct     480
   gacccactgt acgttctgga ccaggctgta ggtgcctacc tgcccctcac aactgcccgg     540
   tacagcgccc tcctggcagg aaaccttcga atctgagaac ttccacacct gaggcacctg     600
   agagaggaac tctgtggggt gggggccgtt gcaggtgtac tgggctgtca gggatctttt     660
   ctataccaga actgcggtca ctattttgta ataaatgtgg ctggagctga tccagctgtc      720
   tctgacctac aaaaaaaaaa aaaaaaaaaa aaa                                  753
<210> 15
   <211> 191
   <212> PRT
   <213> Homo sapiens
<400> 15
   Gln Phe Gly Thr Pro Arg Gly Thr Val Trp Pro His Leu Gln Val Ser
    1               5                  10                  15
   Gln Gly Lys Leu Leu Lys Asp Val Phe Arg Pro Gly Asp Val Phe Phe
               20                  25                  30
   Asn Thr Gly Asp Leu Leu Val Cys Asp Asp Gln Gly Phe Leu Arg Phe
           35                  40                  45
   His Asp Arg Thr Gly Asp Thr Phe Arg Trp Lys Gly Glu Asn Val Ala
       50                  55                  60
   Thr Thr Glu Val Ala Glu Val Phe Glu Ala Leu Asp Phe Leu Gln Glu
   65                  70                  75                  80
   Val Asn Val Tyr Gly Val Thr Val Pro Gly His Glu Gly Arg Ala Gly
                   85                  90                  95
   Met Ala Ala Leu Val Leu Arg Pro Pro His Ala Leu Asp Leu Met Gln
               100                 105                 110
   Leu Tyr Thr His Val Ser Glu Asn Leu Pro Pro Tyr Ala Arg Pro Arg
           115                 120                 125
   Phe Leu Arg Leu Gln Glu Ser Leu Ala Thr Thr Glu Thr Phe Lys Gln
       130                 135                 140
   Gln Lys Val Arg Met Ala Asn Glu Gly Phe Asp Pro Ser Thr Leu Ser
   145                 150                 155                 160
   Asp Pro Leu Tyr Val Leu Asp Gln Ala Val Gly Ala Tyr Leu Pro Leu
                   165                 170                 175
   Thr Thr Ala Arg Tyr Ser Ala Leu Leu Ala Gly Asn Leu Arg Ile
               180                 185                 190
<210> 16
   <211> 734
   <212> DNA
   <213> Homo sapiens
<220>
   <221> misc_feature
   <222> (1)...(734)
   <223> n = A,T,C or G
<400> 16
   tcaagtacaa ctgcacgatt gtcatancat tggtgaactg tgccgntacc tcctgaacca    60
   gccaccgcgg gaggcagaaa accagcacca ggttcgcatg gcactaggca atggcctccg   120
   gcagtccatc tggaccaact tttccagccg cttccacata ccccaggtgg ctgagttyta   180
   cggggccaca gagtgcaact gtagcctggg caacttcgac agccaggtgg gggcctgtgg   240
   tttcaatagc cgcatcctgt ccttcgtgta ccccatccgg ttggtacgtg tcaacgagga   300
   caccatggag ctgatccggg ggcccgacgg cgtctgcatt ccctgccagc caggtgagcc   360
   gggccagctg gtgggccgca tcatccagaa agaccccctg cgccgcttcg atggctacct   420
   caaccagggc gccaacaaca agaagattgc caaggatgtc ttcaagaagg gggaccaggc   480
   ctaccttact ggtgatgtgc tggtgatgga cgagctgggc tacctgtact tccgagaccg   540
   cactggggac acgttccgct ggaaaggtga gaacgtgtcc accaccgagg tggaaggcac   600
   actcagccgc ctgctggaca tggctgacgt ggccgtgtat ggtgtcgagg tgccaggaac   660
   cgagggccgg gccggaatgg ctgctgtggc cagccccact ggcaactgtg acctgggagc   720
   gctttgctca ggtc                                                     734
<210> 17
   <211> 213
   <212> PRT
   <213> Homo sapiens
<400> 17
   Ile Gly Glu Leu Cys Arg Tyr Leu Leu Asn Gln Pro Pro Arg Glu Ala
   1               5                   10                  15
   Glu Asn Gln His Gln Val Arg Met Ala Leu Gly Asn Gly Leu Arg Gln
               20                  25                  30
   Ser Ile Trp Thr Asn Phe Ser Ser Arg Phe His Ile Pro Gln Val Ala
           35                  40                  45
   Glu Phe Tyr Gly Ala Thr Glu Cys Asn Cys Ser Leu Gly Asn Phe Asp
       50                  55                  60
   Ser Gln Val Gly Ala Cys Gly Phe Asn Ser Arg Ile Leu Ser Phe Val
   65                  70                  75                  80
   Tyr Pro Ile Arg Leu Val Arg Val Asn Glu Asp Thr Met Glu Leu Ile
                   85                  90                  95
   Arg Gly Pro Asp Gly Val Cys Ile Pro Cys Gln Pro Gly Glu Pro Gly
               100                 105                 110
   Gln Leu Val Gly Arg Ile Ile Gln Lys Asp Pro Leu Arg Arg Phe Asp
           115                 120                 125
   Gly Tyr Leu Asn Gln Gly Ala Asn Asn Lys Lys Ile Ala Lys Asp Val
       130                 135                 140
   Phe Lys Lys Gly Asp Gln Ala Tyr Leu Thr Gly Asp Val Leu Val Met
   145                 150                 155                 160
   Asp Glu Leu Gly Tyr Leu Tyr Phe Arg Asp Arg Thr Gly Asp Thr Phe
                   165                 170                 175
   Arg Trp Lys Gly Glu Asn Val Ser Thr Thr Glu Val Glu Gly Thr Leu
               180                 185                 190
   Ser Arg Leu Leu Asp Met Ala Asp Val Ala Val Tyr Gly Val Glu Val
           195                 200                 205
   Pro Gly Thr Glu Gly
       210
<210> 18
   <211> 1278
   <212> DNA
   <213> Homo sapiens
<220>
   <221> mise_feature
   <222> (1)...(1278)
   <223> n = A,T,C or G
<400> 18
   cntgcctctt gtaccacgtg atgggacttt gtcgttggga tcctcggctg cttagatctc      60
   ggagccacct gtgttctggc ccccaagttc tctacttcct gcttctggga tgactgtcgg     120
   cagcatggcg tgacagtgat cctgtatgtg ggcgagctcc tgcgmtactt gtgtaacatt     180
   ccccagcaac cagaggaccg gacacataca gtccgcctgg caatgggcaa tggactacgg     240
   gctgatgtgt ggggagacct tccagcagcg tttcggtcct atttcggatc tngggaagtc     300
   ttacgggcty ccacagaagg gcaacatggg gctttagttc aactattgtt gggggcgctg     360
   cggggscctg grggcaaaga tggagcttgc ctcctccgaa tgctgtcccc ctttgagctg     420
   gtgcagttcg acatggaggc ggcggagcct gtgagggaca atcagggctt ctgcatccct     480
   gtagggctag gggagccggg gctgctgttg accaaggtgg taagccagca acccttcgtg     540
   ggctaccgcg gcccccgaga gctgtcggaa cggaagctgg tgcgcaacgt gcggcaatcg     600
   ggcgacgttt actacaacac cggggacgta ctggccatgg accgcgaagg cttcctctac     660
   ttccgcgacc gactcgggga caccttccga tggaagggcg agaacgtgtc cacgcacgag     720
   gtggagggcg tgttgtcgca ggtggacttc ttgcaacagg ttaacgtgta tggcgtgtgc     780
   gtgccaggtt gtgagggtaa ggtgggcatg gctgctgtgg cattagcccc cggccagact     840
   ttcgacgggg agaagttgta ccagcacgtt cgcgcttggc tccctgccta cgctaccccc     900
   catttcatcc gcatccagga cgccatggag gtcaccagca cgttcaaact gatgaagacc     960
   cggttggtgc gtgagggctt caatgtgggg atcgtggttg accctctgtt tgtactggac    1020
   aaccgggccc agtccttccg gcccctgacg gcagaaatgt accaggctgt gtgtgaggga    1080
   acctggaggc tctgatcacc tggccaaccc actggggtag ggatcaaagc cagccacccc    1140
   caccccaaca cactcggtgt ccctttcatc ctgggcctgt gtgaatccca gcctggccat    1200
   accctcaacc tcagtgggct ggaaatgaca gtgggccctg tagcagtggc agaataaact    1260
   cagmtgygtt cacagaaa                                                  1278
<210> 19
   <211> 199
   <212> PRT
   <213> Homo sapiens
<400> 19
   Glu Gly Gln His Gly Ala Leu Val Gln Leu Leu Leu Gly Ala Leu Arg
    1               5                  10                  15
   Gly Pro Gly Gly Lys Asp Gly Ala Cys Leu Leu Arg Met Leu Ser Pro
               20                  25                  30
   Phe Glu Leu Val Gln Phe Asp Met Glu Ala Ala Glu Pro Val Arg Asp
           35                  40                  45
   Asn Gln Gly Phe Cys Ile Pro Val Gly Leu Gly Glu Pro Gly Leu Leu
       50                  55                  60
   Leu Thr Lys Val Val Ser Gln Gln Pro Phe Val Gly Tyr Arg Gly Pro
   65                  70                  75                  80
   Arg Glu Leu Ser Glu Arg Lys Leu Val Arg Asn Val Arg Gln Ser Gly
                   85                  90                  95
   Asp Val Tyr Tyr Asn Thr Gly Asp Val Leu Ala Met Asp Arg Glu Gly
               100                 105                 110
   Phe Leu Tyr Phe Arg Asp Arg Leu Gly Asp Thr Phe Arg Trp Lys Gly
           115                 120                 125
   Glu Asn Val Ser Thr His Glu Val Glu Gly Val Leu Ser Gln Val Asp
       130                 135                 140
   Phe Leu Gln Gln Val Asn Val Tyr Gly Val Cys Val Pro Gly Cys Glu
   145                 150                 155                 160
   Gly Lys Val Gly Met Ala Ala Val Ala Leu Ala Pro Gly Gln Thr Phe
                   165                 170                 175
   Asp Gly Glu Lys Leu Tyr Gln His Val Arg Ala Trp Leu Pro Ala Tyr
               180                 185                 190
   Ala Thr Pro His Phe Ile Arg
           195
<210> 20
   <211> 1361
   <212> DNA
   <213> Homo sapiens
<400> 20
   cgcttgtgtg ttaaagaaga aattttcagc aagccagttt tggagtgact gcaagaagta      60
   tgatgtgact gtgtttcagt atattggaga actttgtcgc tacctttgca aacaatctaa     120
   gagagaagga gaaaaggatc ataaggtgcg tttggcaatt ggaaatggca tacggagtga     180
   tgtatggaga gaatttttag acagatttgg aaatataaag gtgtgtgaac tttatgcagc     240
   taccgaatca agcatatctt tcatgaacta cactgggaga attggagcaa ttgggagaac     300
   aaatttgttt tacaaacttc tttccacttt tgacttaata aagtatgact ttcagaaaga     360
   tgaacccatg agaaatgagc agggttgggt attcatgaga aaaaggagac ctggacttct     420
   catttctcga gtgaatgcaa aaaatccctt ctttggctat gctgggcctt ataagcacac     480
   aaaagacaaa ttgctttgtg atgtttttaa gaagggagat gtttacctta atactggaga     540
   cttaatagtc caggatcagg acaatttcct ttatttttgg gaccgtactg gagacacttt     600
   cagatggaaa ggagaaaatg tcgcaaccac tgaggttgct gatgttattg gaatgttgga     660
   tttcatacag gaagcaaacg tctatggtgt ggctatatca ggttatgaag gaagagcagg     720
   aatggcttct attattttaa aaccaaatac atctttagat ttggaaaaag tttatgaaca     780
   agttgtaaca tttctaccag cttatgcttg tccacgattt ttaagaattc aggaaaaaat     840
   ggaagcaaca ggaacattca aactattgaa gcatcagttg gtggaagatg gatttaatcc     900
   actgaaaatt tctgaaccac tttacttcat ggataacttg aaaaagtctt atgttctact     960
   gaccagggaa ctttatgatc aaataatgtt aggggaaata aaactttaag atttttatat     1020
   ctagaacttt catatgcttt cttaggaaga gtgagagggg ggtatatgat tctttatgaa     1080
   atggggaaag ggagctaaca ttaattatgc atgtactata tttccttaat atgagagata     1140
   attttttaat tgcataagaa ttttaatttc ttttaattga tataaacaga gttgattatt      1200
   ctttttatct atttggagat tcagtgcata actaagtatt ttccttaata ctaaagattt     1260
   taaataataa atagtggcta gcggtttgga caatcactaa aaatgtactt tctaataagt     1320
   aaaatttcta attttgaata aaagattaaa ttttactgaa a                         1361
<210> 21
   <211> 335
   <212> PRT
   <213> Homo sapiens
<400> 21
   Ala Cys Val Leu Lys Lys Lys Phe Ser Ala Ser Gln Phe Trp Ser Asp
    1               5                  10                  15
   Cys Lys Lys Tyr Asp Val Thr Val Phe Gln Tyr Ile Gly Glu Leu Cys
               20                  25                  30
   Arg Tyr Leu Cys Lys Gln Ser Lys Arg Glu Gly Glu Lys Asp His Lys
           35                  40                  45
   Val Arg Leu Ala Ile Gly Asn Gly Ile Arg Ser Asp Val Trp Arg Glu
       50                  55                  60
   Phe Leu Asp Arg Phe Gly Asn Ile Lys Val Cys Glu Leu Tyr Ala Ala
   65                  70                  75                  80
   Thr Glu Ser Ser Ile Ser Phe Met Asn Tyr Thr Gly Arg Ile Gly Ala
                   85                  90                  95
   Ile Gly Arg Thr Asn Leu Phe Tyr Lys Leu Leu Ser Thr Phe Asp Leu
               100                 105                 110
   Ile Lys Tyr Asp Phe Gln Lys Asp Glu Pro Met Arg Asn Glu Gln Gly
           115                 120                 125
   Trp Val Phe Met Arg Lys Arg Arg Pro Gly Leu Leu Ile Ser Arg Val
       130                 135                 140
   Asn Ala Lys Asn Pro Phe Phe Gly Tyr Ala Gly Pro Tyr Lys His Thr
   145                 150                 155                 160
   Lys Asp Lys Leu Leu Cys Asp Val Phe Lys Lys Gly Asp Val Tyr Leu
                   165                 170                 175
   Asn Thr Gly Asp Leu Ile Val Gln Asp Gln Asp Asn Phe Leu Tyr Phe
               180                 185                 190
   Trp Asp Arg Thr Gly Asp Thr Phe Arg Trp Lys Gly Glu Asn Val Ala
           195                 200                 205
   Thr Thr Glu Val Ala Asp Val Ile Gly Met Leu Asp Phe Ile Gln Glu
       210                 215                 220
   Ala Asn Val Tyr Gly Val Ala Ile Ser Gly Tyr Glu Gly Arg Ala Gly
   225                 230                 235                 240
   Met Ala Ser Ile Ile Leu Lys Pro Asn Thr Ser Leu Asp Leu Glu Lys
                   245                 250                 255
   Val Tyr Glu Gln Val Val Thr Phe Leu Pro Ala Tyr Ala Cys Pro Arg
               260                 265                 270
   Phe Leu Arg Ile Gln Glu Lys Met Glu Ala Thr Gly Thr Phe Lys Leu
           275                 280                 285
   Leu Lys His Gln Leu Val Glu Asp Gly Phe Asn Pro Leu Lys Ile Ser
       290                 295                 300
   Glu Pro Leu Tyr Phe Met Asp Asn Leu Lys Lys Ser Tyr Val Leu Leu
   305                 310                 315                 320
   Thr Arg Glu Leu Tyr Asp Gln Ile Met Leu Gly Glu Ile Lys Leu
                   325                 330                 335
<210> 22
   <211> 2007
   <212> DNA
   <213> Mycobacterium tuberculosis
<400> 22
   tagtcgataa cgtcaaggac gctctgcggg cctgcgcacc ttcctgaggt tggtcgacaa      60
   ccaattcgac atttcgcaaa cgaatcgagg gcttacgtgt ccgattacta cggcggcgca     120
   cacacaacgg tcaggctgat cgacctggca actcggatgc cgcgagtgtt ggcggacacg     180
   ccggtgattg tgcgtggggc aatgaccggg ctgctggccc ggccgaattc caaggcgtcg     240
   atcggcacgg tgttccagga ccgggccgct cgctacggtg accgagtctt cctgaaattc     300
   ggcgatcagc agctgaccta ccgcgacgct aacgccaccg ccaaccggta cgccgcggtg     360
   ttggccgccc gcggcgtcgg ccccggcgac gtcgttggca tcatgttgcg taactcaccc     420
   agcacagtct tggcgatgct ggccacggtc aagtgcggcg ctatcgccgg catgctcaac     480
   taccaccagc gcggcgaggt gttggcgcac agcctgggtc tgctggacgc gaaggtactg     540
   atcgcagagt ccgacttggt cagcgccgtc gccgaatgcg gcgcctcgcg cggccgggta     600
   gcgggcgacg tgctgaccgt cgaggacgtg gagcgattcg ccacaacggc gcccgccacc     660
   aacccggcgt cggcgtcggc ggtgcaagcc aaagacaccg cgttctacat cttcacctcg     720
   ggcaccaccg gatttcccaa ggccagtgtc atgacgcatc atcggtggct gcgggcgctg     780
   gccgtcttcg gagggatggg gctgcggctg aagggttccg acacgctcta cagctgcctg     840
   ccgctgtacc acaacaacgc gttaacggtc gcggtgtcgt cggtgatcaa ttctggggcg     900
   accctggcgc tgggtaagtc gttttcggcg tcgcggttct gggatgaggt gattgccaac     960
   cgggcgacgg cgttcgtcta catcggcgaa atctgccgtt atctgctcaa ccagccggcc    1020
   aagccgaccg accgtgccca ccaggtgcgg gtgatctgcg gtaacgggct gcggccggag    1080
   atctgggatg agttcaccac ccgcttcggg gtcgcgcggg tgtgcgagtt ctacgccgcc    1140
   agcgaaggca actcggcctt tatcaacatc ttcaacgtgc ccaggaccgc cggggtatcg    1200
   ccgatgccgc ttgcctttgt ggaatacgac ctggacaccg gcgatccgct gcgggatgcg    1260
   agcgggcgag tgcgtcgggt acccgacggt gaacccggcc tgttgcttag ccgggtcaac    1320
   cggctgcagc cgttcgacgg ctacaccgac ccggttgcca gcgaaaagaa gttggtgcgc    1380
   aacgcttttc gagatggcga ctgttggttc aacaccggtg acgtgatgag cccgcagggc    1440
   atgggccatg ccgccttcgt cgatcggctg ggcgacacct tccgctggaa gggcgagaat    1500
   gtcgccacca ctcaggtcga agcggcactg gcctccgacc agaccgtcga ggagtgcacg    1560
   gtctacggcg tccagattcc gcgcaccggc gggcgcgccg gaatggccgc gatcacactg    1620
   cgcgctggcg ccgaattcga cggccaggcg ctggcccgaa cggtttacgg tcacttgccc    1680
   ggctatgcac ttccgctctt tgttcgggta gtggggtcgc tggcgcacac cacgacgttc    1740
   aagagtcgca aggtggagtt gcgcaaccag gcctatggcg ccgacatcga ggatccgctg    1800
   tacgtactgg ccggcccgga cgaaggatat gtgccgtact acgccgaata ccctgaggag    1860
   gtttcgctcg gaaggcgacc gcagggctag cggattccgg gcgcagtctc gatacccgca    1920
   ctggacgctc gacggtaacc aggcactatg gatgcgtgcg ttcaacaccg ccggcctcag    1980
   ccggtcgttc aacaccgccg gcgttag                                        2007
<210> 23
   <211> 597
   <212> PRT
   <213> Mycobacterium tuberculosis
<400> 23
   Met Ser Asp Tyr Tyr Gly Gly Ala His Thr Thr Val Arg Leu Ile Asp
    1               5                  10                  15
   Leu Ala Thr Arg Met Pro Arg Val Leu Ala Asp Thr Pro Val Ile Val
               20                  25                  30
   Arg Gly Ala Met Thr Gly Leu Leu Ala Arg Pro Asn Ser Lys Ala Ser
           35                  40                  45
   Ile Gly Thr Val Phe Gln Asp Arg Ala Ala Arg Tyr Gly Asp Arg Val
       50                  55                  60
   Phe Leu Lys Phe Gly Asp Gln Gln Leu Thr Tyr Arg Asp Ala Asn Ala
   65                  70                  75                  80
   Thr Ala Asn Arg Tyr Ala Ala Val Leu Ala Ala Arg Gly Val Gly Pro
                   85                  90                  95
   Gly Asp Val Val Gly Ile Met Leu Arg Asn Ser Pro Ser Thr Val Leu
               100                 105                 110
   Ala Met Leu Ala Thr Val Lys Cys Gly Ala Ile Ala Gly Met Leu Asn
           115                 120                 125
   Tyr His Gln Arg Gly Glu Val Leu Ala His Ser Leu Gly Leu Leu Asp
       130                 135                 140
   Ala Lys Val Leu Ile Ala Glu Ser Asp Leu Val Ser Ala Val Ala Glu
   145                 150                 155                 160
   Cys Gly Ala Ser Arg Gly Arg Val Ala Gly Asp Val Leu Thr Val Glu
                   165                 170                 175
   Asp Val Glu Arg Phe Ala Thr Thr Ala Pro Ala Thr Asn Pro Ala Ser
               180                 185                 190
   Ala Ser Ala Val Gln Ala Lys Asp Thr Ala Phe Tyr Ile Phe Thr Ser
           195                 200                 205
   Gly Thr Thr Gly Phe Pro Lys Ala Ser Val Met Thr His His Arg Trp
       210                 215                 220
   Leu Arg Ala Leu Ala Val Phe Gly Gly Met Gly Leu Arg Leu Lys Gly
   225                 230                 235                 240
   Ser Asp Thr Leu Tyr Ser Cys Leu Pro Leu Tyr His Asn Asn Ala Leu
                   245                 250                 255
   Thr Val Ala Val Ser Ser Val Ile Asn Ser Gly Ala Thr Leu Ala Leu
               260                 265                 270
   Gly Lys Ser Phe Ser Ala Ser Arg Phe Trp Asp Glu Val Ile Ala Asn
           275                 280                 285
   Arg Ala Thr Ala Phe Val Tyr Ile Gly Glu Ile Cys Arg Tyr Leu Leu
       290                 295                 300
   Asn Gln Pro Ala Lys Pro Thr Asp Arg Ala His Gln Val Arg Val Ile
   305                 310                 315                 320
   Cys Gly Asn Gly Leu Arg Pro Glu Ile Trp Asp Glu Phe Thr Thr Arg
                   325                 330                 335
   Phe Gly Val Ala Arg Val Cys Glu Phe Tyr Ala Ala Ser Glu Gly Asn
               340                 345                 350
   Ser Ala Phe Ile Asn Ile Phe Asn Val Pro Arg Thr Ala Gly Val Ser
           355                 360                 365
   Pro Met Pro Leu Ala Phe Val Glu Tyr Asp Leu Asp Thr Gly Asp Pro
       370                 375                 380
   Leu Arg Asp Ala Ser Gly Arg Val Arg Arg Val Pro Asp Gly Glu Pro
   385                 390                 395                 400
   Gly Leu Leu Leu Ser Arg Val Asn Arg Leu Gln Pro Phe Asp Gly Tyr
                   405                 410                 415
   Thr Asp Pro Val Ala Ser Glu Lys Lys Leu Val Arg Asn Ala Phe Arg
               420                 425                 430
   Asp Gly Asp Cys Trp Phe Asn Thr Gly Asp Val Met Ser Pro Gln Gly
           435                 440                 445
   Met Gly His Ala Ala Phe Val Asp Arg Leu Gly Asp Thr Phe Arg Trp
       450                 455                 460
   Lys Gly Glu Asn Val Ala Thr Thr Gln Val Glu Ala Ala Leu Ala Ser
   465                 470                 475                 480
   Asp Gln Thr Val Glu Glu Cys Thr Val Tyr Gly Val Gln Ile Pro Arg
                   485                 490                 495
   Thr Gly Gly Arg Ala Gly Met Ala Ala Ile Thr Leu Arg Ala Gly Ala
               500                 505                 510
   Glu Phe Asp Gly Gln Ala Leu Ala Arg Thr Val Tyr Gly His Leu Pro
           515                 520                 525
   Gly Tyr Ala Leu Pro Leu Phe Val Arg Val Val Gly Ser Leu Ala His
       530                 535                 540
   Thr Thr Thr Phe Lys Ser Arg Lys Val Glu Leu Arg Asn Gln Ala Tyr
   545                 550                 555                 560
   Gly Ala Asp Ile Glu Asp Pro Leu Tyr Val Leu Ala Gly Pro Asp Glu
                   565                 570                 575
   Gly Tyr Val Pro Tyr Tyr Ala Glu Tyr Pro Glu Glu Val Ser Leu Gly
               580                 585                 590
   Arg Arg Pro Gln Gly
           595
<210> 24
   <211> 3704
   <212> DNA
   <213> Homo sapiens
<220>
   <221> CDS
   <222> (175)...(2112)
<400> 24
   tcgacccacg gcgtccggga ccccaaagca gaagcccgca cagtaggcac agcgcaccca     60
   agaagggtcc aggagtctgc agaaacagaa aggtccccgg cctcagcctc ctagtccctg    120
   cctgcctcct gcctgagctt ctgggagact gaaggcacgg cttgcagctt cagg atg      177
                                                               Met
                                                                1
   cgg gct ccg ggt gcg ggc gcg gcc tcg gtg gtc tcg ctg gcg ctg ttg      225
   Arg Ala Pro Gly Ala Gly Ala Ala Ser Val Val Ser Leu Ala Leu Leu
               5                   10                  15
   tgg ctg ctg ggg ctg ccg tgg acc tgg agc gcg gca gcg gcg ctc ggc      273
   Trp Leu Leu Gly Leu Pro Trp Thr Trp Ser Ala Ala Ala Ala Leu Gly
           20                  25                  30
   gtg tac gtg ggc agc ggc ggc tgg cgc ttc ctg cgc atc gtc tgc aag      321
   Val Tyr Val Gly Ser Gly Gly Trp Arg Phe Leu Arg Ile Val Cys Lys
       35                  40                  45
   acc gcg agg cga gac ctc ttc ggt ctc tct gtg ctg atc cgc gtg cgc      369
   Thr Ala Arg Arg Asp Leu Phe Gly Leu Ser Val Leu Ile Arg Val Arg
   50                  55                  60                  65
   ctg gag ctg cgg cgg cac cag cgt gcc ggc cac acc atc ccg cgc atc      417
   Leu Glu Leu Arg Arg His Gln Arg Ala Gly His Thr Ile Pro Arg Ile
                   70                  75                  80
   ttt cag gcg gta gtg cag cga cag ccc gag cgc ctg gcg ctg gtg gat      465
   Phe Gln Ala Val Val Gln Arg Gln Pro Glu Arg Leu Ala Leu Val Asp
               85                  90                  95
   gcc ggg acc ggc gag tgc tgg acc ttt gcg cag ctg gac gcc tac tcc      513
   Ala Gly Thr Gly Glu Cys Trp Thr Phe Ala Gln Leu Asp Ala Tyr Ser
           100                 105                 110
   aat gcg gta gcc aac ctc ttc cgc cag ctg ggc ttc gcg ccg ggc gac        561
   Asn Ala Val Ala Asn Leu Phe Arg Gln Leu Gly Phe Ala Pro Gly Asp
       115                 120                 125
   gtg gtg gcc atc ttc ctg gag ggc cgg ccg gag ttc gtg ggg ctg tgg        609
   Val Val Ala Ile Phe Leu Glu Gly Arg Pro Glu Phe Val Gly Leu Trp
   130                 135                 140                 145
   ctg ggc ctg gcc aag gcg ggc atg gag gcc gcg ctg ctc aac gtg aac        657
   Leu Gly Leu Ala Lys Ala Gly Met Glu Ala Ala Leu Leu Asn Val Asn
                   150                 155                 160
   ctg cgg cgc gag ccc ctg gcc ttc tgc ctg ggc acc tcg ggc gct aag        705
   Leu Arg Arg Glu Pro Leu Ala Phe Cys Leu Gly Thr Ser Gly Ala Lys
               165                 170                 175
   gcc ctg atc ttt gga gga gaa atg gtg gcg gcg gtg gcc gaa gtg agc        753
   Ala Leu Ile Phe Gly Gly Glu Met Val Ala Ala Val Ala Glu Val Ser
           180                 185                 190
   ggg cat ctg ggg aaa agt ttg atc aag ttc tgc tct gga gac ttg ggg        801
   Gly His Leu Gly Lys Ser Leu Ile Lys Phe Cys Ser Gly Asp Leu Gly
       195                 200                 205
   ccc gag ggc atc ttg ccg gac acc cac ctc ctg gac ccg ctg ctg aag        849
   Pro Glu Gly Ile Leu Pro Asp Thr His Leu Leu Asp Pro Leu Leu Lys
   210                 215                 220                 225
   gag gcc tct act gcc ccc ttg gca cag atc ccc agc aag ggc atg gac        897
   Glu Ala Ser Thr Ala Pro Leu Ala Gln Ile Pro Ser Lys Gly Met Asp
                   230                 235                 240
   gat cgt ctt ttc tac atc tac acg tcg ggg acc acc ggg ctg ccc aag        945
   Asp Arg Leu Phe Tyr Ile Tyr Thr Ser Gly Thr Thr Gly Leu Pro Lys
               245                 250                 255
   gct gcc att gtc gtg cac agc agg tac tac cgc atg gca gcc ttc ggc        993
   Ala Ala Ile Val Val His Ser Arg Tyr Tyr Arg Met Ala Ala Phe Gly
           260                 265                 270
   cac cac gcc tac cgc atg cag gcg gct gac gtg ctc tat gac tgc ctg       1041
   His His Ala Tyr Arg Met Gln Ala Ala Asp Val Leu Tyr Asp Cys Leu
       275                 280                 285
   ccc ctg tac cac tcg gca gga aac atc atc ggc gtg ggg cag tgt ctc       1089
   Pro Leu Tyr His Ser Ala Gly Asn Ile Ile Gly Val Gly Gln Cys Leu
   290                 295                 300                 305
   atc tat ggg ctg aca gtc gtc ctc cgc aag aaa ttc tcg gcc agc cgc       1137
   Ile Tyr Gly Leu Thr Val Val Leu Arg Lys Lys Phe Ser Ala Ser Arg
                   310                 315                 320
   ttc tgg gac gac tgc atc aag tac aac tgc acg gtg gtt cag tac atc       1185
   Phe Trp Asp Asp Cys Ile Lys Tyr Asn Cys Thr Val Val Gln Tyr Ile
               325                 330                 335
   ggg gag atc tgc cgc tac ctg ctg aag cag ccg gtg cgc gag gcg gag      1233
   Gly Glu Ile Cys Arg Tyr Leu Leu Lys Gln Pro Val Arg Glu Ala Glu
           340                 345                 350
   agg cga cac cgc gtg cgc ctg gcg gtg ggg aac ggg ctg cgt cct gcc      1281
   Arg Arg His Arg Val Arg Leu Ala Val Gly Asn Gly Leu Arg Pro Ala
       355                 360                 365
   atc tgg gag gag ttc acg gag cgc ttc ggc gta cgc caa atc ggg gag      1329
   Ile Trp Glu Glu Phe Thr Glu Arg Phe Gly Val Arg Gln Ile Gly Glu
   370                 375                 380                 385
   ttc tac ggc gcc acc gag tgc aac tgc agc att gcc aac atg gac ggc      1377
   Phe Tyr Gly Ala Thr Glu Cys Asn Cys Ser Ile Ala Asn Met Asp Gly
                   390                 395                 400
   aag gtc ggc tcc tgt ggt ttc aac agc cgc atc ctg ccc cac gtg tac      1425
   Lys Val Gly Ser Cys Gly Phe Asn Ser Arg Ile Leu Pro His Val Tyr
               405                 410                 415
   ccc atc cgg ctg gtg aag gtc aat gag gac aca atg gag ctg ctg cgg      1473
   Pro Ile Arg Leu Val Lys Val Asn Glu Asp Thr Met Glu Leu Leu Arg
           420                 425                 430
   gat gcc cag ggc ctc tgc atc ccc tgc cag gcc ggg gag cct ggc ctc      1521
   Asp Ala Gln Gly Leu Cys Ile Pro Cys Gln Ala Gly Glu Pro Gly Leu
       435                 440                 445
   ctt gtg ggt cag atc aac caa cag gac ccg ctg cgc cgc ttc gat ggc      1569
   Leu Val Gly Gln Ile Asn Gln Gln Asp Pro Leu Arg Arg Phe Asp Gly
   450                 455                 460                 465
   tat gtc agc gag agc gcc acc agc aag aag atc gcc cac agc gtc ttc      1617
   Tyr Val Ser Glu Ser Ala Thr Ser Lys Lys Ile Ala His Ser Val Phe
                   470                 475                 480
   agc aag ggc gac agc gcc tac ctc tca ggt gac gtg cta gtg atg gat      1665
   Ser Lys Gly Asp Ser Ala Tyr Leu Ser Gly Asp Val Leu Val Met Asp
               485                 490                 495
   gag ctg ggc tac atg tac ttc cgg gac cgt agc ggg gac acc ttc cgc      1713
   Glu Leu Gly Tyr Met Tyr Phe Arg Asp Arg Ser Gly Asp Thr Phe Arg
           500                 505                 510
   tgg cga ggg gag aac gtc tcc acc acc gag gtg gag ggc gtg ctg agc      1761
   Trp Arg Gly Glu Asn Val Ser Thr Thr Glu Val Glu Gly Val Leu Ser
       515                 520                 525
   cgc ctg ctg ggc cag aca gac gtg gcc gtc tat ggg gtg gct gtt cca      1809
   Arg Leu Leu Gly Gln Thr Asp Val Ala Val Tyr Gly Val Ala Val Pro
   530                 535                 540                 545
   gga gtg gag ggt aag gca ggg atg gcg gcc gtc gca gac ccc cac agc      1857
   Gly Val Glu Gly Lys Ala Gly Met Ala Ala Val Ala Asp Pro His Ser
                   550                 555                 560
   ctg ctg gac ccc aac gcg ata tac cag gag ctg cag aag gtg ctg gca     1905
   Leu Leu Asp Pro Asn Ala Ile Tyr Gln Glu Leu Gln Lys Val Leu Ala
               565                 570                 575
   ccc tat gcc cgg ccc atc ttc ctg cgc ctc ctg ccc cag gtg gac acc     1953
   Pro Tyr Ala Arg Pro Ile Phe Leu Arg Leu Leu Pro Gln Val Asp Thr
           580                 585                 590
   aca ggc acc ttc aag atc cag aag acg agg ctg cag cga gag ggc ttt     2001
   Thr Gly Thr Phe Lys Ile Gln Lys Thr Arg Leu Gln Arg Glu Gly Phe
       595                 600                 605
   gac cca cgc cag acc tca gac cgg ctc ttc ttc ctg gac ctg aag cag     2049
   Asp Pro Arg Gln Thr Ser Asp Arg Leu Phe Phe Leu Asp Leu Lys Gln
   610                 615                 620                 625
   ggc cac tac ctg ccc tta aat gag gca gtc tac act cgc atc tgc tcg     2097
   Gly His Tyr Leu Pro Leu Asn Glu Ala Val Tyr Thr Arg Ile Cys Ser
                   630                 635                 640
   ggc gcc ttc gcc ctc tgaagctgtt cctctactgg ccacaaactc tgggcctggt     2152
   Gly Ala Phe Ala Leu
               645
   gggagaggcc agcttgagcc agacagcgct gcccaggggt ggccgcctag tacacaccca     2212
   cctggccgag ctgtacctgg cacggcccat cctggactga gaaactggaa cctcagagga     2272
   acccgtgcct ctctgctgcc ttggtgcccc tgtgtctgcc tcctctccct gcttttcagc     2332
   ctctgtctcc ttccatccct gtccctgtct ggccttaact cttccctctc tttcttttct     2392
   ttctttcttt cttttttttt aagatagagt ctcactctgc tgcccgggct agagtgcagt     2452
   ggtgggatct cggctcactg caacctctgc ctcctggggt tcaagtgatc ctcccacctc     2512
   agcctcctga gtagctggga ttacaggcac ccgccaccac gtccagctaa tttttatatt     2572
   tttagtagag acggggtttc accatgttgg tcaggctggt cttgaactcc tgacctcagg     2632
   tgatccgctg gcctcggcct cccagagtgc tgggattata ggcgtgagcc tctggcccgg     2692
   cctttccttt ttcctctcct ctcctgccga gagtggaaca cacgtgtcct gggagctgca     2752
   tcttgtgtag ggtccagctg cttttgggga ctgcaggaat catctcccct gggccctgga     2812
   ctcggactgg ggcctcccca cctccctctc ggctgtgcct tacggagccc caatccaggc     2872
   ctcctgtggc tgttgggttc cagatgctgc agctccatgt gacttccaag caggccctcc     2932
   gccctccctg ctgaatggag gagccggggg tcccccaggc caactggaaa atctcccagg     2992
   ctaggccaat tgccttttgc acttccccgt tcctgtcaca tttccccagc cccaccttcc     3052
   cctcctgatg ccctgaaagc ttccggaatt gactgtgacc acttggatgt caccactgtc     3112
   agcccctgcc ttgatgtccc catttagcca tctccatgga gctcctgctg gagggccctg     3172
   aaccctgcac tgcgtggctg cccagccagc tgcctcctgt cctgggagga ggcctcctgg     3232
   gtgtcctcat ctggtgtgtc tactggaggg tcccacagga gaggcagcag aggggtcagg     3292
   ggaggtctcc tgccgggggt tggcctctca agcctcaggg gttctagcct gttgaatata     3352
   ccccacctgg tgggtggccc ctccgatgtc cccactgatg gctctgacac cgtgttggtg     3412
   gcgatgtccc agacaatccc accaggacgg cccagacatc cctactggct tcgctggtgg     3472
   ctcatctcga acatccacgc cagcctttct ggggccggcc acccaggccg cctgtccgtc     3532
   tgtcctccct ccagcagcac cccctggccc ctggagtggt ggggccatgg caagagacac     3592
   cgtggcgtct catgtgaact ttcctgggca ctgtggtttt atttcctaat tgatttaaga     3652
   aataaacctg aagaccgtct ggtgaaaaaa aaaaaaaaaa aagggcggcc gc             3704
<210> 25
   <211> 646
   <212> PRT
   <213> Homo sapiens
<400> 25
   Met Arg Ala Pro Gly Ala Gly Ala Ala Ser Val Val Ser Leu Ala Leu
    1               5                  10                  15
   Leu Trp Leu Leu Gly Leu Pro Trp Thr Trp Ser Ala Ala Ala Ala Leu
               20                  25                  30
   Gly Val Tyr Val Gly Ser Gly Gly Trp Arg Phe Leu Arg Ile Val Cys
           35                  40                  45
   Lys Thr Ala Arg Arg Asp Leu Phe Gly Leu Ser Val Leu Ile Arg Val
       50                  55                  60
   Arg Leu Glu Leu Arg Arg His Gln Arg Ala Gly His Thr Ile Pro Arg
   65                  70                  75                  80
   Ile Phe Gln Ala Val Val Gln Arg Gln Pro Glu Arg Leu Ala Leu Val
                   85                  90                  95
   Asp Ala Gly Thr Gly Glu Cys Trp Thr Phe Ala Gln Leu Asp Ala Tyr
               100                 105                 110
   Ser Asn Ala Val Ala Asn Leu Phe Arg Gln Leu Gly Phe Ala Pro Gly
           115                 120                 125
   Asp Val Val Ala Ile Phe Leu Glu Gly Arg Pro Glu Phe Val Gly Leu
       130                 135                 140
   Trp Leu Gly Leu Ala Lys Ala Gly Met Glu Ala Ala Leu Leu Asn Val
   145                 150                 155                 160
   Asn Leu Arg Arg Glu Pro Leu Ala Phe Cys Leu Gly Thr Ser Gly Ala
                   165                 170                 175
   Lys Ala Leu Ile Phe Gly Gly Glu Met Val Ala Ala Val Ala Glu Val
               180                 185                 190
   Ser Gly His Leu Gly Lys Ser Leu Ile Lys Phe Cys Ser Gly Asp Leu
           195                 200                 205
   Gly Pro Glu Gly Ile Leu Pro Asp Thr His Leu Leu Asp Pro Leu Leu
       210                 215                 220
   Lys Glu Ala Ser Thr Ala Pro Leu Ala Gln Ile Pro Ser Lys Gly Met
   225                 230                 235                 240
   Asp Asp Arg Leu Phe Tyr Ile Tyr Thr Ser Gly Thr Thr Gly Leu Pro
                   245                 250                 255
   Lys Ala Ala Ile Val Val His Ser Arg Tyr Tyr Arg Met Ala Ala Phe
               260                 265                 270
   Gly His His Ala Tyr Arg Met Gln Ala Ala Asp Val Leu Tyr Asp Cys
           275                 280                 285
   Leu Pro Leu Tyr His Ser Ala Gly Asn Ile Ile Gly Val Gly Gln Cys
       290                 295                 300
   Leu Ile Tyr Gly Leu Thr Val Val Leu Arg Lys Lys Phe Ser Ala Ser
   305                 310                 315                 320
   Arg Phe Trp Asp Asp Cys Ile Lys Tyr Asn Cys Thr Val Val Gln Tyr
                   325                 330                 335
   Ile Gly Glu Ile Cys Arg Tyr Leu Leu Lys Gln Pro Val Arg Glu Ala
               340                 345                 350
   Glu Arg Arg His Arg Val Arg Leu Ala Val Gly Asn Gly Leu Arg Pro
           355                 360                 365
   Ala Ile Trp Glu Glu Phe Thr Glu Arg Phe Gly Val Arg Gln Ile Gly
       370                 375                 380
   Glu Phe Tyr Gly Ala Thr Glu Cys Asn Cys Ser Ile Ala Asn Met Asp
   385                 390                 395                 400
   Gly Lys Val Gly Ser Cys Gly Phe Asn Ser Arg Ile Leu Pro His Val
                   405                 410                 415
   Tyr Pro Ile Arg Leu Val Lys Val Asn Glu Asp Thr Met Glu Leu Leu
               420                 425                 430
   Arg Asp Ala Gln Gly Leu Cys Ile Pro Cys Gln Ala Gly Glu Pro Gly
           435                 440                 445
   Leu Leu Val Gly Gln Ile Asn Gln Gln Asp Pro Leu Arg Arg Phe Asp
   450 455 460
   Gly Tyr Val Ser Glu Ser Ala Thr Ser Lys Lys Ile Ala His Ser Val
   465 470 475 480
   Phe Ser Lys Gly Asp Ser Ala Tyr Leu Ser Gly Asp Val Leu Val Met
   485 490 495
   Asp Glu Leu Gly Tyr Met Tyr Phe Arg Asp Arg Ser Gly Asp Thr Phe
   500 505 510
   Arg Trp Arg Gly Glu Asn Val Ser Thr Thr Glu Val Glu Gly Val Leu
   515 520 525
   Ser Arg Leu Leu Gly Gln Thr Asp Val Ala Val Tyr Gly Val Ala Val
   530 535 540
   Pro Gly Val Glu Gly Lys Ala Gly Met Ala Ala Val Ala Asp Pro His
   545 550 555 560
   Ser Leu Leu Asp Pro Asn Ala Ile Tyr Gln Glu Leu Gln Lys Val Leu
   565 570 575
   Ala Pro Tyr Ala Arg Pro Ile Phe Leu Arg Leu Leu Pro Gln Val Asp
   580 585 590
   Thr Thr Gly Thr Phe Lys Ile Gln Lys Thr Arg Leu Gln Arg Glu Gly
   595 600 605
   Phe Asp Pro Arg Gln Thr Ser Asp Arg Leu Phe Phe Leu Asp Leu Lys
   610 615 620
   Gln Gly His Tyr Leu Pro Leu Asn Glu Ala Val Tyr Thr Arg Ile Cys
   625 630 635 640
   Ser Gly Ala Phe Ala Leu
   645
<210> 26
   <211> 2917
   <212> DNA
   <213> Homo sapiens
<220>
   <221> CDS
   <222> (208)...(2136)
<400> 26
cgacccacgc gtccgggcgg gcggggccgg gcggcgggcg gggctggcgg ggcggccggg     60
   ccatgcaggg cgcagagccg gctaaaccct gctgagaccc ggctccgtgc gtccaggggc    120
   ggctaatgcc cctcacgctg tctacgctgc tgcaaccggg ccgcatctgg acggggcgcc    180
   gcgcggcgga gccgacgccg ggccaca atg ctg ctt gga gcc tct ctg gtg ggg    234
   gtg ctg ctg ttc tcc aag ctg gtg ctg aaa ctg ccc tgg acc cag gtg      282
   gga ttc tcc ctg ttg ttc ctc tac ttg gga tct ggc ggc tgg cgc ttc      330
   atc cgg gtc ttc atc aag acc atc agg cgc gat atc ttt ggc ggc ctg      378
   gtc ctc ctg aag gtg aag gca aag gtg cga cag tgc ctg cag gag cgg        426
   cgg aca gtg ccc att ttg ttt gcc tct acc gtt cgg cgc cac ccc gac        474
   aag acg gcc ctg atc ttc gag ggc aca gat acc cac tgg acc ttc cgc        522
   cag ctg gat gag tac tca agc agt gta gcc aac ttc ctg cag gcc cgg        570
   ggc ctg gcc tcg ggc gat gtg gct gcc atc ttc atg gag aac cgc aat        618
   gag ttc gtg ggc cta tgg ctg ggc atg gcc aag ctc ggt gtg gag gca        666
   gcc ctc atc aac acc aac ctg cgg cgg gat gct ctg ctc cac tgc ctc        714
   acc acc tcg cgc gca cgg gcc ctt gtc ttt ggc agc gaa atg gcc tca        762
   gcc atc tgt gag gtc cat gcc agc ctg gac ccc tcg ctc agc ctc ttc        810
   tgc tct ggc tcc tgg gag ccc ggt gcg gtg cct cca agc aca gaa cac        858
   ctg gac cct ctg ctg aaa gat gct ccc aag cac ctt ccc agt tgc cct        906
   gac aag ggc ttc aca gat aaa ctg ttc tac atc tac aca tcc ggc acc        954
   aca ggg ctg ccc aag gcc gcc atc gtg gtg cac agc agg tat tac cgc       1002
   atg gct gcc ctg gtg tac tat gga ttc cgc atg cgg ccc aac gac atc       1050
   gtc tat gac tgc ctc ccc ctc tac cac tca gca gga aac atc gtg gga     1098
   atc ggc cag tgc ctg ctg cat ggc atg acg gtg gtg att cgg aag aag     1146
   ttc tca gcc tcc cgg ttc tgg gac gat tgt atc aag tac aac tgc acg     1194
   att gtg cag tac att ggt gaa ctg tgc cgc tac ctc ctg aac cag cca     1242
   ccg cgg gag gca gaa aac cag cac cag gtt cgc atg gca cta ggc aat     1290
   ggc ctc cgg cag tcc atc tgg acc aac ttt tcc agc cgc ttc cac ata     1338
   ccc cag gtg gct gag ttc tac ggg gcc aca gag tgc aac tgt agc ctg     1386
   ggc aac ttc gac agc cag gtg ggg gcc tgt ggt ttc aat agc cgc atc     1434
   ctg tcc ttc gtg tac ccc atc cgg ttg gta cgt gtc aac gag gac acc     1482
   atg gag ctg atc cgg ggg ccc gac ggc gtc tgc att ccc tgc cag cca     1530
   ggt gag ccg ggc cag ctg gtg ggc cgc atc atc cag aaa gac ccc ctg     1578
   cgc cgc ttc gat ggc tac ctc aac cag ggc gcc aac aac aag aag att     1626
   gcc aag gat gtc ttc aag aag ggg gac cag gcc tac ctt act ggt gat     1674
   gtg ctg gtg atg gac gag ctg ggc tac ctg tac ttc cga gac cgc act     1722
   ggg gac acg ttc cgc tgg aaa ggt gag aac gtg tcc acc acc gag gtg      1770
   gaa ggc aca ctc agc cgc ctg ctg gac atg gct gac gtg gcc gtg tat      1818
   ggt gtc gag gtg cca gga acc gag ggc cgg gcc gga atg gct gct gtg      1866
   gcc agc ccc act ggc aac tgt gac ctg gag cgc ttt gct cag gtc ttg      1914
   gag aag gaa ctg ccc ctg tat gcg cgc ccc atc ttc ctg cgc ctc ctg      1962
   cct gag ctg cac aaa aca gga acc tac aag ttc cag aag aca gag cta      2010
   cgg aag gag ggc ttt gac ccg gct att gtg aaa gac ccg ctg ttc tat      2058
   cta gat gcc cag aag ggc cgc tac gtc ccg ctg gac caa gag gcc tac      2106
   agc cgc atc cag gca ggc gag gag aag ctg tgattccccc catccctctg        2156
   agggccggcg gatgctggat ccggagcccc aggttccgcc ccagagcggt cctggacaag    2216
   gccagaccaa agcaagcagg gcctggcacc tccatcctga ggtgctgccc ctccatccaa    2276
   aactgccaag tgactcattg ccttcccaac ccttccagag gctttctgtg aaagtctcat    2336
   gtccaagttc cgtcttctgg gctgggcagg ccctctggtt cccaggctga gactgacggg    2396
   ttttctcagg atgatgtctt gggtgagggt agggagagga caaggggtca ccgagccctt    2456
   cccagagagc agggagctta taaatggaac cagagcagaa gtccccagac tcaggaagtc    2516
   aacagagtgg gcagggacag tggtagcatc catctggtgg ccaaagagaa tcgtagcccc    2576
   agagctgccc aagttcactg ggctccaccc ccacctccag gaggggagga gaggacctga    2636
   catctgtagg tggcccctga tgccccatct acagcaggag gtcaggacca cgcccctggc    2696
   ctctccccac tcccccatcc tcctccctgg gtggctgcct gattatccct caggcagggc    2756
   ctctcagtcc ttgtgggtct gtgtcacctc catctcagtc ttggcctggc tatgagggga    2816
   ggaggaatgg gagagggggc tcaggggcca ataaactctg ccttgagtcc tcctaaaaaa    2876
   aaaaaaaaaa aaaaaaaaaa aaaaaaaaaa agggcggccg c                        2917
<210> 27
   <211> 643
   <212> PRT
   <213> Homo sapiens
<400> 27
<210> 28
   <211> 1941
   <212> DNA
   <213> Homo sapiens
<400> 28
   atgcgggctc cgggtgcggg cgcggcctcg gtggtctcgc tggcgctgtt gtggctgctg 60
   gggctgccgt ggacctggag cgcggcagcg gcgctcggcg tgtacgtggg cagcggcggc 120
   tggcgcttcc tgcgcatcgt ctgcaagacc gcgaggcgag acctcttcgg tctctctgtg 180
   ctgatccgcg tgcgcctgga gctgcggcgg caccagcgtg ccggccacac catcccgcgc 240
   atctttcagg cggtagtgca gcgacagccc gagcgcctgg cgctggtgga tgccgggacc 300
   ggcgagtgct ggacctttgc gcagctggac gcctactcca atgcggtagc caacctcttc 360
   cgccagctgg gcttcgcgcc gggcgacgtg gtggccatct tcctggaggg ccggccggag 420
   ttcgtggggc tgtggctggg cctggccaag gcgggcatgg aggccgcgct gctcaacgtg 480
   aacctgcggc gcgagcccct ggccttctgc ctgggcacct cgggcgctaa ggccctgatc 540
   tttggaggag aaatggtggc ggcggtggcc gaagtgagcg ggcatctggg gaaaagtttg 600
   atcaagttct gctctggaga cttggggccc gagggcatct tgccggacac ccacctcctg 660
   gacccgctgc tgaaggaggc ctctactgcc cccttggcac agatccccag caagggcatg 720
   gacgatcgtc ttttctacat ctacacgtcg ggaccaccgg ggctgcccaa ggctgccatt 780
   gtcgtgcaca gcaggtacta ccgcatggca gccttcggcc accacgccta ccgcatgcag 840
   gcggctgacg tgctctatga ctgcctgccc ctgtaccact cggcaggaaa catcatcggc 900
   gtggggcagt gtctcatcta tgggctgaca gtcgtcctcc gcaagaaatt ctcggccagc 960
   cgcttctggg acgactgcat caagtacaac tgcacggtgg ttcagtacat cggggagatc 1020
   tgccgctacc tgctgaagca gccggtgcgc gaggcggaga ggcgacaccg cgtgcgcctg 1080
   gcggtgggga acgggctgcg tcctgccatc tgggaggagt tcacggagcg cttcggcgta 1140
   cgccaaatcg gggagttcta cggcgccacc gagtgcaact gcagcattgc caacatggac 1200
   ggcaaggtcg gctcctgtgg tttcaacagc cgcatcctgc cccacgtgta ccccatccgg 1260
   ctggtgaagg tcaatgagga cacaatggag ctgctgcggg atgcccaggg cctctgcatc 1320
   ccctgccagg ccggggagcc tggcctcctt gtgggtcaga tcaaccaaca ggacccgctg 1380
   cgccgcttcg atggctatgt cagcgagagc gccaccagca agaagatcgc ccacagcgtc 1440
   ttcagcaagg gcgacagcgc ctacctctca ggtgacgtgc tagtgatgga tgagctgggc 1500
   tacatgtact tccgggaccg tagcggggac accttccgct ggcgagggga gaacgtctcc 1560
   accaccgagg tggagggcgt gctgagccgc ctgctgggcc agacagacgt ggccgtctat 1620
   ggggtggctg ttccaggagt ggagggtaag gcagggatgg cggccgtcgc agacccccac 1680
   agcctgctgg accccaacgc gatataccag gagctgcaga aggtgctggc accctatgcc 1740
   cggcccatct tcctgcgcct cctgccccag gtggacacca caggcacctt caagatccag 1800
   aagacgaggc tgcagcgaga gggctttgac ccacgccaga cctcagaccg gctcttcttc 1860
   ctggacctga agcagggcca ctacctgccc ttaaatgagg cagtctacac tcgcatctgc 1920
   tcgggcgcct tcgccctctg a 1941
<210> 29
   <211> 1938
   <212> DNA
   <213> Mus musculus
<400> 29
   atgcgggctc ctggagcagg aacagcctct gtggcctcac tggcgctgct ttggtttctg      60
   ggacttccgt ggacctggag cgcggcggcg gcgttctgtg tgtacgtggg tggcggcggc     120
   tggcgctttc tgcgtatcgt ctgcaagacg gcgaggcgag acctctttgg cctctctgtt     180
   ctgattcgtg ttcggctaga gctgcgacga caccggcgag caggagacac gatcccgtgc     240
   atcttccagg ctgtggcccg gcgacaacca gagcgcctgg cactggtgga cgccagtagt     300
   ggtatatgct ggaccttcgc acagctggac acctactcca atgctgtagc caacctgttc     360
   cgccagctgg gctttgcacc aggcgatgtg gtggctgtgt tcctggaggg ccggccggag     420
   ttcgtgggac tgtggctggg cctggccaag gccggtgtgg tggctgctct tctcaatgtc     480
   aacctgaggc gggagcccct ggccttctgc ctgggcacat cagctgccaa ggccctcatt     540
   tatggcgggg agatggcagc ggcggtggcg gaggtgagcg agcagctggg gaagagcctc     600
   ctcaagttct gctctggaga tctggggcct gagagcatcc tgcctgacac gcagctcctg     660
   gaccccatgc ttgctgaggc gcccaccaca cccctggcac aagccccagg caagggcatg     720
   gatgatcggc tgttttacat ctatacttct gggaccaccg ggcttcctaa ggctgccatt     780
   gtggtgcaca gcaggtacta ccgcattgct gcctttggcc accattccta cagcatgcgt     840
   gccgccgatg tgctctatga ctgcctgcca ctctaccact ctgcagggaa catcatgggt     900
   gtggggcagt gcgtcatcta cgggttgacg gtggtactgc gcaagaagtt ctccgccagc     960
   cgcttctggg atgactgtgt caagtacaat tgcacggtag tgcagtacat aggtgaaatc    1020
   tgccgctacc tgctgaggca gccggttcgc gacgtggagc agcgacaccg cgtgcgcctg    1080
   gccgtgggta atgggctgcg gccagccatc tgggaggagt tcacgcagcg cttcggtgtg    1140
   ccacagatcg gcgagttcta cggcgctacc gagtgcaact gcagcattgc caacatggac    1200
   ggcaaggtcg gctcctgcgg cttcaacagc cgtatcctca cgcatgtgta ccccatccgt    1260
   ctggtcaagg tcaatgagga cacgatggag ccactgcggg actccgaggg cctctgcatc    1320
   ccgtgccagc ccggggaacc cggccttctc gtgggccaga tcaaccagca ggaccctctg    1380
   cggcgtttcg atggttatgt tagtgacagt gccaccaaca agaagattgc ccacagcgtt    1440
   ttccgaaagg gcgatagcgc ctacctctca ggtgacgtgc tagtgatgga cgagctgggc    1500
   tacatgtatt tccgtgaccg cagcggggac accttccgct ggcgcgggga gaacgtgtcc    1560
   accacggagg tggaagccgt gctgagccgc ctactgggcc agacggacgt ggctgtgtat    1620
   ggggtggctg tgccaggagt ggaggggaaa gctggcatgg cagccatcgc agatccccac    1680
   agccagttgg accctaactc aatgtaccag gaattacaga aggttcttgc atcctatgct    1740
   cggcccatct tcctgcgtct tctgccccag gtggatacca caggcacctt caagatccag    1800
   aagacccggc tgcagcgtga aggctttgac ccccgtcaga cctcagacag gctcttcttt    1860
   ctagacctga agcagggacg ctatgtaccc ctggatgaga gagtccatgc ccgcatttgt    1920
   gcaggcgact tctcactc                                                  1938
<210> 30
   <211> 1896
   <212> DNA
   <213> Homo sapiens
<400> 30
   ctgttctcca agctggtgct gaaactgccc tggacccagg tgggattctc cctgttgttc      60
   ctctacttgg gatctggcgg ctggcgcttc atccgggtct tcatcaagac catcaggcgc     120
   gatatctttg gcggcctggt cctcctgaag gtgaaggcaa aggtgcgaca gtgcctgcag     180
   gagcggcgga cagtgcccat tttgtttgcc tctaccgttc ggcgccaccc cgacaagacg     240
   gccctgatct tcgagggcac agatacccac tggaccttcc gccagctgga tgagtactca     300
   agcagtgtag ccaacttcct gcaggcccgg ggcctggcct cgggcgatgt ggctgccatc     360
   ttcatggaga accgcaatga gttcgtgggc ctatggctgg gcatggccaa gctcggtgtg     420
   gaggcagccc tcatcaacac caacctgcgg cgggatgctc tgctccactg cctcaccacc     480
   tcgcgcgcac gggcccttgt ctttggcagc gaaatggcct cagccatctg tgaggtccat     540
   gccagcctgg acccctcgct cagcctcttc tgctctggct cctgggagcc cggtgcggtg     600
   cctccaagca cagaacacct ggaccctctg ctgaaagatg ctcccaagca ccttcccagt     660
   tgccctgaca agggcttcac agataaactg ttctacatct acacatccgg caccacaggg     720
   ctgcccaagg ccgccatcgt ggtgcacagc aggtattacc gcatggctgc cctggtgtac     780
   tatggattcc gcatgcggcc caacgacatc gtctatgact gcctccccct ctaccactca    840
   gcaggaaaca tcgtgggaat cggccagtgc ctgctgcatg gcatgacggt ggtgattcgg     900
   aagaagttct cagcctcccg gttctgggac gattgtatca agtacaactg cacgattgtg     960
   cagtacattg gtgaactgtg ccgctacctc ctgaaccagc caccgcggga ggcagaaaac    1020
   cagcaccagg ttcgcatggc actaggcaat ggcctccggc agtccatctg gaccaacttt    1080
   tccagccgct tccacatacc ccaggtggct gagttctacg gggccacaga gtgcaactgt    1140
   agcctgggca acttcgacag ccaggtgggg gcctgtggtt tcaatagccg catcctgtcc    1200
   ttcgtgtacc ccatccggtt ggtacgtgtc aacgaggaca ccatggagct gatccggggg    1260
   cccgacggcg tctgcattcc ctgccagcca ggtgagccgg gccagctggt gggccgcatc    1320
   atccagaaag accccctgcg ccgcttcgat ggctacctca accagggcgc caacaacaag    1380
   aagattgcca aggatgtctt caagaagggg gaccaggcct accttactgg tgatgtgctg    1440
   gtgatggacg agctgggcta cctgtacttc cgagaccgca ctggggacac gttccgctgg    1500
   aaaggtgaga acgtgtccac caccgaggtg gaaggcacac tcagccgcct gctggacatg    1560
   gctgacgtgg ccgtgtatgg tgtcgaggtg ccaggaaccg agggccgggc cggaatggct    1620
   gctgtggcca gccccactgg caactgtgac ctggagcgct ttgctcaggt cttggagaag    1680
   gaactgcccc tgtatgcgcg ccccatcttc ctgcgcctcc tgcctgagct gcacaaaaca    1740
   ggaacctaca agttccagaa gacagagcta cggaaggagg gctttgaccc ggctattgtg    1800
   aaagacccgc tgttctatct agatgcccag aagggccgct acgtcccgct ggaccaagag    1860
   gcctacagcc gcatccaggc aggcgaggag aagctg                              1896
<210> 31
   <211> 1896
   <212> DNA
   <213> Mus musculus
<400> 31
   cttgggtcca agctagtgct gaagctgccc tggacccagg tgggattctc cctgttgctc      60
   ctgtacttgg ggtctggtgg ctggcgtttc atccgggtct tcatcaagac ggtcaggaga     120
   gatatctttg gtggcatggt gctcctgaag gtgaagacca aggtgcgacg gtaccttcag     180
   gagcggaaga cggtgcccct gctgtttgct tcaatggtac agcgccaccc ggacaagaca     240
   gccctgattt tcgagggcac agacactcac tggaccttcc gccagctgga tgagtactcc     300
   agtagtgtgg ccaacttcct gcaggcccgg ggcctggcct caggcaatgt agttgccctc     360
   tttatggaaa accgcaatga gtttgtgggt ctgtggctag gcatggccaa gctgggcgtg     420
   gaggcggctc tcatcaacac caaccttagg cgggatgccc tgcgccactg tcttgacacc     480
   tcaaaggcac gagctctcat ctttggcagt gagatggcct cagctatctg tgagatccat     540
   gctagcctgg agcccacact cagcctcttc tgctctggat cctgggagcc cagcacagtg     600
   cccgtcagca cagagcatct ggaccctctt ctggaagatg ccccgaagca cctgcccagt     660
   cacccagaca agggttttac agataagctc ttctacatct acacatcggg caccacgggg     720
   ctacccaaag ctgccattgt ggtgcacagc aggtattatc gtatggcttc cctggtgtac     780
   tatggattcc gcatgcggcc tgatgacatt gtctatgact gcctccccct ctaccactca    840
   agcaggaaac atcgtgggga ttggcagtgc ttactccacg gcatgactgt ggtgatccgg     900
   aagaagttct cagcctcccg gttctgggat gattgtatca agtacaactg cacagtggta     960
   cagtacattg gcgagctctg ccgctacctc ctgaaccagc caccccgtga ggctgagtct    1020
   cggcacaagg tgcgcatggc actgggcaac ggtctccggc agtccatctg gaccgacttc     1080
   tccagccgtt tccacatccc ccaggtggct gagttctatg gggccactga atgcaactgt     1140
   agcctgggca actttgacag ccgggtgggg gcctgtggct tcaatagccg catcctgtcc     1200
   tttgtgtacc ctatccgttt ggtacgtgtc aatgaggata ccatggaact gatccgggga     1260
   cccgatggag tctgcattcc ctgtcaacca ggtcagccag gccagctggt gggtcgcatc     1320
   atccagcagg accctctgcg ccgtttcgac gggtacctca accagggtgc caacaacaag     1380
   aagattgcta atgatgtctt caagaagggg gaccaagcct acctcactgg tgacgtcctg     1440
   gtgatggatg agctgggtta cctgtacttc cgagatcgca ctggggacac gttccgctgg     1500
   aaaggggaga atgtatctac cactgaggtg gagggcacac tcagccgcct gcttcatatg     1560
   gcagatgtgg cagtttatgg tgttgaggtg ccaggaactg aaggccgagc aggaatggct     1620
   gccgttgcaa gtcccatcag caactgtgac ctggagagct ttgcacagac cttgaaaaag     1680
   gagctgcctc tgtatgcccg ccccatcttc ctgcgcttct tgcctgagct gcacaagaca     1740
   gggaccttca agttccagaa gacagagttg cggaaggagg gctttgaccc atctgttgtg     1800
   aaagacccgc tgttctatct ggatgctcgg aagggctgct acgttgcact ggaccaggag     1860
   gcctataccc gcatccaggc aggcgaggag aagctg                               1896
<210> 32
   <211> 646
   <212> PRT
   <213> Homo sapiens
<400> 32
   Met Arg Ala Pro Gly Ala Gly Ala Ala Ser Val Val Ser Leu Ala Leu
    1               5                  10                  15
   Leu Trp Leu Leu Gly Leu Pro Trp Thr Trp Ser Ala Ala Ala Ala Leu
               20                  25                  30
   Gly Val Tyr Val Gly Ser Gly Gly Trp Arg Phe Leu Arg Ile Val Cys
           35                  40                  45
   Lys Thr Ala Arg Arg Asp Leu Phe Gly Leu Ser Val Leu Ile Arg Val
       50                  55                  60
   Arg Leu Glu Leu Arg Arg His Gln Arg Ala Gly His Thr Ile Pro Arg
   65                  70                  75                  80
   Ile Phe Gln Ala Val Val Gln Arg Gln Pro Glu Arg Leu Ala Leu Val
                   85                  90                  95
   Asp Ala Gly Thr Gly Glu Cys Trp Thr Phe Ala Gln Leu Asp Ala Tyr
               100                 105                 110
   Ser Asn Ala Val Ala Asn Leu Phe Arg Gln Leu Gly Phe Ala Pro Gly
           115                 120                 125
   Asp Val Val Ala Ile Phe Leu Glu Gly Arg Pro Glu Phe Val Gly Leu
       130                 135                 140
   Trp Leu Gly Leu Ala Lys Ala Gly Met Glu Ala Ala Leu Leu Asn Val
   145                 150                 155                 160
   Asn Leu Arg Arg Glu Pro Leu Ala Phe Cys Leu Gly Thr Ser Gly Ala
                   165                 170                 175
   Lys Ala Leu Ile Phe Gly Gly Glu Met Val Ala Ala Val Ala Glu Val
               180                 185                 190
   Ser Gly His Leu Gly Lys Ser Leu Ile Lys Phe Cys Ser Gly Asp Leu
           195                 200                 205
   Gly Pro Glu Gly Ile Leu Pro Asp Thr His Leu Leu Asp Pro Leu Leu
       210                 215                 220
   Lys Glu Ala Ser Thr Ala Pro Leu Ala Gln Ile Pro Ser Lys Gly Met
   225                 230                 235                 240
   Asp Asp Arg Leu Phe Tyr Ile Tyr Thr Ser Gly Thr Thr Gly Leu Pro
                   245                 250                 255
   Lys Ala Ala Ile Val Val His Ser Arg Tyr Tyr Arg Met Ala Ala Phe
               260                 265                 270
   Gly His His Ala Tyr Arg Met Gln Ala Ala Asp Val Leu Tyr Asp Cys
           275                 280                 285
   Leu Pro Leu Tyr His Ser Ala Gly Asn Ile Ile Gly Val Gly Gln Cys
       290                 295                 300
   Leu Ile Tyr Gly Leu Thr Val Val Leu Arg Lys Lys Phe Ser Ala Ser
   305                 310                 315                 320
   Arg Phe Trp Asp Asp Cys Ile Lys Tyr Asn Cys Thr Val Val Gln Tyr
                   325                 330                 335
   Ile Gly Glu Ile Cys Arg Tyr Leu Leu Lys Gln Pro Val Arg Glu Ala
               340                 345                 350
   Glu Arg Arg His Arg Val Arg Leu Ala Val Gly Asn Gly Leu Arg Pro
           355                 360                 365
   Ala Ile Trp Glu Glu Phe Thr Glu Arg Phe Gly Val Arg Gln Ile Gly
       370                 375                 380
   Glu Phe Tyr Gly Ala Thr Glu Cys Asn Cys Ser Ile Ala Asn Met Asp
   385                 390                 395                 400
   Gly Lys Val Gly Ser Cys Gly Phe Asn Ser Arg Ile Leu Pro His Val
                   405                 410                 415
   Tyr Pro Ile Arg Leu Val Lys Val Asn Glu Asp Thr Met Glu Leu Leu
               420                 425                 430
   Arg Asp Ala Gln Gly Leu Cys Ile Pro Cys Gln Ala Gly Glu Pro Gly
           435                 440                 445
   Leu Leu Val Gly Gln Ile Asn Gln Gln Asp Pro Leu Arg Arg Phe Asp
       450                 455                 460
   Gly Tyr Val Ser Glu Ser Ala Thr Ser Lys Lys Ile Ala His Ser Val
   465                 470                 475                 480
   Phe Ser Lys Gly Asp Ser Ala Tyr Leu Ser Gly Asp Val Leu Val Met
                   485                 490                 495
   Asp Glu Leu Gly Tyr Met Tyr Phe Arg Asp Arg Ser Gly Asp Thr Phe
               500                 505                 510
   Arg Trp Arg Gly Glu Asn Val Ser Thr Thr Glu Val Glu Gly Val Leu
           515                 520                 525
   Ser Arg Leu Leu Gly Gln Thr Asp Val Ala Val Tyr Gly Val Ala Val
       530                 535                 540
   Pro Gly Val Glu Gly Lys Ala Gly Met Ala Ala Val Ala Asp Pro His
   545                 550                 555                 560
   Ser Leu Leu Asp Pro Asn Ala Ile Tyr Gln Glu Leu Gln Lys Val Leu
                   565                 570                 575
   Ala Pro Tyr Ala Arg Pro Ile Phe Leu Arg Leu Leu Pro Gln Val Asp
               580                 585                 590
   Thr Thr Gly Thr Phe Lys Ile Gln Lys Thr Arg Leu Gln Arg Glu Gly
           595                 600                 605
   Phe Asp Pro Arg Gln Thr Ser Asp Arg Leu Phe Phe Leu Asp Leu Lys
       610                 615                 620
   Gln Gly His Tyr Leu Pro Leu Asn Glu Ala Val Tyr Thr Arg Ile Cys
   625                 630                 635                 640
   Ser Gly Ala Phe Ala Leu
                   645
<210> 33
   <211> 646
   <212> PRT
   <213> Mus musculus
<400> 33
   Met Arg Ala Pro Gly Ala Gly Thr Ala Ser Val Ala Ser Leu Ala Leu
   1 5 10 15
   Leu Trp Phe Leu Gly Leu Pro Trp Thr Trp Ser Ala Ala Ala Ala Phe
                   20                  25                  30
   Cys Val Tyr Val Gly Gly Gly Gly Trp Arg Phe Leu Arg Ile Val Cys
               35                  40                  45
   Lys Thr Ala Arg Arg Asp Leu Phe Gly Leu Ser Val Leu Ile Arg Val
       50                  55                  60
   Arg Leu Glu Leu Arg Arg His Arg Arg Ala Gly Asp Thr Ile Pro Cys
   65                  70                  75                  80
   Ile Phe Gln Ala Val Ala Arg Arg Gln Pro Glu Arg Leu Ala Leu Val
                   85                  90                  95
   Asp Ala Ser Ser Gly Ile Cys Trp Thr Phe Ala Gln Leu Asp Thr Tyr
                   100                 105                 110
   Ser Asn Ala Val Ala Asn Leu Phe Arg Gln Leu Gly Phe Ala Pro Gly
               115                 120                 125
   Asp Val Val Ala Val Phe Leu Glu Gly Arg Pro Glu Phe Val Gly Leu
       130                 135                 140
   Trp Leu Gly Leu Ala Lys Ala Gly Val Val Ala Ala Leu Leu Asn Val
   145                 150                 155                 160
   Asn Leu Arg Arg Glu Pro Leu Ala Phe Cys Leu Gly Thr Ser Ala Ala
                   165                 170                 175
   Lys Ala Leu Ile Tyr Gly Gly Glu Met Ala Ala Ala Val Ala Glu Val
                   180                 185                 190
   Ser Glu Gln Leu Gly Lys Ser Leu Leu Lys Phe Cys Ser Gly Asp Leu
               195                 200                 205
   Gly Pro Glu Ser Ile Leu Pro Asp Thr Gln Leu Leu Asp Pro Met Leu
       210                 215                 220
   Ala Glu Ala Pro Thr Thr Pro Leu Ala Gln Ala Pro Gly Lys Gly Met
   225                 230                 235                 240
   Asp Asp Arg Leu Phe Tyr Ile Tyr Thr Ser Gly Thr Thr Gly Leu Pro
                   245                 250                 255
   Lys Ala Ala Ile Val Val His Ser Arg Tyr Tyr Arg Ile Ala Ala Phe
                   260                 265                 270
   Gly His His Ser Tyr Ser Met Arg Ala Ala Asp Val Leu Tyr Asp Cys
               275                 280                 285
   Leu Pro Leu Tyr His Ser Ala Gly Asn Ile Met Gly Val Gly Gln Cys
       290                 295                 300
   Val Ile Tyr Gly Leu Thr Val Val Leu Arg Lys Lys Phe Ser Ala Ser
   305                 310                 315                 320
   Arg Phe Trp Asp Asp Cys Val Lys Tyr Asn Cys Thr Val Val Gln Tyr
                   325                 330                 335
   Ile Gly Glu Ile Cys Arg Tyr Leu Leu Arg Gln Pro Val Arg Asp Val
                   340                 345                 350
   Glu Gln Arg His Arg Val Arg Leu Ala Val Gly Asn Gly Leu Arg Pro
               355                 360                 365
   Ala Ile Trp Glu Glu Phe Thr Gln Arg Phe Gly Val Pro Gln Ile Gly
       370                 375                 380
   Glu Phe Tyr Gly Ala Thr Glu Cys Asn Cys Ser Ile Ala Asn Met Asp
   385                 390                 395                 400
   Gly Lys Val Gly Ser Cys Gly Phe Asn Ser Arg Ile Leu Thr His Val
                   405                 410                 415
   Tyr Pro Ile Arg Leu Val Lys Val Asn Glu Asp Thr Met Glu Pro Leu
                   420                 425                 430
   Arg Asp Ser Glu Gly Leu Cys Ile Pro Cys Gln Pro Gly Glu Pro Gly
               435                 440                 445
   Leu Leu Val Gly Gln Ile Asn Gln Gln Asp Pro Leu Arg Arg Phe Asp
       450                 455                 460
   Gly Tyr Val Ser Asp Ser Ala Thr Asn Lys Lys Ile Ala His Ser Val
   465                 470                 475                 480
   Phe Arg Lys Gly Asp Ser Ala Tyr Leu Ser Gly Asp Val Leu Val Met
                   485                 490                 495
   Asp Glu Leu Gly Tyr Met Tyr Phe Arg Asp Arg Ser Gly Asp Thr Phe
               500                 505                 510
   Arg Trp Arg Gly Glu Asn Val Ser Thr Thr Glu Val Glu Ala Val Leu
           515                 520                 525
   Ser Arg Leu Leu Gly Gln Thr Asp Val Ala Val Tyr Gly Val Ala Val
       530                 535                 540
   Pro Gly Val Glu Gly Lys Ala Gly Met Ala Ala Ile Ala Asp Pro His
   545                 550                 555                 560
   Ser Gln Leu Asp Pro Asn Ser Met Tyr Gln Glu Leu Gln Lys Val Leu
                   565                 570                 575
   Ala Ser Tyr Ala Arg Pro Ile Phe Leu Arg Leu Leu Pro Gln Val Asp
               580                 585                 590
   Thr Thr Gly Thr Phe Lys Ile Gln Lys Thr Arg Leu Gln Arg Glu Gly
           595                 600                 605
   Phe Asp Pro Arg Gln Thr Ser Asp Arg Leu Phe Phe Leu Asp Leu Lys
       610                 615                 620
   Gln Gly Arg Tyr Val Pro Leu Asp Glu Arg Val His Ala Arg Ile Cys
   625                 630                 635                 640
   Ala Gly Asp Phe Ser Leu
                   645
<210> 34
   <211> 632
   <212> PRT
   <213> Homo sapiens
<400> 34
   Leu Phe Ser Lys Leu Val Leu Lys Leu Pro Trp Thr Gln Val Gly Phe
    1               5                  10                  15
   Ser Leu Leu Phe Leu Tyr Leu Gly Ser Gly Gly Trp Arg Phe Ile Arg
               20                  25                  30
   Val Phe Ile Lys Thr Ile Arg Arg Asp Ile Phe Gly Gly Leu Val Leu
           35                  40                  45
   Leu Lys Val Lys Ala Lys Val Arg Gln Cys Leu Gln Glu Arg Arg Thr
       50                  55                  60
   Val Pro Ile Leu Phe Ala Ser Thr Val Arg Arg His Pro Asp Lys Thr
   65                  70                  75                  80
   Ala Leu Ile Phe Glu Gly Thr Asp Thr His Trp Thr Phe Arg Gln Leu
                   85                  90                  95
   Asp Glu Tyr Ser Ser Ser Val Ala Asn Phe Leu Gln Ala Arg Gly Leu
               100                 105                 110
   Ala Ser Gly Asp Val Ala Ala Ile Phe Met Glu Asn Arg Asn Glu Phe
           115                 120                 125
   Val Gly Leu Trp Leu Gly Met Ala Lys Leu Gly Val Glu Ala Ala Leu
       130                 135                 140
   Ile Asn Thr Asn Leu Arg Arg Asp Ala Leu Leu His Cys Leu Thr Thr
   145                 150                 155                 160
   Ser Arg Ala Arg Ala Leu Val Phe Gly Ser Glu Met Ala Ser Ala Ile
                   165                 170                 175
   Cys Glu Val His Ala Ser Leu Asp Pro Ser Leu Ser Leu Phe Cys Ser
               180                 185                 190
   Gly Ser Trp Glu Pro Gly Ala Val Pro Pro Ser Thr Glu His Leu Asp
           195                 200                 205
   Pro Leu Leu Lys Asp Ala Pro Lys His Leu Pro Ser Cys Pro Asp Lys
       210                 215                 220
   Gly Phe Thr Asp Lys Leu Phe Tyr Ile Tyr Thr Ser Gly Thr Thr Gly
   225                 230                 235                 240
   Leu Pro Lys Ala Ala Ile Val Val His Ser Arg Tyr Tyr Arg Met Ala
                   245                 250                 255
   Ala Leu Val Tyr Tyr Gly Phe Arg Met Arg Pro Asn Asp Ile Val Tyr
               260                 265                 270
   Asp Cys Leu Pro Leu Tyr His Ser Ala Gly Asn Ile Val Gly Ile Gly
           275                 280                 285
   Gln Cys Leu Leu His Gly Met Thr Val Val Ile Arg Lys Lys Phe Ser
       290                 295                 300
   Ala Ser Arg Phe Trp Asp Asp Cys Ile Lys Tyr Asn Cys Thr Ile Val
   305                 310                 315                 320
   Gln Tyr Ile Gly Glu Leu Cys Arg Tyr Leu Leu Asn Gln Pro Pro Arg
                   325                 330                 335
   Glu Ala Glu Asn Gln His Gln Val Arg Met Ala Leu Gly Asn Gly Leu
               340                 345                 350
   Arg Gln Ser Ile Trp Thr Asn Phe Ser Ser Arg Phe His Ile Pro Gln
           355                 360                 365
   Val Ala Glu Phe Tyr Gly Ala Thr Glu Cys Asn Cys Ser Leu Gly Asn
       370                 375                 380
   Phe Asp Ser Gln Val Gly Ala Cys Gly Phe Asn Ser Arg Ile Leu Ser
   385                 390                 395                 400
   Phe Val Tyr Pro Ile Arg Leu Val Arg Val Asn Glu Asp Thr Met Glu
                   405                 410                 415
   Leu Ile Arg Gly Pro Asp Gly Val Cys Ile Pro Cys Gln Pro Gly Glu
               420                 425                 430
   Pro Gly Gln Leu Val Gly Arg Ile Ile Gln Lys Asp Pro Leu Arg Arg
           435                 440                 445
   Phe Asp Gly Tyr Leu Asn Gln Gly Ala Asn Asn Lys Lys Ile Ala Lys
       450                 455                 460
   Asp Val Phe Lys Lys Gly Asp Gln Ala Tyr Leu Thr Gly Asp Val Leu
   465                 470                 475                 480
   Val Met Asp Glu Leu Gly Tyr Leu Tyr Phe Arg Asp Arg Thr Gly Asp
                   485                 490                 495
   Thr Phe Arg Trp Lys Gly Glu Asn Val Ser Thr Thr Glu Val Glu Gly
               500                 505                 510
   Thr Leu Ser Arg Leu Leu Asp Met Ala Asp Val Ala Val Tyr Gly Val
           515                 520                 525
   Glu Val Pro Gly Thr Glu Gly Arg Ala Gly Met Ala Ala Val Ala Ser
       530                 535                 540
   Pro Thr Gly Asn Cys Asp Leu Glu Arg Phe Ala Gln Val Leu Glu Lys
   545                 550                 555                 560
   Glu Leu Pro Leu Tyr Ala Arg Pro Ile Phe Leu Arg Leu Leu Pro Glu
                   565                 570                 575
   Leu His Lys Thr Gly Thr Tyr Lys Phe Gln Lys Thr Glu Leu Arg Lys
               580                 585                 590
   Glu Gly Phe Asp Pro Ala Ile Val Lys Asp Pro Leu Phe Tyr Leu Asp
           595                 600                 605
   Ala Gln Lys Gly Arg Tyr Val Pro Leu Asp Gln Glu Ala Tyr Ser Arg
       610                 615                 620
   Ile Gln Ala Gly Glu Glu Lys Leu
   625                 630
<210> 35
   <211> 632
   <212> PRT
   <213> Mus musculus
<400> 35
   Leu Gly Ser Lys Leu Val Leu Lys Leu Pro Trp Thr Gln Val Gly Phe
    1               5                  10                  15
   Ser Leu Leu Leu Leu Tyr Leu Gly Ser Gly Gly Trp Arg Phe Ile Arg
               20                  25                  30
   Val Phe Ile Lys Thr Val Arg Arg Asp Ile Phe Gly Gly Met Val Leu
           35                  40                  45
   Leu Lys Val Lys Thr Lys Val Arg Arg Tyr Leu Gln Glu Arg Lys Thr
       50                  55                  60
   Val Pro Leu Leu Phe Ala Ser Met Val Gln Arg His Pro Asp Lys Thr
   65                  70                  75                  80
   Ala Leu Ile Phe Glu Gly Thr Asp Thr His Trp Thr Phe Arg Gln Leu
                   85                  90                  95
   Asp Glu Tyr Ser Ser Ser Val Ala Asn Phe Leu Gln Ala Arg Gly Leu
               100                 105                 110
   Ala Ser Gly Asn Val Val Ala Leu Phe Met Glu Asn Arg Asn Glu Phe
           115                 120                 125
   Val Gly Leu Trp Leu Gly Met Ala Lys Leu Gly Val Glu Ala Ala Leu
       130                 135                 140
   Ile Asn Thr Asn Leu Arg Arg Asp Ala Leu Arg His Cys Leu Asp Thr
   145                 150                 155                 160
   Ser Lys Ala Arg Ala Leu Ile Phe Gly Ser Glu Met Ala Ser Ala Ile
                   165                 170                 175
   Cys Glu Ile His Ala Ser Leu Glu Pro Thr Leu Ser Leu Phe Cys Ser
               180                 185                 190
   Gly Ser Trp Glu Pro Ser Thr Val Pro Val Ser Thr Glu His Leu Asp
           195                 200                 205
   Pro Leu Leu Glu Asp Ala Pro Lys His Leu Pro Ser His Pro Asp Lys
       210                 215                 220
   Gly Phe Thr Asp Lys Leu Phe Tyr Ile Tyr Thr Ser Gly Thr Thr Gly
   225                 230                 235                 240
   Leu Pro Lys Ala Ala Ile Val Val His Ser Arg Tyr Tyr Arg Met Ala
                   245                 250                 255
   Ser Leu Val Tyr Tyr Gly Phe Arg Met Arg Pro Asp Asp Ile Val Tyr
               260                 265                 270
   Asp Cys Leu Pro Leu Tyr His Ser Ser Arg Lys His Arg Gly Asp Trp
           275                 280                 285
   Gln Cys Leu Leu His Gly Met Thr Val Val Ile Arg Lys Lys Phe Ser
       290                 295                 300
   Ala Ser Arg Phe Trp Asp Asp Cys Ile Lys Tyr Asn Cys Thr Val Val
   305                 310                 315                 320
   Gln Tyr Ile Gly Glu Leu Cys Arg Tyr Leu Leu Asn Gln Pro Pro Arg
                   325                 330                 335
   Glu Ala Glu Ser Arg His Lys Val Arg Met Ala Leu Gly Asn Gly Leu
               340                 345                 350
   Arg Gln Ser Ile Trp Thr Asp Phe Ser Ser Arg Phe His Ile Pro Gln
           355                 360                 365
   Val Ala Glu Phe Tyr Gly Ala Thr Glu Cys Asn Cys Ser Leu Gly Asn
       370                 375                 380
   Phe Asp Ser Arg Val Gly Ala Cys Gly Phe Asn Ser Arg Ile Leu Ser
   385                 390                 395                 400
   Phe Val Tyr Pro Ile Arg Leu Val Arg Val Asn Glu Asp Thr Met Glu
                   405                 410                 415
   Leu Ile Arg Gly Pro Asp Gly Val Cys Ile Pro Cys Gln Pro Gly Gln
               420                 425                 430
   Pro Gly Gln Leu Val Gly Arg Ile Ile Gln Gln Asp Pro Leu Arg Arg
           435                 440                 445
   Phe Asp Gly Tyr Leu Asn Gln Gly Ala Asn Asn Lys Lys Ile Ala Asn
       450                 455                 460
   Asp Val Phe Lys Lys Gly Asp Gln Ala Tyr Leu Thr Gly Asp Val Leu
   465                 470                 475                 480
   Val Met Asp Glu Leu Gly Tyr Leu Tyr Phe Arg Asp Arg Thr Gly Asp
                   485                 490                 495
   Thr Phe Arg Trp Lys Gly Glu Asn Val Ser Thr Thr Glu Val Glu Gly
               500                 505                 510
   Thr Leu Ser Arg Leu Leu His Met Ala Asp Val Ala Val Tyr Gly Val
           515                 520                 525
   Glu Val Pro Gly Thr Glu Gly Arg Ala Gly Met Ala Ala Val Ala Ser
       530                 535                 540
   Pro Ile Ser Asn Cys Asp Leu Glu Ser Phe Ala Gln Thr Leu Lys Lys
   545                 550                 555                 560
   Glu Leu Pro Leu Tyr Ala Arg Pro Ile Phe Leu Arg Phe Leu Pro Glu
                   565                 570                 575
   Leu His Lys Thr Gly Thr Phe Lys Phe Gln Lys Thr Glu Leu Arg Lys
               580                 585                 590
   Glu Gly Phe Asp Pro Ser Val Val Lys Asp Pro Leu Phe Tyr Leu Asp
           595                 600                 605
   Ala Arg Lys Gly Cys Tyr Val Ala Leu Asp Gln Glu Ala Tyr Thr Arg
       610                 615                 620
   Ile Gln Ala Gly Glu Glu Lys Leu
   625                 630
<210> 36
   <211> 2885
   <212> DNA
   <213> Homo sapiens
<400> 36
   aacggcaagt aagcgcaacg caattaatgt gagtagctca ctcattaggc accccaggct      60
   ttacacttta tgcttccggg ctcgtatgtt gtgtggaatt gtgagcggat accaatttca     120
   cacaggaacc agctatgaca tgattacgaa tttaatacga ctcactatag ggaatttggc     180
   cctcgaggcc aagaattcgg cacgaggggt gctgagcccc tgcgcggttt ctggtgcgta     240
   gagactgtaa atcgctgcgc ttctcagtca tcatcatccc agcttttccc ggctcgaatt     300
   cagcctccaa ctcaagctcg cgggaaagac tacctgagag gagaaaagct tctgtccctg     360
   gaccttcttc tgagggtgga gtcggaggct ccctgctttc cagccgccca gtgacccaag     420
   cttaatcttc agcaccactt ggggcgacct tttcggtgca aacctacgat tctgtttctc     480
   aggattcctc cccatcccgc ttcgccccgg aaaagctgac aagaacttca ggtgtaagcc     540
   ctgagtagtg aggatctgcg gtctccgtgg agagctgtgc ctggaagaga aggacgctgg     600
   tgggggctga gatcagagct gtcttctggc ccagttgccc ccatgcttct gtcatggcta     660
   acagttctag gggctggaat ggtcgtcctg cacttcttgc agaaactcct gttcccttac     720
   ttttgggatg acttctggtt cgtgttgaag gtggtgctca ttataattcg gctgaagaag     780
   tatgaaaaga gaggggagct ggtgactgtg ctggataaat tcttgagtca tgccaaaaga     840
   caacctcgga aacctttcat catctatgag ggagacatct acacctatca ggatgtagac     900
   aaaaggagca gcagagtggc ccatgtcttc ctgaaccatt cctctctgaa aaagggggac      960
   acggtggctc tgctgatgag caatgagccg gacttcgttc acgtgtggtt cggcctcgcc    1020
   aagctgggct gcgtggtggc ctttctcaac accaacattc gctccaactc cctcctgaat    1080
   tgcatccgcg cctgtgggcc cagagcccta gtggtgggcg cagatttgct tggaacggta    1140
   gaagaaatcc ttccaagcct ctcagaaaat atcagtgttt gggggatgaa agattctgtt    1200
   ccacaaggtg taatttcact caaagaaaaa ctgagcacct cacctgatga gcccgtgcca    1260
   cgcagccacc atgttgtctc actcctcaag tctacttgtc tttacatttt tacctctgga    1320
   acaacaggtc taccaaaagc agctgtgatt agtcagctgc aggttttaag gggttctgct    1380
   gtcctgtggg cttttggttg tactgctcat gacattgttt atataaccct tcctctgtat    1440
   catagttcag cagctatcct gggaatttct ggatgtgttg agttgggtgc cacttgtgtg    1500
   ttaaagaaga aattttcagc aagccagttt tggagtgact gcaagaagta tgatgtgact    1560
   gtgtttcagt atattggaga actttgtcgc tacctttgca aacaatctaa gagagaagga    1620
   gaaaaggatc ataaggtgcg tttggcaatt ggaaatggca tacggagtga tgtatggaga    1680
   gaatttttag acagatttgg aaatataaag gtgtgtgaac tttatgcagc taccgaatca     1740
   agcatatctt tcatgaacta cactgggaga attggagcaa ttgggagaac aaatttgttt    1800
   tacaaacttc tttccacttt tgacttaata aagtatgact ttcagaaaga tgaacccatg    1860
   agaaatgagc agggttggtg tattcatgtg aaaaaaggag aacctggact tctcatttct    1920
   cgagtgaatg caaaaaatcc cttctttggc tatgctgggc cttataagca cacaaaagac    1980
   aaattgcttt gtgatgtttt taagaaggga gatgtttacc ttaatactgg agacttaata    2040
   gtccaggatc aggacaattt cctttatttt tgggaccgta ctggagacac tttcagatgg    2100
   aaaggagaaa atgtcgcaac cactgaggtt gctgatgtta ttggaatgtt ggatttcata    2160
   caggaagcaa acgtctatgg tgtggctata tcaggttatg aaggaagagc aggaatggct    2220
   tctattattt taaaaccaaa tacatcttta gatttggaaa aagtttatga acaagttgta    2280
   acatttctac cagcttatgc ttgtccacga tttttaagaa ttcaggaaaa aatggaagca    2340
   acaggaacat tcaaactatt gaagcatcag ttggtggaag atggatttaa tccactgaaa    2400
   atttctgaac cactttactt catggataac ttgaaaaagt cttatgttct actgaccagg    2460
   gaactttatg atcaaataat gttaggggaa ataaaacttt aagattttta tatctagaac    2520
   tttcatatgc tttcttagga agagtgagag gggggtatat gattctttat gaaatgggga    2580
   aagggagcta acattaatta tgcatgtact atatttcctt aatatgagag ataatttttt    2640
   aattgcataa gaattttaat ttcttttaat tgatataaac attagttgat tattcttttt    2700
   atctatttgg agattcagtg cataactaag tattttcctt aatactaaag attttaaata    2760
   ataaatagtg gctagcggtt tggacaatca ctaaaaatgt actttctaat aagtaaaatt    2820
   tctaattttg aataaaagat taaattttac tgaaaaaaaa aaaaaaaaaa aaaattggcg    2880
   gccgc                                                                2885
<210> 37
   <211> 619
   <212> PRT
   <213> Homo sapiens
<400> 37
   Met Leu Leu Ser Trp Leu Thr Val Leu Gly Ala Gly Met Val Val Leu
    1               5                  10                  15
   His Phe Leu Gln Lys Leu Leu Phe Pro Tyr Phe Trp Asp Asp Phe Trp
               20                  25                  30
   Phe Val Leu Lys Val Val Leu Ile Ile Ile Arg Leu Lys Lys Tyr Glu
           35                  40                  45
   Lys Arg Gly Glu Leu Val Thr Val Leu Asp Lys Phe Leu Ser His Ala
       50                  55                  60
   Lys Arg Gln Pro Arg Lys Pro Phe Ile Ile Tyr Glu Gly Asp Ile Tyr
   65                  70                  75                  80
   Thr Tyr Gln Asp Val Asp Lys Arg Ser Ser Arg Val Ala His Val Phe
                   85                  90                  95
   Leu Asn His Ser Ser Leu Lys Lys Gly Asp Thr Val Ala Leu Leu Met
               100                 105                 110
   Ser Asn Glu Pro Asp Phe Val His Val Trp Phe Gly Leu Ala Lys Leu
           115                 120                 125
   Gly Cys Val Val Ala Phe Leu Asn Thr Asn Ile Arg Ser Asn Ser Leu
       130                 135                 140
   Leu Asn Cys Ile Arg Ala Cys Gly Pro Arg Ala Leu Val Val Gly Ala
   145                 150                 155                 160
   Asp Leu Leu Gly Thr Val Glu Glu Ile Leu Pro Ser Leu Ser Glu Asn
                   165                 170                 175
   Ile Ser Val Trp Gly Met Lys Asp Ser Val Pro Gln Gly Val Ile Ser
               180                 185                 190
   Leu Lys Glu Lys Leu Ser Thr Ser Pro Asp Glu Pro Val Pro Arg Ser
           195                 200                 205
   His His Val Val Ser Leu Leu Lys Ser Thr Cys Leu Tyr Ile Phe Thr
       210                 215                 220
   Ser Gly Thr Thr Gly Leu Pro Lys Ala Ala Val Ile Ser Gln Leu Gln
   225                 230                 235                 240
   Val Leu Arg Gly Ser Ala Val Leu Trp Ala Phe Gly Cys Thr Ala His
                   245                 250                 255
   Asp Ile Val Tyr Ile Thr Leu Pro Leu Tyr His Ser Ser Ala Ala Ile
               260                 265                 270
   Leu Gly Ile Ser Gly Cys Val Glu Leu Gly Ala Thr Cys Val Leu Lys
           275                 280                 285
   Lys Lys Phe Ser Ala Ser Gln Phe Trp Ser Asp Cys Lys Lys Tyr Asp
       290                 295                 300
   Val Thr Val Phe Gln Tyr Ile Gly Glu Leu Cys Arg Tyr Leu Cys Lys
   305                 310                 315                 320
   Gln Ser Lys Arg Glu Gly Glu Lys Asp His Lys Val Arg Leu Ala Ile
                   325                 330                 335
   Gly Asn Gly Ile Arg Ser Asp Val Trp Arg Glu Phe Leu Asp Arg Phe
               340                 345                 350
   Gly Asn Ile Lys Val Cys Glu Leu Tyr Ala Ala Thr Glu Ser Ser Ile
           355                 360                 365
   Ser Phe Met Asn Tyr Thr Gly Arg Ile Gly Ala Ile Gly Arg Thr Asn
       370                 375                 380
   Leu Phe Tyr Lys Leu Leu Ser Thr Phe Asp Leu Ile Lys Tyr Asp Phe
   385                 390                 395                 400
   Gln Lys Asp Glu Pro Met Arg Asn Glu Gln Gly Trp Cys Ile His Val
                   405                 410                 415
   Lys Lys Gly Glu Pro Gly Leu Leu Ile Ser Arg Val Asn Ala Lys Asn
               420                 425                 430
   Pro Phe Phe Gly Tyr Ala Gly Pro Tyr Lys His Thr Lys Asp Lys Leu
           435                 440                 445
   Leu Cys Asp Val Phe Lys Lys Gly Asp Val Tyr Leu Asn Thr Gly Asp
       450                 455                 460
   Leu Ile Val Gln Asp Gln Asp Asn Phe Leu Tyr Phe Trp Asp Arg Thr
   465                 470                 475                 480
   Gly Asp Thr Phe Arg Trp Lys Gly Glu Asn Val Ala Thr Thr Glu Val
                   485                 490                 495
   Ala Asp Val Ile Gly Met Leu Asp Phe Ile Gln Glu Ala Asn Val Tyr
               500                 505                 510
   Gly Val Ala Ile Ser Gly Tyr Glu Gly Arg Ala Gly Met Ala Ser Ile
           515                 520                 525
   Ile Leu Lys Pro Asn Thr Ser Leu Asp Leu Glu Lys Val Tyr Glu Gln
       530                 535                 540
   Val Val Thr Phe Leu Pro Ala Tyr Ala Cys Pro Arg Phe Leu Arg Ile
   545                 550                 555                 560
   Gln Glu Lys Met Glu Ala Thr Gly Thr Phe Lys Leu Leu Lys His Gln
                   565                 570                 575
   Leu Val Glu Asp Gly Phe Asn Pro Leu Lys Ile Ser Glu Pro Leu Tyr
               580                 585                 590
   Phe Met Asp Asn Leu Lys Lys Ser Tyr Val Leu Leu Thr Arg Glu Leu
           595                 600                 605
   Tyr Asp Gln Ile Met Leu Gly Glu Ile Lys Leu
       610                 615
<210> 38
   <211> 646
   <212> PRT
   <213> Homo sapiens
<400> 38
   Met Arg Ala Pro Gly Ala Gly Ala Ala Ser Val Val Ser Leu Ala Leu
    1               5                  10                  15
   Leu Trp Leu Leu Gly Leu Pro Trp Thr Trp Ser Ala Ala Ala Ala Leu
               20                  25                  30
   Gly Val Tyr Val Gly Ser Gly Gly Trp Arg Phe Leu Arg Ile Val Cys
           35                  40                  45
   Lys Thr Ala Arg Arg Asp Leu Phe Gly Leu Ser Val Leu Ile Arg Val
       50                  55                  60
   Arg Leu Glu Leu Arg Arg His Gln Arg Ala Gly His Thr Ile Pro Arg
   65                  70                  75                  80
   Ile Phe Gln Ala Val Val Gln Arg Gln Pro Glu Arg Leu Ala Leu Val
                   85                  90                  95
   Asp Ala Gly Thr Gly Glu Cys Trp Thr Phe Ala Gln Leu Asp Ala Tyr
               100                 105                 110
   Ser Asn Ala Val Ala Asn Leu Phe Arg Gln Leu Gly Phe Ala Pro Gly
           115                 120                 125
   Asp Val Val Ala Ile Phe Leu Glu Gly Arg Pro Glu Phe Val Gly Leu
       130                 135                 140
   Trp Leu Gly Leu Ala Lys Ala Gly Met Glu Ala Ala Leu Leu Asn Val
   145                 150                 155                 160
   Asn Leu Arg Arg Glu Pro Leu Ala Phe Cys Leu Gly Thr Ser Gly Ala
                   165                 170                 175
   Lys Ala Leu Ile Phe Gly Gly Glu Met Val Ala Ala Val Ala Glu Val
               180                 185                 190
   Ser Gly His Leu Gly Lys Ser Leu Ile Lys Phe Cys Ser Gly Asp Leu
           195                 200                 205
   Gly Pro Glu Gly Ile Leu Pro Asp Thr His Leu Leu Asp Pro Leu Leu
       210                 215                 220
   Lys Glu Ala Ser Thr Ala Pro Leu Ala Gln Ile Pro Ser Lys Gly Met
   225                 230                 235                 240
   Asp Asp Arg Leu Phe Tyr Ile Tyr Thr Ser Gly Thr Thr Gly Leu Pro
                   245                 250                 255
   Lys Ala Ala Ile Val Val His Ser Arg Tyr Tyr Arg Met Ala Ala Phe
               260                 265                 270
   Gly His His Ala Tyr Arg Met Gln Ala Ala Asp Val Leu Tyr Asp Cys
           275                 280                 285
   Leu Pro Leu Tyr His Ser Ala Gly Asn Ile Ile Gly Val Gly Gln Cys
       290                 295                 300
   Leu Ile Tyr Gly Leu Thr Val Val Leu Arg Lys Lys Phe Ser Ala Ser
   305                 310                 315                 320
   Arg Phe Trp Asp Asp Cys Ile Lys Tyr Asn Cys Thr Val Val Gln Tyr
                   325                 330                 335
   Ile Gly Glu Ile Cys Arg Tyr Leu Leu Lys Gln Pro Val Arg Glu Ala
               340                 345                 350
   Glu Arg Arg His Arg Val Arg Leu Ala Val Gly Asn Gly Leu Arg Pro
           355                 360                 365
   Ala Ile Trp Glu Glu Phe Thr Glu Arg Phe Gly Val Arg Gln Ile Gly
       370                 375                 380
   Glu Phe Tyr Gly Ala Thr Glu Cys Asn Cys Ser Ile Ala Asn Met Asp
   385                 390                 395                 400
   Gly Lys Val Gly Ser Cys Gly Phe Asn Ser Arg Ile Leu Pro His Val
   405 410 415
   Tyr Pro Ile Arg Leu Val Lys Val Asn Glu Asp Thr Met Glu Leu Leu
   420 425 430
   Arg Asp Ala Gln Gly Leu Cys Ile Pro Cys Gln Ala Gly Glu Pro Gly
   435 440 445
   Leu Leu Val Gly Gln Ile Asn Gln Gln Asp Pro Leu Arg Arg Phe Asp
   450 455 460
   Gly Tyr Val Ser Glu Ser Ala Thr Ser Lys Lys Ile Ala His Ser Val
   465 470 475 480
   Phe Ser Lys Gly Asp Ser Ala Tyr Leu Ser Gly Asp Val Leu Val Met
   485 490 495
   Asp Glu Leu Gly Tyr Met Tyr Phe Arg Asp Arg Ser Gly Asp Thr Phe
   500 505 510
   Arg Trp Arg Gly Glu Asn Val Ser Thr Thr Glu Val Glu Gly Val Leu
   515 520 525
   Ser Arg Leu Leu Gly Gln Thr Asp Val Ala Val Tyr Gly Val Ala Val
   530 535 540
   Pro Gly Val Glu Gly Lys Ala Gly Met Ala Ala Val Ala Asp Pro His
   545 550 555 560
   Ser Leu Leu Asp Pro Asn Ala Ile Tyr Gln Glu Leu Gln Lys Val Leu
   565 570 575
   Ala Pro Tyr Ala Arg Pro Ile Phe Leu Arg Leu Leu Pro Gln Val Asp
   580 585 590
   Thr Thr Gly Thr Phe Lys Ile Gln Lys Thr Arg Leu Gln Arg Glu Gly
   595 600 605
   Phe Asp Pro Arg Gln Thr Ser Asp Arg Leu Phe Phe Leu Asp Leu Lys
   610 615 620
   Gln Gly His Tyr Leu Pro Leu Asn Glu Ala Val Tyr Thr Arg Ile Cys
   625 630 635 640
   Ser Gly Ala Phe Ala Leu
   645
<210> 39
   <211> 632
   <212> PRT
   <213> Homo sapiens
<400> 39
   Leu Phe Ser Lys Leu Val Leu Lys Leu Pro Trp Thr Gln Val Gly Phe
    1               5                  10                  15
   Ser Leu Leu Phe Leu Tyr Leu Gly Ser Gly Gly Trp Arg Phe Ile Arg
               20                  25                  30
   Val Phe Ile Lys Thr Ile Arg Arg Asp Ile Phe Gly Gly Leu Val Leu
           35                  40                  45
   Leu Lys Val Lys Ala Lys Val Arg Gln Cys Leu Gln Glu Arg Arg Thr
       50                  55                  60
   Val Pro Ile Leu Phe Ala Ser Thr Val Arg Arg His Pro Asp Lys Thr
   65                  70                  75                  80
   Ala Leu Ile Phe Glu Gly Thr Asp Thr His Trp Thr Phe Arg Gln Leu
                   85                  90                  95
   Asp Glu Tyr Ser Ser Ser Val Ala Asn Phe Leu Gln Ala Arg Gly Leu
               100                 105                 110
   Ala Ser Gly Asp Val Ala Ala Ile Phe Met Glu Asn Arg Asn Glu Phe
           115                 120                 125
   Val Gly Leu Trp Leu Gly Met Ala Lys Leu Gly Val Glu Ala Ala Leu
       130                 135                 140
   Ile Asn Thr Asn Leu Arg Arg Asp Ala Leu Leu His Cys Leu Thr Thr
   145                 150                 155                 160
   Ser Arg Ala Arg Ala Leu Val Phe Gly Ser Glu Met Ala Ser Ala Ile
                   165                 170                 175
   Cys Glu Val His Ala Ser Leu Asp Pro Ser Leu Ser Leu Phe Cys Ser
               180                 185                 190
   Gly Ser Trp Glu Pro Gly Ala Val Pro Pro Ser Thr Glu His Leu Asp
           195                 200                 205
   Pro Leu Leu Lys Asp Ala Pro Lys His Leu Pro Ser Cys Pro Asp Lys
       210                 215                 220
   Gly Phe Thr Asp Lys Leu Phe Tyr Ile Tyr Thr Ser Gly Thr Thr Gly
   225                 230                 235                 240
   Leu Pro Lys Ala Ala Ile Val Val His Ser Arg Tyr Tyr Arg Met Ala
                   245                 250                 255
   Ala Leu Val Tyr Tyr Gly Phe Arg Met Arg Pro Asn Asp Ile Val Tyr
               260                 265                 270
   Asp Cys Leu Pro Leu Tyr His Ser Ala Gly Asn Ile Val Gly Ile Gly
           275                 280                 285
   Gln Cys Leu Leu His Gly Met Thr Val Val Ile Arg Lys Lys Phe Ser
       290                 295                 300
   Ala Ser Arg Phe Trp Asp Asp Cys Ile Lys Tyr Asn Cys Thr Ile Val
   305                 310                 315                 320
   Gln Tyr Ile Gly Glu Leu Cys Arg Tyr Leu Leu Asn Gln Pro Pro Arg
                   325                 330                 335
   Glu Ala Glu Asn Gln His Gln Val Arg Met Ala Leu Gly Asn Gly Leu
               340                 345                 350
   Arg Gln Ser Ile Trp Thr Asn Phe Ser Ser Arg Phe His Ile Pro Gln
           355                 360                 365
   Val Ala Glu Phe Tyr Gly Ala Thr Glu Cys Asn Cys Ser Leu Gly Asn
       370                 375                 380
   Phe Asp Ser Gln Val Gly Ala Cys Gly Phe Asn Ser Arg Ile Leu Ser
   385                 390                 395                 400
   Phe Val Tyr Pro Ile Arg Leu Val Arg Val Asn Glu Asp Thr Met Glu
                   405                 410                 415
   Leu Ile Arg Gly Pro Asp Gly Val Cys Ile Pro Cys Gln Pro Gly Glu
               420                 425                 430
   Pro Gly Gln Leu Val Gly Arg Ile Ile Gln Lys Asp Pro Leu Arg Arg
           435                 440                 445
   Phe Asp Gly Tyr Leu Asn Gln Gly Ala Asn Asn Lys Lys Ile Ala Lys
       450                 455                 460
   Asp Val Phe Lys Lys Gly Asp Gln Ala Tyr Leu Thr Gly Asp Val Leu
   465                 470                 475                 480
   Val Met Asp Glu Leu Gly Tyr Leu Tyr Phe Arg Asp Arg Thr Gly Asp
                   485                 490                 495
   Thr Phe Arg Trp Lys Gly Glu Asn Val Ser Thr Thr Glu Val Glu Gly
               500                 505                 510
   Thr Leu Ser Arg Leu Leu Asp Met Ala Asp Val Ala Val Tyr Gly Val
           515                 520                 525
   Glu Val Pro Gly Thr Glu Gly Arg Ala Gly Met Ala Ala Val Ala Ser
       530                 535                 540
   Pro Thr Gly Asn Cys Asp Leu Glu Arg Phe Ala Gln Val Leu Glu Lys
   545                 550                 555                 560
   Glu Leu Pro Leu Tyr Ala Arg Pro Ile Phe Leu Arg Leu Leu Pro Glu
                   565                 570                 575
   Leu His Lys Thr Gly Thr Tyr Lys Phe Gln Lys Thr Glu Leu Arg Lys
               580                 585                 590
   Glu Gly Phe Asp Pro Ala Ile Val Lys Asp Pro Leu Phe Tyr Leu Asp
   595 600 605
   Ala Gln Lys Gly Arg Tyr Val Pro Leu Asp Gln Glu Ala Tyr Ser Arg
   610 615 620
   Ile Gln Ala Gly Glu Glu Lys Leu
   625 630
<210> 40
   <211> 619
   <212> PRT
   <213> Homo sapiens
<400> 40
   Met Leu Leu Ser Trp Leu Thr Val Leu Gly Ala Gly Met Val Val Leu
    1               5                  10                  15
   His Phe Leu Gln Lys Leu Leu Phe Pro Tyr Phe Trp Asp Asp Phe Trp
               20                  25                  30
   Phe Val Leu Lys Val Val Leu Ile Ile Ile Arg Leu Lys Lys Tyr Glu
           35                  40                  45
   Lys Arg Gly Glu Leu Val Thr Val Leu Asp Lys Phe Leu Ser His Ala
       50                  55                  60
   Lys Arg Gln Pro Arg Lys Pro Phe Ile Ile Tyr Glu Gly Asp Ile Tyr
   65                  70                  75                  80
   Thr Tyr Gln Asp Val Asp Lys Arg Ser Ser Arg Val Ala His Val Phe
                   85                  90                  95
   Leu Asn His Ser Ser Leu Lys Lys Gly Asp Thr Val Ala Leu Leu Met
               100                 105                 110
   Ser Asn Glu Pro Asp Phe Val His Val Trp Phe Gly Leu Ala Lys Leu
           115                 120                 125
   Gly Cys Val Val Ala Phe Leu Asn Thr Asn Ile Arg Ser Asn Ser Leu
       130                 135                 140
   Leu Asn Cys Ile Arg Ala Cys Gly Pro Arg Ala Leu Val Val Gly Ala
   145                 150                 155                 160
   Asp Leu Leu Gly Thr Val Glu Glu Ile Leu Pro Ser Leu Ser Glu Asn
                   165                 170                 175
   Ile Ser Val Trp Gly Met Lys Asp Ser Val Pro Gln Gly Val Ile Ser
               180                 185                 190
   Leu Lys Glu Lys Leu Ser Thr Ser Pro Asp Glu Pro Val Pro Arg Ser
           195                 200                 205
   His His Val Val Ser Leu Leu Lys Ser Thr Cys Leu Tyr Ile Phe Thr
       210                 215                 220
   Ser Gly Thr Thr Gly Leu Pro Lys Ala Ala Val Ile Ser Gln Leu Gln
   225                 230                 235                 240
   Val Leu Arg Gly Ser Ala Val Leu Trp Ala Phe Gly Cys Thr Ala His
                   245                 250                 255
   Asp Ile Val Tyr Ile Thr Leu Pro Leu Tyr His Ser Ser Ala Ala Ile
               260                 265                 270
   Leu Gly Ile Ser Gly Cys Val Glu Leu Gly Ala Thr Cys Val Leu Lys
           275                 280                 285
   Lys Lys Phe Ser Ala Ser Gln Phe Trp Ser Asp Cys Lys Lys Tyr Asp
       290                 295                 300
   Val Thr Val Phe Gln Tyr Ile Gly Glu Leu Cys Arg Tyr Leu Cys Lys
   305                 310                 315                 320
   Gln Ser Lys Arg Glu Gly Glu Lys Asp His Lys Val Arg Leu Ala Ile
                   325                 330                 335
   Gly Asn Gly Ile Arg Ser Asp Val Trp Arg Glu Phe Leu Asp Arg Phe
               340                 345                 350
   Gly Asn Ile Lys Val Cys Glu Leu Tyr Ala Ala Thr Glu Ser Ser Ile
           355                 360                 365
   Ser Phe Met Asn Tyr Thr Gly Arg Ile Gly Ala Ile Gly Arg Thr Asn
       370                 375                 380
   Leu Phe Tyr Lys Leu Leu Ser Thr Phe Asp Leu Ile Lys Tyr Asp Phe
   385                 390                 395                 400
   Gln Lys Asp Glu Pro Met Arg Asn Glu Gln Gly Trp Cys Ile His Val
                   405                 410                 415
   Lys Lys Gly Glu Pro Gly Leu Leu Ile Ser Arg Val Asn Ala Lys Asn
               420                 425                 430
   Pro Phe Phe Gly Tyr Ala Gly Pro Tyr Lys His Thr Lys Asp Lys Leu
           435                 440                 445
   Leu Cys Asp Val Phe Lys Lys Gly Asp Val Tyr Leu Asn Thr Gly Asp
       450                 455                 460
   Leu Ile Val Gln Asp Gln Asp Asn Phe Leu Tyr Phe Trp Asp Arg Thr
   465                 470                 475                 480
   Gly Asp Thr Phe Arg Trp Lys Gly Glu Asn Val Ala Thr Thr Glu Val
                   485                 490                 495
   Ala Asp Val Ile Gly Met Leu Asp Phe Ile Gln Glu Ala Asn Val Tyr
               500                 505                 510
   Gly Val Ala Ile Ser Gly Tyr Glu Gly Arg Ala Gly Met Ala Ser Ile
           515                 520                 525
   Ile Leu Lys Pro Asn Thr Ser Leu Asp Leu Glu Lys Val Tyr Glu Gln
       530                 535                 540
   Val Val Thr Phe Leu Pro Ala Tyr Ala Cys Pro Arg Phe Leu Arg Ile
   545                 550                 555                 560
   Gln Glu Lys Met Glu Ala Thr Gly Thr Phe Lys Leu Leu Lys His Gln
                   565                 570                 575
   Leu Val Glu Asp Gly Phe Asn Pro Leu Lys Ile Ser Glu Pro Leu Tyr
               580                 585                 590
   Phe Met Asp Asn Leu Lys Lys Ser Tyr Val Leu Leu Thr Arg Glu Leu
           595                 600                 605
   Tyr Asp Gln Ile Met Leu Gly Glu Ile Lys Leu
       610                 615
<210> 41
   <211> 643
   <212> PRT
   <213> Homo sapiens
<400> 41
<210> 42
   <211> 643
   <212> PRT
   <213> Mus musculus
<220>
   <221> VARIANT
   <222> (1)...(643)
   <223> Xaa = Any Amino Acid
<400> 42
<210> 43
   <211> 646
   <212> PRT
   <213> Homo sapiens
<400> 43
   Met Arg Ala Pro Gly Ala Gly Ala Ala Ser Val Val Ser Leu Ala Leu
   1 5 10 15
   Leu Trp Leu Leu Gly Leu Pro Trp Thr Trp Ser Ala Ala Ala Ala Leu
               20                  25                  30
   Gly Val Tyr Val Gly Ser Gly Gly Trp Arg Phe Leu Arg Ile Val Cys
           35                  40                  45
   Lys Thr Ala Arg Arg Asp Leu Phe Gly Leu Ser Val Leu Ile Arg Val
       50                  55                  60
   Arg Leu Glu Leu Arg Arg His Gln Arg Ala Gly His Thr Ile Pro Arg
   65                  70                  75                  80
   Ile Phe Gln Ala Val Val Gln Arg Gln Pro Glu Arg Leu Ala Leu Val
                   85                  90                  95
   Asp Ala Gly Thr Gly Glu Cys Trp Thr Phe Ala Gln Leu Asp Ala Tyr
               100                 105                 110
   Ser Asn Ala Val Ala Asn Leu Phe Arg Gln Leu Gly Phe Ala Pro Gly
           115                 120                 125
   Asp Val Val Ala Ile Phe Leu Glu Gly Arg Pro Glu Phe Val Gly Leu
       130                 135                 140
   Trp Leu Gly Leu Ala Lys Ala Gly Met Glu Ala Ala Leu Leu Asn Val
   145                 150                 155                 160
   Asn Leu Arg Arg Glu Pro Leu Ala Phe Cys Leu Gly Thr Ser Gly Ala
                   165                 170                 175
   Lys Ala Leu Ile Phe Gly Gly Glu Met Val Ala Ala Val Ala Glu Val
               180                 185                 190
   Ser Gly His Leu Gly Lys Ser Leu Ile Lys Phe Cys Ser Gly Asp Leu
           195                 200                 205
   Gly Pro Glu Gly Ile Leu Pro Asp Thr His Leu Leu Asp Pro Leu Leu
       210                 215                 220
   Lys Glu Ala Ser Thr Ala Pro Leu Ala Gln Ile Pro Ser Lys Gly Met
   225                 230                 235                 240
   Asp Asp Arg Leu Phe Tyr Ile Tyr Thr Ser Gly Thr Thr Gly Leu Pro
                   245                 250                 255
   Lys Ala Ala Ile Val Val His Ser Arg Tyr Tyr Arg Met Ala Ala Phe
               260                 265                 270
   Gly His His Ala Tyr Arg Met Gln Ala Ala Asp Val Leu Tyr Asp Cys
           275                 280                 285
   Leu Pro Leu Tyr His Ser Ala Gly Asn Ile Ile Gly Val Gly Gln Cys
       290                 295                 300
   Leu Ile Tyr Gly Leu Thr Val Val Leu Arg Lys Lys Phe Ser Ala Ser
   305                 310                 315                 320
   Arg Phe Trp Asp Asp Cys Ile Lys Tyr Asn Cys Thr Val Val Gln Tyr
                   325                 330                 335
   Ile Gly Glu Ile Cys Arg Tyr Leu Leu Lys Gln Pro Val Arg Glu Ala
               340                 345                 350
   Glu Arg Arg His Arg Val Arg Leu Ala Val Gly Asn Gly Leu Arg Pro
           355                 360                 365
   Ala Ile Trp Glu Glu Phe Thr Glu Arg Phe Gly Val Arg Gln Ile Gly
       370                 375                 380
   Glu Phe Tyr Gly Ala Thr Glu Cys Asn Cys Ser Ile Ala Asn Met Asp
   385                 390                 395                 400
   Gly Lys Val Gly Ser Cys Gly Phe Asn Ser Arg Ile Leu Pro His Val
                   405                 410                 415
   Tyr Pro Ile Arg Leu Val Lys Val Asn Glu Asp Thr Met Glu Leu Leu
               420                 425                 430
   Arg Asp Ala Gln Gly Leu Cys Ile Pro Cys Gln Ala Gly Glu Pro Gly
           435                 440                 445
   Leu Leu Val Gly Gln Ile Asn Gln Gln Asp Pro Leu Arg Arg Phe Asp
       450                 455                 460
   Gly Tyr Val Ser Glu Ser Ala Thr Ser Lys Lys Ile Ala His Ser Val
   465                 470                 475                 480
   Phe Ser Lys Gly Asp Ser Ala Tyr Leu Ser Gly Asp Val Leu Val Met
                   485                 490                 495
   Asp Glu Leu Gly Tyr Met Tyr Phe Arg Asp Arg Ser Gly Asp Thr Phe
               500                 505                 510
   Arg Trp Arg Gly Glu Asn Val Ser Thr Thr Glu Val Glu Gly Val Leu
           515                 520                 525
   Ser Arg Leu Leu Gly Gln Thr Asp Val Ala Val Tyr Gly Val Ala Val
       530                 535                 540
   Pro Gly Val Glu Gly Lys Ala Gly Met Ala Ala Val Ala Asp Pro His
   545                 550                 555                 560
   Ser Leu Leu Asp Pro Asn Ala Ile Tyr Gln Glu Leu Gln Lys Val Leu
                   565                 570                 575
   Ala Pro Tyr Ala Arg Pro Ile Phe Leu Arg Leu Leu Pro Gln Val Asp
               580                 585                 590
   Thr Thr Gly Thr Phe Lys Ile Gln Lys Thr Arg Leu Gln Arg Glu Gly
           595                 600                 605
   Phe Asp Pro Arg Gln Thr Ser Asp Arg Leu Phe Phe Leu Asp Leu Lys
       610                 615                 620
   Gln Gly His Tyr Leu Pro Leu Asn Glu Ala Val Tyr Thr Arg Ile Cys
   625                 630                 635                 640
   Ser Gly Ala Phe Ala Leu
                   645
<210> 44
   <211> 2710
   <212> DNA
   <213> Mus musculus
<400> 44
   atgctgcttg gagcctctct ggtgggggcg ctacttgggt ccaagctagt gctgaagctg      60
   ccctggaccc aggtgggatt ctccctgttg ctcctgtact tggggtctgg tggctggcgt     120
   ttcatccggg tcttcatcaa gacggtcagg agagatatct ttggtggcat ggtgctcctg     180
   aaggtgaaga ccaaggtgcg acggtacctt caggagcgga agacggtgcc cctgctgttt     240
   gcttcaatgg tacagcgcca cccggacaag acagccctga ttttcgaggg cacagacact     300
   cactggacct tccgccagct ggatgagtac tccagtagtg tggccaactt cctgcaggcc     360
   cggggcctgg cctcaggcaa tgtagttgcc ctctttatgg aaaaccgcaa tgagtttgtg     420
   ggtctgtggc taggcatggc caagctgggc gtggaggcgg ctctcatcaa caccaacctt     480
   aggcgggatg ccctgcgcca ctgtcttgac acctcaaagg cacgagctct catctttggc     540
   agtgagatgg cctcagctat ctgtgagatc catgctagcc tggagcccac actcagcctc     600
   ttctgctctg gatcctggga gcccagcaca gtgcccgtca gcacagagca tctggaccct     660
   cttctggaag atgccccgaa gcacctgccc agtcacccag acaagggttt tacagataag     720
   ctcttctaca tctacacatc gggcaccacg gggctaccca aagctgccat tgtggtgcac     780
   agcaggtatt atcgtatggc ttccctggtg tactatggat tccgcatgcg gcctgatgac     840
   attgtctatg actgcctccc cctctaccac tcaagcagga aacatcgtgg ggattggcag     900
   tgcttactcc acggcatgac tgtggtgatc cggaagaagt tctcagcctc ccggttctgg     960
   gatgattgta tcaagtacaa ctgcacagtg gtacagtaca ttggcgagct ctgccgctac    1020
   ctcctgaacc agccaccccg tgaggctgag tctcggcaca aggtgcgcat ggcactgggc    1080
   aacggtctcc ggcagtccat ctggaccgac ttctccagcc gtttccacat cccccaggtg    1140
   gctgagttct atggggccac tgaatgcaac tgtagcctgg gcaactttga cagccgggtg    1200
   ggggcctgtg gcttcaatag ccgcatcctg tcctttgtgt accctatccg tttggtacgt    1260
   gtcaatgagg ataccatgga actgatccgg ggacccgatg gagtctgcat tccctgtcaa    1320
   ccaggtcagc caggccagct ggtgggtcgc atcatccagc aggaccctct gcgccgtttc    1380
   gacgggtacc tcaaccaggg tgccaacaac aagaagattg ctaatgatgt cttcaagaag    1440
   ggggaccaag cctacctcac tggtgacgtc ctggtgatgg atgagctggg ttacctgtac    1500
   ttccgagatc gcactgggga cacgttccgc tggaaagggg agaatgtatc taccactgag    1560
   gtggagggca cactcagccg cctgcttcat atggcagatg tggcagttta tggtgttgag    1620
   gtgccaggaa ctgaaggccg agcaggaatg gctgccgttg caagtcccat cagcaactgt    1680
   gacctggaga gctttgcaca gaccttgaaa aaggagctgc ctctgtatgc ccgccccatc    1740
   ttcctgcgct tcttgcctga gctgcacaag acagggacct tcaagttcca gaagacagag    1800
   ttgcggaagg agggctttga cccatctgtt gtgaaagacc cgctgttcta tctggatgct    1860
   cggaagggct gctacgttgc actggaccag gaggcctata cccgcatcca ggcaggcgag    1920
   gagaagctgt gatttccccc tacatccctc tgagggccag aagatgctgg attcagagcc    1980
   ctagcgtcca ccccagaggg tcctgggcaa tgccagacca aagctagcag ggcccgcacc    2040
   tccgccccta ggtgctgatc tcccctctcc caaactgcca agtgactcac tgccgcttcc    2100
   ccgaccctcc agaggctttc tgtgaaagtc tcatccaagc tgtgtcttct ggtccaggcg    2160
   tggcccctgg ccccagggtt tctgataggc tcctttagga tggtatcttg ggtccagcgg    2220
   gccagggtgt gggagaggag tcactaagat ccctccaatc agaagggagc ttacaaagga     2280
   accaaggcaa agcctgtaga ctcaggaagc taagtggcca gagactatag tggccagtca    2340
   tcccatgtcc acagaggatc ttggtccaga gctgccaaag tgtcacctct ccctgcctgc    2400
   acctctgggg aaaagaggac agcatgtggc cactgggcac ctgtctcaag aagtcaggat    2460
   cacacactca gtccttgttt ctccaggttc ccttgttctt gtctcgggga gggagggacg    2520
   agtgtcctgt ctgtccttcc tgcctgtctg tgagtctgtg ttgcttctcc atctgtccta    2580
   gcctgagtgt gggtggaaca ggcatgagga gagtgtggct caggggccaa taaactctgc    2640
   cttgactcct cttaaaaaaa aaaaaaaaaa aaaaaaaaaa aaaaaaaaaa aaaaaaaaaa    2700
   aaaaaaaaaa                                                           2710
<210> 45
   <211> 643
   <212> PRT
   <213> Mus musculus
<400> 45
   Met Leu Leu Gly Ala Ser Leu Val Gly Ala Leu Leu Phe Ser Lys Leu
    1               5                  10                  15
   Val Leu Lys Leu Pro Trp Thr Gln Val Gly Phe Ser Leu Leu Leu Leu
               20                  25                  30
   Tyr Leu Gly Ser Gly Gly Trp Arg Phe Ile Arg Val Phe Ile Lys Thr
           35                  40                  45
   Val Arg Arg Asp Ile Phe Gly Gly Met Val Leu Leu Lys Val Lys Thr
       50                  55                  60
   Lys Val Arg Arg Tyr Leu Gln Glu Arg Lys Thr Val Pro Leu Leu Phe
   65                  70                  75                  80
   Ala Ser Met Val Gln Arg His Pro Asp Lys Thr Ala Leu Ile Phe Glu
                   85                  90                  95
   Gly Thr Asp Thr His Trp Thr Phe Arg Gln Leu Asp Glu Tyr Ser Ser
               100                 105                 110
   Ser Val Ala Asn Phe Leu Gln Ala Arg Gly Leu Ala Ser Gly Asn Val
           115                 120                 125
   Val Ala Leu Phe Met Glu Asn Arg Asn Glu Phe Val Gly Leu Trp Leu
       130                 135                 140
   Gly Met Ala Lys Leu Gly Val Glu Ala Ala Leu Ile Asn Thr Asn Leu
   145                 150                 155                 160
   Arg Arg Asp Ala Leu Arg His Cys Leu Asp Thr Ser Lys Ala Arg Ala
                   165                 170                 175
   Leu Ile Phe Gly Ser Glu Met Ala Ser Ala Ile Cys Glu Ile His Ala
               180                 185                 190
   Ser Leu Glu Pro Thr Leu Ser Leu Phe Cys Ser Gly Ser Trp Glu Pro
           195                 200                 205
   Ser Thr Val Pro Val Ser Thr Glu His Leu Asp Pro Leu Leu Glu Asp
       210                 215                 220
   Ala Pro Lys His Leu Pro Ser His Pro Asp Lys Gly Phe Thr Asp Lys
   225                 230                 235                 240
   Leu Phe Tyr Ile Tyr Thr Ser Gly Thr Thr Gly Leu Pro Lys Ala Ala
                   245                 250                 255
   Ile Val Val His Ser Arg Tyr Tyr Arg Met Ala Ser Leu Val Tyr Tyr
               260                 265                 270
   Gly Phe Arg Met Arg Pro Asp Asp Ile Val Tyr Asp Cys Leu Pro Leu
           275                 280                 285
   Tyr His Ser Ser Arg Lys His Arg Gly Asp Trp Gln Cys Leu Leu His
       290                 295                 300
   Gly Met Thr Val Val Ile Arg Lys Lys Phe Ser Ala Ser Arg Phe Trp
   305                 310                 315                 320
   Asp Asp Cys Ile Lys Tyr Asn Cys Thr Val Val Gln Tyr Ile Gly Glu
                   325                 330                 335
   Leu Cys Arg Tyr Leu Leu Asn Gln Pro Pro Arg Glu Ala Glu Ser Arg
               340                 345                 350
   His Lys Val Arg Met Ala Leu Gly Asn Gly Leu Arg Gln Ser Ile Trp
           355                 360                 365
   Thr Asp Phe Ser Ser Arg Phe His Ile Pro Gln Val Ala Glu Phe Tyr
       370                 375                 380
   Gly Ala Thr Glu Cys Asn Cys Ser Leu Gly Asn Phe Asp Ser Arg Val
   385                 390                 395                 400
   Gly Ala Cys Gly Phe Asn Ser Arg Ile Leu Ser Phe Val Tyr Pro Ile
                   405                 410                 415
   Arg Leu Val Arg Val Asn Glu Asp Thr Met Glu Leu Ile Arg Gly Pro
               420                 425                 430
   Asp Gly Val Cys Ile Pro Cys Gln Pro Gly Gln Pro Gly Gln Leu Val
           435                 440                 445
   Gly Arg Ile Ile Gln Gln Asp Pro Leu Arg Arg Phe Asp Gly Tyr Leu
       450                 455                 460
   Asn Gln Gly Ala Asn Asn Lys Lys Ile Ala Asn Asp Val Phe Lys Lys
   465                 470                 475                 480
   Gly Asp Gln Ala Tyr Leu Thr Gly Asp Val Leu Val Met Asp Glu Leu
                   485                 490                 495
   Gly Tyr Leu Tyr Phe Arg Asp Arg Thr Gly Asp Thr Phe Arg Trp Lys
               500                 505                 510
   Gly Glu Asn Val Ser Thr Thr Glu Val Glu Gly Thr Leu Ser Arg Leu
           515                 520                 525
   Leu His Met Ala Asp Val Ala Val Tyr Gly Val Glu Val Pro Gly Thr
       530                 535                 540
   Glu Gly Arg Ala Gly Met Ala Ala Val Ala Ser Pro Ile Ser Asn Cys
   545                 550                 555                 560
   Asp Leu Glu Ser Phe Ala Gln Thr Leu Lys Lys Glu Leu Pro Leu Tyr
                   565                 570                 575
   Ala Arg Pro Ile Phe Leu Arg Phe Leu Pro Glu Leu His Lys Thr Gly
               580                 585                 590
   Thr Phe Lys Phe Gln Lys Thr Glu Leu Arg Lys Glu Gly Phe Asp Pro
           595                 600                 605
   Ser Val Val Lys Asp Pro Leu Phe Tyr Leu Asp Ala Arg Lys Gly Cys
       610                 615                 620
   Tyr Val Ala Leu Asp Gln Glu Ala Tyr Thr Arg Ile Gln Ala Gly Glu
   625                 630                 635                 640
   Glu Lys Leu
<210> 46
   <211> 3694
   <212> DNA
   <213> Homo sapiens
<400> 46
<210> 47
   <211> 646
   <212> PRT
   <213> Homo sapiens
<400> 47
   Met Arg Ala Pro Gly Ala Gly Ala Ala Ser Val Val Ser Leu Ala Leu
   1 5 10 15
   Leu Trp Leu Leu Gly Leu Pro Trp Thr Trp Ser Ala Ala Ala Ala Leu
   20 25 30
   Gly Val Tyr Val Gly Ser Gly Gly Trp Arg Phe Leu Arg Ile Val Cys
   35 40 45
   Lys Thr Ala Arg Arg Asp Leu Phe Gly Leu Ser Val Leu Ile Arg Val
   50 55 60
   Arg Leu Glu Leu Arg Arg His Gln Arg Ala Gly His Thr Ile Pro Arg
   65 70 75 80
   Ile Phe Gln Ala Val Val Gln Arg Gln Pro Glu Arg Leu Ala Leu Val
   85 90 95
   Asp Ala Gly Thr Gly Glu Cys Trp Thr Phe Ala Gln Leu Asp Ala Tyr
   100 105 110
   Ser Asn Ala Val Ala Asn Leu Phe Arg Gln Leu Gly Phe Ala Pro Gly
   115 120 125
   Asp Val Val Ala Ile Phe Leu Glu Gly Arg Pro Glu Phe Val Gly Leu
   130 135 140
   Trp Leu Gly Leu Ala Lys Ala Gly Met Glu Ala Ala Leu Leu Asn Val
   145 150 155 160
   Asn Leu Arg Arg Glu Pro Leu Ala Phe Cys Leu Gly Thr Ser Gly Ala
   165 170 175
   Lys Ala Leu Ile Phe Gly Gly Glu Met Val Ala Ala Val Ala Glu Val
   180 185 190
   Ser Gly His Leu Gly Lys Ser Leu Ile Lys Phe Cys Ser Gly Asp Leu
   195 200 205
   Gly Pro Glu Gly Ile Leu Pro Asp Thr His Leu Leu Asp Pro Leu Leu
   210 215 220
   Lys Glu Ala Ser Thr Ala Pro Leu Ala Gln Ile Pro Ser Lys Gly Met
   225 230 235 240
   Asp Asp Arg Leu Phe Tyr Ile Tyr Thr Ser Gly Thr Thr Gly Leu Pro
   245 250 255
   Lys Ala Ala Ile Val Val His Ser Arg Tyr Tyr Arg Met Ala Ala Phe
   260 265 270
   Gly His His Ala Tyr Arg Met Gln Ala Ala Asp Val Leu Tyr Asp Cys
   275 280 285
   Leu Pro Leu Tyr His Ser Ala Gly Asn Ile Ile Gly Val Gly Gln Cys
   290 295 300
   Leu Ile Tyr Gly Leu Thr Val Val Leu Arg Lys Lys Phe Ser Ala Ser
   305 310 315 320
   Arg Phe Trp Asp Asp Cys Ile Lys Tyr Asn Cys Thr Val Val Gln Tyr
   325 330 335
   Ile Gly Glu Ile Cys Arg Tyr Leu Leu Lys Gln Pro Val Arg Glu Ala
   340 345 350
   Glu Arg Arg His Arg Val Arg Leu Ala Val Gly Asn Gly Leu Arg Pro
           355                 360                 365
   Ala Ile Trp Glu Glu Phe Thr Glu Arg Phe Gly Val Arg Gln Ile Gly
       370                 375                 380
   Glu Phe Tyr Gly Ala Thr Glu Cys Asn Cys Ser Ile Ala Asn Met Asp
   385                 390                 395                 400
   Gly Lys Val Gly Ser Cys Gly Phe Asn Ser Arg Ile Leu Pro His Val
                   405                 410                 415
   Tyr Pro Ile Arg Leu Val Lys Val Asn Glu Asp Thr Met Glu Leu Leu
               420                 425                 430
   Arg Asp Ala Gln Gly Leu Cys Ile Pro Cys Gln Ala Gly Glu Pro Gly
           435                 440                 445
   Leu Leu Val Gly Gln Ile Asn Gln Gln Asp Pro Leu Arg Arg Phe Asp
       450                 455                 460
   Gly Tyr Val Ser Glu Ser Ala Thr Ser Lys Lys Ile Ala His Ser Val
   465                 470                 475                 480
   Phe Ser Lys Gly Asp Ser Ala Tyr Leu Ser Gly Asp Val Leu Val Met
                   485                 490                 495
   Asp Glu Leu Gly Tyr Met Tyr Phe Arg Asp Arg Ser Gly Asp Thr Phe
               500                 505                 510
   Arg Trp Arg Gly Glu Asn Val Ser Thr Thr Glu Val Glu Gly Val Leu
           515                 520                 525
   Ser Arg Leu Leu Gly Gln Thr Asp Val Ala Val Tyr Gly Val Ala Val
       530                 535                 540
   Pro Gly Val Glu Gly Lys Ala Gly Met Ala Ala Val Ala Asp Pro His
   545                 550                 555                 560
   Ser Leu Leu Asp Pro Asn Ala Ile Tyr Gln Glu Leu Gln Lys Val Leu
                   565                 570                 575
   Ala Pro Tyr Ala Arg Pro Ile Phe Leu Arg Leu Leu Pro Gln Val Asp
               580                 585                 590
   Thr Thr Gly Thr Phe Lys Ile Gln Lys Thr Arg Leu Gln Arg Glu Gly
           595                 600                 605
   Phe Asp Pro Arg Gln Thr Ser Asp Arg Leu Phe Phe Leu Asp Leu Lys
       610                 615                 620
   Gln Gly His Tyr Leu Pro Leu Asn Glu Ala Val Tyr Thr Arg Ile Cys
   625                 630                 635                 640
   Ser Gly Ala Phe Ala Leu
                   645
<210> 48
   <211> 2362
   <212> DNA
   <213> Homo sapiens
<400> 48
   ggaattccaa aaaaaaaaaa tacgactaca cctgctccgg agcccgcggc ggtacctgca 60
   gcggaggagc tctgtcttcc ccttcatctc acgcgagccc ggcgtcccgc cgcgtgcgcc 120
   ccggcgcagc ccgccagtcc gcccggagcc cgcccagtcg ccgcgctgca cgcccggggt 180
   gaaccctctg ccctcgctgg gacagagggc cccgcagccg tcatgctttc cgccatctac 240
   acagtcctgg cgggactgct gttcctgccg ctcctggtga acctctgctg cccatacttc 300
   ttccaggaca taggctactt cttgaaggtg gccgccgtgg gccggagggt gcgcagctac 360
   gggcagcggc ggccggcgcg caccatcctg cgggcgttcc tggagaaagc gcgccagacg 420
   ccacacaagc cttttctgct cttccgcgac gagactctca cctacgcgca ggtggaccgg 480
   cgcagcaatc aagtggcccg ggcgctgcac gaccacctcg gcctgcgcca gggagactgc 540
   gtggcgctcc ttatgggtaa cgagccggcc tacgtgtggc tgtggctggg gctggtgaag 600
   ctgggctgtg ccatggcgtg cctcaattac aacatccgcg cgaagtccct gctgcactgc 660
   ttccagtgct gcggggcgaa ggtgctgctg gtgtcgccag aactacaagc agctgtcgaa 720
   gagatactgc caagccttaa aaaagatgat gtgtccatct attatgtgag cagaacttct 780
   aacacagatg ggattgactc tttcctggac aaagtggatg aagtatcaac tgaacctatc 840
   ccagagtcat ggaggtctga agtcactttt tccactcctg ccttatacat ttatacttct 900
   ggaaccacag gtcttccaaa agcagccatg atcactcatc agcgcatatg gtatggaact 960
   ggcctcactt ttgtaagcgg attgaaggca gatgatgtca tctatatcac tctgcccttt 1020
   taccacagtg ctgcactact gattggcatt cacggatgta ttgtggctgg tgctactctt 1080
   gccttgcgga ctaaattttc agccagccag ttttgggatg actgcagaaa atacaacgtc 1140
   actgtcattc agtatatcgg tgaactgctt cggtatttat gcaactcacc acagaaacca 1200
   aatgaccgtg atcataaagt gagactggca ctgggaaatg gcttacgagg agatgtgtgg 1260
   agacaatttg tcaagagatt tggggacata tgcatctatg agttctatgc tgccactgaa 1320
   ggcaatattg gatttatgaa ttatgcgaga aaagttggtg ctgttggaag agtaaactac 1380
   ctacagaaaa aaatcataac ttatgacctg attaaatatg atgtggagaa agatgaacct 1440
   gtccgagatg aaaatggata ttgcgtcaga gttcccaaag gtgaagttgg acttctggtt 1500
   tgcaaaatca cacaacttac accatttaat ggctatgctg gagcaaaggc tcagacagag 1560
   aagaaaaaac tgagagatgt ctttaagaaa ggagacctct atttcaacag tggagatctc 1620
   ttaatggttg accatgaaaa tttcatctat ttccacgaca gagttggaga tacattccgg 1680
   tggaaagggg aaaatgtggc caccactgaa gttgctgata cagttggact ggttgatttt 1740
   gtccaagaag taaatgttta tggagtgcat gtgccagatc atgagggtcg cattggcatg 1800
   gcctccatca aaatgaaaga aaaccatgaa tttgatggaa agaaactctt tcagcacatt 1860
   gctgattacc tacctagtta tgcaaggccc cggtttctaa gaatacagga caccattgag 1920
   atcactggaa cttttaaaca ccgcaaaatg accctggtgg aggagggctt taaccctgct 1980
   gtcatcaaag atgccttgta tttcttggat gacacagcaa aaatgtatgt gcctatgact 2040
   gaggacatct ataatgccat aagtgctaaa accctgaaac tctgaatatt cccaggagga 2100
   taactcaaca tttccagaaa gaaactgaat ggacagccac ttgatataat ccaactttaa 2160
   tttgattgaa gattgtgagg aaattttgta ggaaatttgc atacccgtaa agggagactt 2220
   ttttaaataa cagttgagtc tttgcaagta aaaagattta gagattatta tttttcagtg 2280
   tgcacctact gtttgtattt gcaaactgag cttgttggag ggaaggcatt attttttaaa 2340
   atacttagta aattaaatga ac 2362
<210> 49
   <211> 620
   <212> PRT
   <213> Homo sapiens
<400> 49
   Met Leu Ser Ala Ile Tyr Thr Val Leu Ala Gly Leu Leu Phe Leu Pro
    1               5                  10                  15
   Leu Leu Val Asn Leu Cys Cys Pro Tyr Phe Phe Gln Asp Ile Gly Tyr
               20                  25                  30
   Phe Leu Lys Val Ala Ala Val Gly Arg Arg Val Arg Ser Tyr Gly Gln
           35                  40                  45
   Arg Arg Pro Ala Arg Thr Ile Leu Arg Ala Phe Leu Glu Lys Ala Arg
       50                  55                  60
   Gln Thr Pro His Lys Pro Phe Leu Leu Phe Arg Asp Glu Thr Leu Thr
   65                  70                  75                  80
   Tyr Ala Gln Val Asp Arg Arg Ser Asn Gln Val Ala Arg Ala Leu His
                   85                  90                  95
   Asp His Leu Gly Leu Arg Gln Gly Asp Cys Val Ala Leu Leu Met Gly
               100                 105                 110
   Asn Glu Pro Ala Tyr Val Trp Leu Trp Leu Gly Leu Val Lys Leu Gly
           115                 120                 125
   Cys Ala Met Ala Cys Leu Asn Tyr Asn Ile Arg Ala Lys Ser Leu Leu
       130                 135                 140
   His Cys Phe Gln Cys Cys Gly Ala Lys Val Leu Leu Val Ser Pro Glu
   145                 150                 155                 160
   Leu Gln Ala Ala Val Glu Glu Ile Leu Pro Ser Leu Lys Lys Asp Asp
                   165                 170                 175
   Val Ser Ile Tyr Tyr Val Ser Arg Thr Ser Asn Thr Asp Gly Ile Asp
               180                 185                 190
   Ser Phe Leu Asp Lys Val Asp Glu Val Ser Thr Glu Pro Ile Pro Glu
           195                 200                 205
   Ser Trp Arg Ser Glu Val Thr Phe Ser Thr Pro Ala Leu Tyr Ile Tyr
       210                 215                 220
   Thr Ser Gly Thr Thr Gly Leu Pro Lys Ala Ala Met Ile Thr His Gln
   225                 230                 235                 240
   Arg Ile Trp Tyr Gly Thr Gly Leu Thr Phe Val Ser Gly Leu Lys Ala
                   245                 250                 255
   Asp Asp Val Ile Tyr Ile Thr Leu Pro Phe Tyr His Ser Ala Ala Leu
               260                 265                 270
   Leu Ile Gly Ile His Gly Cys Ile Val Ala Gly Ala Thr Leu Ala Leu
           275                 280                 285
   Arg Thr Lys Phe Ser Ala Ser Gln Phe Trp Asp Asp Cys Arg Lys Tyr
       290                 295                 300
   Asn Val Thr Val Ile Gln Tyr Ile Gly Glu Leu Leu Arg Tyr Leu Cys
   305                 310                 315                 320
   Asn Ser Pro Gln Lys Pro Asn Asp Arg Asp His Lys Val Arg Leu Ala
                   325                 330                 335
   Leu Gly Asn Gly Leu Arg Gly Asp Val Trp Arg Gln Phe Val Lys Arg
               340                 345                 350
   Phe Gly Asp Ile Cys Ile Tyr Glu Phe Tyr Ala Ala Thr Glu Gly Asn
           355                 360                 365
   Ile Gly Phe Met Asn Tyr Ala Arg Lys Val Gly Ala Val Gly Arg Val
       370                 375                 380
   Asn Tyr Leu Gln Lys Lys Ile Ile Thr Tyr Asp Leu Ile Lys Tyr Asp
   385                 390                 395                 400
   Val Glu Lys Asp Glu Pro Val Arg Asp Glu Asn Gly Tyr Cys Val Arg
                   405                 410                 415
   Val Pro Lys Gly Glu Val Gly Leu Leu Val Cys Lys Ile Thr Gln Leu
               420                 425                 430
   Thr Pro Phe Asn Gly Tyr Ala Gly Ala Lys Ala Gln Thr Glu Lys Lys
           435                 440                 445
   Lys Leu Arg Asp Val Phe Lys Lys Gly Asp Leu Tyr Phe Asn Ser Gly
       450                 455                 460
   Asp Leu Leu Met Val Asp His Glu Asn Phe Ile Tyr Phe His Asp Arg
   465                 470                 475                 480
   Val Gly Asp Thr Phe Arg Trp Lys Gly Glu Asn Val Ala Thr Thr Glu
                   485                 490                 495
   Val Ala Asp Thr Val Gly Leu Val Asp Phe Val Gln Glu Val Asn Val
               500                 505                 510
   Tyr Gly Val His Val Pro Asp His Glu Gly Arg Ile Gly Met Ala Ser
           515                 520                 525
   Ile Lys Met Lys Glu Asn His Glu Phe Asp Gly Lys Lys Leu Phe Gln
       530                 535                 540
   His Ile Ala Asp Tyr Leu Pro Ser Tyr Ala Arg Pro Arg Phe Leu Arg
   545                 550                 555                 560
   Ile Gln Asp Thr Ile Glu Ile Thr Gly Thr Phe Lys His Arg Lys Met
                   565                 570                 575
   Thr Leu Val Glu Glu Gly Phe Asn Pro Ala Val Ile Lys Asp Ala Leu
               580                 585                 590
   Tyr Phe Leu Asp Asp Thr Ala Lys Met Tyr Val Pro Met Thr Glu Asp
           595                 600                 605
   Ile Tyr Asn Ala Ile Ser Ala Lys Thr Leu Lys Leu
       610                 615                 620
<210> 50
   <211> 1173
   <212> DNA
   <213> Homo sapiens
<400> 50
   aagttctcgg ctggtcagtt ctgggaagat tgccagcagc acagggtgac ggtgttccag      60
   tacattgggg agctgtgccg ataccttgtc aaccagcccc cgagcaaggc agaacgtggc     120
   cataaggtcc ggctggcagt gggcagcggg ctgcgcccag atacctggga gcgttttgtg     180
   cggcgcttcg ggcccctgca ggtgctggag acatatggac tgacagaggg caacgtggcc    240
   accatcaact acacaggaca gcggggcgct gtggggcgtg cttcctggct ttacaagcat     300
   atcttcccct tctccttgat tcgctatgat gtcaccacag gagagccaat tcgggacccc     360
   caggggcact gtatggccac atctccaggt gagccagggc tgctggtggc cccggtaagc     420
   cagcagtccc cattcctggg ctatgctggc gggccagagc tggcccaggg gaagttgcta     480
   aaggatgtct tccggcctgg ggatgttttc ttcaacactg gggacctgct ggtctgcgat      540
   gaccaaggtt ttctccgctt ccatgatcgt actggagaca ccttcaggtg gaagggggag     600
   aatgtggcca caaccgaggt ggcagaggtc ttcgaggccc tagattttct tcaggaggtg     660
   aacgtctatg gagtcactgt gccagggcat gaaggcaggg ctggaatggc agccctagtt     720
   ctgcgtcccc cccacgcttt ggaccttatg cagctctaca cccacgtgtc tgagaacttg     780
   ccaccttatg cccggccccg attcctcagg ctccaggagt ctttggccac cacagagacc     840
   ttcaaacagc agaaagttcg gatggcaaat gagggcttcg accccagcac cctgtctgac     900
   ccactgtacg ttctggacca ggctgtaggt gcctacctgc ccctcacaac tgcccggtac     960
   agcgccctcc tggcaggaaa ccttcgaatc tgagaacttc cacacctgag gcacctgaga    1020
   gaggaactct gtggggtggg ggccgttgca ggtgtactgg gctgtcaggg atcttttcta    1080
   taccagaact gcggtcacta ttttgtaata aatgtggctg gagctgatcc agctgtctct    1140
   gacaaaaaaa aaaaaaaaaa aaagggcggc cgc                                 1173
<210> 51
   <211> 330
   <212> PRT
   <213> Homo sapiens
<400> 51
   Lys Phe Ser Ala Gly Gln Phe Trp Glu Asp Cys Gln Gln His Arg Val
    1               5                  10                  15
   Thr Val Phe Gln Tyr Ile Gly Glu Leu Cys Arg Tyr Leu Val Asn Gln
               20                  25                  30
   Pro Pro Ser Lys Ala Glu Arg Gly His Lys Val Arg Leu Ala Val Gly
           35                  40                  45
   Ser Gly Leu Arg Pro Asp Thr Trp Glu Arg Phe Val Arg Arg Phe Gly
       50                  55                  60
   Pro Leu Gln Val Leu Glu Thr Tyr Gly Leu Thr Glu Gly Asn Val Ala
   65                  70                  75                  80
   Thr Ile Asn Tyr Thr Gly Gln Arg Gly Ala Val Gly Arg Ala Ser Trp
                   85                  90                  95
   Leu Tyr Lys His Ile Phe Pro Phe Ser Leu Ile Arg Tyr Asp Val Thr
               100                 105                 110
   Thr Gly Glu Pro Ile Arg Asp Pro Gln Gly His Cys Met Ala Thr Ser
           115                 120                 125
   Pro Gly Glu Pro Gly Leu Leu Val Ala Pro Val Ser Gln Gln Ser Pro
       130                 135                 140
   Phe Leu Gly Tyr Ala Gly Gly Pro Glu Leu Ala Gln Gly Lys Leu Leu
   145                 150                 155                 160
   Lys Asp Val Phe Arg Pro Gly Asp Val Phe Phe Asn Thr Gly Asp Leu
                   165                 170                 175
   Leu Val Cys Asp Asp Gln Gly Phe Leu Arg Phe His Asp Arg Thr Gly
               180                 185                 190
   Asp Thr Phe Arg Trp Lys Gly Glu Asn Val Ala Thr Thr Glu Val Ala
           195                 200                 205
   Glu Val Phe Glu Ala Leu Asp Phe Leu Gln Glu Val Asn Val Tyr Gly
       210                 215                 220
   Val Thr Val Pro Gly His Glu Gly Arg Ala Gly Met Ala Ala Leu Val
   225                 230                 235                 240
   Leu Arg Pro Pro His Ala Leu Asp Leu Met Gln Leu Tyr Thr His Val
                   245                 250                 255
   Ser Glu Asn Leu Pro Pro Tyr Ala Arg Pro Arg Phe Leu Arg Leu Gln
               260                 265                 270
   Glu Ser Leu Ala Thr Thr Glu Thr Phe Lys Gln Gln Lys Val Arg Met
           275                 280                 285
   Ala Asn Glu Gly Phe Asp Pro Ser Thr Leu Ser Asp Pro Leu Tyr Val
       290                 295                 300
   Leu Asp Gln Ala Val Gly Ala Tyr Leu Pro Leu Thr Thr Ala Arg Tyr
   305                 310                 315                 320
   Ser Ala Leu Leu Ala Gly Asn Leu Arg Ile
                   325                 330
<210> 52
   <211> 2907
   <212> DNA
   <213> Homo sapiens
<400> 52
   cgacccacgc gtccgggcgg gcggggccgg gcggcgggcg gggctggcgg ggcggccggg     60
   ccatgcaggg cgcagagccg gctaaaccct gctgagaccc ggctccgtgc gtccaggggc    120
   ggctaatgcc cctcacgctg tctacgctgc tgcaaccggg ccgcatctgg acggggcgcc    180
   gcgcggcgga gccgacgccg ggccacaatg ctgcttggag cctctctggt gggggtgctg    240
   ctgttctcca agctggtgct gaaactgccc tggacccagg tgggattctc cctgttgttc    300
   ctctacttgg gatctggcgg ctggcgcttc atccgggtct tcatcaagac catcaggcgc    360
   gatatctttg gcggcctggt cctcctgaag gtgaaggcaa aggtgcgaca gtgcctgcag    420
   gagcggcgga cagtgcccat tttgtttgcc tctaccgttc ggcgccaccc cgacaagacg    480
   gccctgatct tcgagggcac agatacccac tggaccttcc gccagctgga tgagtactca    540
   agcagtgtag ccaacttcct gcaggcccgg ggcctggcct cgggcgatgt ggctgccatc    600
   ttcatggaga accgcaatga gttcgtgggc ctatggctgg gcatggccaa gctcggtgtg    660
   gaggcagccc tcatcaacac caacctgcgg cgggatgctc tgctccactg cctcaccacc    720
   tcgcgcgcac gggcccttgt ctttggcagc gaaatggcct cagccatctg tgaggtccat    780
   gccagcctgg acccctcgct cagcctcttc tgctctggct cctgggagcc cggtgcggtg    840
   cctccaagca cagaacacct ggaccctctg ctgaaagatg ctcccaagca ccttcccagt    900
   tgccctgaca agggcttcac agataaactg ttctacatct acacatccgg caccacaggg    960
   ctgcccaagg ccgccatcgt ggtgcacagc aggtattacc gcatggctgc cctggtgtac   1020
   tatggattcc gcatgcggcc caacgacatc gtctatgact gcctccccct ctaccactca   1080
   gcaggaaaca tcgtgggaat cggccagtgc ctgctgcatg gcatgacggt ggtgattcgg   1140
   aagaagttct cagcctcccg gttctgggac gattgtatca agtacaactg cacgattgtg   1200
   cagtacattg gtgaactgtg ccgctacctc ctgaaccagc caccgcggga ggcagaaaac   1260
   cagcaccagg ttcgcatggc actaggcaat ggcctccggc agtccatctg gaccaacttt   1320
   tccagccgct tccacatacc ccaggtggct gagttctacg gggccacaga gtgcaactgt   1380
   agcctgggca acttcgacag ccaggtgggg gcctgtggtt tcaatagccg catcctgtcc   1440
   ttcgtgtacc ccatccggtt ggtacgtgtc aacgaggaca ccatggagct gatccggggg   1500
   cccgacggcg tctgcattcc ctgccagcca ggtgagccgg gccagctggt gggccgcatc   1560
   atccagaaag accccctgcg ccgcttcgat ggctacctca accagggcgc caacaacaag   1620
   aagattgcca aggatgtctt caagaagggg gaccaggcct accttactgg tgatgtgctg   1680
   gtgatggacg agctgggcta cctgtacttc cgagaccgca ctggggacac gttccgctgg   1740
   aaaggtgaga acgtgtccac caccgaggtg gaaggcacac tcagccgcct gctggacatg   1800
   gctgacgtgg ccgtgtatgg tgtcgaggtg ccaggaaccg agggccgggc cggaatggct   1860
   gctgtggcca gccccactgg caactgtgac ctggagcgct ttgctcaggt cttggagaag   1920
   gaactgcccc tgtatgcgcg ccccatcttc ctgcgcctcc tgcctgagct gcacaaaaca   1980
   ggaacctaca agttccagaa gacagagcta cggaaggagg gctttgaccc ggctattgtg   2040
   aaagacccgc tgttctatct agatgcccag aagggccgct acgtcccgct ggaccaagag   2100
   gcctacagcc gcatccaggc aggcgaggag aagctgtgat tccccccatc cctctgaggg   2160
   ccggcggatg ctggatccgg agccccaggt tccgccccag agcggtcctg gacaaggcca   2220
   gaccaaagca agcagggcct ggcacctcca tcctgaggtg ctgcccctcc atccaaaact   2280
   gccaagtgac tcattgcctt cccaaccctt ccagaggctt tctgtgaaag tctcatgtcc   2340
   aagttccgtc ttctgggctg ggcaggccct ctggttccca ggctgagact gacgggtttt   2400
   ctcaggatga tgtcttgggt gagggtaggg agaggacaag gggtcaccga gcccttccca   2460
   gagagcaggg agcttataaa tggaaccaga gcagaagtcc ccagactcag gaagtcaaca   2520
   gagtgggcag ggacagtggt agcatccatc tggtggccaa agagaatcgt agccccagag   2580
   ctgcccaagt tcactgggct ccacccccac ctccaggagg ggaggagagg acctgacatc   2640
   tgtaggtggc ccctgatgcc ccatctacag caggaggtca ggaccacgcc cctggcctct   2700
   ccccactccc ccatcctcct ccctgggtgg ctgcctgatt atccctcagg cagggcctct   2760
   cagtccttgt gggtctgtgt cacctccatc tcagtcttgg cctggctatg aggggaggag   2820
   gaatgggaga gggggctcag gggccaataa actctgcctt gagtcctcct aaaaaaaaaa   2880
   aaaaaaaaaa aaaaaaaaaa aaaaaaa                                        2907
<210> 53
   <211> 643
   <212> PRT
   <213> Homo sapiens
<400> 53
<210> 54
   <211> 1248
   <212> DNA
   <213> Homo sapiens
<220>
   <221> mise_feature
   <222> (1)...(1248)
   <223> n = A,T,C or G
<400> 54
   gtcgttggga tcctcggctg cttagatctc ggagccacct gtgttctggc ccccaagttc      60
   tctacttcct gcttctggga tgactgtcgg cagcatggcg tgacagtgat cctgtatgtg     120
   ggcgagctcc tgcgatactt gtgtaacatt ccccagcaac cagaggaccg gacacataca     180
   gtccgcctgg caatgggcaa tggactacgg gctgatgtgt ggggagacct tccagcagcg     240
   tttcggtcct atttcggatc tngggaagtc ttacgggctt ccacagaagg gcaacatggg     300
   gctttagttc aaatattgtt gggggcgctg cggggccctg ggggcaaaga tggagcttgc      360
   ctcctccgaa tgctgtcccc ctttgagctg gtgcagttcg acatggaggc ggcggagcct     420
   gtgagggaca atcagggctt ctgcatccct gtagggctag gggagccggg gctgctgttg     480
   accaaggtgg taagccagca acccttcgtg ggctaccgcg gcccccgaga gctgtcggaa     540
   cggaagctgg tgcgcaacgt gcggcaatcg ggcgacgttt actacaacac cggggacgta     600
   ctggccatgg accgcgaagg cttcctctac ttccgcgacc gactcgggga caccttccga     660
   tggaagggcg agaacgtgtc cacgcacgag gtggagggcg tgttgtcgca ggtggacttc     720
   ttgcaacagg ttaacgtgta tggcgtgtgc gtgccaggtt gtgagggtaa ggtgggcatg     780
   gctgctgtgg cattagcccc cggccagact ttcgacgggg agaagttgta ccagcacgtt     840
   cgcgcttggc tccctgccta cgctaccccc catttcatcc gcatccagga cgccatggag     900
   gtcaccagca cgttcaaact gatgaagacc cggttggtgc gtgagggctt caatgtgggg     960
   atcgtggttg accctctgtt tgtactggac aaccgggccc agtccttccg gcccctgacg    1020
   gcagaaatgt accaggctgt gtgtgaggga acctggaggc tctgatcacc tggccaaccc    1080
   actggggtag ggatcaaagc cagccacccc caccccaaca cactcggtgt ccctttcatc    1140
   ctgggcctgt gtgaatccca gcctggccat accctcaacc tcagtgggct ggaaatgaca    1200
   gtgggccctg tagcagtggc agaataaact cagmtgygtt cacagaaa                  1248
<210> 55
   <211> 354
   <212> PRT
   <213> Homo sapiens
<220>
   <221> VARIANT
   <222> (1)...(354)
   <223> Xaa = Any Amino Acid
<400> 55
   Val Val Gly Ile Leu Gly Cys Leu Asp Leu Gly Ala Thr Cys Val Leu
    1               5                  10                  15
   Ala Pro Lys Phe Ser Thr Ser Cys Phe Trp Asp Asp Cys Arg Gln His
               20                  25                  30
   Gly Val Thr Val Ile Leu Tyr Val Gly Glu Leu Leu Arg Tyr Leu Cys
           35                  40                  45
   Asn Ile Pro Gln Gln Pro Glu Asp Arg Thr His Thr Val Arg Leu Ala
       50                  55                  60
   Met Gly Asn Gly Leu Arg Ala Asp Val Trp Gly Asp Leu Pro Ala Ala
   65                  70                  75                  80
   Phe Arg Ser Tyr Phe Gly Ser Xaa Glu Val Leu Arg Ala Ser Thr Glu
                   85                  90                  95
   Gly Gln His Gly Ala Leu Val Gln Ile Leu Leu Gly Ala Leu Arg Gly
               100                 105                 110
   Pro Gly Gly Lys Asp Gly Ala Cys Leu Leu Arg Met Leu Ser Pro Phe
           115                 120                 125
   Glu Leu Val Gln Phe Asp Met Glu Ala Ala Glu Pro Val Arg Asp Asn
       130                 135                 140
   Gln Gly Phe Cys Ile Pro Val Gly Leu Gly Glu Pro Gly Leu Leu Leu
   145 150 155 160
   Thr Lys Val Val Ser Gln Gln Pro Phe Val Gly Tyr Arg Gly Pro Arg
   165 170 175
   Glu Leu Ser Glu Arg Lys Leu Val Arg Asn Val Arg Gln Ser Gly Asp
   180 185 190
   Val Tyr Tyr Asn Thr Gly Asp Val Leu Ala Met Asp Arg Glu Gly Phe
   195 200 205
   Leu Tyr Phe Arg Asp Arg Leu Gly Asp Thr Phe Arg Trp Lys Gly Glu
   210 215 220
   Asn Val Ser Thr His Glu Val Glu Gly Val Leu Ser Gln Val Asp Phe
   225 230 235 240
   Leu Gln Gln Val Asn Val Tyr Gly Val Cys Val Pro Gly Cys Glu Gly
   245 250 255
   Lys Val Gly Met Ala Ala Val Ala Leu Ala Pro Gly Gln Thr Phe Asp
   260 265 270
   Gly Glu Lys Leu Tyr Gln His Val Arg Ala Trp Leu Pro Ala Tyr Ala
   275 280 285
   Thr Pro His Phe Ile Arg Ile Gln Asp Ala Met Glu Val Thr Ser Thr
   290 295 300
   Phe Lys Leu Met Lys Thr Arg Leu Val Arg Glu Gly Phe Asn Val Gly
   305 310 315 320
   Ile Val Val Asp Pro Leu Phe Val Leu Asp Asn Arg Ala Gln Ser Phe
   325 330 335
   Arg Pro Leu Thr Ala Glu Met Tyr Gln Ala Val Cys Glu Gly Thr Trp
   340 345 350
   Arg Leu
<210> 56
   <211> 2885
   <212> DNA
   <213> Homo sapiens
<400> 56
   aacggcaagt aagcgcaacg caattaatgt gagtagctca ctcattaggc accccaggct      60
   ttacacttta tgcttccggg ctcgtatgtt gtgtggaatt gtgagcggat accaatttca     120
   cacaggaacc agctatgaca tgattacgaa tttaatacga ctcactatag ggaatttggc     180
   cctcgaggcc aagaattcgg cacgaggggt gctgagcccc tgcgcggttt ctggtgcgta     240
   gagactgtaa atcgctgcgc ttctcagtca tcatcatccc agcttttccc ggctcgaatt     300
   cagcctccaa ctcaagctcg cgggaaagac tacctgagag gagaaaagct tctgtccctg     360
   gaccttcttc tgagggtgga gtcggaggct ccctgctttc cagccgccca gtgacccaag     420
   cttaatcttc agcaccactt ggggcgacct tttcggtgca aacctacgat tctgtttctc     480
   aggattcctc cccatcccgc ttcgccccgg aaaagctgac aagaacttca ggtgtaagcc     540
   ctgagtagtg aggatctgcg gtctccgtgg agagctgtgc ctggaagaga aggacgctgg     600
   tgggggctga gatcagagct gtcttctggc ccagttgccc ccatgcttct gtcatggcta     660
   acagttctag gggctggaat ggtcgtcctg cacttcttgc agaaactcct gttcccttac     720
   ttttgggatg acttctggtt cgtgttgaag gtggtgctca ttataattcg gctgaagaag     780
   tatgaaaaga gaggggagct ggtgactgtg ctggataaat tcttgagtca tgccaaaaga     840
   caacctcgga aacctttcat catctatgag ggagacatct acacctatca ggatgtagac     900
   aaaaggagca gcagagtggc ccatgtcttc ctgaaccatt cctctctgaa aaagggggac    960
   acggtggctc tgctgatgag caatgagccg gacttcgttc acgtgtggtt cggcctcgcc    1020
   aagctgggct gcgtggtggc ctttctcaac accaacattc gctccaactc cctcctgaat    1080
   tgcatccgcg cctgtgggcc cagagcccta gtggtgggcg cagatttgct tggaacggta    1140
   gaagaaatcc ttccaagcct ctcagaaaat atcagtgttt gggggatgaa agattctgtt    1200
   ccacaaggtg taatttcact caaagaaaaa ctgagcacct cacctgatga gcccgtgcca    1260
   cgcagccacc atgttgtctc actcctcaag tctacttgtc tttacatttt tacctctgga    1320
   acaacaggtc taccaaaagc agctgtgatt agtcagctgc aggttttaag gggttctgct    1380
   gtcctgtggg cttttggttg tactgctcat gacattgttt atataaccct tcctctgtat    1440
   catagttcag cagctatcct gggaatttct ggatgtgttg agttgggtgc cacttgtgtg    1500
   ttaaagaaga aattttcagc aagccagttt tggagtgact gcaagaagta tgatgtgact    1560
   gtgtttcagt atattggaga actttgtcgc tacctttgca aacaatctaa gagagaagga    1620
   gaaaaggatc ataaggtgcg tttggcaatt ggaaatggca tacggagtga tgtatggaga    1680
   gaatttttag acagatttgg aaatataaag gtgtgtgaac tttatgcagc taccgaatca    1740
   agcatatctt tcatgaacta cactgggaga attggagcaa ttgggagaac aaatttgttt    1800
   tacaaacttc tttccacttt tgacttaata aagtatgact ttcagaaaga tgaacccatg    1860
   agaaatgagc agggttggtg tattcatgtg aaaaaaggag aacctggact tctcatttct    1920
   cgagtgaatg caaaaaatcc cttctttggc tatgctgggc cttataagca cacaaaagac    1980
   aaattgcttt gtgatgtttt taagaaggga gatgtttacc ttaatactgg agacttaata    2040
   gtccaggatc aggacaattt cctttatttt tgggaccgta ctggagacac tttcagatgg    2100
   aaaggagaaa atgtcgcaac cactgaggtt gctgatgtta ttggaatgtt ggatttcata    2160
   caggaagcaa acgtctatgg tgtggctata tcaggttatg aaggaagagc aggaatggct    2220
   tctattattt taaaaccaaa tacatcttta gatttggaaa aagtttatga acaagttgta    2280
   acatttctac cagcttatgc ttgtccacga tttttaagaa ttcaggaaaa aatggaagca    2340
   acaggaacat tcaaactatt gaagcatcag ttggtggaag atggatttaa tccactgaaa    2400
   atttctgaac cactttactt catggataac ttgaaaaagt cttatgttct actgaccagg    2460
   gaactttatg atcaaataat gttaggggaa ataaaacttt aagattttta tatctagaac    2520
   tttcatatgc tttcttagga agagtgagag gggggtatat gattctttat gaaatgggga    2580
   aagggagcta acattaatta tgcatgtact atatttcctt aatatgagag ataatttttt    2640
   aattgcataa gaattttaat ttcttttaat tgatataaac attagttgat tattcttttt    2700
   atctatttgg agattcagtg cataactaag tattttcctt aatactaaag attttaaata    2760
   ataaatagtg gctagcggtt tggacaatca ctaaaaatgt actttctaat aagtaaaatt    2820
   tctaattttg aataaaagat taaattttac tgaaaaaaaa aaaaaaaaaa aaaattggcg    2880
   gccgc                                                                 2885
<210> 57
   <211> 619
   <212> PRT
   <213> Homo sapiens
<400> 57
   Met Leu Leu Ser Trp Leu Thr Val Leu Gly Ala Gly Met Val Val Leu
    1               5                  10                  15
   His Phe Leu Gln Lys Leu Leu Phe Pro Tyr Phe Trp Asp Asp Phe Trp
               20                  25                  30
   Phe Val Leu Lys Val Val Leu Ile Ile Ile Arg Leu Lys Lys Tyr Glu
           35                  40                  45
   Lys Arg Gly Glu Leu Val Thr Val Leu Asp Lys Phe Leu Ser His Ala
       50                  55                  60
   Lys Arg Gln Pro Arg Lys Pro Phe Ile Ile Tyr Glu Gly Asp Ile Tyr
   65                  70                  75                  80
   Thr Tyr Gln Asp Val Asp Lys Arg Ser Ser Arg Val Ala His Val Phe
                   85                  90                  95
   Leu Asn His Ser Ser Leu Lys Lys Gly Asp Thr Val Ala Leu Leu Met
               100                 105                 110
   Ser Asn Glu Pro Asp Phe Val His Val Trp Phe Gly Leu Ala Lys Leu
           115                 120                 125
   Gly Cys Val Val Ala Phe Leu Asn Thr Asn Ile Arg Ser Asn Ser Leu
       130                 135                 140
   Leu Asn Cys Ile Arg Ala Cys Gly Pro Arg Ala Leu Val Val Gly Ala
   145                 150                 155                 160
   Asp Leu Leu Gly Thr Val Glu Glu Ile Leu Pro Ser Leu Ser Glu Asn
                   165                 170                 175
   Ile Ser Val Trp Gly Met Lys Asp Ser Val Pro Gln Gly Val Ile Ser
               180                 185                 190
   Leu Lys Glu Lys Leu Ser Thr Ser Pro Asp Glu Pro Val Pro Arg Ser
           195                 200                 205
   His His Val Val Ser Leu Leu Lys Ser Thr Cys Leu Tyr Ile Phe Thr
       210                 215                 220
   Ser Gly Thr Thr Gly Leu Pro Lys Ala Ala Val Ile Ser Gln Leu Gln
   225                 230                 235                 240
   Val Leu Arg Gly Ser Ala Val Leu Trp Ala Phe Gly Cys Thr Ala His
                   245                 250                 255
   Asp Ile Val Tyr Ile Thr Leu Pro Leu Tyr His Ser Ser Ala Ala Ile
               260                 265                 270
   Leu Gly Ile Ser Gly Cys Val Glu Leu Gly Ala Thr Cys Val Leu Lys
           275                 280                 285
   Lys Lys Phe Ser Ala Ser Gln Phe Trp Ser Asp Cys Lys Lys Tyr Asp
       290                 295                 300
   Val Thr Val Phe Gln Tyr Ile Gly Glu Leu Cys Arg Tyr Leu Cys Lys
   305                 310                 315                 320
   Gln Ser Lys Arg Glu Gly Glu Lys Asp His Lys Val Arg Leu Ala Ile
                   325                 330                 335
   Gly Asn Gly Ile Arg Ser Asp Val Trp Arg Glu Phe Leu Asp Arg Phe
               340                 345                 350
   Gly Asn Ile Lys Val Cys Glu Leu Tyr Ala Ala Thr Glu Ser Ser Ile
           355                 360                 365
   Ser Phe Met Asn Tyr Thr Gly Arg Ile Gly Ala Ile Gly Arg Thr Asn
       370                 375                 380
   Leu Phe Tyr Lys Leu Leu Ser Thr Phe Asp Leu Ile Lys Tyr Asp Phe
   385                 390                 395                 400
   Gln Lys Asp Glu Pro Met Arg Asn Glu Gln Gly Trp Cys Ile His Val
                   405                 410                 415
   Lys Lys Gly Glu Pro Gly Leu Leu Ile Ser Arg Val Asn Ala Lys Asn
               420                 425                 430
   Pro Phe Phe Gly Tyr Ala Gly Pro Tyr Lys His Thr Lys Asp Lys Leu
           435                 440                 445
   Leu Cys Asp Val Phe Lys Lys Gly Asp Val Tyr Leu Asn Thr Gly Asp
       450                 455                 460
   Leu Ile Val Gln Asp Gln Asp Asn Phe Leu Tyr Phe Trp Asp Arg Thr
   465                 470                 475                 480
   Gly Asp Thr Phe Arg Trp Lys Gly Glu Asn Val Ala Thr Thr Glu Val
                   485                 490                 495
   Ala Asp Val Ile Gly Met Leu Asp Phe Ile Gln Glu Ala Asn Val Tyr
               500                 505                 510
   Gly Val Ala Ile Ser Gly Tyr Glu Gly Arg Ala Gly Met Ala Ser Ile
           515                 520                 525
   Ile Leu Lys Pro Asn Thr Ser Leu Asp Leu Glu Lys Val Tyr Glu Gln
       530                 535                 540
   Val Val Thr Phe Leu Pro Ala Tyr Ala Cys Pro Arg Phe Leu Arg Ile
   545                 550                 555                 560
   Gln Glu Lys Met Glu Ala Thr Gly Thr Phe Lys Leu Leu Lys His Gln
                   565                 570                 575
   Leu Val Glu Asp Gly Phe Asn Pro Leu Lys Ile Ser Glu Pro Leu Tyr
               580                 585                 590
   Phe Met Asp Asn Leu Lys Lys Ser Tyr Val Leu Leu Thr Arg Glu Leu
           595                 600                 605
   Tyr Asp Gln Ile Met Leu Gly Glu Ile Lys Leu
       610                 615
<210> 58
   <211> 3098
   <212> DNA
   <213> Rattus norvegicus
<400> 58
   aagttcccac tccagacttc tgcgagaacc cgtgaggaag cagcgagaac cgggggtttg     60
   caagccagag aaggatgcgg actccgggag caggaacagc ctctgtggcc tcattggggc    120
   tgctttggct tctgggactt ccgtggacct ggagcgcggc ggcggcgttc ggtgtgtacg    180
   tgggtagcgg tggctggcga tttctgcgta tcgtctgcaa gacggcgagg cgagacctct    240
   ttggcctctc tgttctgatc cgcgtgcggc tagagctacg acgacaccgg cgagcaggag    300
   acacgatccc acgcatcttc caggccgtgg cccagcgaca gccggagcgc ctggcgctgg    360
   tagatgcgag tagcggtatc tgctggacct tcgcacagct agacacctac tccaatgctg    420
   tggccaatct gttcctccag ctgggctttg cgccaggcga tgtggtggct gtgttcctgg    480
   aaggccggcc cgagttcgtg ggactgtggc tgggcctggc caaggccggt gtagtggctg    540
   cgcttctcaa tgtcaacctg aggcgggagc cccttgcctt ctgcttgggc acatcagctg    600
   ccaaggccct catttatggc ggggagatgg cagcggcggt ggcggaggtg agtgagcagc    660
   tggggaagag cctgctcaag ttctgctctg gagatctggg gcctgagagc gtcctgcctg    720
   acacgcagct tctggacccc atgcttgctg aggcgcccac cacacccctg gcacaggccc    780
   caggcaaggg catggatgat cggctatttt acatctatac ttctgggacc accggacttc    840
   ctaaggcggc cattgtggtg cacagcaggt actaccgcat cgcagccttc ggccaccatt    900
   cctacagcat gcgggccaac gatgtgctct atgactgcct acctctctac cactcagcag    960
   ggaacatcat gggcgtggga cagtgtatca tctacgggtt aacggtggta ctgcgcaaga   1020
   agttctccgc cagccgcttc tgggacgact gtgtcaaata taattgcacg gtagtgcagt   1080
   acatcggtga aatatgccgc tacctgctaa ggcagccggt tcgcgatgta gagcggcggc   1140
   accgcgtgcg cctggccgtg ggtaacggac tgcggccagc catctgggag gagttcacgc   1200
   agggtttcgg tgtgcgacag attggcgagt tctacggcgc caccgaatgc aactgcagca   1260
   ttgccaacat ggacggcaag gtcggctcct gcggcttcaa cagccgtatc ctcacgcatg   1320
   tgtaccccat ccgtctggtc aaggtcaacg aggacacgat ggagccactg agggactccc   1380
   aaggcctctg catcccgtgc cagcccgggg aacctgggct tctcgtgggc cagatcaacc   1440
   agcaagaccc tctgcggcgc ttcgatggct atgttagtga cagcgccacc aacaagaaga   1500
   ttgcccacag cgtgttccga aagggggaca gcgcctacct ttcaggtgac gtgctagtga    1560
   tggacgagct ggggtacatg tacttccgtg accgcagcgg ggataccttc cgatggcgcg    1620
   gcgagaacgt atccaccacg gaggtggaag ccgtgctgag ccgcctgttg ggccagacgg   1680
   acgtggctgt gtatggagtg gctgtgccag gagtggaggg gaaaagcggc atggcggcca   1740
   ttgcagaccc ccacaaccag ctggacccta actcaatgta ccaggaattg cagaaggttc   1800
   ttgcatccta tgcccagccc atcttcctgc gtcttctgcc ccaagtggat acaacaggca   1860
   ccttcaagat ccagaagacc cgactacagc gtgaaggctt tgacccccgc cagacctcag   1920
   accggctctt ctttctagac ctgaaacagg gacgctacct acccctggat gagagagtcc   1980
   atgcccgcat ctgcgcaggc gacttctcac tctgagcctg gtgagtggga tggccctgga   2040
   cttgtgagac cagggagccg gacacccctg ttcaggtgtt tctcctgcct ggccacgtgg   2100
   ccagcagcac ctgtgggtgc aggaaactgg aacctgagtg gccgggtgtc cctttcctac   2160
   aacccaccat gcacacatct agcctctgcc ttggtctttt tctccatctc tttcctccgt   2220
   gcccagcagg agccccacag acacattggc tgctgtgtcc tgcagtggga ccggtgtcta   2280
   ggggtccatg ctgcaggctg tgacccgcac tggtgcccac ctcccttccc cattgtgcct   2340
   taggttcctc cactgtgcgc cggtgaagca agtggggacc cacatagctg ttgtccctgc   2400
   tgagggttgg tagcaaatgc accctcatgt cagctgggag acacatgcag tctcccactg   2460
   acccccaatc aactgaagat actgttttgt attattgttt tgagataggg tctcactgtg   2520
   gaggccaagc tggcctcagg ctcaccactc tactgcctcc gggcaccagc ctgcagtttg   2580
   atgacatgta tgcactattg ttctaagggt cttctgagtc cctgctttcc cctcatgtcc   2640
   taaaaccttc cagaactgac tctgatcact tggatgtagc tagtgttggc cctgcccacg   2700
   tgtgtcaatt caggggtccc caggcatcat ctctggaggc cctaaccttg gcaaagcttg   2760
   gatgtcctca catcacagca ggagacccag gaaggttgct gtggtgtctc ttgggcaccc   2820
   ctggcggcag ccgtggacat gcttccctgc tgtgatagcc caaactgttg cctatgacat   2880
   ttgaggtcta cccttctggc tgccatggtc cccattgaga tctttggtga ctcacctcag   2940
   ccaccaagcc aggcctctgc cttccttcag ctctaagggc atgaagggtg tggacagagc   3000
   agccacaggc tgcccacagt cacccacatg caagtgttat ttccttgttt gttttaaaaa   3060
   aataaacatg ctgagccttg aaaaaaaaaa aaaaaaaa 3098
<210> 59
   <211> 646
   <212> PRT
   <213> Rattus norvegicus
<400> 59
   Met Arg Thr Pro Gly Ala Gly Thr Ala Ser Val Ala Ser Leu Gly Leu
    1               5                  10                  15
   Leu Trp Leu Leu Gly Leu Pro Trp Thr Trp Ser Ala Ala Ala Ala Phe
               20                  25                  30
   Gly Val Tyr Val Gly Ser Gly Gly Trp Arg Phe Leu Arg Ile Val Cys
           35                  40                  45
   Lys Thr Ala Arg Arg Asp Leu Phe Gly Leu Ser Val Leu Ile Arg Val
       50                  55                  60
   Arg Leu Glu Leu Arg Arg His Arg Arg Ala Gly Asp Thr Ile Pro Arg
   65                  70                  75                  80
   Ile Phe Gln Ala Val Ala Gln Arg Gln Pro Glu Arg Leu Ala Leu Val
                   85                  90                  95
   Asp Ala Ser Ser Gly Ile Cys Trp Thr Phe Ala Gln Leu Asp Thr Tyr
               100                 105                 110
   Ser Asn Ala Val Ala Asn Leu Phe Leu Gln Leu Gly Phe Ala Pro Gly
           115                 120                 125
   Asp Val Val Ala Val Phe Leu Glu Gly Arg Pro Glu Phe Val Gly Leu
       130                 135                 140
   Trp Leu Gly Leu Ala Lys Ala Gly Val Val Ala Ala Leu Leu Asn Val
   145                 150                 155                 160
   Asn Leu Arg Arg Glu Pro Leu Ala Phe Cys Leu Gly Thr Ser Ala Ala
                   165                 170                 175
   Lys Ala Leu Ile Tyr Gly Gly Glu Met Ala Ala Ala Val Ala Glu Val
               180                 185                 190
   Ser Glu Gln Leu Gly Lys Ser Leu Leu Lys Phe Cys Ser Gly Asp Leu
           195                 200                 205
   Gly Pro Glu Ser Val Leu Pro Asp Thr Gln Leu Leu Asp Pro Met Leu
       210                 215                 220
   Ala Glu Ala Pro Thr Thr Pro Leu Ala Gln Ala Pro Gly Lys Gly Met
   225                 230                 235                 240
   Asp Asp Arg Leu Phe Tyr Ile Tyr Thr Ser Gly Thr Thr Gly Leu Pro
                   245                 250                 255
   Lys Ala Ala Ile Val Val His Ser Arg Tyr Tyr Arg Ile Ala Ala Phe
               260                 265                 270
   Gly His His Ser Tyr Ser Met Arg Ala Asn Asp Val Leu Tyr Asp Cys
           275                 280                 285
   Leu Pro Leu Tyr His Ser Ala Gly Asn Ile Met Gly Val Gly Gln Cys
       290                 295                 300
   Ile Ile Tyr Gly Leu Thr Val Val Leu Arg Lys Lys Phe Ser Ala Ser
   305                 310                 315                 320
   Arg Phe Trp Asp Asp Cys Val Lys Tyr Asn Cys Thr Val Val Gln Tyr
                   325                 330                 335
   Ile Gly Glu Ile Cys Arg Tyr Leu Leu Arg Gln Pro Val Arg Asp Val
               340                 345                 350
   Glu Arg Arg His Arg Val Arg Leu Ala Val Gly Asn Gly Leu Arg Pro
           355                 360                 365
   Ala Ile Trp Glu Glu Phe Thr Gln Gly Phe Gly Val Arg Gln Ile Gly
       370                 375                 380
   Glu Phe Tyr Gly Ala Thr Glu Cys Asn Cys Ser Ile Ala Asn Met Asp
   385                 390                 395                 400
   Gly Lys Val Gly Ser Cys Gly Phe Asn Ser Arg Ile Leu Thr His Val
                   405                 410                 415
   Tyr Pro Ile Arg Leu Val Lys Val Asn Glu Asp Thr Met Glu Pro Leu
               420                 425                 430
   Arg Asp Ser Gln Gly Leu Cys Ile Pro Cys Gln Pro Gly Glu Pro Gly
           435                 440                 445
   Leu Leu Val Gly Gln Ile Asn Gln Gln Asp Pro Leu Arg Arg Phe Asp
       450                 455                 460
   Gly Tyr Val Ser Asp Ser Ala Thr Asn Lys Lys Ile Ala His Ser Val
   465                 470                 475                 480
   Phe Arg Lys Gly Asp Ser Ala Tyr Leu Ser Gly Asp Val Leu Val Met
                   485                 490                 495
   Asp Glu Leu Gly Tyr Met Tyr Phe Arg Asp Arg Ser Gly Asp Thr Phe
               500                 505                 510
   Arg Trp Arg Gly Glu Asn Val Ser Thr Thr Glu Val Glu Ala Val Leu
           515                 520                 525
   Ser Arg Leu Leu Gly Gln Thr Asp Val Ala Val Tyr Gly Val Ala Val
       530                 535                 540
   Pro Gly Val Glu Gly Lys Ser Gly Met Ala Ala Ile Ala Asp Pro His
   545                 550                 555                 560
   Asn Gln Leu Asp Pro Asn Ser Met Tyr Gln Glu Leu Gln Lys Val Leu
                   565                 570                 575
   Ala Ser Tyr Ala Gln Pro Ile Phe Leu Arg Leu Leu Pro Gln Val Asp
               580                 585                 590
   Thr Thr Gly Thr Phe Lys Ile Gln Lys Thr Arg Leu Gln Arg Glu Gly
           595                 600                 605
   Phe Asp Pro Arg Gln Thr Ser Asp Arg Leu Phe Phe Leu Asp Leu Lys
       610                 615                 620
   Gln Gly Arg Tyr Leu Pro Leu Asp Glu Arg Val His Ala Arg Ile Cys
   625                 630                 635                 640
   Ala Gly Asp Phe Ser Leu
                   645
<210> 60
   <211> 2963
   <212> DNA
   <213> Rattus norvegicus
<400> 60
   gacacagtac tgccgatgtt ggacagagga tcgcttaaca gaacgaaatc tcaaaacaaa      60
   ttaacaggac ccggttgctt gatttcccaa atcagaaaag gctcgaaatg tctagagggg     120
   ctgactgatg cagcggtgac ccggactgga gacagttgga cgcgatcatc tctggtgctt     180
   ttgttcaacc ttgaaacctt cgccacagga gacttgcctg agcagagaag caaacgtgga     240
   gaaacaaaga gagatctagc gaaaagcctc tgggaccaag gaggggaggt gggactctgg     300
   gttggcggtg gcacctgctg ccggctatta ataatagggt cgcgatgcgt ttataaggtg     360
   tttgattaaa caaagactct atgagagaag aataactagc aacagcccca cgtctgagtc     420
   gtcgcctccg acctttttca acgtgggttc tttgggccga gcgtcgtttg ccgagaacta     480
   gatctcacct gaccccagac gctgaaaaca agcgctgtgg catcctgggc cacccaagct     540
   gacaagggcg cgccccctga gcacacgagg tgccccacga gggggaggga cccacagccg     600
   tcccgcccgc accgcggtgt ccgctgcggg cacctgcagc cgagccgcca cccgcagtcg     660
   cagcgcgtcc ggcggccgaa cccggtcgtc agctcgtcag cacctgctct gcttctctcc     720
   cgcccgccgc cgcgctgcac gcctcgagcg ctccctcggc cccggcgggg accggggacc     780
   ccgcagccac cgccatgctg cctgtgctct acaccggcct ggcggggctg ctgctgctgc     840
   ctctgctgct cacctgctgc tgcccctacc tcctccagga cgtgcggttc ttcctgcaac     900
   tggccaacat ggcccggcag gtgcgcagct accggcagcg gcgacccgtg cgcaccatcc     960
   tgcatgtctt cttggagcaa gcgcgcaaga ccccgcacaa gcccttcctg ctgtttcgcg    1020
   acgagacgct tacctacgcc caggtagacc ggcgcagcaa ccaagtagcg cgagcgctgc    1080
   atgatcacct gggcctgcgg cagggggatt gcgtggccct cttcatgggc aatgagccgg   1140
   cctacgtgtg gctctggctg ggactgctca aactgggctg tcccatggcg tgcctcaact    1200
   acaacatccg tgccaagtct ctgctacact gctttcagtg ctgcggggcg aaggtgctgc    1260
   tggcctcccc agagctacac gaagctgtcg aggaggttct tccaaccctg aaaaaggagg    1320
   gcgtgtccgt cttctacgta agcagaactt ctaacactaa tggcgtggac acagtactgg    1380
   acaaagtaga cggggtgtcg gcggacccca tcccggagtc gtggaggtct gaagtcacgt    1440
   tcaccacacc cgcagtctac atatatactt cgggcaccac aggtcttcca aaggctgcaa    1500
   ccattaatca ccatcgcctc tggtatggga ccagccttgc cctgaggtcc ggaattaagg    1560
   ctcatgacgt catctacacc accatgcccc tgtaccacag cgcggcgctc atgattggcc    1620
   tccacggatg cattgtggtt ggggctacat ttgctttgcg gagcaaattt tcagccagcc    1680
   agttttggga cgactgcagg aaatacaacg ccactgtcat tcagtacatc ggtgaactgc    1740
   ttcggtacct ctgcaacacg ccccagaaac caaatgaccg ggaccacaaa gtgaaaatag    1800
   cactaggaaa tggcttacga ggagatgtgt ggagagagtt catcaagaga tttggggaca    1860
   ttcacattta tgagttctac gcttccactg aaggcaacat tggatttatg aactatccaa    1920
   gaaaaatcgg agctgttgga agagaaaatt acctacaaaa aaaagttgta aggcacgagc    1980
   tgatcaagta tgacgtggag aaggatgagc ctgtccgtga tgcaaatgga tattgcatca    2040
   aagtccccaa aggagaggtt ggactcttga tttgcaaaat cacagagctc acaccatttt    2100
   ttggctatgc tggaggaaag acccagacag agaagaaaaa gctcagagat gtttttaaga    2160
   aaggagacgt ctacttcaac agtggcgatc tcctgatgat cgaccgtgaa aatttcatct    2220
   attttcacga cagagttgga gacaccttcc ggtggaaagg agagaatgta gctaccacgg    2280
   aagtcgctga cattgtggga ctggtagatt ttgttgaaga agtgaatgtt tacggtgtgc    2340
   ccgtgccagg tcatgaaggt cgcatcggga tggcctcgat caagatgaaa gaaaactacg    2400
   agttcaatgg aaagaaactc tttcagcaca tctcggagta cctgcccagt tactcgaggc    2460
   ctcggttcct gagaatacaa gataccattg agatcaccgg gacttttaaa caccgcaaag    2520
   tgaccctgat ggaagagggc tttaacccct cagtcatcaa agataccttg tatttcatgg    2580
   atgacacaga aaaaacatac gtgcccatga ctgaggacat ttataatgcc ataattgata    2640
   agactctgaa gctctgaatg ttgcctggct cctaacactt ccagaaagaa acacaatagg    2700
   cctagcatag ccccttcaca tgtgtaatcc aactttaact tgattaaagg ttataggtgt    2760
   gatttttcct aggaaattat tcatttaaag gacaattgtt tgtttgtttg tttgtttttt    2820
   attaattaca ccagaacgtt tgcaagtaaa aagatttaaa gtcacttatt tttcaatgtg    2880
   cacctgccat ttgtccttgc aaacttagct tcttggagag agggccttat ttttttaaag    2940
   acataataaa ctatgtaaac act                                            2963
<210> 61
   <211> 620
   <212> PRT
   <213> Rattus norvegicus
<400> 61
   Met Leu Pro Val Leu Tyr Thr Gly Leu Ala Gly Leu Leu Leu Leu Pro
   1               5                   10                  15
   Leu Leu Leu Thr Cys Cys Cys Pro Tyr Leu Leu Gln Asp Val Arg Phe
               20                  25                  30
   Phe Leu Gln Leu Ala Asn Met Ala Arg Gln Val Arg Ser Tyr Arg Gln
           35                  40                  45
   Arg Arg Pro Val Arg Thr Ile Leu His Val Phe Leu Glu Gln Ala Arg
       50                  55                  60
   Lys Thr Pro His Lys Pro Phe Leu Leu Phe Arg Asp Glu Thr Leu Thr
   65                  70                  75                  80
   Tyr Ala Gln Val Asp Arg Arg Ser Asn Gln Val Ala Arg Ala Leu His
                   85                  90                  95
   Asp His Leu Gly Leu Arg Gln Gly Asp Cys Val Ala Leu Phe Met Gly
               100                 105                 110
   Asn Glu Pro Ala Tyr Val Trp Leu Trp Leu Gly Leu Leu Lys Leu Gly
           115                 120                 125
   Cys Pro Met Ala Cys Leu Asn Tyr Asn Ile Arg Ala Lys Ser Leu Leu
       130                 135                 140
   His Cys Phe Gln Cys Cys Gly Ala Lys Val Leu Leu Ala Ser Pro Glu
   145                 150                 155                 160
   Leu His Glu Ala Val Glu Glu Val Leu Pro Thr Leu Lys Lys Glu Gly
                   165                 170                 175
   Val Ser Val Phe Tyr Val Ser Arg Thr Ser Asn Thr Asn Gly Val Asp
               180                 185                 190
   Thr Val Leu Asp Lys Val Asp Gly Val Ser Ala Asp Pro Ile Pro Glu
           195                 200                 205
   Ser Trp Arg Ser Glu Val Thr Phe Thr Thr Pro Ala Val Tyr Ile Tyr
       210                 215                 220
   Thr Ser Gly Thr Thr Gly Leu Pro Lys Ala Ala Thr Ile Asn His His
   225                 230                 235                 240
   Arg Leu Trp Tyr Gly Thr Ser Leu Ala Leu Arg Ser Gly Ile Lys Ala
                   245                 250                 255
   His Asp Val Ile Tyr Thr Thr Met Pro Leu Tyr His Ser Ala Ala Leu
               260                 265                 270
   Met Ile Gly Leu His Gly Cys Ile Val Val Gly Ala Thr Phe Ala Leu
           275                 280                 285
   Arg Ser Lys Phe Ser Ala Ser Gln Phe Trp Asp Asp Cys Arg Lys Tyr
       290                 295                 300
   Asn Ala Thr Val Ile Gln Tyr Ile Gly Glu Leu Leu Arg Tyr Leu Cys
   305                 310                 315                 320
   Asn Thr Pro Gln Lys Pro Asn Asp Arg Asp His Lys Val Lys Ile Ala
                   325                 330                 335
   Leu Gly Asn Gly Leu Arg Gly Asp Val Trp Arg Glu Phe Ile Lys Arg
               340                 345                 350
   Phe Gly Asp Ile His Ile Tyr Glu Phe Tyr Ala Ser Thr Glu Gly Asn
           355                 360                 365
   Ile Gly Phe Met Asn Tyr Pro Arg Lys Ile Gly Ala Val Gly Arg Glu
       370                 375                 380
   Asn Tyr Leu Gln Lys Lys Val Val Arg His Glu Leu Ile Lys Tyr Asp
   385                 390                 395                 400
   Val Glu Lys Asp Glu Pro Val Arg Asp Ala Asn Gly Tyr Cys Ile Lys
                   405                 410                 415
   Val Pro Lys Gly Glu Val Gly Leu Leu Ile Cys Lys Ile Thr Glu Leu
               420                 425                 430
   Thr Pro Phe Phe Gly Tyr Ala Gly Gly Lys Thr Gln Thr Glu Lys Lys
           435                 440                 445
   Lys Leu Arg Asp Val Phe Lys Lys Gly Asp Val Tyr Phe Asn Ser Gly
       450                 455                 460
   Asp Leu Leu Met Ile Asp Arg Glu Asn Phe Ile Tyr Phe His Asp Arg
   465                 470                 475                 480
   Val Gly Asp Thr Phe Arg Trp Lys Gly Glu Asn Val Ala Thr Thr Glu
                   485                 490                 495
   Val Ala Asp Ile Val Gly Leu Val Asp Phe Val Glu Glu Val Asn Val
               500                 505                 510
   Tyr Gly Val Pro Val Pro Gly His Glu Gly Arg Ile Gly Met Ala Ser
           515                 520                 525
   Ile Lys Met Lys Glu Asn Tyr Glu Phe Asn Gly Lys Lys Leu Phe Gln
       530                 535                 540
   His Ile Ser Glu Tyr Leu Pro Ser Tyr Ser Arg Pro Arg Phe Leu Arg
   545                 550                 555                 560
   Ile Gln Asp Thr Ile Glu Ile Thr Gly Thr Phe Lys His Arg Lys Val
                   565                 570                 575
   Thr Leu Met Glu Glu Gly Phe Asn Pro Ser Val Ile Lys Asp Thr Leu
               580                 585                 590
   Tyr Phe Met Asp Asp Thr Glu Lys Thr Tyr Val Pro Met Thr Glu Asp
           595                 600                 605
   Ile Tyr Asn Ala Ile Ile Asp Lys Thr Leu Lys Leu
       610                 615                 620
<210> 62
   <211> 1350
   <212> DNA
   <213> Rattus norvegicus
<400> 62
   gatcagctct tctatatcta cacgtcgggc accacggggc tacccaaagc tgccattgtg     60
   gtgcacagca ggtattaccg aatggctgcc ctggtgtact atggattccg catgcggcct    120
   gatgacattg tctatgactg cctccccctc taccactcag caggaaacat tgtggggatt   180
   ggccagtgcg tactccacgg catgactgtg gtgatccgga agaagttttc agcctcccgg    240
   ttctgggatg actgtatcaa gtacaactgc acaattgtac agtacattgg tgagctttgc    300
   cgctacctcc tgaaccagcc accccgtgag gctgagtctc ggcacaaggt gcgcatggca    360
   ctgggcaacg gtctccggca gtccatctgg accgacttct ccagccgttt ccacattccc    420
   aaggtggccg agttctacgg ggccaccgag tgcaactgta gcttgggcaa ctttgacagc    480
   caggtggggg cctgtggctt caatagccgc atcctgtcct ttgtgtaccc catccgcttg    540
   gtacgagtca atgaggatac catggaactg atccggggac ccgatggcgt ctgcattccc    600
   tgtcaaccag gccagccagg ccagctggtg ggtcgcatca tccagcagga ccccctacgc    660
   cgttttgatg gctacctcaa ccagggtgcc aacaacaaga agattgctag tgatgtcttc    720
   aagaaagggg accaagccta cctcactggt gacgtgctgg tgatggatga gctgggctac    780
   ctgtacttcc gagaccgcac aggggacacg ttccgctgga aaggggagaa tgtgtctacc    840
   actgaagtgg agggcacact cagccgcctg cttcagatgg cagatgtggc tgtttatggt    900
   gttgaggtgc caggagctga gggccgagca ggaatggctg ctgtggcaag ccccactagc    960
   aactgtgacc tggagagctt tgcacagacc ttgaaaaagg agctgcccct gtacgcccgc   1020
   cccatcttcc tccgcttctt gcctgagctg cacaaaacag gaaccttcaa gttccagaag   1080
   acagagttgc ggaaggaggg ctttgacccg tctgttgtga aagacccact cttctatttg   1140
   gatgcccgga caggctgcta tgttgcactg gaccaagagg cctatacccg catccaggca   1200
   ggcgaggaga agctgtgatt tcccccacat ccctctgagg gccagaggat gctggattca   1260
   gagccccagc ttccactcca gaaggggtct gggcaaggcc agaccaaagc tagcagggcc   1320
   cgcaccttca ccctaggtgc tgatccccct                                     1350
<210> 63
   <211> 405
   <212> PRT
   <213> Rattus norvegicus
<400> 63
   Asp Gln Leu Phe Tyr Ile Tyr Thr Ser Gly Thr Thr Gly Leu Pro Lys
   1 5 10 15
   Ala Ala Ile Val Val His Ser Arg Tyr Tyr Arg Met Ala Ala Leu Val
   20 25 30
   Tyr Tyr Gly Phe Arg Met Arg Pro Asp Asp Ile Val Tyr Asp Cys Leu
   35 40 45
   Pro Leu Tyr His Ser Ala Gly Asn Ile Val Gly Ile Gly Gln Cys Val
   50 55 60
   Leu His Gly Met Thr Val Val Ile Arg Lys Lys Phe Ser Ala Ser Arg
   65 70 75 80
   Phe Trp Asp Asp Cys Ile Lys Tyr Asn Cys Thr Ile Val Gln Tyr Ile
   85 90 95
   Gly Glu Leu Cys Arg Tyr Leu Leu Asn Gln Pro Pro Arg Glu Ala Glu
   100 105 110
   Ser Arg His Lys Val Arg Met Ala Leu Gly Asn Gly Leu Arg Gln Ser
   115 120 125
   Ile Trp Thr Asp Phe Ser Ser Arg Phe His Ile Pro Lys Val Ala Glu
   130 135 140
   Phe Tyr Gly Ala Thr Glu Cys Asn Cys Ser Leu Gly Asn Phe Asp Ser
   145 150 155 160
   Gln Val Gly Ala Cys Gly Phe Asn Ser Arg Ile Leu Ser Phe Val Tyr
   165 170 175
   Pro Ile Arg Leu Val Arg Val Asn Glu Asp Thr Met Glu Leu Ile Arg
   180 185 190
   Gly Pro Asp Gly Val Cys Ile Pro Cys Gln Pro Gly Gln Pro Gly Gln
   195 200 205
   Leu Val Gly Arg Ile Ile Gln Gln Asp Pro Leu Arg Arg Phe Asp Gly
   210 215 220
   Tyr Leu Asn Gln Gly Ala Asn Asn Lys Lys Ile Ala Ser Asp Val Phe
   225 230 235 240
   Lys Lys Gly Asp Gln Ala Tyr Leu Thr Gly Asp Val Leu Val Met Asp
   245 250 255
   Glu Leu Gly Tyr Leu Tyr Phe Arg Asp Arg Thr Gly Asp Thr Phe Arg
   260 265 270
   Trp Lys Gly Glu Asn Val Ser Thr Thr Glu Val Glu Gly Thr Leu Ser
   275 280 285
   Arg Leu Leu Gln Met Ala Asp Val Ala Val Tyr Gly Val Glu Val Pro
   290 295 300
   Gly Ala Glu Gly Arg Ala Gly Met Ala Ala Val Ala Ser Pro Thr Ser
   305 310 315 320
   Asn Cys Asp Leu Glu Ser Phe Ala Gln Thr Leu Lys Lys Glu Leu Pro
   325 330 335
   Leu Tyr Ala Arg Pro Ile Phe Leu Arg Phe Leu Pro Glu Leu His Lys
   340 345 350
   Thr Gly Thr Phe Lys Phe Gln Lys Thr Glu Leu Arg Lys Glu Gly Phe
   355 360 365
   Asp Pro Ser Val Val Lys Asp Pro Leu Phe Tyr Leu Asp Ala Arg Thr
   370 375 380
   Gly Cys Tyr Val Ala Leu Asp Gln Glu Ala Tyr Thr Arg Ile Gln Ala
   385 390 395 400
   Gly Glu Glu Lys Leu
   405
<210> 64
   <211> 3217
   <212> DNA
   <213> Mus musculus
<400> 64
   atgcgggctc ctggagcagg aacagcctct gtggcctcac tggcgctgct ttggtttctg      60
   ggacttccgt ggacctggag cgcggcggcg gcgttctgtg tgtacgtggg tggcggcggc     120
   tggcgctttc tgcgtatcgt ctgcaagacg gcgaggcgag acctctttgg cctctctgtt     180
   ctgattcgtg ttcggctaga gctgcgacga caccggcgag caggagacac gatcccgtgc     240
   atcttccagg ctgtggcccg gcgacaacca gagcgcctgg cactggtgga cgccagtagt     300
   ggtatatgct ggaccttcgc acagctggac acctactcca atgctgtagc caacctgttc     360
   cgccagctgg gctttgcacc aggcgatgtg gtggctgtgt tcctggaggg ccggccggag     420
   ttcgtgggac tgtggctggg cctggccaag gccggtgtgg tggctgctct tctcaatgtc     480
   aacctgaggc gggagcccct ggccttctgc ctgggcacat cagctgccaa ggccctcatt     540
   tatggcgggg agatggcagc ggcggtggcg gaggtgagcg agcagctggg gaagagcctc     600
   ctcaagttct gctctggaga tctggggcct gagagcatcc tgcctgacac gcagctcctg     660
   gaccccatgc ttgctgaggc gcccaccaca cccctggcac aagccccagg caagggcatg     720
   gatgatcggc tgttttacat ctatacttct gggaccaccg ggcttcctaa ggctgccatt    780
   gtggtgcaca gcaggtacta ccgcattgct gcctttggcc accattccta cagcatgcgt    840
   gccgccgatg tgctctatga ctgcctgcca ctctaccact ctgcagggaa catcatgggt    900
   gtggggcagt gcgtcatcta cgggttgacg gtggtactgc gcaagaagtt ctccgccagc    960
   cgcttctggg atgactgtgt caagtacaat tgcacggtag tggatgacat aggtgaaatc   1020
   tgccgctacc tgctgaggca gccggttcgc gacgtggagc agcgacaccg cgtgcgcctg   1080
   gccgtgggta atgggctgcg gccagccatc tgggaggagt tcacgcagcg cttcggtgtg   1140
   ccacagatcg gcgagttcta cggcgctacc gagtgcaact gcagcattgc caacatggac   1200
   ggcaaggtcg gctcctgcgg cttcaacagc cgtatcctca cgcatgtgta ccccatccgt   1260
   ctggtcaagg tcaatgagga cacgatggag ccactgcggg actccgaggg cctctgcatc   1320
   ccgtgccagc ccggggaacc cggccttctc gtgggccaga tcaaccagca ggaccctctg   1380
   cggcgtttcg atggttatgt tagtgacagt gccaccaaca agaagattgc ccacagcgtt   1440
   ttccgaaagg gcgatagcgc ctacctctca ggtgacgtgc tagtgatgga cgagctgggc   1500
   tacatgtatt tccgtgaccg cagcggggac accttccgct ggcgcgggga gaacgtgtcc   1560
   accacggagg tggaagccgt gctgagccgc ctactgggcc agacggacgt ggctgtgtat   1620
   ggggtggctg tgccaggagt ggaggggaaa gctggcatgg cagccatcgc agatccccac   1680
   agccagttgg accctaactc aatgtaccag gaattacaga aggttcttgc atcctatgct   1740
   cggcccatct tcctgcgtct tctgccccag gtggatacca caggcacctt caagatccag   1800
   aagacccggc tgcagcgtga aggctttgac ccccgtcaga cctcagacag gctcttcttt   1860
   ctagacctga agtccggcac gaggtatcta cccctggatg agagagtcca tgcccgcatt   1920
   tgcgcaggcg acttctcact ctgagcctgg agagtgggct gggcctggac tcctgagacc   1980
   tgggagcctg acacccctct tcgggtgctt ctcctgcctg gccacatgga cagcagcacc   2040
   tgtgagagta ggaaaatgga acctgagtgg ctgggacccc tctcctactt cccactatgc   2100
   atccattttg cctctgcctt gatctttttc tccatctctt ttctccctac ccagcaggag   2160
   ccccacaaac acatgttggc tgctgtgtcc tgcagttgga ccagtgtcca ggggtacagg   2220
   cttcaggctg tgacccacac tggtacccac ctccctttcc tattttgcct taggttcatc   2280
   cacggttccc ctgtggagca agtgggggcc cacatagctg ctgtccctgc tgagggttgg   2340
   tagcaatcac accctcatgt cagctgggag acacgcgcag tctcccactg acccccaatc   2400
   aactgaaaat attgttttga ctactttttg tttttttgtt tttttgtttt tttttttttt   2460
   cgagacagag tttctctgta tagccctggc tgtcctggaa ctcactttgt agaccaggct   2520
   ggcctcgaac tcaaaaatcc tcctgactct gcctctgctt cccaagtgct gggattaaag   2580
   acgtgcgcca ccaccgcctg gctgttttgt atttttgttt tgttttgacg atagggtctc   2640
   actgtggagg ccaagctggc ctcagactcc ccaccccatt gcctctgggc accattctat   2700
   attctcagac tgatgacaat gcactagtgt ccctaggagt cttgagtctg cactttcccc   2760
   tcatagcctc aagcttccag aactgactct gatcacttgg atgtggctag tgttggctct   2820
   acccacatgt gtcaattcag gggtccccag gcatagtctc tggaagccct cacccggaaa   2880
   aagcttggag agacccagga aggttgttgt gttctcttgg gcaccccctg gtggcagtcc   2940
   tgggcatgct tccgcactgt actggtgcat atagcccaga cctatgacat ttgaggtcta   3000
   cccttctggc tcctgtggtc cccattgaga tccttggtga ctcacctcag tcaccaagca   3060
   gagcctctgc ctgccttcat cttcaaggtc atgaaggatg tggacagagc agctacaggc   3120
   tgccagcagt caaccacatg agagtgttac ttccttgttg gtttttaaaa aataaatgtg   3180
   ctgagcctcg aaaaaaaaaa aaaaaaaaaa aaaaaaa                            3217
<210> 65
   <211> 646
   <212> PRT
   <213> Mus musculus
<400> 65
<210> 66
   <211> 2338
   <212> DNA
   <213> Mus musculus
<400> 66
   gggcggaggc cgagcccagt cgccagctcc tgctctgctc ctctcccgcc tgccgccgcg      60
   ctgcacgcct cgagcactcc ctcggccccg gcggggaccg gggaccccgc agctaccgcc     120
   atgctgccag tgctctacac cggcctggcg gggctgctgc tgctgcctct gctgctcacc     180
   tgctgctgcc cctacctcct ccaagatgtg cggtacttcc tgcggctggc caacatggcc     240
   cggcgggtgc gcagctaccg gcagcggcga cccgtgcgta ccatcctgcg ggccttcctg     300
   gaacaagcgc gcaagacccc acacaagccc ttcctgctgt tccgagacga gacgctcacc     360
   tacgcccagg tggaccggcg cagcaaccaa gtggcgcggg cgctgcacga tcaactgggc     420
   ctacgacagg gggattgcgt agccctcttc atgggcaatg agccggccta cgtgtggatc     480
   tggctgggac tgctcaaact gggctgtccc atggcgtgcc tcaactacaa cattcgtgcc     540
   aagtctctgc tgcactgctt tcaatgctgc ggggcgaagg tgctgctggc ctccccagat     600
   ctacaagaag ctgtggagga ggttcttcca accctgaaaa aggatgccgt gtccgtcttt     660
   tacgtaagca gaacttctaa cacaaatggt gtggacacaa tactggacaa agtagacgga     720
   gtgtcggcgg aacccacccc ggagtcgtgg aggtctgaag tcacttttac cacgccagca     780
   gtatacattt atacttcggg aaccacaggt cttccaaaaa gcggaaccat caatcatcat     840
   cgcctaaggt atgggacaag ccttgctatg tcgagtggga atcacggcca aggatgtcat     900
   ctataccaac aatgcccctg ttccaacagt gcaacgctca agatcggcct tcacggatgc     960
   atcctgggtt ggggctactt taaccttggc ggggcaaatt ctcaagcaag ccaattttgg    1020
   gaacgactgg caggaaatac aacgtcaacg gtcattcagt acattggtga actgcttcgg    1080
   tacctgtgca acacaccgca gaaaccaaat gaccgggacc acaaagtgaa aaaagccctg    1140
   ggaaatggct tacgaggaga tgtgtggaga gagttcatca agagatttgg ggacatccac    1200
   gtgtatgagt tctacgcatc cactgaaggc aacattggat ttgtgaacta tccaaggaaa    1260
   atcggtgctg tcgggagagc aaactaccta caaagaaaag ttgcaaggta tgagctgatc    1320
   aagtatgacg tggagaagga cgagccggtc cgtgacgcaa atggatattg catcaaagtc    1380
   cccaaaggtg aggttggact cttggtttgc aaaatcacac agctcacacc atttattggc    1440
   tatgctggag gaaagaccca gacagagaag aaaaaactca gagatgtctt taagaaaggc    1500
   gacatctact tcaacagcgg agacctcctg atgatcgacc gtgagaactt cgtctacttt    1560
   cacgacaggg ttggagatac tttccggtgg aaaggagaga acgtagctac cacagaagtc    1620
   gctgacatcg tgggactggt agattttgtt gaagaagtga atgtgtatgg cgtgcctgtg    1680
   ccaggtcatg agggtcgaat tgggatggcc tccctcaaga tcaaagaaaa ctacgagttc    1740
   aatggaaaga aactctttca acacatcgcg gagtacctgc ccagttacgc gaggcctcgg    1800
   ttcctgagga tacaagatac cattgagatc actgggactt ttaaacaccg caaagtgacc    1860
   ctgatggaag agggcttcaa tcccacagtc atcaaagata ccttgtattt catggatgat    1920
   gcagagaaaa catttgtgcc catgactgag aacatttata atgccataat tgataaaact    1980
   ctgaagctct gaatattccc tggtggttta gctcatgaca tttccagaaa gaaactcgat    2040
   agacctcgca gagccacttc atacgtagaa tccaacttta acttgattga agactataag    2100
   gtgcgatttt atttttagga aattattcat taaaaggata gttttttttt ttttttttaa    2160
   ttacacctga acctttgcaa gtaaaaagat ttagagacaa ttatttttca atgtgcacct    2220
   gccatttgtc cttgcaaact aagcttcttg gagagagggc cttatttttt taaagacata    2280
   ataaactata ttaacactaa aaaaaaaaaa aaaaaaaaaa aaaaaaaaaa aaaaaaaa      2338
<210> 67
   <211> 623
   <212> PRT
   <213> Mus musculus
<400> 67
   Met Leu Pro Val Leu Tyr Thr Gly Leu Ala Gly Leu Leu Leu Leu Pro
   1 5 10 15
   Leu Leu Leu Thr Cys Cys Cys Pro Tyr Leu Leu Gln Asp Val Arg Tyr
   20 25 30
   Phe Leu Arg Leu Ala Asn Met Ala Arg Arg Val Arg Ser Tyr Arg Gln
   35 40 45
   Arg Arg Pro Val Arg Thr Ile Leu Arg Ala Phe Leu Glu Gln Ala Arg
   50 55 60
   Lys Thr Pro His Lys Pro Phe Leu Leu Phe Arg Asp Glu Thr Leu Thr
   65 70 75 80
   Tyr Ala Gln Val Asp Arg Arg Ser Asn Gln Val Ala Arg Ala Leu His
   85 90 95
   Asp Gln Leu Gly Leu Arg Gln Gly Asp Cys Val Ala Leu Phe Met Gly
   100 105 110
   Asn Glu Pro Ala Tyr Val Trp Ile Trp Leu Gly Leu Leu Lys Leu Gly
   115 120 125
   Cys Pro Met Ala Cys Leu Asn Tyr Asn Ile Arg Ala Lys Ser Leu Leu
   130 135 140
   His Cys Phe Gln Cys Cys Gly Ala Lys Val Leu Leu Ala Ser Pro Asp
   145 150 155 160
   Leu Gln Glu Ala Val Glu Glu Val Leu Pro Thr Leu Lys Lys Asp Ala
   165 170 175
   Val Ser Val Phe Tyr Val Ser Arg Thr Ser Asn Thr Asn Gly Val Asp
   180 185 190
   Thr Ile Leu Asp Lys Val Asp Gly Val Ser Ala Glu Pro Thr Pro Glu
   195 200 205
   Ser Trp Arg Ser Glu Val Thr Phe Thr Thr Pro Ala Val Tyr Ile Tyr
   210 215 220
   Thr Ser Gly Thr Thr Gly Leu Pro Lys Ser Gly Thr Ile Asn His His
   225 230 235 240
   Arg Leu Arg Tyr Gly Thr Ser Leu Ala Met Ser Ser Gly Asn His Gly
   245 250 255
   Gln Gly Cys His Leu Tyr Gln Gln Cys Pro Cys Ser Asn Ser Ala Thr
   260 265 270
   Leu Lys Ile Gly Leu His Gly Cys Ile Leu Gly Trp Gly Tyr Phe Asn
   275 280 285
   Leu Gly Gly Ala Asn Ser Gln Ala Ser Gln Phe Trp Glu Arg Leu Ala
   290 295 300
   Gly Asn Thr Thr Ser Thr Val Ile Gln Tyr Ile Gly Glu Leu Leu Arg
   305 310 315 320
   Tyr Leu Cys Asn Thr Pro Gln Lys Pro Asn Asp Arg Asp His Lys Val
   325 330 335
   Lys Lys Ala Leu Gly Asn Gly Leu Arg Gly Asp Val Trp Arg Glu Phe
               340                 345                 350
   Ile Lys Arg Phe Gly Asp Ile His Val Tyr Glu Phe Tyr Ala Ser Thr
           355                 360                 365
   Glu Gly Asn Ile Gly Phe Val Asn Tyr Pro Arg Lys Ile Gly Ala Val
       370                 375                 380
   Gly Arg Ala Asn Tyr Leu Gln Arg Lys Val Ala Arg Tyr Glu Leu Ile
   385                 390                 395                 400
   Lys Tyr Asp Val Glu Lys Asp Glu Pro Val Arg Asp Ala Asn Gly Tyr
                   405                 410                 415
   Cys Ile Lys Val Pro Lys Gly Glu Val Gly Leu Leu Val Cys Lys Ile
               420                 425                 430
   Thr Gln Leu Thr Pro Phe Ile Gly Tyr Ala Gly Gly Lys Thr Gln Thr
           435                 440                 445
   Glu Lys Lys Lys Leu Arg Asp Val Phe Lys Lys Gly Asp Ile Tyr Phe
       450                 455                 460
   Asn Ser Gly Asp Leu Leu Met Ile Asp Arg Glu Asn Phe Val Tyr Phe
   465                 470                 475                 480
   His Asp Arg Val Gly Asp Thr Phe Arg Trp Lys Gly Glu Asn Val Ala
                   485                 490                 495
   Thr Thr Glu Val Ala Asp Ile Val Gly Leu Val Asp Phe Val Glu Glu
               500                 505                 510
   Val Asn Val Tyr Gly Val Pro Val Pro Gly His Glu Gly Arg Ile Gly
           515                 520                 525
   Met Ala Ser Leu Lys Ile Lys Glu Asn Tyr Glu Phe Asn Gly Lys Lys
       530                 535                 540
   Leu Phe Gln His Ile Ala Glu Tyr Leu Pro Ser Tyr Ala Arg Pro Arg
   545                 550                 555                 560
   Phe Leu Arg Ile Gln Asp Thr Ile Glu Ile Thr Gly Thr Phe Lys His
                   565                 570                 575
   Arg Lys Val Thr Leu Met Glu Glu Gly Phe Asn Pro Thr Val Ile Lys
               580                 585                 590
   Asp Thr Leu Tyr Phe Met Asp Asp Ala Glu Lys Thr Phe Val Pro Met
           595                 600                 605
   Thr Glu Asn Ile Tyr Asn Ala Ile Ile Asp Lys Thr Leu Lys Leu
       610                 615                 620
<210> 68
   <211> 1998
   <212> DNA
   <213> Mus musculus
<400> 68
   gaaagctctg agagcgggtg cagtctggcc tggcgtctcg cgtacctggc ccgggagcag      60
   ccgacacaca ccttcctcat ccacggcgcg cagcgcttta gctacgcgga ggctgagcgc     120
   gagagcaacc ggattgctcg cgcctttctg cgcgcacggg gctggaccgg gggccgccga     180
   ggctcgggca ggggcagcac tgaggaaggc gcacgcgtgg cgcctccggc tggagatgcg     240
   gctgctagag ggacgaccgc gccccctctg gcacccgggg cgaccgtggc gctgctcctc     300
   ccagcgggcc cggatttcct ttggatttgg ttcggactgg ccaaagctgg cctgcgcacg     360
   gcctttgtgc ccaccgcttt acgccgagga cccctgctgc actgcctccg cagctgcggt     420
   gcgagtgcgc tcgtgctggc cacagagttc ctggagtccc tggagccgga cctgccggcc     480
   ttgagagcca tggggctcca cctatgggcg acgggccctg aaactaatgt agctggaatc     540
   agcaatttgc tatcggaagc agcagaccaa gtggatgagc cagtgccggg gtacctctct     600
   gccccccaga acataatgga cacctgcctg tacatcttca cctctggcac tactggcctg     660
   cccaaggctg ctcgaatcag tcatctgaag gttctacagt gccagggatt ctaccatctg     720
   tgtggagtcc accaggagga cgtgatctac ctcgcactcc cactgtacca catgtctggc     780
   tcccttctgg gcattgtggg ctgcttgggc attggggcca ccgtggtgct gaaacccaag     840
   ttctcagcta gccagttctg ggacgattgc cagaaacaca gggtgacagt gttccagtac     900
   attggggagt tgtgccgata cctcgtcaac cagcccccga gcaaggcaga gtttgaccat     960
   aaggtgcgct tggcagtggg cagtgggttg cgcccagaca cctgggagcg tttcctgcgg    1020
   cgatttggac ctctgcagat actggagacg tatggcatga cagagggcaa cgtagctacg    1080
   ttcaattaca caggacggca gggtgcagtg gggcgagctt cctggcttta caagcacatc    1140
   ttccccttct ccttgattcg atacgatgtc atgacagggg agcctattcg gaatgcccag    1200
   gggcactgca tgaccacatc tccaggtgag ccaggcctac tggtggcccc agtgagccag    1260
   cagtccccct tcctgggcta tgctggggct ccggagctgg ccaaggacaa gctgctgaag    1320
   gatgtcttct ggtctgggga cgttttcttc aatactgggg acctcttggt ctgtgatgag    1380
   caaggctttc ttcacttcca cgatcgtact ggagacacca tcaggtggaa gggagagaat    1440
   gtggccacaa ctgaagtggc tgaggtcttg gagaccctgg acttccttca ggaggtgaac    1500
   atctatggag tcacggtgcc agggcacgaa ggcagggcag gcatggcggc cttggctctg    1560
   cggcccccgc aggctctgaa cctggtgcag ctctacagcc atgtttctga gaacttgcca    1620
   ccgtatgccc gacctcggtt tctcaggctc caggaatctt tggccactac tgagaccttc     1680
   aaacagcaga aggttaggat ggccaatgag ggctttgacc ccagtgtact gtctgaccca    1740
   ctctatgttc tggaccaaga tataggggcc tacctgcccc tcacacctgc ccggtacagt    1800
   gccctcctgt ctggagacct tcgaatctga aaccttccac ttgagggagg ggctcggagg    1860
   gtacaggcca ccatggctgc accagggagg gttttcgggt atcttttgta tatggagtca    1920
   ttattttgta ataaacagct ggagcttaaa aaaaaaaaaa aaaaaaaaaa aaaaaaaaaa    1980
   aaaaaaaaaa aaaaaaaa                                                  1998
<210> 69
   <211> 609
   <212> PRT
   <213> Mus musculus
<400> 69
   Glu Ser Ser Glu Ser Gly Cys Ser Leu Ala Trp Arg Leu Ala Tyr Leu
    1               5                  10                  15
   Ala Arg Glu Gln Pro Thr His Thr Phe Leu Ile His Gly Ala Gln Arg
               20                  25                  30
   Phe Ser Tyr Ala Glu Ala Glu Arg Glu Ser Asn Arg Ile Ala Arg Ala
           35                  40                  45
   Phe Leu Arg Ala Arg Gly Trp Thr Gly Gly Arg Arg Gly Ser Gly Arg
       50                  55                  60
   Gly Ser Thr Glu Glu Gly Ala Arg Val Ala Pro Pro Ala Gly Asp Ala
   65                  70                  75                  80
   Ala Ala Arg Gly Thr Thr Ala Pro Pro Leu Ala Pro Gly Ala Thr Val
                   85                  90                  95
   Ala Leu Leu Leu Pro Ala Gly Pro Asp Phe Leu Trp Ile Trp Phe Gly
               100                 105                 110
   Leu Ala Lys Ala Gly Leu Arg Thr Ala Phe Val Pro Thr Ala Leu Arg
           115                 120                 125
   Arg Gly Pro Leu Leu His Cys Leu Arg Ser Cys Gly Ala Ser Ala Leu
       130                 135                 140
   Val Leu Ala Thr Glu Phe Leu Glu Ser Leu Glu Pro Asp Leu Pro Ala
   145                 150                 155                 160
   Leu Arg Ala Met Gly Leu His Leu Trp Ala Thr Gly Pro Glu Thr Asn
                   165                 170                 175
   Val Ala Gly Ile Ser Asn Leu Leu Ser Glu Ala Ala Asp Gln Val Asp
               180                 185                 190
   Glu Pro Val Pro Gly Tyr Leu Ser Ala Pro Gln Asn Ile Met Asp Thr
           195                 200                 205
   Cys Leu Tyr Ile Phe Thr Ser Gly Thr Thr Gly Leu Pro Lys Ala Ala
       210                 215                 220
   Arg Ile Ser His Leu Lys Val Leu Gln Cys Gln Gly Phe Tyr His Leu
   225                 230                 235                 240
   Cys Gly Val His Gln Glu Asp Val Ile Tyr Leu Ala Leu Pro Leu Tyr
                   245                 250                 255
   His Met Ser Gly Ser Leu Leu Gly Ile Val Gly Cys Leu Gly Ile Gly
               260                 265                 270
   Ala Thr Val Val Leu Lys Pro Lys Phe Ser Ala Ser Gln Phe Trp Asp
           275                 280                 285
   Asp Cys Gln Lys His Arg Val Thr Val Phe Gln Tyr Ile Gly Glu Leu
       290                 295                 300
   Cys Arg Tyr Leu Val Asn Gln Pro Pro Ser Lys Ala Glu Phe Asp His
   305                 310                 315                 320
   Lys Val Arg Leu Ala Val Gly Ser Gly Leu Arg Pro Asp Thr Trp Glu
                   325                 330                 335
   Arg Phe Leu Arg Arg Phe Gly Pro Leu Gln Ile Leu Glu Thr Tyr Gly
               340                 345                 350
   Met Thr Glu Gly Asn Val Ala Thr Phe Asn Tyr Thr Gly Arg Gln Gly
           355                 360                 365
   Ala Val Gly Arg Ala Ser Trp Leu Tyr Lys His Ile Phe Pro Phe Ser
       370                 375                 380
   Leu Ile Arg Tyr Asp Val Met Thr Gly Glu Pro Ile Arg Asn Ala Gln
   385                 390                 395                 400
   Gly His Cys Met Thr Thr Ser Pro Gly Glu Pro Gly Leu Leu Val Ala
                   405                 410                 415
   Pro Val Ser Gln Gln Ser Pro Phe Leu Gly Tyr Ala Gly Ala Pro Glu
               420                 425                 430
   Leu Ala Lys Asp Lys Leu Leu Lys Asp Val Phe Trp Ser Gly Asp Val
           435                 440                 445
   Phe Phe Asn Thr Gly Asp Leu Leu Val Cys Asp Glu Gln Gly Phe Leu
       450                 455                 460
   His Phe His Asp Arg Thr Gly Asp Thr Ile Arg Trp Lys Gly Glu Asn
   465                 470                 475                 480
   Val Ala Thr Thr Glu Val Ala Glu Val Leu Glu Thr Leu Asp Phe Leu
                   485                 490                 495
   Gln Glu Val Asn Ile Tyr Gly Val Thr Val Pro Gly His Glu Gly Arg
               500                 505                 510
   Ala Gly Met Ala Ala Leu Ala Leu Arg Pro Pro Gln Ala Leu Asn Leu
           515                 520                 525
   Val Gln Leu Tyr Ser His Val Ser Glu Asn Leu Pro Pro Tyr Ala Arg
       530                 535                 540
   Pro Arg Phe Leu Arg Leu Gln Glu Ser Leu Ala Thr Thr Glu Thr Phe
   545                 550                 555                 560
   Lys Gln Gln Lys Val Arg Met Ala Asn Glu Gly Phe Asp Pro Ser Val
                   565                 570                 575
   Leu Ser Asp Pro Leu Tyr Val Leu Asp Gln Asp Ile Gly Ala Tyr Leu
               580                 585                 590
   Pro Leu Thr Pro Ala Arg Tyr Ser Ala Leu Leu Ser Gly Asp Leu Arg
           595                 600                 605
   Ile
<210> 70
   <211> 2710
   <212> DNA
   <213> Mus musculus
<400> 70
   atgctgcttg gagcctctct ggtgggggcg ctactgttct ccaagctagt gctgaagctg      60
   ccctggaccc aggtgggatt ctccctgttg ctcctgtact tggggtctgg tggctggcgt     120
   ttcatccggg tcttcatcaa gacggtcagg agagatatct ttggtggcat ggtgctcctg     180
   aaggtgaaga ccaaggtgcg acggtacctt caggagcgga agacggtgcc cctgctgttt     240
   gcttcaatgg tacagcgcca cccggacaag acagccctga ttttcgaggg cacagacact     300
   cactggacct tccgccagct ggatgagtac tccagtagtg tggccaactt cctgcaggcc     360
   cggggcctgg cctcaggcaa tgtagttgcc ctctttatgg aaaaccgcaa tgagtttgtg     420
   ggtctgtggc taggcatggc caagctgggc gtggaggcgg ctctcatcaa caccaacctt     480
   aggcgggatg ccctgcgcca ctgtcttgac acctcaaagg cacgagctct catctttggc     540
   agtgagatgg cctcagctat ctgtgagatc catgctagcc tggagcccac actcagcctc     600
   ttctgctctg gatcctggga gcccagcaca gtgcccgtca gcacagagca tctggaccct     660
   cttctggaag atgccccgaa gcacctgccc agtcacccag acaagggttt tacagataag     720
   ctcttctaca tctacacatc gggcaccacg gggctaccca aagctgccat tgtggtgcac     780
   agcaggtatt atcgtatggc ttccctggtg tactatggat tccgcatgcg gcctgatgac     840
   attgtctatg actgcctccc cctctaccac tcaagcagga aacatcgtgg ggattggcag     900
   tgcttactcc acggcatgac tgtggtgatc cggaagaagt tctcagcctc ccggttctgg     960
   gatgattgta tcaagtacaa ctgcacagtg gtacagtaca ttggcgagct ctgccgctac    1020
   ctcctgaacc agccaccccg tgaggctgag tctcggcaca aggtgcgcat ggcactgggc    1080
   aacggtctcc ggcagtccat ctggaccgac ttctccagcc gtttccacat cccccaggtg    1140
   gctgagttct atggggccac tgaatgcaac tgtagcctgg gcaactttga cagccgggtg    1200
   ggggcctgtg gcttcaatag ccgcatcctg tcctttgtgt accctatccg tttggtacgt    1260
   gtcaatgagg ataccatgga actgatccgg ggacccgatg gagtctgcat tccctgtcaa    1320
   ccaggtcagc caggccagct ggtgggtcgc atcatccagc aggaccctct gcgccgtttc    1380
   gacgggtacc tcaaccaggg tgccaacaac aagaagattg ctaatgatgt cttcaagaag    1440
   ggggaccaag cctacctcac tggtgacgtc ctggtgatgg atgagctggg ttacctgtac    1500
   ttccgagatc gcactgggga cacgttccgc tggaaagggg agaatgtatc taccactgag    1560
   gtggagggca cactcagccg cctgcttcat atggcagatg tggcagttta tggtgttgag    1620
   gtgccaggaa ctgaaggccg agcaggaatg gctgccgttg caagtcccat cagcaactgt    1680
   gacctggaga gctttgcaca gaccttgaaa aaggagctgc ctctgtatgc ccgccccatc    1740
   ttcctgcgct tcttgcctga gctgcacaag acagggacct tcaagttcca gaagacagag    1800
   ttgcggaagg agggctttga cccatctgtt gtgaaagacc cgctgttcta tctggatgct    1860
   cggaagggct gctacgttgc actggaccag gaggcctata cccgcatcca ggcaggcgag    1920
   gagaagctgt gatttccccc tacatccctc tgagggccag aagatgctgg attcagagcc    1980
   ctagcgtcca ccccagaggg tcctgggcaa tgccagacca aagctagcag ggcccgcacc    2040
   tccgccccta ggtgctgatc tcccctctcc caaactgcca agtgactcac tgccgcttcc    2100
   ccgaccctcc agaggctttc tgtgaaagtc tcatccaagc tgtgtcttct ggtccaggcg    2160
   tggcccctgg ccccagggtt tctgataggc tcctttagga tggtatcttg ggtccagcgg    2220
   gccagggtgt gggagaggag tcactaagat ccctccaatc agaagggagc ttacaaagga    2280
   accaaggcaa agcctgtaga ctcaggaagc taagtggcca gagactatag tggccagtca    2340
   tcccatgtcc acagaggatc ttggtccaga gctgccaaag tgtcacctct ccctgcctgc    2400
   acctctgggg aaaagaggac agcatgtggc cactgggcac ctgtctcaag aagtcaggat    2460
   cacacactca gtccttgttt ctccaggttc ccttgttctt gtctcgggga gggagggacg    2520
   agtgtcctgt ctgtccttcc tgcctgtctg tgagtctgtg ttgcttctcc atctgtccta    2580
   gcctgagtgt gggtggaaca ggcatgagga gagtgtggct caggggccaa taaactctgc    2640
   cttgactcct cttaaaaaaa aaaaaaaaaa aaaaaaaaaa aaaaaaaaaa aaaaaaaaaa    2700
   aaaaaaaaaa                                                          2710
<210> 71
   <211> 643
   <212> PRT
   <213> Mus musculus
<400> 71
   Met Leu Leu Gly Ala Ser Leu Val Gly Ala Leu Leu Gly Ser Lys Leu
    1               5                  10                  15
   Val Leu Lys Leu Pro Trp Thr Gln Val Gly Phe Ser Leu Leu Leu Leu
               20                  25                  30
   Tyr Leu Gly Ser Gly Gly Trp Arg Phe Ile Arg Val Phe Ile Lys Thr
           35                  40                  45
   Val Arg Arg Asp Ile Phe Gly Gly Met Val Leu Leu Lys Val Lys Thr
   50 55 60
   Lys Val Arg Arg Tyr Leu Gln Glu Arg Lys Thr Val Pro Leu Leu Phe
   65 70 75 80
   Ala Ser Met Val Gln Arg His Pro Asp Lys Thr Ala Leu Ile Phe Glu
   85 90 95
   Gly Thr Asp Thr His Trp Thr Phe Arg Gln Leu Asp Glu Tyr Ser Ser
   100 105 110
   Ser Val Ala Asn Phe Leu Gln Ala Arg Gly Leu Ala Ser Gly Asn Val
   115 120 125
   Val Ala Leu Phe Met Glu Asn Arg Asn Glu Phe Val Gly Leu Trp Leu
   130 135 140
   Gly Met Ala Lys Leu Gly Val Glu Ala Ala Leu Ile Asn Thr Asn Leu
   145 150 155 160
   Arg Arg Asp Ala Leu Arg His Cys Leu Asp Thr Ser Lys Ala Arg Ala
   165 170 175
   Leu Ile Phe Gly Ser Glu Met Ala Ser Ala Ile Cys Glu Ile His Ala
   180 185 190
   Ser Leu Glu Pro Thr Leu Ser Leu Phe Cys Ser Gly Ser Trp Glu Pro
   195 200 205
   Ser Thr Val Pro Val Ser Thr Glu His Leu Asp Pro Leu Leu Glu Asp
   210 215 220
   Ala Pro Lys His Leu Pro Ser His Pro Asp Lys Gly Phe Thr Asp Lys
   225 230 235 240
   Leu Phe Tyr Ile Tyr Thr Ser Gly Thr Thr Gly Leu Pro Lys Ala Ala
   245 250 255
   Ile Val Val His Ser Arg Tyr Tyr Arg Met Ala Ser Leu Val Tyr Tyr
   260 265 270
   Gly Phe Arg Met Arg Pro Asp Asp Ile Val Tyr Asp Cys Leu Pro Leu
   275 280 285
   Tyr His Ser Ser Arg Lys His Arg Gly Asp Trp Gln Cys Leu Leu His
   290 295 300
   Gly Met Thr Val Val Ile Arg Lys Lys Phe Ser Ala Ser Arg Phe Trp
   305 310 315 320
   Asp Asp Cys Ile Lys Tyr Asn Cys Thr Val Val Gln Tyr Ile Gly Glu
   325 330 335
   Leu Cys Arg Tyr Leu Leu Asn Gln Pro Pro Arg Glu Ala Glu Ser Arg
   340 345 350
   His Lys Val Arg Met Ala Leu Gly Asn Gly Leu Arg Gln Ser Ile Trp
   355 360 365
   Thr Asp Phe Ser Ser Arg Phe His Ile Pro Gln Val Ala Glu Phe Tyr
   370 375 380
   Gly Ala Thr Glu Cys Asn Cys Ser Leu Gly Asn Phe Asp Ser Arg Val
   385 390 395 400
   Gly Ala Cys Gly Phe Asn Ser Arg Ile Leu Ser Phe Val Tyr Pro Ile
   405 410 415
   Arg Leu Val Arg Val Asn Glu Asp Thr Met Glu Leu Ile Arg Gly Pro
   420 425 430
   Asp Gly Val Cys Ile Pro Cys Gln Pro Gly Gln Pro Gly Gln Leu Val
   435 440 445
   Gly Arg Ile Ile Gln Gln Asp Pro Leu Arg Arg Phe Asp Gly Tyr Leu
   450 455 460
   Asn Gln Gly Ala Asn Asn Lys Lys Ile Ala Asn Asp Val Phe Lys Lys
   465 470 475 480
   Gly Asp Gln Ala Tyr Leu Thr Gly Asp Val Leu Val Met Asp Glu Leu
   485 490 495
   Gly Tyr Leu Tyr Phe Arg Asp Arg Thr Gly Asp Thr Phe Arg Trp Lys
               500                 505                 510
   Gly Glu Asn Val Ser Thr Thr Glu Val Glu Gly Thr Leu Ser Arg Leu
           515                 520                 525
   Leu His Met Ala Asp Val Ala Val Tyr Gly Val Glu Val Pro Gly Thr
       530                 535                 540
   Glu Gly Arg Ala Gly Met Ala Ala Val Ala Ser Pro Ile Ser Asn Cys
   545                 550                 555                 560
   Asp Leu Glu Ser Phe Ala Gln Thr Leu Lys Lys Glu Leu Pro Leu Tyr
                   565                 570                 575
   Ala Arg Pro Ile Phe Leu Arg Phe Leu Pro Glu Leu His Lys Thr Gly
               580                 585                 590
   Thr Phe Lys Phe Gln Lys Thr Glu Leu Arg Lys Glu Gly Phe Asp Pro
           595                 600                 605
   Ser Val Val Lys Asp Pro Leu Phe Tyr Leu Asp Ala Arg Lys Gly Cys
       610                 615                 620
   Tyr Val Ala Leu Asp Gln Glu Ala Tyr Thr Arg Ile Gln Ala Gly Glu
   625                 630                 635                 640
   Glu Lys Leu
<210> 72
   <211> 2277
   <212> DNA
   <213> Mus musculus
<400> 72
   cactcatcag agctaagaga gactacacgc tctcatctac ttcagaaaga gccaatgcca      60
   tgggtatttg gaagaaacta accttactgc tgttgctgct tctgctggtt ggcctggggc     120
   agcccccatg gccagcagct atggctctgg ccctgcgttg gttcctggga gaccccacat     180
   gccttgtgct gcttggcttg gcattgctgg gcagaccctg gatcagctcc tggatgcccc     240
   actggctgag cctggtagga gcagctctta ccttattcct attgcctcta cagccacccc     300
   cagggctacg ctggctgcat aaagatgtgg ctttcacctt caagatgctt ttctatggcc     360
   taaagttcag gcgacgcctt aacaaacatc ctccagagac ctttgtggat gctttagagc     420
   ggcaagcact ggcatggcct gaccgggtgg ccttggtgtg tactgggtct gagggctcct     480
   caatcacaaa tagccagctg gatgccaggt cctgtcaggc agcatgggtc ctgaaagcaa     540
   agctgaagga tgccgtaatc cagaacacaa gagatgctgc tgctatctta gttctcccgt     600
   ccaagaccat ttctgctttg agtgtgtttc tggggttggc caagttgggc tgccctgtgg     660
   cctggatcaa tccacacagc cgagggatgc ccttgctaca ctctgtacgg agctctgggg     720
   ccagtgtgct gattgtggat ccagacctcc aggagaacct ggaagaagtc cttcccaagc     780
   tgctagctga gaacattcac tgcttctacc ttggccacag ctcacccacc ccgggagtag     840
   aggctctggg agcttccctg gatgctgcac cttctgaccc agtacctgcc agccttcgag     900
   ctacgattaa gtggaaatct cctgccatat tcatctttac ttcagggacc actggactcc     960
   caaagccagc catcttatca catgagcggg tcatacaagt gagcaacgtg ctgtccttct    1020
   gtggatgcag agctgatgat gtggtctatg acgtcctacc tctgtaccat acgatagggc    1080
   ttgtccttgg attccttggc tgcttacaag ttggagccac ctgtgtcctg gcccccaagt    1140
   tctctgcctc ccgattctgg gctgagtgcc ggcagcatgg cgtaacagtg atcttgtatg    1200
   tgggtgaaat cctgcggtac ttgtgtaacg tccctgagca accagaagac aagatacata    1260
   cagtgcgctt ggccatggga actggacttc gggcaaatgt gtggaaaaac ttccagcaac    1320
   gctttggtcc cattcggatc tgggaattct acggatccac agagggcaat gtgggcttaa    1380
   tgaactatgt gggccactgc ggggctgtgg gaaggaccag ctgcatcctt cgaatgctga    1440
   ctccctttga gcttgtacag ttcgacatag agacagcaga gcctctgagg gacaaacagg    1500
   gtttttgcat tcctgtggag ccaggaaagc caggacttct tttgaccaag gttcgaaaga    1560
   accaaccctt cctgggctac cgtggttccc aggccgagtc caatcggaaa cttgttgcga    1620
   atgtacgacg cgtaggagac ctgtacttca acactgggga cgtgctgacc ttggaccagg    1680
   aaggcttctt ctactttcaa gaccgccttg gtgacacctt ccggtggaag ggcgaaaacg    1740
   tatctactgg agaggtggag tgtgttttgt ctagcctaga cttcctagag gaagtcaatg    1800
   tctatggtgt gcctgtgcca gggtgtgagg gtaaggttgg catggctgct gtgaaactgg    1860
   ctcctgggaa gacttttgat gggcagaagc tataccagca tgtccgctcc tggctccctg    1920
   cctatgccac acctcatttc atccgtatcc aggattccct ggagatcaca aacacctaca    1980
   agctggtaaa gtcacggctg gtgcgtgagg gttttgatgt ggggatcatt gctgaccccc    2040
   tctacatact ggacaacaag gcccagacct tccggagtct gatgccagat gtgtaccagg    2100
   ctgtgtgtga aggaacctgg aatctctgac cacctagcca actggaaggc aatccaaaag    2160
   tgtagagatt gacactagtc agcttcacaa agttgtccgg gttccagatg cccatggccc    2220
   agtagtactt agagaataaa cttgaatgtg tatacaaaaa aaaaaaaaaa aaaaaaa       2277
<210> 73
   <211> 689
   <212> PRT
   <213> Mus musculus
<400> 73
   Met Gly Ile Trp Lys Lys Leu Thr Leu Leu Leu Leu Leu Leu Leu Leu
    1               5                  10                  15
   Val Gly Leu Gly Gln Pro Pro Trp Pro Ala Ala Met Ala Leu Ala Leu
               20                  25                  30
   Arg Trp Phe Leu Gly Asp Pro Thr Cys Leu Val Leu Leu Gly Leu Ala
           35                  40                  45
   Leu Leu Gly Arg Pro Trp Ile Ser Ser Trp Met Pro His Trp Leu Ser
       50                  55                  60
   Leu Val Gly Ala Ala Leu Thr Leu Phe Leu Leu Pro Leu Gln Pro Pro
   65                  70                  75                  80
   Pro Gly Leu Arg Trp Leu His Lys Asp Val Ala Phe Thr Phe Lys Met
                   85                  90                  95
   Leu Phe Tyr Gly Leu Lys Phe Arg Arg Arg Leu Asn Lys His Pro Pro
               100                 105                 110
   Glu Thr Phe Val Asp Ala Leu Glu Arg Gln Ala Leu Ala Trp Pro Asp
           115                 120                 125
   Arg Val Ala Leu Val Cys Thr Gly Ser Glu Gly Ser Ser Ile Thr Asn
       130                 135                 140
   Ser Gln Leu Asp Ala Arg Ser Cys Gln Ala Ala Trp Val Leu Lys Ala
   145                 150                 155                 160
   Lys Leu Lys Asp Ala Val Ile Gln Asn Thr Arg Asp Ala Ala Ala Ile
                   165                 170                 175
   Leu Val Leu Pro Ser Lys Thr Ile Ser Ala Leu Ser Val Phe Leu Gly
               180                 185                 190
   Leu Ala Lys Leu Gly Cys Pro Val Ala Trp Ile Asn Pro His Ser Arg
           195                 200                 205
   Gly Met Pro Leu Leu His Ser Val Arg Ser Ser Gly Ala Ser Val Leu
       210                 215                 220
   Ile Val Asp Pro Asp Leu Gln Glu Asn Leu Glu Glu Val Leu Pro Lys
   225                 230                 235                 240
   Leu Leu Ala Glu Asn Ile His Cys Phe Tyr Leu Gly His Ser Ser Pro
                   245                 250                 255
   Thr Pro Gly Val Glu Ala Leu Gly Ala Ser Leu Asp Ala Ala Pro Ser
               260                 265                 270
   Asp Pro Val Pro Ala Ser Leu Arg Ala Thr Ile Lys Trp Lys Ser Pro
           275                 280                 285
   Ala Ile Phe Ile Phe Thr Ser Gly Thr Thr Gly Leu Pro Lys Pro Ala
       290                 295                 300
   Ile Leu Ser His Glu Arg Val Ile Gln Val Ser Asn Val Leu Ser Phe
   305                 310                 315                 320
   Cys Gly Cys Arg Ala Asp Asp Val Val Tyr Asp Val Leu Pro Leu Tyr
                   325                 330                 335
   His Thr Ile Gly Leu Val Leu Gly Phe Leu Gly Cys Leu Gln Val Gly
               340                 345                 350
   Ala Thr Cys Val Leu Ala Pro Lys Phe Ser Ala Ser Arg Phe Trp Ala
           355                 360                 365
   Glu Cys Arg Gln His Gly Val Thr Val Ile Leu Tyr Val Gly Glu Ile
       370                 375                 380
   Leu Arg Tyr Leu Cys Asn Val Pro Glu Gln Pro Glu Asp Lys Ile His
   385                 390                 395                 400
   Thr Val Arg Leu Ala Met Gly Thr Gly Leu Arg Ala Asn Val Trp Lys
                   405                 410                 415
   Asn Phe Gln Gln Arg Phe Gly Pro Ile Arg Ile Trp Glu Phe Tyr Gly
               420                 425                 430
   Ser Thr Glu Gly Asn Val Gly Leu Met Asn Tyr Val Gly His Cys Gly
           435                 440                 445
   Ala Val Gly Arg Thr Ser Cys Ile Leu Arg Met Leu Thr Pro Phe Glu
       450                 455                 460
   Leu Val Gln Phe Asp Ile Glu Thr Ala Glu Pro Leu Arg Asp Lys Gln
   465                 470                 475                 480
   Gly Phe Cys Ile Pro Val Glu Pro Gly Lys Pro Gly Leu Leu Leu Thr
                   485                 490                 495
   Lys Val Arg Lys Asn Gln Pro Phe Leu Gly Tyr Arg Gly Ser Gln Ala
               500                 505                 510
   Glu Ser Asn Arg Lys Leu Val Ala Asn Val Arg Arg Val Gly Asp Leu
           515                 520                 525
   Tyr Phe Asn Thr Gly Asp Val Leu Thr Leu Asp Gln Glu Gly Phe Phe
       530                 535                 540
   Tyr Phe Gln Asp Arg Leu Gly Asp Thr Phe Arg Trp Lys Gly Glu Asn
   545                 550                 555                 560
   Val Ser Thr Gly Glu Val Glu Cys Val Leu Ser Ser Leu Asp Phe Leu
                   565                 570                 575
   Glu Glu Val Asn Val Tyr Gly Val Pro Val Pro Gly Cys Glu Gly Lys
               580                 585                 590
   Val Gly Met Ala Ala Val Lys Leu Ala Pro Gly Lys Thr Phe Asp Gly
           595                 600                 605
   Gln Lys Leu Tyr Gln His Val Arg Ser Trp Leu Pro Ala Tyr Ala Thr
       610                 615                 620
   Pro His Phe Ile Arg Ile Gln Asp Ser Leu Glu Ile Thr Asn Thr Tyr
   625                 630                 635                 640
   Lys Leu Val Lys Ser Arg Leu Val Arg Glu Gly Phe Asp Val Gly Ile
                   645                 650                 655
   Ile Ala Asp Pro Leu Tyr Ile Leu Asp Asn Lys Ala Gln Thr Phe Arg
               660                 665                 670
   Ser Leu Met Pro Asp Val Tyr Gln Ala Val Cys Glu Gly Thr Trp Asn
           675                 680                 685
   Leu
<210> 74
   <211> 2221
   <212> DNA
   <213> Drosophila melanogaster
<400> 74
   gctctctggg cctatatcaa gctgctgagg tacacgaagc gccatgagcg gctcaactac      60
   acggtggcgg acgtcttcga acgaaatgtt caggcccatc cggacaaggt ggctgtggtc     120
   agtgagacgc aacgctggac cttccgtcag gtgaacgagc atgcgaacaa ggtggccaat     180
   gtgctgcagg ctcagggcta caaaaagggc gatgtggtgg ccctgttgct ggagaaccgc     240
   gccgagtacg tggccacctg gctgggtctc tccaagatcg gtgtgatcac accgctgatc     300
   aacacgaatc tgcgcggtcc ctccctgctg cacagcatca cggtggccca ttgctcggct     360
   ctcatttacg gcgaggactt cctggaagct gtcaccgacg tggccaagga tctgccagcg     420
   aacctcacac tcttccagtt caacaacgag aacaacaaca gcgagacgga aaagaacata     480
   ccgcaggcca agaatctgaa cgcgctgctg accacggcca gctatgagaa gcctaacaag     540
   acgcaggtta accaccacga caagctggtc tacatctaca cctccggcac cacaggattg     600
   ccaaaggctg cggttatctc tcactcccgt tatctgttta tcgctgctgg catccactac     660
   accatgggtt tccaggagga ggacatcttc tacacgccct tgcctttgta ccacaccgct     720
   ggtggcatta tgtgcatggg tcagtcggtg ctctttggct ccacggtctc cattcgcaag     780
   aagttctcgg catccaacta tttcgccgac tgcgccaagt ataatgcaac tattggtcag     840
   tatatcggtg agatggctcg ctacattcta gctacgaaac cctcggaata cgaccagaaa     900
   caccgagtgc gtctggtctt tggaaacgga ctgcgaccgc agatttggcc acagtttgtg     960
   cagcgcttca acattgccaa ggttggcgag ttctacggcg ccaccgaggg taatgcgaac    1020
   atcatgaatc atgacaacac ggtgggcgcc atcggctttg tgtcgcgcat cctgcccaag    1080
   atctacccaa tctcgatcat tcgcgccgat ccggacaccg gagagcccat tagagatagg    1140
   aatggcctat gccaactgtg cgctcccaac gagccaggcg tattcatcgg caagatcgtc    1200
   aaaggaaatc cttctcgcga attcctcgga tacgtcgatg aaaaggcctc cgcgaagaag    1260
   attgttaagg atgtgttcaa gcatggcgat atggctttca tctccggaga tctgctggtt    1320
   gccgacgaga agggttatct gtacttcaag gatcgcaccg gtgacacctt ccgctggaag    1380
   ggcgagaatg tttccaccag cgaggtggag gcgcaagtca gcaatgtggc cggttacaag    1440
   gataccgtcg tttacggcgt aaccattccg cacaccgagg gaagggccgg catggccgcc    1500
   atctatgatc cggagcgaga attggacctc gacgtcttcg ccgctagctt ggccaaggtg    1560
   ctgcccgcgt acgctcgtcc ccagatcatt cgattgctca ccaaggtgga cctgactgga    1620
   acctttaagc tgcgcaaggt agacctgcag aaggagggct acgatccgaa cgcgatcaag    1680
   gacgcgctgt actaccagac ttccaagggt cggtacgagc tgctcacgcc ccaggtttac    1740
   gaccaggtgc agcgcaacga aatccgcttc taagagctgc aatagagttg tgtctgaacc    1800
   ttgccttttg cccaatatgc tgttaattag tttgtaaggc taagtgtagt agaggaaaat    1860
   cgggggaaat cggcagcaaa gatcattcag cctaggagag atgcatccga agcacatttc    1920
   catgtcaaca atgcactttt gtatatcgta agcatatata tatcgtatat cgtaaacgta    1980
   gttgtatctg catttgtgta gatgatagcc tcctatacgc atttcaattg tttttagcgt    2040
   gctaaagaac cttgttaaat gcaatttcag ctattgttta gtcagtttta gtggcattta    2100
   cacttccatt ctcgttgcgt ttcgtttttg cctgtacata tgagaagctc tgatgttttt     2160
   gtatcaaata aagttttttc cttcaccacg gaccacgtga aaaaaaaaaa aaaaaaaaaa     2220
   a                                                                    2221
<210> 75
   <211> 590
   <212> PRT
   <213> Drosophila melanogaster
<400> 75
   Ala Leu Trp Ala Tyr Ile Lys Leu Leu Arg Tyr Thr Lys Arg His Glu
    1               5                  10                  15
   Arg Leu Asn Tyr Thr Val Ala Asp Val Phe Glu Arg Asn Val Gln Ala
               20                  25                  30
   His Pro Asp Lys Val Ala Val Val Ser Glu Thr Gln Arg Trp Thr Phe
           35                  40                  45
   Arg Gln Val Asn Glu His Ala Asn Lys Val Ala Asn Val Leu Gln Ala
       50                  55                  60
   Gln Gly Tyr Lys Lys Gly Asp Val Val Ala Leu Leu Leu Glu Asn Arg
   65                  70                  75                  80
   Ala Glu Tyr Val Ala Thr Trp Leu Gly Leu Ser Lys Ile Gly Val Ile
                   85                  90                  95
   Thr Pro Leu Ile Asn Thr Asn Leu Arg Gly Pro Ser Leu Leu His Ser
               100                 105                 110
   Ile Thr Val Ala His Cys Ser Ala Leu Ile Tyr Gly Glu Asp Phe Leu
           115                 120                 125
   Glu Ala Val Thr Asp Val Ala Lys Asp Leu Pro Ala Asn Leu Thr Leu
   130 135 140
   Phe Gln Phe Asn Asn Glu Asn Asn Asn Ser Glu Thr Glu Lys Asn Ile
   145 150 155 160
   Pro Gln Ala Lys Asn Leu Asn Ala Leu Leu Thr Thr Ala Ser Tyr Glu
   165 170 175
   Lys Pro Asn Lys Thr Gln Val Asn His His Asp Lys Leu Val Tyr Ile
   180 185 190
   Tyr Thr Ser Gly Thr Thr Gly Leu Pro Lys Ala Ala Val Ile Ser His
   195 200 205
   Ser Arg Tyr Leu Phe Ile Ala Ala Gly Ile His Tyr Thr Met Gly Phe
   210 215 220
   Gln Glu Glu Asp Ile Phe Tyr Thr Pro Leu Pro Leu Tyr His Thr Ala
   225 230 235 240
   Gly Gly Ile Met Cys Met Gly Gln Ser Val Leu Phe Gly Ser Thr Val
   245 250 255
   Ser Ile Arg Lys Lys Phe Ser Ala Ser Asn Tyr Phe Ala Asp Cys Ala
   260 265 270
   Lys Tyr Asn Ala Thr Ile Gly Gln Tyr Ile Gly Glu Met Ala Arg Tyr
   275 280 285
   Ile Leu Ala Thr Lys Pro Ser Glu Tyr Asp Gln Lys His Arg Val Arg
   290 295 300
   Leu Val Phe Gly Asn Gly Leu Arg Pro Gln Ile Trp Pro Gln Phe Val
   305 310 315 320
   Gln Arg Phe Asn Ile Ala Lys Val Gly Glu Phe Tyr Gly Ala Thr Glu
   325 330 335
   Gly Asn Ala Asn Ile Met Asn His Asp Asn Thr Val Gly Ala Ile Gly
   340 345 350
   Phe Val Ser Arg Ile Leu Pro Lys Ile Tyr Pro Ile Ser Ile Ile Arg
   355 360 365
   Ala Asp Pro Asp Thr Gly Glu Pro Ile Arg Asp Arg Asn Gly Leu Cys
   370 375 380
   Gln Leu Cys Ala Pro Asn Glu Pro Gly Val Phe Ile Gly Lys Ile Val
   385 390 395 400
   Lys Gly Asn Pro Ser Arg Glu Phe Leu Gly Tyr Val Asp Glu Lys Ala
   405 410 415
   Ser Ala Lys Lys Ile Val Lys Asp Val Phe Lys His Gly Asp Met Ala
   420 425 430
   Phe Ile Ser Gly Asp Leu Leu Val Ala Asp Glu Lys Gly Tyr Leu Tyr
   435 440 445
   Phe Lys Asp Arg Thr Gly Asp Thr Phe Arg Trp Lys Gly Glu Asn Val
   450 455 460
   Ser Thr Ser Glu Val Glu Ala Gln Val Ser Asn Val Ala Gly Tyr Lys
   465 470 475 480
   Asp Thr Val Val Tyr Gly Val Thr Ile Pro His Thr Glu Gly Arg Ala
   485 490 495
   Gly Met Ala Ala Ile Tyr Asp Pro Glu Arg Glu Leu Asp Leu Asp Val
   500 505 510
   Phe Ala Ala Ser Leu Ala Lys Val Leu Pro Ala Tyr Ala Arg Pro Gln
   515 520 525
   Ile Ile Arg Leu Leu Thr Lys Val Asp Leu Thr Gly Thr Phe Lys Leu
   530 535 540
   Arg Lys Val Asp Leu Gln Lys Glu Gly Tyr Asp Pro Asn Ala Ile Lys
   545 550 555 560
   Asp Ala Leu Tyr Tyr Gln Thr Ser Lys Gly Arg Tyr Glu Leu Leu Thr
   565 570 575
   Pro Gln Val Tyr Asp Gln Val Gln Arg Asn Glu Ile Arg Phe
                   580                 585                 590
<210> 76
   <211> 173
   <212> DNA
   <213> Danio rerio
<400> 76
   agtgtagata ccacaggaac gtttaaaatc cagaagacca gactgcaaag ggaaggatac      60
   gatccacggc tcacaactga ccagatctac ttcctaaact ccagagcagg gcgttacgag     120
   cttgtcaacg aggagctgta caatgcattt gaacaagggc aggatttccc ttt            173
<210> 77
   <211> 57
   <212> PRT
   <213> Danio rerio
<400> 77
   Ser Val Asp Thr Thr Gly Thr Phe Lys Ile Gln Lys Thr Arg Leu Gln
    1              5                   10                  15
   Arg Glu Gly Tyr Asp Pro Arg Leu Thr Thr Asp Gln Ile Tyr Phe Leu
               20                  25                  30
   Asn Ser Arg Ala Gly Arg Tyr Glu Leu Val Asn Glu Glu Leu Tyr Asn
           35                  40                  45
   Ala Phe Glu Gln Gly Gln Asp Phe Pro
       50                  55
<210> 78
   <211> 1953
   <212> DNA
   <213> Caenorhabditis elegans
<400> 78
   atgaagctgg aggagcttgt gacagttatg cttctcacag tggctgtcat tgctcagaat      60
   cttccgattg gagtaatatt ggctggagtt cttattttat acatcacagt ggttcatgga     120
   gatttcattt atagaagtta tcttacgttg aatagggatt taacaggatt ggctctaatt     180
   attgaagtca aaatcgacct atggtggagg ttgcatcaga ataaaggaat ccatgaactg     240
   tttttggata ttgtgaaaaa gaatccaaat aagccggcga tgattgacat cgagacgaat     300
   acaacagaaa catacgcaga gttcaatgca cattgtaata gatatgccaa ttatttccag     360
   ggtcttggct atcgatccgg agacgttgtc gccttgtaca tggagaactc ggtcgagttt     420
   gtggccgcgt ggatgggact cgcaaaaatc ggagttgtaa cggcttggat caactcgaat     480
   ttgaaaagag agcaacttgt tcattgtatc actgcgagca agacaaaggc gattatcaca     540
   agtgtaacac ttcagaatat tatgcttgat gctatcgatc agaagctgtt tgatgttgag     600
   ggaattgagg tttactctgt cggagagccc aagaagaatt ctggattcaa gaatctcaag     660
   aagaagttgg atgctcaaat tactacggaa ccaaagaccc ttgacatagt agattttaaa     720
   agtattcttt gcttcatcta tacaagtggt actactggaa tgccaaaagc cgctgtcatg     780
   aagcacttca gatattactc gattgccgtt ggagccgcaa aatcattcgg aatccgccct     840
   tctgatcgta tgtacgtctc gatgccaatt tatcacactg cagctggaat tcttggagtt     900
   gggcaagctc tgttgggtgg atcatcgtgt gtcattagaa aaaaattctc ggctagcaac     960
   ttttggaggg attgtgtaaa gtatgattgt acagtttcac aatacattgg agagatttgt    1020
   cggtacttgt tggctcagcc agttgtggaa gaggaatcca ggcatagaat gagattgttg    1080
   gttggaaacg gactccgtgc tgaaatctgg caaccatttg tagatcgatt ccgtgtcaga    1140
   attggagaac tttatggttc aactgaagga acttcatctc tcgtgaacat tgacggacat    1200
   gtcggagctt gcggattctt gccaatatcc ccattaacaa agaaaatgca tccggttcga    1260
   ttaattaagg ttgatgatgt cactggagaa gcaatccgaa cttccgatgg actttgcatt    1320
   gcatgtaatc caggagagtc tggagcaatg gtgtcgacga tcagaaaaaa taatccatta    1380
   ttgcaattcg agggatatct gaataagaag gaaacgaata aaaagattat cagagatgtc    1440
   ttcgcaaagg gagatagttg ctttttgact ggagatcttc ttcattggga tcgtcttggt    1500
   tatgtatatt tcaaggatcg tactggagat actttccgtt ggaagggaga gaatgtgtcg    1560
   actactgaag tcgaggcaat tcttcatcca attactggat tgtctgatgc aactgtttat    1620
   ggtgtagagg ttcctcaaag agagggaaga gttggaatgg cgtcagttgt tcgagttgta    1680
   tcgcatgagg aagatgaaac tcaatttgtt catagagttg gagcaagact tgcctcttcg    1740
   cttaccagct acgcgattcc tcagtttatg cgaatttgtc aggatgttga gaaaacaggt    1800
   acattcaaac ttgtgaagac gaatctacaa cgattaggta tcatggatgc tccttcagat    1860
   tcaatttaca tctacaattc tgaaaatcgc aattttgtgc cgttcgacaa tgatttgagg    1920
   tgcaaggtct cactgggaag ttatccattt taa                                 1953
<210> 79
   <211> 650
   <212> PRT
   <213> Caenorhabditis elegans
<400> 79
   Met Lys Leu Glu Glu Leu Val Thr Val Met Leu Leu Thr Val Ala Val
   1 5 10 15
   Ile Ala Gln Asn Leu Pro Ile Gly Val Ile Leu Ala Gly Val Leu Ile
   20 25 30
   Leu Tyr Ile Thr Val Val His Gly Asp Phe Ile Tyr Arg Ser Tyr Leu
   35 40 45
   Thr Leu Asn Arg Asp Leu Thr Gly Leu Ala Leu Ile Ile Glu Val Lys
   50 55 60
   Ile Asp Leu Trp Trp Arg Leu His Gln Asn Lys Gly Ile His Glu Leu
   65 70 75 80
   Phe Leu Asp Ile Val Lys Lys Asn Pro Asn Lys Pro Ala Met Ile Asp
   85 90 95
   Ile Glu Thr Asn Thr Thr Glu Thr Tyr Ala Glu Phe Asn Ala His Cys
   100 105 110
   Asn Arg Tyr Ala Asn Tyr Phe Gln Gly Leu Gly Tyr Arg Ser Gly Asp
   115 120 125
   Val Val Ala Leu Tyr Met Glu Asn Ser Val Glu Phe Val Ala Ala Trp
   130 135 140
   Met Gly Leu Ala Lys Ile Gly Val Val Thr Ala Trp Ile Asn Ser Asn
   145 150 155 160
   Leu Lys Arg Glu Gln Leu Val His Cys Ile Thr Ala Ser Lys Thr Lys
   165 170 175
   Ala Ile Ile Thr Ser Val Thr Leu Gln Asn Ile Met Leu Asp Ala Ile
   180 185 190
   Asp Gln Lys Leu Phe Asp Val Glu Gly Ile Glu Val Tyr Ser Val Gly
   195 200 205
   Glu Pro Lys Lys Asn Ser Gly Phe Lys Asn Leu Lys Lys Lys Leu Asp
   210 215 220
   Ala Gln Ile Thr Thr Glu Pro Lys Thr Leu Asp Ile Val Asp Phe Lys
   225 230 235 240
   Ser Ile Leu Cys Phe Ile Tyr Thr Ser Gly Thr Thr Gly Met Pro Lys
   245 250 255
   Ala Ala Val Met Lys His Phe Arg Tyr Tyr Ser Ile Ala Val Gly Ala
   260 265 270
   Ala Lys Ser Phe Gly Ile Arg Pro Ser Asp Arg Met Tyr Val Ser Met
   275 280 285
   Pro Ile Tyr His Thr Ala Ala Gly Ile Leu Gly Val Gly Gln Ala Leu
   290 295 300
   Leu Gly Gly Ser Ser Cys Val Ile Arg Lys Lys Phe Ser Ala Ser Asn
   305 310 315 320
   Phe Trp Arg Asp Cys Val Lys Tyr Asp Cys Thr Val Ser Gln Tyr Ile
                   325                 330                 335
   Gly Glu Ile Cys Arg Tyr Leu Leu Ala Gln Pro Val Val Glu Glu Glu
               340                 345                 350
   Ser Arg His Arg Met Arg Leu Leu Val Gly Asn Gly Leu Arg Ala Glu
           355                 360                 365
   Ile Trp Gln Pro Phe Val Asp Arg Phe Arg Val Arg Ile Gly Glu Leu
       370                 375                 380
   Tyr Gly Ser Thr Glu Gly Thr Ser Ser Leu Val Asn Ile Asp Gly His
   385                 390                 395                 400
   Val Gly Ala Cys Gly Phe Leu Pro Ile Ser Pro Leu Thr Lys Lys Met
                   405                 410                 415
   His Pro Val Arg Leu Ile Lys Val Asp Asp Val Thr Gly Glu Ala Ile
               420                 425                 430
   Arg Thr Ser Asp Gly Leu Cys Ile Ala Cys Asn Pro Gly Glu Ser Gly
           435                 440                 445
   Ala Met Val Ser Thr Ile Arg Lys Asn Asn Pro Leu Leu Gln Phe Glu
       450                 455                 460
   Gly Tyr Leu Asn Lys Lys Glu Thr Asn Lys Lys Ile Ile Arg Asp Val
   465                 470                 475                 480
   Phe Ala Lys Gly Asp Ser Cys Phe Leu Thr Gly Asp Leu Leu His Trp
                   485                 490                 495
   Asp Arg Leu Gly Tyr Val Tyr Phe Lys Asp Arg Thr Gly Asp Thr Phe
               500                 505                 510
   Arg Trp Lys Gly Glu Asn Val Ser Thr Thr Glu Val Glu Ala Ile Leu
           515                 520                 525
   His Pro Ile Thr Gly Leu Ser Asp Ala Thr Val Tyr Gly Val Glu Val
       530                 535                 540
   Pro Gln Arg Glu Gly Arg Val Gly Met Ala Ser Val Val Arg Val Val
   545                 550                 555                 560
   Ser His Glu Glu Asp Glu Thr Gln Phe Val His Arg Val Gly Ala Arg
                   565                 570                 575
   Leu Ala Ser Ser Leu Thr Ser Tyr Ala Ile Pro Gln Phe Met Arg Ile
               580                 585                 590
   Cys Gln Asp Val Glu Lys Thr Gly Thr Phe Lys Leu Val Lys Thr Asn
           595                 600                 605
   Leu Gln Arg Leu Gly Ile Met Asp Ala Pro Ser Asp Ser Ile Tyr Ile
       610                 615                 620
   Tyr Asn Ser Glu Asn Arg Asn Phe Val Pro Phe Asp Asn Asp Leu Arg
   625                 630                 635                 640
   Cys Lys Val Ser Leu Gly Ser Tyr Pro Phe
                   645                 650
<210> 80
   <211> 1968
   <212> DNA
   <213> Caenorhabditis elegans
<400> 80
   atgagggaaa tgccggacag tcccaagttt gcgttagtca cgtttgttgt gtatgcagtg      60
   gttttgtaca atgtcaacag cgttttctgg aaatttgtat tcatcggata tgttgtattt     120
   aggctgcttc gcactgattt tggaagaaga gcacttgcca cgttacctag agattttgcg     180
   ggactgaagc tcttaatatc ggttaagtcg acaattcgtg gcttgttcaa gaaagatcgc     240
   ccaattcatg aaatcttttt gaatcaggtg aaacagcatc caaacaaagt ggcgattatt     300
   gaaattgaaa gtggtaggca gttgacgtat caagaattga atgcgttagc taatcagtat     360
   gctaaccttt acgtgagtga aggttacaaa atgggcgacg ttgtcgcttt gtttatggaa     420
   aatagcatcg acttctttgc aatttggctg ggactttcca agattggagt cgtgtcggcg     480
   ttcatcaact caaacttgaa gttggagcca ttggcacatt cgattaatgt ttcgaagtgc     540
   aaatcatgca ttaccaatat caatctgttg ccgatgttca aagccgctcg tgaaaagaat     600
   ctgatcagtg acgagatcca cgtgtttctg gctggaactc aggttgatgg acgtcataga     660
   agtcttcagc aagatctcca tcttttctct gaggatgaac ctccagttat agacggactc     720
   aattttagaa gcgttctgtg ttatatttac acttccggta ctaccggaaa tccaaagcca     780
   gccgtcatta aacacttccg ttacttctgg attgcgatgg gagcaggaaa agcatttgga     840
   attaataagt cagacgttgt gtacattacg atgccaatgt atcactctgc cgccggtatc     900
   atgggtattg gatcattaat tgcattcggg tcgaccgctg ttattaggaa aaagttttcg     960
   gcaagcaact tctggaaaga ttgcgtcaag tacaacgtca cagcgacaca gtacattgga    1020
   gaaatctgca ggtatcttct ggcagcgaat ccatgtcctg aagagaaaca acacaacgtg    1080
   cgattgatgt ggggaaatgg tttgagagga caaatttgga aagagtttgt aggaagattt    1140
   ggaattaaga aaattggaga gttgtacggc tcaacagaag gaaactccaa tattgttaac    1200
   gtggataacc atgttggagc ttgtggattc atgccaattt atccccatat tggatccctc    1260
   tacccagttc gacttattaa ggttgataga gccactggag agcttgaacg tgataagaac    1320
   ggactctgtg tgccgtgtgt gcctggtgaa actggggaaa tggttggcgt tatcaaggag    1380
   aaagatattc ttctaaagtt cgaaggatat gtcagcgaag gggatactgc aaagaaaatc     1440
   tacagagatg tgttcaagca tggagataag gtgtttgcaa gtggagatat tcttcattgg    1500
   gatgatcttg gatacttgta ctttgtggac cgttgtggag acactttccg ttggaaaggg    1560
   gagaacgtgt caactactga agttgaggga attcttcagc ctgtgatgga tgtggaagat    1620
   gcaactgttt atggagtcac tgtcggtaaa atggaggggc gtgccggaat ggctggtatt    1680
   gtcgtcaagg atggaacgga tgttgagaaa ttcatcgccg atattacttc tcgactgacc    1740
   gaaaatctgg cgtcttacgc aatccctgtt ttcattcggc tgtgcaagga agttgatcga    1800
   accggaacct tcaaactcaa gaagactgat cttcaaaaac aaggttacga cctggttgct    1860
   tgtaaaggag acccaattta ctactggtca gctgcagaaa aatcctacaa accactgact    1920
   gacaaaatgc aacaggatat tgacactggt gtttatgatc gcatttaa                 1968
<210> 81
   <211> 655
   <212> PRT
   <213> Caenorhabditis elegans
<400> 81
   Met Arg Glu Met Pro Asp Ser Pro Lys Phe Ala Leu Val Thr Phe Val
    1               5                  10                  15
   Val Tyr Ala Val Val Leu Tyr Asn Val Asn Ser Val Phe Trp Lys Phe
               20                  25                  30
   Val Phe Ile Gly Tyr Val Val Phe Arg Leu Leu Arg Thr Asp Phe Gly
           35                  40                  45
   Arg Arg Ala Leu Ala Thr Leu Pro Arg Asp Phe Ala Gly Leu Lys Leu
       50                  55                  60
   Leu Ile Ser Val Lys Ser Thr Ile Arg Gly Leu Phe Lys Lys Asp Arg
   65                  70                  75                  80
   Pro Ile His Glu Ile Phe Leu Asn Gln Val Lys Gln His Pro Asn Lys
                   85                  90                  95
   Val Ala Ile Ile Glu Ile Glu Ser Gly Arg Gln Leu Thr Tyr Gln Glu
               100                 105                 110
   Leu Asn Ala Leu Ala Asn Gln Tyr Ala Asn Leu Tyr Val Ser Glu Gly
           115                 120                 125
   Tyr Lys Met Gly Asp Val Val Ala Leu Phe Met Glu Asn Ser Ile Asp
       130                 135                 140
   Phe Phe Ala Ile Trp Leu Gly Leu Ser Lys Ile Gly Val Val Ser Ala
   145                 150                 155                 160
   Phe Ile Asn Ser Asn Leu Lys Leu Glu Pro Leu Ala His Ser Ile Asn
                   165                 170                 175
   Val Ser Lys Cys Lys Ser Cys Ile Thr Asn Ile Asn Leu Leu Pro Met
               180                 185                 190
   Phe Lys Ala Ala Arg Glu Lys Asn Leu Ile Ser Asp Glu Ile His Val
           195                 200                 205
   Phe Leu Ala Gly Thr Gln Val Asp Gly Arg His Arg Ser Leu Gln Gln
       210                 215                 220
   Asp Leu His Leu Phe Ser Glu Asp Glu Pro Pro Val Ile Asp Gly Leu
   225                 230                 235                 240
   Asn Phe Arg Ser Val Leu Cys Tyr Ile Tyr Thr Ser Gly Thr Thr Gly
                   245                 250                 255
   Asn Pro Lys Pro Ala Val Ile Lys His Phe Arg Tyr Phe Trp Ile Ala
               260                 265                 270
   Met Gly Ala Gly Lys Ala Phe Gly Ile Asn Lys Ser Asp Val Val Tyr
           275                 280                 285
   Ile Thr Met Pro Met Tyr His Ser Ala Ala Gly Ile Met Gly Ile Gly
       290                 295                 300
   Ser Leu Ile Ala Phe Gly Ser Thr Ala Val Ile Arg Lys Lys Phe Ser
   305                 310                 315                 320
   Ala Ser Asn Phe Trp Lys Asp Cys Val Lys Tyr Asn Val Thr Ala Thr
                   325                 330                 335
   Gln Tyr Ile Gly Glu Ile Cys Arg Tyr Leu Leu Ala Ala Asn Pro Cys
               340                 345                 350
   Pro Glu Glu Lys Gln His Asn Val Arg Leu Met Trp Gly Asn Gly Leu
           355                 360                 365
   Arg Gly Gln Ile Trp Lys Glu Phe Val Gly Arg Phe Gly Ile Lys Lys
       370                 375                 380
   Ile Gly Glu Leu Tyr Gly Ser Thr Glu Gly Asn Ser Asn Ile Val Asn
   385                 390                 395                 400
   Val Asp Asn His Val Gly Ala Cys Gly Phe Met Pro Ile Tyr Pro His
                   405                 410                 415
   Ile Gly Ser Leu Tyr Pro Val Arg Leu Ile Lys Val Asp Arg Ala Thr
               420                 425                 430
   Gly Glu Leu Glu Arg Asp Lys Asn Gly Leu Cys Val Pro Cys Val Pro
           435                 440                 445
   Gly Glu Thr Gly Glu Met Val Gly Val Ile Lys Glu Lys Asp Ile Leu
       450                 455                 460
   Leu Lys Phe Glu Gly Tyr Val Ser Glu Gly Asp Thr Ala Lys Lys Ile
   465                 470                 475                 480
   Tyr Arg Asp Val Phe Lys His Gly Asp Lys Val Phe Ala Ser Gly Asp
                   485                 490                 495
   Ile Leu His Trp Asp Asp Leu Gly Tyr Leu Tyr Phe Val Asp Arg Cys
               500                 505                 510
   Gly Asp Thr Phe Arg Trp Lys Gly Glu Asn Val Ser Thr Thr Glu Val
           515                 520                 525
   Glu Gly Ile Leu Gln Pro Val Met Asp Val Glu Asp Ala Thr Val Tyr
       530                 535                 540
   Gly Val Thr Val Gly Lys Met Glu Gly Arg Ala Gly Met Ala Gly Ile
   545                 550                 555                 560
   Val Val Lys Asp Gly Thr Asp Val Glu Lys Phe Ile Ala Asp Ile Thr
                   565                 570                 575
   Ser Arg Leu Thr Glu Asn Leu Ala Ser Tyr Ala Ile Pro Val Phe Ile
               580                 585                 590
   Arg Leu Cys Lys Glu Val Asp Arg Thr Gly Thr Phe Lys Leu Lys Lys
           595                 600                 605
   Thr Asp Leu Gln Lys Gln Gly Tyr Asp Leu Val Ala Cys Lys Gly Asp
       610                 615                 620
   Pro Ile Tyr Tyr Trp Ser Ala Ala Glu Lys Ser Tyr Lys Pro Leu Thr
   625                 630                 635                 640
   Asp Lys Met Gln Gln Asp Ile Asp Thr Gly Val Tyr Asp Arg Ile
   645 650 655
<210> 82
   <211> 1932
   <212> DNA
   <213> Cochliobolu heterostrophus
<400> 82
   atggcgtgta tgcatcaggc tcagctatac aatgatctag aggaattgct aactggtcca      60
   tcagtaccca tcgttgctgg agctgctgga gctgcagctc tcactgccta cattaacgcc     120
   aaataccaca tagcccatga tctcaagacc ctcggtggtg gattgacaca atcgtccgaa     180
   gcgattgatt tcataaaccg ccgcgtcgca caaaagcgcg tcctcacgca ccacatcttc     240
   caggagcagg tccaaaaaca atcaaatcat ccctttctta tctttgaggg caagacatgg     300
   tcttacaagg agttctctga ggcatacacg agggtcgcga actggctgat tgatgagctg     360
   gacgtacaag taggggagat ggtcgcaatt gatggcggaa atagtgcaga gcacctgatg     420
   ctttggcttg cacttgatgc aatcggtgcg gctacgagtt ttttgaactg gaacctgaca     480
   ggggcagggt taattcattg cataaagcta tgcgaatgtc gattcgttat cgcagacatc     540
   gatattaaag cgaacattga accgtgccgt ggcgaactgg aggagacggg catcaacatt     600
   cactactatg acccatcctt catctcatcg ctaccgaata acacgccaat tcccgacagc     660
   cgcactgaga acattgaatt agattcagta cgaggactga tatacacatc tggaaccact     720
   ggtctaccta aaggcgtgtt tataagcact ggccgcgagc ttaggactga ctggtcgatt     780
   tcaaagtatc taaatctcaa gcccacggat cgaatgtata catgtatgcc gctctaccat     840
   gccgctgcac acagcctctg tacagcatca gttattcatg gtggaggtac cgtggtattg     900
   agcaggaaat tctcacacaa gaagttctgg cctgaagttg tggcttcgga agcaaatatc     960
   attcagtacg ttggtgaatt aggtcgatat ctcctgaatg gtccaaagag tccttacgac    1020
   agggcccata aagtccagat ggcgtggggc aatggcatgc gtccagacgt gtgggaagcg    1080
   tttcgtgaac gcttcaacat accaattatt catgagctct atgccgcaac cgatgggctc    1140
   gggtcaatga ccaatcgtaa cgcgggccct tttacagcaa actgtattgc gctgcgaggg    1200
   ctgatctggc actggaaatt tcgaaatcag gaagtgctgg tcaagatgga tctcgatact    1260
   gatgagatca tgagagatcg caatgggttt gcgatacgat gcgctgtcaa tgaacctgga    1320
   cagatgcttt ttcggctgac acccgaaact ctggctggtg caccaagcta ctacaacaac    1380
   gaaacggcca cacagagcag gcggattaca gatgtgtttc aaaagggtga cctgtggttc    1440
   aagtccggtg acatgctacg gcaagacgcc gaaggccgcg tctactttgt cgatcgacta    1500
   ggcgatacgt tccgctggaa atccgaaaac gtttctacca atgaagtcgc ggacgtgatg    1560
   ggcacatttc ctcagattgc tgaaacgaat gtatacggtg tccttgtgcc gggtaacgat    1620
   ggtcgagtgc gcagcctcaa ttgtcatggc agacggcgtg acagagtcga cattcgcttc    1680
   gctgcccttg caaagcacgc ccgagatcgg ttaccgggtt atgctgtacc actgtttctg    1740
   agggtaactc cagcacttga atatacgggc acattaaaga ttcagaaagg acgcctcaag    1800
   caggaaggta tagacccaga taagatttcc ggcgaagata agttatactg gctgccgcct    1860
   ggtagcgata tatatttacc atttggaaag atggagtggc agggaattgt agataagcgt    1920
   atacggctgt ga                                                        1932
<210> 83
   <211> 643
   <212> PRT
   <213> Cochliobolu heterostrophus
<400> 83
   Met Ala Cys Met His Gln Ala Gln Leu Tyr Asn Asp Leu Glu Glu Leu
    1               5                  10                  15
   Leu Thr Gly Pro Ser Val Pro Ile Val Ala Gly Ala Ala Gly Ala Ala
               20                  25                  30
   Ala Leu Thr Ala Tyr Ile Asn Ala Lys Tyr His Ile Ala His Asp Leu
           35                  40                  45
   Lys Thr Leu Gly Gly Gly Leu Thr Gln Ser Ser Glu Ala Ile Asp Phe
       50                  55                  60
   Ile Asn Arg Arg Val Ala Gln Lys Arg Val Leu Thr His His Ile Phe
   65                  70                  75                  80
   Gln Glu Gln Val Gln Lys Gln Ser Asn His Pro Phe Leu Ile Phe Glu
                   85                  90                  95
   Gly Lys Thr Trp Ser Tyr Lys Glu Phe Ser Glu Ala Tyr Thr Arg Val
               100                 105                 110
   Ala Asn Trp Leu Ile Asp Glu Leu Asp Val Gln Val Gly Glu Met Val
           115                 120                 125
   Ala Ile Asp Gly Gly Asn Ser Ala Glu His Leu Met Leu Trp Leu Ala
       130                 135                 140
   Leu Asp Ala Ile Gly Ala Ala Thr Ser Phe Leu Asn Trp Asn Leu Thr
   145                 150                 155                 160
   Gly Ala Gly Leu Ile His Cys Ile Lys Leu Cys Glu Cys Arg Phe Val
                   165                 170                 175
   Ile Ala Asp Ile Asp Ile Lys Ala Asn Ile Glu Pro Cys Arg Gly Glu
               180                 185                 190
   Leu Glu Glu Thr Gly Ile Asn Ile His Tyr Tyr Asp Pro Ser Phe Ile
           195                 200                 205
   Ser Ser Leu Pro Asn Asn Thr Pro Ile Pro Asp Ser Arg Thr Glu Asn
       210                 215                 220
   Ile Glu Leu Asp Ser Val Arg Gly Leu Ile Tyr Thr Ser Gly Thr Thr
   225                 230                 235                 240
   Gly Leu Pro Lys Gly Val Phe Ile Ser Thr Gly Arg Glu Leu Arg Thr
                   245                 250                 255
   Asp Trp Ser Ile Ser Lys Tyr Leu Asn Leu Lys Pro Thr Asp Arg Met
               260                 265                 270
   Tyr Thr Cys Met Pro Leu Tyr His Ala Ala Ala His Ser Leu Cys Thr
           275                 280                 285
   Ala Ser Val Ile His Gly Gly Gly Thr Val Val Leu Ser Arg Lys Phe
       290                 295                 300
   Ser His Lys Lys Phe Trp Pro Glu Val Val Ala Ser Glu Ala Asn Ile
   305                 310                 315                 320
   Ile Gln Tyr Val Gly Glu Leu Gly Arg Tyr Leu Leu Asn Gly Pro Lys
                   325                 330                 335
   Ser Pro Tyr Asp Arg Ala His Lys Val Gln Met Ala Trp Gly Asn Gly
               340                 345                 350
   Met Arg Pro Asp Val Trp Glu Ala Phe Arg Glu Arg Phe Asn Ile Pro
           355                 360                 365
   Ile Ile His Glu Leu Tyr Ala Ala Thr Asp Gly Leu Gly Ser Met Thr
       370                 375                 380
   Asn Arg Asn Ala Gly Pro Phe Thr Ala Asn Cys Ile Ala Leu Arg Gly
   385                 390                 395                 400
   Leu Ile Trp His Trp Lys Phe Arg Asn Gln Glu Val Leu Val Lys Met
                   405                 410                 415
   Asp Leu Asp Thr Asp Glu Ile Met Arg Asp Arg Asn Gly Phe Ala Ile
               420                 425                 430
   Arg Cys Ala Val Asn Glu Pro Gly Gln Met Leu Phe Arg Leu Thr Pro
           435                 440                 445
   Glu Thr Leu Ala Gly Ala Pro Ser Tyr Tyr Asn Asn Glu Thr Ala Thr
       450                 455                 460
   Gln Ser Arg Arg Ile Thr Asp Val Phe Gln Lys Gly Asp Leu Trp Phe
   465                 470                 475                 480
   Lys Ser Gly Asp Met Leu Arg Gln Asp Ala Glu Gly Arg Val Tyr Phe
                   485                 490                 495
   Val Asp Arg Leu Gly Asp Thr Phe Arg Trp Lys Ser Glu Asn Val Ser
               500                 505                 510
   Thr Asn Glu Val Ala Asp Val Met Gly Thr Phe Pro Gln Ile Ala Glu
           515                 520                 525
   Thr Asn Val Tyr Gly Val Leu Val Pro Gly Asn Asp Gly Arg Val Arg
       530                 535                 540
   Ser Leu Asn Cys His Gly Arg Arg Arg Asp Arg Val Asp Ile Arg Phe
   545                 550                 555                 560
   Ala Ala Leu Ala Lys His Ala Arg Asp Arg Leu Pro Gly Tyr Ala Val
                   565                 570                 575
   Pro Leu Phe Leu Arg Val Thr Pro Ala Leu Glu Tyr Thr Gly Thr Leu
               580                 585                 590
   Lys Ile Gln Lys Gly Arg Leu Lys Gln Glu Gly Ile Asp Pro Asp Lys
           595                 600                 605
   Ile Ser Gly Glu Asp Lys Leu Tyr Trp Leu Pro Pro Gly Ser Asp Ile
       610                 615                 620
   Tyr Leu Pro Phe Gly Lys Met Glu Trp Gln Gly Ile Val Asp Lys Arg
   625                 630                 635                 640
   Ile Arg Leu
<210> 84
   <211> 597
   <212> DNA
   <213> Aspergillus nidulans
<400> 84
   ctttaccatt catcagcttc attctgcatt tttagcttga cggcagccgg gtctacgctg      60
   atcatcggcc gcaagttctc cgcgagaaac ttcataaagg aagcgcgcga gaacgacgcc     120
   acggtcatcc agtacgtggg tgagaccttg cgatatctgc tcgccacccc cggtgaaacc     180
   gatccagtta ctggcgaaga cctggacaaa aagcacaata ttcgagcagt atacggcaac     240
   gggctacggc cggatatctg gaaccgcttc aaggagcgct tcaacgtgcc gacggttgcc     300
   gaattttatg ctgcaaccga gagcccaggc ggaacatgga actattcaac aaatgacttc     360
   actgccggag ccattgggca cactggcgtg cttagtggat ggcttcttgg acgcggcctt     420
   actattgtcg aggtggacca ggaatcacag gaaccatggc gcgatcccca aaccgggttc     480
   tgcaagccgg tcccgcgagg cgaagcaggc gagctcctgt atgccattga tccggccgac     540
   ccgggcgaga ccttccaggg ctactaccgc aactccttta gagcacactg gcggccg        597
<210> 85
   <211> 199
   <212> PRT
   <213> Aspergillus nidulans
<400> 85
   Leu Tyr His Ser Ser Ala Ser Phe Cys Ile Phe Ser Leu Thr Ala Ala
    1               5                  10                  15
   Gly Ser Thr Leu Ile Ile Gly Arg Lys Phe Ser Ala Arg Asn Phe Ile
               20                  25                  30
   Lys Glu Ala Arg Glu Asn Asp Ala Thr Val Ile Gln Tyr Val Gly Glu
           35                  40                  45
   Thr Leu Arg Tyr Leu Leu Ala Thr Pro Gly Glu Thr Asp Pro Val Thr
       50                  55                  60
   Gly Glu Asp Leu Asp Lys Lys His Asn Ile Arg Ala Val Tyr Gly Asn
   65                  70                  75                  80
   Gly Leu Arg Pro Asp Ile Trp Asn Arg Phe Lys Glu Arg Phe Asn Val
                   85                  90                  95
   Pro Thr Val Ala Glu Phe Tyr Ala Ala Thr Glu Ser Pro Gly Gly Thr
               100                 105                 110
   Trp Asn Tyr Ser Thr Asn Asp Phe Thr Ala Gly Ala Ile Gly His Thr
           115                 120                 125
   Gly Val Leu Ser Gly Trp Leu Leu Gly Arg Gly Leu Thr Ile Val Glu
       130                 135                 140
   Val Asp Gln Glu Ser Gln Glu Pro Trp Arg Asp Pro Gln Thr Gly Phe
   145                 150                 155                 160
   Cys Lys Pro Val Pro Arg Gly Glu Ala Gly Glu Leu Leu Tyr Ala Ile
                   165                 170                 175
   Asp Pro Ala Asp Pro Gly Glu Thr Phe Gln Gly Tyr Tyr Arg Asn Ser
               180                 185                 190
   Phe Arg Ala His Trp Arg Pro
           195
<210> 86
   <211> 522
   <212> DNA
   <213> Magnaporthe grisea
<220>
   <221> misc_feature
   <222> (1) ... (522)
   <223> n = A,T,C or G
<400> 86
   gcaaaggccg acgcgtggct gcggacgggt aacgtgatca gggcggacaa cgaagggcga      60
   ctcttcttcc acgaccggat cggagacacg ttccgatgga agggagagac ngtcagcaca     120
   caagaggtca gtttggtgct cggacgacac gactcaatca aggaggccaa cgtgtacggc     180
   gtgacggtgc cgaaccacga cgggcgggcc ggctgcgctg cgctcacgct atcagacgct     240
   ctggcgactg aaaagaagct gggcgatgag ctgctaaagg gattggctac tcactcgtcg     300
   acttcgcttc ccaagtttgc ggtgccgcag ttcctacggg tggtgcgcgg cgagatgcag     360
   tcaacgggca ccaacaagca acagaagcac gacctgaggg tgcagggtgt agagccgggc     420
   aaggtggcgg tagacgaggt gtactggttg cggggaggga catatgtacc attcggaaca     480
   gaggattggg atgggttgaa gaagggtctt gtgaagttgt ga                        522
<210> 87
   <211> 173
   <212> PRT
   <213> Magnaporthe grisea
<400> 87
   Ala Lys Ala Asp Ala Trp Leu Arg Thr Gly Asn Val Ile Arg Ala Asp
    1               5                  10                  15
   Asn Glu Gly Arg Leu Phe Phe His Asp Arg Ile Gly Asp Thr Phe Arg
               20                  25                  30
   Trp Lys Gly Glu Thr Val Ser Thr Gln Glu Val Ser Leu Val Leu Gly
           35                  40                  45
   Arg His Asp Ser Ile Lys Glu Ala Asn Val Tyr Gly Val Thr Val Pro
       50                  55                  60
   Asn His Asp Gly Arg Ala Gly Cys Ala Ala Leu Thr Leu Ser Asp Ala
   65                  70                  75                  80
   Leu Ala Thr Glu Lys Lys Leu Gly Asp Glu Leu Leu Lys Gly Leu Ala
                   85                  90                  95
   Thr His Ser Ser Thr Ser Leu Pro Lys Phe Ala Val Pro Gln Phe Leu
               100                 105                 110
   Arg Val Val Arg Gly Glu Met Gln Ser Thr Gly Thr Asn Lys Gln Gln
           115                 120                 125
   Lys His Asp Leu Arg Val Gln Gly Val Glu Pro Gly Lys Val Gly Val
       130                 135                 140
   Asp Glu Val Tyr Trp Leu Arg Gly Gly Thr Tyr Val Pro Phe Gly Thr
   145                 150                 155                 160
   Glu Asp Trp Asp Gly Leu Lys Lys Gly Leu Val Lys Leu
                   165                 170
<210> 88
   <211> 1872
   <212> DNA
   <213> Saccharomyces cerevisiae
<400> 88
   atgtctccca tacaggttgt tgtctttgcc ttgtcaagga ttttcctgct attattcaga      60
   cttatcaagc taattataac ccctatccag aaatcactgg gttatctatt tggtaattat     120
   tttgatgaat tagaccgtaa atatagatac aaggaggatt ggtatattat tccttacttt     180
   ttgaaaagcg tgttttgtta tatcattgat gtgagaagac ataggtttca aaactggtac     240
   ttatttatta aacaggtcca acaaaatggt gaccatttag cgattagtta cacccgtccc     300
   atggccgaaa agggagaatt tcaactcgaa acctttacgt atattgaaac ttataacata     360
   gtgttgagat tgtctcatat tttgcatttt gattataacg ttcaggccgg tgactacgtg     420
   gcaatcgatt gtactaataa acctcttttc gtatttttat ggctttcttt gtggaacatt     480
   ggggctattc cagctttttt aaactataat actaaaggca ctccgctggt tcactcccta     540
   aagatttcca atattacgca ggtatttatt gaccctgatg ccagtaatcc gatcagagaa     600
   tcggaagaag aaatcaaaaa cgcacttcct gatgttaaat taaactatct tgaagaacaa     660
   gacttaatgc atgaactttt aaattcgcaa tcaccggaat tcttacaaca agacaacgtt     720
   aggacaccac taggcttgac cgattttaaa ccctctatgt taatttatac atctggaacc     780
   actggtttgc ctaaatccgc tattatgtct tggagaaaat cctccgtagg ttgtcaagtt     840
   tttggtcatg ttttacatat gactaatgaa agcactgtgt tcacagccat gccattgttc     900
   cattcaactg ctgccttatt aggtgcgtgc gccattctat ctcacggtgg ttgccttgcg     960
   ttatcgcata aattttctgc cagtacattt tggaagcaag tttatttaac aggagccacg    1020
   cacatccaat atgtcggaga agtctgtaga tacctgttac atacgccaat ttctaagtat    1080
   gaaaagatgc ataaggtgaa ggttgcttat ggtaacgggc tgagacctga catctggcag    1140
   gacttcagga agaggttcaa catagaagtt attggtgaat tctatgccgc aactgaagct    1200
   ccttttgcta caactacctt ccagaaaggt gactttggaa ttggcgcatg taggaactat    1260
   ggtactataa ttcaatggtt tttgtcattc caacaaacat tggtaaggat ggacccaaat    1320
   gacgattccg ttatatatag aaattccaag ggtttctgcg aagtggcccc tgttggcgaa    1380
   ccaggagaaa tgttaatgag aatctttttc cctaaaaaac cagaaacatc ttttcaaggt    1440
   tatcttggta atgccaagga aacaaagtcc aaagttgtga gggatgtctt cagacgtggc    1500
   gatgcttggt atagatgtgg agatttatta aaagcggacg aatatggatt atggtatttc    1560
   cttgatagaa tgggtgatac tttcagatgg aaatctgaaa atgtttccac tactgaagta    1620
   gaagatcagt tgacggccag taacaaagaa caatatgcac aagttctagt tgttggtatt    1680
   aaagtaccta aatatgaagg tagagctggt tttgcagtta ttaaactaac tgacaactct    1740
   cttgacatca ctgcaaagac caaattatta aatgattcct tgagccggtt aaatctaccg    1800
   tcttatgcta tgcccctatt tgttaaattt gttgatgaaa ttaaaatgac agataacctc    1860
   ataaaatttt ga                                                        1872
<210> 89
   <211> 623
   <212> PRT
   <213> Saccharomyces cerevisiae
<400> 89
   Met Ser Pro Ile Gln Val Val Val Phe Ala Leu Ser Arg Ile Phe Leu
   1               5                   10                  15
   Leu Leu Phe Arg Leu Ile Lys Leu Ile Ile Thr Pro Ile Gln Lys Ser
               20                  25                  30
   Leu Gly Tyr Leu Phe Gly Asn Tyr Phe Asp Glu Leu Asp Arg Lys Tyr
           35                  40                  45
   Arg Tyr Lys Glu Asp Trp Tyr Ile Ile Pro Tyr Phe Leu Lys Ser Val
       50                  55                  60
   Phe Cys Tyr Ile Ile Asp Val Arg Arg His Arg Phe Gln Asn Trp Tyr
   65                  70                  75                  80
   Leu Phe Ile Lys Gln Val Gln Gln Asn Gly Asp His Leu Ala Ile Ser
                   85                  90                  95
   Tyr Thr Arg Pro Met Ala Glu Lys Gly Glu Phe Gln Leu Glu Thr Phe
               100                 105                 110
   Thr Tyr Ile Glu Thr Tyr Asn Ile Val Leu Arg Leu Ser His Ile Leu
           115                 120                 125
   His Phe Asp Tyr Asn Val Gln Ala Gly Asp Tyr Val Ala Ile Asp Cys
       130                 135                 140
   Thr Asn Lys Pro Leu Phe Val Phe Leu Trp Leu Ser Leu Trp Asn Ile
   145                 150                 155                 160
   Gly Ala Ile Pro Ala Phe Leu Asn Tyr Asn Thr Lys Gly Thr Pro Leu
                   165                 170                 175
   Val His Ser Leu Lys Ile Ser Asn Ile Thr Gln Val Phe Ile Asp Pro
               180                 185                 190
   Asp Ala Ser Asn Pro Ile Arg Glu Ser Glu Glu Glu Ile Lys Asn Ala
           195                 200                 205
   Leu Pro Asp Val Lys Leu Asn Tyr Leu Glu Glu Gln Asp Leu Met His
       210                 215                 220
   Glu Leu Leu Asn Ser Gln Ser Pro Glu Phe Leu Gln Gln Asp Asn Val
   225                 230                 235                 240
   Arg Thr Pro Leu Gly Leu Thr Asp Phe Lys Pro Ser Met Leu Ile Tyr
                   245                 250                 255
   Thr Ser Gly Thr Thr Gly Leu Pro Lys Ser Ala Ile Met Ser Trp Arg
               260                 265                 270
   Lys Ser Ser Val Gly Cys Gln Val Phe Gly His Val Leu His Met Thr
           275                 280                 285
   Asn Glu Ser Thr Val Phe Thr Ala Met Pro Leu Phe His Ser Thr Ala
       290                 295                 300
   Ala Leu Leu Gly Ala Cys Ala Ile Leu Ser His Gly Gly Cys Leu Ala
   305                 310                 315                 320
   Leu Ser His Lys Phe Ser Ala Ser Thr Phe Trp Lys Gln Val Tyr Leu
                   325                 330                 335
   Thr Gly Ala Thr His Ile Gln Tyr Val Gly Glu Val Cys Arg Tyr Leu
               340                 345                 350
   Leu His Thr Pro Ile Ser Lys Tyr Glu Lys Met His Lys Val Lys Val
           355                 360                 365
   Ala Tyr Gly Asn Gly Leu Arg Pro Asp Ile Trp Gln Asp Phe Arg Lys
       370                 375                 380
   Arg Phe Asn Ile Glu Val Ile Gly Glu Phe Tyr Ala Ala Thr Glu Ala
   385                 390                 395                 400
   Pro Phe Ala Thr Thr Thr Phe Gln Lys Gly Asp Phe Gly Ile Gly Ala
                   405                 410                 415
   Cys Arg Asn Tyr Gly Thr Ile Ile Gln Trp Phe Leu Ser Phe Gln Gln
               420                 425                 430
   Thr Leu Val Arg Met Asp Pro Asn Asp Asp Ser Val Ile Tyr Arg Asn
           435                 440                 445
   Ser Lys Gly Phe Cys Glu Val Ala Pro Val Gly Glu Pro Gly Glu Met
       450                 455                 460
   Leu Met Arg Ile Phe Phe Pro Lys Lys Pro Glu Thr Ser Phe Gln Gly
   465                 470                 475                 480
   Tyr Leu Gly Asn Ala Lys Glu Thr Lys Ser Lys Val Val Arg Asp Val
                   485                 490                 495
   Phe Arg Arg Gly Asp Ala Trp Tyr Arg Cys Gly Asp Leu Leu Lys Ala
               500                 505                 510
   Asp Glu Tyr Gly Leu Trp Tyr Phe Leu Asp Arg Met Gly Asp Thr Phe
           515                 520                 525
   Arg Trp Lys Ser Glu Asn Val Ser Thr Thr Glu Val Glu Asp Gln Leu
       530                 535                 540
   Thr Ala Ser Asn Lys Glu Gln Tyr Ala Gln Val Leu Val Val Gly Ile
   545                 550                 555                 560
   Lys Val Pro Lys Tyr Glu Gly Arg Ala Gly Phe Ala Val Ile Lys Leu
                   565                 570                 575
   Thr Asp Asn Ser Leu Asp Ile Thr Ala Lys Thr Lys Leu Leu Asn Asp
               580                 585                 590
   Ser Leu Ser Arg Leu Asn Leu Pro Ser Tyr Ala Met Pro Leu Phe Val
           595                 600                 605
   Lys Phe Val Asp Glu Ile Lys Met Thr Asp Asn Leu Ile Lys Phe
       610                 615                 620
<210> 90
   <211> 1794
   <212> DNA
   <213> Mycobacterium tuberculosis
<400> 90
   gtgtccgatt actacggcgg cgcacacaca acggtcaggc tgatcgacct ggcaactcgg      60
   atgccgcgag tgttggcgga cacgccggtg attgtgcgtg gggcaatgac cgggctgctg     120
   gcccggccga attccaaggc gtcgatcggc acggtgttcc aggaccgggc cgctcgctac     180
   ggtgaccgag tcttcctgaa attcggcgat cagcagctga cctaccgcga cgctaacgcc     240
   accgccaacc ggtacgccgc ggtgttggcc gcccgcggcg tcggccccgg cgacgtcgtt     300
   ggcatcatgt tgcgtaactc acccagcaca gtcttggcga tgctggccac ggtcaagtgc     360
   ggcgctatcg ccggcatgct caactaccac cagcgcggcg aggtgttggc gcacagcctg     420
   ggtctgctgg acgcgaaggt actgatcgca gagtccgact tggtcagcgc cgtcgccgaa     480
   tgcggcgcct cgcgcggccg ggtagcgggc gacgtgctga ccgtcgagga cgtggagcga     540
   ttcgccacaa cggcgcccgc caccaacccg gcgtcggcgt cggcggtgca agccaaagac     600
   accgcgttct acatcttcac ctcgggcacc accggatttc ccaaggccag tgtcatgacg     660
   catcatcggt ggctgcgggc gctggccgtc ttcggaggga tggggctgcg gctgaagggt     720
   tccgacacgc tctacagctg cctgccgctg taccacaaca acgcgttaac ggtcgcggtg     780
   tcgtcggtga tcaattctgg ggcgaccctg gcgctgggta agtcgttttc ggcgtcgcgg     840
   ttctgggatg aggtgattgc caaccgggcg acggcgttcg tctacatcgg cgaaatctgc     900
   cgttatctgc tcaaccagcc ggccaagccg accgaccgtg cccaccaggt gcgggtgatc     960
   tgcggtaacg ggctgcggcc ggagatctgg gatgagttca ccacccgctt cggggtcgcg    1020
   cgggtgtgcg agttctacgc cgccagcgaa ggcaactcgg cctttatcaa catcttcaac    1080
   gtgcccagga ccgccggggt atcgccgatg ccgcttgcct ttgtggaata cgacctggac    1140
   accggcgatc cgctgcggga tgcgagcggg cgagtgcgtc gggtacccga cggtgaaccc    1200
   ggcctgttgc ttagccgggt caaccggctg cagccgttcg acggctacac cgacccggtt    1260
   gccagcgaaa agaagttggt gcgcaacgct tttcgagatg gcgactgttg gttcaacacc    1320
   ggtgacgtga tgagcccgca gggcatgggc catgccgcct tcgtcgatcg gctgggcgac    1380
   accttccgct ggaagggcga gaatgtcgcc accactcagg tcgaagcggc actggcctcc    1440
   gaccagaccg tcgaggagtg cacggtctac ggcgtccaga ttccgcgcac cggcgggcgc    1500
   gccggaatgg ccgcgatcac actgcgcgct ggcgccgaat tcgacggcca ggcgctggcc    1560
   cgaacggttt acggtcactt gcccggctat gcacttccgc tctttgttcg ggtagtgggg    1620
   tcgctggcgc acaccacgac gttcaagagt cgcaaggtgg agttgcgcaa ccaggcctat    1680
   ggcgccgaca tcgaggatcc gctgtacgta ctggccggcc cggacgaagg atatgtgccg    1740
   tactacgccg aataccctga ggaggtttcg ctcggaaggc gaccgcaggg ctag          1794
<210> 91
   <211> 597
   <212> PRT
   <213> Mycobacterium tuberculosis
<400> 91
   Met Ser Asp Tyr Tyr Gly Gly Ala His Thr Thr Val Arg Leu Ile Asp
   1 5 10 15
   Leu Ala Thr Arg Met Pro Arg Val Leu Ala Asp Thr Pro Val Ile Val
   20 25 30
   Arg Gly Ala Met Thr Gly Leu Leu Ala Arg Pro Asn Ser Lys Ala Ser
   35 40 45
   Ile Gly Thr Val Phe Gln Asp Arg Ala Ala Arg Tyr Gly Asp Arg Val
   50 55 60
   Phe Leu Lys Phe Gly Asp Gln Gln Leu Thr Tyr Arg Asp Ala Asn Ala
   65 70 75 80
   Thr Ala Asn Arg Tyr Ala Ala Val Leu Ala Ala Arg Gly Val Gly Pro
   85 90 95
   Gly Asp Val Val Gly Ile Met Leu Arg Asn Ser Pro Ser Thr Val Leu
   100 105 110
   Ala Met Leu Ala Thr Val Lys Cys Gly Ala Ile Ala Gly Met Leu Asn
   115 120 125
   Tyr His Gln Arg Gly Glu Val Leu Ala His Ser Leu Gly Leu Leu Asp
   130 135 140
   Ala Lys Val Leu Ile Ala Glu Ser Asp Leu Val Ser Ala Val Ala Glu
   145 150 155 160
   Cys Gly Ala Ser Arg Gly Arg Val Ala Gly Asp Val Leu Thr Val Glu
   165 170 175
   Asp Val Glu Arg Phe Ala Thr Thr Ala Pro Ala Thr Asn Pro Ala Ser
   180 185 190
   Ala Ser Ala Val Gln Ala Lys Asp Thr Ala Phe Tyr Ile Phe Thr Ser
   195 200 205
   Gly Thr Thr Gly Phe Pro Lys Ala Ser Val Met Thr His His Arg Trp
   210 215 220
   Leu Arg Ala Leu Ala Val Phe Gly Gly Met Gly Leu Arg Leu Lys Gly
   225 230 235 240
   Ser Asp Thr Leu Tyr Ser Cys Leu Pro Leu Tyr His Asn Asn Ala Leu
   245 250 255
   Thr Val Ala Val Ser Ser Val Ile Asn Ser Gly Ala Thr Leu Ala Leu
   260 265 270
   Gly Lys Ser Phe Ser Ala Ser Arg Phe Trp Asp Glu Val Ile Ala Asn
   275 280 285
   Arg Ala Thr Ala Phe Val Tyr Ile Gly Glu Ile Cys Arg Tyr Leu Leu
   290 295 300
   Asn Gln Pro Ala Lys Pro Thr Asp Arg Ala His Gln Val Arg Val Ile
   305 310 315 320
   Cys Gly Asn Gly Leu Arg Pro Glu Ile Trp Asp Glu Phe Thr Thr Arg
   325 330 335
   Phe Gly Val Ala Arg Val Cys Glu Phe Tyr Ala Ala Ser Glu Gly Asn
   340 345 350
   Ser Ala Phe Ile Asn Ile Phe Asn Val Pro Arg Thr Ala Gly Val Ser
   355 360 365
   Pro Met Pro Leu Ala Phe Val Glu Tyr Asp Leu Asp Thr Gly Asp Pro
   370 375 380
   Leu Arg Asp Ala Ser Gly Arg Val Arg Arg Val Pro Asp Gly Glu Pro
   385 390 395 400
   Gly Leu Leu Leu Ser Arg Val Asn Arg Leu Gln Pro Phe Asp Gly Tyr
                   405                 410                 415
   Thr Asp Pro Val Ala Ser Glu Lys Lys Leu Val Arg Asn Ala Phe Arg
               420                 425                 430
   Asp Gly Asp Cys Trp Phe Asn Thr Gly Asp Val Met Ser Pro Gln Gly
           435                 440                 445
   Met Gly His Ala Ala Phe Val Asp Arg Leu Gly Asp Thr Phe Arg Trp
       450                 455                 460
   Lys Gly Glu Asn Val Ala Thr Thr Gln Val Glu Ala Ala Leu Ala Ser
   465                 470                 475                 480
   Asp Gln Thr Val Glu Glu Cys Thr Val Tyr Gly Val Gln Ile Pro Arg
                   485                 490                 495
   Thr Gly Gly Arg Ala Gly Met Ala Ala Ile Thr Leu Arg Ala Gly Ala
               500                 505                 510
   Glu Phe Asp Gly Gln Ala Leu Ala Arg Thr Val Tyr Gly His Leu Pro
           515                 520                 525
   Gly Tyr Ala Leu Pro Leu Phe Val Arg Val Val Gly Ser Leu Ala His
       530                 535                 540
   Thr Thr Thr Phe Lys Ser Arg Lys Val Glu Leu Arg Asn Gln Ala Tyr
   545                 550                 555                 560
   Gly Ala Asp Ile Glu Asp Pro Leu Tyr Val Leu Ala Gly Pro Asp Glu
                   565                 570                 575
   Gly Tyr Val Pro Tyr Tyr Ala Glu Tyr Pro Glu Glu Val Ser Leu Gly
               580                 585                 590
   Arg Arg Pro Gln Gly
           595
<210> 92
   <211> 646
   <212> PRT
   <213> Mus musculus
<400> 92
   Met Arg Ala Pro Gly Ala Gly Thr Ala Ser Val Ala Ser Leu Ala Leu
    1               5                  10                  15
   Leu Trp Phe Leu Gly Leu Pro Trp Thr Trp Ser Ala Ala Ala Ala Phe
               20                  25                  30
   Cys Val Tyr Val Gly Gly Gly Gly Trp Arg Phe Leu Arg Ile Val Cys
           35                  40                  45
   Lys Thr Ala Arg Arg Asp Leu Phe Gly Leu Ser Val Leu Ile Arg Val
       50                  55                  60
   Arg Leu Glu Leu Arg Arg His Arg Arg Ala Gly Asp Thr Ile Pro Cys
   65                  70                  75                  80
   Ile Phe Gln Ala Val Ala Arg Arg Gln Pro Glu Arg Leu Ala Leu Val
                   85                  90                  95
   Asp Ala Ser Ser Gly Ile Cys Trp Thr Phe Ala Gln Leu Asp Thr Tyr
               100                 105                 110
   Ser Asn Ala Val Ala Asn Leu Phe Arg Gln Leu Gly Phe Ala Pro Gly
           115                 120                 125
   Asp Val Val Ala Val Phe Leu Glu Gly Arg Pro Glu Phe Val Gly Leu
       130                 135                 140
   Trp Leu Gly Leu Ala Lys Ala Gly Val Val Ala Ala Leu Leu Asn Val
   145                 150                 155                 160
   Asn Leu Arg Arg Glu Pro Leu Ala Phe Cys Leu Gly Thr Ser Ala Ala
                   165                 170                 175
   Lys Ala Leu Ile Tyr Gly Gly Glu Met Ala Ala Ala Val Ala Glu Val
               180                 185                 190
   Ser Glu Gln Leu Gly Lys Ser Leu Leu Lys Phe Cys Ser Gly Asp Leu
   195 200 205
   Gly Pro Glu Ser Ile Leu Pro Asp Thr Gln Leu Leu Asp Pro Met Leu
   210 215 220
   Ala Glu Ala Pro Thr Thr Pro Leu Ala Gln Ala Pro Gly Lys Gly Met
   225 230 235 240
   Asp Asp Arg Leu Phe Tyr Ile Tyr Thr Ser Gly Thr Thr Gly Leu Pro
   245 250 255
   Lys Ala Ala Ile Val Val His Ser Arg Tyr Tyr Arg Ile Ala Ala Phe
   260 265 270
   Gly His His Ser Tyr Ser Met Arg Ala Ala Asp Val Leu Tyr Asp Cys
   275 280 285
   Leu Pro Leu Tyr His Ser Ala Gly Asn Ile Met Gly Val Gly Gln Cys
   290 295 300
   Val Ile Tyr Gly Leu Thr Val Val Leu Arg Lys Lys Phe Ser Ala Ser
   305 310 315 320
   Arg Phe Trp Asp Asp Cys Val Lys Tyr Asn Cys Thr Val Val Gln Tyr
   325 330 335
   Ile Gly Glu Ile Cys Arg Tyr Leu Leu Arg Gln Pro Val Arg Asp Val
   340 345 350
   Glu Gln Arg His Arg Val Arg Leu Ala Val Gly Asn Gly Leu Arg Pro
   355 360 365
   Ala Ile Trp Glu Glu Phe Thr Gln Arg Phe Gly Val Pro Gln Ile Gly
   370 375 380
   Glu Phe Tyr Gly Ala Thr Glu Cys Asn Cys Ser Ile Ala Asn Met Asp
   385 390 395 400
   Gly Lys Val Gly Ser Cys Gly Phe Asn Ser Arg Ile Leu Thr His Val
   405 410 415
   Tyr Pro Ile Arg Leu Val Lys Val Asn Glu Asp Thr Met Glu Pro Leu
   420 425 430
   Arg Asp Ser Glu Gly Leu Cys Ile Pro Cys Gln Pro Gly Glu Pro Gly
   435 440 445
   Leu Leu Val Gly Gln Ile Asn Gln Gln Asp Pro Leu Arg Arg Phe Asp
   450 455 460
   Gly Tyr Val Ser Asp Ser Ala Thr Asn Lys Lys Ile Ala His Ser Val
   465 470 475 480
   Phe Arg Lys Gly Asp Ser Ala Tyr Leu Ser Gly Asp Val Leu Val Met
   485 490 495
   Asp Glu Leu Gly Tyr Met Tyr Phe Arg Asp Arg Ser Gly Asp Thr Phe
   500 505 510
   Arg Trp Arg Gly Glu Asn Val Ser Thr Thr Glu Val Glu Ala Val Leu
   515 520 525
   Ser Arg Leu Leu Gly Gln Thr Asp Val Ala Val Tyr Gly Val Ala Val
   530 535 540
   Pro Gly Val Glu Gly Lys Ala Gly Met Ala Ala Ile Ala Asp Pro His
   545 550 555 560
   Ser Gln Leu Asp Pro Asn Ser Met Tyr Gln Glu Leu Gln Lys Val Leu
   565 570 575
   Ala Ser Tyr Ala Arg Pro Ile Phe Leu Arg Leu Leu Pro Gln Val Asp
   580 585 590
   Thr Thr Gly Thr Phe Lys Ile Gln Lys Thr Arg Leu Gln Arg Glu Gly
   595 600 605
   Phe Asp Pro Arg Gln Thr Ser Asp Arg Leu Phe Phe Leu Asp Leu Lys
   610 615 620
   Gln Gly Arg Tyr Leu Pro Leu Asp Glu Arg Val His Ala Arg Ile Cys
   625 630 635 640
   Ala Gly Asp Phe Ser Leu
   645
<210> 93
   <211> 620
   <212> PRT
   <213> Mus musculus
<220>
   <221> VARIANT
   <222> (1)...(620)
   <223> Xaa = Any Amino Acid
<400> 93
   Met Leu Pro Val Leu Tyr Thr Gly Leu Ala Gly Leu Leu Leu Leu Pro
   1 5 10 15
   Leu Leu Leu Thr Cys Cys Cys Pro Tyr Leu Leu Gln Asp Val Arg Tyr
   20 25 30
   Phe Leu Arg Leu Ala Asn Met Ala Arg Arg Val Arg Ser Tyr Arg Gln
   35 40 45
   Arg Arg Pro Val Arg Thr Ile Leu Arg Ala Phe Leu Glu Gln Ala Arg
   50 55 60
   Lys Thr Pro His Lys Pro Phe Leu Leu Phe Arg Asp Glu Thr Leu Thr
   65 70 75 80
   Tyr Ala Gln Val Asp Arg Arg Ser Asn Gln Val Ala Arg Ala Leu His
   85 90 95
   Asp Gln Leu Gly Leu Arg Gln Gly Asp Cys Val Ala Leu Phe Met Gly
   100 105 110
   Asn Glu Pro Ala Tyr Val Trp Ile Trp Leu Gly Leu Leu Lys Leu Gly
   115 120 125
   Cys Pro Met Ala Cys Leu Asn Tyr Asn Ile Arg Ala Lys Ser Leu Leu
   130 135 140
   His Cys Phe Gln Cys Cys Gly Ala Lys Val Leu Leu Ala Ser Pro Asp
   145 150 155 160
   Leu Gln Glu Ala Val Glu Glu Val Leu Pro Thr Leu Lys Lys Asp Ala
   165 170 175
   Val Ser Val Phe Tyr Val Ser Arg Thr Ser Asn Thr Asn Gly Val Asp
   180 185 190
   Thr Ile Leu Asp Lys Val Asp Gly Val Ser Ala Glu Pro Thr Pro Glu
   195 200 205
   Ser Trp Arg Ser Glu Val Thr Phe Thr Thr Pro Ala Val Tyr Ile Tyr
   210 215 220
   Thr Ser Gly Thr Thr Gly Leu Pro Lys Ala Ala Thr Ile Asn His His
   225 230 235 240
   Arg Leu Arg Tyr Gly Thr Gly Leu Ala Met Ser Ser Gly Ile Thr Ala
   245 250 255
   Gln Asp Val Ile Tyr Thr Thr Met Pro Leu Tyr His Ser Ala Ala Leu
   260 265 270
   Met Ile Gly Leu His Gly Cys Ile Val Val Gly Ala Xaa Xaa Xaa Leu
   275 280 285
   Cys Asp Lys Phe Ser Ala Ser Gln Phe Trp Asp Asp Cys Arg Lys Tyr
   290 295 300
   Asn Val Thr Val Ile Gln Tyr Ile Gly Glu Leu Leu Arg Tyr Leu Cys
   305 310 315 320
   Asn Thr Pro Gln Lys Pro Asn Asp Arg Asp His Lys Val Lys Lys Ala
   325 330 335
   Leu Gly Asn Gly Leu Arg Gly Asp Val Trp Arg Glu Phe Ile Lys Arg
               340                 345                 350
   Phe Gly Asp Ile His Val Tyr Glu Phe Tyr Ala Ser Thr Glu Gly Asn
           355                 360                 365
   Ile Gly Phe Val Asn Tyr Pro Arg Lys Ile Gly Ala Val Gly Arg Ala
       370                 375                 380
   Asn Tyr Leu Gln Arg Lys Val Ala Arg Tyr Glu Leu Ile Lys Tyr Asp
   385                 390                 395                 400
   Val Glu Lys Asp Glu Pro Val Arg Asp Ala Asn Gly Tyr Cys Ile Lys
                   405                 410                 415
   Val Pro Lys Gly Glu Val Gly Leu Leu Val Cys Lys Ile Thr Gln Leu
               420                 425                 430
   Thr Pro Phe Ile Gly Tyr Ala Gly Gly Lys Thr Gln Thr Glu Lys Lys
           435                 440                 445
   Lys Leu Arg Asp Val Phe Lys Lys Gly Asp Ile Tyr Phe Asn Ser Gly
       450                 455                 460
   Asp Leu Leu Met Ile Asp Arg Glu Asn Phe Val Tyr Phe His Asp Arg
   465                 470                 475                 480
   Val Gly Asp Thr Phe Arg Trp Lys Gly Glu Asn Val Ala Thr Thr Glu
                   485                 490                 495
   Val Ala Asp Ile Val Gly Leu Val Asp Phe Val Glu Glu Val Asn Val
               500                 505                 510
   Tyr Gly Val Pro Val Pro Gly His Glu Gly Arg Ile Gly Met Ala Ser
           515                 520                 525
   Leu Lys Ile Lys Glu Asn Tyr Glu Phe Asn Gly Lys Lys Leu Phe Gln
       530                 535                 540
   His Ile Ala Glu Tyr Leu Pro Ser Tyr Ala Arg Pro Arg Phe Leu Arg
   545                 550                 555                 560
   Ile Gln Asp Thr Ile Glu Ile Thr Gly Thr Phe Lys His Arg Lys Val
                   565                 570                 575
   Thr Leu Met Glu Glu Gly Phe Asn Pro Thr Val Ile Lys Asp Thr Leu
               580                 585                 590
   Tyr Phe Met Asp Asp Ala Glu Lys Thr Phe Val Pro Met Thr Glu Asn
           595                 600                 605
   Ile Tyr Asn Ala Ile Ile Asp Lys Thr Leu Lys Leu
       610                 615                 620
<210> 94
   <211> 613
   <212> PRT
   <213> Mus musculus
<400> 94
   Ala Ala Asp Pro Glu Ser Ser Glu Ser Gly Cys Ser Leu Ala Trp Arg
   1 5 10 15
   Leu Ala Tyr Leu Ala Arg Glu Gln Pro Thr His Thr Phe Leu Ile His
   20 25 30
   Gly Ala Gln Arg Phe Ser Tyr Ala Glu Ala Glu Arg Glu Ser Asn Arg
   35 40 45
   Ile Ala Arg Ala Phe Leu Arg Ala Arg Gly Trp Thr Gly Gly Arg Arg
   50 55 60
   Gly Ser Gly Arg Gly Ser Thr Glu Glu Gly Ala Arg Val Ala Pro Pro
   65 70 75 80
   Ala Gly Asp Ala Ala Ala Arg Gly Thr Thr Ala Pro Pro Leu Ala Pro
   85 90 95
   Gly Ala Thr Val Ala Leu Leu Leu Pro Ala Gly Pro Asp Phe Leu Trp
   100 105 110
   Ile Trp Phe Gly Leu Ala Lys Ala Gly Leu Arg Thr Ala Phe Val Pro
   115 120 125
   Thr Ala Leu Arg Arg Gly Pro Leu Leu His Cys Leu Arg Ser Cys Gly
   130 135 140
   Ala Ser Ala Leu Val Leu Ala Thr Glu Phe Leu Glu Ser Leu Glu Pro
   145 150 155 160
   Asp Leu Pro Ala Leu Arg Ala Met Gly Leu His Leu Trp Ala Thr Gly
   165 170 175
   Pro Glu Thr Asn Val Ala Gly Ile Ser Asn Leu Leu Ser Glu Ala Ala
   180 185 190
   Asp Gln Val Asp Glu Pro Val Pro Gly Tyr Leu Ser Ala Pro Gln Asn
   195 200 205
   Ile Met Asp Thr Cys Leu Tyr Ile Phe Thr Ser Gly Thr Thr Gly Leu
   210 215 220
   Pro Lys Ala Ala Arg Ile Ser His Leu Lys Val Leu Gln Cys Gln Gly
   225 230 235 240
   Phe Tyr His Leu Cys Gly Val His Gln Glu Asp Val Ile Tyr Leu Ala
   245 250 255
   Leu Pro Leu Tyr His Met Ser Gly Ser Leu Leu Gly Ile Val Gly Cys
   260 265 270
   Leu Gly Ile Gly Ala Thr Val Val Leu Lys Pro Lys Phe Ser Ala Ser
   275 280 285
   Gln Phe Trp Asp Asp Cys Gln Lys His Arg Val Thr Val Phe Gln Tyr
   290 295 300
   Ile Gly Glu Leu Cys Arg Tyr Leu Val Asn Gln Pro Pro Ser Lys Ala
   305 310 315 320
   Glu Phe Asp His Lys Val Arg Leu Ala Val Gly Ser Gly Leu Arg Pro
   325 330 335
   Asp Thr Trp Glu Arg Phe Leu Arg Arg Phe Gly Pro Leu Gln Ile Leu
   340 345 350
   Glu Thr Tyr Gly Met Thr Glu Gly Asn Val Ala Thr Phe Asn Tyr Thr
   355 360 365
   Gly Arg Gln Gly Ala Val Gly Arg Ala Ser Trp Leu Tyr Lys His Ile
   370 375 380
   Phe Pro Phe Ser Leu Ile Arg Tyr Asp Val Met Thr Gly Glu Pro Ile
   385 390 395 400
   Arg Asn Ala Gln Gly His Cys Met Thr Thr Ser Pro Gly Glu Pro Gly
   405 410 415
   Leu Leu Val Ala Pro Val Ser Gln Gln Ser Pro Phe Leu Gly Tyr Ala
   420 425 430
   Gly Ala Pro Glu Leu Ala Lys Asp Lys Leu Leu Lys Asp Val Phe Trp
   435 440 445
   Ser Gly Asp Val Phe Phe Asn Thr Gly Asp Leu Leu Val Cys Asp Glu
   450 455 460
   Gln Gly Phe Leu His Phe His Asp Arg Thr Gly Asp Thr Ile Arg Trp
   465 470 475 480
   Lys Gly Glu Asn Val Ala Thr Thr Glu Val Ala Glu Val Leu Glu Thr
   485 490 495
   Leu Asp Phe Leu Gln Glu Val Asn Ile Tyr Gly Val Thr Val Pro Gly
   500 505 510
   His Glu Gly Arg Ala Gly Met Ala Ala Leu Ala Leu Arg Pro Pro Gln
   515 520 525
   Ala Leu Asn Leu Val Gln Leu Tyr Ser His Val Ser Glu Asn Leu Pro
   530 535 540
   Pro Tyr Ala Arg Pro Arg Phe Leu Arg Leu Gln Glu Ser Leu Ala Thr
   545 550 555 560
   Thr Glu Thr Phe Lys Gln Gln Lys Val Arg Met Ala Asn Glu Gly Phe
                   565                 570                 575
   Asp Pro Ser Val Leu Ser Asp Pro Leu Tyr Val Leu Asp Gln Asp Ile
               580                 585                 590
   Gly Ala Tyr Leu Pro Leu Thr Pro Ala Arg Tyr Ser Ala Leu Leu Ser
           595                 600                 605
   Gly Asp Leu Arg Ile
       610
<210> 95
   <211> 506
   <212> PRT
   <213> Mus musculus
<400> 95
   His Ala Ser Ala His Ala Ser Gly Met Ala Lys Leu Gly Val Glu Ala
    1               5                  10                  15
   Ala Leu Ile Asn Thr Asn Leu Arg Arg Asp Ala Leu Arg His Cys Leu
               20                  25                  30
   Asp Thr Ser Lys Ala Arg Ala Leu Ile Phe Gly Ser Glu Met Ala Ser
           35                  40                  45
   Ala Ile Cys Glu Ile His Ala Ser Leu Glu Pro Thr Leu Ser Leu Phe
       50                  55                  60
   Cys Ser Gly Ser Trp Glu Pro Ser Thr Val Pro Val Ser Thr Glu His
   65                  70                  75                  80
   Leu Asp Pro Leu Leu Glu Asp Ala Pro Lys His Leu Pro Ser His Pro
                   85                  90                  95
   Asp Lys Gly Phe Thr Asp Lys Leu Phe Tyr Ile Tyr Thr Ser Gly Thr
               100                 105                 110
   Thr Gly Leu Pro Lys Ala Ala Ile Val Val His Ser Arg Tyr Tyr Arg
           115                 120                 125
   Met Ala Ser Leu Val Tyr Tyr Gly Phe Arg Met Arg Pro Asp Asp Ile
       130                 135                 140
   Val Tyr Asp Cys Leu Pro Leu Tyr His Ser Ser Arg Lys His Arg Gly
   145                 150                 155                 160
   Asp Trp Gln Cys Leu Leu His Gly Met Thr Val Val Ile Arg Lys Lys
                   165                 170                 175
   Phe Ser Ala Ser Arg Phe Trp Asp Asp Cys Ile Lys Tyr Asn Cys Thr
               180                 185                 190
   Val Val Gln Tyr Ile Gly Glu Leu Cys Arg Tyr Leu Leu Asn Gln Pro
           195                 200                 205
   Pro Arg Glu Ala Glu Ser Arg His Lys Val Arg Met Ala Leu Gly Asn
       210                 215                 220
   Gly Leu Arg Gln Ser Ile Trp Thr Asp Phe Ser Ser Arg Phe His Ile
   225                 230                 235                 240
   Pro Gln Val Ala Glu Phe Tyr Gly Ala Thr Glu Cys Asn Cys Ser Leu
                   245                 250                 255
   Gly Asn Phe Asp Ser Arg Val Gly Ala Cys Gly Phe Asn Ser Arg Ile
               260                 265                 270
   Leu Ser Phe Val Tyr Pro Ile Arg Leu Val Arg Val Asn Glu Asp Thr
           275                 280                 285
   Met Glu Leu Ile Arg Gly Pro Asp Gly Val Cys Ile Pro Cys Gln Pro
       290                 295                 300
   Gly Gln Pro Gly Gln Leu Val Gly Arg Ile Ile Gln Gln Asp Pro Leu
   305                 310                 315                 320
   Arg Arg Phe Asp Gly Tyr Leu Asn Gln Gly Ala Asn Asn Lys Lys Ile
                   325                 330                 335
   Ala Asn Asp Val Phe Lys Lys Gly Asp Gln Ala Tyr Leu Thr Gly Asp
               340                 345                 350
   Val Leu Val Met Asp Glu Leu Gly Tyr Leu Tyr Phe Arg Asp Arg Thr
           355                 360                 365
   Gly Asp Thr Phe Arg Trp Lys Gly Glu Asn Val Ser Thr Thr Glu Val
       370                 375                 380
   Glu Gly Thr Leu Ser Arg Leu Leu His Met Ala Asp Val Ala Val Tyr
   385                 390                 395                 400
   Gly Val Glu Val Pro Gly Thr Glu Gly Arg Ala Gly Met Ala Ala Val
                   405                 410                 415
   Ala Ser Pro Ile Ser Asn Cys Asp Leu Glu Ser Phe Ala Gln Thr Leu
               420                 425                 430
   Lys Lys Glu Leu Pro Leu Tyr Ala Arg Pro Ile Phe Leu Arg Phe Leu
           435                 440                 445
   Pro Glu Leu His Lys Thr Gly Thr Phe Lys Phe Gln Lys Thr Glu Leu
       450                 455                 460
   Arg Lys Glu Gly Phe Asp Pro Ser Val Val Lys Asp Pro Leu Phe Tyr
   465                 470                 475                 480
   Leu Asp Ala Arg Lys Gly Cys Tyr Val Ala Leu Asp Gln Glu Ala Tyr
                   485                 490                 495
   Thr Arg Ile Gln Ala Gly Glu Glu Lys Leu
               500                 505
<210> 96
   <211> 662
   <212> PRT
   <213> Mus musculus
<400> 96
   Met Ala Leu Ala Leu Arg Trp Phe Leu Gly Asp Pro Thr Cys Leu Val
    1               5                  10                  15
   Leu Leu Gly Leu Ala Leu Leu Gly Arg Pro Trp Ile Ser Ser Trp Met
               20                  25                  30
   Pro His Trp Leu Ser Leu Val Gly Ala Ala Leu Thr Leu Phe Leu Leu
           35                  40                  45
   Pro Leu Gln Pro Pro Pro Gly Leu Arg Trp Leu His Lys Asp Val Ala
       50                  55                  60
   Phe Thr Phe Lys Met Leu Phe Tyr Gly Leu Lys Phe Arg Arg Arg Leu
   65                  70                  75                  80
   Asn Lys His Pro Pro Glu Thr Phe Val Asp Ala Leu Glu Arg Gln Ala
                   85                  90                  95
   Leu Ala Trp Pro Asp Arg Val Ala Leu Val Cys Thr Gly Ser Glu Gly
               100                 105                 110
   Ser Ser Ile Thr Asn Ser Gln Leu Asp Ala Arg Ser Cys Gln Ala Ala
           115                 120                 125
   Trp Val Leu Lys Ala Lys Leu Lys Asp Ala Val Ile Gln Asn Thr Arg
       130                 135                 140
   Asp Ala Ala Ala Ile Leu Val Leu Pro Ser Lys Thr Ile Ser Ala Leu
   145                 150                 155                 160
   Ser Val Phe Leu Gly Leu Ala Lys Leu Gly Cys Pro Val Ala Trp Ile
                   165                 170                 175
   Asn Pro His Ser Arg Gly Met Pro Leu Leu His Ser Val Arg Ser Ser
               180                 185                 190
   Gly Ala Ser Val Leu Ile Val Asp Pro Asp Leu Gln Glu Asn Leu Glu
           195                 200                 205
   Glu Val Leu Pro Lys Leu Leu Ala Glu Asn Ile His Cys Phe Tyr Leu
       210                 215                 220
   Gly His Ser Ser Pro Thr Pro Gly Val Glu Ala Leu Gly Ala Ser Leu
   225 230 235 240
   Asp Ala Ala Pro Ser Asp Pro Val Pro Ala Ser Leu Arg Ala Thr Ile
   245 250 255
   Lys Trp Lys Ser Pro Ala Ile Phe Ile Phe Thr Ser Gly Thr Thr Gly
   260 265 270
   Leu Pro Lys Pro Ala Ile Leu Ser His Glu Arg Val Ile Gln Val Ser
   275 280 285
   Asn Val Leu Ser Phe Cys Gly Cys Arg Ala Asp Asp Val Val Tyr Asp
   290 295 300
   Val Leu Pro Leu Tyr His Thr Ile Gly Leu Val Leu Gly Phe Leu Gly
   305 310 315 320
   Cys Leu Gln Val Gly Ala Thr Cys Val Leu Ala Pro Lys Phe Ser Ala
   325 330 335
   Ser Arg Phe Trp Ala Glu Cys Arg Gln His Gly Val Thr Val Ile Leu
   340 345 350
   Tyr Val Gly Glu Ile Leu Arg Tyr Leu Cys Asn Val Pro Glu Gln Pro
   355 360 365
   Glu Asp Lys Ile His Thr Val Arg Leu Ala Met Gly Thr Gly Leu Arg
   370 375 380
   Ala Asn Val Trp Lys Asn Phe Gln Gln Arg Phe Gly Pro Ile Arg Ile
   385 390 395 400
   Trp Glu Phe Tyr Gly Ser Thr Glu Gly Asn Val Gly Leu Met Asn Tyr
   405 410 415
   Val Gly His Cys Gly Ala Val Gly Arg Thr Ser Cys Ile Leu Arg Met
   420 425 430
   Leu Thr Pro Phe Glu Leu Val Gln Phe Asp Ile Glu Thr Ala Glu Pro
   435 440 445
   Leu Arg Asp Lys Gln Gly Phe Cys Ile Pro Val Glu Pro Gly Lys Pro
   450 455 460
   Gly Leu Leu Leu Thr Lys Val Arg Lys Asn Gln Pro Phe Leu Gly Tyr
   465 470 475 480
   Arg Gly Ser Gln Ala Glu Ser Asn Arg Lys Leu Val Ala Asn Val Arg
   485 490 495
   Arg Val Gly Asp Leu Tyr Phe Asn Thr Gly Asp Val Leu Thr Leu Asp
   500 505 510
   Gln Glu Gly Phe Phe Tyr Phe Gln Asp Arg Leu Gly Asp Thr Phe Arg
   515 520 525
   Trp Lys Gly Glu Asn Val Ser Thr Gly Glu Val Glu Cys Val Leu Ser
   530 535 540
   Ser Leu Asp Phe Leu Glu Glu Val Asn Val Tyr Gly Val Pro Val Pro
   545 550 555 560
   Gly Cys Glu Gly Lys Val Gly Met Ala Ala Val Lys Leu Ala Pro Gly
   565 570 575
   Lys Thr Phe Asp Gly Gln Lys Leu Tyr Gln His Val Arg Ser Trp Leu
   580 585 590
   Pro Ala Tyr Ala Thr Pro His Phe Ile Arg Ile Gln Asp Ser Leu Glu
   595 600 605
   Ile Thr Asn Thr Tyr Lys Leu Val Lys Ser Arg Leu Val Arg Glu Gly
   610 615 620
   Phe Asp Val Gly Ile Ile Ala Asp Pro Leu Tyr Ile Leu Asp Asn Lys
   625 630 635 640
   Ala Gln Thr Phe Arg Ser Leu Met Pro Asp Val Tyr Gln Ala Val Cys
   645 650 655
   Glu Gly Thr Trp Asn Leu
   660
<210> 97
   <211> 650
   <212> PRT
   <213> Caenorhabditis elegans
<400> 97
   Met Lys Leu Glu Glu Leu Val Thr Val Met Leu Leu Thr Val Ala Val
   1               5                   10                  15
   Ile Ala Gln Asn Leu Pro Ile Gly Val Ile Leu Ala Gly Val Leu Ile
               20                  25                  30
   Leu Tyr Ile Thr Val Val His Gly Asp Phe Ile Tyr Arg Ser Tyr Leu
           35                  40                  45
   Thr Leu Asn Arg Asp Leu Thr Gly Leu Ala Leu Ile Ile Glu Val Lys
       50                  55                  60
   Ile Asp Leu Trp Trp Arg Leu His Gln Asn Lys Gly Ile His Glu Leu
   65                  70                  75                  80
   Phe Leu Asp Ile Val Lys Lys Asn Pro Asn Lys Pro Ala Met Ile Asp
                   85                  90                  95
   Ile Glu Thr Asn Thr Thr Glu Thr Tyr Ala Glu Phe Asn Ala His Cys
               100                 105                 110
   Asn Arg Tyr Ala Asn Tyr Phe Gln Gly Leu Gly Tyr Arg Ser Gly Asp
           115                 120                 125
   Val Val Ala Leu Tyr Met Glu Asn Ser Val Glu Phe Val Ala Ala Trp
       130                 135                 140
   Met Gly Leu Ala Lys Ile Gly Val Val Thr Ala Trp Ile Asn Ser Asn
   145                 150                 155                 160
   Leu Lys Arg Glu Gln Leu Val His Cys Ile Thr Ala Ser Lys Thr Lys
                   165                 170                 175
   Ala Ile Ile Thr Ser Val Thr Leu Gln Asn Ile Met Leu Asp Ala Ile
               180                 185                 190
   Asp Gln Lys Leu Phe Asp Val Glu Gly Ile Glu Val Tyr Ser Val Gly
           195                 200                 205
   Glu Pro Lys Lys Asn Ser Gly Phe Lys Asn Leu Lys Lys Lys Leu Asp
       210                 215                 220
   Ala Gln Ile Thr Thr Glu Pro Lys Thr Leu Asp Ile Val Asp Phe Lys
   225                 230                 235                 240
   Ser Ile Leu Cys Phe Ile Tyr Thr Ser Gly Thr Thr Gly Met Pro Lys
                   245                 250                 255
   Ala Ala Val Met Lys His Phe Arg Tyr Tyr Ser Ile Ala Val Gly Ala
               260                 265                 270
   Ala Lys Ser Phe Gly Ile Arg Pro Ser Asp Arg Met Tyr Val Ser Met
           275                 280                 285
   Pro Ile Tyr His Thr Ala Ala Gly Ile Leu Gly Val Gly Gln Ala Leu
       290                 295                 300
   Leu Gly Gly Ser Ser Cys Val Ile Arg Lys Lys Phe Ser Ala Ser Asn
   305                 310                 315                 320
   Phe Trp Arg Asp Cys Val Lys Tyr Asp Cys Thr Val Ser Gln Tyr Ile
                   325                 330                 335
   Gly Glu Ile Cys Arg Tyr Leu Leu Ala Gln Pro Val Val Glu Glu Glu
               340                 345                 350
   Ser Arg His Arg Met Arg Leu Leu Val Gly Asn Gly Leu Arg Ala Glu
           355                 360                 365
   Ile Trp Gln Pro Phe Val Asp Arg Phe Arg Val Arg Ile Gly Glu Leu
       370                 375                 380
   Tyr Gly Ser Thr Glu Gly Thr Ser Ser Leu Val Asn Ile Asp Gly His
   385                 390                 395                 400
   Val Gly Ala Cys Gly Phe Leu Pro Ile Ser Pro Leu Thr Lys Lys Met
   405 410 415
   His Pro Val Arg Leu Ile Lys Val Asp Asp Val Thr Gly Glu Ala Ile
   420 425 430
   Arg Thr Ser Asp Gly Leu Cys Ile Ala Cys Asn Pro Gly Glu Ser Gly
   435 440 445
   Ala Met Val Ser Thr Ile Arg Lys Asn Asn Pro Leu Leu Gln Phe Glu
   450 455 460
   Gly Tyr Leu Asn Lys Lys Glu Thr Asn Lys Lys Ile Ile Arg Asp Val
   465 470 475 480
   Phe Ala Lys Gly Asp Ser Cys Phe Leu Thr Gly Asp Leu Leu His Trp
   485 490 495
   Asp Arg Leu Gly Tyr Val Tyr Phe Lys Asp Arg Thr Gly Asp Thr Phe
   500 505 510
   Arg Trp Lys Gly Glu Asn Val Ser Thr Thr Glu Val Glu Ala Ile Leu
   515 520 525
   His Pro Ile Thr Gly Leu Ser Asp Ala Thr Val Tyr Gly Val Glu Val
   530 535 540
   Pro Gln Arg Glu Gly Arg Val Gly Met Ala Ser Val Val Arg Val Val
   545 550 555 560
   Ser His Glu Glu Asp Glu Thr Gln Phe Val His Arg Val Gly Ala Arg
   565 570 575
   Leu Ala Ser Ser Leu Thr Ser Tyr Ala Ile Pro Gln Phe Met Arg Ile
   580 585 590
   Cys Gln Asp Val Glu Lys Thr Gly Thr Phe Lys Leu Val Lys Thr Asn
   595 600 605
   Leu Gln Arg Leu Gly Ile Met Asp Ala Pro Ser Asp Ser Ile Tyr Ile
   610 615 620
   Tyr Asn Ser Glu Asn Arg Asn Phe Val Pro Phe Asp Asn Asp Leu Arg
   625 630 635 640
   Cys Lys Val Ser Leu Gly Ser Tyr Pro Phe
   645 650
<210> 98
   <211> 623
   <212> PRT
   <213> Saccharomyces cerevisiae
<400> 98
   Met Ser Pro Ile Gln Val Val Val Phe Ala Leu Ser Arg Ile Phe Leu
    1               5                  10                  15
   Leu Leu Phe Arg Leu Ile Lys Leu Ile Ile Thr Pro Ile Gln Lys Ser
               20                  25                  30
   Leu Gly Tyr Leu Phe Gly Asn Tyr Phe Asp Glu Leu Asp Arg Lys Tyr
           35                  40                  45
   Arg Tyr Lys Glu Asp Trp Tyr Ile Ile Pro Tyr Phe Leu Lys Ser Val
       50                  55                  60
   Phe Cys Tyr Ile Ile Asp Val Arg Arg His Arg Phe Gln Asn Trp Tyr
   65                  70                  75                  80
   Leu Phe Ile Lys Gln Val Gln Gln Asn Gly Asp His Leu Ala Ile Ser
                   85                  90                  95
   Tyr Thr Arg Pro Met Ala Glu Lys Gly Glu Phe Gln Leu Glu Thr Phe
               100                 105                 110
   Thr Tyr Ile Glu Thr Tyr Asn Ile Val Leu Arg Leu Ser His Ile Leu
           115                 120                 125
   His Phe Asp Tyr Asn Val Gln Ala Gly Asp Tyr Val Ala Ile Asp Cys
       130                 135                 140
   Thr Asn Lys Pro Leu Phe Val Phe Leu Trp Leu Ser Leu Trp Asn Ile
   145 150 155 160
   Gly Ala Ile Pro Ala Phe Leu Asn Tyr Asn Thr Lys Gly Thr Pro Leu
   165 170 175
   Val His Ser Leu Lys Ile Ser Asn Ile Thr Gln Val Phe Ile Asp Pro
   180 185 190
   Asp Ala Ser Asn Pro Ile Arg Glu Ser Glu Glu Glu Ile Lys Asn Ala
   195 200 205
   Leu Pro Asp Val Lys Leu Asn Tyr Leu Glu Glu Gln Asp Leu Met His
   210 215 220
   Glu Leu Leu Asn Ser Gln Ser Pro Glu Phe Leu Gln Gln Asp Asn Val
   225 230 235 240
   Arg Thr Pro Leu Gly Leu Thr Asp Phe Lys Pro Ser Met Leu Ile Tyr
   245 250 255
   Thr Ser Gly Thr Thr Gly Leu Pro Lys Ser Ala Ile Met Ser Trp Arg
   260 265 270
   Lys Ser Ser Val Gly Cys Gln Val Phe Gly His Val Leu His Met Thr
   275 280 285
   Asn Glu Ser Thr Val Phe Thr Ala Met Pro Leu Phe His Ser Thr Ala
   290 295 300
   Ala Leu Leu Gly Ala Cys Ala Ile Leu Ser His Gly Gly Cys Leu Ala
   305 310 315 320
   Leu Ser His Lys Phe Ser Ala Ser Thr Phe Trp Lys Gln Val Tyr Leu
   325 330 335
   Thr Gly Ala Thr His Ile Gln Tyr Val Gly Glu Val Cys Arg Tyr Leu
   340 345 350
   Leu His Thr Pro Ile Ser Lys Tyr Glu Lys Met His Lys Val Lys Val
   355 360 365
   Ala Tyr Gly Asn Gly Leu Arg Pro Asp Ile Trp Gln Asp Phe Arg Lys
   370 375 380
   Arg Phe Asn Ile Glu Val Ile Gly Glu Phe Tyr Ala Ala Thr Glu Ala
   385 390 395 400
   Pro Phe Ala Thr Thr Thr Phe Gln Lys Gly Asp Phe Gly Ile Gly Ala
   405 410 415
   Cys Arg Asn Tyr Gly Thr Ile Ile Gln Trp Phe Leu Ser Phe Gln Gln
   420 425 430
   Thr Leu Val Arg Met Asp Pro Asn Asp Asp Ser Val Ile Tyr Arg Asn
   435 440 445
   Ser Lys Gly Phe Cys Glu Val Ala Pro Val Gly Glu Pro Gly Glu Met
   450 455 460
   Leu Met Arg Ile Phe Phe Pro Lys Lys Pro Glu Thr Ser Phe Gln Gly
   465 470 475 480
   Tyr Leu Gly Asn Ala Lys Glu Thr Lys Ser Lys Val Val Arg Asp Val
   485 490 495
   Phe Arg Arg Gly Asp Ala Trp Tyr Arg Cys Gly Asp Leu Leu Lys Ala
   500 505 510
   Asp Glu Tyr Gly Leu Trp Tyr Phe Leu Asp Arg Met Gly Asp Thr Phe
   515 520 525
   Arg Trp Lys Ser Glu Asn Val Ser Thr Thr Glu Val Glu Asp Gln Leu
   530 535 540
   Thr Ala Ser Asn Lys Glu Gln Tyr Ala Gln Val Leu Val Val Gly Ile
   545 550 555 560
   Lys Val Pro Lys Tyr Glu Gly Arg Ala Gly Phe Ala Val Ile Lys Leu
   565 570 575
   Thr Asp Asn Ser Leu Asp Ile Thr Ala Lys Thr Lys Leu Leu Asn Asp
   580 585 590
   Ser Leu Ser Arg Leu Asn Leu Pro Ser Tyr Ala Met Pro Leu Phe Val
   595 600 605
   Lys Phe Val Asp Glu Ile Lys Met Thr Asp Asn Leu Ile Lys Phe
   610 615 620
<210> 99
   <211> 597
   <212> PRT
   <213> Mycobacterium tuberculosis
<400> 99
   Met Ser Asp Tyr Tyr Gly Gly Ala His Thr Thr Val Arg Leu Ile Asp
    1               5                  10                  15
   Leu Ala Thr Arg Met Pro Arg Val Leu Ala Asp Thr Pro Val Ile Val
               20                  25                  30
   Arg Gly Ala Met Thr Gly Leu Leu Ala Arg Pro Asn Ser Lys Ala Ser
           35                  40                  45
   Ile Gly Thr Val Phe Gln Asp Arg Ala Ala Arg Tyr Gly Asp Arg Val
       50                  55                  60
   Phe Leu Lys Phe Gly Asp Gln Gln Leu Thr Tyr Arg Asp Ala Asn Ala
   65                  70                  75                  80
   Thr Ala Asn Arg Tyr Ala Ala Val Leu Ala Ala Arg Gly Val Gly Pro
                   85                  90                  95
   Gly Asp Val Val Gly Ile Met Leu Arg Asn Ser Pro Ser Thr Val Leu
               100                 105                 110
   Ala Met Leu Ala Thr Val Lys Cys Gly Ala Ile Ala Gly Met Leu Asn
           115                 120                 125
   Tyr His Gln Arg Gly Glu Val Leu Ala His Ser Leu Gly Leu Leu Asp
       130                 135                 140
   Ala Lys Val Leu Ile Ala Glu Ser Asp Leu Val Ser Ala Val Ala Glu
   145                 150                 155                 160
   Cys Gly Ala Ser Arg Gly Arg Val Ala Gly Asp Val Leu Thr Val Glu
                   165                 170                 175
   Asp Val Glu Arg Phe Ala Thr Thr Ala Pro Ala Thr Asn Pro Ala Ser
               180                 185                 190
   Ala Ser Ala Val Gln Ala Lys Asp Thr Ala Phe Tyr Ile Phe Thr Ser
           195                 200                 205
   Gly Thr Thr Gly Phe Pro Lys Ala Ser Val Met Thr His His Arg Trp
       210                 215                 220
   Leu Arg Ala Leu Ala Val Phe Gly Gly Met Gly Leu Arg Leu Lys Gly
   225                 230                 235                 240
   Ser Asp Thr Leu Tyr Ser Cys Leu Pro Leu Tyr His Asn Asn Ala Leu
                   245                 250                 255
   Thr Val Ala Val Ser Ser Val Ile Asn Ser Gly Ala Thr Leu Ala Leu
               260                 265                 270
   Gly Lys Ser Phe Ser Ala Ser Arg Phe Trp Asp Glu Val Ile Ala Asn
           275                 280                 285
   Arg Ala Thr Ala Phe Val Tyr Ile Gly Glu Ile Cys Arg Tyr Leu Leu
       290                 295                 300
   Asn Gln Pro Ala Lys Pro Thr Asp Arg Ala His Gln Val Arg Val Ile
   305                 310                 315                 320
   Cys Gly Asn Gly Leu Arg Pro Glu Ile Trp Asp Glu Phe Thr Thr Arg
                   325                 330                 335
   Phe Gly Val Ala Arg Val Cys Glu Phe Tyr Ala Ala Ser Glu Gly Asn
               340                 345                 350
   Ser Ala Phe Ile Asn Ile Phe Asn Val Pro Arg Thr Ala Gly Val Ser
           355                 360                 365
   Pro Met Pro Leu Ala Phe Val Glu Tyr Asp Leu Asp Thr Gly Asp Pro
       370                 375                 380
   Leu Arg Asp Ala Ser Gly Arg Val Arg Arg Val Pro Asp Gly Glu Pro
   385                 390                 395                 400
   Gly Leu Leu Leu Ser Arg Val Asn Arg Leu Gln Pro Phe Asp Gly Tyr
                   405                 410                 415
   Thr Asp Pro Val Ala Ser Glu Lys Lys Leu Val Arg Asn Ala Phe Arg
               420                 425                 430
   Asp Gly Asp Cys Trp Phe Asn Thr Gly Asp Val Met Ser Pro Gln Gly
           435                 440                 445
   Met Gly His Ala Ala Phe Val Asp Arg Leu Gly Asp Thr Phe Arg Trp
       450                 455                 460
   Lys Gly Glu Asn Val Ala Thr Thr Gln Val Glu Ala Ala Leu Ala Ser
   465                 470                 475                 480
   Asp Gln Thr Val Glu Glu Cys Thr Val Tyr Gly Val Gln Ile Pro Arg
                   485                 490                 495
   Thr Gly Gly Arg Ala Gly Met Ala Ala Ile Thr Leu Arg Ala Gly Ala
               500                 505                 510
   Glu Phe Asp Gly Gln Ala Leu Ala Arg Thr Val Tyr Gly His Leu Pro
           515                 520                 525
   Gly Tyr Ala Leu Pro Leu Phe Val Arg Val Val Gly Ser Leu Ala His
       530                 535                 540
   Thr Thr Thr Phe Lys Ser Arg Lys Val Glu Leu Arg Asn Gln Ala Tyr
   545                 550                 555                 560
   Gly Ala Asp Ile Glu Asp Pro Leu Tyr Val Leu Ala Gly Pro Asp Glu
                   565                 570                 575
   Gly Tyr Val Pro Tyr Tyr Ala Glu Tyr Pro Glu Glu Val Ser Leu Gly
               580                 585                 590
   Arg Arg Pro Gln Gly
           595
<210> 100
   <211> 304
   <212> PRT
   <213> concensus FATP signature sequence
<400> 100
   Tyr Ile Tyr Thr Ser Gly Thr Thr Gly Leu Pro Lys Ala Ala Ile Ile
    1               5                  10                  15
   Val His Ser Arg Tyr Tyr Arg Gly Ala Ala Leu His Ser Gly Arg Met
               20                  25                  30
   Arg Pro Asp Val Val Tyr Asp Cys Leu Pro Leu Tyr His Ser Ala Ala
           35                  40                  45
   Leu Ile Leu Gly Ile Gly Gln Cys Leu Leu His Gly Ala Thr Val Val
       50                  55                  60
   Leu Arg Lys Lys Phe Ser Ala Ser Arg Phe Trp Asp Asp Cys Val Lys
   65                  70                  75                  80
   Tyr Asn Val Thr Val Ile Gln Tyr Ile Gly Glu Leu Cys Arg Tyr Leu
                   85                  90                  95
   Leu Asn Gln Pro Pro Arg Pro Ala Glu Arg Arg His Lys Val Arg Leu
               100                 105                 110
   Ala Val Gly Asn Gly Leu Arg Pro Asp Ile Trp Glu Glu Phe Val Ser
           115                 120                 125
   Arg Phe Gly Ile Pro Gln Ile Gly Glu Phe Tyr Gly Ala Thr Glu Gly
       130                 135                 140
   Asn Cys Ser Leu Met Asn Tyr Asp Gly Lys Val Gly Ala Cys Gly Ser
   145                 150                 155                 160
   Arg Ile Leu Lys Lys Val Tyr Pro Ile Arg Leu Val Lys Val Asp Glu
                   165                 170                 175
   Asp Thr Gly Glu Pro Ile Arg Asp Ala Gln Gly Leu Cys Ile Pro Cys
               180                 185                 190
   Gln Pro Gly Glu Pro Gly Leu Leu Val Gly Arg Ile Asn Gln Gln Asp
           195                 200                 205
   Pro Phe Arg Gly Phe Gly Tyr Gly Ser Glu Gly Ala Thr Asn Lys Lys
       210                 215                 220
   Ile Ala Arg Asp Val Phe Lys Lys Gly Asp Val Ala Phe Asn Thr Gly
   225                 230                 235                 240
   Asp Val Leu Val Met Asp Glu Leu Gly Tyr Leu Tyr Phe Arg Asp Arg
                   245                 250                 255
   Thr Gly Asp Thr Phe Arg Trp Lys Gly Glu Asn Val Ser Thr Thr Glu
               260                 265                 270
   Val Glu Gly Val Leu Ser Arg Leu Asp Phe Val Ala Glu Val Asn Val
           275                 280                 285
   Tyr Gly Val Thr Val Pro Gly His Glu Gly Arg Ala Gly Met Ala Ala
       290                 295                 300
<210> 101
   <211> 2166
   <212> DNA
   <213> Homo sapiens
<220>
   <221> CDS
   <222> (19)...(2124)
<400> 101
   cgacccacgc gtccgggg atg ttt gcg agc ggc tgg aac cag acg gtg ccg          51
                       Met Phe Ala Ser Gly Trp Asn Gln Thr Val Pro
                       1               5                   10
   ata gag gaa gcg ggc tcc atg gct gcc ctc ctg ctg ctg ccc ctg ctg          99
   Ile Glu Glu Ala Gly Ser Met Ala Ala Leu Leu Leu Leu Pro Leu Leu
               15                  20                  25
   ctg ttg cta ccg ctg ctg ctg ctg ctg aag cta cac ctc tgg ccg cag         147
   Leu Leu Leu Pro Leu Leu Leu Leu Leu Lys Leu His Leu Trp Pro Gln
           30                  35                  40
   ttg cgc tgg ctt ccg gcg gac ttg gcc ttt gcg gtg cga gct ctg tgc         195
   Leu Arg Trp Leu Pro Ala Asp Leu Ala Phe Ala Val Arg Ala Leu Cys
       45                  50                  55
   tgc aaa agg gct ctt cga gct cgc gcc ctg gcc gcg gct gcc gcc gac         243
   Cys Lys Arg Ala Leu Arg Ala Arg Ala Leu Ala Ala Ala Ala Ala Asp
   60                  65                  70                  75
   ccg gaa ggt ccc gag ggg ggc tgc agc ctg gcc tgg cgc ctc gcg gaa         291
   Pro Glu Gly Pro Glu Gly Gly Cys Ser Leu Ala Trp Arg Leu Ala Glu
                   80                  85                  90
   ctg gcc cag cag cgc gcc gcg cac acc ttt ctc att cac ggc tcg cgg         339
   Leu Ala Gln Gln Arg Ala Ala His Thr Phe Leu Ile His Gly Ser Arg
               95                  100                 105
   cgc ttt agc tac tca gag gcg gag cgc gag agt aac agg gct gca cgc      387
   Arg Phe Ser Tyr Ser Glu Ala Glu Arg Glu Ser Asn Arg Ala Ala Arg
           110                 115                 120
   gcc ttc cta cgt gcg cta ggc tgg gac tgg gga ccc gac ggc ggc gac      435
   Ala Phe Leu Arg Ala Leu Gly Trp Asp Trp Gly Pro Asp Gly Gly Asp
       125                 130                 135
   agc ggc gag ggg agc gct gga gaa ggc gag cgg gca gcg ccg gga gcc      483
   Ser Gly Glu Gly Ser Ala Gly Glu Gly Glu Arg Ala Ala Pro Gly Ala
   140                 145                 150                 155
   gga gat gca gcg gcc gga agc ggc gcg gag ttt gcc gga ggg gac ggt      531
   Gly Asp Ala Ala Ala Gly Ser Gly Ala Glu Phe Ala Gly Gly Asp Gly
                   160                 165                 170
   gcc gcc aga ggt gga gga gag ccc gcc gcc cct ctg tca cct gga gca      579
   Ala Ala Arg Gly Gly Gly Glu Pro Ala Ala Pro Leu Ser Pro Gly Ala
               175                 180                 185
   act gtg gcg ctg ctc ctc ccc gct ggc cca gag ttt ctg tgg ctc tgg      627
   Thr Val Ala Leu Leu Leu Pro Ala Gly Pro Glu Phe Leu Trp Leu Trp
           190                 195                 200
   ttc ggg ctg gcc aag gcc ggc ctg cgc act gcc ttt gtg ccc acc gcc      675
   Phe Gly Leu Ala Lys Ala Gly Leu Arg Thr Ala Phe Val Pro Thr Ala
       205                 210                 215
   ctg cgc cgg ggc ccc ctg ctg cac tgc ctc cgc agc tgc ggc gcg cgc      723
   Leu Arg Arg Gly Pro Leu Leu His Cys Leu Arg Ser Cys Gly Ala Arg
   220                 225                 230                 235
   gcg ctg gtg ctg gcg cca gag ttt ctg gag tcc ctg gag ccg gac ctg      771
   Ala Leu Val Leu Ala Pro Glu Phe Leu Glu Ser Leu Glu Pro Asp Leu
                   240                 245                 250
   ccc gcc ctg aga gcc atg ggg ctc cac ctg tgg gct gca ggc cca gga      819
   Pro Ala Leu Arg Ala Met Gly Leu His Leu Trp Ala Ala Gly Pro Gly
               255                 260                 265
   acc cac cct gct gga att agc gat ttg ctg gct gaa gtg tcc gct gaa      867
   Thr His Pro Ala Gly Ile Ser Asp Leu Leu Ala Glu Val Ser Ala Glu
           270                 275                 280
   gtg gat ggg cca gtg cca gga tac ctc tct tcc ccc cag agc ata aca      915
   Val Asp Gly Pro Val Pro Gly Tyr Leu Ser Ser Pro Gln Ser Ile Thr
       285                 290                 295
   gac acg tgc ctg tac atc ttc acc tct ggc acc acg ggc ctc ccc aag      963
   Asp Thr Cys Leu Tyr Ile Phe Thr Ser Gly Thr Thr Gly Leu Pro Lys
   300                 305                 310                 315
   gct gct cgg atc agt cat ctg aag atc ctg caa tgc cag ggc ttc tat     1011
   Ala Ala Arg Ile Ser His Leu Lys Ile Leu Gln Cys Gln Gly Phe Tyr
                   320                 325                 330
   cag ctg tgt ggt gtc cac cag gaa gat gtg atc tac ctc gcc ctc cca      1059
   Gln Leu Cys Gly Val His Gln Glu Asp Val Ile Tyr Leu Ala Leu Pro
               335                 340                 345
   ctc tac cac atg tcc ggt tcc ctg ctg ggc atc gtg ggc tgc atg ggc      1107
   Leu Tyr His Met Ser Gly Ser Leu Leu Gly Ile Val Gly Cys Met Gly
           350                 355                 360
   att ggg gcc aca gtg gtg ctg aaa tcc aag ttc tcg gct ggt cag ttc      1155
   Ile Gly Ala Thr Val Val Leu Lys Ser Lys Phe Ser Ala Gly Gln Phe
       365                 370                 375
   tgg gaa gat tgc cag cag cac agg gtg acg gtg ttc cag tac att ggg      1203
   Trp Glu Asp Cys Gln Gln His Arg Val Thr Val Phe Gln Tyr Ile Gly
   380                 385                 390                 395
   gag ctg tgc cga tac ctt gtc aac cag ccc ccg agc aag gca gaa cgt      1251
   Glu Leu Cys Arg Tyr Leu Val Asn Gln Pro Pro Ser Lys Ala Glu Arg
                   400                 405                 410
   ggc cat aag gtc cgg ctg gca gtg ggc agc ggg ctg cgc cca gat acc      1299
   Gly His Lys Val Arg Leu Ala Val Gly Ser Gly Leu Arg Pro Asp Thr
               415                 420                 425
   tgg gag cgt ttt gtg cgg cgc ttc ggg ccc ctg cag gtg ctg gag aca      1347
   Trp Glu Arg Phe Val Arg Arg Phe Gly Pro Leu Gln Val Leu Glu Thr
           430                 435                 440
   tat gga ctg aca gag ggc aac gtg gcc acc atc aac tac aca gga cag      1395
   Tyr Gly Leu Thr Glu Gly Asn Val Ala Thr Ile Asn Tyr Thr Gly Gln
       445                 450                 455
   cgg ggc gct gtg ggg cgt gct tcc tgg ctt tac aag cat atc ttc ccc      1443
   Arg Gly Ala Val Gly Arg Ala Ser Trp Leu Tyr Lys His Ile Phe Pro
   460                 465                 470                 475
   ttc tcc ttg att cgc tat gat gtc acc aca gga gag cca att cgg gac      1491
   Phe Ser Leu Ile Arg Tyr Asp Val Thr Thr Gly Glu Pro Ile Arg Asp
                   480                 485                 490
   ccc cag ggg cac tgt atg gcc aca tct cca ggt gag cca ggg ctg ctg      1539
   Pro Gln Gly His Cys Met Ala Thr Ser Pro Gly Glu Pro Gly Leu Leu
               495                 500                 505
   gtg gcc ccg gta agc cag cag tcc cca ttc ctg ggc tat gct ggc ggg      1587
   Val Ala Pro Val Ser Gln Gln Ser Pro Phe Leu Gly Tyr Ala Gly Gly
           510                 515                 520
   cca gag ctg gcc cag ggg aag ttg cta aag gat gtc ttc cgg cct ggg      1635
   Pro Glu Leu Ala Gln Gly Lys Leu Leu Lys Asp Val Phe Arg Pro Gly
       525                 530                 535
   gat gtt ttc ttc aac act ggg gac ctg ctg gtc tgc gat gac caa ggt      1683
   Asp Val Phe Phe Asn Thr Gly Asp Leu Leu Val Cys Asp Asp Gln Gly
   540                 545                 550                 555
   ttt ctc cgc ttc cat gat cgt act gga gac acc ttc agg tgg aag ggg      1731
   Phe Leu Arg Phe His Asp Arg Thr Gly Asp Thr Phe Arg Trp Lys Gly
                   560                 565                 570
   gag aat gtg gcc aca acc gag gtg gca gag gtc ttc gag gcc cta gat      1779
   Glu Asn Val Ala Thr Thr Glu Val Ala Glu Val Phe Glu Ala Leu Asp
               575                 580                 585
   ttt ctt cag gag gtg aac gtc tat gga gtc act gtg cca ggg cat gaa      1827
   Phe Leu Gln Glu Val Asn Val Tyr Gly Val Thr Val Pro Gly His Glu
           590                 595                 600
   ggc agg gct gga atg gca gcc cta gtt ctg cgt ccc ccc cac gct ttg      1875
   Gly Arg Ala Gly Met Ala Ala Leu Val Leu Arg Pro Pro His Ala Leu
       605                 610                 615
   gac ctt atg cag ctc tac acc cac gtg tct gag aac ttg cca cct tat      1923
   Asp Leu Met Gln Leu Tyr Thr His Val Ser Glu Asn Leu Pro Pro Tyr
   620                 625                 630                 635
   gcc cgg ccc cga ttc ctc agg ctc cag gag tct ttg gcc acc aca gag      1971
   Ala Arg Pro Arg Phe Leu Arg Leu Gln Glu Ser Leu Ala Thr Thr Glu
                   640                 645                 650
   acc ttc aaa cag cag aaa gtt cgg atg gca aat gag ggc ttc gac ccc      2019
   Thr Phe Lys Gln Gln Lys Val Arg Met Ala Asn Glu Gly Phe Asp Pro
               655                 660                 665
   agc acc ctg tct gac cca ctg tac gtt ctg gac cag gct gta ggt gcc      2067
   Ser Thr Leu Ser Asp Pro Leu Tyr Val Leu Asp Gln Ala Val Gly Ala
           670                 675                 680
   tac ctg ccc ctc aca act gcc cgg tac agc gcc ctc ctg gca gga aac      2115
   Tyr Leu Pro Leu Thr Thr Ala Arg Tyr Ser Ala Leu Leu Ala Gly Asn
       685                 690                 695
   ctt cga atc tgagaacttc cacacctgag gcacctgaga gaggaactct              2164
   Leu Arg Ile
   700
   gt                                                                   2166
<210> 102
   <211> 702
   <212> PRT
   <213> Homo sapiens
<400> 102
   Met Phe Ala Ser Gly Trp Asn Gln Thr Val Pro Ile Glu Glu Ala Gly
    1               5                  10                  15
   Ser Met Ala Ala Leu Leu Leu Leu Pro Leu Leu Leu Leu Leu Pro Leu
               20                  25                  30
   Leu Leu Leu Leu Lys Leu His Leu Trp Pro Gln Leu Arg Trp Leu Pro
           35                  40                  45
   Ala Asp Leu Ala Phe Ala Val Arg Ala Leu Cys Cys Lys Arg Ala Leu
       50                  55                  60
   Arg Ala Arg Ala Leu Ala Ala Ala Ala Ala Asp Pro Glu Gly Pro Glu
   65 70 75 80
   Gly Gly Cys Ser Leu Ala Trp Arg Leu Ala Glu Leu Ala Gln Gln Arg
   85 90 95
   Ala Ala His Thr Phe Leu Ile His Gly Ser Arg Arg Phe Ser Tyr Ser
   100 105 110
   Glu Ala Glu Arg Glu Ser Asn Arg Ala Ala Arg Ala Phe Leu Arg Ala
   115 120 125
   Leu Gly Trp Asp Trp Gly Pro Asp Gly Gly Asp Ser Gly Glu Gly Ser
   130 135 140
   Ala Gly Glu Gly Glu Arg Ala Ala Pro Gly Ala Gly Asp Ala Ala Ala
   145 150 155 160
   Gly Ser Gly Ala Glu Phe Ala Gly Gly Asp Gly Ala Ala Arg Gly Gly
   165 170 175
   Gly Glu Pro Ala Ala Pro Leu Ser Pro Gly Ala Thr Val Ala Leu Leu
   180 185 190
   Leu Pro Ala Gly Pro Glu Phe Leu Trp Leu Trp Phe Gly Leu Ala Lys
   195 200 205
   Ala Gly Leu Arg Thr Ala Phe Val Pro Thr Ala Leu Arg Arg Gly Pro
   210 215 220
   Leu Leu His Cys Leu Arg Ser Cys Gly Ala Arg Ala Leu Val Leu Ala
   225 230 235 240
   Pro Glu Phe Leu Glu Ser Leu Glu Pro Asp Leu Pro Ala Leu Arg Ala
   245 250 255
   Met Gly Leu His Leu Trp Ala Ala Gly Pro Gly Thr His Pro Ala Gly
   260 265 270
   Ile Ser Asp Leu Leu Ala Glu Val Ser Ala Glu Val Asp Gly Pro Val
   275 280 285
   Pro Gly Tyr Leu Ser Ser Pro Gln Ser Ile Thr Asp Thr Cys Leu Tyr
   290 295 300
   Ile Phe Thr Ser Gly Thr Thr Gly Leu Pro Lys Ala Ala Arg Ile Ser
   305 310 315 320
   His Leu Lys Ile Leu Gln Cys Gln Gly Phe Tyr Gln Leu Cys Gly Val
   325 330 335
   His Gln Glu Asp Val Ile Tyr Leu Ala Leu Pro Leu Tyr His Met Ser
   340 345 350
   Gly Ser Leu Leu Gly Ile Val Gly Cys Met Gly Ile Gly Ala Thr Val
   355 360 365
   Val Leu Lys Ser Lys Phe Ser Ala Gly Gln Phe Trp Glu Asp Cys Gln
   370 375 380
   Gln His Arg Val Thr Val Phe Gln Tyr Ile Gly Glu Leu Cys Arg Tyr
   385 390 395 400
   Leu Val Asn Gln Pro Pro Ser Lys Ala Glu Arg Gly His Lys Val Arg
   405 410 415
   Leu Ala Val Gly Ser Gly Leu Arg Pro Asp Thr Trp Glu Arg Phe Val
   420 425 430
   Arg Arg Phe Gly Pro Leu Gln Val Leu Glu Thr Tyr Gly Leu Thr Glu
   435 440 445
   Gly Asn Val Ala Thr Ile Asn Tyr Thr Gly Gln Arg Gly Ala Val Gly
   450 455 460
   Arg Ala Ser Trp Leu Tyr Lys His Ile Phe Pro Phe Ser Leu Ile Arg
   465 470 475 480
   Tyr Asp Val Thr Thr Gly Glu Pro Ile Arg Asp Pro Gln Gly His Cys
   485 490 495
   Met Ala Thr Ser Pro Gly Glu Pro Gly Leu Leu Val Ala Pro Val Ser
   500 505 510
   Gln Gln Ser Pro Phe Leu Gly Tyr Ala Gly Gly Pro Glu Leu Ala Gln
           515                 520                 525
   Gly Lys Leu Leu Lys Asp Val Phe Arg Pro Gly Asp Val Phe Phe Asn
       530                 535                 540
   Thr Gly Asp Leu Leu Val Cys Asp Asp Gln Gly Phe Leu Arg Phe His
   545                 550                 555                 560
   Asp Arg Thr Gly Asp Thr Phe Arg Trp Lys Gly Glu Asn Val Ala Thr
                   565                 570                 575
   Thr Glu Val Ala Glu Val Phe Glu Ala Leu Asp Phe Leu Gln Glu Val
               580                 585                 590
   Asn Val Tyr Gly Val Thr Val Pro Gly His Glu Gly Arg Ala Gly Met
           595                 600                 605
   Ala Ala Leu Val Leu Arg Pro Pro His Ala Leu Asp Leu Met Gln Leu
       610                 615                 620
   Tyr Thr His Val Ser Glu Asn Leu Pro Pro Tyr Ala Arg Pro Arg Phe
   625                 630                 635                 640
   Leu Arg Leu Gln Glu Ser Leu Ala Thr Thr Glu Thr Phe Lys Gln Gln
                   645                 650                 655
   Lys Val Arg Met Ala Asn Glu Gly Phe Asp Pro Ser Thr Leu Ser Asp
               660                 665                 670
   Pro Leu Tyr Val Leu Asp Gln Ala Val Gly Ala Tyr Leu Pro Leu Thr
           675                 680                 685
   Thr Ala Arg Tyr Ser Ala Leu Leu Ala Gly Asn Leu Arg Ile
       690                 695                 700
<210> 103
   <211> 19
   <212> DNA
   <213> Artificial Sequence
<220>
   <223> Oligonucleotide
<400> 103
   cccccaccag agaggctcc 19
<210> 104
   <211> 19
   <212> DNA
   <213> Artificial Sequence
<220>
   <223> Oligonucleotide
<400> 104
   ccacccccgg aaagcctgc 19
<210> 105
   <211> 19
   <212> DNA
   <213> Artificial Sequence
<220>
   <223> Oligonucleotide
<400> 105
   ggagcctctc tggtggggg 19